# EUROPEAN PATENT APPLICATION

(11) **EP 4 843 985 A1**
(43) Date of publication of application: **25.11.2026**
(21) Application number: 25741493.8
(22) Date of filing: 14.01.2025
(51) Int. Cl.: C07D 487/04, C07D 487/16, C07D 519/00, A61K 31/519, A61P 35/00

(54) **TRIAZOLOPYRIMIDINE DERIVATIVE, PREPARATION METHOD THEREFOR AND USE THEREOF**

(30) Priority: 15.01.2024 CN 202410058492; 06.02.2024 CN 202410172162; 18.02.2024 CN 202410181991; 26.03.2024 CN 202410355630; 30.04.2024 CN 202410545121; 10.05.2024 CN 202410580648; 14.05.2024 CN 202410599924; 16.05.2024 CN 202410613540; 12.06.2024 CN 202410758291; 31.07.2024 CN 202411046535; 07.11.2024 CN 202411591670; 08.01.2025 CN 202510033487
(71) Applicant: Shanghai Qilu Pharmaceutical Research and Development Centre Ltd., Shanghai 201203 (CN)
(72) Inventor: ZHU, Weibo, Shanghai 201203 (CN); FU, Jiasheng, Shanghai 201203 (CN); SUN, Daqing, Shanghai 201203 (CN); ZHANG, Wen, Shanghai 201203 (CN); XU, Kai, Shanghai 201203 (CN); SUN, Weimei, Shanghai 201203 (CN); TAO, Weikang, Shanghai 201203 (CN)
(74) Representative: Cameron Intellectual Property Ltd
(86) International application number: PCT/CN2025/072316
(87) International publication number: WO 2025/152932

(57) **Abstract**

A triazolopyrimidine derivative, a preparation method therefor and a use thereof. The present invention particularly relates to a compound represented by general formula (IC) or a stereoisomer thereof, a preparation method therefor, a pharmaceutical composition containing the compound, and a use of the compound in preparing a drug for treating diseases. The compound has a structure represented by formula (IC).

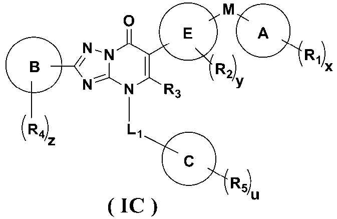

## Description

The present application claims priority to Chinese Patent Application No. 2024100584923 filed on January 15, 2024; Chinese Patent Application No. 2024101721627 filed on February 6, 2024; Chinese Patent Application No. 2024101819911 filed on February 18, 2024; Chinese Patent Application No. 2024103556304 filed on March 26, 2024; Chinese Patent Application No. 2024105451218 filed on April 30, 2024; Chinese Patent Application No. 2024105806484 filed on May 10, 2024; Chinese Patent Application No. 2024105999241 filed on May 14, 2024; Chinese Patent Application No. 2024106135400 filed on May 16, 2024; Chinese Patent Application No. 2024107582914 filed on June 12, 2024; Chinese Patent Application No. 2024110465352 filed on July 31, 2024; Chinese Patent Application No. 2024115916705 filed on November 7, 2024; and Chinese Patent Application No. 2025100334871 filed on January 8, 2025. The contents of the above Chinese patent applications are incorporated herein by reference in their entirety.

### TECHNICAL FIELD

The present disclosure belongs to the field of medicinal chemistry, and specifically relates to a triazolopyrimidine derivative, a preparation method therefor, and a use thereof.

### BACKGROUND

The RecQ helicase subfamily is a class of enzymes capable of unwinding nucleotide double strands. Within cells, they are involved in DNA replication, repair, transcription, recombination, as well as RNA splicing, ribosome assembly, protein translation, and telomere stability. This family was first discovered in prokaryotes. The members of the RecQ family are highly conserved from prokaryotes (RecQ) to yeast (sgs1) and humans (five RecQ helicases: RECQ1, BLM, WRN, RECQ4, and RECQ5). WRN is a member of the RecQ family of DNA helicases, consisting of 1432 amino acids with a molecular mass of 160 KD. WRN is primarily localized in the nucleolus and rapidly translocates to other nuclear regions upon DNA damage. WRN is the only member of the human RecQ helicase family that possesses 3'→5' exonuclease activity. Its N-terminal exonuclease domain facilitates WRN in processing complex nucleic acid structures, while the central helicase domain can bind and hydrolyze ATP, thereby providing energy to unwind double-stranded DNA. The C-terminal HRDC domain can regulate the catalytic activity of WRN binding to DNA.

Microsatellite (MS) refers to DNA sequences composed of tandem repeats of a few nucleotides (mostly 1 to 6) as a unit in the cellular genome, also known as short tandem repeat (STR). When the function of DNA mismatch repair (NMR) is abnormal, replication errors occurring in microsatellites are not corrected and accumulate continuously, resulting in changes in the length or base composition of microsatellite sequences, which is termed microsatellite instability (MSI), and simultaneously leading to a hypermutable phenotype in the genome. In tumors, functional deletion mutations of key MMR genes (MLH1, MSH2, MSH6, and PMS2) and their related genes, or loss of MLH1 expression caused by hypermethylation of the MLH1 promoter region, often result in defects in the DNA mismatch repair (MMR) function. Based on the microsatellite stability status of tumor cells, tumors can be classified into microsatellite instability (MSI) tumors and microsatellite stability (MSS) tumors. MSI is prevalent in cancers, with solid tumors having high incidence rates including endometrial cancer (approximately 30%), gastric cancer (approximately 20%), and colorectal cancer (approximately 15%), among others.

There is a synthetic lethal relationship between WRN and MSI-H: in MSI tumor cell lines, knockout of WRN leads to characteristics such as genomic instability, cell cycle arrest, and DNA double-strand breaks in these cancer cells, ultimately resulting in the death of the cancer cells. Therefore, by developing small molecule inhibitors targeting WRN, it is expected to achieve selective killing of MSI-H tumor cells, providing personalized anti-tumor therapy for MSI tumor patients.

The protein product encoded by the hERG gene is the inner pore-forming part of a key membrane-bound potassium ion channel in myocardial tissue, which is controlled by membrane potential and regulates the efflux of potassium ions from cells in a gated manner. When a compound binds to the hERG potassium channel, it blocks the efflux of potassium ions, thereby prolonging the repolarization time of cardiomyocytes, which is reflected as QT interval prolongation on an electrocardiogram, and may further induce the fatal risk of TdP. Therefore, inhibition of hERG can lead to severe toxic side effects. The larger the IC₅₀ of a compound, the lower the risk of hERG inhibition.

### SUMMARY

The present disclosure aims to provide a triazolopyrimidine derivative, a preparation method therefor, and a use thereof.

The present disclosure provides a compound of formula (IC), a stereoisomer thereof, or a pharmaceutically acceptable salt thereof: 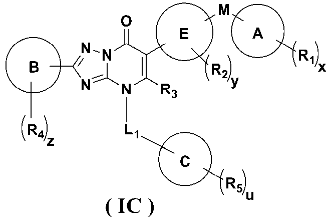 wherein
M is selected from the group consisting of a bond, CO, -NHCO-, -C(R₆₋₁R₆₋₂)-, and 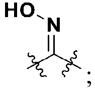
ring A is selected from the group consisting of C₆₋₁₄ aryl and 5- to 14-membered heteroaryl;
ring B is selected from the group consisting of C₃₋₁₂ cycloalkyl, C₃₋₁₂ cycloalkenyl, 3-to 12-membered heterocycloalkyl, 3- to 12-membered heterocycloalkenyl, C₆₋₁₄ aryl, and 5- to 14-membered heteroaryl; alternatively, ring B is absent, and R₄ is directly attached to the parent structure;
ring C is selected from the group consisting of C₃₋₁₂ cycloalkyl, C₆₋₁₄ aryl, and 5- to 14-membered heteroaryl;
ring E is selected from the group consisting of C₃₋₁₂ cycloalkylene, C₃₋₁₂ cycloalkenylene, 3- to 12-membered heterocycloalkylene, 3- to 12-membered heterocycloalkenylene, C₆₋₁₄ arylene, and 5- to 14-membered heteroarylene; alternatively, ring E is directly attached to the parent to form 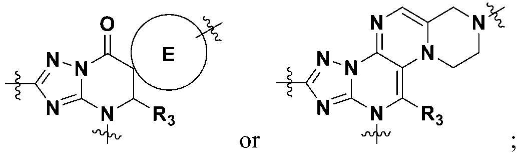
Li is selected from the group consisting of a bond, -O-, -S-, imino, C₁₋₆ alkylene, - CONH-, -CO-C₁₋₆ alkylene-NH-, -C₁₋₆ alkylene-CONH-, -C₁₋₆ alkylene-CONH-C₁₋₆ alkylene, -C₁₋₆ alkylene-SO₂NH-, -C₁₋₆ alkylene-NHCONH-, -C₁₋₆ alkylene-NHCO-, -C₁₋₆ alkylene-NHSO₂-, -C₁₋₆ alkylene-CO-, -C₁₋₆ alkylene-C(=N-OH)NH-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -C₁₋₆ alkylene-3- to 8-membered heterocycloalkylene-, -C₁₋₆ alkylene-3- to 8-membered heterocycloalkenylene-, -C₁₋₆ alkylene-C₆₋₁₄ arylene-, C₆₋₁₄ arylene, -C₁₋₆ alkylene-5- to 14-membered heteroarylene-, and 5- to 14-membered heteroarylene, wherein the imino, C₁₋₆ alkylene, -CONH-, -CO-C₁₋₆ alkylene-NH-, -C₁₋₆ alkylene-CONH-, -C₁₋₆ alkylene-CONH-C₁₋₆ alkylene, -C₁₋₆ alkylene-SO₂NH-, -C₁₋₆ alkylene-NHCONH-, -C₁₋₆ alkylene-NHCO-, -C₁₋₆ alkylene-NHSO₂-, -C₁₋₆ alkylene-CO-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -C₁₋₆ alkylene-3- to 8-membered heterocycloalkylene-, -C₁₋₆ alkylene-3- to 8-membered heterocycloalkenylene-, - C₁₋₆ alkylene-C₆₋₁₄ arylene-, C₆₋₁₄ arylene, -C₁₋₆ alkylene-5- to 14-membered heteroarylene-, or 5- to 14-membered heteroarylene is optionally further substituted with one or more substituents selected from the group consisting of deuterium, halogen, amino, hydroxyl, cyano, oxo, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5-to 14-membered heteroaryl;
alternatively, L₁, R₃, and the parent form a moiety 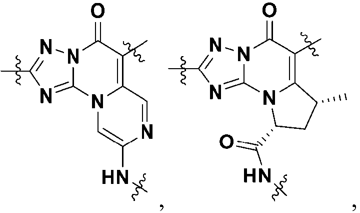 or 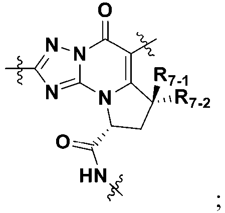
R₁ is independently selected from the group consisting of hydrogen, deuterium, halogen, amino, hydroxyl, mercapto, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₁ₐ; the R₁ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₂ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₂ₐ; the R₂ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
alternatively, two R₂ are connected to each other to form a C₃₋₈ cycloalkyl or a 3- to 8-membered heterocycloalkyl, wherein the C₃₋₈ cycloalkyl or 3- to 8-membered heterocycloalkyl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
alternatively, one R₂ and R₃ are connected to form a 5- to 8-membered heterocycloalkenyl, wherein the 5- to 8-membered heterocycloalkenyl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₃ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₃ₐ; the R₃ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
Li and R₃ are connected to form a 3- to 8-membered heterocycloalkenyl or a 5- to 10-membered heteroaryl; wherein the 3- to 8-membered heterocycloalkenyl or 5- to 10-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, 5- to 14-membered heteroaryl, -COR₄₋₁, - COOR₄₋₁, -OC(=O)R₄₋₁, -SO₂R₄₋₂, and -OR₄₋₃, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₄ₐ; the R₄ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl; alternatively, two R₄ₐ are connected to form a 3- to 8-membered heterocycloalkyl or a 3- to 8-membered heterocycloalkenyl;
R₄₋₁ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄₋₂ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄₋₃ is selected from the group consisting of hydrogen, deuterium, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₃₋₈ cycloalkyl, 3- to 8-membered heterocycloalkyl, C₆₋₁₄ aryl, and 5- to 14-membered heteroaryl, wherein the C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₅ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, -SO₂-C₁₋₆ alkyl, -SF₅, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₅ₐ; the R₅ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₆₋₁ and R₆₋₂ are independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₅ₐ; the R₅ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
alternatively, R₆₋₁ and R₆₋₂, together with the carbon atom to which they are attached, form a C₃₋₈ cycloalkyl or a 3- to 8-membered heterocycloalkyl;
R₇₋₁ and R₇₋₂ are each independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, and C₁₋₆ haloalkoxy;
alternatively, R₇₋₁ and R₇₋₂ are connected to form a C₃₋₈ cycloalkyl;
x is selected from the group consisting of 0, 1, 2, and 3;
y is selected from the group consisting of 0, 1, 2, 3, and 4;
z is selected from the group consisting of 0, 1, 2, 3, and 4;
u is selected from the group consisting of 0, 1, 2, 3, and 4.

The present disclosure provides a compound of formula (IC), a stereoisomer thereof, or a pharmaceutically acceptable salt thereof: 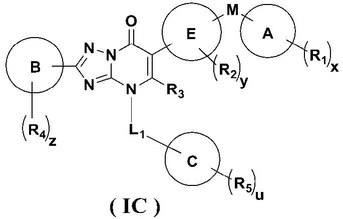 wherein
M is selected from the group consisting of a bond, CO, -NHCO-, -C(R₆₋₁R₆₋₂)-, and 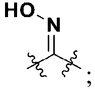
ring A is selected from the group consisting of C₆₋₁₄ aryl and 5- to 14-membered heteroaryl;
ring B is selected from the group consisting of C₃₋₁₂ cycloalkyl, C₃₋₁₂ cycloalkenyl, 3-to 12-membered heterocycloalkyl, 3- to 12-membered heterocycloalkenyl, C₆₋₁₄ aryl, and 5- to 14-membered heteroaryl; alternatively, ring B is absent, and R₄ is directly attached to the parent structure;
ring C is selected from the group consisting of C₃₋₁₂ cycloalkyl, C₆₋₁₄ aryl, and 5- to 14-membered heteroaryl;
ring E is selected from the group consisting of C₃₋₁₂ cycloalkylene, C₃₋₁₂ cycloalkenylene, 3- to 12-membered heterocycloalkylene, 3- to 12-membered heterocycloalkenylene, C₆₋₁₄ arylene, and 5- to 14-membered heteroarylene; alternatively, ring E is directly attached to the parent to form 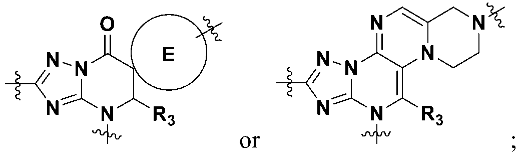
Li is selected from the group consisting of a bond, -O-, -S-, imino, C₁₋₆ alkylene, - CONH-, -CO-C₁₋₆ alkylene-NH-, -C₁₋₆ alkylene-CONH-, -C₁₋₆ alkylene-CONH-C₁₋₆ alkylene, -C₁₋₆ alkylene-SO₂NH-, -C₁₋₆ alkylene-NHCONH-, -C₁₋₆ alkylene-NHCO-, -C₁₋₆ alkylene-NHSO₂-, -C₁₋₆ alkylene-CO-, -C₁₋₆ alkylene-C(=N-OH)NH-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -C₁₋₆ alkylene-3- to 8-membered heterocycloalkylene-, -C₁₋₆ alkylene-3- to 8-membered heterocycloalkenylene-, -C₁₋₆ alkylene-C₆₋₁₄ arylene-, C₆₋₁₄ arylene, -C₁₋₆ alkylene-5- to 14-membered heteroarylene-, and 5- to 14-membered heteroarylene, wherein the imino, C₁₋₆ alkylene, -CONH-, -CO-C₁₋₆ alkylene-NH-, -C₁₆ alkylene-CONH-, -C₁₋₆ alkylene-CONH-C₁₋₆ alkylene, -C₁₋₆ alkylene-SO₂NH-, -C₁₋₆ alkylene-NHCONH-, -C₁₋₆ alkylene-NHCO-, -C₁₋₆ alkylene-NHSO₂-, -C₁₋₆ alkylene-CO-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -C₁₋₆ alkylene-3- to 8-membered heterocycloalkylene-, -C₁₋₆ alkylene-3- to 8-membered heterocycloalkenylene-, - C₁₋₆ alkylene-C₆₋₁₄ arylene-, C₆₋₁₄ arylene, -C₁₋₆ alkylene-5- to 14-membered heteroarylene-, or 5- to 14-membered heteroarylene is optionally further substituted with one or more substituents selected from the group consisting of deuterium, halogen, amino, hydroxyl, cyano, oxo, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5-to 14-membered heteroaryl;
alternatively, L₁, R₃, and the parent form a moiety 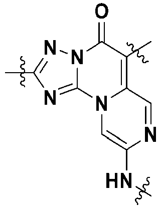 or 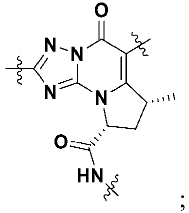
R₁ is independently selected from the group consisting of hydrogen, deuterium, halogen, amino, hydroxyl, mercapto, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₁ₐ; the R₁ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₂ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₂ₐ; the R₂ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
alternatively, two R₂ are connected to each other to form a C₃₋₈ cycloalkyl or a 3- to 8-membered heterocycloalkyl, wherein the C₃₋₈ cycloalkyl or 3- to 8-membered heterocycloalkyl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
alternatively, one R₂ and R₃ are connected to form a 5- to 8-membered heterocycloalkenyl, wherein the 5- to 8-membered heterocycloalkenyl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₃ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₃ₐ; the R₃ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
Li and R₃ are connected to form a 3- to 8-membered heterocycloalkenyl or a 5- to 10-membered heteroaryl; wherein the 3- to 8-membered heterocycloalkenyl or 5- to 10-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, 5- to 14-membered heteroaryl, -COR₄₋₁, - COOR₄₋₁, -OC(=O)R₄₋₁, -SO₂R₄₋₂, and -OR₄₋₃, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₄ₐ; the R₄ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl; alternatively, two R₄ₐ are connected to form a 3- to 8-membered heterocycloalkyl or a 3- to 8-membered heterocycloalkenyl;
R₄₋₁ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄₋₂ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄₋₃ is selected from the group consisting of hydrogen, deuterium, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₃₋₈ cycloalkyl, 3- to 8-membered heterocycloalkyl, C₆₋₁₄ aryl, and 5- to 14-membered heteroaryl, wherein the C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₅ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, -SO₂-C₁₋₆ alkyl, -SF₅, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₅ₐ; the R₅ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₆₋₁ and R₆₋₂ are each independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₅ₐ; the R₅ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
alternatively, R₆₋₁ and R₆₋₂, together with the carbon atom to which they are attached, form a C₃₋₈ cycloalkyl or a 3- to 8-membered heterocycloalkyl;
x is selected from the group consisting of 0, 1, 2, and 3;
y is selected from the group consisting of 0, 1, 2, 3, and 4;
z is selected from the group consisting of 0, 1, 2, 3, and 4;
u is selected from the group consisting of 0, 1, 2, 3, and 4.

The present disclosure provides a compound of formula (IC), a stereoisomer thereof, or a pharmaceutically acceptable salt thereof: 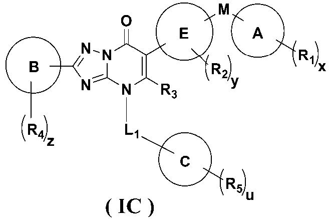 wherein
M is selected from the group consisting of a bond, CO, -NHCO-, -C(R₆₋₁R₆₋₂)-, and 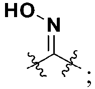
ring A is selected from the group consisting of C₆₋₁₄ aryl and 5- to 14-membered heteroaryl;
ring B is selected from the group consisting of C₃₋₁₂ cycloalkyl, C₃₋₁₂ cycloalkenyl, 3-to 12-membered heterocycloalkyl, 3- to 12-membered heterocycloalkenyl, C₆₋₁₄ aryl, and 5- to 14-membered heteroaryl; or, ring B is absent, and R₄ is directly attached to the parent structure;
ring C is selected from the group consisting of C₃₋₁₂ cycloalkyl, C₆₋₁₄ aryl, and 5- to 14-membered heteroaryl;
ring E is selected from the group consisting of C₃₋₁₂ cycloalkylene, C₃₋₁₂ cycloalkenylene, 3- to 12-membered heterocycloalkylene, 3- to 12-membered heterocycloalkenylene, C₆₋₁₄ arylene, and 5- to 14-membered heteroarylene; alternatively, ring E is directly attached to the parent to form 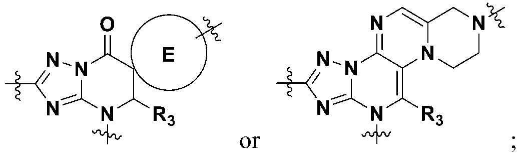
Li is selected from the group consisting of a bond, -O-, -S-, imino, C₁₋₆ alkylene, - CONH-, -CO-C₁₋₆ alkylene-NH-, -C₁₋₆ alkylene-CONH-, -C₁₋₆ alkylene-CONH-C₁₋₆ alkylene, -C₁₋₆ alkylene-SO₂NH-, -C₁₋₆ alkylene-NHCONH-, -C₁₋₆ alkylene-NHCO-, -C₁₋₆ alkylene-NHSO₂-, -C₁₋₆ alkylene-CO-, -C₁₋₆ alkylene-C(=N-OH)NH-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -C₁₋₆ alkylene-3- to 8-membered heterocycloalkylene-, -C₁₋₆ alkylene-3- to 8-membered heterocycloalkenylene-, -C₁₋₆ alkylene-C₆₋₁₄ arylene-, C₆₋₁₄ arylene, -C₁₋₆ alkylene-5- to 14-membered heteroarylene-, and 5- to 14-membered heteroarylene, wherein the imino, C₁₋₆ alkylene, -CONH-, -CO-C₁₋₆ alkylene-NH-, -C₁₋₆ alkylene-CONH-, -C₁₋₆ alkylene-CONH-C₁₋₆ alkylene, -C₁₋₆ alkylene-SO₂NH-, -C₁₋₆ alkylene-NHCONH-, -C₁₋₆ alkylene-NHCO-, -C₁₋₆ alkylene-NHSO₂-, -C₁₋₆ alkylene-CO-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -C₁₋₆ alkylene-3- to 8-membered heterocycloalkylene-, -C₁₋₆ alkylene-3- to 8-membered heterocycloalkenylene-, - C₁₋₆ alkylene-C₆₋₁₄ arylene-, C₆₋₁₄ arylene, -C₁₋₆ alkylene-5- to 14-membered heteroarylene-, or 5- to 14-membered heteroarylene is optionally further substituted with one or more substituents selected from the group consisting of deuterium, halogen, amino, hydroxyl, cyano, oxo, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5-to 14-membered heteroaryl;
alternatively, L₁, R₃, and the parent form a moiety 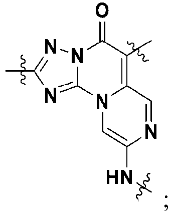
R₁ is independently selected from the group consisting of hydrogen, deuterium, halogen, amino, hydroxyl, mercapto, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₁ₐ; the R₁ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₂ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₂ₐ; the R₂ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
alternatively, two R₂ are connected to each other to form a C₃₋₈ cycloalkyl or a 3- to 8-membered heterocycloalkyl, wherein the C₃₋₈ cycloalkyl or 3- to 8-membered heterocycloalkyl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
alternatively, one R₂ and R₃ are connected to form a 5- to 8-membered heterocycloalkenyl, wherein the 5- to 8-membered heterocycloalkenyl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₃ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₃ₐ; the R₃ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
L₁ and R₃ are connected to form a 3- to 8-membered heterocycloalkenyl or a 5- to 10-membered heteroaryl; wherein the 3- to 8-membered heterocycloalkenyl or 5- to 10-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, 5- to 14-membered heteroaryl, -COR₄₋₁, - COOR₄₋₁, -OC(=O)R₄₋₁, -SO₂R₄₋₂, and -OR₄₋₃, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₄ₐ; the R₄ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl; alternatively, two R₄ₐ are connected to form a 3- to 8-membered heterocycloalkyl or a 3- to 8-membered heterocycloalkenyl;
R₄₋₁ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄₋₂ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄₋₃ is selected from the group consisting of hydrogen, deuterium, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₃₋₈ cycloalkyl, 3- to 8-membered heterocycloalkyl, C₆₋₁₄ aryl, and 5- to 14-membered heteroaryl, wherein the C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₅ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₅ₐ; the R₅ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₆₋₁ and R₆₋₂ are each independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₅ₐ; the R₅ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
alternatively, R₆₋₁ and R₆₋₂, together with the carbon atom to which they are attached, form a C₃₋₈ cycloalkyl or a 3- to 8-membered heterocycloalkyl;
x is selected from the group consisting of 0, 1, 2, and 3;
y is selected from the group consisting of 0, 1, 2, 3, and 4;
z is selected from the group consisting of 0, 1, 2, 3, and 4;
u is selected from the group consisting of 0, 1, 2, 3, and 4.

The present disclosure provides a compound of formula (IC), a stereoisomer thereof, or a pharmaceutically acceptable salt thereof: 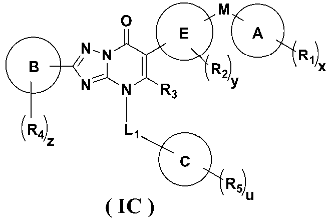 wherein
M is selected from the group consisting of a bond, CO, -NHCO-, and -C(R₆₋₁R₆₋₂)-;
ring A is selected from the group consisting of C₆₋₁₄ aryl and 5- to 14-membered heteroaryl;
ring B is selected from the group consisting of C₃₋₁₂ cycloalkyl, C₃₋₁₂ cycloalkenyl, 3-to 12-membered heterocycloalkyl, 3- to 12-membered heterocycloalkenyl, C₆₋₁₄ aryl, and 5- to 14-membered heteroaryl; or, ring B is absent, and R₄ is directly attached to the parent structure;
ring C is selected from the group consisting of C₆₋₁₄ aryl and 5- to 14-membered heteroaryl;
ring E is selected from the group consisting of C₃₋₁₂ cycloalkylene, C₃₋₁₂ cycloalkenylene, 3- to 12-membered heterocycloalkylene, 3- to 12-membered heterocycloalkenylene, C₆₋₁₄ arylene, and 5- to 14-membered heteroarylene; alternatively, ring E is directly attached to the parent to form 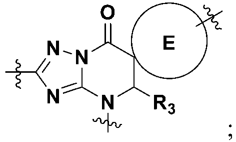
Li is selected from the group consisting of a bond, -O-, -S-, imino, C₁₋₆ alkylene, - CONH-, -CO-C₁₋₆ alkylene-NH-, -C₁₋₆ alkylene-CONH-, -C₁₋₆ alkylene-CONH-C₁₋₆ alkylene, -C₁₋₆ alkylene-SO₂NH-, -C₁₋₆ alkylene-NHCONH-, -C₁₋₆ alkylene-NHCO-, -C₁₋₆ alkylene-NHSO₂-, -C₁₋₆ alkylene-CO-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -C₁₋₆ alkylene-3- to 8-membered heterocycloalkylene-, -C₁₋₆ alkylene-3- to 8-membered heterocycloalkenylene-, -C₁₋₆ alkylene-C₆₋₁₄ arylene-, C₆₋₁₄ arylene, -C₁₋₆ alkylene-5- to 14-membered heteroarylene-, and 5- to 14-membered heteroarylene, wherein the imino, C₁₋₆ alkylene, -CONH-, -CO-C₁₋₆ alkylene-NH-, -C₁₋₆ alkylene-CONH-, -C₁₋₆ alkylene-CONH-C₁₋₆ alkylene, -C₁₋₆ alkylene-SO₂NH-, -C₁₋₆ alkylene-NHCONH-, -C₁₋₆ alkylene-NHCO-, -C₁₋₆ alkylene-NHSO₂-, -C₁₋₆ alkylene-CO-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -C₁₋₆ alkylene-3- to 8-membered heterocycloalkylene-, -C₁₋₆ alkylene-3- to 8-membered heterocycloalkenylene-, -C₁₋₆ alkylene-C₆₋₁₄ arylene-, C₆₋₁₄ arylene, -C₁₋₆ alkylene-5- to 14-membered heteroarylene-, or 5- to 14-membered heteroarylene is optionally further substituted with one or more substituents selected from the group consisting of deuterium, halogen, amino, hydroxyl, cyano, oxo, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₁ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₁ₐ; the R₁ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₂ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₂ₐ; the R₂ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
alternatively, two R₂ are connected to each other to form a C₃₋₈ cycloalkyl or a 3- to 8-membered heterocycloalkyl, wherein the C₃₋₈ cycloalkyl or 3- to 8-membered heterocycloalkyl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
alternatively, one R₂ and R₃ are connected to form a 5- to 8-membered heterocycloalkenyl, wherein the 5- to 8-membered heterocycloalkenyl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₃ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C_{1- 6} hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₃ₐ; the R₃ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
Li and R₃ are connected to form a 3- to 8-membered heterocycloalkenyl or a 5- to 10-membered heteroaryl; wherein the 3- to 8-membered heterocycloalkenyl or 5- to 10-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, 5- to 14-membered heteroaryl, -COR₄₋₁, - COOR₄₋₁, -OC(=O)R₄₋₁, -SO₂R₄₋₂, and -OR₄₋₃, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₄ₐ; the R₄ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl; alternatively, two R₄ₐ are connected to form a 3- to 8-membered heterocycloalkyl or a 3- to 8-membered heterocycloalkenyl;
R₄₋₁ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄₋₂ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄₋₃ is selected from the group consisting of hydrogen, deuterium, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₃₋₈ cycloalkyl, 3- to 8-membered heterocycloalkyl, C₆₋₁₄ aryl, and 5- to 14-membered heteroaryl, wherein the C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₅ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₅ₐ; the R₅ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₆₋₁ and R₆₋₂ are each independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₅ₐ; the R₅ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
alternatively, R₆₋₁ and R₆₋₂, together with the carbon atom to which they are attached, form a C₃₋₈ cycloalkyl or a 3- to 8-membered heterocycloalkyl;
x is selected from the group consisting of 0, 1, 2, and 3;
y is selected from the group consisting of 0, 1, 2, 3, and 4;
z is selected from the group consisting of 0, 1, 2, 3, and 4;
u is selected from the group consisting of 0, 1, 2, 3, and 4.

The present disclosure provides a compound of formula (IB), a stereoisomer thereof, or a pharmaceutically acceptable salt thereof: 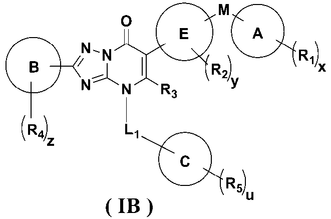 wherein
M is selected from the group consisting of a bond and CO;
ring A is selected from the group consisting of C₆₋₁₄ aryl and 5- to 14-membered heteroaryl;
ring B is selected from the group consisting of C₃₋₁₂ cycloalkyl, C₃₋₁₂ cycloalkenyl, 3-to 12-membered heterocycloalkyl, 3- to 12-membered heterocycloalkenyl, C₆₋₁₄ aryl, and 5- to 14-membered heteroaryl; alternatively, ring B is absent, and R₄ is directly attached to the parent structure;
ring C is selected from the group consisting of C₆₋₁₄ aryl and 5- to 14-membered heteroaryl;
ring E is selected from the group consisting of C₃₋₁₂ cycloalkylene, C₃₋₁₂ cycloalkenylene, 3- to 12-membered heterocycloalkylene, 3- to 12-membered heterocycloalkenylene, C₆₋₁₄ arylene, and 5- to 14-membered heteroarylene; alternatively, ring E is directly attached to the parent to form 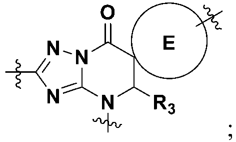
Li is selected from the group consisting of a bond, -O-, -S-, imino, C₁₋₆ alkylene, - CONH-, -CO-C₁₋₆ alkylene-NH-, -C₁₋₆ alkylene-CONH-, -C₁₋₆ alkylene-SO₂NH-, -C₁₋₆ alkylene-NHCONH-, -C₁₋₆ alkylene-NHCO-, -C₁₋₆ alkylene-NHSO₂-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -alkylene-3- to 8-membered heterocycloalkylene-, C₆₋₁₄ arylene, and 5- to 14-membered heteroarylene, wherein the imino, C₁₋₆ alkylene, -CONH-, -CO-C₁₋₆ alkylene-NH-, -C₁₋₆ alkylene-CONH-, -C₁₋₆ alkylene-SO₂NH-, -C₁₋₆ alkylene-NHCONH-, -C₁₋₆ alkylene-NHCO-, -C₁₋₆ alkylene-NHSO₂-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -alkylene-3- to 8-membered heterocycloalkylene-, C₆₋₁₄ arylene, or 5- to 14-membered heteroarylene is optionally further substituted with one or more substituents selected from the group consisting of deuterium, halogen, amino, hydroxyl, cyano, oxo, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₁ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₁ₐ; the R₁ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₂ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₂ₐ; the R₂ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
alternatively, two R₂ are connected to each other to form a C₃₋₈ cycloalkyl or a 3- to 8-membered heterocycloalkyl, wherein the C₃₋₈ cycloalkyl or 3- to 8-membered heterocycloalkyl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
alternatively, one R₂ and R₃ are connected to form a 5- to 8-membered heterocycloalkenyl, wherein the 5- to 8-membered heterocycloalkenyl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₃ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₃ₐ; the R₃ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
L₁ and R₃ are connected to form a 3- to 8-membered heterocycloalkenyl or a 5- to 10-membered heteroaryl; wherein the 3- to 8-membered heterocycloalkenyl or 5- to 10-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, 5- to 14-membered heteroaryl, -COR₄₋₁, and - SO₂R₄₋₂, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₄ₐ; the R₄ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄₋₁ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄₋₂ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₅ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₅ₐ; the R₅ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
x is selected from the group consisting of 0, 1, 2, and 3;
y is selected from the group consisting of 0, 1, 2, 3, and 4;
z is selected from the group consisting of 0, 1, 2, 3, and 4;
u is selected from the group consisting of 0, 1, 2, 3, and 4.

In certain embodiments of the present disclosure, M is CO; the definitions of other groups are as defined in any technical solution herein.

In certain embodiments of the present disclosure, M is CO; the definitions of other groups are as defined in any technical solution herein.

In certain embodiments of the present disclosure, ring A is selected from the group consisting of 5- to 6-membered heteroaryl, 5- to 6-membered heteroaryl-fused C₃₋₆ cycloalkenyl, 5- to 6-membered heteroaryl-fused 5- to 6-membered heteroaryl, 5- to 6-membered heteroaryl-fused 3- to 6-membered heterocycloalkenyl, and 5- to 6-membered heteroaryl-fused phenyl; the definitions of other groups are as defined in any technical solution herein.

In certain embodiments of the present disclosure, ring A is selected from the group consisting of 5- to 6-membered heteroaryl, 5- to 6-membered heteroaryl-fused C₃₋₆ cycloalkenyl, 5- to 6-membered heteroaryl-fused 5- to 6-membered heteroaryl, and 5- to 6-membered heteroaryl-fused 3- to 6-membered heterocycloalkenyl;
preferably, ring A is selected from the group consisting of 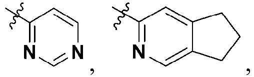 and 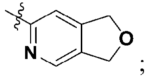
the definitions of other groups are as defined in any technical solution herein.

In certain embodiments of the present disclosure, ring A is 5- to 6-membered heteroaryl, preferably 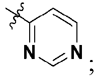 the definitions of other groups are as defined in any technical solution herein.

In certain embodiments of the present disclosure, ring A is 5- to 6-membered heteroaryl-fused 5- to 6-membered heteroaryl; the definitions of other groups are as defined in any technical solution herein.

In certain embodiments of the present disclosure, ring A is 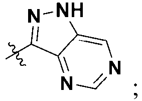 the definitions of other groups are as defined in any technical solution herein.

In certain embodiments of the present disclosure, ring A is 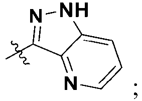 the definitions of other groups are as defined in any technical solution herein.

In certain embodiments of the present disclosure, ring A is 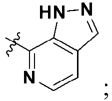 or, ring A is 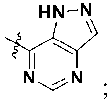 or, ring A is 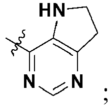 or, ring A is selected from the group consisting of 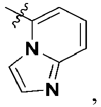 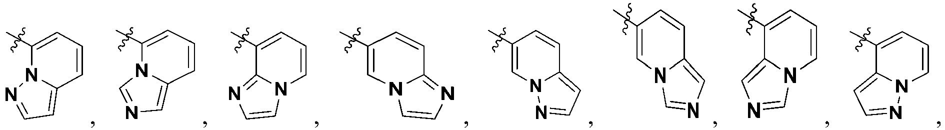 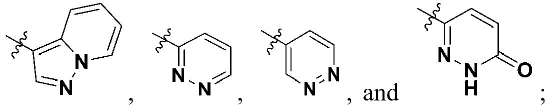 or, ring A is selected from the group consisting of 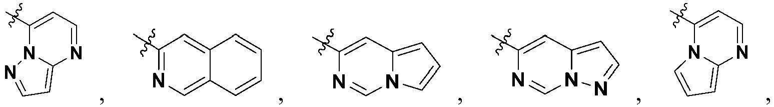 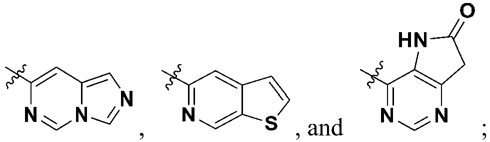 the definitions of other groups are as defined in any technical solution herein.

In certain embodiments of the present disclosure, ring B is selected from the group consisting of C₃₋₁₀ cycloalkenyl-spiro-3- to 10-membered heterocycloalkyl, 3- to 10-membered heterocycloalkenyl, 5- to 6-membered heteroaryl-fused 3- to 10-membered heterocycloalkenyl, 3- to 10-membered heterocycloalkenyl-fused 5- to 6-membered heteroaryl, 3- to 10-membered heterocycloalkenyl-spiro-3- to 10-membered heterocycloalkyl, 5- to 6-membered heteroaryl-fused 5- to 6-membered heteroaryl, benzo-fused C₃₋₁₀ cycloalkenyl, benzo-fused 3- to 10-membered heterocycloalkenyl, C₃₋₁₀ cycloalkenyl, 3- to 10-membered heterocycloalkenyl-fused phenyl, 5- to 6-membered heteroaryl, 3- to 10-membered heterocycloalkenyl-spiro-C₃₋₁₀ cycloalkyl, 3- to 10-membered heterocycloalkenyl-fused 3- to 10-membered heterocycloalkyl, benzo-fused 5- to 6-membered heteroaryl, C₃₋₁₀ cycloalkenyl-spiro-C₃₋₁₀ cycloalkyl, 3- to 10-membered heterocycloalkenyl-fused C₃₋₁₀ cycloalkyl, and 3- to 10-membered heterocycloalkyl; the definitions of other groups are as defined in any technical solution herein.

In certain embodiments of the present disclosure, ring B is selected from the group consisting of C₃₋₁₀ cycloalkenyl-spiro-3- to 10-membered heterocycloalkyl, 3- to 10-membered heterocycloalkenyl, 5- to 6-membered heteroaryl-fused 3- to 10-membered heterocycloalkenyl, 3- to 10-membered heterocycloalkenyl-fused 5- to 6-membered heteroaryl, 3- to 10-membered heterocycloalkenyl-spiro-3- to 10-membered heterocycloalkyl, 5- to 6-membered heteroaryl-fused 5- to 6-membered heteroaryl, benzo-fused C₃₋₁₀ cycloalkenyl, benzo-fused 3- to 10-membered heterocycloalkenyl, C₃₋₁₀ cycloalkenyl, 3- to 10-membered heterocycloalkenyl-fused phenyl, 5- to 6-membered heteroaryl, 3- to 10-membered heterocycloalkenyl-spiro-C₃₋₁₀ cycloalkyl, 3- to 10-membered heterocycloalkenyl-fused 3- to 10-membered heterocycloalkyl, benzo-fused 5- to 6-membered heteroaryl, and C₃₋₁₀ cycloalkenyl-spiro-C₃₋₁₀ cycloalkyl; the definitions of other groups are as defined in any technical solution herein.

In certain embodiments of the present disclosure, ring B is selected from the group consisting of C₃₋₆ cycloalkenyl-spiro-3- to 6-membered heterocycloalkyl, 3- to 8-membered heterocycloalkenyl, 5- to 6-membered heteroaryl-fused 3- to 6-membered heterocycloalkenyl, 3- to 6-membered heterocycloalkenyl-fused 5- to 6-membered heteroaryl, 3- to 6-membered heterocycloalkenyl-spiro-3- to 6-membered heterocycloalkyl, 5- to 6-membered heteroaryl-fused 5- to 6-membered heteroaryl, benzo-fused C₃₋₆ cycloalkenyl, benzo-fused 3- to 6-membered heterocycloalkenyl, C₃₋₈ cycloalkenyl, 3- to 6-membered heterocycloalkenyl-fused phenyl, 5- to 6-membered heteroaryl, 3- to 6-membered heterocycloalkenyl-spiro-C₃₋₆ cycloalkyl, and 3- to 6-membered heterocycloalkenyl-fused 3- to 6-membered heterocycloalkyl; the definitions of other groups are as defined in any technical solution herein.

In certain embodiments of the present disclosure, ring B is selected from the group consisting of C₃₋₆ cycloalkenyl-spiro-3- to 6-membered heterocycloalkyl, 3- to 8-membered heterocycloalkenyl, 5- to 6-membered heteroaryl-fused 3- to 6-membered heterocycloalkenyl, 3- to 6-membered heterocycloalkenyl-fused 5- to 6-membered heteroaryl, 3- to 6-membered heterocycloalkenyl-spiro-3- to 6-membered heterocycloalkyl, 5- to 6-membered heteroaryl-fused 5- to 6-membered heteroaryl, benzo-fused C₃₋₆ cycloalkenyl, and benzo-fused 3- to 6-membered heterocycloalkenyl;
the definitions of other groups are as defined in any technical solution herein.

In certain embodiments of the present disclosure, ring B is selected from the group consisting of C₃₋₆ cycloalkenyl-spiro-3- to 6-membered heterocycloalkyl, 3- to 6-membered heterocycloalkenyl, 5- to 6-membered heteroaryl-fused 3- to 6-membered heterocycloalkenyl, 3- to 6-membered heterocycloalkenyl-fused 5- to 6-membered heteroaryl, 3- to 6-membered heterocycloalkenyl-spiro-3- to 6-membered heterocycloalkyl, 5- to 6-membered heteroaryl-fused 5- to 6-membered heteroaryl, benzo-fused C₃₋₆ cycloalkenyl, and benzo-fused 3- to 6-membered heterocycloalkenyl;
preferably, ring B is selected from the group consisting of 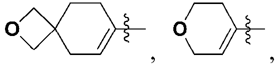 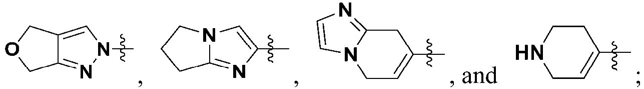
the definitions of other groups are as defined in any technical solution herein.

In certain embodiments of the present disclosure, ring B is 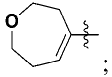 the definitions of other groups are as defined in any technical solution herein.

In certain embodiments of the present disclosure, ring B is 5- to 6-membered heteroaryl-fused 3- to 6-membered heterocycloalkenyl, preferably 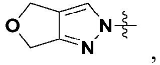 or preferably 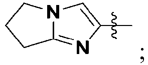 the definitions of other groups are as defined in any technical solution herein.

In certain embodiments of the present disclosure, ring B is 3- to 6-membered heterocycloalkenyl-fused 5- to 6-membered heteroaryl, preferably 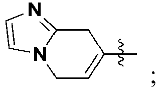 the definitions of other groups are as defined in any technical solution herein.

In certain embodiments of the present disclosure, ring B is 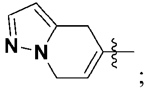 or, ring B is 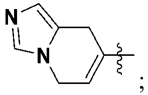 or, ring B is selected from the group consisting of 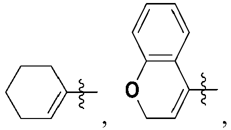 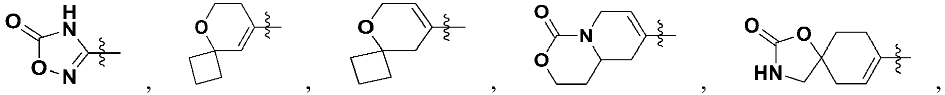 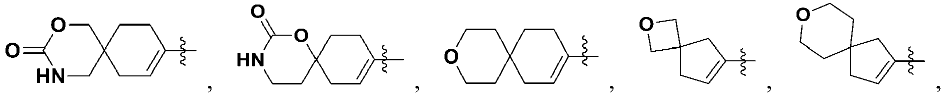 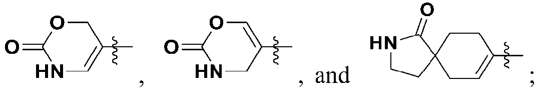 or, ring B is selected from the group consisting of 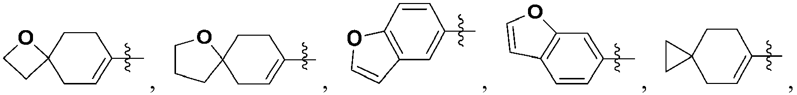 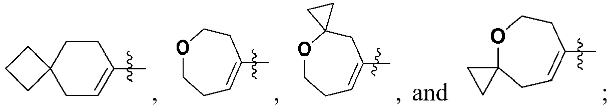 or, ring B is selected from the group consisting of 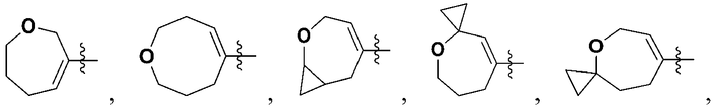 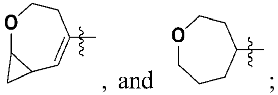 the definitions of other groups are as defined in any technical solution herein.

In certain embodiments of the present disclosure, ring B is 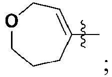 the definitions of other groups are as defined in any technical solution herein.

In certain embodiments of the present disclosure, ring C is selected from the group consisting of C₆₋₁₀ aryl, 5- to 6-membered heteroaryl, 5- to 6-membered heteroaryl-fused 5- to 6-membered heteroaryl, benzo-fused 5- to 6-membered heteroaryl, benzo-fused 3- to 6-membered heterocycloalkenyl, 5- to 6-membered heteroaryl-fused phenyl, 3- to 6-membered heterocycloalkenyl-fused phenyl, 5- to 6-membered heteroaryl-fused C₃₋₆ cycloalkenyl, and C₃₋₈ cycloalkyl; the definitions of other groups are as defined in any technical solution herein.

In certain embodiments of the present disclosure, ring C is selected from the group consisting of C₆₋₁₀ aryl, 5- to 6-membered heteroaryl, 5- to 6-membered heteroaryl-fused 5- to 6-membered heteroaryl, benzo-fused 5- to 6-membered heteroaryl, benzo-fused 3- to 6-membered heterocycloalkenyl, 5- to 6-membered heteroaryl-fused phenyl, 3- to 6-membered heterocycloalkenyl-fused phenyl, and 5- to 6-membered heteroaryl-fused C₃₋₆ cycloalkenyl; the definitions of other groups are as defined in any technical solution herein.

In certain embodiments of the present disclosure, ring C is selected from the group consisting of C₆₋₁₀ aryl, 5- to 6-membered heteroaryl, 5- to 6-membered heteroaryl-fused 5- to 6-membered heteroaryl, benzo-fused 5- to 6-membered heteroaryl, benzo-fused 3- to 6-membered heterocycloalkenyl, and 5- to 6-membered heteroaryl-fused phenyl.

In certain embodiments of the present disclosure, ring C is selected from the group consisting of C₆₋₁₀ aryl, 5- to 6-membered heteroaryl, 5- to 6-membered heteroaryl-fused 5- to 6-membered heteroaryl, benzo-fused 5- to 6-membered heteroaryl, and benzo-fused 3- to 6-membered heterocycloalkenyl;
preferably, ring C is selected from the group consisting of phenyl, 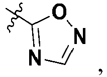 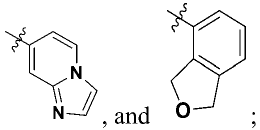
the definitions of other groups are as defined in any technical solution herein.

In certain embodiments of the present disclosure, ring C is C₆₋₁₀ aryl, preferably phenyl; the definitions of other groups are as defined in any technical solution herein.

In certain embodiments of the present disclosure, ring C is 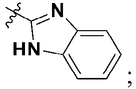 or, ring C is 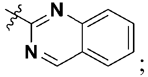 or, ring C is 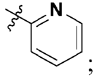 or, ring C is 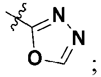 or, ring C is 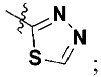 or, ring C is selected from the group consisting of 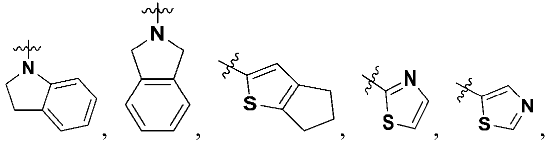 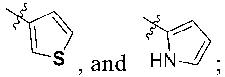 or, ring C is selected from the group consisting of 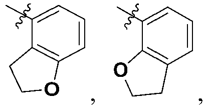 and 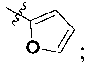 or, ring C is selected from the group consisting of 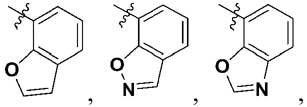 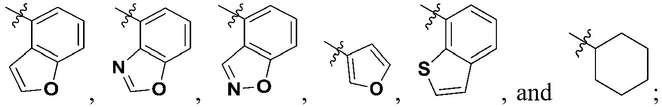 or, ring C is selected from the group consisting of 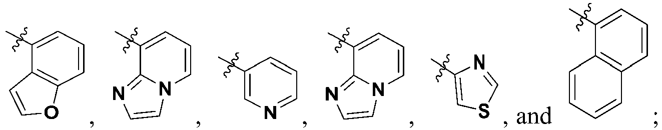 the definitions of other groups are as defined in any technical solution herein.

In certain embodiments of the present disclosure, ring C is 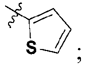 the definitions of other groups are as defined in any technical solution herein.

In certain embodiments of the present disclosure, ring E is selected from the group consisting of 5- to 6-membered heterocycloalkylene, 4- to 6-membered heterocycloalkylene-spiro-4- to 6-membered heterocycloalkylene, 5- to 6-membered heterocycloalkylene-fused 5-to 6-membered heterocycloalkylene, 5- to 6-membered heteroaryl-fused 5- to 6-membered heterocycloalkenyl, and 5- to 6-membered heterocycloalkylene-fused C₃₋₆ cycloalkylene; the definitions of other groups are as defined in any technical solution herein.

In certain embodiments of the present disclosure, ring E is selected from the group consisting of 5- to 6-membered heterocycloalkylene, 4- to 6-membered heterocycloalkylene-spiro-4- to 6-membered heterocycloalkylene, 5- to 6-membered heterocycloalkylene-fused 5-to 6-membered heterocycloalkylene, and 5- to 6-membered heteroaryl-fused 5- to 6-membered heterocycloalkenyl; the definitions of other groups are as defined in any technical solution herein.

In certain embodiments of the present disclosure, ring E is selected from the group consisting of 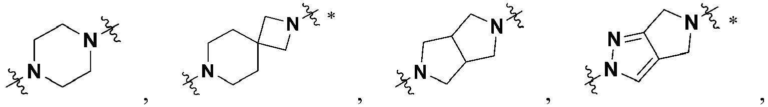 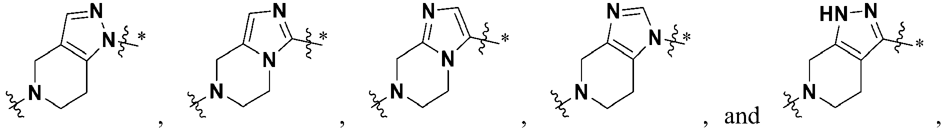 wherein the end with * is connected to M; the definitions of other groups are as defined in any technical solution herein.

In certain embodiments of the present disclosure, ring E is 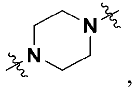 wherein the end with * is connected to M; the definitions of other groups are as defined in any technical solution herein.

In certain embodiments of the present disclosure, ring E is selected from the group consisting of 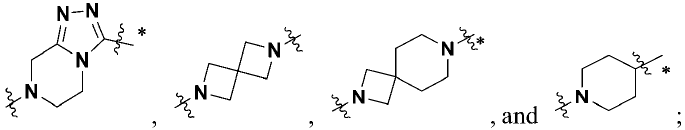 or, ring E is selected from the group consisting of 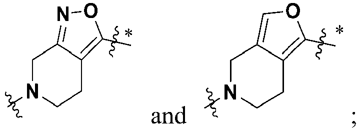 wherein the end with * is connected to M; the definitions of other groups are as defined in any technical solution herein.

In certain embodiments of the present disclosure, ring E is 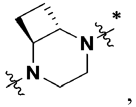 wherein the end with * is connected to M; the definitions of other groups are as defined in any technical solution herein.

In certain embodiments of the present disclosure, the compound is represented by formula (II), 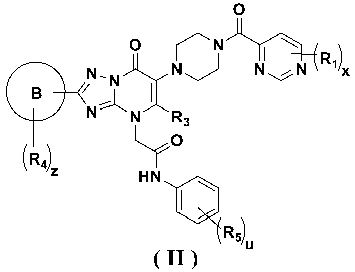 wherein
ring B is selected from the group consisting of C₃₋₁₀ cycloalkenyl-spiro-3- to 10-membered heterocycloalkyl, 5- to 6-membered heteroaryl-fused 3- to 10-membered heterocycloalkenyl, 3- to 10-membered heterocycloalkenyl-fused 5- to 6-membered heteroaryl, 3- to 10-membered heterocycloalkenyl-spiro-3- to 10-membered heterocycloalkyl, 5- to 6-membered heteroaryl-fused 5- to 6-membered heteroaryl, 7- to 10-membered heterocycloalkenyl, C₃₋₁₀ cycloalkenyl, 3- to 10-membered heterocycloalkenyl-fused phenyl, 5- to 6-membered heteroaryl, 3- to 10-membered heterocycloalkenyl-spiro-C₃₋₁₀ cycloalkyl, 3-to 10-membered heterocycloalkenyl-fused 3- to 10-membered heterocycloalkyl, 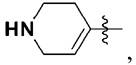 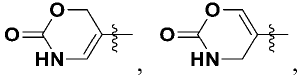 benzo-fused 5- to 6-membered heteroaryl, C₃₋₁₀ cycloalkenyl-spiro-C₃₋₁₀ cycloalkyl, 3- to 10-membered heterocycloalkenyl-fused C₃₋₁₀ cycloalkyl, and 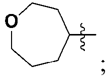 the definitions of other groups are as defined in any technical solution herein.

In certain embodiments of the present disclosure, the compound is represented by formula (II), 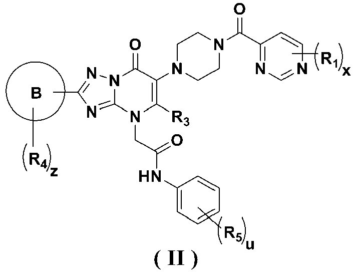 wherein
ring B is selected from the group consisting of C₃₋₁₀ cycloalkenyl-spiro-3- to 10-membered heterocycloalkyl, 5- to 6-membered heteroaryl-fused 3- to 10-membered heterocycloalkenyl, 3- to 10-membered heterocycloalkenyl-fused 5- to 6-membered heteroaryl, 3- to 10-membered heterocycloalkenyl-spiro-3- to 10-membered heterocycloalkyl, 5- to 6-membered heteroaryl-fused 5- to 6-membered heteroaryl, 7- to 10-membered heterocycloalkenyl, C₃₋₁₀ cycloalkenyl, 3- to 10-membered heterocycloalkenyl-fused phenyl, 5- to 6-membered heteroaryl, 3- to 10-membered heterocycloalkenyl-spiro-C₃₋₁₀ cycloalkyl, 3-to 10-membered heterocycloalkenyl-fused 3- to 10-membered heterocycloalkyl, 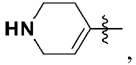 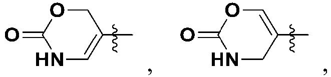 benzo-fused 5- to 6-membered heteroaryl, and C₃₋₁₀ cycloalkenyl-spiro-C₃₋₁₀ cycloalkyl; the definitions of other groups are as defined in any technical solution herein.

In certain embodiments of the present disclosure, the compound is represented by formula (II), 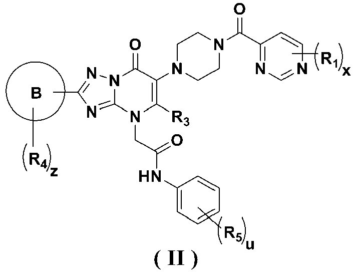 wherein
ring B is selected from the group consisting of C₃₋₆ cycloalkenyl-spiro-3- to 6-membered heterocycloalkyl, 5- to 6-membered heteroaryl-fused 3- to 6-membered heterocycloalkenyl, 3- to 6-membered heterocycloalkenyl-fused 5- to 6-membered heteroaryl, 3- to 6-membered heterocycloalkenyl-spiro-3- to 6-membered heterocycloalkyl, 5- to 6-membered heteroaryl-fused 5- to 6-membered heteroaryl, 7- to 8-membered heterocycloalkenyl, C₃₋₈ cycloalkenyl, 3- to 6-membered heterocycloalkenyl-fused phenyl, 5-to 6-membered heteroaryl, 3- to 6-membered heterocycloalkenyl-spiro-C₃₋₆ cycloalkyl, 3- to 6-membered heterocycloalkenyl-fused 3- to 6-membered heterocycloalkyl, 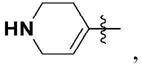 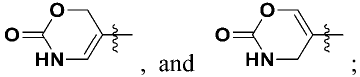 the definitions of other groups are as defined in any technical solution herein.

In certain embodiments of the present disclosure, the compound is represented by formula (II), 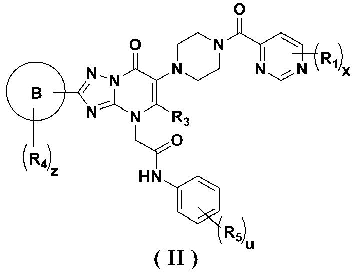 wherein
ring B is selected from the group consisting of C₃₋₆ cycloalkenyl-spiro-3- to 6-membered heterocycloalkyl, 5- to 6-membered heteroaryl-fused 3- to 6-membered heterocycloalkenyl, 3- to 6-membered heterocycloalkenyl-fused 5- to 6-membered heteroaryl, 3- to 6-membered heterocycloalkenyl-spiro-3- to 6-membered heterocycloalkyl, 5- to 6-membered heteroaryl-fused 5- to 6-membered heteroaryl, and 7- to 8-membered heterocycloalkenyl; preferably 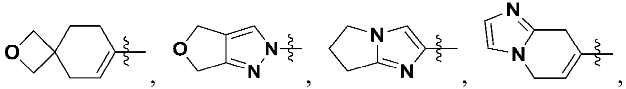 and 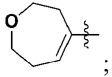 the definitions of other groups are as defined in any technical solution herein.

In certain embodiments of the present disclosure, ring B is selected from the group consisting of 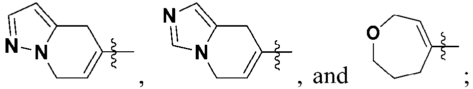 or, ring B is selected from the group consisting of 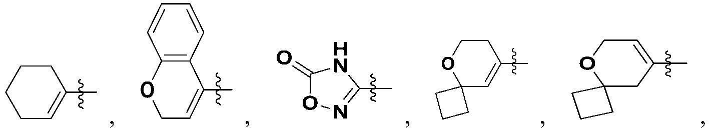 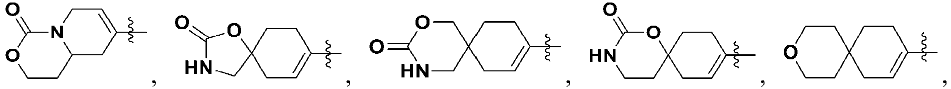 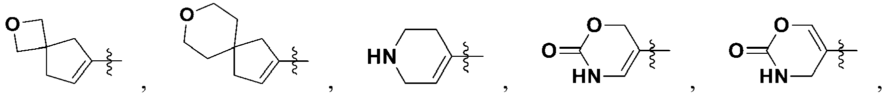 and 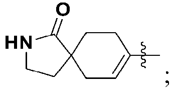 or, ring B is selected from the group consisting of 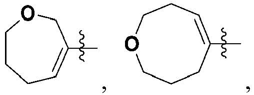 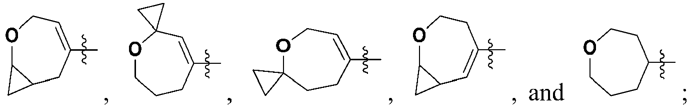 the definitions of other groups are as defined in any technical solution herein.

In certain embodiments of the present disclosure, when ring B is 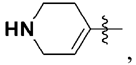 the structural moiety 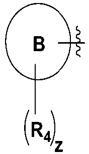 is 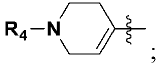 the definitions of other groups are as defined in any technical solution herein.

In certain embodiments of the present disclosure, when ring B is 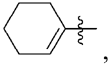 the structural moiety 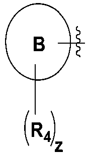 is 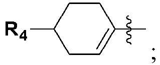 the definitions of other groups are as defined in any technical solution herein.

In certain embodiments of the present disclosure, the compound is represented by formula (II), 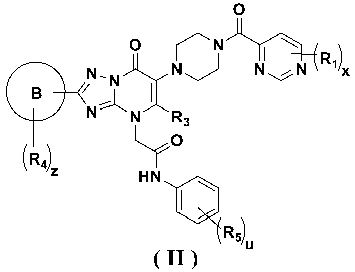 wherein
ring B is selected from the group consisting of C₃₋₆ cycloalkenyl-spiro-3- to 6-membered heterocycloalkyl, 5- to 6-membered heteroaryl-fused 3- to 6-membered heterocycloalkenyl, 3- to 6-membered heterocycloalkenyl-fused 5- to 6-membered heteroaryl, 3- to 6-membered heterocycloalkenyl-spiro-3- to 6-membered heterocycloalkyl, and 5- to 6-membered heteroaryl-fused 5- to 6-membered heteroaryl; preferably 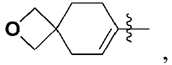 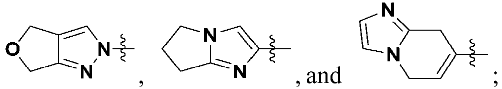 the definitions of other groups are as defined in any technical solution herein.

In certain embodiments of the present disclosure, ring B is selected from the group consisting of 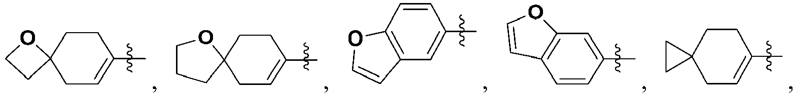 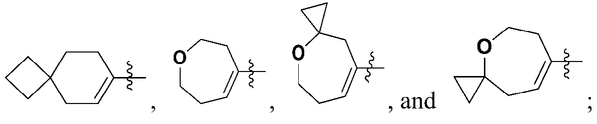 the definitions of other groups are as defined in any technical solution herein.

In certain embodiments of the present disclosure, the structural moiety 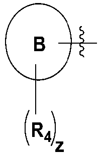 is 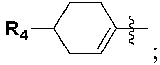 the definitions of other groups are as defined in any technical solution herein.

In certain embodiments of the present disclosure, the compound is represented by formula (III), 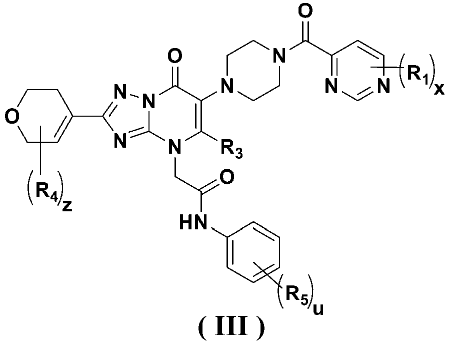
wherein R₃ is selected from the group consisting of hydrogen, deuterium, halogen, amino, hydroxyl, cyano, methyl, C₃₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3-to 8-membered heterocycloalkyl, C₆₋₁₄ aryl, and 5- to 14-membered heteroaryl, wherein the amino, C₃₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₃ₐ; the R₃ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl; the methyl is optionally further substituted with one or more R₃ₐ; the R₃ₐ is independently selected from the group consisting of deuterium, halogen, amino, hydroxyl, cyano, C₂₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₂₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₂₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
preferably, R₃ is selected from the group consisting of hydrogen, deuterium, halogen, amino, hydroxyl, cyano, methyl, C₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3-to 6-membered heterocycloalkyl, C₆₋₁₀ aryl, and 5- to 6-membered heteroaryl, wherein the amino, C₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₃ₐ; the R₃ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl; the methyl is optionally further substituted with one or more R₃ₐ; the R₃ₐ is independently selected from the group consisting of deuterium, halogen, amino, hydroxyl, cyano, C₂₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₂₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₂₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
more preferably, R₃ is selected from the group consisting of trifluoromethoxy, vinyl, ethynyl, and cyano;
the definitions of other groups are as defined in any technical solution herein.

In certain embodiments of the present disclosure, R₃ is 5- to 6-membered heteroaryl, preferably 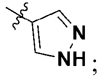 the 5- to 6-membered heteroaryl or 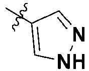 is optionally substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl; The definitions of other groups are as defined in any technical solution herein.

In certain embodiments of the present disclosure, R₃ is 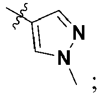 the definitions of other groups are as defined in any technical solution herein.

In certain embodiments of the present disclosure, the compound is represented by formula (IV), 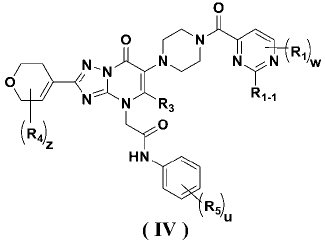
R₁₋₁ is selected from the group consisting of deuterium, halogen, amino, hydroxyl, cyano, C₂₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, C₆₋₁₄ aryl, and 5- to 14-membered heteroaryl, wherein the amino, C₂₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, or C₃₋₈ cycloalkyl is optionally further substituted with one or more R₁ₐ; the R₁ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
preferably, R₁-₁ is selected from the group consisting of deuterium, halogen, amino, hydroxyl, cyano, C₂₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, C₆₋₁₀ aryl, and 5- to 6-membered heteroaryl, wherein the amino, C₂₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, C₆₋₁₀ aryl, or 5- to 6-membered heteroaryl is optionally further substituted with one or more R₁ₐ; the R₁ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
more preferably, R₁₋₁ is 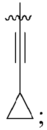
w is selected from the group consisting of 0, 1, and 2;
the definitions of other groups are as defined in any technical solution herein.

In certain embodiments of the present disclosure, the compound is represented by formula (IV'), 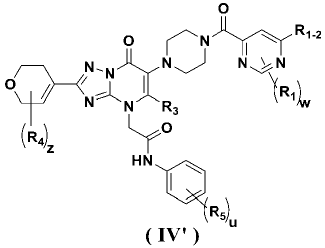
R₁₋₂ is selected from the group consisting of deuterium, halogen, amino, hydroxyl, cyano, C₂₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₂₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₁ₐ; the R₁ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl; preferably, R₁₋₂ is selected from the group consisting of deuterium, halogen, amino, hydroxyl, cyano, C₂₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₂₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₁ₐ; the R₁ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
more preferably, R₁₋₂ is 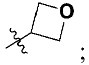
w is selected from the group consisting of 0, 1, and 2;
the definitions of other groups are as defined in any technical solution herein.

In certain embodiments of the present disclosure, the compound is represented by formula (V), 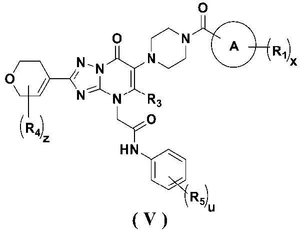 wherein ring A is selected from the group consisting of 5- to 6-membered heteroaryl-fused C₃₋₆ cycloalkenyl, 5- to 6-membered heteroaryl-fused 5- to 6-membered heteroaryl, 5- to 6-membered heteroaryl-fused 3- to 6-membered heterocycloalkenyl, 5- to 6-membered heteroaryl-fused phenyl, 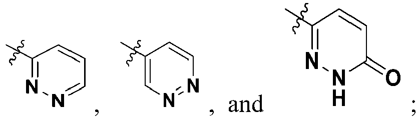 the definitions of other groups are as defined in any technical solution herein.

In certain embodiments of the present disclosure, the compound is represented by formula (V), 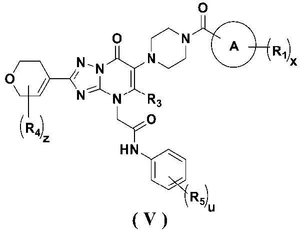 wherein ring A is selected from the group consisting of 5- to 6-membered heteroaryl-fused C₃₋₆ cycloalkenyl, 5- to 6-membered heteroaryl-fused 5- to 6-membered heteroaryl, 5- to 6-membered heteroaryl-fused 3- to 6-membered heterocycloalkenyl, 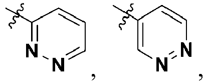 and 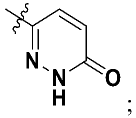 the definitions of other groups are as defined in any technical solution herein.

In certain embodiments of the present disclosure, the compound is represented by formula (V), 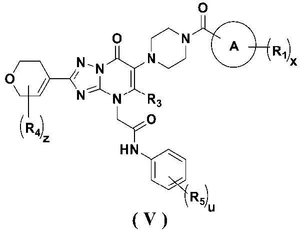
wherein ring A is selected from the group consisting of 5- to 6-membered heteroaryl-fused C₃₋₆ cycloalkenyl, 5- to 6-membered heteroaryl-fused 5- to 6-membered heteroaryl, and 5- to 6-membered heteroaryl-fused 3- to 6-membered heterocycloalkenyl; preferably, ring A is selected from the group consisting of 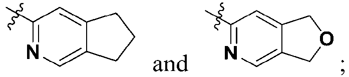
the definitions of other groups are as defined in any technical solution herein.

In certain embodiments of the present disclosure, the compound is represented by formula (VI), 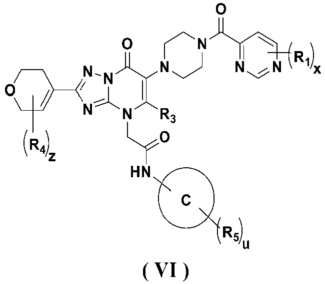 wherein
ring C is selected from the group consisting of 5-membered heteroaryl, 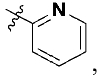 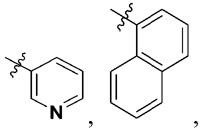 5- to 6-membered heteroaryl-fused 5- to 6-membered heteroaryl, benzo-fused 5- to 6-membered heteroaryl, benzo-fused 3- to 6-membered heterocycloalkenyl, 3- to 6-membered heterocycloalkenyl-fused phenyl, 5- to 6-membered heteroaryl-fused C₃₋₆ cycloalkenyl, and C₃₋₈ cycloalkyl; the definitions of other groups are as defined in any technical solution herein.

In certain embodiments of the present disclosure, the compound is represented by formula (VI), 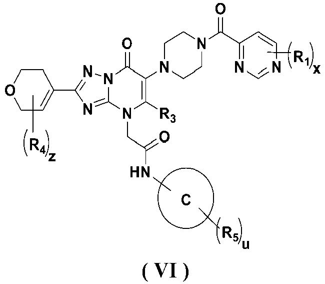 wherein
ring C is selected from the group consisting of 5-membered heteroaryl, 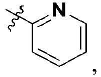 5-to 6-membered heteroaryl-fused 5- to 6-membered heteroaryl, benzo-fused 5- to 6-membered heteroaryl, benzo-fused 3- to 6-membered heterocycloalkenyl, 3- to 6-membered heterocycloalkenyl-fused phenyl, 5- to 6-membered heteroaryl-fused C₃₋₆ cycloalkenyl, and C₃₋₈ cycloalkyl; the definitions of other groups are as defined in any technical solution herein.

In certain embodiments of the present disclosure, the compound is represented by formula (VI), 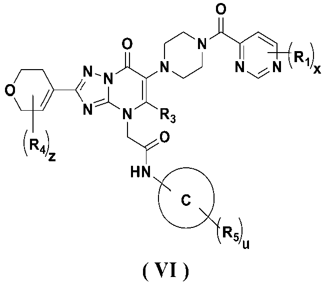 wherein
ring C is selected from the group consisting of 5-membered heteroaryl, 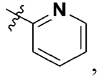 5-to 6-membered heteroaryl-fused 5- to 6-membered heteroaryl, benzo-fused 5- to 6-membered heteroaryl, benzo-fused 3- to 6-membered heterocycloalkenyl, 3- to 6-membered heterocycloalkenyl-fused phenyl, and 5- to 6-membered heteroaryl-fused C₃₋₆ cycloalkenyl; the definitions of other groups are as defined in any technical solution herein.

In certain embodiments of the present disclosure, the compound is represented by formula (VI), 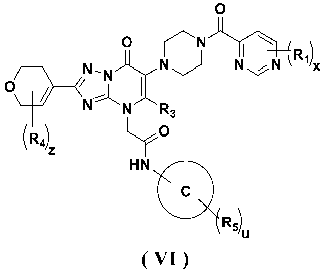 wherein
ring C is selected from the group consisting of 5-membered heteroaryl, 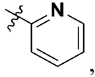 5-to 6-membered heteroaryl-fused 5- to 6-membered heteroaryl, benzo-fused 5- to 6-membered heteroaryl, and benzo-fused 3- to 6-membered heterocycloalkenyl; preferably, ring C is selected from the group consisting of 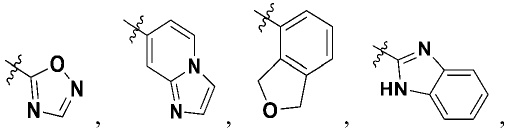 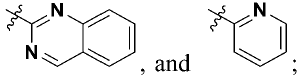 the definitions of other groups are as defined in any technical solution herein.

In certain embodiments of the present disclosure, the compound is represented by formula (VI), 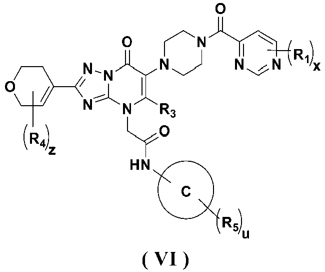 wherein
ring C is selected from the group consisting of 5-membered heteroaryl, 5- to 6-membered heteroaryl-fused 5- to 6-membered heteroaryl, benzo-fused 5- to 6-membered heteroaryl, and benzo-fused 3- to 6-membered heterocycloalkenyl; preferably, ring C is selected from the group consisting of 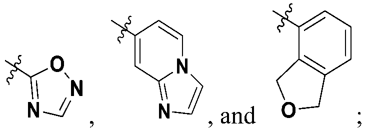
the definitions of other groups are as defined in any technical solution herein.

In certain embodiments of the present disclosure, ring C is selected from the group consisting of 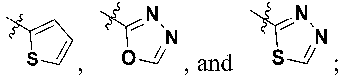 the definitions of other groups are as defined in any technical solution herein.

In certain embodiments of the present disclosure, ring C is 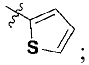 the definitions of other groups are as defined in any technical solution herein.

In certain embodiments of the present disclosure, the compound is represented by formula (VII) or formula (VIII), 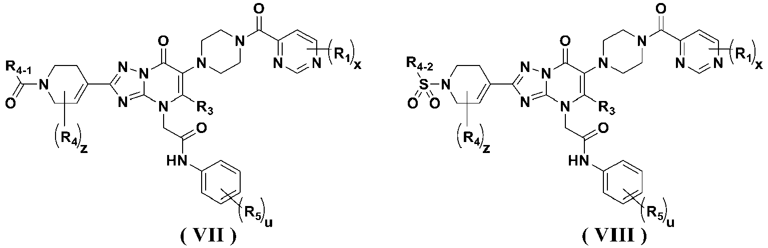 wherein
R₄₋₁ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
preferably, R₄₋₁ is selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
more preferably, R₄₋₁ is selected from the group consisting of cyclopropyl, isopropyl, and ethyl;
R₄₋₂ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkylthio, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
preferably, R₄₋₂ is selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
preferably, R₄₋₂ is methyl;
z is selected from the group consisting of 0, 1, 2, and 3;
the definitions of other groups are as defined in any technical solution herein.

In certain embodiments of the present disclosure, R₄₋₁ is C₃₋₆ cycloalkyl, preferably cyclopropyl; the definitions of other groups are as defined in any technical solution herein.

In certain embodiments of the present disclosure, R₄₋₁ is C₁₋₃ alkyl; preferably isopropyl; or preferably ethyl; the definitions of other groups are as defined in any technical solution herein.

In certain embodiments of the present disclosure, R₄₋₁ is -CH₂CF₃; or, R₄₋₁ is -NHCH₃; R₄₋₁ is -N(CH₃)₂; or, R₄₋₁ is -C(OH)(CH₃)₂; or, R₄₋₁ is *tert*-butyl; or, R₄₋₁ is -CH₂-cyclopropyl; the definitions of other groups are as defined in any technical solution herein.

In certain embodiments of the present disclosure, the compound is represented by formula (X), 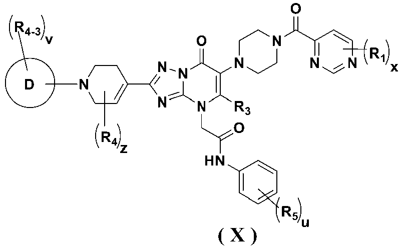
ring D is 5- to 6-membered heterocyclyl; preferably 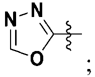
R₄₋₃ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
preferably, R₄₋₃ is selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
more preferably, R₄₋₃ is methyl;
z is selected from the group consisting of 0, 1, 2, and 3;
v is selected from the group consisting of 0, 1, 2, and 3;
the definitions of other groups are as defined in any technical solution herein.

In certain embodiments of the present disclosure, ring D is selected from the group consisting of 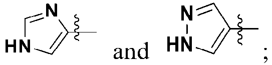 the definitions of other groups are as defined in any technical solution herein.

In certain embodiments of the present disclosure, the compound is represented by formula (IX), 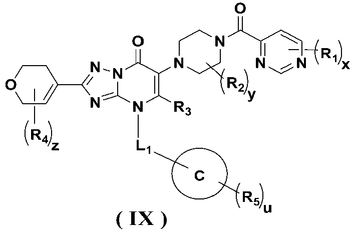
L₁ is selected from the group consisting of a bond, -O-, -S-, imino, C₁₋₆ alkylene, - CONH-, -CO-C₁₋₆ alkylene-NH-, -C₁₋₆ alkylene-CONH-C₁₋₆ alkylene, -C₁₋₆ alkylene-SO₂NH-, -C₁₋₆ alkylene-NHCONH-, -C₁₋₆ alkylene-NHCO-, -C₁₋₆ alkylene-NHSO₂-, -C₁₋₆ alkylene-CO-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -C₁₋₆ alkylene-3- to 8-membered heterocycloalkylene-, -C₁₋₆ alkylene-C₆₋₁₄ heterocycloalkenylene-, -C₁₋₆ alkylene-C₆₋₁₄ arylene-, C₆₋₁₄ arylene, -C₁₋₆ alkylene-5- to 14-membered heteroarylene-, 5- to 14-membered heteroarylene, and -CH₂CH₂CONH-, wherein the imino, C₁₋₆ alkylene, - CONH-, -CO-C₁₋₆ alkylene-NH-, -C₁₋₆ alkylene-CONH-, -C₁₋₆ alkylene-CONH-C₁₋₆ alkylene, -C₁₋₆ alkylene-SO₂NH-, -C₁₋₆ alkylene-NHCONH-, -C₁₋₆ alkylene-NHCO-, -C₁₋₆ alkylene-NHSO₂-, -C₁₋₆ alkylene-CO-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -C₁₋₆ alkylene-3- to 8-membered heterocycloalkylene-, -C₁₋₆ alkylene-C₆₋₁₄ heterocycloalkenylene-, -C₁₋₆ alkylene-C₆₋₁₄ arylene-, C₆₋₁₄ arylene, -C₁₋₆ alkylene-5- to 14-membered heteroarylene-, 5- to 14-membered heteroarylene, or -CH₂CH₂CONH- is optionally further substituted with one or more substituents selected from the group consisting of deuterium, halogen, amino, hydroxyl, cyano, oxo, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
preferably, L₁ is selected from the group consisting of a bond, -O-, -S-, imino, C₁₋₃ alkylene, -CONH-, -CO-C₁₋₃ alkylene-NH-, -C₁₋₃ alkylene-CONH-C₁₋₃ alkylene, -C₁₋₃ alkylene-SO₂NH-, -C₁₋₃ alkylene-SO₂NH-, -C₁₋₃ alkylene-NHCONH-, -C₁₋₃ alkylene-NHCO-, -C₁₋₃ alkylene-NHSO₂-, -C₁₋₃ alkylene-CO-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃ haloalkyleneoxy, C₃₋₈ cycloalkylene, 3- to 8-membered heterocycloalkylene, 3- to 8-membered heterocycloalkenylene, -C₁₋₃ alkylene-3- to 8-membered heterocycloalkylene-, -C₁₋₃ alkylene-3- to 8-membered heterocycloalkenylene-, -C₁₋₃ alkylene-C₆₋₁₀ arylene-, C₆₋₁₀ arylene, -C₁₋₃ alkylene-5- to 6-membered heteroarylene-, 5- to 6-membered heteroarylene, and - CH₂CH₂CONH-, wherein the imino, C₁₋₃ alkylene, -C₁₋₃ alkylene-CONH-C₁₋₃ alkylene, -C₁₋₃ alkylene-SO₂NH-, -C₁₋₃ alkylene-SO₂NH-, -C₁₋₃ alkylene-NHCONH-, -C₁₋₃ alkylene-NHCO-, -C₁₋₃ alkylene-NHSO₂-, -C₁₋₃ alkylene-CO-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃ haloalkyleneoxy, C₃₋₈ cycloalkylene, 3- to 8-membered heterocycloalkylene, 3- to 8-membered heterocycloalkenylene, -C₁₋₃ alkylene-3- to 8-membered heterocycloalkylene-, -C₁₋₃ alkylene-3- to 8-membered heterocycloalkenylene-, -C₁₋₃ alkylene-C₆₋₁₀ arylene-, C₆₋₁₀ arylene, -C₁₋₃ alkylene-5- to 6-membered heteroarylene-, 5- to 6-membered heteroarylene, or - CH₂CH₂CONH- is optionally further substituted with one or more substituents selected from the group consisting of deuterium, halogen, amino, hydroxyl, cyano, oxo, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
the definitions of other groups are as defined in any technical solution herein.

In certain embodiments of the present disclosure, the compound is represented by formula (IX), 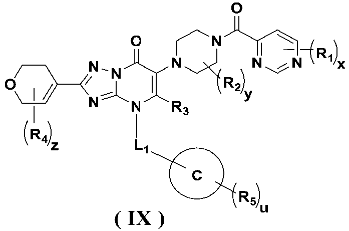
L₁ is selected from the group consisting of a bond, -O-, -S-, imino, C₁₋₆ alkylene, - CONH-, -CO-C₁₋₆ alkylene-NH-, -C₁₋₆ alkylene-CONH-C₁₋₆ alkylene, -C₁₋₆ alkylene-SO₂NH-, -C₁₋₆ alkylene-NHCONH-, -C₁₋₆ alkylene-NHCO-, -C₁₋₆ alkylene-NHSO₂-, -C₁₋₆ alkylene-CO-, -C₁₋₆ alkylene-C(=N-OH)NH-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -C₁₋₆ alkylene-3- to 8-membered heterocycloalkylene-, -C₁₋₆ alkylene-C₆₋₁₄ heterocycloalkenylene-, -C₁₋₆ alkylene-C₆₋₁₄ arylene-, C₆₋₁₄ arylene, -C₁₋₆ alkylene-5- to 14-membered heteroarylene-, 5- to 14-membered heteroarylene, and -CH₂CH₂CONH-, wherein the imino, C₁₋₆ alkylene, -CONH-, -CO-C₁₋₆ alkylene-NH-, -C₁₋₆ alkylene-CONH-, - C₁₋₆ alkylene-CONH-C₁₋₆ alkylene, -C₁₋₆ alkylene-SO₂NH-, -C₁₋₆ alkylene-NHCONH-, -C₁₋₆ alkylene-NHCO-, -C₁₋₆ alkylene-NHSO₂-, -C₁₋₆ alkylene-CO-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -C₁₋₆ alkylene-3- to 8-membered heterocycloalkylene-, -C₁₋₆ alkylene-C₆₋₁₄ heterocycloalkenylene-, -C₁₋₆ alkylene-C₆₋₁₄ arylene-, C₆₋₁₄ arylene, -C₁₋₆ alkylene-5- to 14-membered heteroarylene-, 5- to 14-membered heteroarylene, or -CH₂CH₂CONH- is optionally further substituted with one or more substituents selected from the group consisting of deuterium, halogen, amino, hydroxyl, cyano, oxo, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
preferably, L₁ is selected from the group consisting of a bond, -O-, -S-, imino, C₁₋₃ alkylene, -CONH-, -CO-C₁₋₃ alkylene-NH-, -C₁₋₃ alkylene-CONH-C₁₋₃ alkylene, -C₁₋₃ alkylene-SO₂NH-, -C₁₋₃ alkylene-SO₂NH-, -C₁₋₃ alkylene-NHCONH-, -C₁₋₃ alkylene-NHCO-, -C₁₋₃ alkylene-NHSO₂-, -C₁₋₃ alkylene-CO-, -C₁₋₃ alkylene-C(=N-OH)NH-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃ haloalkyleneoxy, C₃₋₈ cycloalkylene, 3- to 8-membered heterocycloalkylene, 3- to 8-membered heterocycloalkenylene, -C₁₋₃ alkylene-3- to 8-membered heterocycloalkylene-, -C₁₋₃ alkylene-3- to 8-membered heterocycloalkenylene-, -C₁₋₃ alkylene-C₆₋₁₀ arylene-, C₆₋₁₀ arylene, -C₁₋₃ alkylene-5- to 6-membered heteroarylene-, 5- to 6-membered heteroarylene, and -CH₂CH₂CONH-, wherein the imino, C₁₋₃ alkylene, -C₁₋₃ alkylene-CONH-C₁₋₃ alkylene, -C₁₋₃ alkylene-SO₂NH-, -C₁₋₃ alkylene-SO₂NH-, -C₁₋₃ alkylene-NHCONH-, -C₁₋₃ alkylene-NHCO-, -C₁₋₃ alkylene-NHSO₂-, -C₁₋₃ alkylene-CO-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃ haloalkyleneoxy, C₃₋₈ cycloalkylene, 3- to 8-membered heterocycloalkylene, 3- to 8-membered heterocycloalkenylene, -C₁₋₃ alkylene-3- to 8-membered heterocycloalkylene-, -C₁₋₃ alkylene-3- to 8-membered heterocycloalkenylene-, - C₁₋₃ alkylene-C₆₋₁₀ arylene-, C₆₋₁₀ arylene, -C₁₋₃ alkylene-5- to 6-membered heteroarylene-, 5-to 6-membered heteroarylene, or -CH₂CH₂CONH- is optionally further substituted with one or more substituents selected from the group consisting of deuterium, halogen, amino, hydroxyl, cyano, oxo, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl; the definitions of other groups are as defined in any technical solution herein.

In certain embodiments of the present disclosure, the compound is represented by formula (IX), 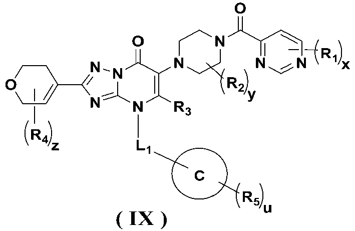
L₁ is selected from the group consisting of a bond, -O-, -S-, imino, C₁₋₆ alkylene, - CONH-, -CO-C₁₋₆ alkylene-NH-, -C₁₋₆ alkylene-SO₂NH-, -C₁₋₆ alkylene-NHCONH-, -C₁₋₆ alkylene-NHCO-, -C₁₋₆ alkylene-NHSO₂-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -alkylene-3- to 8-membered heterocycloalkylene-, C₆₋₁₄ arylene, and 5-to 14-membered heteroarylene, wherein the imino, C₁₋₆ alkylene, -CONH-, -CO-C₁₋₆ alkylene-NH-, -C₁₋₆ alkylene-CONH-, -C₁₋₆ alkylene-SO₂NH-, -C₁₋₆ alkylene-NHCONH-, -C₁₋₆ alkylene-NHCO-, -C₁₋₆ alkylene-NHSO₂-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -alkylene-3- to 8-membered heterocycloalkylene-, C₆₋₁₄ arylene, or 5-to 14-membered heteroarylene is optionally further substituted with one or more substituents selected from the group consisting of deuterium, halogen, amino, hydroxyl, cyano, oxo, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5-to 14-membered heteroaryl;
preferably, L₁ is selected from the group consisting of a bond, -O-, -S-, imino, C₁₋₃ alkylene, -CONH-, -CO-C₁₋₃ alkylene-NH-, -C₁₋₃ alkylene-SO₂NH-, -C₁₋₃ alkylene-SO₂NH-, - C₁₋₃ alkylene-NHCONH-, -C₁₋₃alkylene-NHCO-, -C₁₋₃ alkylene-NHSO₂-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃ haloalkyleneoxy, C₃₋₈ cycloalkylene, 3- to 8-membered heterocycloalkylene, 3- to 8-membered heterocycloalkenylene, -alkylene-3- to 8-membered heterocycloalkylene-, C₆₋₁₀ arylene, and 5- to 6-membered heteroarylene, wherein the imino, C₁₋₃ alkylene, -C₁₋₃ alkylene-SO₂NH-, -C₁₋₃ alkylene-SO₂NH-, -C₁₋₃ alkylene-NHCONH-, -C₁₋₃ alkylene-NHCO-, -C₁₋₃ alkylene-NHSO₂-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃ haloalkyleneoxy, C₃₋₈ cycloalkylene, 3- to 8-membered heterocycloalkylene, 3- to 8-membered heterocycloalkenylene, -alkylene-3- to 8-membered heterocycloalkylene-, C₆₋₁₀ arylene, or 5-to 6-membered heteroarylene is optionally further substituted with one or more substituents selected from the group consisting of deuterium, halogen, amino, hydroxyl, cyano, oxo, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5-to 6-membered heteroaryl;
more preferably, L₁ is selected from the group consisting of -CH₂-, -CH₂SO₂NH-*, - COCH₂NH-*, -CONH-*, 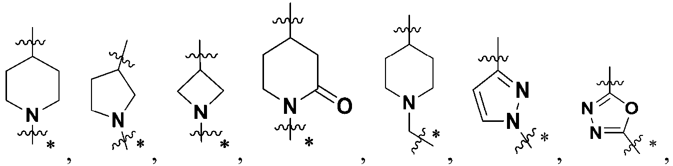 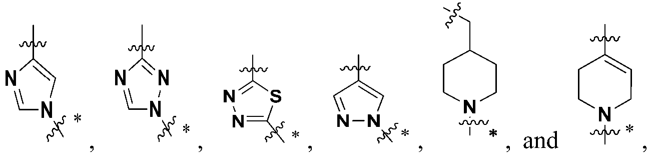 wherein the side with * is connected to ring C;
the definitions of other groups are as defined in any technical solution herein.

In certain embodiments of the present disclosure, the compound is represented by formula (IX), 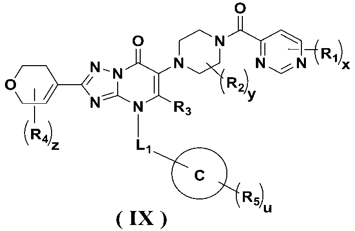
L₁ is selected from the group consisting of a bond, -O-, -S-, imino, C₁₋₆ alkylene, -C₁₋₆ alkylene-SO₂NH-, -C₁₋₆ alkylene-NHCONH-, -C₁₋₆ alkylene-NHCO-, -C₁₋₆ alkylene-NHSO₂-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, -alkylene-3- to 8-membered heterocycloalkylene-, C₆₋₁₄ arylene, and 5- to 14-membered heteroarylene, wherein the imino, C₁₋₆ alkylene, -C₁₋₆ alkylene-CONH-, -C₁₋₆ alkylene-SO₂NH-, -C₁₋₆ alkylene-NHCONH-, -C₁₋₆ alkylene-NHCO-, -C₁₋₆ alkylene-NHSO₂-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, -alkylene-3- to 8-membered heterocycloalkylene-, C₆₋₁₄ arylene, or 5- to 14-membered heteroarylene is optionally further substituted with one or more substituents selected from the group consisting of deuterium, halogen, amino, hydroxyl, cyano, oxo, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
preferably, L₁ is selected from the group consisting of a bond, -O-, -S-, imino, C₁₋₃ alkylene, -C₁₋₃ alkylene-SO₂NH-, -C₁₋₃alkylene-SO₂NH-, -C₁₋₃ alkylene-NHCONH-, -C₁₋₃ alkylene-NHCO-, -C₁₋₃ alkylene-NHSO₂-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃ haloalkyleneoxy, C₃₋₈ cycloalkylene, 3- to 8-membered heterocycloalkylene, -alkylene-3- to 8-membered heterocycloalkylene-, C₆₋₁₀ arylene, and 5- to 6-membered heteroarylene, wherein the imino, C₁₋₃ alkylene, -C₁₋₃ alkylene-SO₂NH-, -C₁₋₃ alkylene-SO₂NH-, -C₁₋₃ alkylene-NHCONH-, -C₁₋₃ alkylene-NHCO-, -C₁₋₃ alkylene-NHSO₂-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃ haloalkyleneoxy, C₃₋₈ cycloalkylene, 3- to 8-membered heterocycloalkylene, -alkylene-3- to 8-membered heterocycloalkylene-, C₆₋₁₀ arylene, or 5- to 6-membered heteroarylene is optionally further substituted with one or more substituents selected from the group consisting of deuterium, halogen, amino, hydroxyl, cyano, oxo, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
more preferably, L₁ is selected from the group consisting of -CH₂-, -CH₂SO₂NH-*, 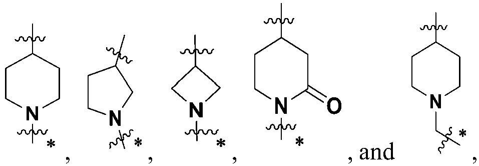 wherein the side with * is connected to ring C;
the definitions of other groups are as defined in any technical solution herein.

In certain embodiments of the present disclosure, L₁ is -COCH₂NH-*; or, L₁ is - CONH-*; or, L₁ is 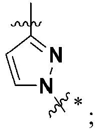 or, L₁ is 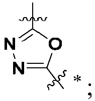 or, L₁ is 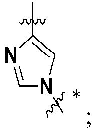 or, L₁ is 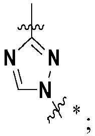 or, L₁ is 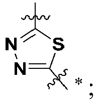 or, L₁ is 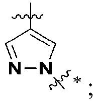 or, L₁ is 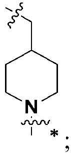 or, L₁ is 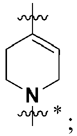 or, L₁ is selected from the group consisting of -CH₂CO-*, -CH₂CON(CH₃)-*, -CH₂CONHCH₂-*, -CH₂CH₂CONH-*, 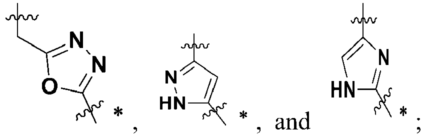 or, L₁ is selected from the group consisting of 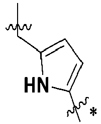 and -CH₂-C(=N-OH)NH-*; the side with * is connected to ring C; the definitions of other groups are as defined in any technical solution herein.

In certain embodiments of the present disclosure, the compound is represented by formula (XI), 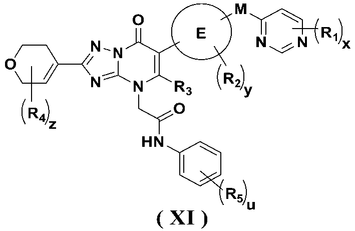
wherein ring E is selected from the group consisting of 4- to 6-membered heterocycloalkylene-spiro-4- to 6-membered heterocycloalkylene, 5- to 6-membered heterocycloalkylene-fused 5- to 6-membered heterocycloalkylene, 5- to 6-membered heteroaryl-fused 5- to 6-membered heterocycloalkenyl, and 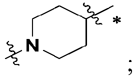
alternatively, ring E is directly connected to the parent to form

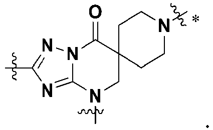

In certain embodiments of the present disclosure, the compound is represented by formula (XI), 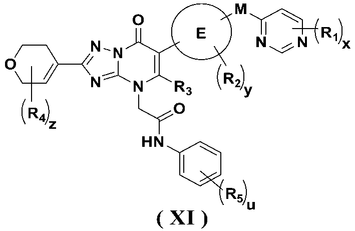
wherein ring E is selected from the group consisting of 4- to 6-membered heterocycloalkylene-spiro-4- to 6-membered heterocycloalkylene, 5- to 6-membered heterocycloalkylene-fused 5- to 6-membered heterocycloalkylene, and 5- to 6-membered heteroaryl-fused 5- to 6-membered heterocycloalkenyl;
alternatively, ring E is directly connected to the parent to form 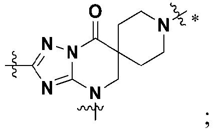
the definitions of other groups are as defined in any technical solution herein.

In certain embodiments of the present disclosure, ring E is selected from the group consisting of 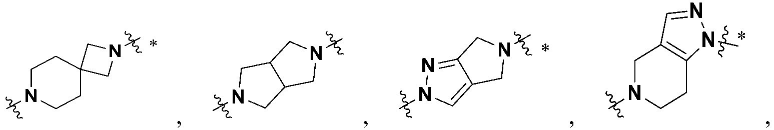 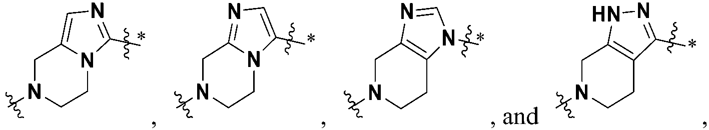 wherein the end with * is connected to M; the definitions of other groups are as defined in any technical solution herein.

In certain embodiments of the present disclosure, the compound is represented by formula (XII), 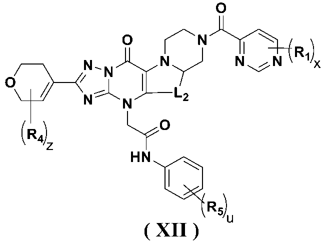 wherein
L₂ is selected from the group consisting of -CH₂OCH₂-, -CH₂CH₂CH₂-, -CH₂NHCH₂-, and -CH₂CH₂-, wherein the -CH₂OCH₂-, -CH₂CH₂CH₂-, -CH₂NHCH₂-, or -CH₂CH₂- is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl; the definitions of other groups are as defined in any technical solution herein.

In certain embodiments of the present disclosure, L₂ is -CH₂OCH₂-; or, L₂ is - CH₂CH₂CH₂-; or, L₂ is -CH₂NHCH₂-; or, L₂ is -CH₂CH₂-; the definitions of other groups are as defined in any technical solution herein.

In certain embodiments of the present disclosure, the compound is represented by formula (XIII), 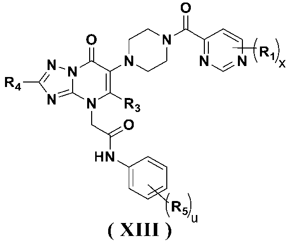
R₄ is independently selected from the group consisting of hydrogen, deuterium, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, 5- to 14-membered heteroaryl, -COR₄₋₁, -COOR₄₋₁, -OC(=O)R₄₋₁, -SO₂R₄₋₂, and -OR₄₋₃, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₄ₐ; the R₄ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl; alternatively, two R₄ₐ are connected to form a 3- to 8-membered heterocycloalkyl or a 3- to 8-membered heterocycloalkenyl;
R₄₋₁ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄₋₂ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄₋₃ is selected from the group consisting of hydrogen, deuterium, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₃₋₈ cycloalkyl, 3- to 8-membered heterocycloalkyl, C₆₋₁₄ aryl, and 5- to 14-membered heteroaryl, wherein the C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
the definitions of other groups are as defined in any technical solution herein.

In certain embodiments of the present disclosure, R₄ is independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, 5-to 6-membered heteroaryl, -COR₄₋₁, -COOR₄₋₁, -OC(=O)R₄₋₁, -SO₂R₄₋₂, and -OR₄₋₃, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₄ₐ; the R₄ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl; or, two R₄ₐ are connected to form a 3- to 6-membered heterocycloalkyl or a 3- to 6-membered heterocycloalkenyl;
R₄₋₁ is selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
R₄₋₂ is selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
R₄₋₃ is selected from the group consisting of hydrogen, deuterium, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₃₋₆ cycloalkyl, 3- to 6-membered heterocycloalkyl, C₆₋₁₀ aryl, and 5- to 6-membered heteroaryl, wherein the C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl; the definitions of other groups are as defined in any technical solution herein.

In certain embodiments of the present disclosure, R₄ is selected from the group consisting of 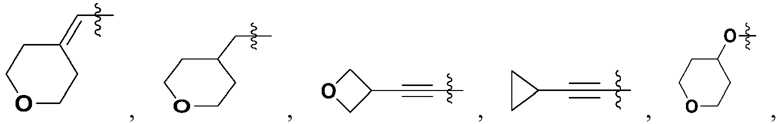 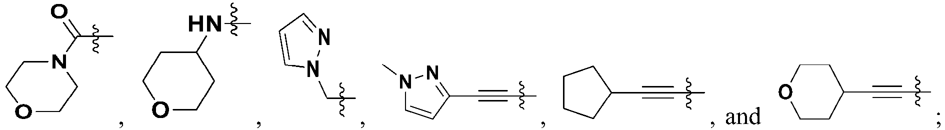 the definitions of other groups are as defined in any technical solution herein.

In certain embodiments of the present disclosure, the compound is represented by formula (XIV), 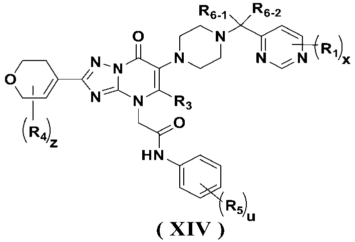
R₆₋₁ and R₆₋₂ are each independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₅ₐ; the R₅ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
alternatively, R₆₋₁ and R₆₋₂, together with the carbon atom to which they are attached, form a C₃₋₆ cycloalkyl or a 3- to 6-membered heterocycloalkyl;
the definitions of other groups are as defined in any technical solution herein.

In certain embodiments of the present disclosure, R₆₋₁ is selected from the group consisting of hydrogen, cyano, and trifluoromethyl; R₆₋₂ is hydrogen; the definitions of other groups are as defined in any technical solution herein.

In certain embodiments of the present disclosure, R₆₋₁ and R₆₋₂, together with the carbon atom to which they are attached, form 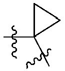 (i.e., M is 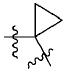 ) or 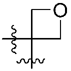 (i.e., M is 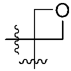 ); the definitions of other groups are as defined in any technical solution herein.

In certain embodiments of the present disclosure, the compound is represented by formula (XVII), 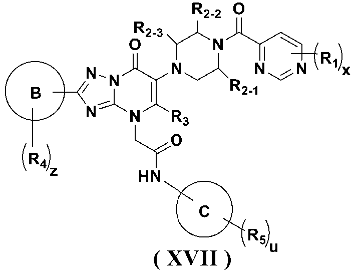
R₂₋₁, R₂₋₂, and R₂₋₃ are each independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy; or, R₂₋₂ and R₂₋₃ are connected to form a C₃₋₆ cycloalkyl or a 3- to 6-membered heterocycloalkyl, wherein the C₃₋₆ cycloalkyl and 3- to 6-membered heterocycloalkyl are optionally further substituted with one or more substituents selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, and C₁₋₃ haloalkoxy;
the definitions of other groups are as defined in any technical solution herein.

In certain embodiments of the present disclosure, the formula (XVII) is further represented by formula (XVII-1), 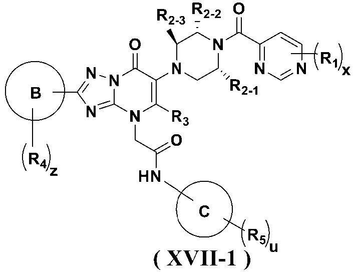 the definitions of groups are as defined in any technical solution herein.

In certain embodiments of the present disclosure, the compound is represented by formula (XV), 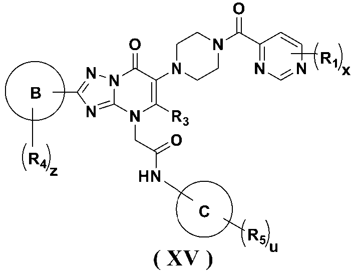 the definitions of groups are as defined in any technical solution herein.

In certain embodiments of the present disclosure, the compound is represented by formula (XVI), 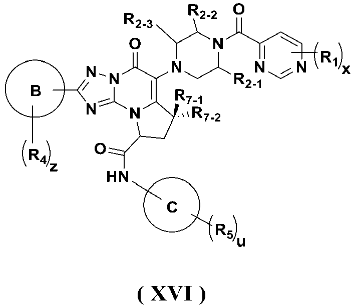
wherein R₂₋₁, R₂₋₂, and R₂₋₃ are each independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, and C₁₋₃ haloalkoxy; or, R₂₋₂ and R₂₋₃ are connected to form a C₃₋₆ cycloalkyl or a 3- to 6-membered heterocycloalkyl, wherein the C₃₋₆ cycloalkyl and 3- to 6-membered heterocycloalkyl are optionally further substituted with one or more substituents selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, and C₁₋₃ haloalkoxy;
R₇₋₁ and R₇₋₂ are each independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, and C₁₋₃ haloalkoxy;
alternatively, R₇₋₁ and R₇₋₂ are connected to form a C₃₋₆ cycloalkyl;
the definitions of other groups are as defined in any technical solution herein.

In certain embodiments of the present disclosure, R₂₋₂ and R₂₋₃ are connected to form a cyclobutyl, wherein the cyclobutyl is optionally further substituted with one or more substituents selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, and C₁₋₃ haloalkoxy; the definitions of other groups are as defined in any technical solution herein.

In certain embodiments of the present disclosure, the formula (XVI) is further represented by formula (XVI-1), 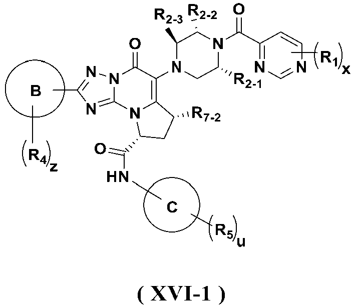 the definitions of groups are as defined in any technical solution herein.

In certain embodiments of the present disclosure, the structural moiety 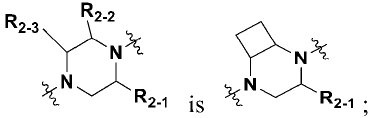 the definitions of other groups are as defined in any technical solution herein.

In certain embodiments of the present disclosure, the structural moiety 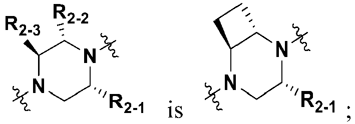 the definitions of other groups are as defined in any technical solution herein.

In certain embodiments of the present disclosure, R₂₋₁ is hydrogen; or, R₂₋₁ is methyl; the definitions of other groups are as defined in any technical solution herein.

In certain embodiments of the present disclosure, R₇₋₁ is hydrogen, R₇₋₂ is methyl; or, R₇₋₁ is methyl, R₇₋₂ is methyl; or, R₇₋₁ and R₇₋₂ are connected to form a cyclopropyl; the definitions of other groups are as defined in any technical solution herein.

In certain embodiments of the present disclosure, ring B is 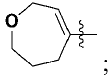 ring C is 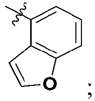 the definitions of other groups are as defined in any technical solution herein.

In certain embodiments of the present disclosure, ring B is 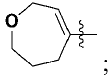 ring C is 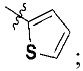 the definitions of other groups are as defined in any technical solution herein.

In certain embodiments of the present disclosure, 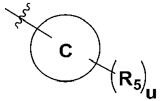 is 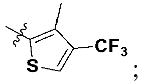 the definitions of other groups are as defined in any technical solution herein. The inventors have found that when ring C is modified into 5-membered heteroaryl, the thiophene ring can maintain excellent cell inhibitory activity, while other 5-membered heteroaryl groups, such as thiazolyl, furanyl, and pyrrolyl, all lead to a decrease in cell inhibitory activity, as evidenced by the comparison of compound 295 with compounds 342, 344, 345, and 348.

In certain embodiments of the present disclosure, 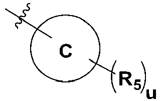 is 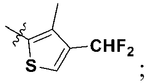 the definitions of other groups are as defined in any technical solution herein.

In certain embodiments of the present disclosure, 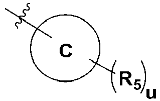 is 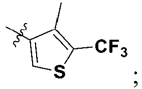 the definitions of other groups are as defined in any technical solution herein.

In certain embodiments of the present disclosure, 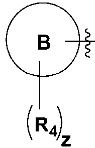 is 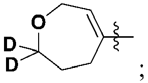 the definitions of other groups are as defined in any technical solution herein. The inventors have found that modifying 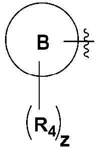 from 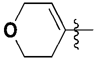 to 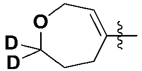 can significantly reduce the inhibitory effect of the compound on hERG potassium channel current, thereby improving safety, as evidenced by the comparison of 21A and compound 303-P4.

In certain embodiments of the present disclosure, 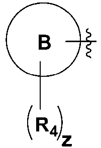 is 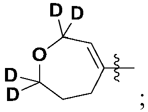 the definitions of other groups are as defined in any technical solution herein.

In certain embodiments of the present disclosure, 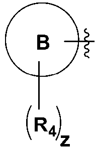 is 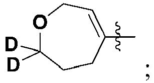 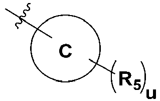 is 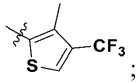 the definitions of other groups are as defined in any technical solution herein. The inventors have found that modifying 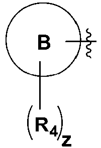 from 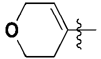 to 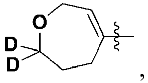 and modifying 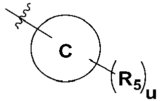 from 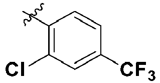 to 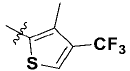 can further significantly reduce the inhibitory effect of the compound on hERG potassium channel current, thereby improving safety, as evidenced by the comparison of 21A and compound 295-P4.

In certain embodiments of the present disclosure, 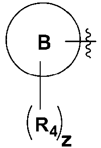 is 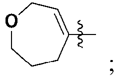 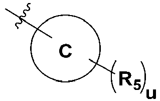 is 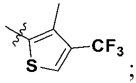 the definitions of other groups are as defined in any technical solution herein.

In certain embodiments of the present disclosure, 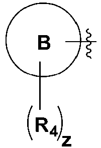 is 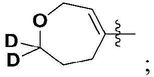 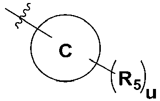 is 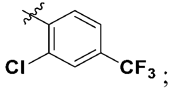 the definitions of other groups are as defined in any technical solution herein.

In certain embodiments of the present disclosure, 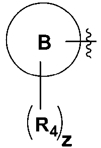 is 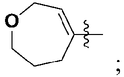 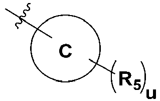 is 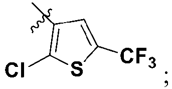 the definitions of other groups are as defined in any technical solution herein.

In certain embodiments of the present disclosure, 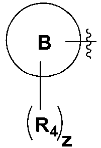 is 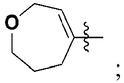 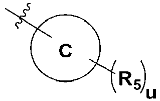 is 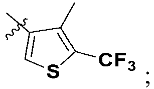 the definitions of other groups are as defined in any technical solution herein.

In certain embodiments of the present disclosure, R₁ is independently selected from the group consisting of hydrogen, deuterium, halogen, amino, hydroxyl, mercapto, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₁ₐ; the R₁ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl; the definitions of other groups are as defined in any technical solution herein.

In certain embodiments of the present disclosure, R₁ is independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₁ₐ; the R₁ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
preferably, R₁ is independently selected from the group consisting of hydroxyl, methyl, hydrogen, fluorine, and 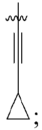
the definitions of other groups are as defined in any technical solution herein.

In certain embodiments of the present disclosure, R₁ is independently 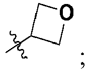 or, R₁ is independently selected from the group consisting of cyclopropyl, trifluoromethyl, mercapto, -CH₂OH, and -CD₃; or, R₁ is independently -N(CH₃)₂; the definitions of other groups are as defined in any technical solution herein.

In certain embodiments of the present disclosure, R₂ is independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₂ₐ; the R₂ₐ is independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
alternatively, two R₂ are connected to each other to form a C₃₋₆ cycloalkyl or a 3- to 6-membered heterocycloalkyl, wherein the C₃₋₆ cycloalkyl or 3- to 6-membered heterocycloalkyl is optionally further substituted with deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl;
preferably, R₂ is independently hydrogen;
the definitions of other groups are as defined in any technical solution herein.

In certain embodiments of the present disclosure, R₂ is independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₂ₐ; the R₂ₐ is independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
alternatively, two R₂ are connected to each other to form a C₃₋₆ cycloalkyl or a 3- to 6-membered heterocycloalkyl, wherein the C₃₋₆ cycloalkyl or 3- to 6-membered heterocycloalkyl is optionally further substituted with deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl;
alternatively, one R₂ and R₃ are connected to form 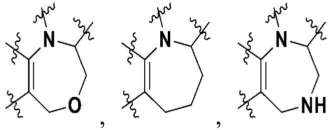 or 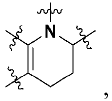 wherein the 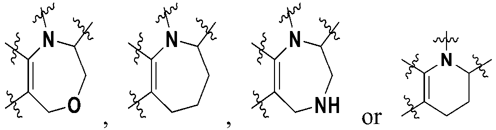 is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl; the definitions of other groups are as defined in any technical solution herein.

In certain embodiments of the present disclosure, R₂ is independently methyl; the definitions of other groups are as defined in any technical solution herein.

In certain embodiments of the present disclosure, R₃ is selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₃ₐ; the R₃ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
L₁ and R₃ are connected to form a 3- to 8-membered heterocycloalkenyl or a 5- to 6-membered heteroaryl; wherein the 3- to 8-membered heterocycloalkenyl or 5- to 10-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 10-membered heteroaryl;
R₃ is selected from the group consisting of ethyl, trifluoromethoxy, vinyl, ethynyl, cyano, and 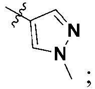
the definitions of other groups are as defined in any technical solution herein.

In certain embodiments of the present disclosure, R₃ is selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₃ₐ; the R₃ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
R₃ is selected from the group consisting of ethyl, trifluoromethoxy, vinyl, ethynyl, and cyano;
the definitions of other groups are as defined in any technical solution herein.

In certain embodiments of the present disclosure, R₄ is independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, 5-to 6-membered heteroaryl, -COR₄₋₁, -COOR₄₋₁, -OC(=O)R₄₋₁, -SO₂R₄₋₂, and -OR₄₋₃, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₄ₐ; the R₄ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl; or, two R₄ₐ are connected to form a 3- to 6-membered heterocycloalkyl;
R₄₋₁ is selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
R₄₋₂ is selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C_{1- 3} hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
R₄₋₃ is selected from the group consisting of hydrogen, deuterium, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₃₋₆ cycloalkyl, 3- to 6-membered heterocycloalkyl, C₆₋₁₀ aryl, and 5- to 6-membered heteroaryl, wherein the C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl; the definitions of other groups are as defined in any technical solution herein.
the definitions of other groups are as defined in any technical solution herein.

In certain embodiments of the present disclosure, R₄ is independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, 5-to 6-membered heteroaryl, -COR₄₋₁, and -SO₂R₄₋₂, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₄ₐ; the R₄ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
preferably, R₄ is independently selected from the group consisting of hydrogen, methyl, 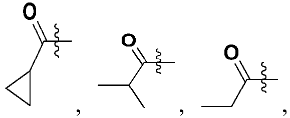 and -SO₂CH₃;
the definitions of other groups are as defined in any technical solution herein.

In certain embodiments of the present disclosure, R₄ is independently selected from the group consisting of 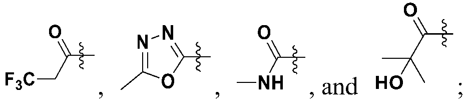 or, R₄ is independently selected from the group consisting of 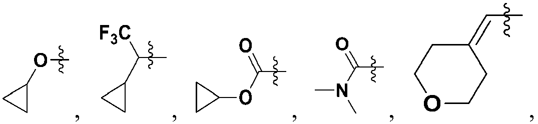 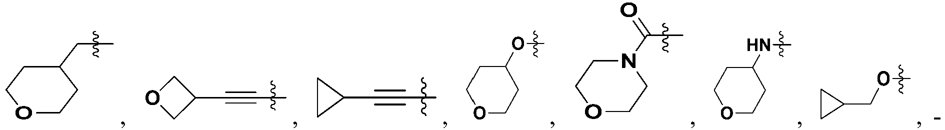 CH₂CN, 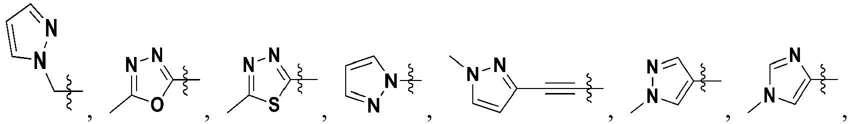 phenyl, 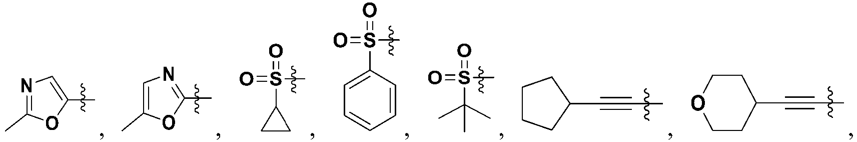 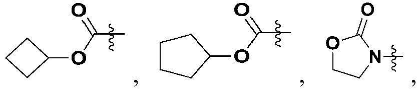 cyclopropyl, 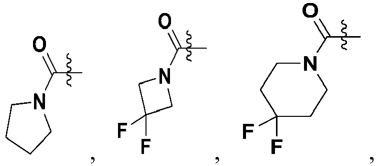 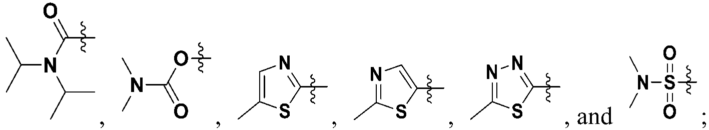 or, R₄ is independently selected from the group consisting of 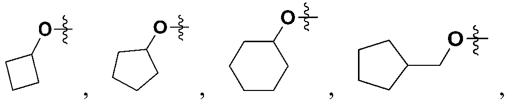 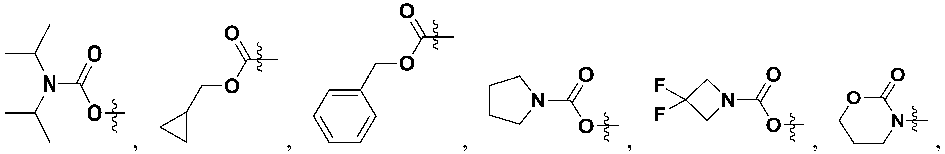 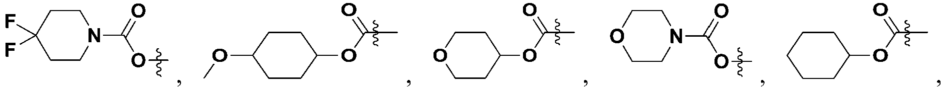 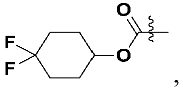 and fluorine; or, R₄ is independently deuterium; the definitions of other groups are as defined in any technical solution herein.

In certain embodiments of the present disclosure, R₄₋₁ is selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
more preferably, R₄₋₁ is selected from the group consisting of cyclopropyl, isopropyl, and ethyl;
the definitions of other groups are as defined in any technical solution herein.

In certain embodiments of the present disclosure, R₄₋₁ is selected from the group consisting of -CH₂CF₃, -NHCH₃, -N(CH₃)₂, -C(OH)(CH₃)₂, *tert-*butyl*,* and -CH₂-cyclopropyl.

In certain embodiments of the present disclosure, R₄₋₂ is selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
preferably, R₄₋₂ is methyl;
the definitions of other groups are as defined in any technical solution herein.

In certain embodiments of the present disclosure, R₅ is independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, -SO₂-C₁₋₃ alkyl, -SF₅, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₅ₐ; the R₅ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl; the definitions of other groups are as defined in any technical solution herein.

In certain embodiments of the present disclosure, R₅ is independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₅ₐ; the R₅ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
preferably, R₅ is independently selected from the group consisting of hydrogen, chlorine, and trifluoromethyl;
the definitions of other groups are as defined in any technical solution herein.

In certain embodiments of the present disclosure, R₅ is independently selected from the group consisting of fluorine and methyl; or, R₅ is independently cyclopropyl; or, R₅ is independently selected from the group consisting of 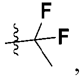 -SF₅, ethyl, and -SO₂CH₃; the definitions of other groups are as defined in any technical solution herein.

In certain embodiments of the present disclosure, L₁ is selected from the group consisting of a bond, -O-, -S-, imino, C₁₋₃ alkylene, -CONH-, -CO-C₁₋₃ alkylene-NH-, -C₁₋₃ alkylene-CONH-, -C₁₋₃ alkylene-CONH-C₁₋₃ alkylene, -C₁₋₃ alkylene-SO₂NH-, -C₁₋₃ alkylene-SO₂NH-, -C₁₋₃ alkylene-NHCONH-, -C₁₋₃ alkylene-NHCO-, -C₁₋₃ alkylene-NHSO₂-, -C₁₋₃ alkylene-CO-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃ haloalkyleneoxy, C₃₋₈ cycloalkylene, 3- to 8-membered heterocycloalkylene, 3- to 8-membered heterocycloalkenylene, -C₁₋₃ alkylene-3- to 8-membered heterocycloalkylene-, -C₁₋₃ alkylene-3- to 8-membered heterocycloalkenylene-, -C₁₋₃ alkylene-C₆₋₁₀ arylene-, C₆₋₁₀ arylene, -C₁₋₃ alkylene-5- to 6-membered heteroarylene-, and 5- to 6-membered heteroarylene, wherein the imino, C₁₋₃ alkylene, -CONH-, -CO-C₁₋₃ alkylene-NH-, -C₁₋₃ alkylene-CONH-, -C₁₋₃ alkylene-CONH-C₁₋₃ alkylene-C₁₋₃ alkylene-SO₂NH-, -C₁₋₃ alkylene-SO₂NH-, -C₁₋₃ alkylene-NHCONH-, -C₁₋₃ alkylene-NHCO-, -C₁₋₃ alkylene-NHSO₂-, -C₁₋₃ alkylene-CO-, -C₁₋₃ alkylene-C(=N-OH)NH-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃ haloalkyleneoxy, C₃₋₈ cycloalkylene, 3- to 8-membered heterocycloalkylene, 3- to 8-membered heterocycloalkenylene, -C₁₋₃ alkylene-3- to 8-membered heterocycloalkylene-, -C₁₋₃ alkylene-3- to 8-membered heterocycloalkenylene-, -C₁₋₃ alkylene-C₆₋₁₀ arylene-, C₆₋₁₀ arylene, -C₁₋₃ alkylene-5- to 6-membered heteroarylene-, or 5- to 6-membered heteroarylene is optionally further substituted with one or more substituents selected from the group consisting of deuterium, halogen, amino, hydroxyl, cyano, oxo, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl; the definitions of other groups are as defined in any technical solution herein.

In certain embodiments of the present disclosure, L₁ is selected from the group consisting of a bond, -O-, -S-, imino, C₁₋₃ alkylene, -CONH-, -CO-C₁₋₃ alkylene-NH-, -C₁₋₃ alkylene-CONH-, -C₁₋₃ alkylene-CONH-C₁₋₃ alkylene, -C₁₋₃ alkylene-SO₂NH-, -C₁₋₃ alkylene-SO₂NH-, -C₁₋₃ alkylene-NHCONH-, -C₁₋₃ alkylene-NHCO-, -C₁₋₃ alkylene-NHSO₂-, -C₁₋₃ alkylene-CO-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃ haloalkyleneoxy, C₃₋₈ cycloalkylene, 3- to 8-membered heterocycloalkylene, 3- to 8-membered heterocycloalkenylene, -C₁₋₃ alkylene-3- to 8-membered heterocycloalkylene-, -C₁₋₃ alkylene-3- to 8-membered heterocycloalkenylene-, -C₁₋₃ alkylene-C₆₋₁₀ arylene-, C₆₋₁₀ arylene, -C₁₋₃ alkylene-5- to 6-membered heteroarylene-, and 5- to 6-membered heteroarylene, wherein the imino, C₁₋₃ alkylene, -CONH-, -CO-C₁₋₃ alkylene-NH-, -C₁₋₃ alkylene-CONH-, -C₁₋₃ alkylene-CONH-C₁₋₃ alkylene-C₁₋₃ alkylene-SO₂NH-, -C₁₋₃ alkylene-SO₂NH-, -C₁₋₃ alkylene-NHCONH-, -C₁₋₃ alkylene-NHCO-, -C₁₋₃ alkylene-NHSO₂-, -C₁₋₃ alkylene-CO-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃haloalkyleneoxy, C₃₋₈ cycloalkylene, 3- to 8-membered heterocycloalkylene, 3- to 8-membered heterocycloalkenylene, -C₁₋₃ alkylene-3- to 8-membered heterocycloalkylene-, -C₁₋₃ alkylene-3- to 8-membered heterocycloalkenylene-, -C₁₋₃ alkylene-C₆₋₁₀ arylene-, C₆₋₁₀ arylene, -C₁₋₃ alkylene-5- to 6-membered heteroarylene-, or 5-to 6-membered heteroarylene is optionally further substituted with one or more substituents selected from the group consisting of deuterium, halogen, amino, hydroxyl, cyano, oxo, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5-to 6-membered heteroaryl;
the definitions of other groups are as defined in any technical solution herein.

In certain embodiments of the present disclosure, L₁ is selected from the group consisting of a bond, -O-, -S-, imino, C₁₋₃ alkylene, -CONH-, -CO-C₁₋₃ alkylene-NH-, -C₁₋₃ alkylene-CONH-, -C₁₋₃ alkylene-SO₂NH-, -C₁₋₃ alkylene-SO₂NH-, -C₁₋₃ alkylene-NHCONH-, -C₁₋₃ alkylene-NHCO-, -C₁₋₃ alkylene-NHSO₂-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃ haloalkyleneoxy, C₃₋₈ cycloalkylene, 3- to 8-membered heterocycloalkylene, 3- to 8-membered heterocycloalkenylene, -alkylene-3- to 8-membered heterocycloalkylene-, C₆₋₁₀ arylene, and 5- to 6-membered heteroarylene, wherein the imino, C₁₋₃ alkylene, -CONH-, -CO-C₁₋₃ alkylene-NH-, -C₁₋₃ alkylene-CONH-, -C₁₋₃ alkylene-SO₂NH-, -C₁₋₃ alkylene-SO₂NH-, -C₁₋₃ alkylene-NHCONH-, -C₁₋₃ alkylene-NHCO-, -C₁₋₃ alkylene-NHSO₂-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃ haloalkyleneoxy, C₃₋₈ cycloalkylene, 3- to 8-membered heterocycloalkylene, 3- to 8-membered heterocycloalkenylene, -alkylene-3- to 8-membered heterocycloalkylene-, C₆₋₁₀ arylene, or 5- to 6-membered heteroarylene is optionally further substituted with one or more substituents selected from the group consisting of deuterium, halogen, amino, hydroxyl, cyano, oxo, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
the definitions of other groups are as defined in any technical solution herein.

In certain embodiments of the present disclosure, L₁ is selected from the group consisting of a bond, -O-, -S-, imino, C₁₋₃ alkylene, -C₁₋₃ alkylene-CONH-, -C₁₋₃ alkylene-SO₂NH-, -C₁₋₃ alkylene-SO₂NH-, -C₁₋₃ alkylene-NHCONH-, -C₁₋₃ alkylene-NHCO-, -C₁₋₃ alkylene-NHSO₂-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃ haloalkyleneoxy, C₃₋₈ cycloalkylene, 3- to 8-membered heterocycloalkylene, -alkylene-3- to 8-membered heterocycloalkylene-, C₆₋₁₀ arylene, and 5- to 6-membered heteroarylene, wherein the imino, C₁₋₃ alkylene, -C₁₋₃ alkylene-CONH-, -C₁₋₃ alkylene-SO₂NH-, -C₁₋₃ alkylene-SO₂NH-, -C₁₋₃ alkylene-NHCONH-, -C₁₋₃ alkylene-NHCO-, -C₁₋₃ alkylene-NHSO₂-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃ haloalkyleneoxy, C₃₋₈ cycloalkylene, 3- to 8-membered heterocycloalkylene, -alkylene-3- to 8-membered heterocycloalkylene-, C₆₋₁₀ arylene, or 5- to 6-membered heteroarylene is optionally further substituted with one or more substituents selected from the group consisting of deuterium, halogen, amino, hydroxyl, cyano, oxo, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5-to 6-membered heteroaryl;
more preferably, L₁ is selected from the group consisting of -CH₂-, -CH₂CONH-*, - CH₂SO₂NH-*, 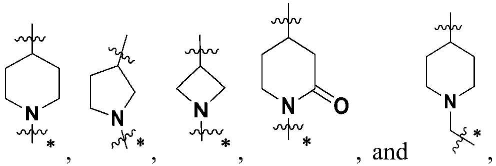 wherein the side with * is connected to ring C;
the definitions of other groups are as defined in any technical solution herein.

In certain embodiments of the present disclosure, L₁ is selected from the group consisting of -COCH₂NH-*, -CONH-*, 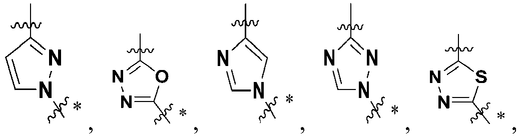 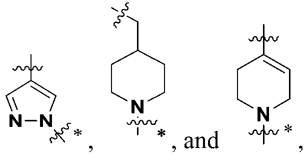 wherein the side with * is connected to ring C;
the definitions of other groups are as defined in any technical solution herein.

In certain embodiments of the present disclosure, R₆₋₁ and R₆₋₂ are each independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₅ₐ; the R₅ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
alternatively, R₆₋₁ and R₆₋₂, together with the carbon atom to which they are attached, form a C₃₋₆ cycloalkyl or a 3- to 6-membered heterocycloalkyl;
the definitions of other groups are as defined in any technical solution herein.

In certain embodiments of the present disclosure, R₆₋₁ is selected from the group consisting of hydrogen, cyano, and trifluoromethyl; R₆₋₂ is hydrogen; the definitions of other groups are as defined in any technical solution herein.

In certain embodiments of the present disclosure, R₆₋₁ and R₆₋₂, together with the carbon atom to which they are attached, form 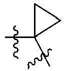 (i.e., M is 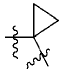 ) or 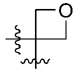 (i.e., M is 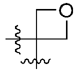 ); the definitions of other groups are as defined in any technical solution herein.

In certain embodiments of the present disclosure, R₇₋₁ and R₇₋₂ are each independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, and C₁₋₃ haloalkoxy; the definitions of other groups are as defined in any technical solution herein.

In certain embodiments of the present disclosure, R₇₋₁ is selected from the group consisting of hydrogen and methyl; the definitions of other groups are as defined in any technical solution herein.

In certain embodiments of the present disclosure, R₇₋₂ is methyl; the definitions of other groups are as defined in any technical solution herein.

In certain embodiments of the present disclosure, R₇₋₁ and R₇₋₂ are connected to form a C₃₋₆ cycloalkyl; the definitions of other groups are as defined in any technical solution herein.

The present disclosure also provides the following compounds, stereoisomers thereof, or pharmaceutically acceptable salts thereof:

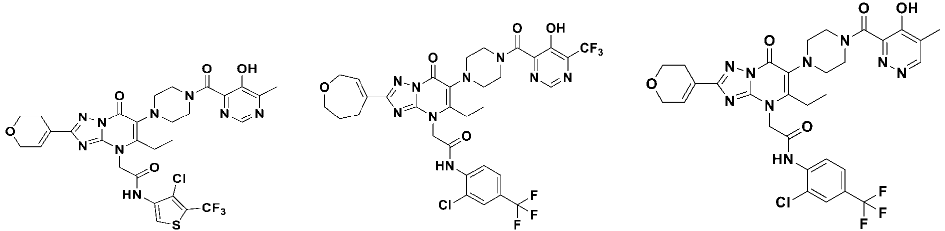

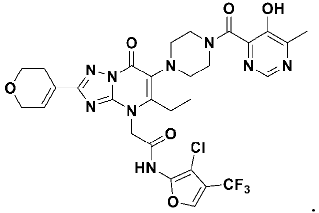

In certain embodiments of the present disclosure, ring A is 5- to 14-membered heteroaryl; preferably 5- to 6-membered heteroaryl, for example, 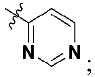
ring B is selected from the group consisting of 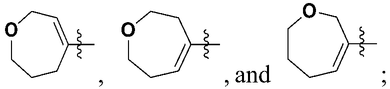 preferably 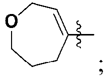
ring C is selected from the group consisting of C₆₋₁₄ aryl and 5- to 14-membered heteroaryl; preferably C₆₋₁₀ aryl or 5- to 6-membered heteroaryl; for example, phenyl, 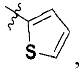 or 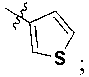 more particularly 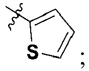
ring E is 3- to 12-membered heterocycloalkylene; preferably 5- to 6-membered **heterocycloalkylene-fused** C₃₋₆ cycloalkylene; for example, 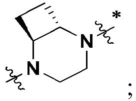
L₁, R₃, and the parent form a moiety 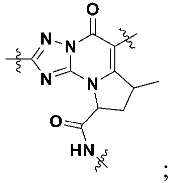 preferably

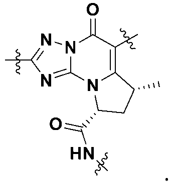

In certain embodiments of the present disclosure, M is -CO-;
ring A is 5- to 14-membered heteroaryl; preferably 5- to 6-membered heteroaryl, for example, 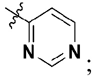
ring B is selected from the group consisting of 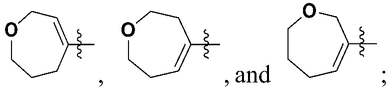 preferably 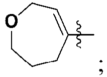
ring C is selected from the group consisting of C₆₋₁₄ aryl and 5- to 14-membered heteroaryl; preferably C₆₋₁₀ aryl or 5- to 6-membered heteroaryl; for example, phenyl, 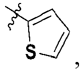 or 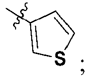 more particularly 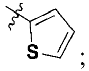
ring E is 3- to 12-membered heterocycloalkylene; preferably 5- to 6-membered heterocycloalkylene-fused C₃₋₆ cycloalkylene; for example, 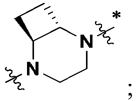
L₁, R₃, and the parent form a moiety 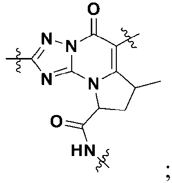 preferably 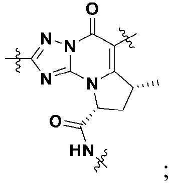
R₁ is independently selected from the group consisting of hydroxyl and C₁₋₆ alkyl;
R₄ is deuterium;
R₅ is independently selected from the group consisting of halogen, C₁₋₆ alkyl, and C₁₋₆ haloalkyl;
x is selected from the group consisting of 0, 1, and 2; preferably 2;
y is 0;
z is selected from the group consisting of 0, 1, and 2; preferably 2;
u is selected from the group consisting of 0, 1, and 2; preferably 2.

In certain embodiments of the present disclosure, ring B is 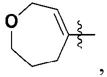 and ring C is phenyl or

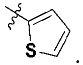

In certain embodiments of the present disclosure, the compound is any one of the following compounds: 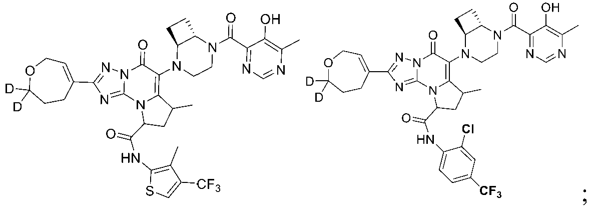 preferably, the compound is any one of the following compounds:

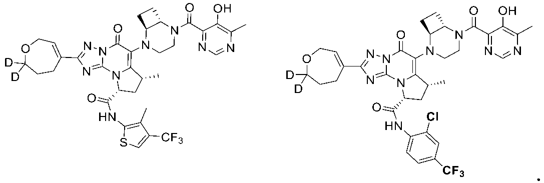

The present disclosure also provides a crystal form of a compound shown below, 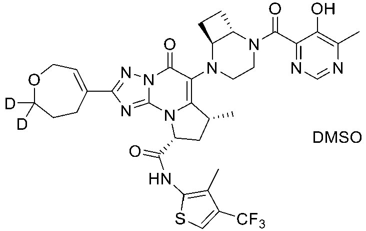 the parameters of the crystal form are shown below:

| | |
|---|---|
| C₃₄H₃₄D₂F₃N₉O₅S·C₂H₆OS | *D*ₓ = 1.399 Mg m⁻³ |
| *Mᵣ* = 819.92 | Cu Kα radiation, λ = 1.54178 Å |
| Orthorhombic, *P*2₁2₁2₁ | Cell parameters from 9935 reflections |
| *a* = 11.3002 (3) Å | θ = 3.5-63.8° |
| *b* = 15.9609 (4) Å | µ = 1.86 mm⁻¹ |
| *c* = 21.5818 (6) Å | *T =* 150 K |
| *V* = 3892.52 (18) Å³ | Block, colourless |
| *Z* = 4 | 0.15 × 0.06 × 0.05 mm |
| *F*(000) = 1712 | |

.

The present disclosure also provides a crystal form of a compound shown below, 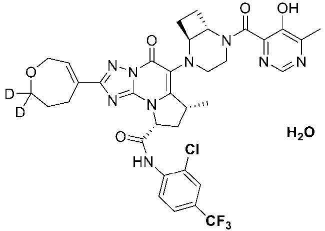 the parameters of the crystal form are shown below:

| | |
|---|---|
| C₃₅H₃₅ClF₃N₉O₅·H₂O | *F*(000) = 1608 |
| *Mᵣ* = 772.18 | Dx = 1.421 Mg m⁻³ |
| Monoclinic, *P*2₁ | Cu Kα radiation, λ = 1.54178 Å |
| *a* = 14.4250 (8) Å | Cell parameters from 2512 reflections |
| *b* = 19.2839 (10) Å | θ = 4.1-60.8° |
| *c* = 14.1565 (9) Å | µ = 1.58 mm⁻¹ |
| β = 113.585 (4)° | *T* = 170 K |
| *V* = 3609.0 (4) Å³ | Block, colourless |
| *Z* = 4 | 0.09 × 0.06 × 0.04 mm |

**.**

The present disclosure also provides a pharmaceutical composition comprising the compound, the stereoisomer thereof, or the pharmaceutically acceptable salt thereof of the present disclosure, and a pharmaceutically acceptable carrier.

In certain embodiments of the present disclosure, in the pharmaceutical composition, the content of the compound, the stereoisomer thereof, or the pharmaceutically acceptable salt thereof is selected from 0.1 mg to 1000 mg.

In certain embodiments of the present disclosure, in the pharmaceutical composition, the pharmaceutically acceptable carrier comprises one or more selected from the group consisting of a filler, a disintegrant, a binder, a glidant, and a lubricant.

The present disclosure also provides a use of the aforementioned compound, the stereoisomer thereof, or the pharmaceutically acceptable salt thereof, or the aforementioned pharmaceutical composition in the manufacture of a medicament for treating a WRN-mediated disease.

In certain embodiments of the present disclosure, the disease is cancer; preferably, the cancer is selected from the group consisting of colorectal cancer, endometrial cancer, ovarian cancer, and gastric cancer; more preferably, the cancer is microsatellite instability-high (MSI-H) or mismatch repair-deficient (dMMR) cancer.

The present disclosure also provides a method of treating a WRN-mediated disease in a human, wherein the method comprises administering to a human in need thereof an effective prophylactic or therapeutic amount of the aforementioned compound, the stereoisomer, or the pharmaceutically acceptable salt thereof, or the aforementioned pharmaceutical composition.

In certain embodiments of the present disclosure, the disease is cancer; preferably, the cancer is selected from the group consisting of colorectal cancer, endometrial cancer, ovarian cancer, and gastric cancer; more preferably, the cancer is microsatellite instability-high (MSI-H) or mismatch repair-deficient (dMMR) cancer.

### Technical effects

The compounds of the present disclosure have good WRN enzyme and cell inhibitory activities, and have selectivity for MSS and WRN-KO cells.

### BRIEF DESCRIPTION OF THE DRAWINGS

FIG. 1 shows the crystal structure of a DMSO solvate of compound **295-P4.**
FIG. 2 shows the crystal structure of a monohydrate of compound **303-P4.**

### DESCRIPTION AND DEFINITION

Unless otherwise specified, the following terms and phrases used herein have the following meanings. A specific term or phrase should not be considered indefinite or unclear in the absence of a particular definition, but should be understood in the ordinary sense.

The term "pharmaceutically acceptable" refers to those compounds, materials, compositions and/or dosage forms, which are suitable for use in contact with human and animal tissues within the scope of reasonable medical judgment, without excessive toxicity, irritation, allergic reaction or other problems or complications, and is commensurate with a reasonable benefit/risk ratio.

The term "pharmaceutically acceptable salt" refers to a derivative prepared by the compound of the present disclosure with a relatively nontoxic acid or base. These salts may be prepared during the synthesis, separation and purification of the compound, or the free form of the purified compound may be used alone to react with a suitable acid or base. When the compound contains relatively acidic functional groups (e.g., -COOH, -OH, or -SO₃H), the compound reacts with appropriate inorganic or organic cations (bases) to form base addition salts, including salts formed with alkali metals or alkaline earth metals, ammonium salts formed with amines or their derivatives, salts formed with amino acids, etc. When the compound contains relatively basic functional groups (e.g., -NH₂,), the compound reacts with appropriate inorganic or organic anions (acids) to form acid addition salts, including salts formed with inorganic acids or organic acids (e.g., carboxylic acids).

The term "pharmaceutically acceptable carrier" refers to a medium generally acceptable in the art for delivering a biologically active agent to animals, particularly mammals, including, for example, an adjuvant, an excipient or a vehicle, such as a diluent, a preservative, a filler, a flow regulator, a disintegrant, a wetting agent, an emulsifier, a suspending agent, a sweetener, a flavor, a fragrance, an antibacterial agent, an antifungal agent, a lubricants agent and a dispersant, according to the mode of administration and the nature of dosage forms. The pharmaceutically acceptable carrier is formulated according to a number of factors that are within the purview of those skill in the art. The factors include, but are not limited to, the type and nature of the active agent formulated, the subjects to which the composition containing the agent is to be administered, the expected route of administration of the composition, and the target therapeutic indication. The pharmaceutically acceptable carriers include both aqueous and non-aqueous media and various solid and semisolid dosage forms. In addition to the active agent, such carriers include many different ingredients and additives, and such additional ingredients included in prescriptions for a variety of reasons (e.g., stabilizing the active agent and an adhesive, etc.) are well known to those of ordinary skill in the art.

The term "isotopic derivative" refers to a compound in which at least one atom is replaced by an atom having the same atomic number but a different atomic mass. Examples of isotopes in compounds of the present disclosure include, but are not limited to, isotopes of hydrogen (e.g., deuterium (²H), tritium (³H)); isotopes of carbon (e.g., ¹¹C, ¹³C, and ¹⁴C); isotopes of chlorine (e.g., ³⁶Cl); isotopes of fluorine (e.g., ¹⁸F); isotopes of iodine (e.g., ¹²³I and ¹²⁵I); isotopes of nitrogen (e.g., ¹³N and ¹⁵N); isotopes of oxygen (e.g., ¹⁵O, ¹⁷O, and ¹⁸O); isotopes of phosphorus (e.g., ³²P); and isotopes of sulfur (e.g., ³⁵S). Isotopically labeled compounds of the present disclosure can generally be prepared by conventional techniques known to those skilled in the art, or can be prepared by methods analogous to those described herein using an appropriate isotopically labeled reagent in place of an unlabeled reagent.

The term "prodrug" refers to certain derivatives of the compounds of the present disclosure that themselves have little or no pharmacological activity, which contain a cleavable group and decompose into the compounds of the present disclosure by solvolysis or under physiological conditions. Such prodrug types include, but are not limited to, amides, esters, anhydrides, salts, and the like. The term "ester" refers to a derivative formed when a compound of the present disclosure contains an acidic group (such as a carboxylic acid) and reacts with a suitable alcohol; or when a compound of the present disclosure contains a hydroxyl group and reacts with a suitable acid (including organic or inorganic acids). Methods for preparing prodrugs are well known to those skilled in the art.

The term "solvate" refers to an association or complex of one or more solvent molecules with a compound of the present disclosure. When the solvent is water, the term "hydrate" may be used. The solvent molecules may be stoichiometric or non-stoichiometric. Methods for preparing solvates are known in the art.

The term "nitrogen oxide" or "N-oxide" refers to a derivative formed by further oxidation of a nitrogen atom in a nitrogen-containing group. Common N-oxides include N-oxides of tertiary amines or N-oxides of nitrogen atoms in nitrogen-containing heterocycles. Methods for synthesizing N-oxides are well known to those skilled in the art, including oxidation of heterocycles and tertiary amines with peroxy acids such as peracetic acid and m-chloroperoxybenzoic acid, hydrogen peroxide, alkyl hydroperoxides such as *tert-*butyl hydroperoxide, sodium perborate, and dioxiranes such as dimethyldioxirane.

The compounds of the present disclosure and their salts may exist in the form of isotopic derivatives, and the compounds of the present disclosure include various isotopic derivatives and mixtures thereof.

The compounds of the present disclosure and their salts may exist in the form of solvates, for example, hydrates, and the compounds of the present disclosure include various solvates and mixtures thereof.

The compounds of the present disclosure and their salts may exist in the form of "N-oxides", and the compounds of the present disclosure include various N-oxides and mixtures thereof.

The compounds described in the present disclosure, their pharmaceutically acceptable salts, isotopic derivatives, solvates, and N-oxides have the same or similar biological activities, and all of them are included within the scope of the present disclosure.

The term "prophylactically or therapeutically effective amount" refers to a sufficient amount of the compound of the present disclosure, a pharmaceutically acceptable salt thereof, or a stereoisomer thereof, to treat a disorder with a reasonable benefit/risk ratio applicable to any medical treatment and/or prevention. However, it should be recognized that the total daily dosage of the compounds of the present disclosure, stereoisomers thereof, or pharmaceutically acceptable salts and compositions thereof must be determined by the attending physician within the scope of sound medical judgment. For any specific patient, the specific therapeutically effective dose level depends on a variety of factors, including a disorder being treated and the severity of the disorder; activity of a specific compound used; a specific composition used; age, weight, general health status, sex and diet of the patient; administration time, administration route and excretion rate of the specific compound used; duration of treatment; drugs used in combination or simultaneously with the specific compounds used; and similar factors known in the medical field.

The absolute stereo configuration of a compound may be confirmed by conventional technical means in the art. For example, a single crystal X-ray diffraction method may also confirm the absolute configuration of the compound by the chiral structure of the raw material and the reaction mechanism of asymmetric synthesis. Alternatively, after resolution, the stereochemical configuration may be determined by comparison with a product of determined absolute configuration. Compounds marked herein as "absolute configuration unknown/undetermined" are typically split from racemic compounds into single isomers by chiral preparative SFC, which are then characterized and tested.

The term "optionally" means that it may be substituted or may not be substituted. Unless otherwise specified, the type and number of substituents may be arbitrary, provided they are chemically feasible. For example, the term "optionally substituted with one or more R₁" means that it may be substituted with one or more R₁ or may not be substituted with R₁.

When any variable (such as R₁) occurs more than once in the composition or structure of a compound, the definition of the variable at each occurrence is independent. For example, 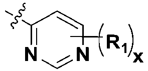 indicates that the pyrimidinyl group is substituted with x R₁ groups, and each R₁ is independently selected.

When the bond of a substituent may be cross-connected to two atoms on a ring, the substituent may be bonded to any atom on the ring. For example, the structure moiety 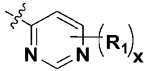 indicates that the substituent R₁ may be substituted at any position on the pyrimidinyl group.

When a listed substituent does not indicate through which atom it is attached to a compound included in the general formula but not specifically mentioned, such substituent may be bonded through any of its atoms. For example, pyrimidine as a substituent means that any carbon atom or nitrogen atom on the pyrimidine ring is attached to the substituted group; when 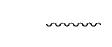 appears in the structure, it indicates that the atom is a bonding atom, for example, 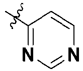 indicates that the C atom on the pyrimidine ring is the bonding atom.

Unless otherwise specified, the term "alkyl" refers to a group derived from a branched or straight-chain saturated aliphatic alkane having the specified number of carbon atoms by removal of one hydrogen atom. For example, "C₁₋₆ alkyl" refers to including C₁, C₂, C₃, C₄, C₅, and C₆ alkyl, "C₁₋₆ alkyl", and "C₁₋₃ alkyl"; specific examples include, but are not limited to: methyl, ethyl, n-propyl, isopropyl, sec-butyl, 2-methylbutyl, 1,1-dimethylbutyl, etc.

Unless otherwise specified, the term "alkylene" refers to a group derived from an alkyl group by removal of one hydrogen atom, wherein the alkyl is as defined above.

Unless otherwise specified, the term "alkenyl" refers to a group derived from a straight-chain or branched alkene (containing at least one carbon-carbon double bond) by removal of one hydrogen atom, including "C₂₋₆ alkenyl", "C₂₋₅ alkenyl", "C₂₋₄ alkenyl", and "C₂₋₃ alkenyl". Specific examples include, but are not limited to: -CH=CH₂, -CH=CHCH₃, - C(CH₂)=CH₂, -CH=CHCH₂CH₃, -CH₂CH=CHCH₃, etc.

Unless otherwise specified, the term "alkenylene" refers to a group derived from an alkenyl group by removal of one hydrogen atom, wherein the alkenyl is as defined above.

Unless otherwise specified, the term "alkynyl" refers to a group derived from a straight-chain or branched alkyne (containing at least one carbon-carbon triple bond) by removal of one hydrogen atom, including "C₂₋₆ alkynyl", "C₂₋₄ alkynyl", and "C₂₋₃ alkynyl". Specific examples include, but are not limited to: -C≡CH, -C≡CHCH₃, CH≡CHCH₂-, CH=C-C≡C-, etc.

Unless otherwise specified, the term "alkynylene" refers to a group derived from an alkynyl group by removal of one hydrogen atom, wherein the alkynyl is as defined above.

Unless otherwise specified, the term "alkoxy" refers to an alkyl group as defined herein attached to another group via an oxygen atom, i.e., "alkyl-O-". "C₁₋₆ alkoxy" (structure: C₁₋₆ alkyl-O-) and "C₁₋₃ alkoxy" are included. Specific examples include, but are not limited to, methoxy, ethoxy, propoxy, 1-methylethoxy, butoxy, 2-methylbutoxy, 3-methylbutoxy, 1,1-dimethylpropoxy, 1,2-dimethylpropoxy, etc.; preferably, the "alkoxy" described in the present disclosure is preferably C₁₋₃ alkoxy.

Unless otherwise specified, the term "alkyleneoxy" refers to a group derived from an alkoxy group by removal of one hydrogen atom, wherein the alkoxy is as defined above.

Unless otherwise specified, the term "halogen" refers to fluorine, chlorine, bromine or iodine atom.

Unless otherwise specified, the term "haloalkyl" refers to alkyl in which one or more hydrogen atoms are substituted with halogen atoms. C₁₋₆ haloalkyl is preferred, and C₁₋₃ haloalkyl is more preferred. Examples of haloalkyl include, but are not limited to, fluoromethyl, difluoromethyl, trifluoromethyl, trichloromethyl, tribromomethyl, 2,2,2-trifluoroethyl, 2,2,2-trichloroethyl, etc. The alkyl is as defined above.

Unless otherwise specified, the term "haloalkylene" refers to a group derived from a haloalkyl group by removal of one hydrogen atom, wherein the haloalkyl is as defined above.

Unless otherwise specified, the term "hydroxyalkyl" refers to a group derived by substituting one or more hydrogen atoms in an alkyl group with a hydroxyl group. The "hydroxyalkyl" described in the present disclosure includes "C₁₋₆ hydroxyalkyl" and "C₁₋₃ hydroxyalkyl"; specific examples include, but are not limited to, -CH₂OH, -CH₂CH₂OH, - CH(OH)CH₃, -CH₂CH₂CH₂OH, 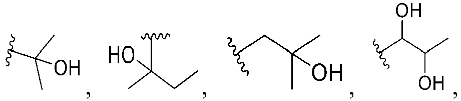 etc.

Unless otherwise specified, the term "hydroxyalkylene" refers to a group derived from a hydroxyalkyl group by removal of one hydrogen atom, wherein the hydroxyalkyl is as defined above.

Unless otherwise specified, the term "alkylthio" refers to -S-alkyl, wherein the alkyl is as defined above.

Unless otherwise specified, the term "alkylenethio" refers to a group derived from an alkylthio group by removal of one hydrogen atom, wherein the alkylthio is as defined above.

Unless otherwise specified, the term "haloalkoxy" refers to an alkoxy group in which one or more hydrogen atoms are substituted with halogen. Preferably, the "haloalkoxy" described in the present disclosure is preferably "C₁₋₆ haloalkoxy" or "C₁₋₃ haloalkoxy". Specific examples described in the present disclosure include: fluoromethoxy (including monofluoromethoxy, difluoromethoxy, trifluoromethoxy), -OCH₂CF₃, -OCHFCH₃, etc. The alkoxy is as defined above.

Unless otherwise specified, the term "haloalkyleneoxy" refers to a group derived from a haloalkoxy group by removal of one hydrogen atom, wherein the haloalkoxy is as defined above.

A group derived from a ring by removal of a hydrogen atom is referred to as a "cyclic group", which includes monovalent rings, divalent rings (commonly referred to as cycloalkylene), trivalent rings, tetravalent rings, etc., with the specific valence depending on the number of substituents attached to the ring. In the present disclosure, the description of "cyclic group" does not specifically distinguish between different valences of the ring. Representative "cyclic group" includes substituted or unsubstituted cycloalkyl, heterocycloalkyl, cycloalkenyl, heterocycloalkenyl, cycloalkynyl, heterocycloalkynyl, aryl, or heteroaryl.

Unless otherwise specified, the term "hetero" refers to substituted or unsubstituted heteroatoms and oxidized forms of heteroatoms (also known as heteroatom groups), the heteroatoms being generally selected from N, O, and S, and the oxidized forms generally including NO, SO, S(O)₂, and P(O). Nitrogen atoms may be substituted, namely NR (R is H or other substituents defined herein); the number of atoms on the ring is usually defined as the number of the ring members, for example, "3- to 6-membered heterocycloalkyl" refers to a ring formed by 3 to 6 atoms arranged around it, and each ring optionally contains 1 to 3 heteroatoms, namely N, O, CO, S, NO, SO, S(O)₂, or NR, and each ring is optionally substituted with R, and R is a group as defined herein.

Unless otherwise specified, the term "cycloalkyl" refers to a saturated cyclic alkyl group derived from cycloalkane by removal of one hydrogen atom, including monocyclic or polycyclic saturated hydrocarbon groups; the polycyclic saturated hydrocarbon groups refer to polycyclic groups formed by connecting two or more cyclic alkyl structures via spiro, bridged, fused, or other linking modes. The carbon atom(s) in the cycloalkyl may be further substituted with oxo, i.e., forming C(O). Unless otherwise specified, the term "certain-membered cycloalkyl" as described herein can be understood as a monocyclic cycloalkyl, and when it is polycyclic, it will be specifically indicated as a spiro, fused, or bridged ring group. The cycloalkyl includes "C₃₋₁₂ cycloalkyl," "C₃₋₈ cycloalkyl," "C₃₋₆ cycloalkyl", and "C₃₋₅ cycloalkyl". Preferably, specific examples of the cycloalkyl include, but are not limited to: cyclopropyl, cyclobutyl, cyclopentyl, cyclohexyl, cycloheptyl, and cyclooctyl.

Unless otherwise specified, the term "cycloalkylene" refers to a group derived from a cycloalkyl group by removal of one hydrogen atom, wherein the cycloalkyl is as defined above.

Unless otherwise specified, the term "heterocycloalkyl" refers to a saturated cyclic group derived from a cycloalkyl group by replacing one or more ring carbon atoms with heteroatoms and/or heteroatom groups. The heteroatoms and/or heteroatom groups are generally selected from N, O, S, NO, SO, S(O)₂, P(O), and NR, wherein the carbon atoms in the heterocycle are optionally oxidized, i.e., forming -C(O); preferably, the heteroatoms are independently selected from 1 to 3 N and/or O. The heterocycloalkyl includes "3- to 12-membered heterocycloalkyl", "3- to 8-membered heterocycloalkyl", "3- to 6-membered heterocycloalkyl", "3- to 5-membered heterocycloalkyl", "4- to 6-membered heterocycloalkyl", and "5- to 6-membered heterocycloalkyl". Examples of heterocycloalkyl include, but are not limited to:

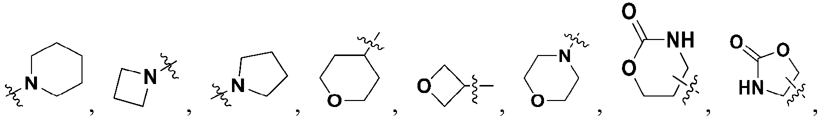

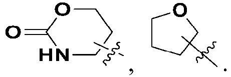

Unless otherwise specified, the term "heterocycloalkylene" refers to a group derived from a heterocycloalkyl group by removal of one hydrogen atom, wherein the heterocycloalkyl is as defined above. Examples of heterocycloalkylene include, but are not limited to,

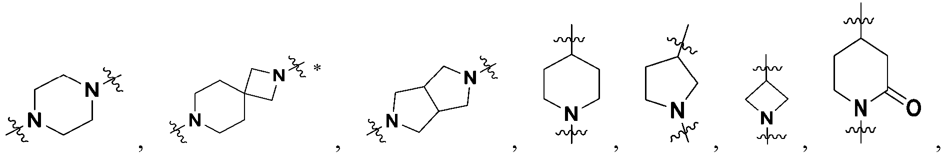

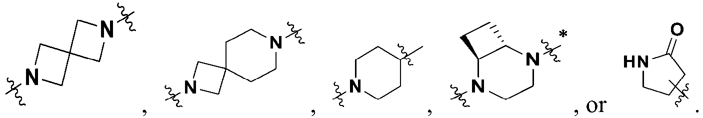

Unless otherwise specified, "cycloalkenyl" refers to a "cycloalkyl" in which one or more ring-forming bonds are double bonds and the cycloalkenyl is non-aromatic. The carbon atoms in the cycloalkenyl may be further substituted with oxo, i.e., forming C(O). The cycloalkenyl may be fused to aryl, heteroaryl, or heterocycloalkenyl, wherein the ring attached to the parent structure is cycloalkenyl. The cycloalkenyl includes "3- to 12-membered cycloalkenyl", "3- to 8-membered cycloalkenyl", "3- to 6-membered cycloalkenyl", "3- to 5-membered cycloalkenyl", and "5- to 6-membered cycloalkenyl". The "cycloalkyl" is as defined above.

Unless otherwise specified, the term "heterocycloalkenyl" refers to a "heterocycloalkyl" in which one or more ring-forming bonds are double bonds and which is non-aromatic. The "heterocycloalkyl" is as described above.

The heterocycloalkenyl may be fused to aryl or heteroaryl, wherein the ring attached to the parent structure is heterocycloalkenyl, etc. Preferably, the heteroatoms are independently selected from 1 to 3 N and/or O. The heterocycloalkenyl includes "3- to 12-membered heterocycloalkenyl", "3- to 8-membered heterocycloalkenyl", "3- to 6-membered heterocycloalkenyl", "3- to 5-membered heterocycloalkenyl", and "5- to 6-membered heterocycloalkenyl". Specific examples include, but are not limited to: 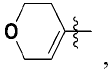 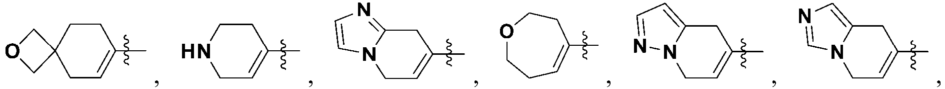 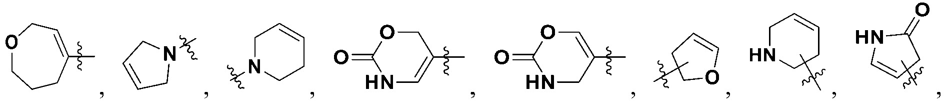 etc.

Unless otherwise specified, the term "aryl" refers to an unsaturated, usually aromatic, hydrocarbon group, which may be a single ring or multiple rings fused together. Fused-ring aryl refers to a group formed by two or more cyclic structures sharing two adjacent atoms with each other, wherein the ring directly attached to the parent structure is aromatic (the ring not directly attached to the parent structure is an aromatic ring or a non-aromatic ring). The aryl may be fused to heterocycloalkenyl or cycloalkenyl, wherein the ring connected to the parent structure is aryl, etc. Preferably C₆₋₁₄ aryl, preferably C₆₋₁₀ aryl; examples of aryl include, but are not limited to, phenyl, naphthyl,

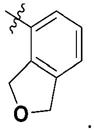

The term "arylene" refers to a group derived from an aryl group by removal of one hydrogen atom, wherein the aryl is as defined above.

The "heteroaryl" in the present disclosure refers to an aromatic monocyclic or polycyclic group containing one or more heteroatoms in the ring, wherein the heteroatoms are generally selected from N, O, and S; preferably, the heteroatoms are independently selected from 1 to 3 N and/or O. Additionally, the N and S atoms may optionally be oxidized, and the N atoms may optionally be quaternized. The "heteroaryl" includes "monocyclic heteroaryl" and "fused heteroaryl". The fused heteroaryl refers to a group formed by two or more cyclic structures sharing two adjacent atoms with each other, containing one or more heteroatoms, wherein the ring directly attached to the parent structure is aromatic (the ring not directly attached the parent structure is an aromatic ring or a non-aromatic ring). The heteroaryl in the present disclosure is preferably "nitrogen-containing heteroaryl" and preferably "5- to 6-membered nitrogen-containing aryl". The heteroatom in the "nitrogen-containing heteroaryl" comprises at least one nitrogen atom, for example, only one, two, or three nitrogen atoms; or one nitrogen atom and one or two other heteroatoms (e.g., S and/or O atoms); or two nitrogen atoms and one or two other heteroatoms. Specific examples of the heteroaryl include, but are not limited to, furanyl, thienyl, pyrrolyl, thiazolyl, isothiazolyl, thiadiazolyl, oxazolyl, isoxazolyl, oxadiazolyl, imidazolyl, pyrazolyl, pyrimidinyl, 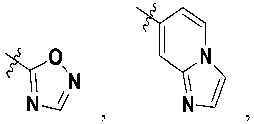 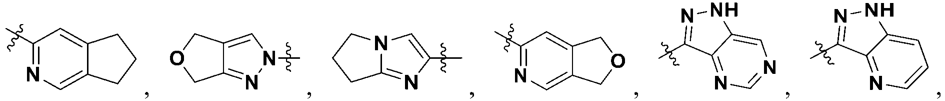 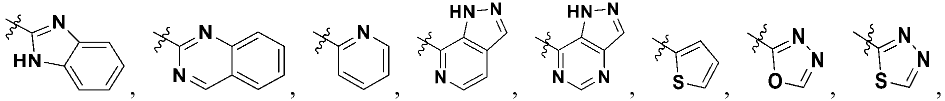 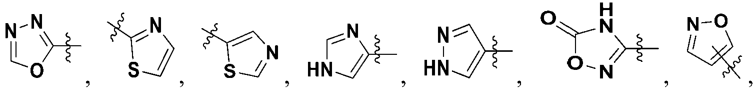 etc.

The term "heteroarylene" refers to a group derived from a heteroaryl group by removal of one hydrogen atom, wherein the heteroaryl is as defined above.

In various sections of the present disclosure, linking substituents are described. When the structure clearly requires a linking group, the Markush variables listed for that group should be understood as linking groups. For example, if the structure requires a linking group and the Markush group definition for that variable lists "alkyl" or "aryl", it should be understood that the "alkyl" or "aryl" represents a connecting alkylene group or arylene group, respectively.

Combinations of substituents and/or variables in the present disclosure are permissible only when such combinations yield stable compounds or usable synthetic intermediates. A stable compound or stable structure refers to a compound that is sufficiently stable to undergo chemical reactions, be isolated in a useful purity, and be formulated into an efficacious therapeutic agent.

In the examples of the present disclosure, the nomenclature of the title compound is converted from the compound structure by means of Chemdraw. If there is any inconsistency between the compound name and the compound structure, it may be determined by synthesizing relevant information and reaction routes; if it cannot be confirmed by other methods, the given structural formula of the compound shall prevail.

The preparation methods of some compounds in the present disclosure refer to the preparation methods of the aforementioned similar compounds. Those skilled in the art should know that when using or referring to the preparation methods cited therein, the feed ratio of reactants, reaction solvent, reaction temperature, etc. may be appropriately adjusted according to the different reactants.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, and preferred embodiments include, but are not limited to, the examples of the present disclosure.

### DETAILED DESCRIPTION OF THE PREFERRED EMBODIMENT

The compound structure of the present disclosure was determined by nuclear magnetic resonance (NMR) mass spectrometry. NMR chemical shift (δ) was given in units of parts per million (ppm). NMR was determined using a Bruker Ascend 400 NMR instrument with deuterated dimethyl sulfoxide (DMSO-*d*₆), deuterated methanol (CD₃OD), deuterated chloroform (CDCl₃), and deuterium oxide (D₂O) as solvents and tetramethylsilane (TMS) as an internal standard.

Thin-layer chromatography (TLC) silica gel plates were HSGF254 silica gel plates from Yantai Jiangyou Silica Gel Development Co., Ltd. The specification of TLC was 0.20 mm ± 0.03 mm, and preparative plate was 20 × 20 cm. For column chromatography, 200-300 mesh silica gel from Qingdao Hailang Silicone Desiccant Co., Ltd. was used as the carrier.

The starting materials in the examples of the present disclosure are known and commercially available, or can be synthesized by using or following methods known in the art.

Unless otherwise specified, all reactions in the present disclosure are carried out under continuous magnetic stirring and dry nitrogen or argon atmosphere. The solvent is a dry solvent, and the unit of reaction temperature is Celsius.

### General Procedures for Preparation

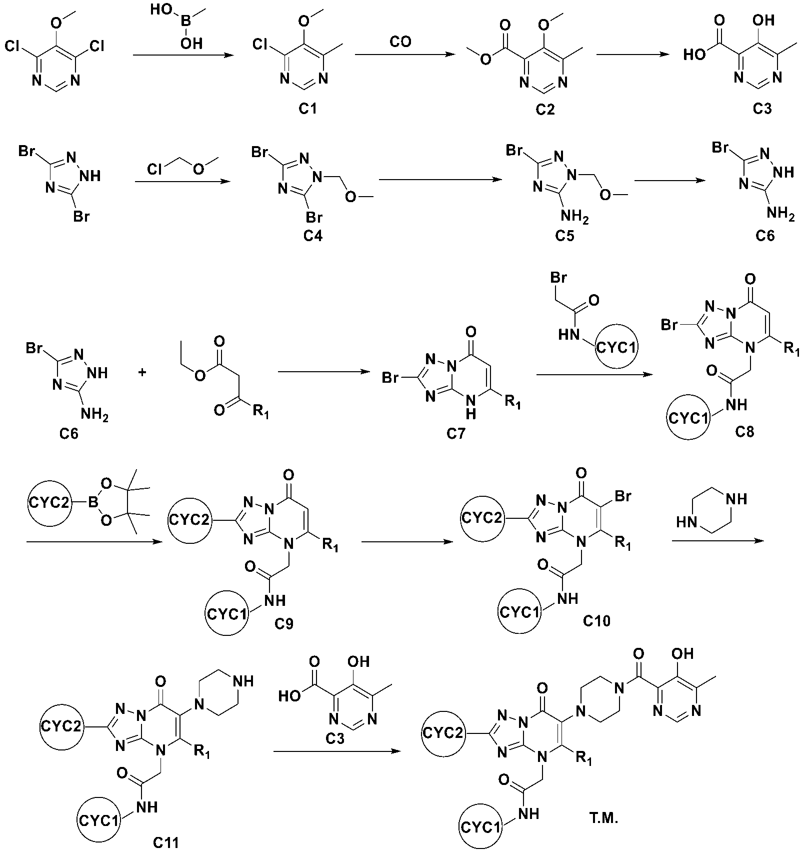

The following examples were synthesized according to the general procedures for preparation.

| Example No. | Structure | Example No. | |
|---|---|---|---|
| 1 | 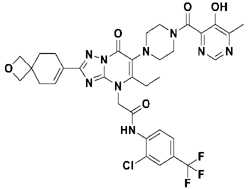 | 173-1 | 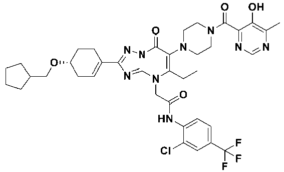 |
| 2 | 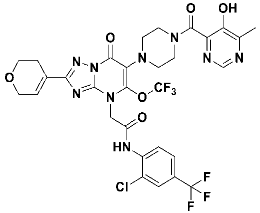 | 173-2 | 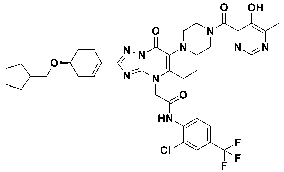 |
| 3 | 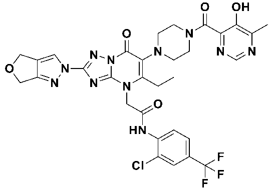 | 174 | 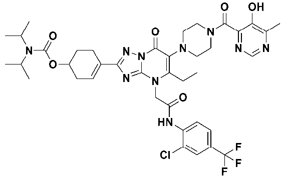 |
| 4 | 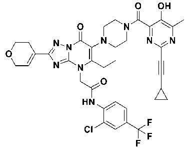 | 174-1 | 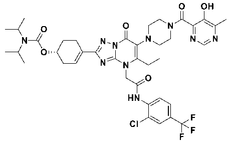 |
| 5 | 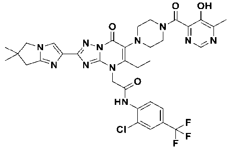 | 174-2 | 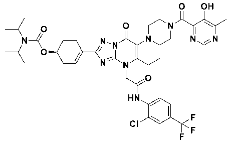 |
| 6 | 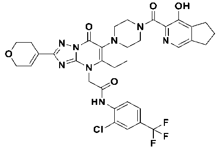 | 175 | 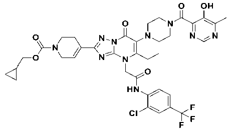 |
| 7 | 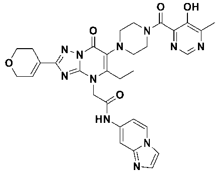 | 176 | 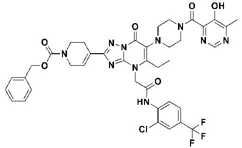 |
| 8 | 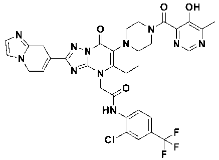 | 177 | 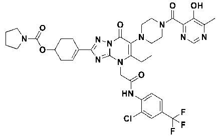 |
| 9 | 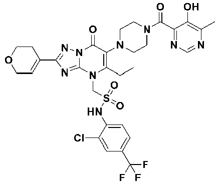 | 177-1 | 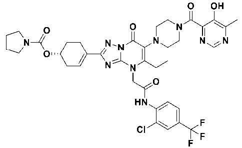 |
| 10 | 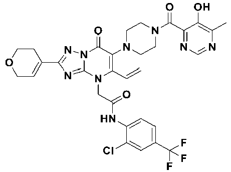 | 177-2 | 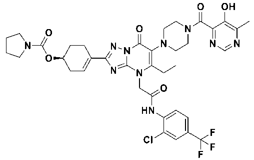 |
| 11 | 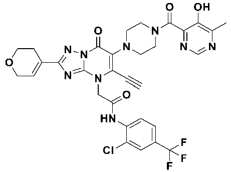 | 178 | 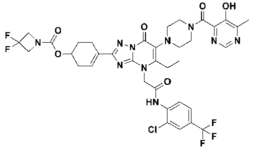 |
| 12 | 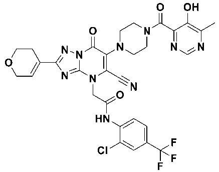 | 178-1 | 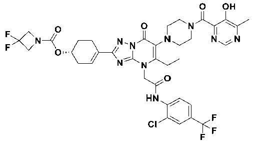 |
| 13 | 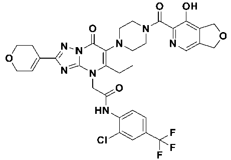 | 178-2 | 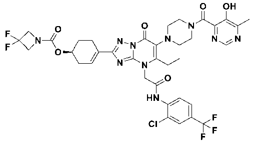 |
| 14 | 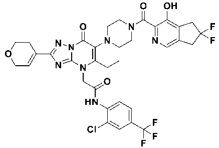 | 179 | 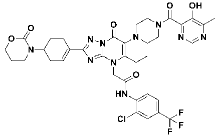 |
| 15 | 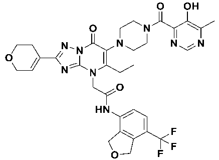 | 179-1 | 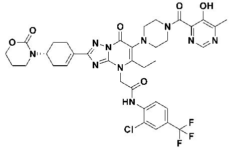 |
| 16 | 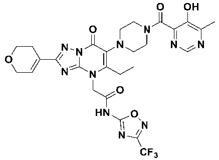 | 179-2 | 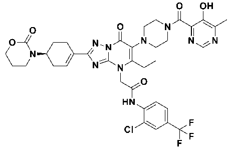 |
| 17 | 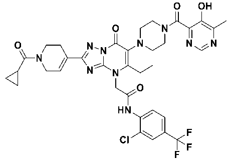 | 180 | 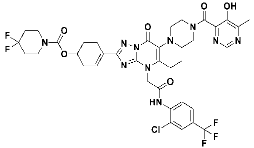 |
| 18 | 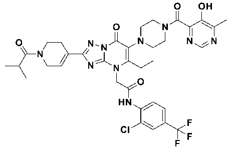 | 180-1 | 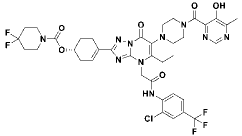 |
| 19 | 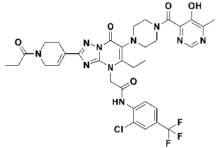 | 180-2 | 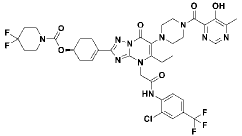 |
| 20 | 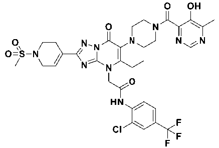 | 181 | 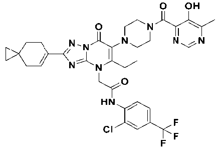 |
| 21 | 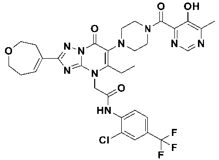 | 182 | 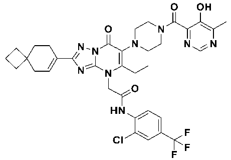 |
| 22 | 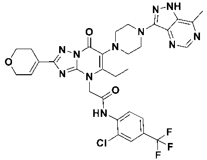 | 183 | 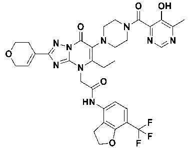 |
| 23 | 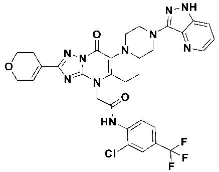 | 184 | 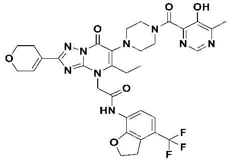 |
| 24 | 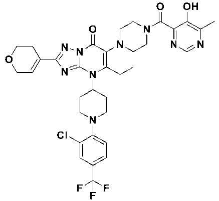 | 185 | 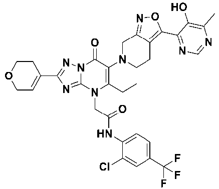 |
| 25 | 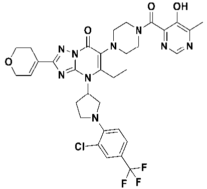 | 186 | 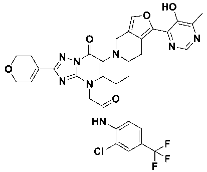 |
| 25-1 | 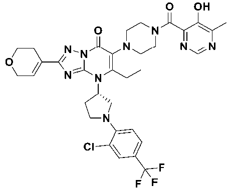 | 187 | 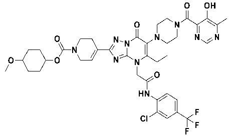 |
| 25-2 | 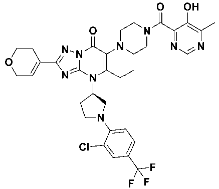 | 188 | 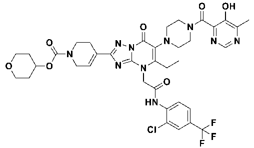 |
| 26 | 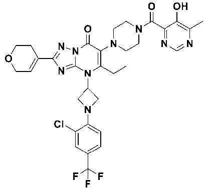 | 189 | 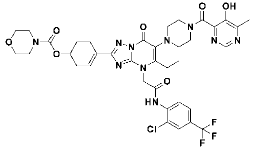 |
| 27 | 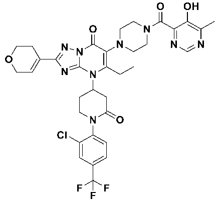 | 189-1 | 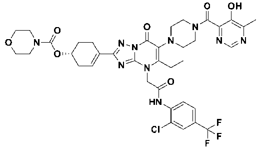 |
| 27-1 | 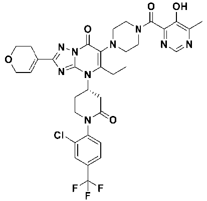 | 189-2 | 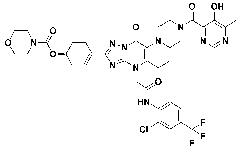 |
| 27-2 | 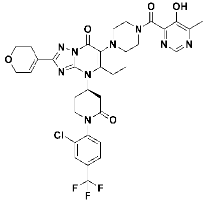 | 190 | 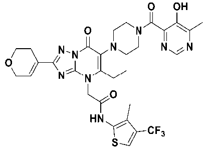 |
| 28 | 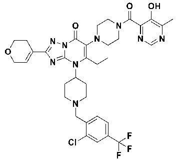 | 191 | 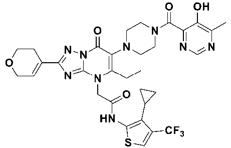 |
| 29 | 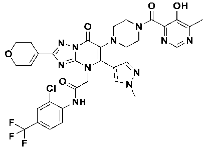 | 192 | 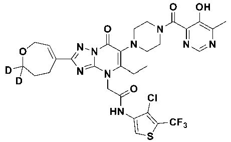 |
| 30 | 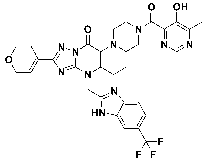 | 193 | 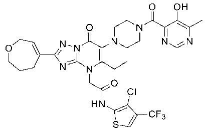 |
| 31 | 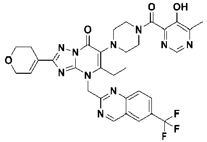 | 194 | 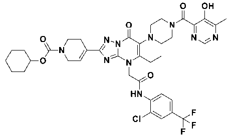 |
| 32 | 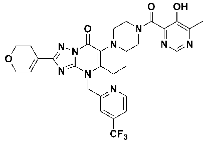 | 195 | 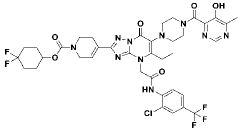 |
| 33 | 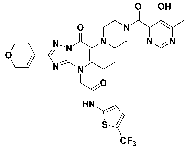 | 196 | 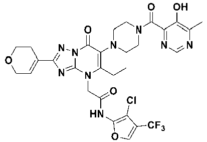 |
| 34 | 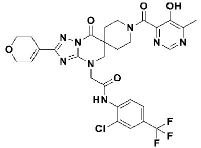 | 197 | 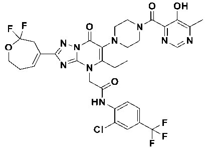 |
| 35 | 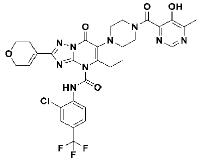 | 198 | 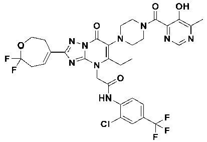 |
| 36 | 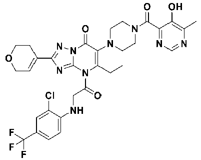 | 199 | 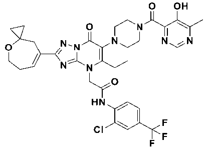 |
| 37 | 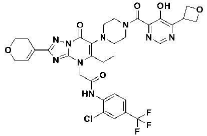 | 200 | 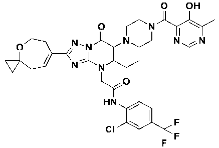 |
| 38 | 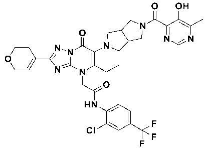 | 201 | 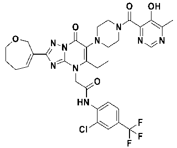 |
| 39 | 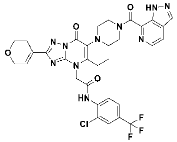 | 202 | 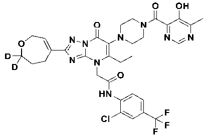 |
| 40 | 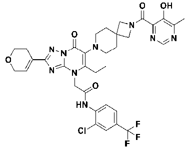 | 203 | 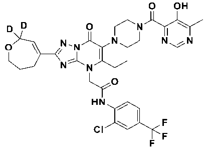 |
| 41 | 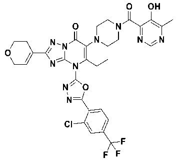 | 204 | 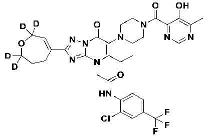 |
| 42 | 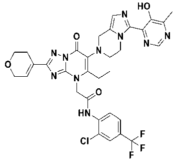 | 205 | 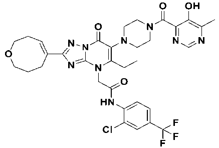 |
| 43 | 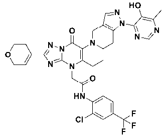 | 206 | 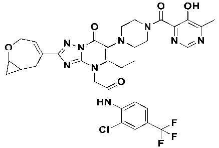 |
| 44 | 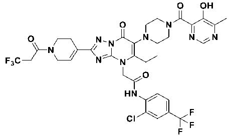 | 206-1 | 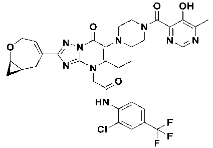 |
| 45 | 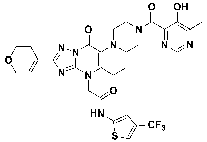 | 206-2 | 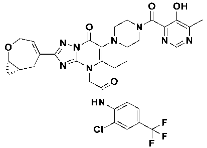 |
| 46 | 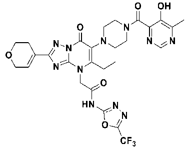 | 207 | 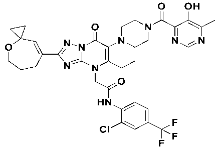 |
| 47 | 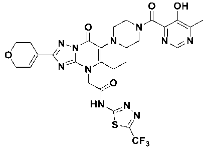 | 208 | 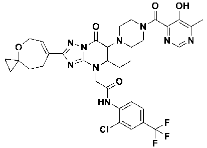 |
| 48 | 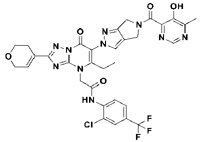 | 209 | 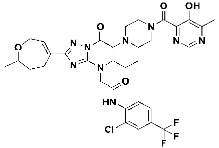 |
| 49 | 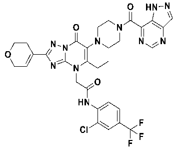 | 209-1 | 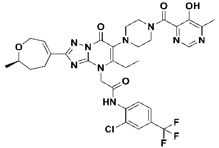 |
| 50 | 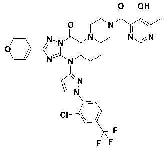 | 209-2 | 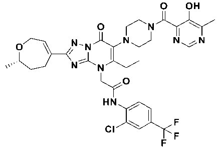 |
| 51 | 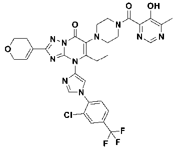 | 210 | 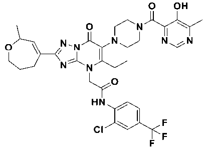 |
| 52 | 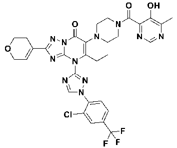 | 210-1 | 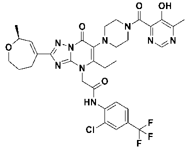 |
| 53 | 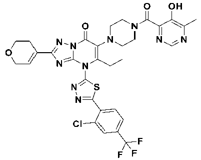 | 210-2 | 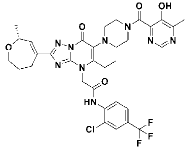 |
| 54 | 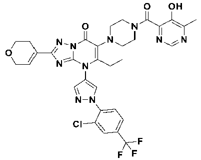 | 211 | 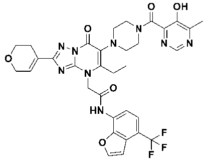 |
| 55-1 | 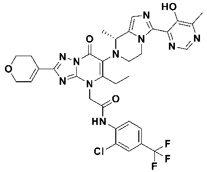 | 212 | 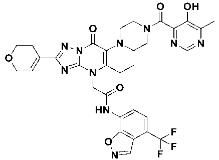 |
| 55-2 | 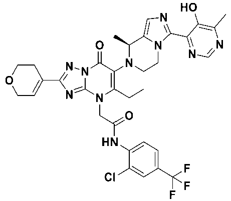 | 213 | 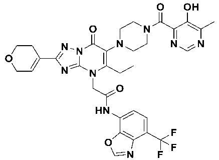 |
| 55 | 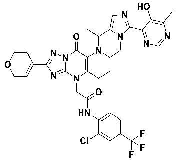 | 214 | 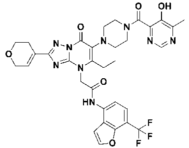 |
| 56 | 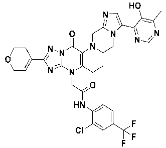 | 215 | 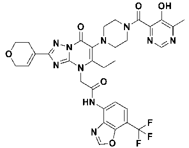 |
| 57 | 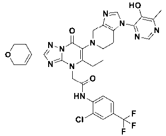 | 216 | 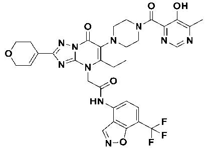 |
| 58 | 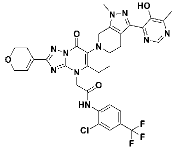 | 217 | 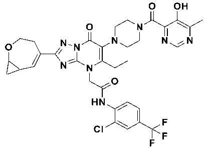 |
| 59-1 | 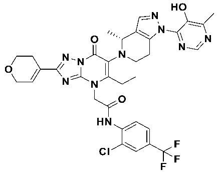 | 217-1 | 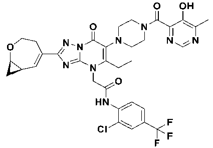 |
| 59-2 | 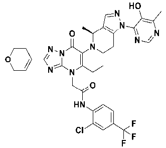 | 217-2 | 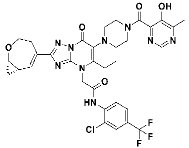 |
| 59 | 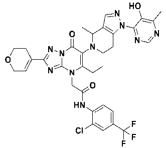 | 218 | 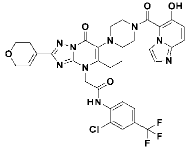 |
| 60 | 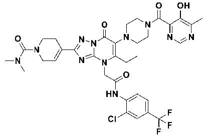 | 219 | 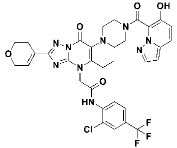 |
| 61 | 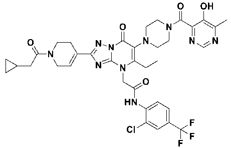 | 220 | 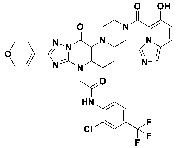 |
| 62 | 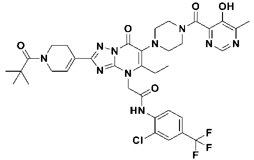 | 221 | 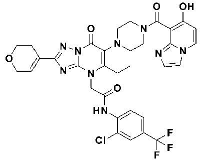 |
| 63 | 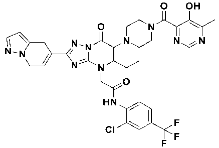 | 222 | 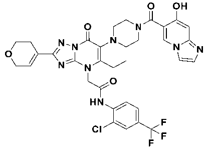 |
| 64 | 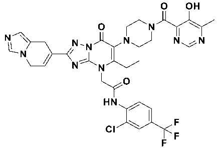 | 223 | 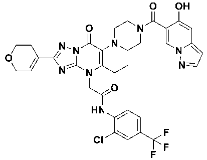 |
| 65 | 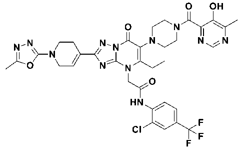 | 224 | 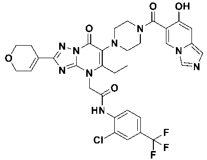 |
| 66 | 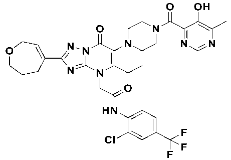 | 225 | 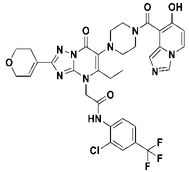 |
| 67 | 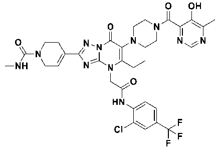 | 226 | 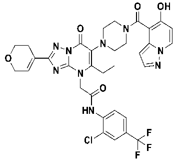 |
| 68 | 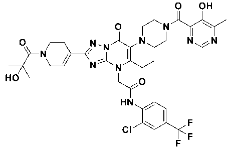 | 227 | 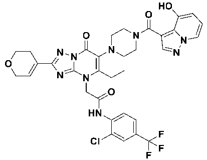 |
| 69 | 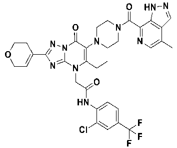 | 228 | 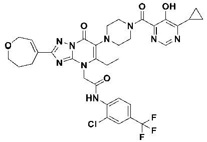 |
| 70-1 | 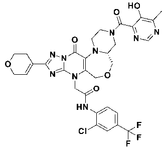 | 229 | 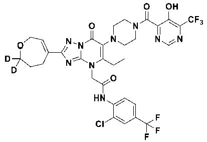 |
| 70-2 | 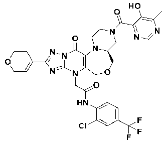 | 230 | 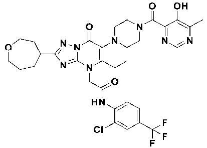 |
| 70 | 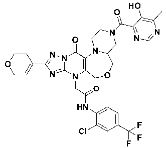 | 230-1 | 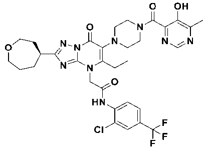 |
| 71 | 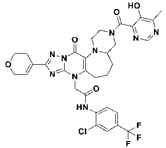 | 230-2 | 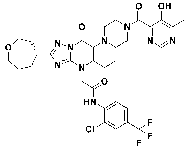 |
| 71-1 | 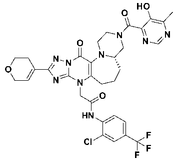 | 231 | 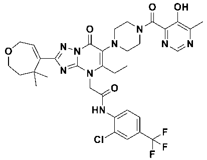 |
| 71-2 | 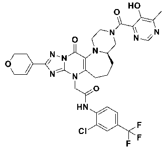 | 232 | 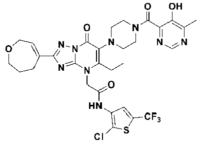 |
| 72 | 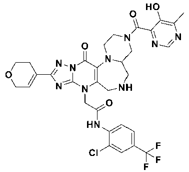 | 233 | 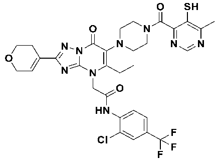 |
| 72-1 | 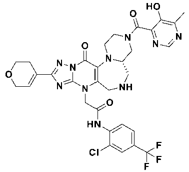 | 234 | 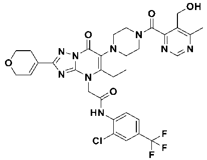 |
| 72-2 | 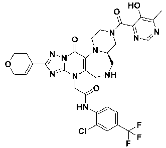 | 235 | 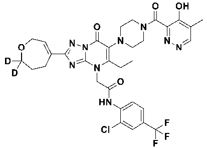 |
| 73 | 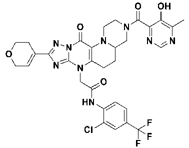 | 236 | 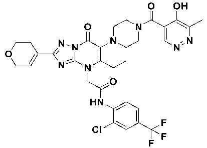 |
| 73-1 | 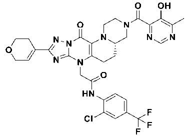 | 237 | 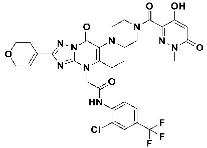 |
| 73-2 | 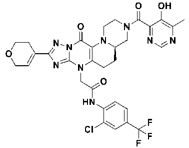 | 238 | 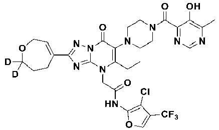 |
| 74 | 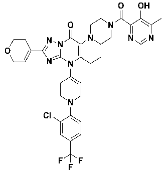 | 239 | 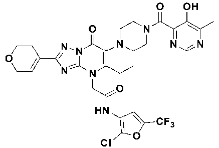 |
| 75 | 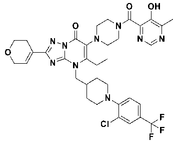 | 240 | 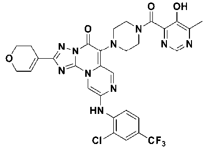 |
| 76 | 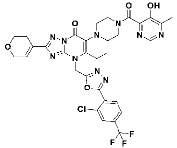 | 241 | 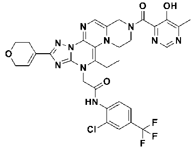 |
| 77 | 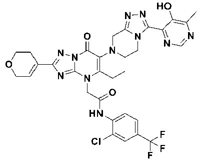 | 242 | 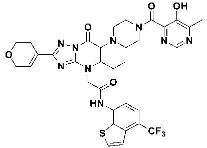 |
| 78 | 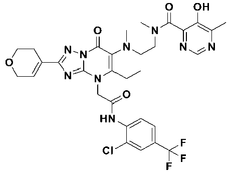 | 243 | 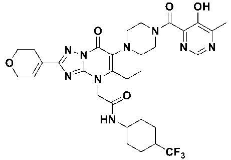 |
| 79 | 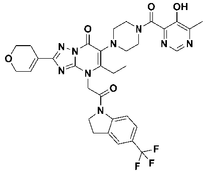 | 244 | 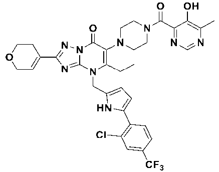 |
| 80 | 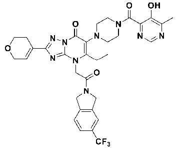 | 245 | 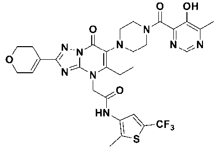 |
| 81 | 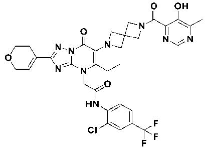 | 246 | 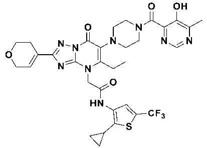 |
| 82 | 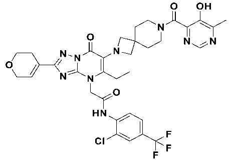 | 247 | 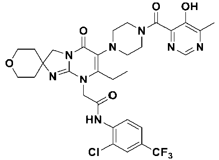 |
| 83 | 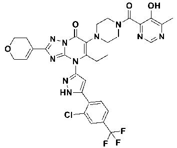 | 248 | 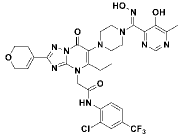 |
| 84 | 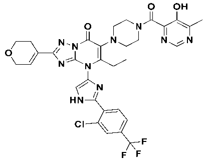 | 249 | 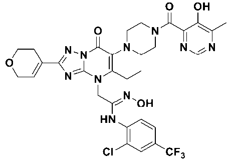 |
| 85 | 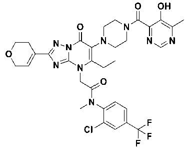 | 250 | 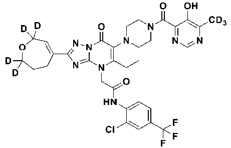 |
| 86 | 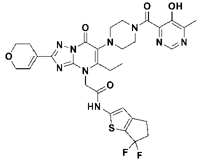 | 251 | 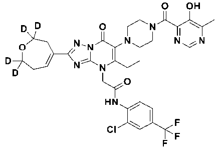 |
| 87 | 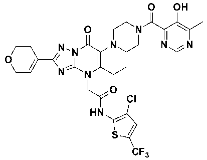 | 252 | 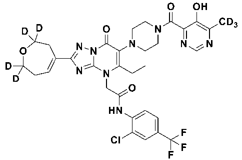 |
| 88 | 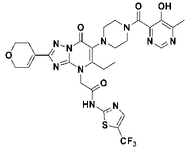 | 253 | 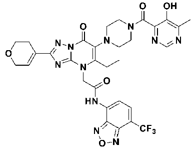 |
| 89 | 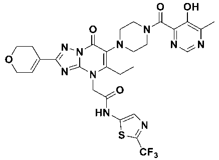 | 254 | 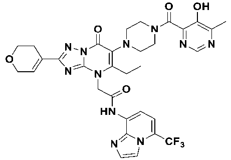 |
| 90 | 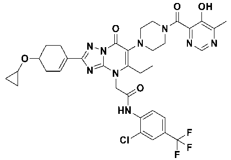 | 255 | 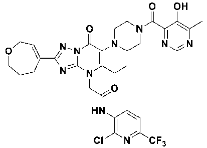 |
| 90-1 | 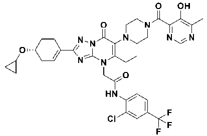 | 256 | 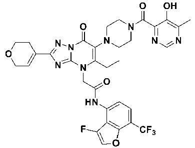 |
| 90-2 | 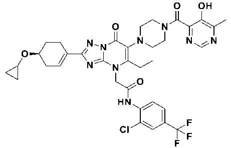 | 257 | 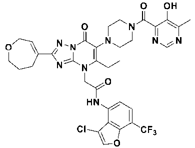 |
| 91 | 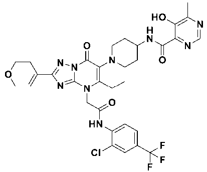 | 258 | 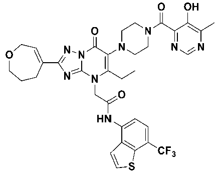 |
| 92 | 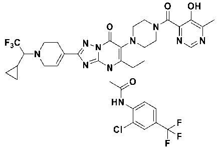 | 259 | 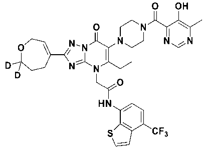 |
| 93 | 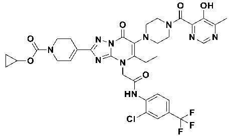 | 260 | 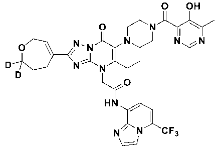 |
| 94 | 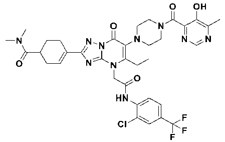 | 261 | 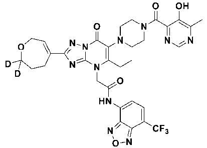 |
| 94 in a single configuration | 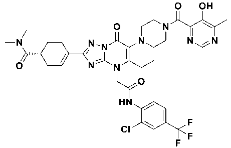 | 262 | 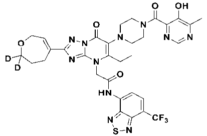 |
| 94 in a single configuration | 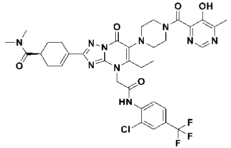 | 263 | 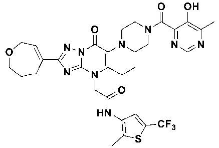 |
| 95 | 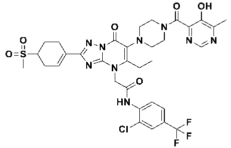 | 264 | 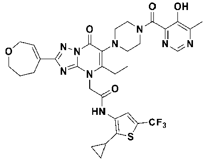 |
| 95-1 | 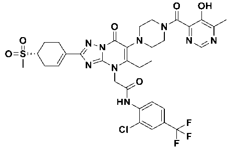 | 265 | 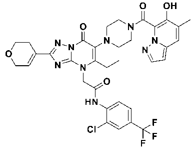 |
| 95-2 | 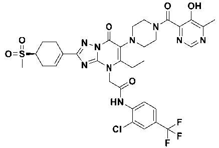 | 266 | 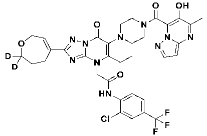 |
| 96 | 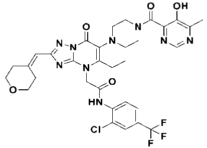 | 267 | 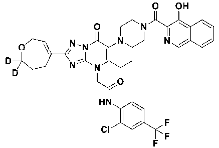 |
| 97 | 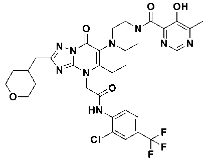 | 268 | 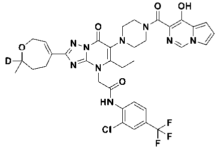 |
| 98 | 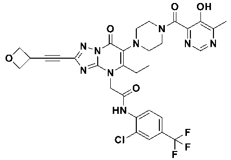 | 269 | 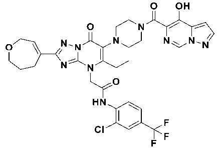 |
| 99 | 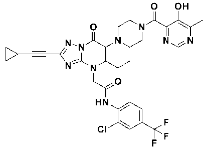 | 270 | 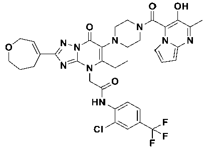 |
| 100 | 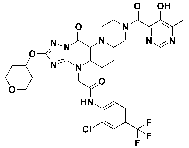 | 271 | 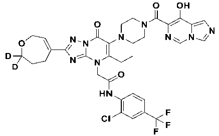 |
| 101 | 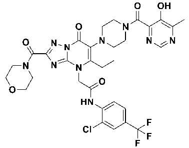 | 272 | 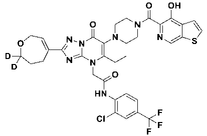 |
| 102 | 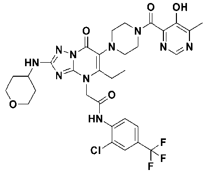 | 273 | 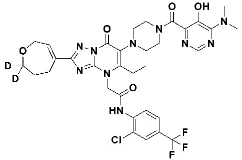 |
| 103 | 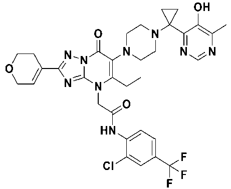 | 274 | 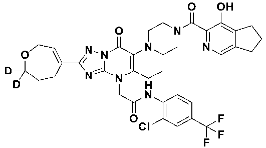 |
| 104 | 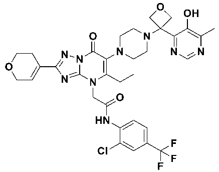 | 275 | 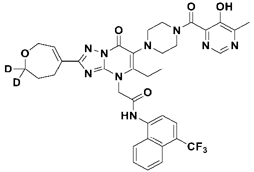 |
| 105 | 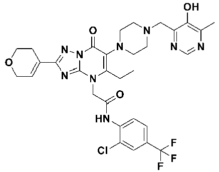 | 276 | 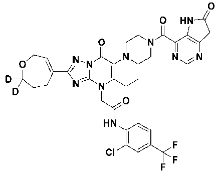 |
| 106 | 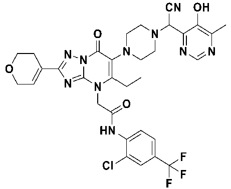 | 277 | 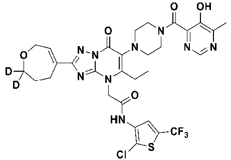 |
| 106-1 | 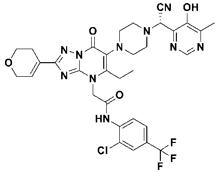 | 278 | 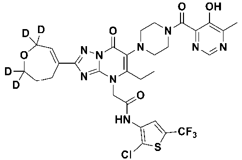 |
| 106-2 | 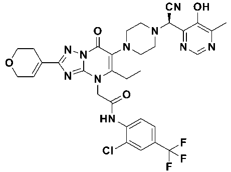 | 279 | 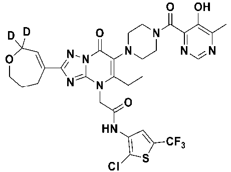 |
| 107 | 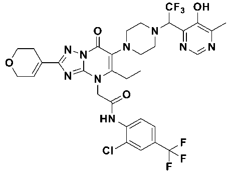 | 280 | 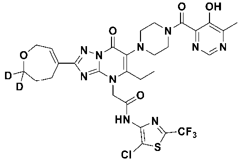 |
| 107-1 | 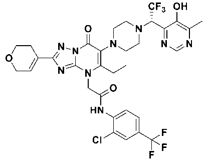 | 281 | 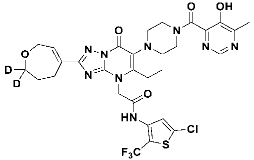 |
| 107-2 | 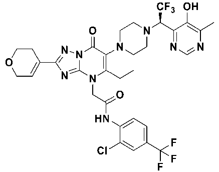 | 282 | 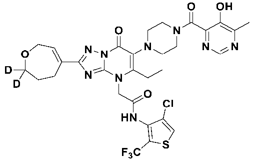 |
| 108 | 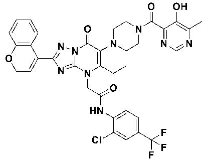 | 283 | 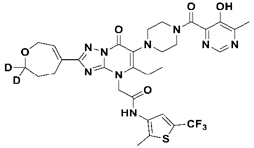 |
| 109 | 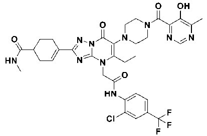 | 284 | 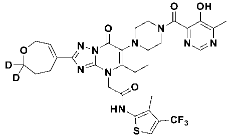 |
| 109-1 | 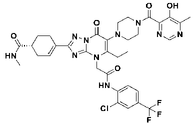 | 285 | 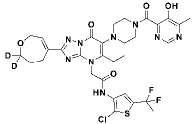 |
| 109-2 | 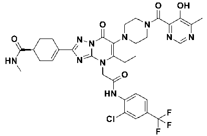 | 286 | 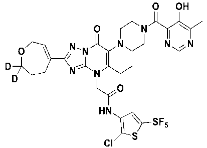 |
| 110 | 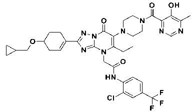 | 287 | 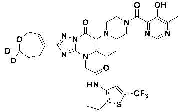 |
| 110-1 | 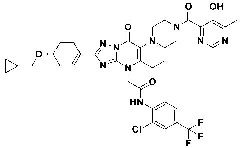 | 288 | 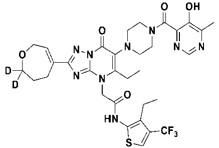 |
| 110-2 | 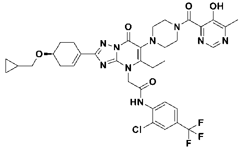 | 289 | 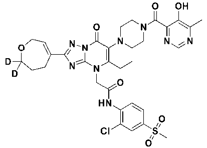 |
| 111 | 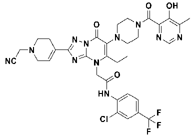 | 290 | 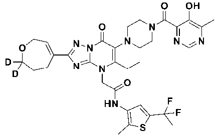 |
| 112 | 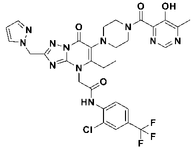 | 291 | 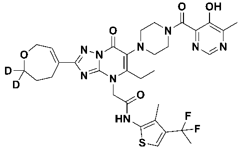 |
| 113 | 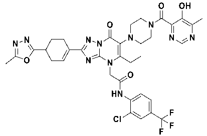 | 292 | 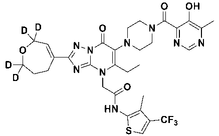 |
| 113-1 | 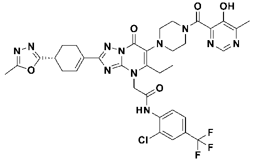 | 293 | 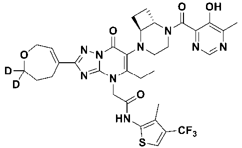 |
| 113-2 | 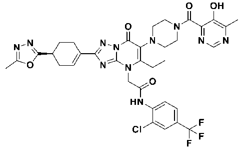 | 294 | 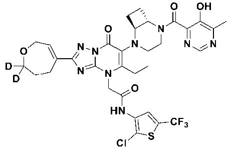 |
| 114 | 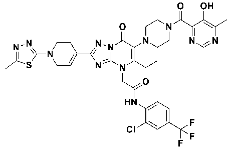 | 295 | 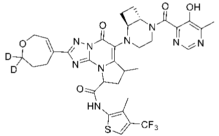 |
| 115 | 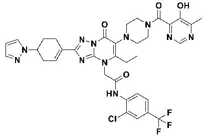 | 295 in a single configuration | 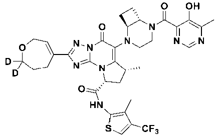 |
| 115-1 | 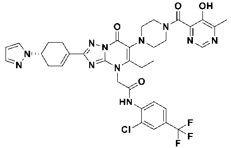 | 295 in a single configuration | 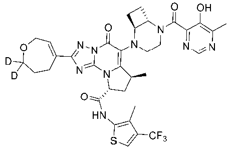 |
| 115-2 | 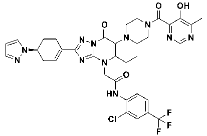 | 295 in a single configuration | 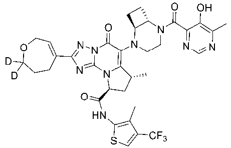 |
| 116 | 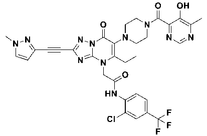 | 295 in a single configuration | 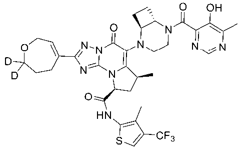 |
| 117 | 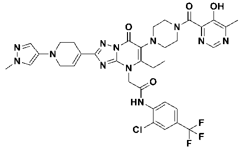 | 296 | 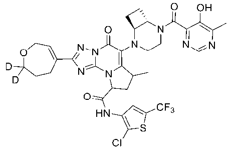 |
| 118 | 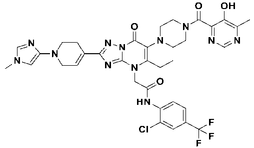 | 296 in a single configuration | 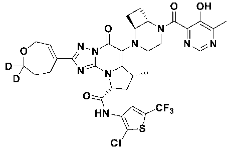 |
| 119 | 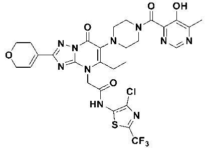 | 296 in a single configuration | 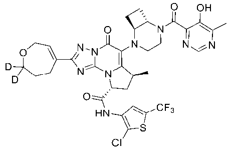 |
| 120 | 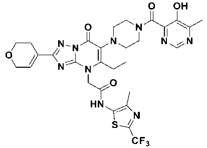 | 296 in a single configuration | 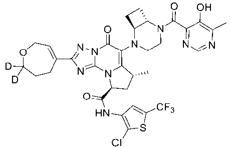 |
| 121 | 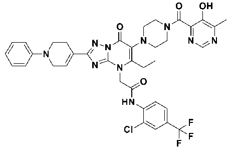 | 296 in a single configuration | 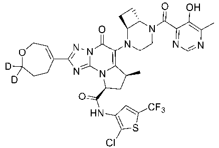 |
| 122 | 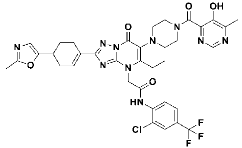 | 297 | 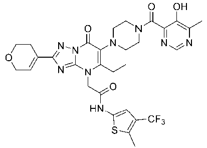 |
| 122-1 | 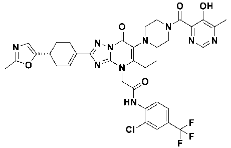 | 298 | 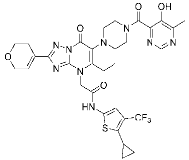 |
| 122-2 | 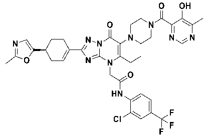 | 299 | 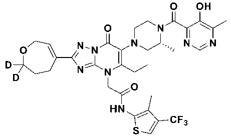 |
| 123 | 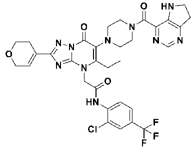 | 300 | 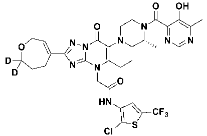 |
| 124 | 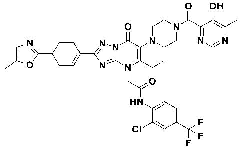 | 301 | 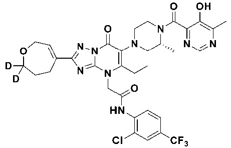 |
| 124-1 | 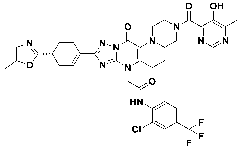 | 302 | 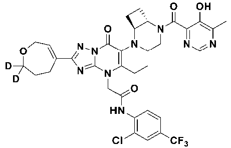 |
| 124-2 | 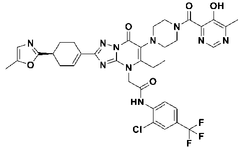 | 303 | 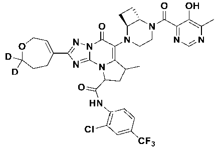 |
| 125 | 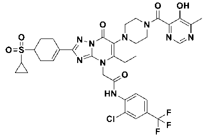 | 303 in a single configuration | 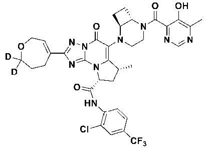 |
| 125-1 | 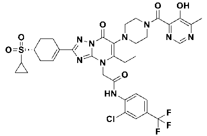 | 303 in a single configuration | 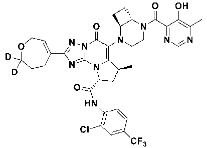 |
| 125-2 | 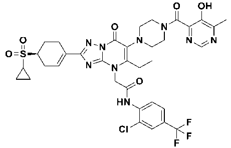 | 303 in a single configuration | 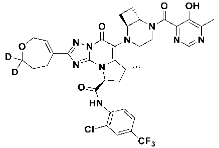 |
| 126 | 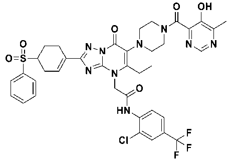 | 303 in a single configuration | 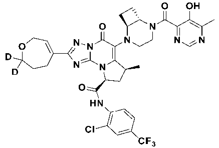 |
| 126-1 | 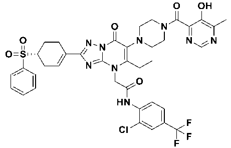 | 304 | 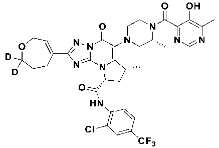 |
| 126-2 | 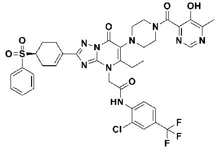 | 305 | 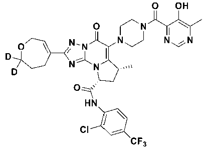 |
| 127 | 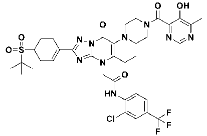 | 306 | 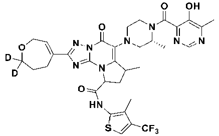 |
| 127-1 | 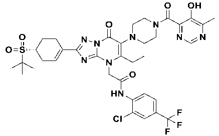 | 306 in a single configuration | 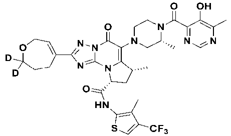 |
| 127-2 | 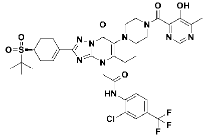 | 306 in a single configuration | 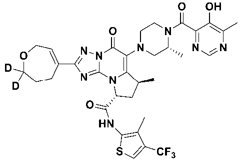 |
| 128 | 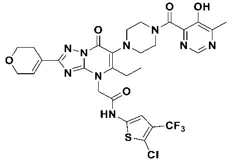 | 306 in a single configuration | 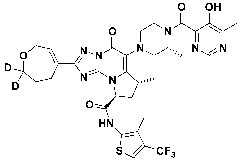 |
| 129 | 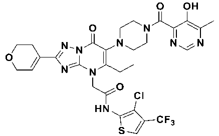 | 306 in a single configuration | 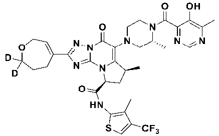 |
| 130 | 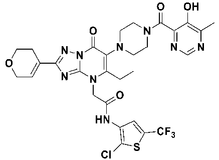 | 307 | 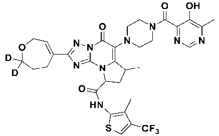 |
| 131 | 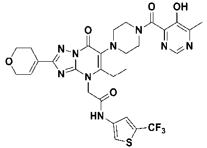 | 307 in a single configuration | 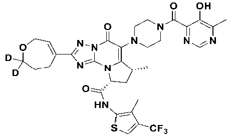 |
| 132 | 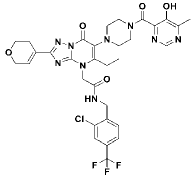 | 307 in a single configuration | 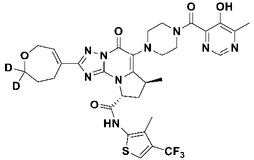 |
| 133 | 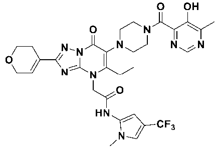 | 307 in a single configuration | 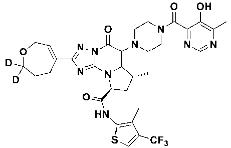 |
| 134 | 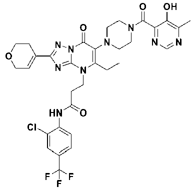 | 307 in a single configuration | 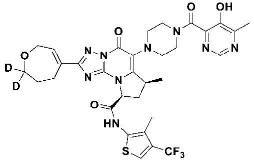 |
| 135 | 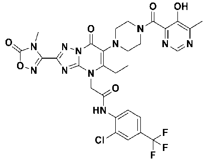 | 308 | 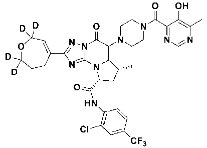 |
| 136 | 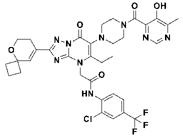 | 309 | 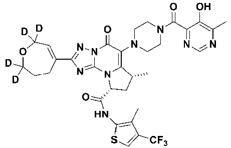 |
| 137 | 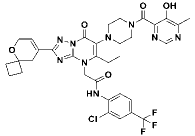 | 310 | 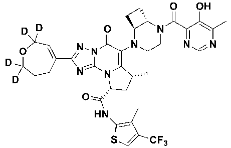 |
| 138 | 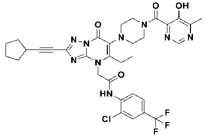 | 311 | 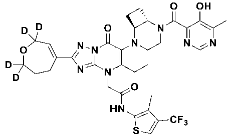 |
| 139 | 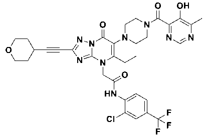 | 312 | 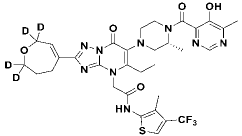 |
| 140 | 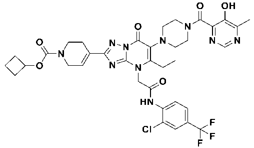 | 313 | 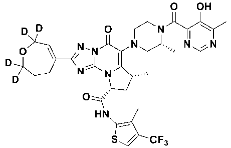 |
| 141 | 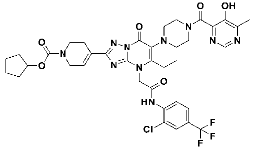 | 314 | 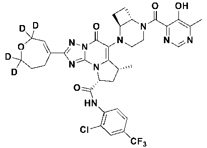 |
| 142 | 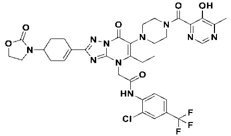 | 315 | 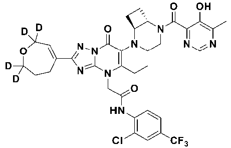 |
| 142-1 | 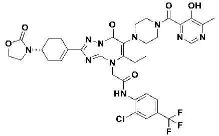 | 316 | 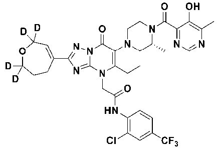 |
| 142-2 | 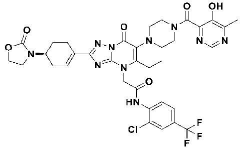 | 317 | 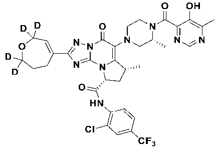 |
| 143 | 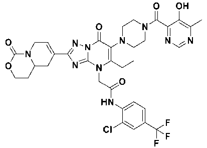 | 318 | 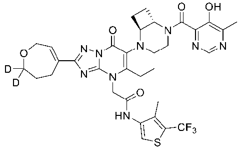 |
| 143-1 | 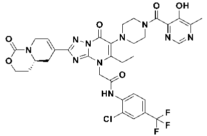 | 319 | 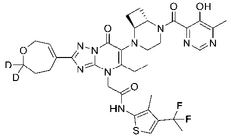 |
| 143-2 | 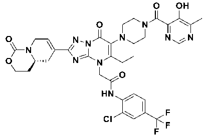 | 320 | 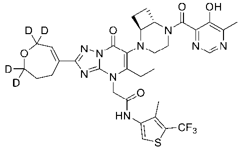 |
| 144 | 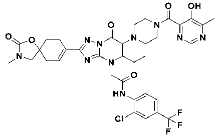 | 321 | 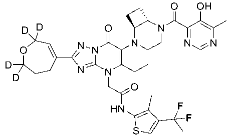 |
| 144-1 | 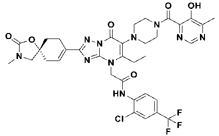 | 322 | 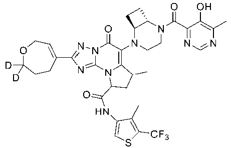 |
| 144-2 | 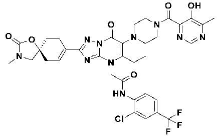 | 322 in a single configuration | 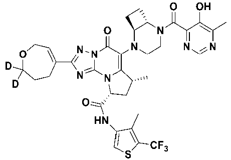 |
| 145 | 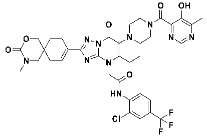 | 322 in a single configuration | 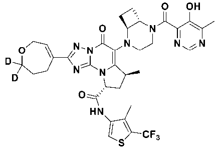 |
| 145-1 | 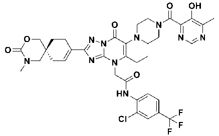 | 322 in a single configuration | 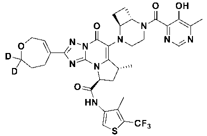 |
| 145-2 | 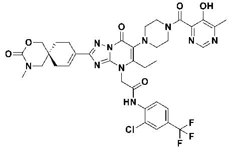 | 322 in a single configuration | 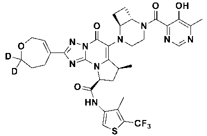 |
| 146 | 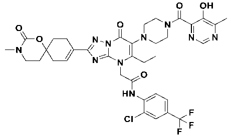 | 323 | 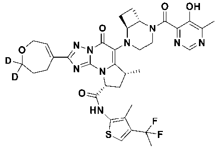 |
| 146-1 | 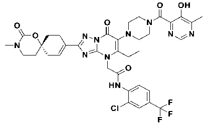 | 324 | 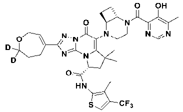 |
| 146-2 | 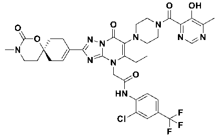 | 325 | 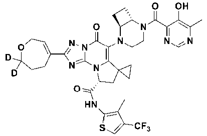 |
| 147 | 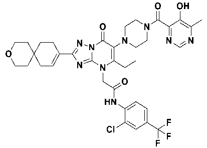 | 326 | 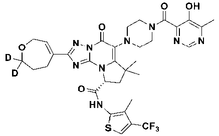 |
| 148 | 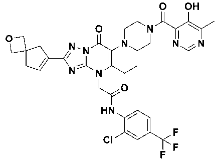 | 327 | 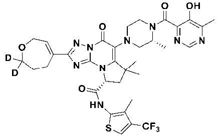 |
| 149 | 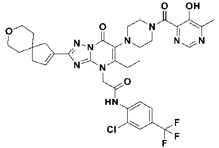 | 328 | 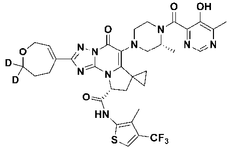 |
| 150 | 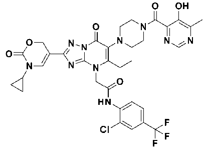 | 329 | 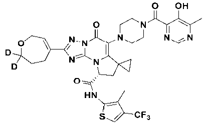 |
| 151 | 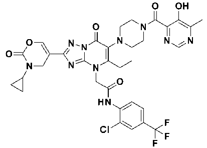 | 330 | 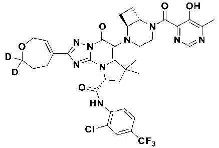 |
| 152 | 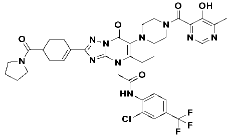 | 331 | 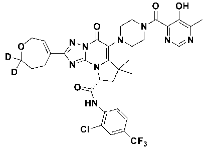 |
| 152 in a single configuration | 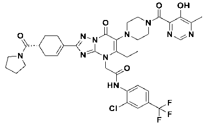 | 332 | 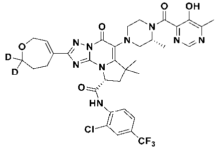 |
| 152 in a single configuration | 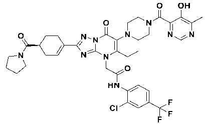 | 333 | 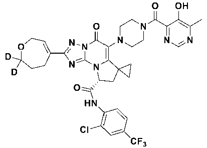 |
| 153 | 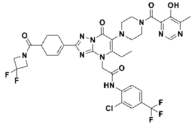 | 334 | 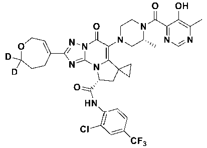 |
| 153-1 | 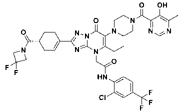 | 335 | 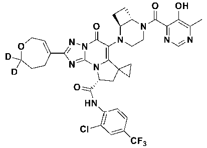 |
| 153-2 | 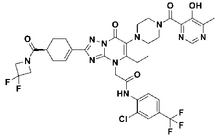 | 336 | 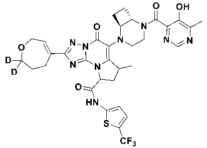 |
| 154 | 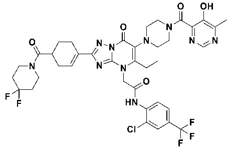 | 337 | 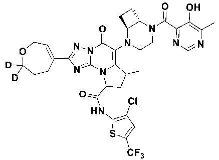 |
| 154-1 | 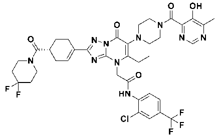 | 338 | 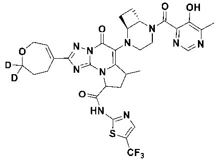 |
| 154-2 | 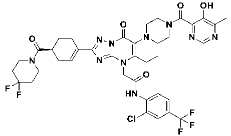 | 339 | 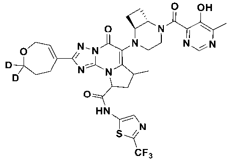 |
| 155 | 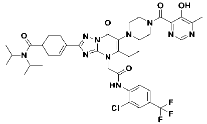 | 340 | 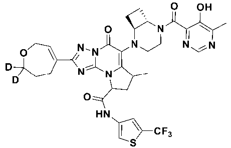 |
| 155-1 | 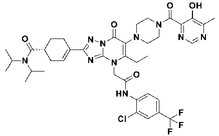 | 341 | 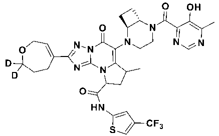 |
| 155-2 | 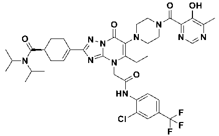 | 342 | 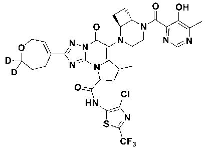 |
| 156 | 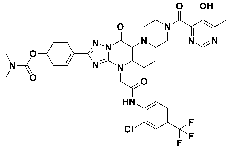 | 343 | 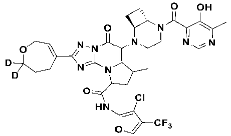 |
| 156-1 | 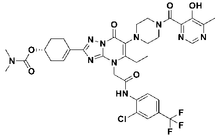 | 344 | 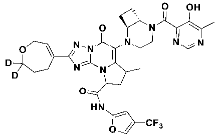 |
| 156-2 | 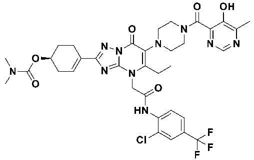 | 345 | 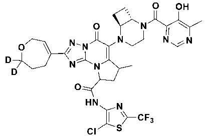 |
| 157 | 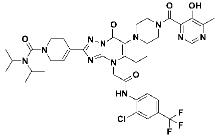 | 346 | 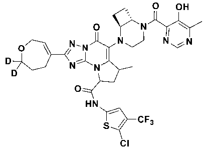 |
| 158 | 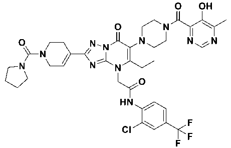 | 347 | 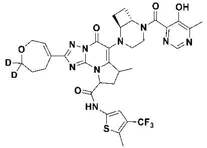 |
| 159 | 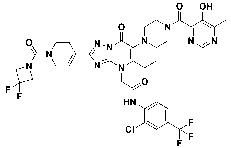 | 348 | 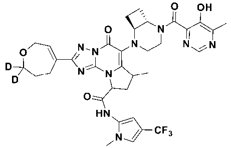 |
| 160 | 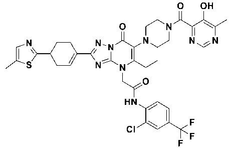 | 349 | 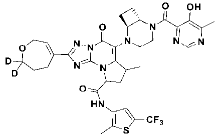 |
| 160-1 | 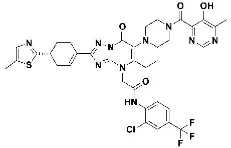 | 350 | 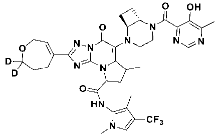 |
| 160-2 | 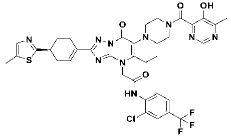 | 351 | 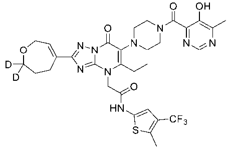 |
| 161 | 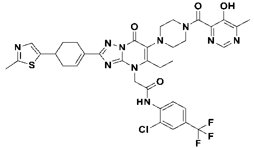 | 352 | 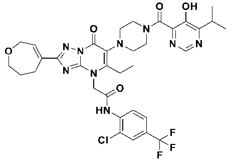 |
| 161-1 | 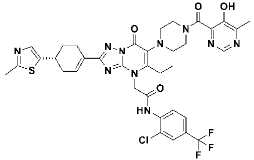 | 353 | 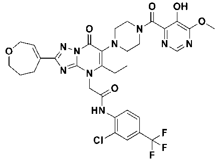 |
| 161-2 | 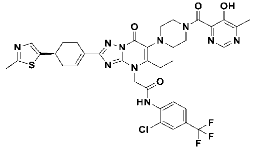 | 354 | 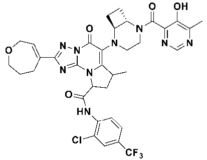 |
| 162 | 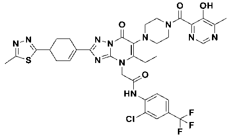 | 354-1 | 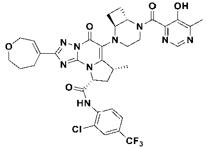 |
| 162-1 | 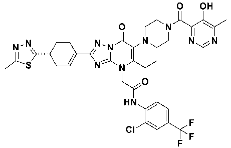 | 355 | 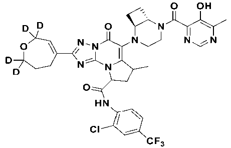 |
| 162-2 | 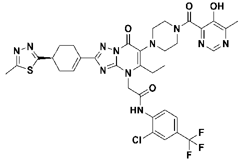 | 355-1 | 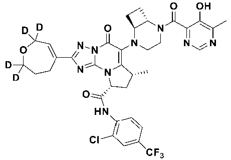 |
| 163 | 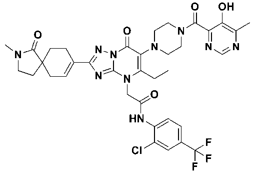 | 356 | 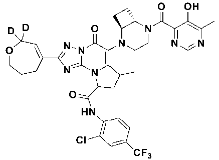 |
| 163-1 | 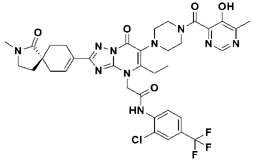 | 356-1 | 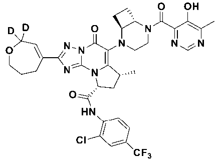 |
| 163-2 | 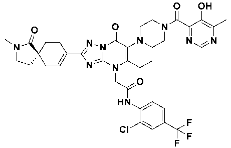 | 357 | 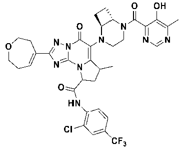 |
| 164 | 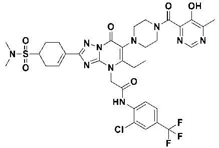 | 357-1 | 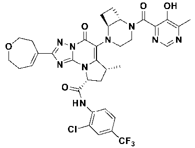 |
| 164-1 | 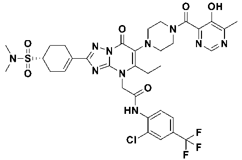 | 358 | 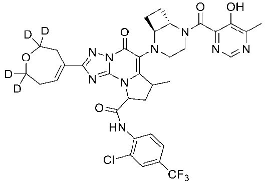 |
| 164-2 | 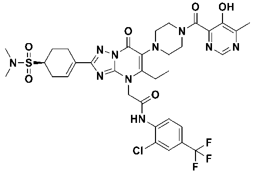 | 358-1 | 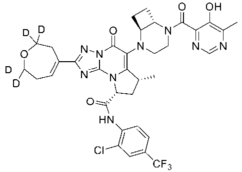 |
| 165 | 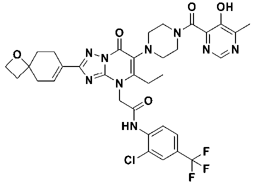 | 359 | 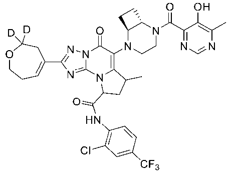 |
| 165-1 | 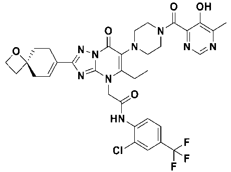 | 359-1 | 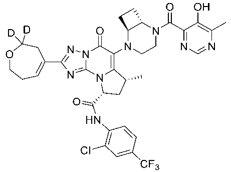 |
| 165-2 | 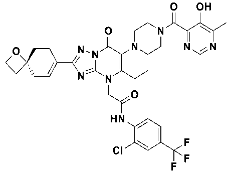 | 360 | 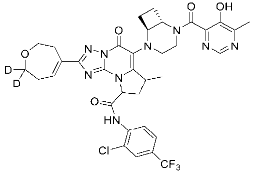 |
| 166 | 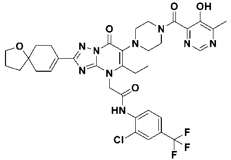 | 360-1 | 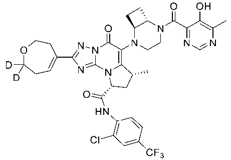 |
| 166-1 | 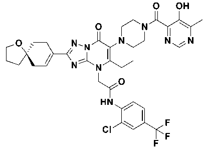 | 361 | 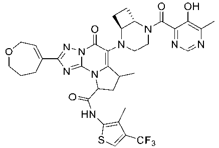 |
| 166-2 | 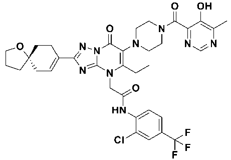 | 361 in a single configuration | 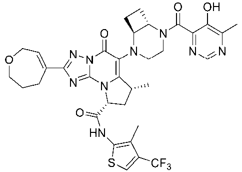 |
| 167 | 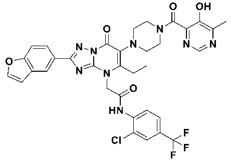 | 362 | 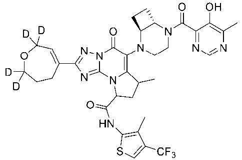 |
| 168 | 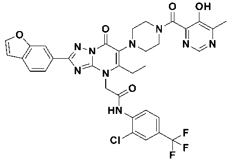 | 362-1 | 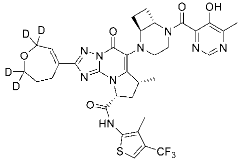 |
| 169 | 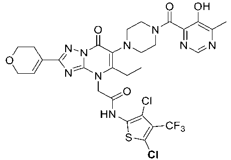 | 363 | 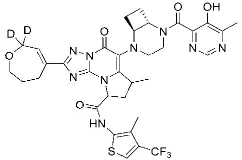 |
| 170 | 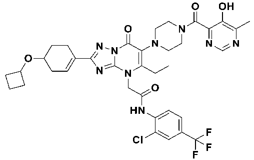 | 363-1 | 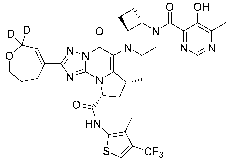 |
| 170-1 | 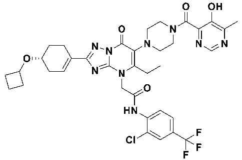 | 364 | 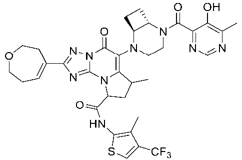 |
| 170-2 | 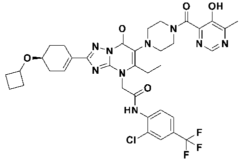 | 364-1 | 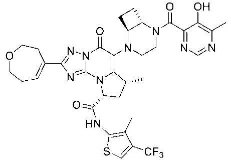 |
| 171 | 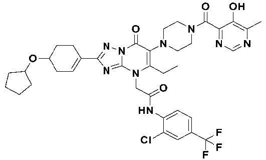 | 365 | 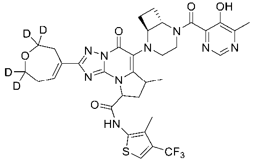 |
| 171-1 | 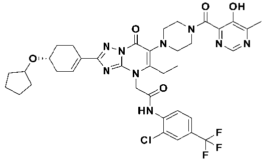 | 365-1 | 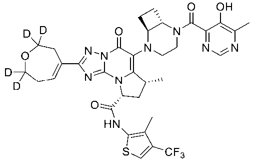 |
| 171-2 | 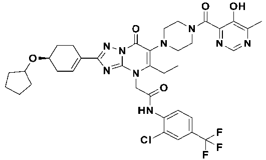 | 366 | 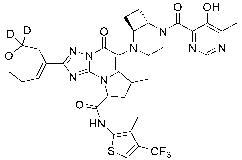 |
| 172 | 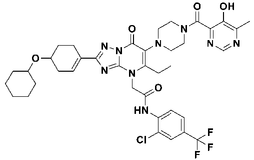 | 366-1 | 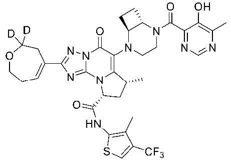 |
| 172-1 | 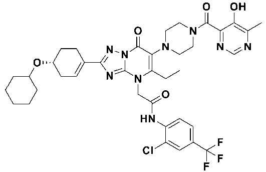 | 367 | 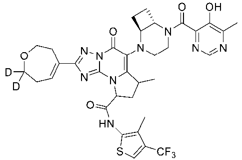 |
| 172-2 | 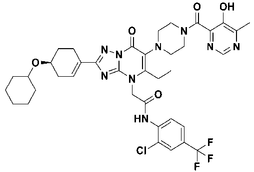 | 367-1 | 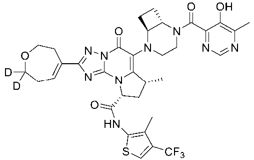 |
| 368 | 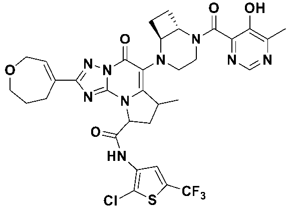 | 368-1 | 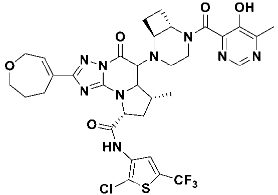 |
| 368-2 | 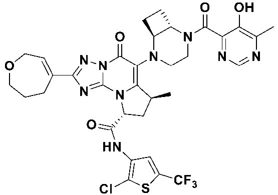 | 368-3 | 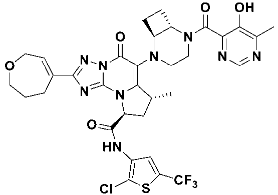 |
| 368-4 | 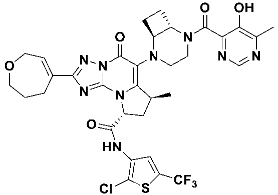 | 369 | 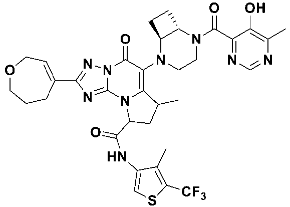 |
| 369-1 | 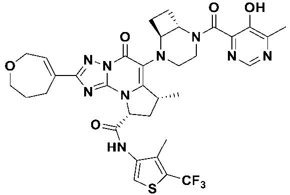 | 369-2 | 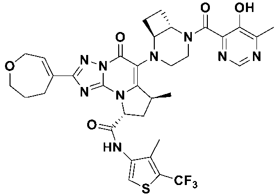 |
| 369-3 | 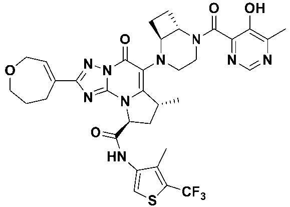 | 369-4 | 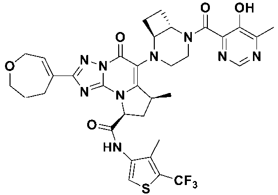 |
| 370 | 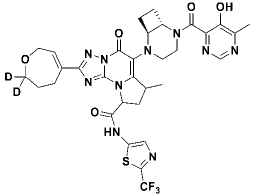 | 370-1 | 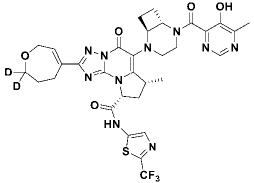 |
| 371 | 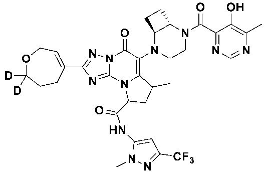 | 371-1 | 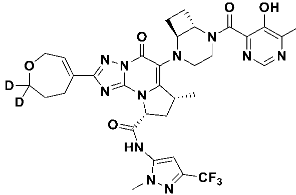 |
| 372 | 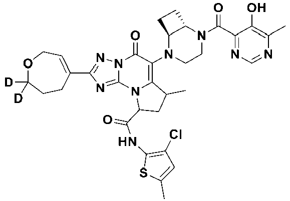 | 372-1 | 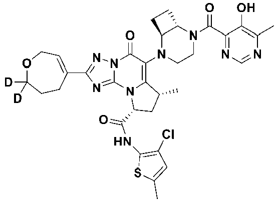 |
| 373 | 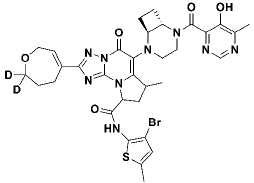 | 373-1 | 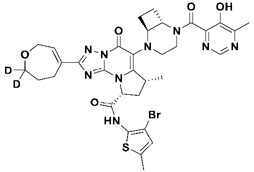 |
| 374 | 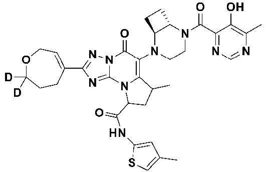 | 374-1 | 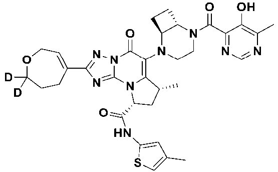 |
| 375 | 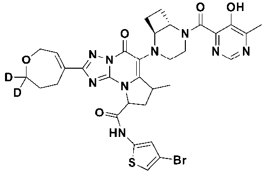 | 375-1 | 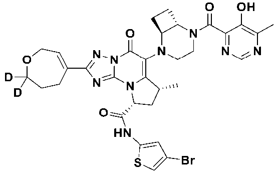 |
| 376 | 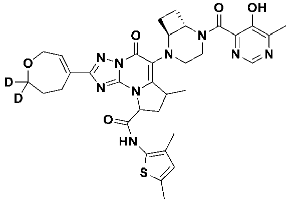 | 376-1 | 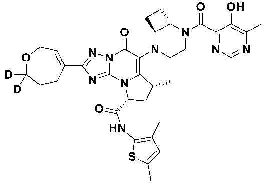 |

### Intermediate 1

### 2-(2-Bromo-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamide

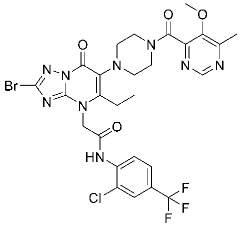

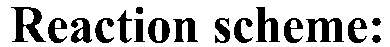

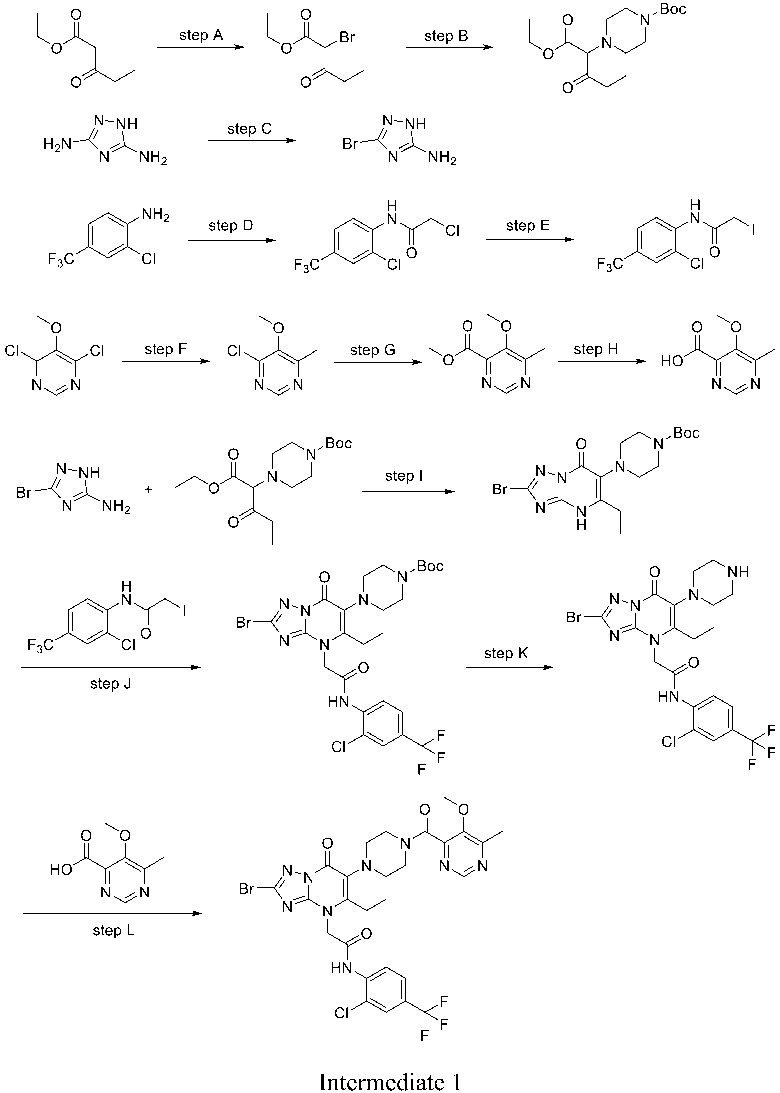

### Procedure:

Step A: Ethyl 3-oxopentanoate (40.0 g, 0.28 mol) was dissolved in DCM (400 mL), and NBS (52 g, 0.29 mol) was added, followed by the addition of p-toluenesulfonic acid monohydrate (10.56 g, 0.056 mol). The reaction mixture was stirred at room temperature for 2.5 hours.

After TLC monitoring indicated the disappearance of the starting material, the mixture was filtered. The filtrate was added with water (500 mL) and stirred, and the phases were separated. The organic phase was washed with saturated brine (200 mL), dried over anhydrous sodium sulfate, and concentrated under reduced pressure. The resulting residue, ethyl 2-bromo-3-oxopentanoate (63 g), was used directly in the next step.

**Step B:** Ethyl 2-bromo-3-oxopentanoate (63 g, 0.28 mol) and 1-Boc-piperazine (56 g, 0.30 mol) were dissolved in acetonitrile (1.2 L), and potassium carbonate (78 g, 0.57 mol) was added. The reaction mixture was stirred at room temperature for 5 hours.

LCMS monitoring showed the disappearance of the starting material. The mixture was filtered, and the filtrate was concentrated under reduced pressure. The resulting residue was purified by column chromatography to obtain tert-butyl 4-(1-ethoxy-1,3-dioxolan-2-yl)piperazine-1-carboxylate (70 g).

MS (ESI) M/Z: 329.4 [M+H]⁺.

**Step** C: 1H-1,2,4-triazole-3,5-diamine (50 g, 0.5 mol) was dissolved in aqueous hydrobromic acid solution (400 mL), and cooled to 0°C in an ice-water bath. A solution of sodium nitrite (52 g, 0.76 mol) in water (130 mL) was added dropwise thereto. The reaction was warmed to room temperature and stirred for 1 hour, and then stirred at 100°C overnight.

After LCMS monitoring showed the disappearance of the starting material, the mixture was cooled to room temperature and filtered. The filtrate was adjusted to pH 7-7.5 with 10% aqueous sodium hydroxide solution. The mixture was extracted with ethyl acetate (300 mL × 5), and the combined organic phases were washed with saturated brine (200 mL), dried over anhydrous sodium sulfate, and concentrated to dryness under reduced pressure to obtain crude 3-bromo-1H-1,2,4-triazol-5-amine (27.8 g), which was used directly in the next step.

MS (ESI) M/Z: 163.0 [M+H]⁺.

**Step D:** At 0°C, 2-chloro-4-(trifluoromethyl)aniline (20 g, 0.1 mol) was dissolved in dichloromethane (200 mL). A solution of 2-chloroacetyl chloride (11.55 g, 0.1 mol) in dichloromethane (40 mL) was added dropwise. The reaction mixture was stirred at 0°C for 30 minutes, then warmed to room temperature and stirred overnight.

After TLC monitoring indicated the disappearance of most of the starting material, the reaction mixture was poured into saturated aqueous sodium bicarbonate solution (500 mL), extracted with dichloromethane (200 mL), and the organic phase was dried and concentrated to dryness. The resulting crude product was purified by column chromatography to obtain 2-chloro-N-(2-chloro-4-(trifluoromethyl)phenyl)acetamide (20.1 g).

MS (ESI) M/Z: 269.8 [M-H]⁻.

**Step E:** 2-Chloro-N-(2-chloro-4-(trifluoromethyl)phenyl)acetamide (20.1 g, 73.9 mmol) was dissolved in acetone (200 mL), then potassium iodide (14.22 g, 85.7 mmol) was added, and the reaction mixture was heated to reflux and stirred for 4 hours.

After LCMS monitoring showed the disappearance of the starting material, the mixture was cooled and filtered. The filtrate was concentrated to dryness under reduced pressure to obtain 2-iodo-N-(2-chloro-4-(trifluoromethyl)phenyl)acetamide (26.8 g), and the crude product was used directly in the next step.

MS (ESI) M/Z: 362.1 [M-H]⁻.

**Step F:** 4,6-Dichloro-5-methoxypyrimidine (30 g, 0.168 mol) was dissolved in tetrahydrofuran (240 mL) and cooled to 0°C in an ice-water bath. 3 M methylmagnesium bromide (61.4 mL, 0.184 mol) was added dropwise, and after the addition, the mixture was stirred for 1 hour in the ice-water bath.

After TLC monitoring indicated the disappearance of the starting material, the reaction was quenched by adding saturated aqueous ammonium chloride solution and extracted with ethyl acetate (300 mL). The organic phase was dried over anhydrous sodium sulfate and concentrated under reduced pressure. The resulting crude product was purified by column chromatography to obtain 4-chloro-5-methoxy-6-methylpyrimidine (20.6 g).

¹H NMR (400 MHz, CDCl₃) δ 8.54 (s, 1H), 3.84 (s, 3H), 2.49 (s, 3H).

**Step G:** 4-Chloro-5-methoxy-6-methylpyrimidine (20.6 g, 0.13 mol), Pd(dpphH)Cl₂•DCM (5.7 g, 7 mmol), and triethylamine (26.03 g, 0.257 mol) were dissolved in methanol (300 mL). Carbon monoxide (2M Pa) was introduced into the reaction mixture, and the reaction mixture was stirred at 60°C overnight.

After TLC monitoring indicated the disappearance of most of the starting material, the mixture was cooled. The mixture was concentrated under reduced pressure. The crude product was added to water and ethyl acetate, stirred, and the phases were separated. The organic phase was dried over anhydrous sodium sulfate and concentrated under reduced pressure. The resulting crude product was purified by column chromatography to obtain 4-chloro-5-methoxy-6-methylpyrimidine (12.4 g).

**Step H:** 4-Chloro-5-methoxy-6-methylpyrimidine (12.4 g, 68 mmol) was dissolved in tetrahydrofuran (124 mL), and a solution of lithium hydroxide monohydrate (3.14 g, 74.8 mmol) in water (45 mL) was added. The reaction mixture was stirred at room temperature overnight.

After TLC monitoring indicated the disappearance of the starting material, the reaction mixture was concentrated to dryness under reduced pressure to obtain the lithium salt of 5-methoxy-6-methylpyrimidine-4-carboxylic acid (11.8 g), and the crude product was used directly in the next step.

MS (ESI) M/Z: 169.4 [M+H]⁺.

**Step I:** 3-Bromo-1H-1,2,4-triazol-5-amine (20 g, 0.12 mol) and tert-butyl 4-(1-ethoxy-1,3-dioxolan-2-yl)piperazine-1-carboxylate (44.7 g, 0.132 mol) were dissolved in n-butanol (110 mL), and 85% phosphoric acid (12.1 g, 0.12 mol) was added. The reaction mixture was stirred at 100°C overnight. After cooling, the reaction mixture was concentrated to dryness under reduced pressure. The residue was dispersed in dichloromethane (300 mL), and triethylamine (30.3 g, 0.3 mol) and Boc₂O (26.2 g, 0.12 mol) were added. The reaction mixture was stirred at room temperature overnight.

After LCMS monitoring indicated the disappearance of the starting material, water (200 mL) was added, stirred, and the phases were separated. The organic phase was washed with saturated brine (100 mL), dried over sodium sulfate, and concentrated to dryness under pressure. The crude product was purified by column chromatography to obtain *tert-butyl 4-*(2-bromo-5-ethyl-7-oxo-4,7-dihydro-[1,2,4]triazolo[1,5-a]pyrimidin-6-yl)piperazine-1-carboxylate (16 g).

MS (ESI) M/Z: 427.1 [M+H]⁺.

**Step J:** *tert-Butyl* 4-(2-bromo-5-ethyl-7-oxo-4,7-dihydro-[1,2,4]triazolo[1,5-a]pyrimidin-6-yl)piperazine-1-carboxylate (2.34 g, 5.49 mmol) and 2-iodo-N-(2-chloro-4-(trifluoromethyl)phenyl)acetamide (2.19 g, 6.04 mmol) were dissolved in DMF (30 mL), and diisopropylethylamine (1.77 g, 13.73 mmol) was added. The reaction mixture was stirred at 70°C overnight.

After LCMS monitoring indicated the disappearance of the starting material, the mixture was cooled, diluted with water (100 mL), and adjusted to pH 3-4 with dilute hydrochloric acid. The mixture was extracted with ethyl acetate (100 mL × 2). The organic phase was washed with saturated brine (50 mL), dried over sodium sulfate, and concentrated to dryness under pressure. The crude product was purified by column chromatography to obtain tert-butyl 4-(2-bromo-4-(2-((2-chloro-4-(trifluoromethyl)phenyl)amino)-2-oxoethyl)-5-ethyl-7-oxo-4,7-dihydro-[1,2,4]triazolo[1,5-a]pyrimidin-6-yl)piperazine-1-carboxylate (1.8 g).

MS (ESI) M/Z: 660.06 [M-H]⁻.

**Step K:** *tert-Butyl* 4-(2-bromo-4-(2-((2-chloro-4-(trifluoromethyl)phenyl)amino)-2-oxoethyl)-5-ethyl-7-oxo-4,7-dihydro-[1,2,4]triazolo[1,5-a]pyrimidin-6-yl)piperazine-1-carboxylate (1.8 g, 2.72 mmol) was dissolved in dichloromethane (20 mL), and trifluoroacetic acid (10 mL) was added. The reaction mixture was stirred at room temperature overnight.

After LCMS monitoring indicated the disappearance of the starting material, the reaction mixture was concentrated to dryness under reduced pressure to obtain crude 2-(2-bromo-5-ethyl-7-oxo-6-(piperazin-1-yl)-[1,2,4]triazolo[1,5-a]pyrimidin-4(7H)-yl)-N-(2-chloro-4-(trifluoromethyl)phenyl)acetamide trifluoroacetate, which was used directly in the next step.

MS (ESI) M/Z: 560.3 [M-H]⁻.

**Step L:** The crude 2-(2-bromo-5-ethyl-7-oxo-6-(piperazin-1-yl)-[1,2,4]triazolo[1,5-a]pyrimidin-4(7H)-yl)-N-(2-chloro-4-(trifluoromethyl)phenyl)acetamide trifluoroacetate (2.72 mmol) from the previous step and lithium 5-methoxy-6-methylpyrimidine-4-carboxylate (0.62 g, 3.52 mmol) were dissolved in DMF (30 mL). Diisopropylethylamine (1 g, 7.7 mmol) and HATU (1.3 g, 3.42 mmol) were added in an ice-water bath, and the reaction mixture was stirred at room temperature for 1 hour.

After LCMS monitoring indicated the disappearance of the starting material, the reaction mixture was poured into water (100 mL) and extracted with ethyl acetate (100 mL × 2). The organic phase was washed with saturated brine (50 mL), dried over sodium sulfate, and concentrated to dryness under reduced pressure. The crude product was purified by column chromatography to obtain 2-(2-bromo-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamide (1.1 g).

MS (ESI) M/Z: 712.1 [M+H] ⁺.

### Intermediate 2

### 2-(6-Bromo-2-(3,6-dihydro-2H-pyran-4-yl)-5-ethyl-7-oxo-[1,2,4]triazolo[1,5-a]pyrimidin-4(7H)-yl)-N-(2-chloro-4-(trifluoromethyl)phenyl)acetamide

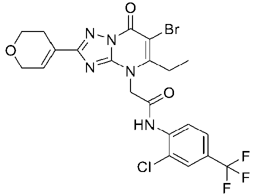

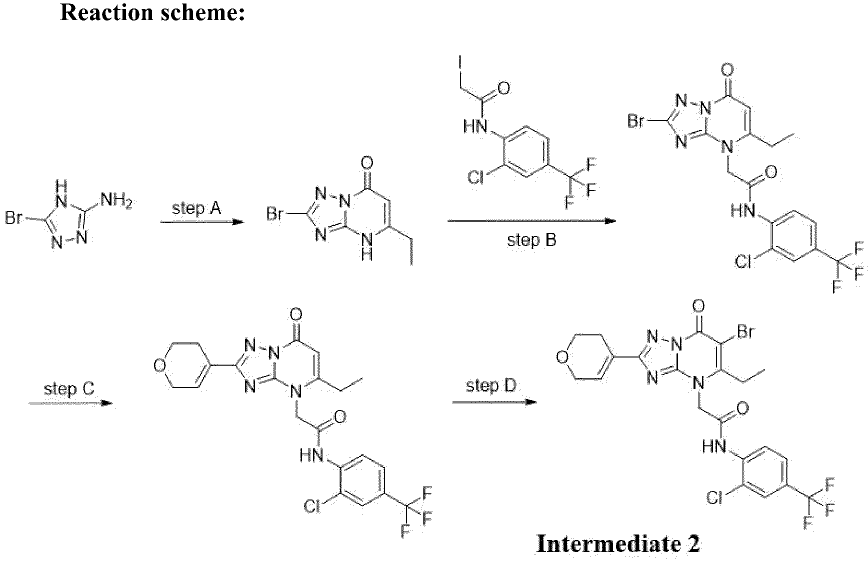

### Procedure:

Step A: 5-Bromo-4H-1,2,4-triazol-3-amine (10 g, 61 mmol) and ethyl propionylacetate (11.05 g, 77 mmol) were dissolved in acetic acid (35 mL), and the mixture was heated and stirred at 80°C overnight.

After LCMS monitoring indicated the disappearance of the starting material, the mixture was cooled in an ice-water bath, stirred for 1 hour, and filtered to obtain a white solid product. The filtrate was concentrated under reduced pressure, the residue was stirred with ethanol, then filtered again, and the combined solids were dried to obtain 2-bromo-5-ethyl-[1,2,4]triazolo[1,5-a]pyrimidin-7(4H)-one (5.6 g).

MS (ESI) M/Z: 243.1 [M+H]⁺.

**Step B:** 2-Bromo-5-ethyl-[1,2,4]triazolo[1,5-a]pyrimidin-7(4H)-one (5.6 g, 23 mmol) and N-(2-chloro-4-(trifluoromethyl)phenyl)-2-iodoacetamide (9.24 g, 25 mmol) were added to 1,4-dioxane (120 mL), and then diisopropylethylamine (8.96 g, 69 mmol) was added. The reaction mixture was stirred at 80°C overnight.

After LCMS monitoring indicated the disappearance of the starting material, the mixture was cooled to room temperature. Water (200 mL) and ethyl acetate (200 mL) were added. The mixture was stirred, and the phases were separated. The organic phase was washed with saturated brine (50 mL), dried over anhydrous sodium sulfate, and concentrated under reduced pressure. The resulting residue was purified by silica gel column chromatography to obtain 2-(2-bromo-5-ethyl-7-oxo-[1,2,4]triazolo[1,5-a]pyrimidin-4(7H)-yl)-N-(2-chloro-4-(trifluoromethyl)phenyl)acetamide (6 g).

MS (ESI) M/Z: 478.1 [M+H]⁺.

**Step C:** 2-(2-Bromo-5-ethyl-7-oxo-[1,2,4]triazolo[1,5-a]pyrimidin-4(7H)-yl)-N-(2-chloro-4-(trifluoromethyl)phenyl)acetamide (6 g, 12.5 mmol), 2-(3,6-dihydro-2H-pyran-4-yl)-4,4,5,5-tetramethyl-1,3,2-dioxaborolane (3.42 g, 16.3 mmol), potassium phosphate (7.98 g, 37.6 mmol), and [1,1'-bis(diphenylphosphino)ferrocene]dichloropalladium(II) dichloromethane complex (0.51 g, 0.63 mmol) were added to 1,4-dioxane (120 mL) and water (60 mL). The reaction system was purged three times with nitrogen, and the reaction mixture was stirred at 80°C for 1 hour.

After LCMS monitoring indicated the disappearance of the starting material, the mixture was cooled to room temperature. The reaction mixture was poured into water (500 mL), stirred, and filtered. The solid was slurried, filtered, and dried to obtain N-(2-chloro-4-(trifluoromethyl)phenyl)-2-(2-(3,6-dihydro-2H-pyran-4-yl)-5-ethyl-7-oxo-[1,2,4]triazolo[1,5-a]pyrimidin-4(7H)-yl)acetamide (4.9 g).

MS (ESI) M/Z: 482.1 [M+H] ⁺.

¹H NMR (400 MHz, DMSO-*d*₆) δ 10.43 (s, 1H), 8.05 (d, *J* = 8.6 Hz, 1H), 7.92 (s, 1H), 7.67 (d, *J =* 8.6 Hz, 1H), 6.84 (s, 1H), 5.95 (s, 1H), 5.26 (s, 2H), 4.26 (d, *J =* 2.9 Hz, 2H), 3.81 (t, *J=* 5.4 Hz, 2H), 2.73 (q, *J =* 7.3 Hz, 2H), 2.49 - 2.33 (m, 2H), 1.23 (t, *J= 7.3* Hz, 3H).

**Step** D: N-(2-Chloro-4-(trifluoromethyl)phenyl)-2-(2-(3,6-dihydro-2H-pyran-4-yl)-5-ethyl-7-oxo-[1,2,4]triazolo[1,5-a]pyrimidin-4(7H)-yl)acetamide (2 g, 4.16 mmol) was dissolved in DMF (20 mL), and NBS (1.11 g, 6.25 mmol) was added. The reaction mixture was stirred at 50°C overnight.

After LCMS monitoring indicated the disappearance of most of the starting material, the mixture was cooled. The reaction mixture was poured into water (100 mL) and extracted with ethyl acetate (100 mL × 2). The combined organic phases were washed with saturated brine (50 mL), dried over anhydrous sodium sulfate, and concentrated to dryness under reduced pressure. The resulting crude product was purified by silica gel column chromatography to obtain 2-(6-bromo-2-(3,6-dihydro-2H-pyran-4-yl)-5-ethyl-7-oxo-[1,2,4]triazolo[1,5-a]pyrimidin-4(7H)-yl)-N-(2-chloro-4-(trifluoromethyl)phenyl)acetamide (1.6 g).

MS (ESI) M/Z: 558.0 [M-H]⁻.

### Intermediate 3

### 4,4,5,5-Tetramethyl-2-(2,5,6,7-tetrahydrooxepin-4-yl)-1,3,2-dioxaborolane

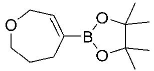

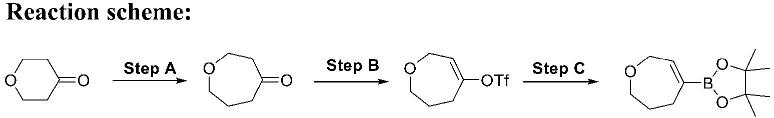

### Procedure:

**Step** A: In a three-neck flask, compound tetrahydro-4H-pyran-4-one (15.5 g, 154.8 mmol) was dissolved in ultra-dry dichloromethane (400 mL). The reaction system was then cooled to -78°C, followed by the slow addition of boron trifluoride etherate solution (22 mL) under a nitrogen atmosphere. After the dropwise addition was completed, (trimethylsilyl)diazomethane (86 mL) was slowly added dropwise. The reaction mixture was slowly warmed from -78°C to room temperature and stirred for 3 hours.

After TLC monitoring indicated the disappearance of the starting material, water was added to the reaction mixture, and the mixture was extracted with dichloromethane. The organic phase was dried over anhydrous sodium sulfate, then filtered, and concentrated under reduced pressure. The crude product was purified by silica gel column chromatography to obtain oxepan-4-one (4.7 g).

¹HNMR (400 MHz, Chloroform-d) δ 3.91 - 3.81 (m, 4H), 2.74 - 2.63 (m, 4H), 1.92 - 1.80 (m, 2H).

**Step B:** Oxepan-4-one (4.7 g, 41.2 mmol) was dissolved in anhydrous tetrahydrofuran (20 mL). The reaction system was evacuated and purged with nitrogen, and then lithium bis(trimethylsilyl)amide (45 mL, 45.0 mmol) was added dropwise at -78°C. After the dropwise addition was completed, the reaction mixture was stirred at -78°C for 2 hours. Subsequently, N-phenylbis(trifluoromethanesulfonyl)imide (16.2 g, 45.3 mmol) dissolved in anhydrous tetrahydrofuran was added dropwise to the reaction flask. The reaction system was slowly warmed to room temperature and then stirred for 1 hour.

After TLC monitoring indicated the disappearance of the starting material, the reaction mixture was quenched with saturated aqueous ammonium chloride solution, and then extracted with ethyl acetate. The organic phase was dried over anhydrous sodium sulfate, then filtered, and concentrated under reduced pressure. The crude product was purified by silica gel column chromatography to obtain 2,5,6,7-tetrahydrooxepin-4-yl trifluoromethanesulfonate (2.5 g).

¹HNMR (400 MHz, DMSO-*d₆*) δ 6.00 (d, *J*= 4.0 Hz, 1H), 4.22 - 4.10 (m, 2H), 3.75 (t, *J=* 5.6 Hz, 2H), 2.62 (s, 2H), 1.97 - 1.84 (m, 2H).

**Step C:** 2,5,6,7-Tetrahydrooxepin-4-yl trifluoromethanesulfonate (1.0 g, 4.06 mmol) and bis(pinacolato)diboron (1.2 g, 4.8 mmol) were dissolved in ultra-dry 1,4-dioxane (15 mL). Then, [1,1'-bis(diphenylphosphino)ferrocene]dichloropalladium(II) (300 mg, 406.2 µmol) and potassium acetate (1.2 g, 12.2 mmol) were added to the reaction system. The reaction system was stirred at 80°C for 3 hours under a nitrogen atmosphere.

After TLC monitoring indicated the disappearance of the starting material, the reaction mixture was directly concentrated under reduced pressure to obtain the crude product. The resulting mixture was purified by silica gel column chromatography to obtain 4,4,5,5-tetramethyl-2-(2,5,6,7-tetrahydrooxepin-4-yl)-1,3,2-dioxaborolane (300 mg).

¹H NMR (400 MHz, DMSO-*d₆*) δ 6.41 (t, *J* = 4.2 Hz, 1H), 4.13 - 4.12 (m, 2H), 3.71 *(t, J=* 5.9 Hz, 2H), 2.31-2.28 (m, 2H), 1.74-1.69 (m, 2H), 1.20 (s, 12H).

### Intermediate 4

### 4-Methyl-5-(trifluoromethyl)thiophen-3-amine hydrochloride

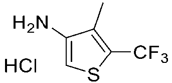

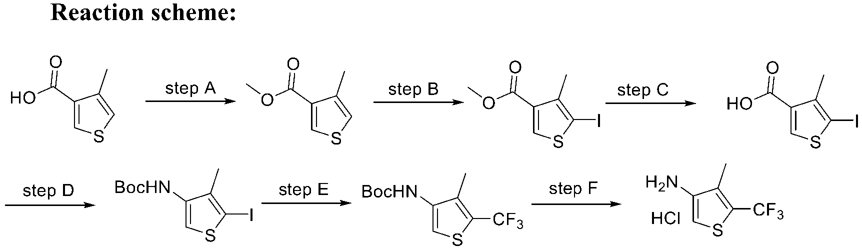

### Procedure:

**Step A:** 4-Methylthiophene-3-carboxylic acid (10 g, 70.3 mmol) was dissolved in methanol (100 mL), then concentrated sulfuric acid (6.89 g, 70.3 mmol) was added, and the reaction was carried out at 75°C overnight.

After TLC monitoring indicated the disappearance of the starting material, the reaction mixture was concentrated under reduced pressure. Aqueous sodium bicarbonate solution was then added to adjust the pH to neutral, and the mixture was extracted twice with ethyl acetate (50 mL). The combined organic phases were washed with saturated brine (20 mL), dried over anhydrous sodium sulfate, filtered, and concentrated to dryness under reduced pressure to obtain crude methyl 4-methylthiophene-3-carboxylate (9.5 g), which was used directly in the next step.

**Step B:** Methyl 4-methylthiophene-3-carboxylate (4 g, 26.3 mmol) was dissolved in chloroform (80 mL) and acetic acid (80 mL), and N-iodosuccinimide (6.51 g, 28.9 mmol) was added. The reaction mixture was stirred at room temperature for 3 hours.

After TLC monitoring indicated the disappearance of the starting material, the reaction mixture was poured into sodium sulfite solution (100 mL) and extracted twice with dichloromethane (100 mL). The combined organic phases were washed with sodium bicarbonate solution and saturated brine (100 mL), dried over anhydrous sodium sulfate, filtered, and concentrated to dryness under reduced pressure. The resulting residue was purified by silica gel column chromatography to obtain methyl 5-iodo-4-methylthiophene-3-carboxylate (5.5 g).

¹H NMR (400 MHz, CDCl₃) δ 8.22 (s, 1H), 3.85 (s, 3H), 2.44 (s, 3H).

**Step C:** Methyl 5-iodo-4-methylthiophene-3-carboxylate (5.5 g, 19.5 mmol) was dissolved in tetrahydrofuran (30 mL), water (20 mL), and methanol (20 mL), and then sodium hydroxide (1.56 g, 39 mmol) was added. The reaction was carried out at room temperature for 2 hours.

After TLC monitoring indicated the disappearance of the starting material, the mixture was concentrated under reduced pressure. Water (100 mL) and methyl tert-butyl ether (100 mL) were added to extract impurities. The resulting aqueous phase was adjusted to pH 3-4 with dilute hydrochloric acid, and extracted twice with ethyl acetate (100 mL). The combined organic phases were washed with saturated brine (100 mL), dried over anhydrous sodium sulfate, filtered, and concentrated to dryness under reduced pressure to obtain crude 5-iodo-4-methylthiophene-3-carboxylic acid, which was used directly in the next step.

MS (ESI) M/Z: 267.0 [M-H]⁻.

**Step D:** 5-Iodo-4-methylthiophene-3-carboxylic acid (5.1 g, 19 mmol) was dissolved in tert-butanol (50 mL), and diphenylphosphoryl azide (5.5 g, 19.97 mmol) and triethylamine (2 g, 19.97 mmol) were added. The mixture was heated at 60°C and reacted for 1 hour, then the temperature was raised to 90°C and the reaction was carried out overnight.

After TLC monitoring indicated the disappearance of the starting material, the mixture was cooled. Water (100 mL) was added, and the mixture was extracted twice with ethyl acetate (100 mL). The combined organic phases were washed with saturated brine (100 mL), dried over anhydrous sodium sulfate, filtered, and concentrated to dryness under reduced pressure. The resulting residue was purified by silica gel column chromatography to obtain tert-butyl (5-iodo-4-methylthiophen-3-yl)carbamate (3.9 g).

¹H NMR (400 MHz, CDCl₃) δ 7.48 (s, 1H), 6.28 (s, 1H), 2.04 (s, 3H), 1.46 (s, 9H).

**Step E:** *tert*-Butyl (5-iodo-4-methylthiophen-3-yl)carbamate (2.1 g, 6.18 mmol) was dissolved in *N,N*-dimethylformamide (20 mL), and diphenyl(trifluoromethyl)sulfonium trifluoromethanesulfonate (5 g, 12.36 mmol) and copper powder (1.19 g, 18.58 mmol) were added. The mixture was heated at 60°C and reacted overnight.

After TLC monitoring indicated the disappearance of the starting material, the mixture was cooled. The mixture was filtered through diatomite. Water (100 mL) was added, and the mixture was extracted twice with ethyl acetate (100 mL). The combined organic phases were washed with saturated brine (100 mL), dried over anhydrous sodium sulfate, filtered, and concentrated to dryness under reduced pressure. The resulting residue was purified by silica gel column chromatography to obtain tert-butyl (4-methyl-5-(trifluoromethyl)thiophen-3-yl)carbamate (1.5 g).

¹H NMR (400 MHz, CDCl₃) δ 7.49 (s, 1H), 6.29 (s, 1H), 2.15 (s, 3H), 1.46 (s, 9H).

**Step F:** tert-Butyl (4-methyl-5-(trifluoromethyl)thiophen-3-yl)carbamate (1.5 g, 5.33 mmol) was dissolved in ethyl acetate (10 mL), and hydrogen chloride in ethyl acetate (4 M, 20 mL) was added. The reaction was carried out at room temperature for 2 hours.

After TLC monitoring indicated the disappearance of the starting material, the mixture was concentrated under reduced pressure to obtain 4-methyl-5-(trifluoromethyl)thiophen-3-amine hydrochloride (560 mg).

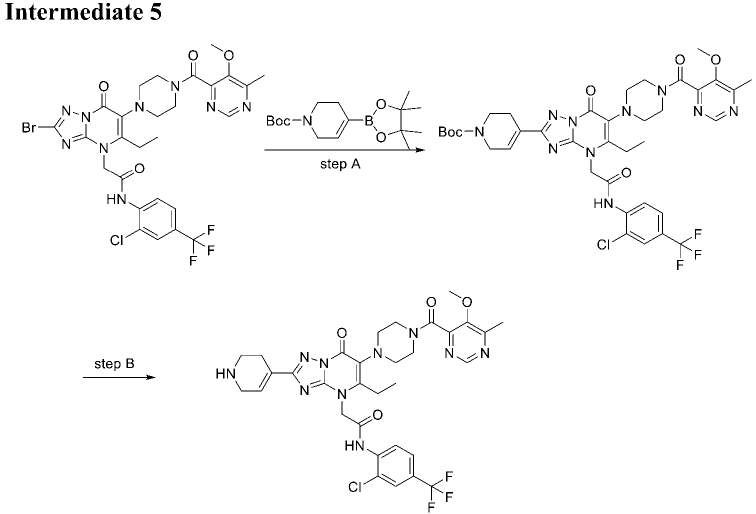

Step A: 2-(2-Bromo-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamide (1 g, 1.4 mmol), tert-butyl 4-(4,4,5,5-tetramethyl-1,3,2-dioxaborolan-2-yl)-3,6-dihydropyridine-1(2H)-carboxylate (430 mg, 1.4 mmol), sodium carbonate (372 mg, 3.5 mmol), and [1,1'-bis(diphenylphosphino)ferrocene]dichloropalladium(II) dichloromethane complex (60 mg, 0.07 mmol) were added to 1,4-dioxane (20 mL) and water (2.5 mL). The reaction system was purged three times with nitrogen, and the reaction mixture was stirred at 100°C overnight.

After LCMS monitoring indicated the disappearance of the starting material, the mixture was cooled to room temperature. Water (50 mL) and ethyl acetate (50 mL) were added for extraction, stirred, and the phases were separated. The aqueous phase was extracted once more with ethyl acetate (50 mL). The combined organic phases were washed with saturated brine (50 mL), dried over anhydrous sodium sulfate, and concentrated under reduced pressure. The resulting residue was purified by silica gel column chromatography to obtain *tert-butyl* 4-(4-(2-((2-chloro-4-(trifluoromethyl)phenyl)amino)-2-oxoethyl)-5-ethyl-6-(4-(5-methoxy-6-methylpyrimidine-4-carbonyl)piperazin-1-yl)-7-oxo-4,7-dihydro-[1,2,4]triazolo[1,5-a]pyrimidin-2-yl)-3,6-dihydropyridine-1(2H)-carboxylate (450 mg).

MS (ESI) M/Z: 813.2 [M-H]⁻.

**Step B:** *tert*-Butyl 4-(4-(2-((2-chloro-4-(trifluoromethyl)phenyl)amino)-2-oxoethyl)-5-ethyl-6-(4-(5-methoxy-6-methylpyrimidine-4-carbonyl)piperazin-1-yl)-7-oxo-4,7-dihydro-[1,2,4]triazolo[1,5-a]pyrimidin-2-yl)-3,6-dihydropyridine-1(2H)-carboxylate (150 mg, 0.18 mmol) was dissolved in dichloromethane (2 mL), then trifluoroacetic acid (1 mL) was added, and the reaction mixture was stirred at room temperature for 2 hours.

After LCMS monitoring indicated the disappearance of the starting material, the reaction mixture was concentrated to dryness under reduced pressure. The resulting crude N-(2-chloro-4-(trifluoromethyl)phenyl)-2-(5-ethyl-6-(4-(5-methoxy-6-methylpyrimidine-4-carbonyl)piperazin-1-yl)-7-oxo-2-(1,2,3,6-tetrahydropyridin-4-yl)-[1,2,4]triazolo[1,5-a]pyrimidin-4(7H)-yl)acetamide was used directly in the next step.

MS (ESI) M/Z: 715.3 [M+H]⁺.

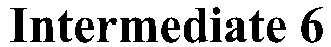

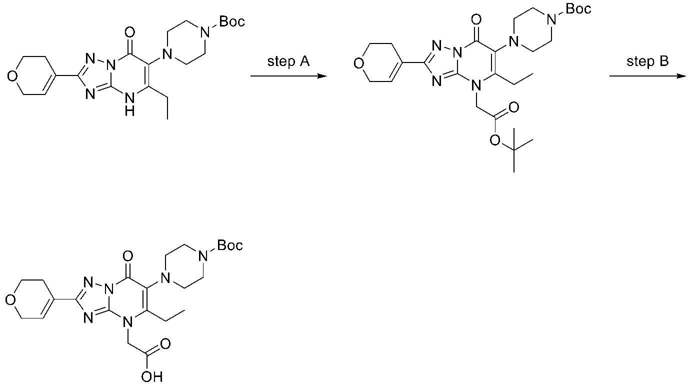

**Step A:** *tert*-Butyl 4-(2-(3,6-dihydro-2H-pyran-4-yl)-5-ethyl-7-oxo-4,7-dihydro-[1,2,4]triazolo[1,5-a]pyrimidin-6-yl)piperazine-1-carboxylate (500 mg, 1.16 mmol) and *tert-*butyl 2-bromoacetate (238 mg, 1.22 mmol) were dissolved in *N,N*-dimethylformamide (4 mL). Then, N,N-diisopropylethylamine (450 mg, 3.49 mmol) was added, and the reaction mixture was stirred at 55°C overnight.

After LCMS monitoring indicated the disappearance of the starting material, water (30 mL) was added, and the mixture was extracted twice with ethyl acetate (40 mL). The combined organic phases were first washed with saturated brine (30 mL), then dried over anhydrous sodium sulfate, and finally concentrated under reduced pressure. The resulting residue was purified by silica gel column chromatography to obtain tert-butyl *4-(4-(2-(tert-*butoxy)-2-oxoethyl)-2-(3,6-dihydro-2H-pyran-4-yl)-5-ethyl-7-oxo-4,7-dihydro-[1,2,4]triazolo[1,5-a]pyrimidin-6-yl)piperazine-1-carboxylate (500 mg).

MS (ESI) M/Z: 567.4 [M+Na]⁺.

**Step B:** *tert*-Butyl 4-(4-(2-(tert-butoxy)-2-oxoethyl)-2-(3,6-dihydro-2H-pyran-4-yl)-5-ethyl-7-oxo-4,7-dihydro-[1,2,4]triazolo[1,5-a]pyrimidin-6-yl)piperazine-1-carboxylate (500 mg, 0.92 mmol) was dissolved in tetrahydrofuran (10 mL) and methanol (4 mL), and then 1 M aqueous sodium hydroxide solution (1.4 mL) was added. The reaction mixture was stirred at room temperature overnight.

After LCMS monitoring indicated the disappearance of the starting material, the mixture was cooled to room temperature. The pH was adjusted to 3 with 1 M dilute aqueous hydrochloric acid, and the mixture was extracted twice with ethyl acetate (40 mL). The combined organic phases were washed with saturated brine (10 mL), dried over anhydrous sodium sulfate, and concentrated under reduced pressure to obtain *2-(6-(4-(tert-*butoxycarbonyl)piperazin-1-yl)-2-(3,6-dihydro-2H-pyran-4-yl)-5-ethyl-7-oxo-[1,2,4]triazolo[1,5-a]pyrimidin-4(7H)-yl)acetic acid (400 mg).

MS (ESI) M/Z: 487.6 [M-H]⁻.

### Example 1

### N-(2-Chloro-4-(trifluoromethyl)phenyl)-2-(5-ethyl-6-(4-(5-hydroxy-6-methylpyrimidine-4-carbonyl)piperazin-1-yl)-7-oxo-2-(2-oxaspiro[3.5]non-6-en-7-yl)-[1,2,4]triazolo[1,5-a]pyrimidin-4(7H)-yl)acetamide

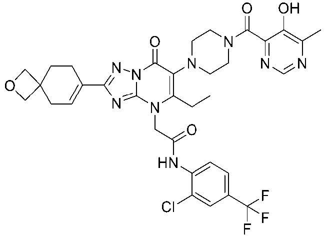

**The compound may also be synthesized according to the following steps:**

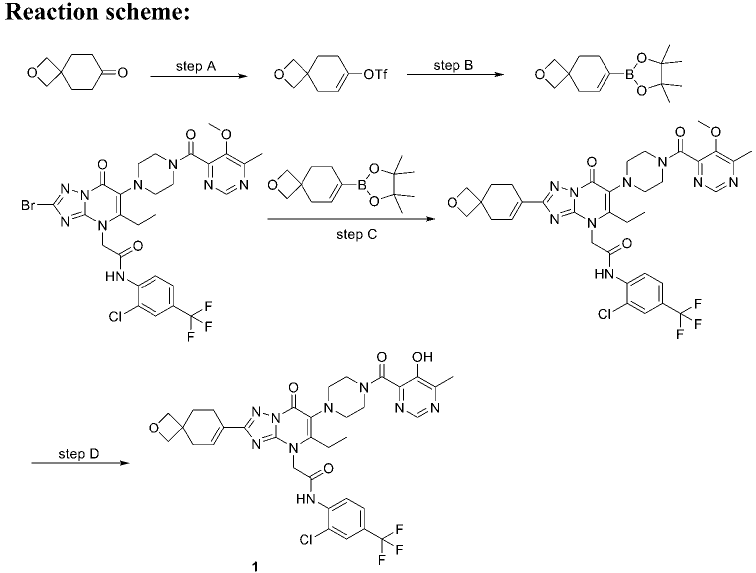

### Procedure:

**Step A:** 2-Oxaspiro[3.5]nonan-7-one (500 mg, 3.57 mmol) was dissolved in anhydrous tetrahydrofuran (10 mL). Lithium bis(trimethylsilyl)amide (1 M, 4.28 mL, 4.28 mmol) was added dropwise at -70°C, and the reaction was carried out at -70°C for 2 hours.

A solution of 1,1,1-trifluoro-*N*-phenyl-*N-*((trifluoromethyl)sulfonyl)methanesulfonamide (1.91 g, 5.35 mmol) in tetrahydrofuran (10 mL) was added dropwise to the reaction mixture at -70°C, and the reaction was carried out at room temperature overnight.

After TLC monitoring indicated the disappearance of the starting material, aqueous ammonium chloride solution (30 mL) was added, and the mixture was stirred for 10 minutes. The mixture was extracted twice with ethyl acetate (40 mL). The combined organic phases were first washed with saturated brine (50 mL), then dried over anhydrous sodium sulfate, and finally concentrated under reduced pressure. The resulting residue was purified by silica gel column chromatography to obtain 2-oxaspiro[3.5]non-6-en-7-yl trifluoromethanesulfonate (800 mg).

¹H NMR (400 MHz, CDCl₃) δ 5.71 (s, 1H), 4.53 - 4.43 (m, 4H), 2.54 (m, 2H), 2.41 (m, 2H), 2.13 - 2.03 (m, 2H).

**Step B:** 2-Oxaspiro[3.5]non-6-en-7-yl trifluoromethanesulfonate (800 mg, 2.94 mmol) and bis(pinacolato)diboron (895.47 mg, 3.53 mmol) were dissolved in dioxane (20 mL). Potassium acetate (865.18 mg, 8.81 mmol) and [1,1'-bis(diphenylphosphino)ferrocene]dichloropalladium(II) dichloromethane complex (238.3 mg, 0.294 mmol) were added. The system was evacuated and purged with nitrogen 2-3 times, and the reaction was carried out at 80°C overnight.

After TLC monitoring indicated the disappearance of the starting material, water (30 mL) was added, and the mixture was extracted with ethyl acetate (20 mL × 2). The organic phase was first washed with saturated brine (50 mL), then dried over anhydrous sodium sulfate, and finally concentrated under reduced pressure to obtain crude 4,4,5,5-tetramethyl-2-(2-oxaspiro[3.5]non-6-en-7-yl)-1,3,2-dioxaborolane (1.2 g), which was used directly in the next step.

**Step C:** 2-(2-Bromo-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamide (200 mg, 0.28 mmol) was dissolved in dioxane (4 mL) and water (1 mL). Crude 4,4,5,5-tetramethyl-2-(2-oxaspiro[3.5]non-6-en-7-yl)-1,3,2-dioxaborolane (140.26 mg, 0.56 mmol), potassium carbonate (76 mg, 0.56 mmol), and [1,1'-bis(diphenylphosphino)ferrocene]dichloropalladium(II) dichloromethane complex (22 mg, 0.028 mmol) were added. The system was evacuated and purged with nitrogen, and the reaction was carried out at 100°C for 2 hours.

After LC monitoring indicated the disappearance of the starting material, water (30 mL) was added, and the mixture was extracted twice with ethyl acetate (40 mL). The combined organic phases were first washed with saturated brine (50 mL), then dried over anhydrous sodium sulfate, and finally concentrated under reduced pressure. The resulting residue was purified by silica gel column chromatography to obtain N-(2-chloro-4-(trifluoromethyl)phenyl)-2-(5-ethyl-6-(4-(5-methoxy-6-methylpyrimidine-4-carbonyl)piperazin-1-yl)-7-oxo-2-(2-oxaspiro[3.5]non-6-en-7-yl)-[1,2,4]triazolo[1,5-a]pyrimidin-4(7H)-yl)acetamide (70 mg).

MS (ESI) M/Z: 756.4 [M+H]⁺.

**Step** D: N-(2-Chloro-4-(trifluoromethyl)phenyl)-2-(5-ethyl-6-(4-(5-methoxy-6-methylpyrimidine-4-carbonyl)piperazin-1-yl)-7-oxo-2-(2-oxaspiro[3.5]non-6-en-7-yl)-[1,2,4]triazolo[1,5-a]pyrimidin-4(7H)-yl)acetamide (70 mg, 0.093 mmol) was dissolved in DMF (3 mL), and lithium chloride (39.3 mg, 0.93 mmol) was added. The reaction mixture was reacted at 140°C for 4 hours.

After LC-MS monitoring indicated the disappearance of the starting material, the mixture was cooled to room temperature. The mixture was concentrated to dryness under reduced pressure, and the resulting residue was subjected to preparative chromatography N-(2-chloro-4-(trifluoromethyl)phenyl)-2-(5-ethyl-6-(4-(5-hydroxy-6-methylpyrimidine-4-carbonyl)piperazin-1-yl)-7-oxo-2-(2-oxaspiro[3.5]non-6-en-7-yl)-[1,2,4]triazolo[1,5-a]pyrimidin-4(7H)-yl)acetamide (1.49 mg).

MS (ESI) M/Z: 742.3 [M+H] ⁺.

¹H NMR (400 MHz, DMSO-*d*₆) δ 10.67 (s, 1H), 8.38 (s, 1H), 8.05 - 7.99 (m, 1H), 7.99 - 7.93 (m, 1H), 7.76 - 7.67 (m, 1H), 6.83 - 6.74 (m, 1H), 5.34 (s, 2H), 4.71 - 4.62 (m, 1H), 4.58 - 4.46 (m, 2H), 4.39 - 4.35 (m, 2H), 4.32 - 4.28 (m, 2H), 3.49 - 3.48 (m, 2H), 2.99 - 2.96 (m, 3H), 2.81 - 2.79 (m, 1H), 2.68 - 2.67 (m, 1H), 2.59 - 2.57 (m, 2H), 2.40 (s, 3H), 1.99 - 1.94 (m, 4H), 1.19 - 1.16 (m, 3H).

### Example: 18

### N-(2-Chloro-4-(trifluoromethyl)phenyl)-2-(5-ethyl-6-(4-(5-hydroxy-6-methylpyrimidine-4-carbonyl)piperazin-1-yl)-2-(1-isobutyryl-1,2,3,6-tetrahydropyridin-4-yl)-7-oxo-[1,2,4]triazolo[1,5-a]pyrimidin-4(7H)-yl)acetamide

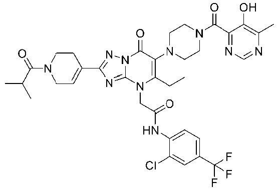

**The compound may also be synthesized according to the following steps:**

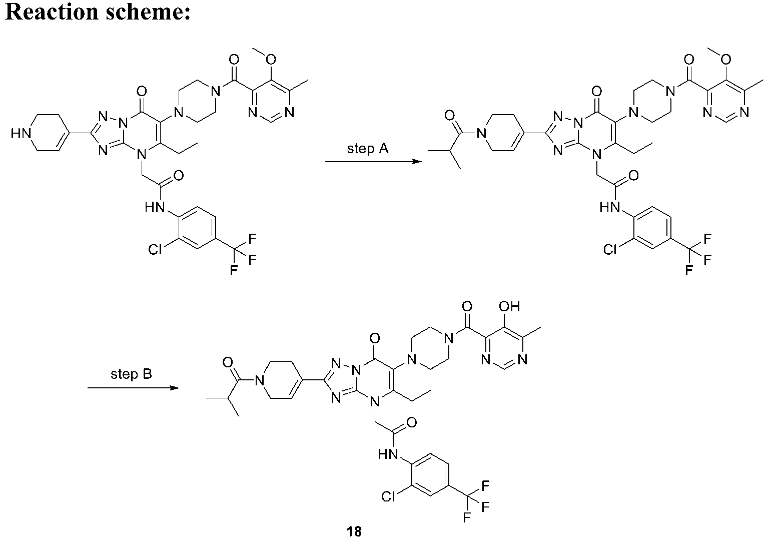

### Procedure:

Step A: The crude *N*-(2-chloro-4-(trifluoromethyl)phenyl)-2-(5-ethyl-6-(4-(5-methoxy-6-methylpyrimidine-4-carbonyl)piperazin-l-yl)-7-oxo-2-(1,2,3,6-tetrahydropyridin-4-yl)-[1,2,4]triazolo[1,5-a]pyrimidin-4(7H)-yl)acetamide (0.18 mmol) was dissolved in dichloromethane (5 mL), and triethylamine (220 mg, 2.18 mmol) and isobutyryl chloride (29 mg, 0.27 mmol) were added. The reaction mixture was stirred at room temperature overnight.

After LCMS monitoring indicated the disappearance of the starting material, water (10 mL) was added, and the mixture was extracted twice with dichloromethane (20 mL). The combined organic phases were washed with saturated brine (10 mL), dried over anhydrous sodium sulfate, and concentrated to dryness under reduced pressure. The resulting crude N-(2-chloro-4-(trifluoromethyl)phenyl)-2-(5-ethyl-2-(1-isobutyryl-1,2,3,6-tetrahydropyridin-4-yl)-6-(4-(5-methoxy-6-methylpyrimidine-4-carbonyl)piperazin-1-yl)-7-oxo-[1,2,4]triazolo[1,5-a]pyrimidin-4(7H)-yl)acetamide (77 mg) was used directly in the next step.

MS (ESI) M/Z: 785.5 [M+H]⁺.

**Step B:** The crude N-(2-chloro-4-(trifluoromethyl)phenyl)-2-(5-ethyl-2-(1-isobutyryl-1,2,3,6-tetrahydropyridin-4-yl)-6-(4-(5-methoxy-6-methylpyrimidine-4-carbonyl)piperazin-1-yl)-7-oxo-[1,2,4]triazolo[1,5-a]pyrimidin-4(7H)-yl)acetamide (77 mg, 0.098 mmol) from the previous step was dissolved in dichloromethane (3 mL), and a 2 M solution of boron tribromide in dichloromethane (0.49 mL, 0.98 mmol) was added. The mixture was stirred at room temperature overnight.

After LCMS monitoring indicated the disappearance of the starting material, the reaction mixture was poured into ice water, the pH was adjusted to neutral with aqueous sodium bicarbonate solution, and the mixture was extracted with dichloromethane (20 mL × 2). The organic phase was dried and concentrated to dryness. The resulting crude product was purified by preparative chromatography to obtain N-(2-chloro-4-(trifluoromethyl)phenyl)-2-(5-ethyl-6-(4-(5-hydroxy-6-methylpyrimidine-4-carbonyl)piperazin-1-yl)-2-(1-isobutyryl-1,2,3,6-tetrahydropyridin-4-yl)-7-oxo-[1,2,4]triazolo[1,5-a]pyrimidin-4(7H)-yl)acetamide (2.86 mg).

MS (ESI) M/Z: 771.6 [M+H]⁺.

¹H NMR (400 MHz, DMSO-*d*₆) δ 10.38 (s, 1H), 10.24 (s, 1H), 8.58 (s, 1H), 8.06 (d, *J =* 8.4 Hz, 1H), 7.98 (s, 1H), 7.72 (d, *J =* 8.4 Hz, 1H), 6.86 - 6.77 (m, 1H), 5.32 (s, 2H), 4.56 - 4.48 (m, 1H), 4.32 - 4.24 (m, 1H), 4.16 - 4.11 (m, 1H), 3.71 - 3.64 (m, 2H), 3.51 - 3.45 (m, 3H), 3.26 - 3.23 (m, 1H), 3.02 - 2.95 (m, 3H), 2.84 - 2.78 (m, 1H), 2.68 - 2.54 (m, 4H), 2.44 (s, 3H), 1.18 (t, *J* = 7.5 Hz, 3H), 1.03 - 0.99 (m, 6H).

### Example 33

### 2-(2-(3,6-Dihydro-2H-pyran-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N(5-(trifluoromethyl)thiophen-2-yl)acetamide

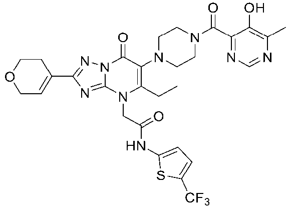

**The compound may also be synthesized according to the following steps:**

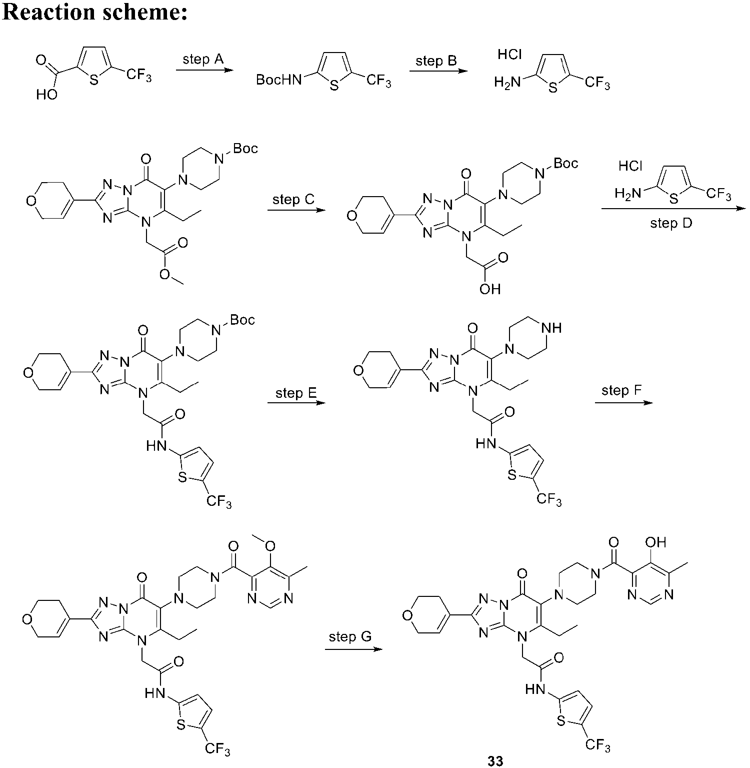

### Procedure:

Step A: 5-(Trifluoromethyl)thiophene-2-carboxylic acid (1.0 g, 5.1 mmol) was dissolved in tert-butanol (20 mL), and triethylamine (928.6 mg, 9.17 mmol) and diphenylphosphoryl azide (2.52 g, 9.17 mmol) were added. The reaction was carried out at 90°C overnight.

After TLC monitoring indicated the disappearance of the starting material, the *tert-*butanol was concentrated to dryness. Aqueous sodium bicarbonate solution (20 mL) was added and stirred for 10 minutes, and the mixture was extracted with ethyl acetate (40 mL). The organic phase was washed with dilute hydrochloric acid to adjust the pH to acidic, and the phases were separated. The organic phase was washed with saturated brine (30 mL), then dried over anhydrous sodium sulfate, and finally concentrated under reduced pressure. The resulting residue was purified by silica gel column chromatography to obtain *tert-butyl (5-*(trifluoromethyl)thiophen-2-yl)carbamate (880 mg).

MS (ESI) M/Z: 266.0 [M+H]⁺.

**Step B:** *tert*-Butyl (5-(trifluoromethyl)thiophen-2-yl)carbamate (880 mg, 3.29 mmol) was dissolved in ethyl acetate (5 mL), and then 4 M hydrochloric acid in ethyl acetate (10 mL) was added. The reaction was carried out at room temperature overnight.

After TLC monitoring indicated the disappearance of the starting material, the reaction mixture was concentrated under reduced pressure to obtain crude 5-(trifluoromethyl)thiophen-2-amine hydrochloride, which was used directly in the next step.

**Step C:** *tert*-Butyl 4-(2-(3,6-dihydro-2H-pyran-4-yl)-5-ethyl-4-(2-methoxy-2-oxoethyl)-7-oxo-4,7-dihydro-[1,2,4]triazolo[1,5-a]pyrimidin-6-yl)piperazine-1-carboxylate (550 mg, 1.09 mmol) was dissolved in tetrahydrofuran (10 mL) and methanol (4 mL), and 1 M aqueous sodium hydroxide solution (1.6 mL) was added. The reaction was carried out at room temperature overnight.

After TLC monitoring indicated the disappearance of the starting material, the pH was adjusted to 3 with 1 M dilute hydrochloric acid, and the mixture was extracted with ethyl acetate (20 mL × 2). The organic phase was first washed with saturated brine (20 mL), then dried over anhydrous sodium sulfate, and finally concentrated under reduced pressure to obtain 2-(6-(4-(tert-butoxycarbonyl)piperazin-1-yl)-2-(3,6-dihydro-2H-pyran-4-yl)-5-ethyl-7-oxo-[1,2,4]triazolo[1,5-a]pyrimidin-4(7H)-yl)acetic acid (480 mg).

MS (ESI) M/Z: 489.0 [M+H]⁺.

**Step D:** 2-(6-(4-(*tert*-Butoxycarbonyl)piperazin-1-yl)-2-(3,6-dihydro-2H-pyran-4-yl)-5-ethyl-7-oxo-[1,2,4]triazolo[1,5-a]pyrimidin-4(7H)-yl)acetic acid (250 mg, 0.5 mmol) and 5-(trifluoromethyl)thiophen-2-amine hydrochloride (124.7 mg, 0.61 mmol) were dissolved in N,Ndimethylformamide (6 mL). N,N-Diisopropylethylamine (264.8 mg, 2.05 mmol) was added, and HATU (233.7 mg, 0.61 mmol) was added in an ice-water bath. The reaction was carried out at room temperature for 1 hour.

After TLC monitoring indicated the disappearance of the starting material, water (30 mL) was added, and the mixture was extracted with ethyl acetate (20 mL × 2). The organic phase was first washed with saturated brine (30 mL), then dried over anhydrous sodium sulfate, and finally concentrated under reduced pressure. The resulting residue was purified by silica gel column chromatography to obtain tert-butyl 4-(2-(3,6-dihydro-2H-pyran-4-yl)-5-ethyl-7-oxo-4-(2-oxo-2-((5-(trifluoromethyl)thiophen-2-yl)amino)ethyl)-4,7-dihydro-[1,2,4]triazolo[1,5-a]pyrimidin-6-yl)piperazine-1-carboxylate (170 mg).

MS (ESI) M/Z: 638.4 [M+H] ⁺.

**Step E:** *tert*-Butyl 4-(2-(3,6-dihydro-2H-pyran-4-yl)-5-ethyl-7-oxo-4-(2-oxo-2-((5-(trifluoromethyl)thiophen-2-yl)amino)ethyl)-4,7-dihydro-[1,2,4]triazolo[1,5-a]pyrimidin-6-yl)piperazine-1-carboxylate (170 mg, 0.26 mmol) was dissolved in dichloromethane (6 mL), and trifluoroacetic acid (4.5 mL) was added. The reaction was carried out at room temperature for 1 hour.

After TLC monitoring indicated the disappearance of the starting material, the mixture was concentrated to dryness under reduced pressure to obtain crude 2-(2-(3,6-dihydro-2H-pyran-4-yl)-5-ethyl-7-oxo-6-(piperazin-1-yl)-[1,2,4]triazolo[1,5-a]pyrimidin-4(7H)-yl)-N-(5-(trifluoromethyl)thiophen-2-yl)acetamide trifluoroacetate, which was used directly in the next step.

**Step F:** 2-(2-(3,6-Dihydro-2H-pyran-4-yl)-5-ethyl-7-oxo-6-(piperazin-1-yl)-[1,2,4]triazolo[1,5-a]pyrimidin-4(7H)-yl)-*N*-(5-(trifluoromethyl)thiophen-2-yl)acetamide trifluoroacetate (173.7 mg, 0.26 mmol) and 5-methoxy-6-methylpyrimidine-4-carboxylic acid (49.3 mg, 0.29 mmol) were dissolved in N,N-dimethylformamide (4 mL). *N*,*N-*Diisopropylethylamine (137.9 mg, 1.06 mmol) was added, and HATU (121.7 mg, 0.32 mmol) was added in an ice-water bath. The reaction was carried out at room temperature for 1 hour.

After TLC monitoring indicated the disappearance of the starting material, water (30 mL) was added, and the mixture was extracted with ethyl acetate (20 mL × 2). The organic phase was first washed with saturated brine (30 mL), then dried over anhydrous sodium sulfate, and finally concentrated under reduced pressure. The resulting residue was purified by silica gel column chromatography to obtain 2-(2-(3,6-dihydro-2H-pyran-4-yl)-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-N-(5-(trifluoromethyl)thiophen-2-yl)acetamide (90 mg).

MS (ESI) M/Z: 688.3 [M+H]⁺.

Step G: 2-(2-(3,6-Dihydro-2H-pyran-4-yl)-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-N-(5-(trifluoromethyl)thiophen-2-yl)acetamide (90 mg, 0.13 mmol) was dissolved in *N,N-*dimethylformamide (4 mL), and lithium chloride (55.5 mg, 1.3 mmol) was added. The reaction mixture was stirred at 140°C for 4 hours, and then at 98°C overnight.

After LCMS monitoring indicated the disappearance of the starting material, the reaction mixture was cooled and concentrated to dryness under reduced pressure. The crude product was purified by preparative chromatography to obtain 2-(2-(3,6-dihydro-2H-pyran-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(5-(trifluoromethyl)thiophen-2-yl)acetamide (9.08 mg).

MS (ESI) M/Z: 674.2 [M+H] ⁺.

¹H NMR (400 MHz, DMSO-*d*₆) δ 8.57 (s, 1H), 8.09 - 8.03 (m, 1H), 7.47 (s, 1H), 6.83 - 6.72 (m, 2H), 5.24 (s, 2H), 4.57 - 4.45 (m, 1H), 4.27 - 4.15 (m, 2H), 3.82 - 3.74 (m, 2H), 3.55 - 3.42 (m, 3H), 3.25 - 3.22 (m, 1H), 3.03 - 2.92 (m, 3H), 2.85 - 2.78 (m, 1H), 2.68 - 2.63 (m, 1H), 2.50 - 2.50 (m, 2H), 2.44 (s, 3H), 1.15 - 1.13 (m, 3H).

### Example 61

### N-(2-Chloro-4-(trifluoromethyl)phenyl)-2-(2-(1-(2-cyclopropylacetyl)-1,2,3,6-tetrahydropyridin-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide

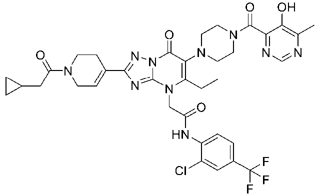

**The compound may also be synthesized according to the following steps:**

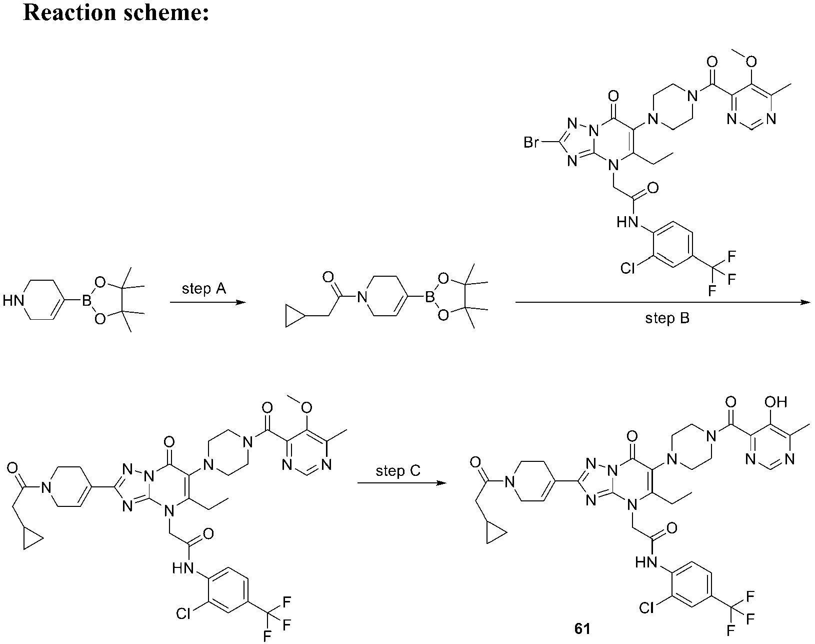

### Procedure:

**Step A:** Crude 4-(4,4,5,5-tetramethyl-1,3,2-dioxaborolan-2-yl)-1,2,3,6-tetrahydropyridine trifluoroacetate (675 mg, 3.23 mmol) was dissolved in dichloromethane (20 mL), and triethylamine (1.64 g, 16.17 mmol) and cyclopropylacetyl chloride (498 mg, 4.2 mmol) were added. The reaction was carried out at room temperature overnight.

After TLC monitoring indicated the disappearance of the starting material, the reaction mixture was concentrated to dryness under reduced pressure to obtain crude 2-cyclopropyl-1-(4-(4,4,5,5-tetramethyl-1,3,2-dioxaborolan-2-yl)-3,6-dihydropyridin-1(2H)-yl)ethan-1-one (950 mg), which was used directly in the next step.

**Step B:** 2-(2-Bromo-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamide (400 mg, 0.56 mmol), 2-cyclopropyl-1-(4-(4,4,5,5-tetramethyl-1,3,2-dioxaborolan-2-yl)-3,6-dihydropyridin-1(2H)-yl)ethan-1-one (131 mg, 0.45 mmol), anhydrous potassium carbonate (193 mg, 1.4 mmol), and [1,1'\-bis(diphenylphosphino)ferrocene]dichloropalladium(II) dichloromethane complex (22.85 mg, 0.028 mmol) were added to 1,4-dioxane (6 mL) and water (0.75 mL). The reaction system was purged three times with nitrogen, and the reaction mixture was stirred at 100°C for 2 hours.

After TLC monitoring indicated the disappearance of the starting material, the mixture was cooled. Water (20 mL) was added, and the mixture was extracted twice with ethyl acetate (30 mL). The combined organic phases were washed with saturated brine (20 mL), dried over anhydrous sodium sulfate, filtered, and concentrated to dryness under reduced pressure. The resulting residue was purified by silica gel column chromatography to obtain N-(2-chloro-4-(trifluoromethyl)phenyl)-2-(2-(1-(2-cyclopropylacetyl)-1,2,3,6-tetrahydropyridin-4-yl)-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)acetamide (160 mg).

MS (ESI) M/Z: 797.4 [M+H]⁺.

**Step C:** *N*-(2-Chloro-4-(trifluoromethyl)phenyl)-2-(2-(1-(2-cyclopropylacetyl)-1,2,3,6-tetrahydropyridin-4-yl)-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)acetamide (130 mg, 0.16 mmol) and lithium chloride (69 mg, 1.63 mmol) were added to anhydrous *N,N-*dimethylformamide (4 mL), and the mixture was heated at 140°C and reacted for 4 hours.

After LCMS monitoring indicated the disappearance of the starting material, the mixture was cooled. The mixture was concentrated to dryness under reduced pressure, and the resulting residue was purified by preparative chromatography to obtain N-(2-chloro-4-(trifluoromethyl)phenyl)-2-(2-(1-(2-cyclopropylacetyl)-1,2,3,6-tetrahydropyridin-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide (22.24 mg).

MS (ESI) M/Z: 783.4 [M+H]⁺.

¹H NMR (400 MHz, DMSO-*d*₆) δ 10.40 (s, 1H), 10.25 (s, 1H), 8.57 (s, 1H), 8.06 (d, *J* = 8.6 Hz, 1H), 7.98 (s, 1H), 7.72 (d, *J =* 8.6 Hz, 1H), 6.80 (d, *J =* 15.6 Hz, 1H), 5.33 (s, 2H), 4.57 - 4.48 (m, 1H), 4.22 - 4.11 (m, 2H), 3.70 - 3.60 (m, 2H), 3.51 - 3.44 (m, 3H), 3.27 - 3.22 (m, 1H), 3.03 - 2.94 (m, 3H), 2.85 - 2.79 (m, 1H), 2.67 - 2.64 (m, 1H), 2.61 - 2.57 (m, 2H), 2.44 (s, 3H), 2.35 - 2.28 (m, 2H), 1.19 (t, *J=* 7.5 Hz, 3H), 1.00 - 0.94 (m, 1H), 0.48 - 0.40 (m, 2H), 0.17 - 0.07 (m, 2H).

### Example 65

### N-(2-Chloro-4-(trifluoromethyl)phenyl)-2-(5-ethyl-6-(4-(5-hydroxy-6-methylpyrimidine-4-carbonyl)piperazin-1-yl)-2-(1-(5-methyl-1,3,4-oxadiazol-2-yl)-1,2,3,6-tetrahydropyridin-4-yl)-7-oxo-[1,2,4]triazolo[1,5-a]pyrimidin-4(7H)-yl)acetamide

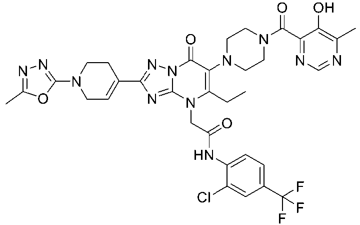

**The compound may also be synthesized according to the following steps:**

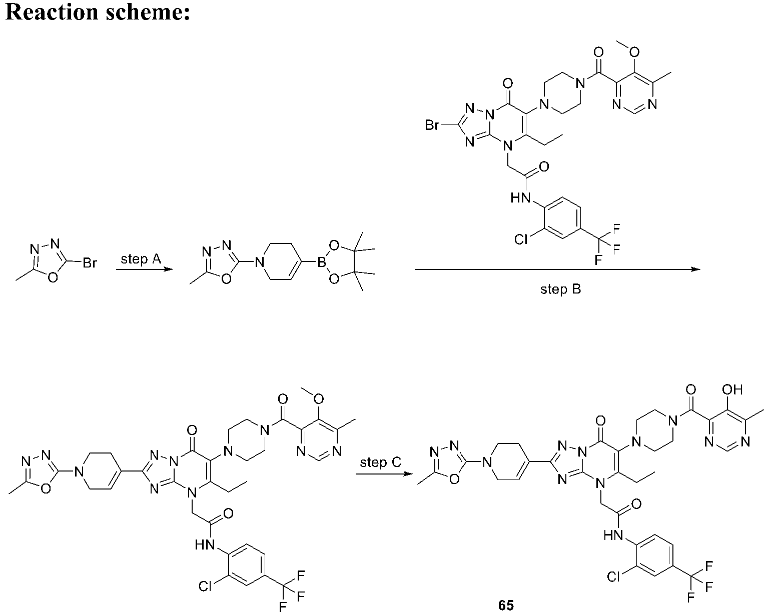

### Procedure:

**Step A:** 2-Bromo-5-methyl-1,3,4-oxadiazole (450 mg, 2.76 mmol) and 4-(4,4,5,5-tetramethyl-1,3,2-dioxaborolan-2-yl)-1,2,3,6-tetrahydropyridine (606.24 mg, 2.9 mmol) were dissolved in DMF (10 mL), and potassium carbonate (1.91 g, 13.81 mmol) was added. The reaction was carried out at 50°C for 2 hours.

After TLC monitoring indicated the disappearance of the starting material, the mixture was filtered and rinsed with a small amount of DMF to obtain a DMF solution (14 mL, 2.76 mmol) of 2-methyl-5-(4-(4,4,5,5-tetramethyl-1,3,2-dioxaborolan-2-yl)-3,6-dihydropyridin-1(2H)-yl)-1,3,4-oxadiazole, which was used directly in the next step.

**Step B:** 2-(2-Bromo-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamide (300 mg, 0.42 mmol) was dissolved in dioxane (8 mL) and water (2 mL), and the DMF solution of 2-methyl-5-(4-(4,4,5,5-tetramethyl-1,3,2-dioxaborolan-2-yl)-3,6-dihydropyridin-1(2H)-yl)-1,3,4-oxadiazole (2.14 mL, 0.42 mmol), potassium carbonate (116.46 mg, 0.84 mmol), and [1,1'-bis(diphenylphosphino)ferrocene]dichloropalladium(II) dichloromethane complex (34.18 mg, 0.042 mmol) were added. The reaction system was purged with nitrogen, and the reaction was carried out at 100°C for 2 hours.

After LC monitoring indicated the disappearance of the starting material, the mixture was cooled. Water (30 mL) was added, and the mixture was extracted twice with ethyl acetate (40 mL). The combined organic phases were first washed with saturated brine (50 mL), then dried over anhydrous sodium sulfate, and finally concentrated under reduced pressure. The resulting residue was purified by silica gel column chromatography to obtain N-(2-chloro-4-(trifluoromethyl)phenyl)-2-(5-ethyl-6-(4-(5-methoxy-6-methylpyrimidine-4-carbonyl)piperazin-1-yl)-2- 1-( 5-methyl-l ,3,4-oxadiazol-2-yl)-1 ,2,3,6-tetrahydropyridin-4-yl)-7-oxo-[1,2,4]triazolo[1,5-a]pyrimidin-4(7H)-yl)acetamide (140 mg).

MS (ESI) M/Z: 797.3 [M+H] ⁺.

**Step C:** *N*-(2-Chloro-4-(trifluoromethyl)phenyl)-2-(5-ethyl-6-(4-(5-methoxy-6-methylpyrimidine-4-carbonyl)piperazin-1-yl)-2-(1-(5-methyl-1,3,4-oxadiazol-2-yl)-1,2,3,6-tetrahydropyridin-4-yl)-7-oxo-[1,2,4]triazolo[1,5-a]pyrimidin-4(7H)-yl)acetamide (120 mg, 0.15 mmol) was dissolved in DMF (3 mL), and lithium chloride (63.82 mg, 1.51 mmol) was added. The reaction was carried out at 140°C for 4 hours.

After LC-MS monitoring indicated the disappearance of the starting material, the mixture was concentrated under reduced pressure, and the resulting residue was purified by preparative chromatography to obtain N-(2-chloro-4-(trifluoromethyl)phenyl)-2-(5-ethyl-6-(4-(5-hydroxy-6-methylpyrimidine-4-carbonyl)piperazin-1-yl)-2-(1-(5-methyl-1,3,4-oxadiazol-2-yl)-1,2,3,6-tetrahydropyridin-4-yl)-7-oxo-[1,2,4]triazolo[1,5-a]pyrimidin-4(7H)-yl)acetamide (20.12 mg).

MS (ESI) M/Z: 783.0 [M+H] ⁺.

¹H NMR (400 MHz, DMSO-*d*₆) δ 10.44 (s, 1H), 10.28 (s, 1H), 8.57 (s, 1H), 8.06 (d, *J=* 8.5 Hz, 1H), 7.98 (s, 1H), 7.72 (d, *J =* 8.9 Hz, 1H), 6.98 - 6.84 (m, 1H), 5.36 (s, 2H), 4.57 - 4.48 (m, 1H), 4.22 - 4.05 (m, 2H), 3.67 - 3.60 (m, 2H), 3.50 - 3.44 (m, 3H), 3.29 - 3.21 (m, 1H), 3.05 - 2.96 (m, 3H), 2.86 - 2.77 (m, 2H), 2.69 - 2.67 (m, 2H), 2.44 (s, 3H), 2.35 (s, 3H), 1.20 - 1.17 (m, 3H).

### Example 93

### Cyclopropyl 4-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)-3,6-dihydropyridine-1(2H)-carboxylate

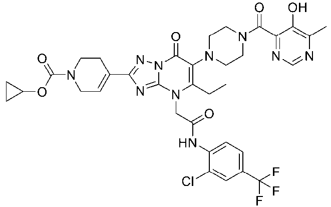

The compound may also be synthesized according to the following steps:

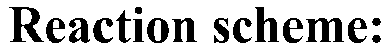

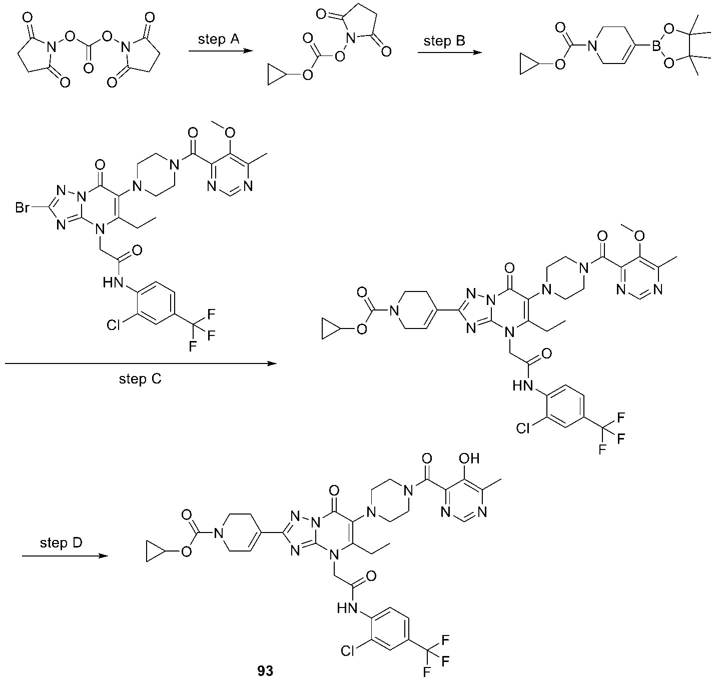

### Procedure:

**Step A:** Cyclopropanol (600 mg, 10.32 mmol) was added to acetonitrile (100 mL), and bis(2,5-dioxopyrrolidin-1-yl) carbonate (2.91 g, 11.34 mmol) and triethylamine (2.16 mL, 15.5 mmol) were added. The reaction mixture was reacted at room temperature overnight.

After TLC monitoring indicated the disappearance of the starting material, dichloromethane (200 mL) and aqueous sodium bicarbonate solution (100 mL) were added, the mixture was stirred, and the phases were separated. The organic phase was washed with saturated brine, dried over anhydrous sodium sulfate, filtered, and concentrated to dryness under reduced pressure to obtain crude cyclopropyl (2,5-dioxopyrrolidin-1-yl) carbonate (290 mg).

¹H NMR (400 MHz, CDCl₃) δ 4.58 - 4.24 (m, 1H), 3.00 - 2.72 (m, 4H), 1.12 - 0.61 (m, 4H).

**Step B:** Crude 4-(4,4,5,5-tetramethyl-1,3,2-dioxaborolan-2-yl)-1,2,3,6-tetrahydropyridine trifluoroacetate (0.97 mmol) was dissolved in dichloromethane (10 mL), and triethylamine (491 mg, 4.83 mmol) and crude cyclopropyl (2,5-dioxopyrrolidin-1-yl) carbonate (290 mg, 1.46 mmol) were added. The reaction was carried out at room temperature overnight.

After TLC monitoring indicated the disappearance of the starting material, dilute hydrochloric acid (1 M, 10 mL) was added, and the mixture was extracted twice with dichloromethane (10 mL). The combined organic phases were washed sequentially with aqueous sodium bicarbonate solution (10 mL) and saturated brine (10 mL), dried over anhydrous sodium sulfate, and concentrated to dryness under reduced pressure to obtain crude cyclopropyl 4-(4,4,5,5-tetramethyl-1,3,2-dioxaborolan-2-yl)-3,6-dihydropyridine-1 (2H)-carboxylate (320 mg).

MS (ESI) M/Z: 294.2 [M+H] ⁺.

**Step** C: 2-(2-Bromo-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamide (300 mg, 0.42 mmol), crude cyclopropyl 4-(4,4,5,5-tetramethyl-1,3,2-dioxaborolan-2-yl)-3,6-dihydropyridine-1(2H)-carboxylate (123 mg, 0.42 mmol), potassium carbonate (144.9 mg, 1.05 mmol), and [1,1'-bis(diphenylphosphino)ferrocene]dichloropalladium(II) dichloromethane complex (17.14 mg, 0.021 mmol) were added to 1,4-dioxane (4 mL) and water (0.5 mL). The reaction mixture was refluxed at 100°C for 2 hours.

After TLC monitoring indicated the disappearance of the starting material, the mixture was cooled. Water (20 mL) was added, and the mixture was extracted twice with ethyl acetate (30 mL). The combined organic phases were washed with saturated brine (20 mL), dried over anhydrous sodium sulfate, filtered, and concentrated to dryness under reduced pressure. The resulting residue was purified by silica gel column chromatography to obtain cyclopropyl 4-(4-(2-((2-chloro-4-(trifluoromethyl)phenyl)amino)-2-oxoethyl)-5-ethyl-6-(4-(5-methoxy-6-methylpyrimidine-4-carbonyl)piperazin-1-yl)-7-oxo-4,7-dihydro-[1,2,4]triazolo[1,5-a]pyrimidin-2-yl)-3,6-dihydropyridine-1(2H)-carboxylate (160 mg).

MS (ESI) M/Z: 799.2 [M+H] ⁺.

**Step D:** Cyclopropyl 4-(4-(2-((2-chloro-4-(trifluoromethyl)phenyl)amino)-2-oxoethyl)-5-ethyl-6-(4-(5-methoxy-6-methylpyrimidine-4-carbonyl)piperazin-1-yl)-7-oxo-4,7-dihydro-[1,2,4]triazolo[1,5-a]pyrimidin-2-yl)-3,6-dihydropyridine-1(2H)-carboxylate (125 mg, 0.16 mmol) and lithium chloride (66.3 mg, 1.6 mmol) were added to anhydrous *N,N-*dimethylformamide (4 mL), and the reaction was carried out at 140°C for 4 hours.

After LCMS monitoring indicated the disappearance of the starting material, the mixture was cooled. The mixture was concentrated to dryness under reduced pressure, and the resulting residue was purified by preparative chromatography to obtain cyclopropyl 4-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)-3,6-dihydropyridine-1(2H)-carboxylate (55.47 mg).

MS (ESI) M/Z: 785.3 [M+H] ⁺.

¹H NMR (400 MHz, DMSO-*d*₆) δ 10.39 (s, 1H), 10.26 (s, 1H), 8.57 (s, 1H), 8.06 (d, *J=* 8.7 Hz, 1H), 7.98 (s, 1H), 7.72 (d, *J =* 8.7 Hz, 1H), 6.90 - 6.66 (m, 1H), 5.32 (s, 2H), 4.59 - 4.43 (m, 1H), 4.13 - 3.93 (m, 3H), 3.59 - 3.42 (m, 5H), 3.28 - 3.20 (m, 1H), 3.07 - 2.90 (m, 3H), 2.84 - 2.76 (m, 1H), 2.69 - 2.61 (m, 1H), 2.58 - 2.53 (m, 2H), 2.44 (s, 3H), 1.18 (t, 3H), 0.82 - 0.74 (m, 2H), 0.65 - 0.63 (m, 2H).

### Example 94

### 4-(4-(2-((2-Chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)-N,N-dimethylcyclohex-3-ene-1-carboxamide

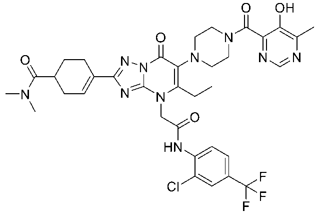

The compound may also be synthesized according to the following steps:

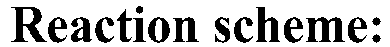

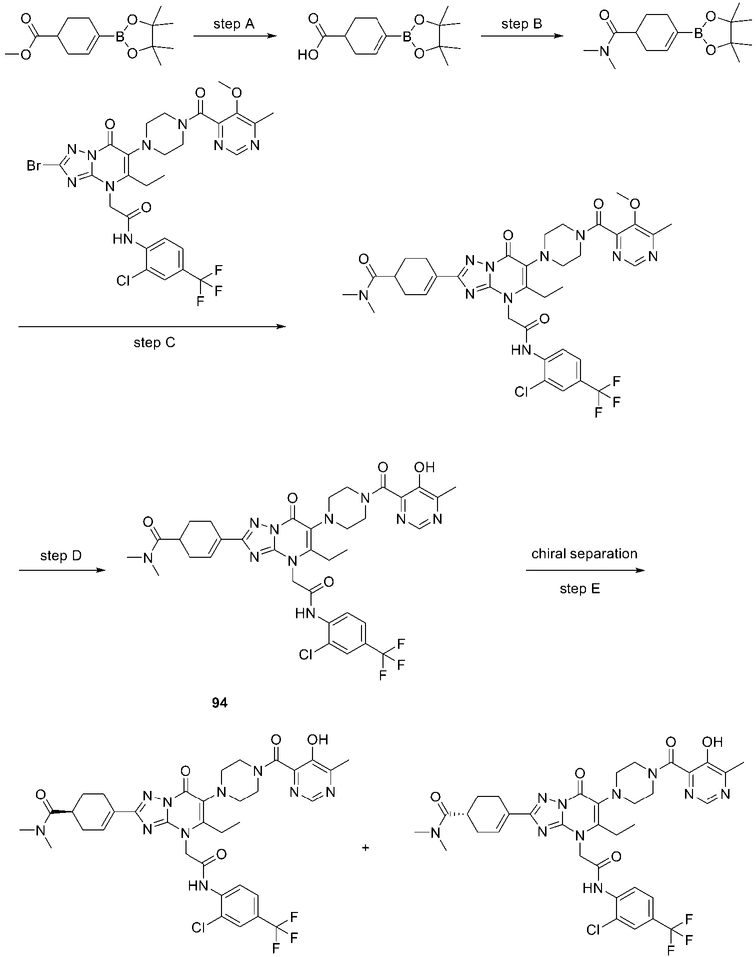

### Procedure:

**Step** A: Methyl 4-(4,4,5,5-tetramethyl-1,3,2-dioxaborolan-2-yl)cyclohex-3-ene-1-carboxylate (500 mg, 1.88 mmol) was dissolved in tetrahydrofuran (5 mL), water (1 mL), and methanol (2.5 mL), and lithium hydroxide monohydrate (315 mg, 7.52 mmol) was added. The reaction was carried out at room temperature overnight.

After TLC monitoring indicated the disappearance of the starting material, the pH of the reaction mixture was adjusted to 5-6 with 2M hydrochloric acid, and the mixture was concentrated to dryness under reduced pressure to obtain crude 4-(4,4,5,5-tetramethyl-1,3,2-dioxaborolan-2-yl)cyclohex-3-ene-1-carboxylic acid, which was used directly in the next step.

MS (ESI) M/Z: 275.1 [M+Na] ⁺.

**Step B:** 4-(4,4,5,5-Tetramethyl-1,3,2-dioxaborolan-2-yl)cyclohex-3-ene-1-carboxylic acid (236 mg, 0.94 mmol) was dissolved in *N,N*-dimethylformamide (6 mL), and dimethylamine hydrochloride (91 mg, 1.12 mmol), *N,N*-diisopropylethylamine (362 mg, 2.81 mmol), and 2-(7-azabenzotriazol-1-yl)-*N,N,N',N*'-tetramethyluronium hexafluorophosphate (462 mg, 1.22 mmol) were added. The reaction mixture was stirred at room temperature overnight.

After TLC monitoring indicated the disappearance of the starting material, water (20 mL) was added, and the mixture was extracted twice with ethyl acetate (30 mL). The combined organic phases were washed with saturated brine (20 mL), dried over anhydrous sodium sulfate, filtered, and concentrated to dryness under reduced pressure. The resulting residue was purified by silica gel column chromatography (eluent: petroleum ether/ethyl acetate = 2:1) to obtain *N,N-*dimethyl-4-(4,4,5,5-tetramethyl-1,3,2-dioxaborolan-2-yl)cyclohex-3-ene-1-carboxamide (200 mg).

MS (ESI) M/Z: 280.2 [M+H] ⁺.

**Step C:** 2-(2-Bromo-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamide (300 mg, 0.42 mmol), N,N-dimethyl-4-(4,4,5,5-tetramethyl-1,3,2-dioxaborolan-2-yl)cyclohex-3-ene-1-carboxamide (106 mg, 0.38 mmol), anhydrous potassium carbonate (145 mg, 1.05 mmol), and [1,1'-bis(diphenylphosphino)ferrocene]dichloropalladium(II) dichloromethane complex (17 mg, 0.02 mmol) were added to 1,4-dioxane (8 mL) and water (1 mL). The reaction system was purged three times with nitrogen, and the reaction mixture was stirred at 100°C for 2 hours.

After LCMS monitoring indicated the disappearance of the starting material, the mixture was cooled. Water (20 mL) was added, and the mixture was extracted twice with ethyl acetate (30 mL). The combined organic phases were washed with saturated brine (20 mL), dried over anhydrous sodium sulfate, filtered, and concentrated to dryness under reduced pressure. The resulting residue was purified by silica gel column chromatography to obtain 4-(4-(2-((2-chloro-4-(trifluoromethyl)phenyl)amino)-2-oxoethyl)-5-ethyl-6-(4-(5-methoxy-6-methylpyrimidine-4-carbonyl)piperazin-1-yl)-7-oxo-4,7-dihydro-[1,2,4]triazolo[1,5-a]pyrimidin-2-yl)-N,N-dimethylcyclohex-3-ene-l-carboxamide (180 mg).

MS (ESI) M/Z: 785.4 [M+H] ⁺.

**Step D:** 4-(4-(2-((2-Chloro-4-(trifluoromethyl)phenyl)amino)-2-oxoethyl)-5-ethyl-6-(4-(5-methoxy-6-methylpyrimidine-4-carbonyl)piperazin-1-yl)-7-oxo-4,7-dihydro-[1,2,4]triazolo[1,5-a]pyrimidin-2-yl)-N,N-dimethylcyclohex-3-ene-1-carboxamide (180 mg, 0.23 mmol) and lithium chloride (97 mg, 2.29 mmol) were added to anhydrous *N,N-*dimethylformamide (8 mL), and the mixture was heated at 140°C and reacted for 4 hours.

After LCMS monitoring indicated the disappearance of the starting material, the mixture was cooled. The mixture was concentrated to dryness under reduced pressure, and the resulting residue was purified by preparative chromatography to obtain 4-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)-N,N-dimethylcyclohex-3-ene-1-carboxamide (63.08 mg).

MS (ESI) M/Z: 771.2 [M+H] ⁺.

¹H NMR (400 MHz, DMSO-*d*₆) δ 10.37 (s, 1H), 10.28 (s, 1H), 8.56 (s, 1H), 8.06 (d, *J= 8.6* Hz, 1H), 8.01 - 7.90 (m, 1H), 7.72 (d, *J =* 8.7 Hz, 1H), 6.88 (s, 1H), 5.32 (s, 2H), 4.60 - 4.44 (m, 1H), 3.58 - 3.44 (m, 3H), 3.26 - 3.20 (m, 1H), 3.05 (s, 3H), 3.02 - 2.94 (m, 3H), 2.93 - 2.76 (m, 6H), 2.68 - 2.60 (m, 2H), 2.44 (s, 3H), 2.35 - 2.26 (m, 2H), 1.95 - 1.85 (m, 1H), 1.61 - 1.51 (m, 1H), 1.19 (t, *J =* 7.5 Hz, 3H).

**Step E:** 4-(4-(2-((2-Chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)-*N,N-*dimethylcyclohex-3-ene-1-carboxamide (60 mg, 0.078 mmol) was separated by preparative chiral HPLC. Preparative column: CHIRALPAK IC (5 _{[}tm, 30 mm I.D. × 250 mm L), mobile phase gradient: 90% acetonitrile/10% water (containing 0.1% trifluoroacetic acid), flow rate: 30 mL/min, to obtain Example 94-peak 1 (16.64 mg; retention time: 46 min) and Example 94-peak 2 (15.24 mg; retention time: 60 min).

### Example 94-peak 1:

MS (ESI) M/Z: 771.5 [M+H] ⁺.

¹H NMR (400 MHz, DMSO-*d*₆) δ 10.37 (s, 1H), 10.21 (s, 1H), 8.56 (s, 1H), 8.06 (d, *J= 8.6* Hz, 1H), 8.01 - 7.90 (m, 1H), 7.72 (d, *J =* 8.7 Hz, 1H), 6.88 (s, 1H), 5.32 (s, 2H), 4.60 - 4.44 (m, 1H), 3.58 - 3.44 (m, 3H), 3.26 - 3.20 (m, 1H), 3.05 (s, 3H), 3.02 - 2.94 (m, 3H), 2.93 - 2.76 (m, 6H), 2.68 - 2.60 (m, 2H), 2.44 (s, 3H), 2.35 - 2.26 (m, 2H), 1.95 - 1.85 (m, 1H), 1.61 - 1.51 (m, 1H), 1.19 (t, *J* = 7.5 Hz, 3H).

### Example 94-peak 2:

MS (ESI) M/Z: 771.5 [M+H] ⁺.

¹H NMR (400 MHz, DMSO-*d*₆) δ 10.39 (s, 1H), 10.24 (s, 1H), 8.56 (s, 1H), 8.06 (d, *J= 8.6* Hz, 1H), 8.01 - 7.90 (m, 1H), 7.72 (d, *J =* 8.7 Hz, 1H), 6.88 (s, 1H), 5.32 (s, 2H), 4.60 - 4.44 (m, 1H), 3.58 - 3.44 (m, 3H), 3.26 - 3.20 (m, 1H), 3.05 (s, 3H), 3.02 - 2.94 (m, 3H), 2.93 - 2.76 (m, 6H), 2.68 - 2.60 (m, 2H), 2.44 (s, 3H), 2.35 - 2.26 (m, 2H), 1.95 - 1.85 (m, 1H), 1.61 - 1.51 (m, 1H), 1.19 (t, *J=* 7.5 Hz, 3H).

### Example 95

### N-(2-Chloro-4-(trifluoromethyl)phenyl)-2-(5-ethyl-6-(4-(5-hydroxy-6-methylpyrimidine-4-carbonyl)piperazin-1-yl)-2-(4-(methylsulfonyl)cyclohex-1-en-1-yl)-7-oxo-[1,2,4]triazolo[1,5-a]pyrimidin-4(7H)-yl)acetamide

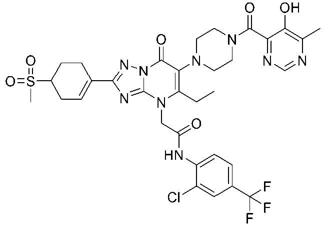

**The compound may also be synthesized according to the following steps:**

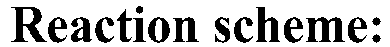

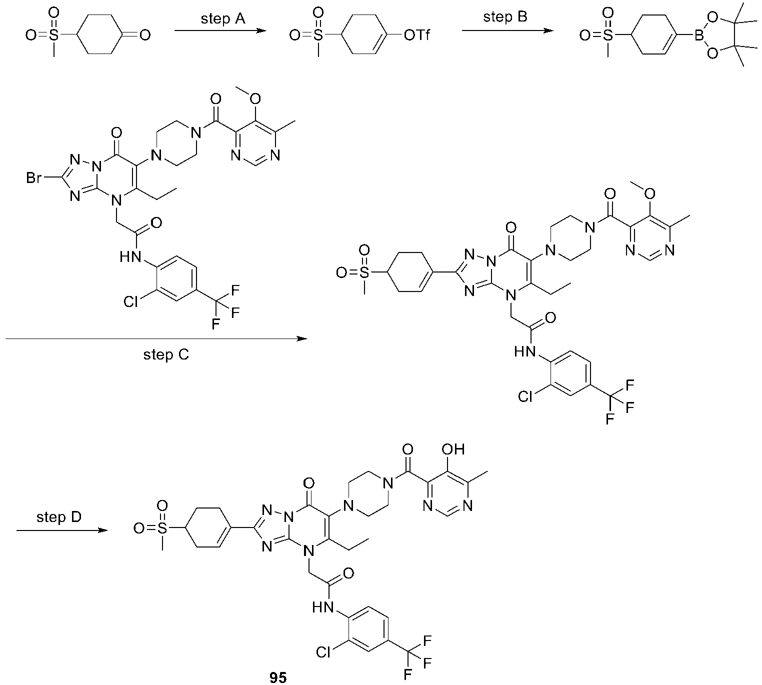

### Procedure:

**Step A:** 4-(Methylsulfonyl)cyclohexan-1-one (200 mg, 1.14 mmol) and *2,6-di-tert-*butyl-4-methylpyridine (513.2 mg, 2.5 mmol) were dissolved in dichloromethane (15 mL). Trifluoromethanesulfonic anhydride (641 mg, 2.27 mmol) was added dropwise in an ice bath, and the reaction was carried out at 40°C overnight.

After TLC monitoring indicated the disappearance of the starting material, the reaction mixture was concentrated to dryness under reduced pressure. Petroleum ether (10 mL) was added, and the mixture was filtered. The filtrate was evaporated to dryness by rotary evaporation, and the resulting residue was purified by silica gel column chromatography to obtain 4-(methylsulfonyl)cyclohex-1-en-1-yl trifluoromethanesulfonate (330 mg).

¹H NMR (400 MHz, CDCl₃) δ 5.84 (s, 1H), 3.19 - 3.06 (m, 1H), 2.97 (s, 3H), 2.76 - 2.51 (m, 4H), 2.44 (m, 1H), 2.10 - 1.94 (m, 1H).

**Step B:** 4-(Methylsulfonyl)cyclohex-1-en-1-yl trifluoromethanesulfonate (330 mg, 1.1 mmol), bis(pinacolato)diboron (328.63 mg, 1.29 mmol), potassium acetate (317.5 mg, 3.24 mmol), and [1,1'-bis(diphenylphosphino)ferrocene]dichloropalladium(II) dichloromethane complex (43.71 mg, 0.054 mmol) were added to 1,4-dioxane (6 mL). The reaction system was purged three times with nitrogen, and the reaction mixture was reacted at 80°C overnight.

After TLC monitoring indicated the disappearance of the starting material, the mixture was cooled. Water (20 mL) was added, and the mixture was extracted twice with ethyl acetate (30 mL). The combined organic phases were washed with saturated brine (20 mL), dried over anhydrous sodium sulfate, filtered, and concentrated under reduced pressure to obtain crude 4,4,5,5-tetramethyl-2-(4-(methylsulfonyl)cyclohex-1-en-1-yl)-1,3,2-dioxaborolane.

**Step** C: 2-(2-Bromo-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamide (250 mg, 0.35 mmol) and 4,4,5,5-tetramethyl-2-(4-(methylsulfonyl)cyclohex-1-en-1-yl)-1,3,2-dioxaborolane (233.82 mg, 0.82 mmol) were dissolved in dioxane (8 mL) and water (2 mL), and potassium carbonate (121.31 mg, 0.88 mmol) and [1,1'-bis(diphenylphosphino)ferrocene]dichloropalladium(II) dichloromethane complex (22.78 mg, 0.028 mmol) were added. The system was evacuated and purged with nitrogen, and the reaction was carried out at 100°C for 3 hours.

After TLC monitoring indicated the disappearance of most of the starting material, water (20 mL) was added to the reaction mixture, and the mixture was extracted twice with ethyl acetate (20 mL). The combined organic phases were washed with saturated brine (20 mL), dried over anhydrous sodium sulfate, filtered, and concentrated to dryness under reduced pressure. The resulting residue was purified by silica gel column chromatography (eluent: dichloromethane:methanol = 20:1) to obtain N-(2-chloro-4-(trifluoromethyl)phenyl)-2-(5-ethyl-6-(4-(5-methoxy-6-methylpyrimidine-4-carbonyl)piperazin-1-yl)-2-(4-(methylsulfonyl)cyclohex-1-en-1-yl)-7-oxo-[ 1,2,4]triazolo [ 1,5-a]pyrimidin-4(7H)-yl)acetamide (180 mg).

MS (ESI) M/Z: 790.2 [M-H] ⁻.

**Step D:** N-(2-Chloro-4-(trifluoromethyl)phenyl)-2-(5-ethyl-6-(4-(5-methoxy-6-methylpyrimidine-4-carbonyl)piperazin-1-yl)-2-(4-(methylsulfonyl)cyclohex-1-en-1-yl)-7-oxo-[1,2,4]triazolo[1,5-a]pyrimidin-4(7H)-yl)acetamide (180 mg, 0.23 mmol) and lithium chloride (96.34 mg, 2.27 mmol) were added to anhydrous N,N-dimethylformamide (4 mL), and the mixture was heated at 140°C and reacted for 4 hours.

After LCMS monitoring indicated the disappearance of the starting material, the mixture was cooled. The mixture was concentrated to dryness under reduced pressure, and the resulting residue was purified by preparative chromatography to obtain N-(2-chloro-4-(trifluoromethyl)phenyl)-2-(5-ethyl-6-(4-(5-hydroxy-6-methylpyrimidine-4-carbonyl)piperazin-1-yl)-2-(4-(methylsulfonyl)cyclohex-1-en-1-yl)-7-oxo-[1,2,4]triazolo[1,5-a]pyrimidin-4(7H)-yl)acetamide (26.15 mg).

MS (ESI) M/Z: 778.2 [M+H] ⁺.

¹H NMR (400 MHz, DMSO-*d*₆) δ 10.54 (s, 2H), 8.44 (s, 1H), 8.04 (d, *J* = 8.6 Hz, 1H), 7.97 (s, 1H), 7.78 - 7.65 (m, 1H), 6.93 - 6.78 (m, 1H), 5.34 (s, 2H), 4.57 - 4.46 (m, 1H), 3.55 - 3.44 (m, 3H), 3.26 - 3.22 (m, 1H), 3.03 - 2.91 (m, 6H), 2.85 - 2.75 (m, 2H), 2.70 - 2.56 (m, 3H), 2.41 (s, 3H), 2.35 - 2.28 (m, 2H), 1.76 - 1.51 (m, 2H), 1.19 - 1.16 (m, 3H).

### Example 109

### 4-(4-(2-((2-Chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)-N-methylcyclohex-3-ene-1-carboxamide

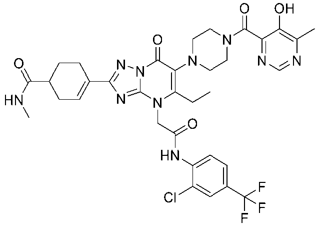

**The compound may also be synthesized according to the following steps:**

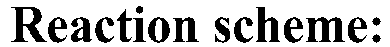

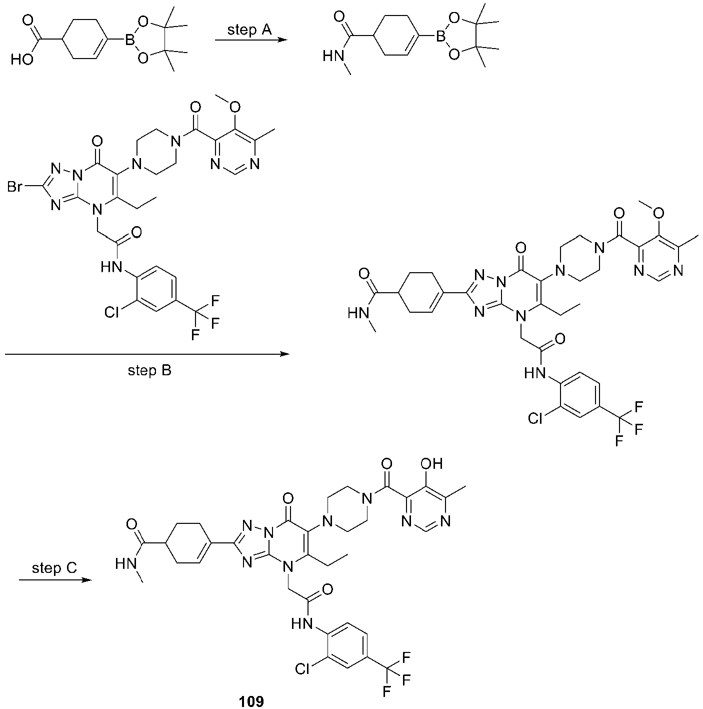

### Procedure:

**Step A:** 4-(4,4,5,5-Tetramethyl-1,3,2-dioxaborolan-2-yl)cyclohex-3-ene-1-carboxylic acid (236 mg, 0.94 mmol) was dissolved in *N,N*-dimethylformamide (6 mL), and methylamine hydrochloride (75 mg, 1.12 mmol), *N,N-*diisopropylethylamine (362 mg, 2.81 mmol), and 2-(7-azabenzotriazol-1-yl)-*N,N,N',N*'-tetramethyluronium hexafluorophosphate (462 mg, 1.22 mmol) were added. The reaction mixture was stirred at room temperature overnight.

After TLC monitoring indicated the disappearance of the starting material, water (20 mL) was added, and the mixture was extracted twice with ethyl acetate (30 mL). The combined organic phases were washed with saturated brine (20 mL), dried over anhydrous sodium sulfate, filtered, and concentrated to dryness under reduced pressure. The resulting residue was purified by silica gel column chromatography to obtain N-methyl-4-(4,4,5,5-tetramethyl-1,3,2-dioxaborolan-2-yl)cyclohex-3-ene-1-carboxamide (150 mg).

MS (ESI) M/Z: 266.2 [M+H] ⁺.

**Step B:** 2-(2-Bromo-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-*N*-(2-chloro-4-(trifluoromethyl)phenyl)acetamide (300 mg, 0.42 mmol), *N*-methyl-4-(4,4,5,5-tetramethyl-1,3,2-dioxaborolan-2-yl)cyclohex-3-ene-1-carboxamide (111 mg, 0.42 mmol), anhydrous potassium carbonate (145 mg, 1.05 mmol), and [1,1'-bis(diphenylphosphino)ferrocene]dichloropalladium(II) dichloromethane complex (17 mg, 0.02 mmol) were added to 1,4-dioxane (8 mL) and water (1 mL). The reaction system was purged three times with nitrogen, and the reaction mixture was stirred at 100°C for 2 hours.

After LCMS monitoring indicated the disappearance of the starting material, the mixture was cooled. Water (20 mL) was added, and the mixture was extracted twice with ethyl acetate (30 mL). The combined organic phases were washed with saturated brine (20 mL), dried over anhydrous sodium sulfate, filtered, and concentrated to dryness under reduced pressure. The resulting residue was purified by silica gel column chromatography to obtain 4-(4-(2-((2-chloro-4-(trifluoromethyl)phenyl)amino)-2-oxoethyl)-5-ethyl-6-(4-(5-methoxy-6-methylpyrimidine-4-carbonyl)piperazin-1-yl)-7-oxo-4,7-dihydro-[1,2,4]triazolo[1,5-a]pyrimidin-2-yl)-N-methylcyclohex-3-ene-1-carboxamide (200 mg).

MS (ESI) M/Z: 771.4 [M+H] ⁺.

**Step C:** 4-(4-(2-((2-Chloro-4-(trifluoromethyl)phenyl)amino)-2-oxoethyl)-5-ethyl-6-(4-(5-methoxy-6-methylpyrimidine-4-carbonyl)piperazin-1-yl)-7-oxo-4,7-dihydro-[1,2,4]triazolo[1,5-a]pyrimidin-2-yl)-N-methylcyclohex-3-ene-1-carboxamide (200 mg, 0.26 mmol) and lithium chloride (109 mg, 2.59 mmol) were added to anhydrous *N,N-*dimethylformamide (8 mL), and the mixture was heated at 140°C and reacted for 4 hours.

After LCMS monitoring indicated the disappearance of the starting material, the mixture was cooled. The mixture was concentrated to dryness under reduced pressure, and the resulting residue was purified by preparative chromatography to obtain 4-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)-N-methylcyclohex-3-ene-1-carboxamide (69.22 mg).

MS (ESI) M/Z: 757.3 [M+H] ⁺.

¹H NMR (400 MHz, DMSO-d₆) δ 10.39 (s, 1H), 10.23 (s, 1H), 8.59 (d, *J* = 15.1 Hz, 1H), 8.13 - 7.93 (m, 2H), 7.88 - 7.66 (m, 2H), 6.88 (d, *J =* 13.7 Hz, 1H), 5.33 (m, 2H), 4.52 (m, 1H), 3.58 - 3.43 (m, 3H), 3.37 (s, 3H), 3.30 - 3.20 (m, 1H), 3.07 - 2.90 (m, 3H), 2.88 - 2.75 (m, 1H), 2.64 - 2.56 (m, 4H), 2.43 (s, 3H), 2.37 - 2.32 (m, 2H), 2.00 - 1.86 (m, 1H), 1.68 - 1.55 (m, 1H), 1.27 - 1.12 (m, 3H).

### Example 60

### 4-(4-(2-((2-Chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)-N,N-dimethyl-3,6-dihydropyridine-1(2H)-carboxamide

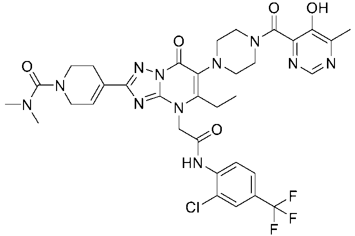

**The compound may also be synthesized according to the following steps:**

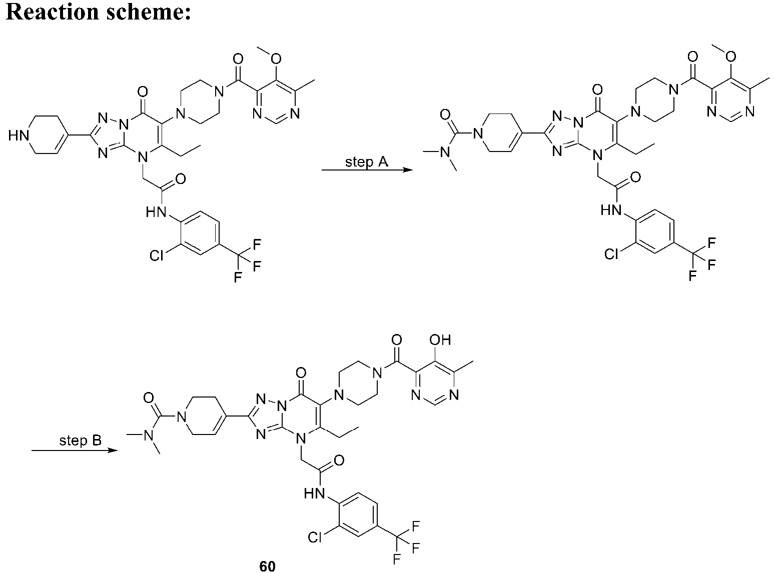

### Procedure:

**Step A:** *N*-(2-Chloro-4-(trifluoromethyl)phenyl)-2-(5-ethyl-6-(4-(5-methoxy-6-methylpyrimidine-4-carbonyl)piperazin-1-yl)-7-oxo-2-(1,2,3,6-tetrahydropyridin-4-yl)-[1,2,4]triazolo[1,5-a]pyrimidin-4(7H)-yl)acetamide (90 mg, 0.13 mmol) was dissolved in dichloromethane (5 mL), and triethylamine (25.5 mg, 0.25 mmol) and dimethylcarbamoyl chloride (20.3 mg, 0.19 mmol) were added. The reaction was carried out at room temperature overnight.

After TLC monitoring indicated the disappearance of the starting material, dilute hydrochloric acid (0.5 M, 10 mL) was added to the reaction mixture, and the mixture was extracted with dichloromethane (20 mL). The organic phase was washed sequentially with aqueous sodium bicarbonate solution and saturated brine, dried over anhydrous sodium sulfate, filtered, and concentrated under reduced pressure to obtain crude 4-(4-(2-((2-chloro-4-(trifluoromethyl)phenyl)amino)-2-oxoethyl)-5-ethyl-6-(4-(5-methoxy-6-methylpyrimidine-4-carbonyl)piperazin-1-yl)-7-oxo-4,7-dihydro-[1,2,4]triazolo[1,5-a]pyrimidin-2-yl)-*N,N-*dimethyl-3,6-dihydropyridine-1(2H)-carboxamide, which was used directly in the next step.

MS (ESI) M/Z: 784.0 [M-H] ⁻.

**Step B:** Crude 4-(4-(2-((2-chloro-4-(trifluoromethyl)phenyl)amino)-2-oxoethyl)-5-ethyl-6-(4-(5-methoxy-6-methylpyrimidine-4-carbonyl)piperazin-1-yl)-7-oxo-4,7-dihydro-[1,2,4]triazolo[1,5-a]pyrimidin-2-yl)-*N,N*-dimethyl-3,6-dihydropyridine-1(2H)-carboxamide (0.13 mmol) and lithium chloride (53.4 mg, 1.3 mmol) were added to anhydrous *N,N-*dimethylformamide (4 mL), and the mixture was heated at 140°C and reacted for 4 hours.

After LCMS monitoring indicated the disappearance of the starting material, the mixture was cooled. The mixture was concentrated to dryness under reduced pressure, and the resulting residue was purified by preparative chromatography to obtain 4-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)-*N,N'-*dimethyl-3,6-dihydropyridine-1(2H)-carboxamide (45.93 mg).

MS (ESI) M/Z: 772.2 [M+H] ⁺.

¹H NMR (400 MHz, DMSO-d₆) δ 10.38 (s, 1H), 10.24 (s, 1H), 8.58 (s, 1H), 8.06 (d, *J* = 8.7 Hz, 1H), 7.98 (s, 1H), 7.72 (d, *J* = 8.8 Hz, 1H), 6.86 - 6.73 (m, 1H), 5.32 (s, 2H), 4.57 - 4.46 (m, 1H), 3.87 (s, 2H), 3.53 - 3.43 (m, 3H), 3.26 - 3.22 (m, 1H), 3.03 - 2.94 (m, 3H), 2.85 - 2.79 (m, 1H), 2.75 (s, 6H), 2.66 - 2.62 (m, 1H), 2.59 - 2.55 (m, 2H), 2.44 (s, 3H), 2.37 - 2.30 (m, 2H), 1.24 - 1.18 (m, 3H).

### Example 140

### Cyclobutyl 4-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)-3,6-dihydropyridine-1(2H)-carboxylate

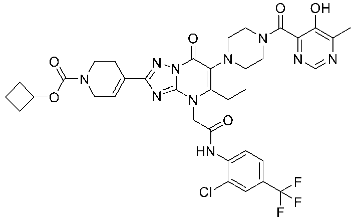

**The compound may also be synthesized according to the following steps:**

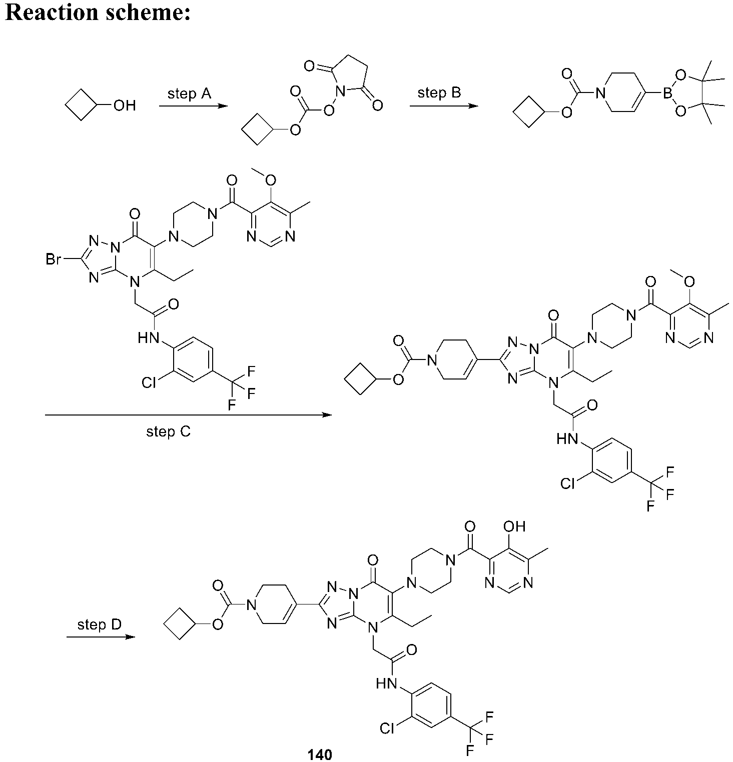

### Procedure:

**Step A:** Cyclobutanol (200 mg, 2.77 mmol) was dissolved in acetonitrile (5 mL), and N,N'-disuccinimidyl carbonate (781.3 mg, 3.05 mmol) and triethylamine (420.4 mg, 4.16 mmol) were added. The reaction was carried out at room temperature overnight.

After TLC monitoring indicated the disappearance of the starting material, aqueous sodium bicarbonate solution (20 mL) was added, and the mixture was extracted twice with dichloromethane (20 mL). The combined organic phases were washed with saturated brine, dried over anhydrous sodium sulfate, and filtered. The filtrate was concentrated to dryness under reduced pressure to obtain crude cyclobutyl (2,5-dioxopyrrolidin-1-yl) carbonate (400 mg), which was used directly in the next step.

**Step B:** 4-(4,4,5,5-Tetramethyl-1,3,2-dioxaborolan-2-yl)-1,2,3,6-tetrahydropyridine hydrochloride (200 mg, 0.81 mmol), crude cyclobutyl (2,5-dioxopyrrolidin-1-yl) carbonate (260.2 mg, 1.22 mmol), and triethylamine (247.1 mmol, 2.44 mg) were added to dichloromethane (5 mL), and the reaction was carried out at room temperature overnight.

After LCMS monitoring indicated the disappearance of the starting material, the mixture was diluted with dichloromethane (20 mL), and the reaction mixture was washed sequentially with dilute hydrochloric acid (15 mL), aqueous sodium bicarbonate solution (15 mL), and saturated brine (15 mL), dried over anhydrous sodium sulfate, and filtered. The filtrate was concentrated to dryness under reduced pressure to obtain crude cyclobutyl 4-(4,4,5,5-tetramethyl-1,3,2-dioxaborolan-2-yl)-3,6-dihydropyridine-1(2H)-carboxylate, which was used directly in the next step.

MS (ESI) M/Z: 308.1 [M+H] ⁺.

**Step C:** 2-(2-Bromo-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-*N*-(2-chloro-4-(trifluoromethyl)phenyl)acetamide (200 mg, 0.28 mmol), cyclobutyl 4-(4,4,5,5-tetramethyl-1,3,2-dioxaborolan-2-yl)-3,6-dihydropyridine-1(2H)-carboxylate (86.4 mg, 0.28 mmol), anhydrous potassium carbonate (96.6 mg, 0.7 mmol), and [1,1'-bis(diphenylphosphino)ferrocene]dichloropalladium(II) dichloromethane complex (11.3 mg, 0.014 mmol) were added to 1,4-dioxane (4 mL) and water (0.5 mL). The reaction system was purged three times with nitrogen, and the reaction mixture was stirred at 100°C for 2 hours.

After TLC monitoring indicated the disappearance of the starting material, the mixture was cooled. Water (20 mL) was added, and the mixture was extracted twice with ethyl acetate (30 mL). The combined organic phases were washed with saturated brine (20 mL), dried over anhydrous sodium sulfate, and filtered. The filtrate was concentrated to dryness under reduced pressure, and the resulting residue was purified by silica gel column chromatography to obtain cyclobutyl 4-(4-(2-((2-chloro-4-(trifluoromethyl)phenyl)amino)-2-oxoethyl)-5-ethyl-6-(4-(5-methoxy-6-methylpyrimidine-4-carbonyl)piperazin-1-yl)-7-oxo-4,7-dihydro-[1,2,4]triazolo[1,5-a]pyrimidin-2-yl)-3,6-dihydropyridine-1(2H)-carboxylate (130 mg).

MS (ESI) M/Z: 813.4 [M+H] ⁺.

**Step D:** Cyclobutyl 4-(4-(2-((2-chloro-4-(trifluoromethyl)phenyl)amino)-2-oxoethyl)-5-ethyl-6-(4-(5-methoxy-6-methylpyrimidine-4-carbonyl)piperazin-1-yl)-7-oxo-4,7-dihydro-[1,2,4]triazolo[1,5-a]pyrimidin-2-yl)-3,6-dihydropyridine-1(2H)-carboxylate (130 mg, 0.16 mmol) and lithium chloride (68 mg, 1.6 mmol) were added to anhydrous *N,N-*dimethylformamide (4 mL), and the mixture was heated at 140°C and reacted for 4 hours.

After LCMS monitoring indicated the disappearance of the starting material, the mixture was cooled. The mixture was concentrated to dryness under reduced pressure, and the resulting residue was purified by preparative chromatography to obtain cyclobutyl 4-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)-3,6-dihydropyridine-1(2H)-carboxylate (38.17 mg).

MS (ESI) M/Z: 799.5 [M+H] ⁺.

¹H NMR (400 MHz, DMSO-d₆) δ 10.40 (s, 1H), 10.25 (s, 1H), 8.56 (s, 1H), 8.14 - 8.01 (m, 1H), 7.98 (s, 1H), 7.72 (d, *J =* 8.7 Hz, 1H), 6.87 - 6.72 (m, 1H), 5.32 (s, 2H), 4.92 - 4.78 (m, 1H), 4.57 - 4.45 (m, 1H), 4.15 - 3.99 (m, 2H), 3.63 - 3.53 (m, 2H), 3.52 - 3.43 (m, 3H), 3.27 - 3.21 (m, 1H), 3.07 - 2.90 (m, 3H), 2.86 - 2.76 (m, 1H), 2.69 - 2.61 (m, 1H), 2.58 - 2.54 (m, 2H), 2.44 (s, 3H), 2.28 - 2.22 (m, 2H), 2.04 - 1.96 (m, 2H), 1.74 - 1.68 (m, 1H), 1.59 - 1.52 (m, 1H), 1.17 (t, *J =* 7.9 Hz, 3H).

### Example 141

### Cyclopentyl 4-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)-3,6-dihydropyridine-1(2H)-carboxylate

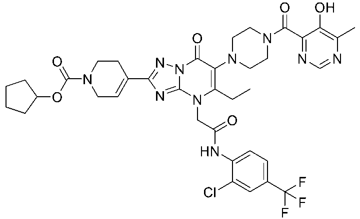

**The compound may also be synthesized according to the following steps:**

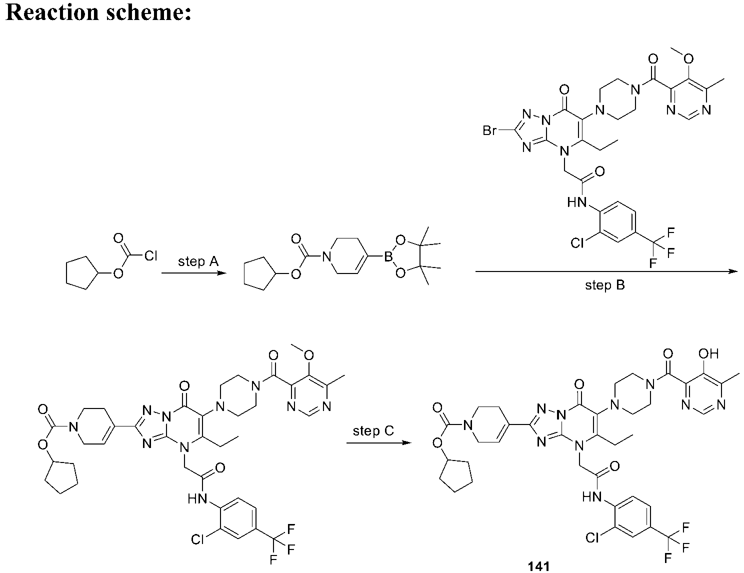

### Procedure:

**Step A:** 4-(4,4,5,5-Tetramethyl-1,3,2-dioxaborolan-2-yl)-1,2,3,6-tetrahydropyridine hydrochloride (200 mg, 0.81 mmol) was dissolved in dichloromethane (5 mL), and triethylamine (246 mg, 2.43 mmol) and cyclopentyl chloroformate (181.5 mg, 1.22 mmol) were added. The reaction was carried out at room temperature overnight.

After TLC monitoring indicated the disappearance of the starting material, dichloromethane (20 mL) was added, and the mixture was washed sequentially with dilute hydrochloric acid (15 mL), aqueous sodium bicarbonate solution (15 mL), and saturated brine (15 mL), dried over anhydrous sodium sulfate, and filtered. The filtrate was concentrated to dryness under reduced pressure to obtain crude cyclopentyl 4-(4,4,5,5-tetramethyl-1,3,2-dioxaborolan-2-yl)-3,6-dihydropyridine-1(2H)-carboxylate, which was used directly in the next step.

MS (ESI) M/Z: 322.1 [M+H]⁺.

**Step B:** 2-(2-Bromo-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-*N*-(2-chloro-4-(trifluoromethyl)phenyl)acetamide (200 mg, 0.28 mmol), crude cyclopentyl 4-(4,4,5,5-tetramethyl-1,3,2-dioxaborolan-2-yl)-3,6-dihydropyridine-1(2H)-carboxylate (90.3 mg, 0.28 mmol), anhydrous potassium carbonate (96.6 mg, 0.7 mmol), and [1,1'-bis(diphenylphosphino)ferrocene]dichloropalladium(II) dichloromethane complex (11.3 mg, 0.014 mmol) were added to 1,4-dioxane (4 mL) and water (0.5 mL). The reaction system was purged three times with nitrogen, and the reaction mixture was stirred at 100°C for 2 hours.

After TLC monitoring indicated the disappearance of the starting material, the mixture was cooled. Water (20 mL) was added, and the mixture was extracted twice with ethyl acetate (30 mL). The combined organic phases were washed with saturated brine (20 mL), dried over anhydrous sodium sulfate, filtered, and concentrated under reduced pressure to dryness to obtain crude cyclopentyl 4-(4-(2-((2-chloro-4-(trifluoromethyl)phenyl)amino)-2-oxoethyl)-5-ethyl-6-(4-(5-methoxy-6-methylpyrimidine-4-carbonyl)piperazin-1-yl)-7-oxo-4,7-dihydro-[1,2,4]triazolo[1,5-a]pyrimidin-2-yl)-3,6-dihydropyridine-1(2H)-carboxylate, which was used directly in the next step.

MS (ESI) M/Z: 827.3 [M+H] ⁺.

**Step C:** Crude cyclopentyl 4-(4-(2-((2-chloro-4-(trifluoromethyl)phenyl)amino)-2-oxoethyl)-5-ethyl-6-(4-(5-methoxy-6-methylpyrimidine-4-carbonyl)piperazin-1-yl)-7-oxo-4,7-dihydro-[1,2,4]triazolo[1,5-a]pyrimidin-2-yl)-3,6-dihydropyridine-1(2H)-carboxylate (0.28 mmol) and lithium chloride (118.7 mg, 2.8 mmol) were added to anhydrous *N,N-*dimethylformamide (4 mL), and the mixture was heated at 140°C and reacted for 4 hours.

After LCMS monitoring indicated the disappearance of the starting material, the mixture was cooled. The mixture was concentrated to dryness under reduced pressure, and the resulting residue was purified by preparative chromatography to obtain cyclopentyl 4-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)-3,6-dihydropyridine-1(2H)-carboxylate (47.37 mg).

MS (ESI) M/Z: 811.3 [M-H]⁻.

¹H NMR (400 MHz, DMSO-d₆) δ 10.39 (s, 1H), 10.24 (s, 1H), 8.58 (s, 1H), 8.06 (d, *J =* 8.6 Hz, 1H), 7.98 (s, 1H), 7.72 (d, *J =* 8.7 Hz, 1H), 6.87 - 6.72 (m, 1H), 5.32 (s, 2H), 5.07 - 4.94 (m, 1H), 4.58 - 4.46 (m, 1H), 4.07 (s, 2H), 3.58 - 3.53 (m, 2H), 3.52 - 3.44 (m, 3H), 3.28 - 3.21 (m, 1H), 3.02 - 2.93 (m, 3H), 2.84 - 2.79 (m, 1H), 2.66 - 2.62 (m, 1H), 2.56 - 2.53 (m, 2H), 2.44 (s, 3H), 1.83 - 1.76 (m, 2H), 1.68 - 1.61 (m, 4H), 1.57 - 1.52 (m, 2H), 1.20 - 1.17 (m, 3H).

### Example 66

### N-(2-Chloro-4-(trifluoromethyl)phenyl)-2-(5-ethyl-6-(4-(5-hydroxy-6-methylpyrimidine-4-carbonyl)piperazin-1-yl)-7-oxo-2-(2,5,6,7-tetrahydrooxepin-4-yl)-[1,2,4]triazolo[1,5-a]pyrimidin-4(7H)-yl)acetamide

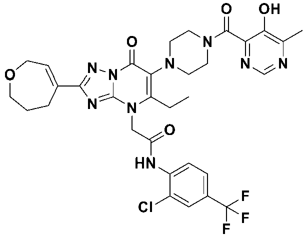

**The compound may also be synthesized according to the following steps:**

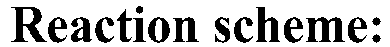

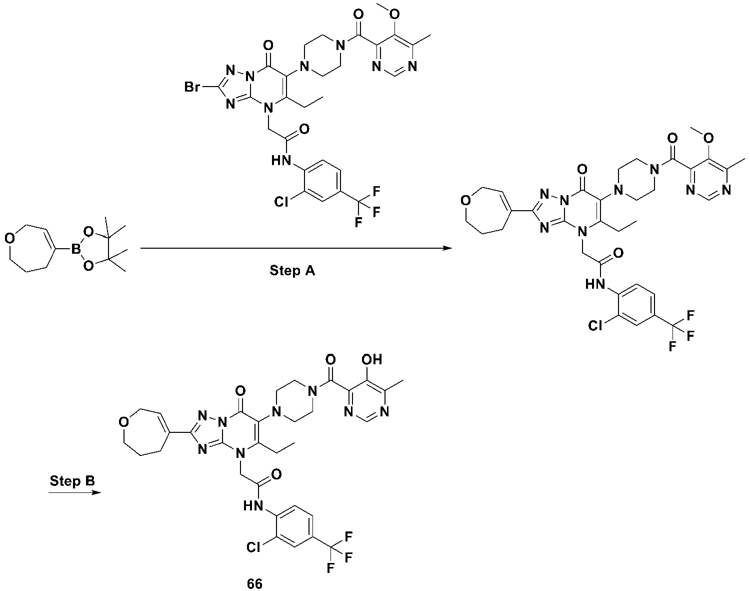

### Procedure:

**Step A:** 2-(2-Bromo-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-*N*-(2-chloro-4-(trifluoromethyl)phenyl)acetamide (90 mg, 0.123 mmol) was dissolved in 1,4-dioxane (1.6 mL)/water (0.2 mL), and 4,4,5,5-tetramethyl-2-(2,5,6,7-tetrahydrooxepin-4-yl)-1,3,2-dioxaborolane (110 mg, 0.492 mmol), PdCl₂(dppf) (9 mg, 0.0123 mmol), and potassium carbonate (51 mg, 0.369 mol) were added. The reaction system was purged with nitrogen, and the mixture was stirred at 100°C for 2 hours.

After LCMS detection indicated the disappearance of the starting material and the formation of the product, the reaction mixture was filtered, and the filtrate was directly concentrated. The resulting mixture was purified by thin-layer chromatography on a silica gel plate to obtain N-(2-chloro-4-(trifluoromethyl)phenyl)-2-(5-ethyl-6-(4-(5-methoxy-6-methylpyrimidine-4-carbonyl)piperazin-1-yl)-7-oxo-2-(2,5,6,7-tetrahydrooxepin-4-yl)-[1,2,4]triazolo[1,5-a]pyrimidin-4(7H)-yl)acetamide (60 mg).

MS (ESI) M/Z: 730.1 [M+H]⁺.

**Step B**: *N*-(2-Chloro-4-(trifluoromethyl)phenyl)-2-(5-ethyl-6-(4-(5-methoxy-6-methylpyrimidine-4-carbonyl)piperazin-1-yl)-7-oxo-2-(2,5,6,7-tetrahydrooxepin-4-yl)-[1,2,4]triazolo[1,5-a]pyrimidin-4(7H)-yl)acetamide (60 mg, 0.082 mmol) was dissolved in dimethyl sulfoxide (1.5 mL), and lithium chloride (83 mg, 1.973 mmol) was added. The reaction system was purged with nitrogen, and the mixture was stirred at 150°C for 5 hours.

After LCMS detection indicated the disappearance of the starting material and the formation of the product, the reaction mixture was filtered, and the resulting residue was purified by preparative high-performance liquid chromatography. The purification conditions were as follows: preparative column welch XBC18, 21.2 × 150 mm, 5 µm (particle size); mobile phase: water (containing 10 mmol/L ammonium bicarbonate) and acetonitrile; flow rate: 20 mL/min; gradient: acetonitrile from 40% to 60% within 10 minutes; detection wavelength: 214 nm. The product was collected and lyophilized under reduced pressure. *N*-(2-Chloro-4-(trifluoromethyl)phenyl)-2-(5-ethyl-6-(4-(5-hydroxy-6-methylpyrimidine-4-carbonyl)piperazin-1-yl)-7-oxo-2-(2,5,6,7-tetrahydrooxepin-4-yl)-[1,2,4]triazolo[1,5-a]pyrimidin-4(7H)-yl)acetamide (11.06 mg) was obtained.

MS (ESI) M/Z: 716.2 [M+H]⁺.

¹H NMR (400 MHz, DMSO-d₆) δ 10.46 (s, 1H), 10.03 (s, 1H), 8.57 (s, 1H), 8.06 (d, *J* = 8.3 Hz, 1H), 7.97 (s, 1H), 7.72 (d, *J =* 9.0 Hz, 1H), 6.91 (t, *J* = 4.7 Hz, 1H), 5.31 (s, 2H), 4.57 - 4.46 (m, 1H), 4.27 (d, *J =* 4.7 Hz, 2H), 3.81 (t, *J* = 5.8 Hz, 2H), 3.53 - 3.45 (m, 3H), 3.26 - 3.20 (m, 1H), 3.02 - 2.96 (m, 3H), 2.87 - 2.79 (m, 3H), 2.66 - 2.61 (m, 1H), 2.44 (s, 3H), 1.90 - 1.82 (m, 2H), 1.18 (t, *J* = 7.5 Hz, 3H).

### Example 90

### N-(2-Chloro-4-(trifluoromethyl)phenyl)-2-(2-(4-cyclopropoxycyclohex-1-en-1-yl)-5-ethyl-6-(4-(5-hydroxy-6-methylpyrimidine-4-carbonyl)piperazin-1-yl)-7-oxo-[1,2,4] triazolo [1,5-a] pyrimidin-4(7H)-yl)acetamide

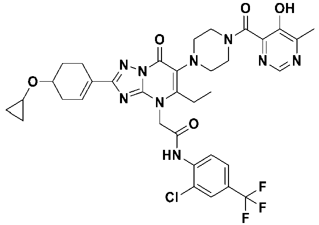

**The compound may also be synthesized according to the following steps:**

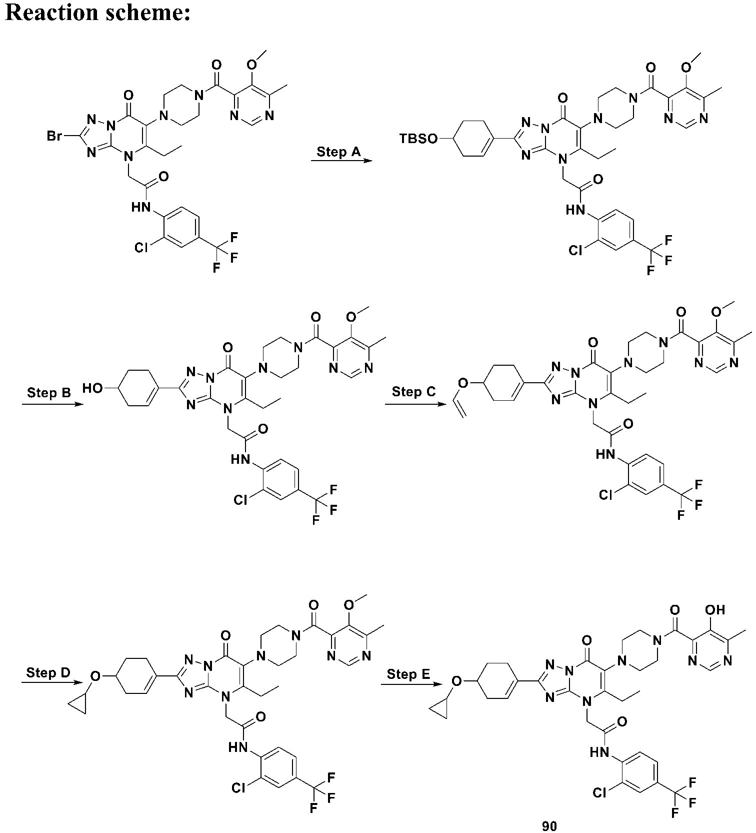

### Procedure:

**Step A:** 2-(2-Bromo-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-*N*-(2-chloro-4-(trifluoromethyl)phenyl)acetamide (200 mg, 0.28 mmol) was dissolved in 1,4-dioxane (3.2 mL)/water (0.4 mL), and tert-butyldimethyl((4-(4,4,5,5-tetramethyl-1,3,2-dioxaborolan-2-yl)cyclohex-3-en-1-yl)oxy)silane (190 mg, 0.56 mmol), PdCl₂(dppf).DCM (23 mg, 0.028 mmol), and potassium carbonate (97 mg, 0.70 mol) were added. The reaction system was purged with nitrogen, and the mixture was stirred at 100°C for 2 hours.

After LCMS detection indicated the disappearance of the starting material and the formation of the product, the reaction mixture was filtered, and the filtrate was directly concentrated. The resulting mixture was purified by silica gel column chromatography to obtain 2-(2-(4-((*tert*-butyldimethylsilyl)oxy)cyclohex-1-en-1-yl)-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-*N*-(2-chloro-4-(trifluoromethyl)phenyl)acetamide (256 mg).

MS (ESI) M/Z: 784.3 [M-60+H]⁻.

**Step B:** In a single-neck flask, 2-(2-(4-((*tert*-butyldimethylsilyl)oxy)cyclohex-1-en-1-yl)-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-*N*-(2-chloro-4-(trifluoromethyl)phenyl)acetamide (256 mg, 0.30 mmol) was dissolved in 1,4-dioxane (3 mL), and a solution of hydrogen chloride in dioxane (4N, 1 mL) was added. The reaction mixture was stirred at room temperature for 2 hours.

After LCMS detection indicated the disappearance of the starting material and the formation of the product, the reaction mixture was cooled to room temperature, and the pH was adjusted to basic by adding saturated aqueous sodium bicarbonate solution. The mixture was extracted with ethyl acetate (40 mL ×3). The combined organic phases were washed with saturated brine (20 mL), dried over anhydrous sodium sulfate, filtered, and concentrated under reduced pressure. The resulting mixture was purified by silica gel column chromatography to obtain *N*-(2-chloro-4-(trifluoromethyl)phenyl)-2-(5-ethyl-2-(4-hydroxycyclohex-1-en-1-yl)-6-(4-(5-methoxy-6-methylpyrimidine-4-carbonyl)piperazin-1-yl)-7-oxo-[1,2,4]triazolo[1,5-a]pyrimidin-4(7H)-yl)acetamide (153 mg).

MS (ESI) M/Z: 730.3 [M+H]⁺.

¹H NMR (400 MHz, DMSO-d₆) δ 10.35 (s, 1H), 8.81 (s, 1H), 8.06 (d, *J* = 8.5 Hz, 1H), 7.97 (d, *J* = 1.5 Hz, 1H), 7.75 - 7.66 (m, 1H), 6.74 (s, 1H), 5.30 (s, 2H), 4.73 (s, 1H), 4.53 (d, *J* = 12.2 Hz, 1H), 3.87 (s, 3H), 3.79 (m, 1H), 3.55 - 3.42 (m, 2H), 3.27 - 3.22 (m, 1H), 3.08 - 2.91 (m, 3H), 2.88 - 2.78 (m, 1H), 2.65 - 2.55 (m, 2H), 2.50 (s, 3H), 2.47 - 2.35 (m, 3H), 2.14 - 2.01 (m, 1H), 1.93 - 1.78 (m, 1H), 1.65 - 1.50 (m, 1H), 1.18 (t, *J* = 7.6, 6.6 Hz, 3H).

**Step C:** In a sealed tube, 1,10-phenanthroline (8 mg, 0.036 mmol) and palladium acetate (8 mg, 0.030 mmol) were dissolved in n-propyl vinyl ether (2 mL). The system was purged with nitrogen and stirred at room temperature for ten minutes. Then, *N*-(2-chloro-4-(trifluoromethyl)phenyl)-2-(5-ethyl-2-(4-hydroxycyclohex-1-en-1-yl)-6-(4-(5-methoxy-6-methylpyrimidine-4-carbonyl)piperazin-1-yl)-7-oxo-[1,2,4]triazolo[1,5-a]pyrimidin-4(7H)-yl)acetamide (153 mg, 0.21 mmol) and triethylamine (21 mg, 0.21 mmol) were added to the reaction system. The reaction system was purged with nitrogen, and the mixture was stirred at 75°C overnight.

After LCMS detection indicated the formation of the product, the reaction mixture was filtered, and the filtrate was directly concentrated. The resulting mixture was purified by silica gel column chromatography to obtain *N*-(2-chloro-4-(trifluoromethyl)phenyl)-2-(5-ethyl-6-(4-(5-methoxy-6-methylpyrimidine-4-carbonyl)piperazin-1-yl)-7-oxo-2-(4-(vinyloxy)cyclohex-1-en-1-yl)-[1,2,4]triazolo[1,5-a]pyrimidin-4(7H)-yl)acetamide (64 mg).

MS (ESI) M/Z: 756.2 [M+H]⁺.

**Step D:** In a 100 mL three-neck flask, a solution of diethylzinc (0.24 mL, 0.48 mmol) in dichloromethane (1 mL) was added. The system was purged with nitrogen, and the mixture was cooled to -5°C in an ice-salt bath. A solution of diiodomethane (128 mg, 0.48 mmol) in dichloromethane (0.1 mL) was added. The reaction mixture was stirred at this temperature for ten minutes. Then, a solution of N-(2-chloro-4-(trifluoromethyl)phenyl)-2-(5-ethyl-6-(4-(5-methoxy-6-methylpyrimidine-4-carbonyl)piperazin-1-yl)-7-oxo-2-(4-(vinyloxy)cyclohex-1-en-1-yl)-[1,2,4]triazolo[1,5-a]pyrimidin-4(7H)-yl)acetamide (64 mg, 0.08 mmol) in dichloromethane (0.5 mL) was added dropwise to the reaction system. The reaction system was stirred at this temperature for another 3 hours.

After LCMS detection indicated the disappearance of the starting material and the formation of the product, the reaction was quenched by adding saturated aqueous ammonium chloride solution, and extracted with ethyl acetate (100 mL ×3). The combined organic phases were washed with saturated brine (50 mL ×3), dried over anhydrous sodium sulfate, filtered, and concentrated under reduced pressure. The resulting mixture was purified by thin-layer chromatography on a silica gel plate to obtain *N*-(2-chloro-4-(trifluoromethyl)phenyl)-2-(2-(4-cyclopropoxycyclohex-1-en-1-yl)-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)acetamide (20 mg).

MS (ESI) M/Z: 770.3 [M+H]⁺.

**Step E:** *N*-(2-Chloro-4-(trifluoromethyl)phenyl)-2-(2-(4-cyclopropoxycyclohex-1-en-1-yl)-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)acetamide (20 mg, 0.026 mmol) was dissolved in N,N-dimethylformamide (1 mL), and lithium chloride (32 mg, 0.78 mmol) was added. The reaction system was purged with nitrogen, and the mixture was stirred at 150°C for 3 hours.

After LCMS detection indicated the disappearance of the starting material and the formation of the product, the reaction mixture was filtered, and the resulting residue was purified by preparative high-performance liquid chromatography. The purification conditions were as follows: preparative column welch XBC18, 21.2 × 150 mm, 5 µm (particle size); mobile phase: water (containing 10 mmol/L ammonium bicarbonate) and acetonitrile; flow rate: 20 mL/min; gradient: acetonitrile from 40% to 60% within 10 minutes; detection wavelength: 214 nm. The product was collected and lyophilized under reduced pressure. *N*-(2-Chloro-4-(trifluoromethyl)phenyl)-2-(2-(4-cyclopropoxycyclohex-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide (1.20 mg) was obtained.

MS (ESI) M/Z: 756.1 [M+H]⁺.

¹H NMR (400 MHz, Methanol-d₄) δ 8.54 (s, 1H), 8.15 (d, *J =* 8.3 Hz, 1H), 7.81 (s, 1H), 7.61 (d, *J* = 8.7, 1H), 6.87 (t, *J =* 4.3 Hz, 1H), 5.37 (s, 2H), 4.76 - 4.62 (m, 1H), 4.15 - 4.01 (m, 1H), 3.88 - 3.80 (m, 1H), 3.79 - 3.66 (m, 2H), 3.46 - 3.39 (m, 2H), 3.16 - 3.03 (m, 3H), 2.97 - 2.85 (m, 1H), 2.83 - 2.66 (m, 2H), 2.63 - 2.54 (m, 2H), 2.52 (s, 3H), 2.30 - 2.16 (m, 1H), 2.11 - 2.00 (m, 1H), 1.87 - 1.74 (m, 1H), 1.30 (t, *J =* 7.5 Hz, 3H), 0.59 - 0.45 (m, 4H).

### Example 110

### N-(2-Chloro-4-(trifluoromethyl)phenyl)-2-(2-(4-(cyclopropylmethoxy)cyclohex-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide

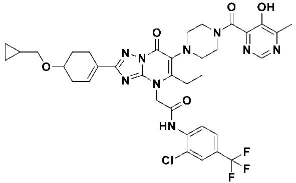

**The compound may also be synthesized according to the following steps:**

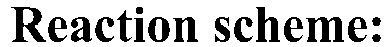

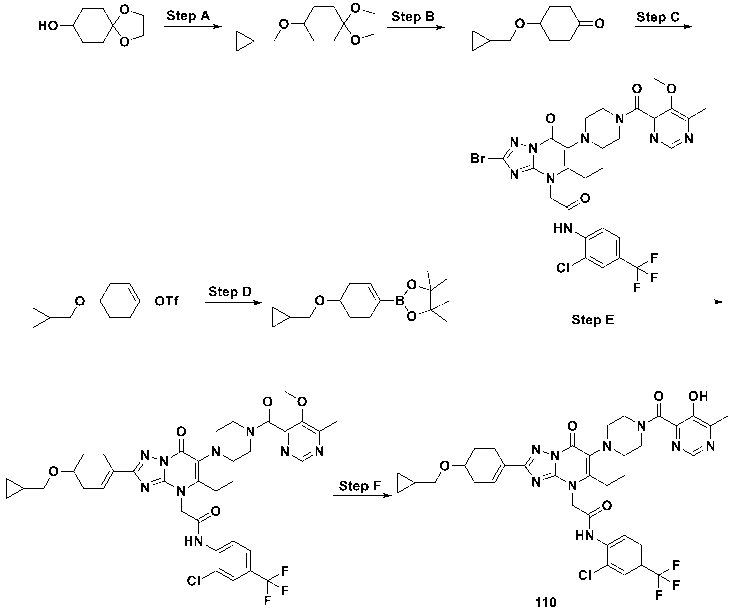

### Procedure:

**Step A:** In a 100 mL three-neck flask, a solution of 1,4-dioxaspiro[4.5]decan-8-ol (4 g, 25.3 mmol) in *N*-methylpyrrolidone (20 mL) was added. The system was purged with nitrogen, and the mixture was cooled to 0°C in an ice bath. NaH (60%, 2 g, 50 mmol) was added in portions. The reaction mixture was stirred at this temperature for thirty minutes. Then, a solution of (bromomethyl)cyclopropane (4.1 g, 30.3 mmol) in N-methylpyrrolidone (5 mL) was added dropwise to the reaction system. The reaction system was then warmed to room temperature and stirred for 18 hours.

After TLC detection indicated the disappearance of the starting material and the formation of the product, the reaction was quenched by adding ice water. The mixture was extracted with ethyl acetate (40 mL ×3). The combined organic phases were washed with saturated brine (30 mL), dried over anhydrous sodium sulfate, filtered, and concentrated under reduced pressure. The resulting mixture was purified by silica gel column chromatography to obtain 8-(cyclopropylmethoxy)-1,4-dioxaspiro[4.5]decane (4.3 g).

¹H NMR (400 MHz, DMSO-d₆) δ 3.74 - 3.63 (m, 4H), 3.23 (m, 1H), 3.07 (d, *J* = 6.7 Hz, 2H), 1.63 - 1.46 (m, 4H), 1.44 - 1.26 (m, 4H), 0.86 - 0.76 (m, 1H), 0.34 - 0.26 (m, 2H), 0.03 - -0.03 (m, 2H).

**Step B:** In a single-neck flask, 8-(cyclopropylmethoxy)-1,4-dioxaspiro[4.5]decane (2 g, 9.42 mmol) was dissolved in tetrahydrofuran (10 mL), and dilute hydrochloric acid (1N, 10 mL) was added. The resulting reaction mixture was heated to 90°C and reacted for 2 hours.

After TLC detection indicated the disappearance of the starting material and the formation of the product, the reaction mixture was cooled to room temperature and slowly poured into saturated aqueous sodium bicarbonate solution (20 mL). The mixture was extracted with ethyl acetate (40 mL × 3). The combined organic phases were washed with saturated brine (30 mL), dried over anhydrous sodium sulfate, filtered, and concentrated under reduced pressure. The resulting mixture was purified by silica gel column chromatography to obtain 4-(cyclopropylmethoxy)cyclohexan-1-one (1.33 g).

¹H NMR (400 MHz, DMSO-*d*₆) δ 3.57 - 3.48 (m, 1H), 3.13 (d, *J* = 6.7 Hz, 2H), 2.23 - 2.13 (m, 2H), 2.08 - 1.97 (m, 2H), 1.82 - 1.61 (m, 4H), 0.90 - 0.77 (m, 1H), 0.35 - 0.22 (m, 2H), 0.06 - -0.03 (m, 2H).

**Step C:** In a 100 mL three-neck flask, a solution of 4-(cyclopropylmethoxy)cyclohexan-1-one (800 mg, 4.76 mmol) and *N-*fluorobenzenesulfonimide (2.55 g, 8.08 mmol) in tetrahydrofuran (11 mL) was added. The system was purged with nitrogen and cooled to -78°C. Then, lithium bis(trimethylsilyl)amide (1N in THF, 6.66 mL) was added dropwise to the reaction system. After the dropwise addition was completed, the reaction system was warmed to room temperature and stirred for an additional 1 hour.

After TLC detection indicated the disappearance of the starting material and the formation of the product, the reaction was quenched by adding saturated aqueous ammonium chloride solution, and the mixture was extracted with ethyl acetate (100 mL × 3). The combined organic phases were washed with saturated brine (50 mL × 3), dried over anhydrous sodium sulfate, filtered, and concentrated under reduced pressure. The resulting mixture was purified by silica gel column chromatography to obtain 4-(cyclopropylmethoxy)cyclohex-1-en-1-yl trifluoromethanesulfonate (1.2 g).

¹H NMR (400 MHz, DMSO-d₆) δ 5.78 - 5.74 (m, 1H), 3.66 - 3.58 (m, 1H), 3.27 (d, *J* = 8.1 Hz, 2H), 2.46 - 2.23 (m, 3H), 2.18 - 2.10 (m, 1H), 1.90 - 1.78 (m, 2H), 1.03 - 0.90 (m, 1H), 0.47 - 0.36 (m, 2H), 0.24 - 0.08 (m, 2H).

**Step D:** 4-(Cyclopropylmethoxy)cyclohex-1-en-1-yl trifluoromethanesulfonate (900 mg, 3 mmol) was dissolved in 1,4-dioxane (15 mL), and 4,4,4',4',5,5',5',5'-octamethyl-2,2'-bi(1,3,2-dioxaborolane) (1.14 g, 4.5 mmol), PdCl₂(dppf)•DCM (245 mg, 0.3 mmol), and potassium acetate (588 mg, 6 mol) were added. The reaction system was purged with nitrogen, and the mixture was stirred at 95°C for 4 hours.

After TLC detection indicated the disappearance of the starting material and the formation of the product, the reaction mixture was filtered, and the filtrate was directly concentrated. The resulting mixture was purified by silica gel column chromatography to obtain 2-(4-(cyclopropylmethoxy)cyclohex-1-en-1-yl)-4,4,5,5-tetramethyl-1,3,2-dioxaborolane (200 mg).

¹H NMR (400 MHz, DMSO-d₆) δ 6.38 - 6.25 (m, 1H), 3.56 - 3.44 (m, 1H), 3.25 (d, *J* = 6.7 Hz, 2H), 2.43 - 2.34 (m, 1H), 2.19 - 2.08 (m, 1H), 2.04 - 1.99 (m, 1H), 1.98 - 1.90 (m, 1H), 1.82 - 1.73 (m, 1H), 1.46 - 1.36 (m, 1H), 1.19 (s, 12H), 0.99 - 0.89 (m, 1H), 0.48 - 0.41 (m, 2H), 0.18 - 0.09 (m, 2H).

**Step E:** 2-(2-Bromo-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)-*N*-(2-chloro-4-(trifluoromethyl)phenyl)acetamide (50 mg, 0.07 mmol) was dissolved in 1,4-dioxane (1.6 mL)/water (0.2 mL), and 2-(4-(cyclopropylmethoxy)cyclohex-1-en-1-yl)-4,4,5,5-tetramethyl-1,3,2-dioxaborolane (68 mg, 0.24 mmol), PdCl₂(dppf).DCM (6 mg, 0.007 mmol), and potassium carbonate (24 mg, 0.17 mol) were added. The reaction system was purged with nitrogen, and the mixture was stirred at 100°C for 2 hours.

After LCMS detection indicated the disappearance of the starting material and the formation of the product, the reaction mixture was filtered, and the filtrate was directly concentrated. The resulting mixture was purified by thin-layer chromatography on a silica gel plate to obtain *N*-(2-chloro-4-(trifluoromethyl)phenyl)-2-(2-(4-(cyclopropylmethoxy)cyclohex-1-en-1-yl)-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)acetamide (14 mg).

MS (ESI) M/Z: 784.3 [M+H]⁺.

**Step F:** *N*-(2-Chloro-4-(trifluoromethyl)phenyl)-2-(2-(4-(cyclopropylmethoxy)cyclohex-1-en-1-yl)-5-ethyl-6-(4-(5-methoxy-6-methylpyrimidine-4-carbonyl)piperazin-1-yl)-7-oxo-[1,2,4]triazolo[1,5-a]pyrimidin-4(7H)-yl)acetamide (14 mg, 0.017 mmol) was dissolved in *N,N-*dimethylformamide (0.5 mL), and lithium chloride (22 mg, 0.53 mmol) was added. The reaction system was purged with nitrogen, and the mixture was stirred at 150°C for 3 hours.

After LCMS detection indicated the disappearance of the starting material and the formation of the product, the reaction mixture was filtered, and the resulting residue was purified by preparative high-performance liquid chromatography. The purification conditions were as follows: preparative column welch XBC18, 21.2 × 150 mm, 5 µm (particle size); mobile phase: water (containing 10 mmol/L ammonium bicarbonate) and acetonitrile; flow rate: 20 mL/min; gradient: acetonitrile from 40% to 60% within 10 minutes; detection wavelength: 214 nm. The product was collected and lyophilized under reduced pressure. *N*-(2-Chloro-4-(trifluoromethyl)phenyl)-2-(2-(4-(cyclopropylmethoxy)cyclohex-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide (1 mg) was obtained.

MS (ESI) M/Z: 770.0 [M+H]⁺.

¹H NMR (400 MHz, DMSO-d₆) δ 10.35 (s, 1H), 10.22 (s, 1H), 8.57 (s, 1H), 8.06 (d, *J= 8.6* Hz, 1H), 7.97 (s, 1H), 7.71 (d, *J* = 8.9 Hz, 1H), 6.78 - 6.68 (m, 1H), 5.31 (s, 2H), 4.56 - 4.48 (m, 1H), 3.68 - 3.59 (m, 1H), 3.56 - 3.42 (m, 3H), 3.28 - 3.19 (m, 3H), 3.04 - 2.91 (m, 3H), 2.85 - 2.75 (m, 1H), 2.66 - 2.60 (m, 1H), 2.57 - 2.52 (m, 3H), 2.44 (s, 3H), 2.17 - 2.09 (m, 1H), 1.96 - 1.88 (m, 1H), 1.70 - 1.60 (m, 1H), 1.18 (t, *J* = 7.5 Hz, 3H), 1.03 - 0.94 (m, 1H), 0.48 - 0.39 (m, 2H), 0.20 - 0.11 (m, 2H).

### Example 129

### N-(3-Chloro-4-(trifluoromethyl)thiophen-2-yl)-2-(2-(3,6-dihydro-2H-pyran-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide

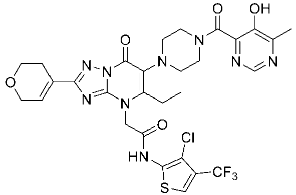

**The compound may also be synthesized according to the following steps:**

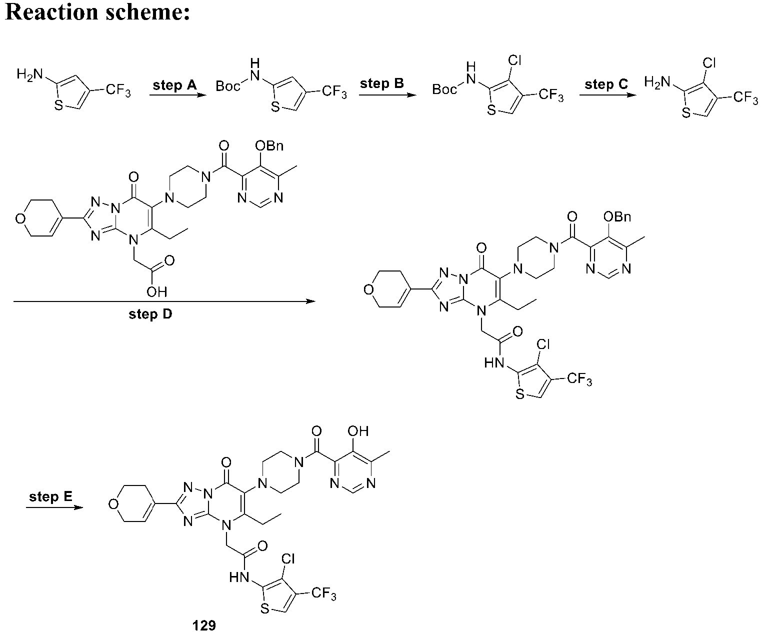

### Procedure:

**Step A:** 4-(Trifluoromethyl)thiophen-2-amine (150 mg, 0.9 mmol) was dissolved in ethanol (2 mL), and di-tert-butyl dicarbonate (235 mg, 1.1 mmol) was added slowly with stirring at 25°C. The resulting mixture was stirred at 50°C for 8 hours.

After TLC monitoring indicated the disappearance of the starting material, the reaction mixture was concentrated under reduced pressure. The resulting mixture was then purified by silica gel column chromatography to obtain *tert-butyl* (4-(trifluoromethyl)thiophen-2-yl)carbamate (177 mg).

MS (ESI) M/Z: 266.0 [M-H] ⁻.

**Step B:** *tert*-Butyl (4-(trifluoromethyl)thiophen-2-yl)carbamate (177 mg, 0.7 mmol) was dissolved in acetonitrile (5 mL), and NCS (106 mg, 0.8 mmol) was added dropwise at 0°C. The resulting mixture was stirred at 50°C for 2 hours.

After LCMS monitoring indicated the disappearance of the starting material, the resulting mixture was diluted with water (30 mL), and the reaction mixture was extracted with ethyl acetate (30 mL × 3). The combined organic phases were washed with saturated brine (10 mL × 2), dried over anhydrous sodium sulfate, filtered, and the filtrate was concentrated under reduced pressure. The resulting mixture was purified by silica gel column chromatography to obtain *tert*-butyl (3-chloro-4-(trifluoromethyl)thiophen-2-yl)carbamate (90 mg).

MS (ESI) M/Z: 300.0 [M-H] ⁻.

**Step C:** At 0°C, to a stirred solution of *tert*-butyl (3-chloro-4-(trifluoromethyl)thiophen-2-yl)carbamate (90 mg, 0.3 mmol) was added hydrochloric acid (2 mL of a 4.0 M solution in 1,4-dioxane), and the reaction system was stirred at 25°C for 12 hours.

After LCMS monitoring indicated the disappearance of the starting material, the resulting mixture was concentrated under reduced pressure to obtain 3-chloro-4-(trifluoromethyl)thiophen-2-amine hydrochloride (80 mg). The crude product was used directly in the next step without further purification.

**Step D:** 3-Chloro-4-(trifluoromethyl)thiophen-2-amine (80 mg, crude product) and 2-[6-[4-(5-benzyloxy-6-methylpyrimidine-4-carbonyl)-2-(3,6-dihydro-2*H*-pyran-4-yl)-5-ethyl-7-oxo-[1,2,4]triazolo[1,5-a]pyrimidin-4-yl]acetic acid (183 mg, 0.3 mmol) were dissolved in acetonitrile (5 mL), and TCFH (251 mg, 0.9 mmol) and NMI (122 mg, 1.5 mmol) were added under a nitrogen atmosphere at 0°C. The reaction mixture was stirred at 25°C for 1 hour.

After LCMS monitoring indicated the disappearance of the starting material, the reaction mixture was extracted with dichloromethane (10 mL × 3). The combined organic phases were washed with saturated brine (10 mL × 3), dried over anhydrous sodium sulfate, filtered, and concentrated under reduced pressure. The resulting mixture was purified by silica gel column chromatography to obtain 2-[6-[4-(5-benzyloxy-6-methylpyrimidine-4-carbonyl)piperazin-1-yl]-2-(3,6-dihydro-2*H*-pyran-4-yl)-5-ethyl-7-oxo-[1,2,4]triazolo[1,5-a]pyrimidin-4-yl]-*N*-[3-chloro-4-(trifluoromethyl)-2-thienyl]acetamide (150 mg).

MS (ESI) M/Z: 798.3 [M+H] ⁺.

**Step E:** Compound 2-[6-[4-(5-benzyloxy-6-methylpyrimidine-4-carbonyl)piperazin-1-yl]-2-(3,6-dihydro-2*H*-pyran-4-yl)-5-ethyl-7-oxo-[1,2,4]triazolo[1,5-a]pyrimidin-4-yl]-*N-*[3-chloro-4-(trifluoromethyl)-2-thienyl]acetamide (80 mg, 0.1 mmol) was dissolved in trifluoroacetic acid (4 mL), and the reaction mixture was stirred at 60°C for 2 hours.

After LCMS monitoring indicated the disappearance of the starting material, the reaction mixture was diluted with dichloromethane and then concentrated under reduced pressure. The resulting mixture was purified by preparative high-performance liquid chromatography. The purification conditions were as follows: chromatographic column: xbridge-C18 21.2 mm × 150 mm; mobile phase: water (containing 0.1% formic acid) and acetonitrile; flow rate: 25 mL/min; gradient: acetonitrile from 40% to 70% within 20 minutes; detection wavelength: 254 nm. The product was collected and lyophilized under reduced pressure to obtain the final product *N*-[3-chloro-4-(trifluoromethyl)-2-thienyl]-2-[2-(3,6-dihydro-2*H*-pyran-4-yl)-5-ethyl-6-[4-(5-hydroxy-6-methylpyrimidine-4-carbonyl)piperazin-1-yl]-7-oxo-[1,2,4]triazolo[1,5-a]pyrimidin-4-yl]acetamide (5.9 mg).

MS (ESI) M/Z: 708.4 [M+H] ⁺.

¹H NMR (400 MHz, DMSO-*d₆*) δ 11.18 (s, 1H), 8.54 (s, 1H), 8.24 (s, 1H), 7.57 (s, 1H), 6.81 (t, *J* = 2.1 Hz, 1H), 5.17 (s, 2H), 4.58 - 4.47 (m, 1H), 4.24 (d, *J* = 2.9 Hz, 2H), 3.80 (t, *J* = 5.4 Hz, 2H), 3.59 - 3.47 (m, 3H), 3.27 - 3.22 (m, 1H), 3.05 - 2.89 (m, 3H), 2.86 - 2.80 (m, 1H), 2.69 - 2.63 (m, 1H), 2.5(m, 2H), 2.44 (s, 3H), 1.16 (t, *J* = 7.4 Hz, 3H).

### Example 152

### N-(2-Chloro-4-(trifluoromethyl)phenyl)-2-(5-ethyl-6-(4-(5-hydroxy-6-methylpyrimidine-4-carbonyl)piperazin-1-yl)-7-oxo-2-(4-(pyrrolidine-1-carbonyl)cyclohex-1-en-1-yl)-[1,2,4]triazolo[1,5-a]pyrimidin-4(7H)-yl)acetamide

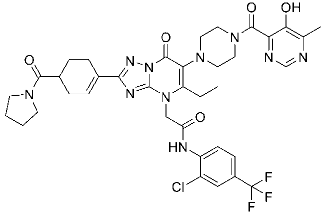

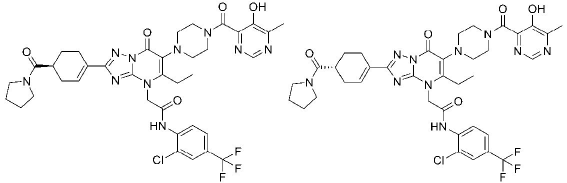

*N*-(2-Chloro-4-(trifluoromethyl)phenyl)-2-(5-ethyl-6-(4-(5-hydroxy-6-methylpyrimidine-4-carbonyl)piperazin-1-yl)-7-oxo-2-(4-(pyrrolidine-1-carbonyl)cyclohex-1-en-1-yl)-[1,2,4]triazolo[1,5-a]pyrimidin-4(7H)-yl)acetamide (60.0 mg) may also be prepared with reference to the preparation method of Example 94. Separation was performed by preparative chiral HPLC using a preparative column: CHIRALPAK IC (5 µm, 30 mm I.D. × 250 mm L), mobile phase gradient: 90% acetonitrile/10% water (containing 0.1% trifluoroacetic acid), flow rate: 30 mL/min, to obtain Example 152-peak 1 (12.5 mg, retention time: 65.2 min) and Example 152-peak 2 (20.2 mg, retention time: 85.76 min).

### Example 152-peak 1:

MS (ESI) M/Z: 797.3 [M+H] ⁺.

¹H NMR (400 MHz, DMSO-d₆) δ 10.39 (s, 1H), 10.27 (s, 1H), 8.56 (s, 1H), 8.06 (d, *J* = 8.4 Hz, 1H), 7.98 (s, 1H), 7.72 (d, *J =* 8.7 Hz, 1H), 6.95 - 6.82 (m, 1H), 5.31 (s, 2H), 4.60 - 4.41 (m, 1H), 3.57 - 3.41 (m, 5H), 3.31 - 3.22 (m, 3H), 3.08 - 2.88 (m, 3H), 2.86 - 2.76 (m, 1H), 2.71 - 2.58 (m, 3H), 2.43 (s, 3H), 2.40 - 2.26 (m, 3H), 1.97 - 1.82 (m, 3H), 1.81 - 1.72 (m, 2H), 1.60 - 1.51 (m, 1H), 1.17 (t, *J =* 8.5 Hz, 3H).

### Example 152-peak 2:

MS (ESI) M/Z: 797.3 [M+H] ⁺.

¹H NMR (400 MHz, DMSO-d₆) δ 10.41 (s, 1H), 10.27 (s, 1H), 8.57 (s, 1H), 8.06 (d, *J* = 8.4 Hz, 1H), 7.98 (s, 1H), 7.72 (d, *J* = 8.6 Hz, 1H), 6.87 (t, *J* = 4.8 Hz, 1H), 5.32 (s, 2H), 4.59 - 4.44 (m, 1H), 3.56 - 3.43 (m, 5H), 3.31 - 3.22 (m, 3H), 3.06 - 2.90 (m, 3H), 2.86 - 2.77 (m, 1H), 2.72 - 2.59 (m, 3H), 2.44 (s, 3H), 2.41 - 2.31 (m, 3H), 1.95 - 1.84 (m, 3H), 1.80 - 1.73 (m, 2H), 1.60 - 1.52 (m, 1H), 1.19 - 1.16 (m, 3H).

### Example 154

### N-(2-Chloro-4-(trifluoromethyl)phenyl)-2-(2-(4-(4,4-difluoropiperidine-1-carbonyl)cyclohex-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide

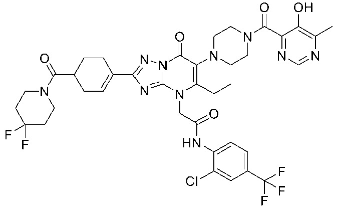

*N*-(2-Chloro-4-(trifluoromethyl)phenyl)-2-(2-(4-(4,4-difluoropiperidine-1-carbonyl)cyclohex-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide (45.3 mg) may also be prepared with reference to the preparation method of Example 94.

MS (ESI) M/Z: 847.6 [M+H] ⁺.

¹H NMR (400 MHz, DMSO-d₆) δ 10.39 (s, 1H), 10.25 (s, 1H), 8.58 (s, 1H), 8.07 (d, *J* = 8.6 Hz, 1H), 7.99 (s, 1H), 7.73 (d, *J* = 8.7 Hz, 1H), 6.94 - 6.78 (m, 1H), 5.32 (s, 2H), 4.59 - 4.44 (m, 1H), 3.71 - 3.59 (m, 3H), 3.56 - 3.43 (m, 4H), 3.29 - 3.20 (m, 1H), 3.06 - 2.89 (m, 4H), 2.86 - 2.75 (m, 1H), 2.70 - 2.60 (m, 2H), 2.44 (s, 3H), 2.42 - 2.22 (m, 3H), 2.06 - 1.84 (m, 5H), 1.65 - 1.50 (m, 1H), 1.18 (t, *J* = 7.5 Hz, 3H).

### Example 166

### N-(2-Chloro-4-(trifluoromethyl)phenyl)-2-(5-ethyl-6-(4-(5-hydroxy-6-methylpyrimidine-4-carbonyl)piperazin-1-yl)-7-oxo-2-(1-oxaspiro[4.5]dec-7-en-8-yl)-[1,2,4]triazolo[1,5-a]pyrimidin-4(7H)-yl)acetamide

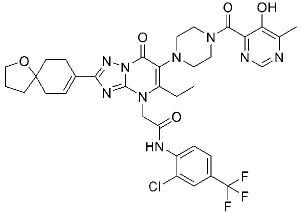

*N*-(2-Chloro-4-(trifluoromethyl)phenyl)-2-(5-ethyl-6-(4-(5-hydroxy-6-methylpyrimidine-4-carbonyl)piperazin-1-yl)-7-oxo-2-(1-oxaspiro[4.5]dec-7-en-8-yl)-**[1,2,4]triazolo[1,5-a]pyrimidin-4(7H)-yl)acetamide** (47.1 mg) may also be prepared with reference to the preparation method of Example 1.

MS (ESI) M/Z: 756.4 [M+H] ⁺.

¹H NMR (400 MHz, DMSO-*d*₆) δ 10.38 (s, 1H), 10.25 (s, 1H), 8.56 (s, 1H), 8.06 (d, *J* = 8.6 Hz, 1H), 7.97 (s, 1H), 7.71 (d, *J =* 8.8 Hz, 1H), 6.83 - 6.65 (m, 1H), 5.31 (s, 2H), 4.59 - 4.43 (m, 1H), 3.74 (t, *J* = 6.9 Hz, 2H), 3.57 - 3.41 (m, 3H), 3.26 - 3.20 (m, 1H), 3.07 - 2.89 (m, 3H), 2.86 - 2.74 (m, 1H), 2.67 - 2.55 (m, 2H), 2.44 (s, 3H), 2.38 - 2.19 (m, 3H), 1.94 - 1.82 (m, 2H), 1.78 - 1.61 (m, 4H), 1.17 (t, *J* = 7.6 Hz, 3H).

### Example 142

### N-(2-Chloro-4-(trifluoromethyl)phenyl)-2-(5-ethyl-6-(4-(5-hydroxy-6-methylpyrimidine-4-carbonyl)piperazin-1-yl)-7-oxo-2-(4-(2-oxooxazolidin-3-yl)cyclohex-1-en-1-yl)-[1,2,4]triazolo[1,5-a]pyrimidin-4(7H)-yl)acetamide

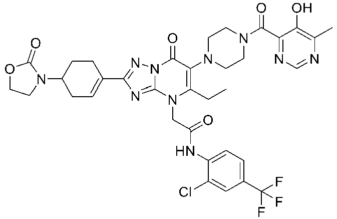

*N*-(2-Chloro-4-(trifluoromethyl)phenyl)-2-(5-ethyl-6-(4-(5-hydroxy-6-methylpyrimidine-4-carbonyl)piperazin-1-yl)-7-oxo-2-(4-(2-oxooxazolidin-3-yl)cyclohex-1-en-1-yl)-[1,2,4]triazolo[1,5-*a*]pyrimidin-4(7H)-yl)acetamide (52.4 mg) may also be prepared with reference to the preparation method of Example 94.

MS (ESI) M/Z: 785.0 [M+H] ⁺.

¹H NMR (400 MHz, DMSO-d₆) δ 10.38 (s, 1H), 10.25 (s, 1H), 8.57 (s, 1H), 8.05 (d, *J* = 8.7 Hz, 1H), 7.97 (s, 1H), 7.72 (d, *J* = 8.8 Hz, 1H), 6.86 - 6.78 (m, 1H), 5.31 (s, 2H), 4.56 - 4.46 (m, 1H), 4.28 (t, *J =* 8.9 Hz, 2H), 3.86 - 3.77 (m, 1H), 3.57 - 3.45 (m, 5H), 3.28 - 3.21 (m, 1H), 3.05 - 2.91 (m, 3H), 2.84 - 2.72 (m, 2H), 2.70 - 2.57 (m, 2H), 2.44 (s, 3H), 2.41 - 2.31 (m, 2H), 1.95 - 1.73 (m, 2H), 1.17 (t, *J =* 8.9 Hz, 3H).

### Example 114

### N-(2-Chloro-4-(trifluoromethyl)phenyl)-2-(5-ethyl-6-(4-(5-hydroxy-6-methylpyrimidine-4-carbonyl)piperazin-1-yl)-2-(1-(5-methyl-1,3,4-thiadiazol-2-yl)-1,2,3,6-tetrahydropyridin-4-yl)-7-oxo-[1,2,4]triazolo[1,5-a]pyrimidin-4(7H)-yl)acetamide

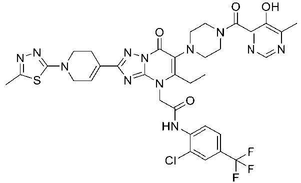

*N*-(2-Chloro-4-(trifluoromethyl)phenyl)-2-(5-ethyl-6-(4-(5-hydroxy-6-methylpyrimidine-4-carbonyl)piperazin-1-yl)-2-(1-(5-methyl-1,3,4-thiadiazol-2-yl)-1,2,3,6-tetrahydropyridin-4-yl)-7-oxo-[1,2,4]triazolo[1,5-a]pyrimidin-4(7H)-yl)acetamide (7.32 mg) may also be prepared with reference to the preparation method of **Example 65.**

MS (ESI) M/Z: 799.4 [M+H] ⁺.

¹H NMR (400 MHz, DMSO-d₆) δ 10.33 (s, 1H), 8.54 (s, 1H), 8.08 - 7.99 (m, 1H), 7.99 - 7.92 (m, 1H), 7.74 - 7.68 (m, 1H), 6.91 - 6.77 (m, 1H), 5.34 - 5.25 (m, 2H), 4.57 - 4.45 (m, 1H), 4.17 - 4.10 (m, 1H), 3.90 - 3.64 (m, 2H), 3.57 - 3.42 (m, 3H), 3.26 - 3.19 (m, 2H), 3.03 - 2.95 (m, 3H), 2.87 - 2.77 (m, 1H), 2.75 - 2.69 (m, 1H), 2.55 - 2.53 (m, 2H), 2.52 (s, 3H), 2.43 (s, 3H), 1.18 (t, *J =* 6.7 Hz, 3H).

### Example 121

### N-(2-Chloro-4-(trifluoromethyl)phenyl)-2-(5-ethyl-6-(4-(5-hydroxy-6-methylpyrimidine-4-carbonyl)piperazin-1-yl)-7-oxo-2-(1-phenyl-1,2,3,6-tetrahydropyridin-4-yl)-[1,2,4]triazolo[1,5-a]pyrimidin-4(7H)-yl)acetamide

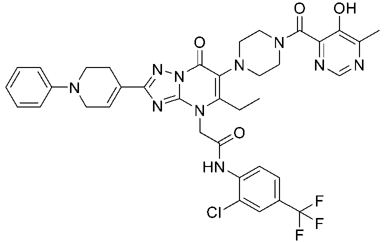

*N*-(2-Chloro-4-(trifluoromethyl)phenyl)-2-(5-ethyl-6-(4-(5-hydroxy-6-methylpyrimidine-4-carbonyl)piperazin-1-yl)-7-oxo-2-(1-phenyl-1,2,3,6-tetrahydropyridin-4-yl)-[1,2,4]triazolo[1,5-a]pyrimidin-4(7H)-yl)acetamide (5.53 mg) **may also be prepared with reference to the preparation method of Example 65.**

MS (ESI) M/Z: 777.5 [M+H] ⁺.

¹H NMR (400 MHz, DMSO-d₆) δ 10.36 (s, 1H), 8.54 (s, 1H), 8.07 (d, *J* = 8.6 Hz, 1H), 7.99 - 7.94 (m, 1H), 7.75 - 7.68 (m, 1H), 7.26 - 7.17 (m, 2H), 7.01 - 6.96 (m, 2H), 6.95 - 6.89 (m, 1H), 6.80 - 6.71 (m, 1H), 5.33 (s, 2H), 4.57 - 4.48 (m, 1H), 3.90 (s, 2H), 3.52 - 3.46 (m, 3H), 3.27 - 3.24 (m, 1H), 3.04 - 2.93(m, 3H), 2.86 - 2.78 (m, 1H), 2.69 - 2.64 (m, 3H), 2.43 (s, 3H), 2.33 - 2.31 (m, 2H), 1.19 (t, *J* = 7.5 Hz, 3H).

### Example 130

### N-(2-Chloro-5-(trifluoromethyl)thiophen-3-yl)-2-(2-(3,6-dihydro-2H-pyran-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide

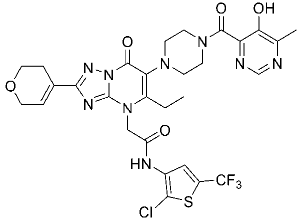

**The compound may also be synthesized according to the following steps:**

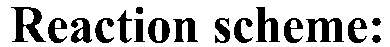

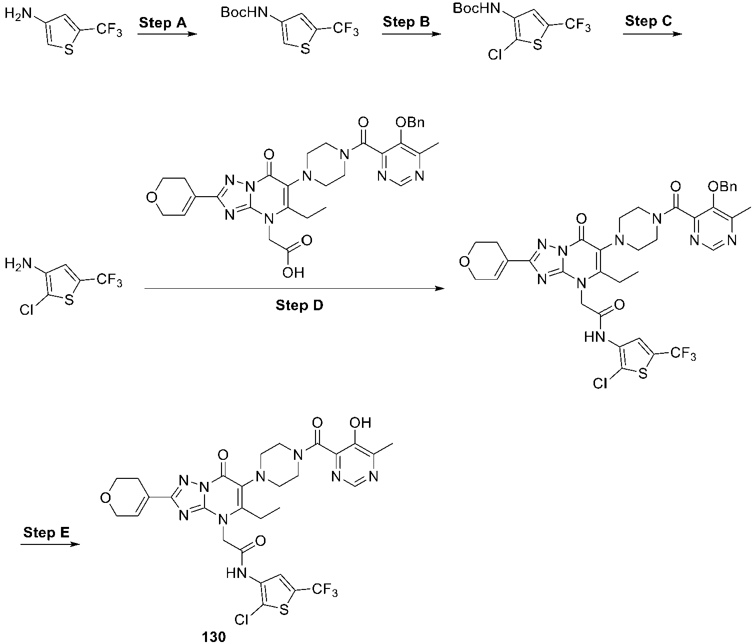

### Procedure:

**Step A:** 5-(Trifluoromethyl)thiophen-3-amine (220 mg, 1.3 mmol) was dissolved in anhydrous ethanol (5 mL), and *tert*-butyl tert-butoxycarbonyl carbonate (1.2 g, 5.3 mmol) was added. The reaction mixture was stirred at 50°C for 16 hours.

After LCMS monitoring indicated the disappearance of the starting material, the reaction mixture was concentrated under reduced pressure, and the resulting mixture was purified by silica gel column chromatography (eluent: petroleum ether/ethyl acetate = 40/60) to obtain *tert*-butyl N-[5-(trifluoromethyl)-3-thienyl]carbamate (180 mg).

MS (ESI) M/Z: 266.1 [M-H]⁻.

**Step B:** *tert*-Butyl N-[5-(trifluoromethyl)-3-thienyl]carbamate (130 mg, 486.4 µmol) was dissolved in acetonitrile (2 mL), and *N*-chlorosuccinimide (78 mg, 583.7 µmol) was added. The reaction mixture was stirred at 50°C for 2 hours.

After LCMS monitoring indicated the disappearance of the starting material, the mixture was diluted with water (15 mL) and extracted with ethyl acetate (30 mL × 3). The combined organic phases were dried, filtered, and concentrated under reduced pressure. The resulting mixture was purified by silica gel column chromatography (eluent: petroleum ether/ethyl acetate = 95/5) to obtain *tert-butyl* (2-chloro-5-(trifluoromethyl)thiophen-3-yl)carbamate (120 mg).

MS (ESI) M/Z: 299.9 [M-H]⁻.

**Step C:** *tert*-Butyl (2-chloro-5-(trifluoromethyl)thiophen-3-yl)carbamate (60.0 mg, 198.9 µmol) was dissolved in hydrogen chloride (4.0 M in 1,4-dioxane), and the reaction mixture was stirred at room temperature for 16 hours.

After LCMS monitoring indicated the disappearance of the starting material, the mixture was diluted with dichloromethane (30 mL) and then concentrated under reduced pressure to obtain 2-chloro-5-(trifluoromethyl)thiophen-3-amine hydrochloride (50.0 mg, yellow solid). The crude product was used directly in the next step.

MS (ESI) M/Z: 201.8 [M+H] ⁺.

**Step D:** 2-[6-[4-(5-Benzyloxy-6-methylpyrimidine-4-carbonyl)piperazin-1-yl]-2-(3,6-dihydro-2H-pyran-4-yl)-5-ethyl-7-oxo-[1,2,4]triazolo[1,5-a]pyrimidin-4-yl]acetic acid (100.0 mg, 162.8 µmol) and 2-chloro-5-(trifluoromethyl)thiophen-3-amine hydrochloride (50 mg, crude product) were dissolved in acetonitrile (6 mL), followed by the sequential addition of *N,N,N',N'*-tetramethylchloroformamidinium hexafluorophosphate (55 mg, 195.4 µmol) and *N*-methylimidazole (67 mg, 814.3 µmol). The reaction mixture was stirred at room temperature for 1 hour.

After LCMS monitoring indicated the disappearance of the starting material, water (30 mL) was added, and the mixture was extracted with ethyl acetate (40 mL × 3). The combined organic phases were dried over anhydrous sodium sulfate, filtered, and concentrated under reduced pressure. The resulting mixture was purified by silica gel column chromatography (eluent: dichloromethane/methanol = 10/1) to obtain 2-(6-(4-(5-(benzyloxy)-6-methylpyrimidine-4-carbonyl)piperazin-1-yl)-2-(3,6-dihydro-2H-pyran-4-yl)-5-ethyl-7-oxo-[1,2,4]triazolo[1,5-a]pyrimidin-4(7H)-yl)-N-(2-chloro-5-(trifluoromethyl)thiophen-3-yl)acetamide (120 mg).

MS (ESI) M/Z: 798.4 [M+H] ⁺.

**Step E:** 2-(6-(4-(5-(Benzyloxy)-6-methylpyrimidine-4-carbonyl)piperazin-1-yl)-2-(3,6-dihydro-2H-pyran-4-yl)-5-ethyl-7-oxo-[1,2,4]triazolo[1,5-a]pyrimidin-4(7H)-yl)-*N*-(2-chloro-5-(trifluoromethyl)thiophen-3-yl)acetamide (120.0 mg, 150.6 µmol) was dissolved in trifluoroacetic acid (3 mL), and the reaction mixture was stirred at 60°C for 1 hour.

After LCMS monitoring indicated the disappearance of the starting material, the mixture was diluted with dichloromethane and then concentrated under reduced pressure. The resulting mixture was purified by preparative high-performance liquid chromatography. The purification conditions were as follows: chromatographic column: xbridge-C18 21.2 mm × 150 mm; mobile phase: water (containing 0.1% formic acid) and acetonitrile; flow rate: 25 mL/min; gradient: acetonitrile from 40% to 60% within 15 minutes; detection wavelength: 254 nm. *N*-(2-Chloro-5-(trifluoromethyl)thiophen-3-yl)-2-(2-(3,6-dihydro-2H-pyran-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide (29.08 mg) was obtained.

MS (ESI) M/Z: 708.3 [M+H] ⁺.

¹H NMR (400 MHz, DMSO-d₆) δ 10.68 (s, 1H), 10.22 (s, 1H), 8.58 (s, 1H), 7.97 (s, 1H), 6.85 - 6.79 (m, 1H), 5.24 (s, 2H), 4.56 - 4.47 (m, 1H), 4.29 - 4.21 (m, 2H), 3.80 (t, *J* = 5.5 Hz, 2H), 3.56 - 3.43 (m, 3H), 3.28 - 3.20 (m, 1H), 3.04 - 2.89 (m, 3H), 2.84 - 2.75 (m, 1H), 2.65 - 2.59 (m, 1H), 2.50 (m, 2H), 2.44 (s, 3H), 1.17 (t, *J* = 7.5 Hz, 3H).

### Example 131

### 2-(2-(3,6-Dihydro-2H-pyran-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(5-(trifluoromethyl)thiophen-3-yl)acetamide

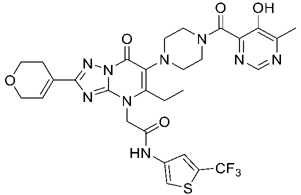

**The compound may also be synthesized according to the following steps:**

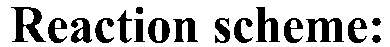

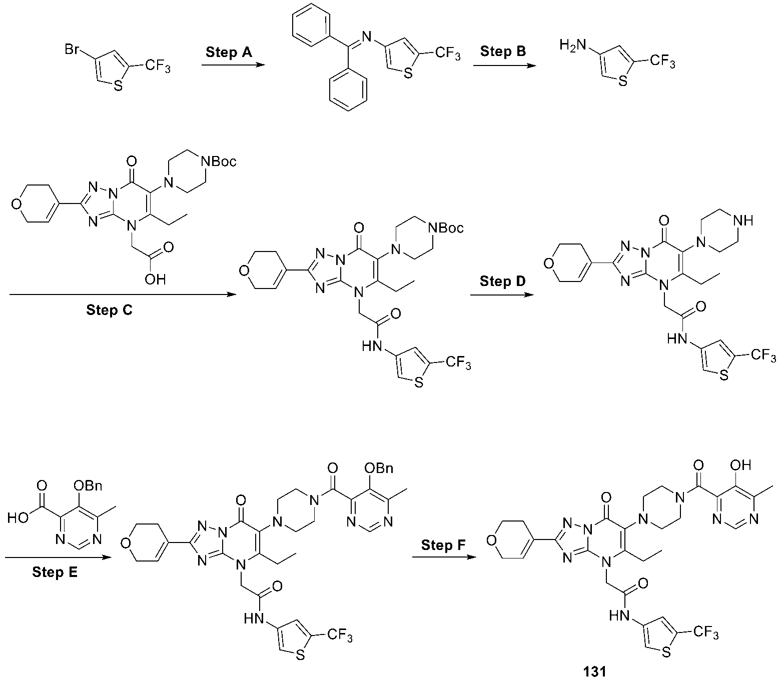

### Procedure:

**Step A:** In a 50 mL single-neck flask, diphenylmethanimine (86 mg, 475.1 µmol), (5-diphenylphosphino-9,9-dimethyl-xanthen-4-yl)-diphenylphosphine (100.2 mg, 173.1 µmol), sodium *tert*-butoxide (104.0 mg, 1.1 µmol), and tris(dibenzylideneacetone)dipalladium (79.3 mg, 86.6 µmol) were dissolved in 1,4-dioxane (10 mL), followed by the addition of 4-bromo-2-(trifluoromethyl)thiophene (100.0 mg, 432.8 µmol). The reaction system was purged with nitrogen, and then stirred at 100°C for 2 hours under a nitrogen atmosphere.

After LCMS monitoring indicated the disappearance of the starting material, water was added to the reaction mixture, and the mixture was extracted with ethyl acetate (50 mL × 3). The combined organic phases were dried over anhydrous sodium sulfate, filtered, and concentrated under reduced pressure. The resulting mixture was purified by silica gel column chromatography to obtain 1,1-diphenyl-*N*-[5-(trifluoromethyl)-3-thienyl]methanamine (150.0 mg).

MS (ESI) M/Z: 332.2 [M+H] ⁺.

**Step B:** In a 25 mL single-neck flask, 1,1-diphenyl-*N*-[5-(trifluoromethyl)-3-thienyl]methanamine (150.0 mg, 452.7 µmol) was dissolved in tetrahydrofuran (2.5 mL), and then concentrated hydrochloric acid (0.5 mL) was added dropwise. The reaction mixture was stirred at room temperature for 2 hours.

After LCMS monitoring indicated the disappearance of the starting material, the reaction mixture was diluted with dichloromethane and then concentrated under reduced pressure. The resulting mixture was purified by preparative thin-layer chromatography (developing solvent: petroleum ether/ethyl acetate = 85/15) to obtain the product 5-(trifluoromethyl)thiophen-3-amine (80.0 mg).

MS (ESI) M/Z: 168.1 [M+H] ⁺.

**Step C:** In a 50 mL single-neck flask, 5-(trifluoromethyl)thiophen-3-amine (23 mg, 136.8 µmol) and 2-[6-(4-*tert*-butoxycarbonylpiperazin-1-yl)-2-(3,3,6-dihydro-2H-pyran-4-yl)-5-ethyl-7-oxo-[1,2,4]triazolo[1,5-a]pyrimidin-4-yl]acetic acid (60 mg, 122.8 µmol) were dissolved in acetonitrile (5 mL), followed by the sequential addition of *N,N,N',N'-*tetramethylchloroformamidinium hexafluorophosphate (69 mg, 245.6 µmol) and *N-*methylimidazole (30 mg, 368.5 µmol). The reaction mixture was stirred at room temperature for 1 hour.

After LCMS monitoring indicated the disappearance of the starting material, water was added to the reaction mixture, and the mixture was extracted with ethyl acetate (50 mL × 3). The combined organic phases were dried over anhydrous sodium sulfate, filtered, and concentrated under reduced pressure. The resulting mixture was purified by silica gel column chromatography to obtain the product *tert*-butyl 4-(2-(3,6-dihydro-2H-pyran-4-yl)-5-ethyl-7-oxo-4-(2-oxo-2-((5-(trifluoromethyl)thiophen-3-yl)amino)ethyl)-4,7-dihydro-[1,2,4]triazolo[1,5-a]pyrimidin-6-yl)piperazine-1-carboxylate (50.0 mg).

MS (ESI) M/Z: 582.2 [M-56+H] ⁺.

**Step D:** In a 25 mL single-neck flask, *tert*-butyl 4-[2-(3,6-dihydro-2H-pyran-4-yl)-5-ethyl-7-oxo-4-[2-oxo-2-[[5-(trifluoromethyl)-3-thienyl]amino]ethyl]-[1,1,2,4]triazolo[1,5-a]pyrimidin-6-yl]piperazine-1-carboxylate (50 mg, 78.4 µmol) was dissolved in dichloromethane (2 mL), and then trifluoroacetic acid (1 mL) was added. The reaction mixture was stirred at room temperature for 1 hour.

After LCMS monitoring indicated the disappearance of the starting material, the reaction mixture was diluted with dichloromethane and then concentrated under reduced pressure to obtain the product 2-[2-(3,6-dihydro-2H-pyran-4-yl)-5-ethyl-7-oxo-6-piperazin-1-yl-[1,2,4]triazolo[1,5-a]pyrimidin-4-yl]-N-[5-(trifluoromethyl)-3-thienyl]acetamide (50 mg).

MS (ESI) M/Z: 538.2 [M+H] ⁺.

**Step E:** In a 50 mL single-neck flask, 2-(2-(3,6-dihydro-2H-pyran-4-yl)-5-ethyl-7-oxo-6-(piperazin-1-yl)-[1,2,4]triazolo[1,5-a]pyrimidin-4(7H)-yl)-*N*-(5-(trifluoromethyl)thiophen-3-yl)acetamide (50.0 mg, crude product) and 5-benzyloxy-6-methylpyrimidine-4-carboxylic acid (25.0 mg, 102.3 µmol) were dissolved in acetonitrile (5 mL), followed by the sequential addition of *N,N,N',N'*-tetramethylchloroformamidinium hexafluorophosphate (52 mg, 186.0 µmol) and N-methylimidazole (23 mg, 279.0 µmol). The reaction mixture was stirred at room temperature for 1 hour.

After LCMS monitoring indicated the disappearance of the starting material, water was added to the reaction mixture, and the mixture was extracted with ethyl acetate (50 mL × 3). The combined organic phases were dried over anhydrous sodium sulfate, filtered, and concentrated under reduced pressure. The resulting mixture was purified by silica gel column chromatography to obtain the product 2-[6-[4-(5-benzyloxy-6-methylpyrimidine-4-carbonyl)piperazin-1-yl]-2-(3,6-dihydro-2H-pyran-4-yl)-5-ethyl-7-oxo-[1,2,4]triazolo[1,5-a]pyrimidin-4-yl]-*N*-[5-(trifluoromethyl)-3-thienyl]acetamide (37 mg).

MS (ESI) M/Z: 764.4 [M+H] ⁺.

**Step F:** In a 50 mL single-neck flask, 2-[6-[4-(5-benzyloxy-6-methylpyrimidine-4-carbonyl)piperazin-1-yl]-2-(3,6-dihydro-2H-pyran-4-yl)-5-ethyl-7-oxo-[1,2,4]triazolo[1,5-a]pyrimidin-4-yl]-*N*-[5-(trifluoromethyl)-3-thienyl]acetamide (37 mg, 48.4 µmol) was dissolved in trifluoroacetic acid (2 mL), and then the reaction mixture was stirred at 60°C for 1 hour.

After LCMS monitoring indicated the disappearance of the starting material, the reaction mixture was diluted with dichloromethane and then concentrated under reduced pressure. The resulting mixture was purified by preparative high-performance liquid chromatography. The purification conditions were as follows: chromatographic column: xbridge-C18 21.2 mm × 150 mm; mobile phase: water (containing 0.1% formic acid) and acetonitrile; flow rate: 25 mL/min; gradient: acetonitrile from 40% to 60% within 15 minutes; detection wavelength: 254 nm. The product was collected and lyophilized under reduced pressure to obtain 2-(2-(3,6-dihydro-2H-pyran-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(5-(trifluoromethyl)thiophen-3-yl)acetamide (5.42 mg).

MS (ESI) M/Z: 674.0 [M+H] ⁺.

¹H NMR (400 MHz, DMSO-d₆) δ 11.11 (s, 1H), 8.49 (s, 1H), 7.84 - 7.79 (m, 1H), 7.64 (s, 1H), 6.82 - 6.76 (m, 1H), 5.15 (s, 2H), 4.56 - 4.48 (m, 1H), 4.27 - 4.21 (m, 2H), 3.79 (t, *J* = 5.5 Hz, 2H), 3.56 - 3.43 (m, 3H), 3.25 - 3.20 (m, 1H), 3.03 - 2.91 (m, 3H), 2.85 - 2.77 (m, 1H), 2.66 - 2.61 (m, 1H), 2.50 (m, 2H), 2.42 (s, 3H), 1.16 (t, *J* = 7.4 Hz, 3H).

### Example 162

### N-(2-Chloro-4-(trifluoromethyl)phenyl)-2-(5-ethyl-6-(4-(5-hydroxy-6-methylpyrimidine-4-carbonyl)piperazin-1-yl)-2-(4-(5-methyl-1,3,4-thiadiazol-2-yl)cyclohex-1-en-1-yl)-7-oxo-[1,2,4]triazolo[1,5-a]pyrimidin-4(7H)-yl)acetamide

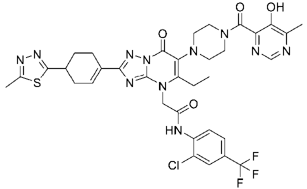

*N*-(2-Chloro-4-(trifluoromethyl)phenyl)-2-(5-ethyl-6-(4-(5-hydroxy-6-methylpyrimidine-4-carbonyl)piperazin-1-yl)-2-(4-(5-methyl-1,3,4-thiadiazol-2-yl)cyclohex-1-en-1-yl)-7-oxo-[1,2,4]triazolo[1,5-a]pyrimidin-4(7H)-yl)acetamide (45 mg) may also be prepared with reference to the preparation method of **Example 65.**

MS (ESI) M/Z: 798.5 [M+H] ⁺.

¹H NMR (400 MHz, DMSO-d₆) δ 10.37 (s, 1H), 8.48 (s, 1H), 8.06 (d, *J* = 8.6 Hz, 1H), 7.96 (d, *J* = 2.1 Hz, 1H), 7.71 (dd, *J* = 8.7, 2.1 Hz, 1H), 6.94 - 6.86 (m, 1H), 5.32 (s, 2H), 4.57 - 4.46 (m, 1H), 3.58 - 3.42 (m, 4H), 3.26 - 3.22 (m, 1H), 3.03 - 2.93 (m, 3H), 2.84 - 2.78 (m, 1H), 2.77 - 2.70 (m, 1H), 2.69 (s, 3H), 2.67 - 2.52 (m, 4H), 2.42 (s, 3H), 2.28 - 2.21 (m, 1H), 1.93 - 1.84 (m, 1H), 1.19 (t, *J* = 7.5 Hz, 3H).

### Example 164

### N-(2-Chloro-4-(trifluoromethyl)phenyl)-2-(2-(4-(N,N-dimethylsulfamoyl)cyclohex-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide

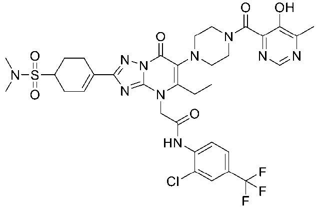

**The compound may also be synthesized according to the following steps:**

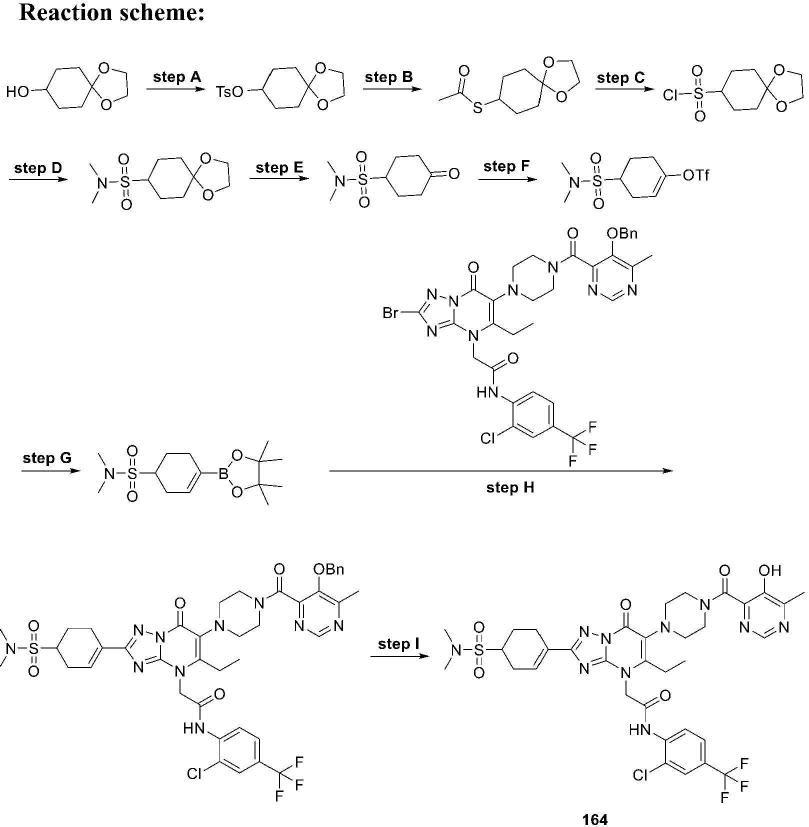

### Procedure:

Step A: 4-Hydroxycyclohexanone ethylene glycol ketal (10 g, 63.2 mmol) was dissolved in pyridine (40 mL), and *p*-toluenesulfonyl chloride (12 g, 63.2 mmol) was added to the reaction mixture. The reaction mixture was stirred at 55°C for 2 hours.

After TLC monitoring indicated the disappearance of the starting material, water was added to the reaction mixture, and the mixture was extracted with ethyl acetate (300 mL × 3). The combined organic phases were washed with saturated brine (100 mL × 3), dried over anhydrous sodium sulfate, filtered, and concentrated under reduced pressure. The resulting mixture was purified by silica gel column chromatography to obtain 1,4-dioxaspiro[4.5]decan-8-yl 4-methylbenzenesulfonate (5 g).

¹H NMR (400 MHz, DMSO-d₆) δ 7.80 (d, *J* = 8.2 Hz, 2H), 7.47 (d, *J* = 8.2 Hz, 2H), 4.67 - 4.59 (m, 1H), 3.83 (t, J = 3.6 Hz, 4H), 2.42 (s, 3H), 1.76 - 1.46 (m, 8H).

**Step B:** 1,4-Dioxaspiro[4.5]decan-8-yl 4-methylbenzenesulfonate (5 g, 16.0 mmol) and potassium thioacetate (3.6 g, 32.0 mmol) were sequentially added to *N,N-*dimethylformamide (20 mL). The reaction system was stirred at 80°C for 2 hours.

After TLC monitoring indicated the disappearance of the starting material, water was added to the reaction mixture, and the mixture was extracted with ethyl acetate (200 mL × 3). The combined organic phases were washed with saturated brine (50 mL × 3), dried over anhydrous sodium sulfate, filtered, and concentrated under reduced pressure. The resulting mixture was purified by silica gel column chromatography to obtain 8-ethynyl-1,4-dioxaspiro[4.5]decane (2.5 g).

¹H NMR (400 MHz, DMSO-d₆) δ 3.85 (s, 4H), 3.46 (s, 1H), 2.30 (s, 3H), 1.92 - 1.83 (m, 2H), 1.66 - 1.50 (m, 6H).

**Step C:** *N*-Chlorosuccinimide (6.1 g, 45.7 mmol) was dissolved in acetonitrile (60 mL), and hydrochloric acid (2 M in water, 6 mL) was added at 0°C. The reaction was maintained at this temperature for 15 minutes. Then, S-(1,4-dioxaspiro[4.5]decan-8-yl) thioacetate (3.3 g, 15.3 mmol) was added to the reaction mixture, and the reaction system was stirred at 0°C for 3 hours.

After TLC monitoring indicated the disappearance of the starting material, the reaction mixture was used directly in the next step without further treatment.

**Step D:** Dimethylamine hydrochloride (2.4 g, 29.9 mmol) was added to the reaction mixture from the previous step, followed by the addition of triethylamine (4.5 g, 44.8 mmol). The reaction mixture was stirred at room temperature for 2 hours.

After TLC monitoring indicated the disappearance of the starting material, water was added to the reaction mixture, and the mixture was extracted with ethyl acetate (150 mL × 3). The combined organic phases were washed with saturated brine (50 mL × 3), dried over anhydrous sodium sulfate, filtered, and concentrated under reduced pressure. The resulting mixture was purified by silica gel column chromatography to obtain 8-ethynyl-1,4-dioxaspiro[4.5]decane (700 mg).

¹H NMR (400 MHz, CDCl₃) δ 3.95 (s, 4H), 3.04 - 2.95 (m, 1H), 2.93 (s, 6H), 2.15 - 2.06 (m, 2H), 1.98 - 1.84 (m, 4H), 1.59 (m, 2H).

**Step E:** 8-Ethynyl-1,4-dioxaspiro[4.5]decane (700 mg, 2.8 mmol) was dissolved in acetonitrile (15 mL), and HCl (4 M in water) (10 mL) was added. The reaction system was stirred at room temperature for 3 hours.

After TLC monitoring indicated the disappearance of the starting material, the pH of the reaction mixture was adjusted to neutral with 2N sodium hydroxide solution, and the mixture was extracted with ethyl acetate (150 mL × 3). The combined organic phases were washed with saturated brine (30 mL × 3), dried over anhydrous sodium sulfate, filtered, and concentrated under reduced pressure. The resulting mixture was purified by normal-phase silica gel column chromatography to obtain S-(1,4-dioxaspiro[4.5]decan-8-yl) thioacetate (570 mg).

¹H NMR (400 MHz, CDCl₃) δ 3.41 - 3.34 (m, 1H), 2.96 (s, 6H), 2.65 - 2.56 (m, 2H), 2.44 - 2.33 (m, 4H), 2.16 - 2.06 (m, 2H).

**Step F:** Compound 4-(2-methyloxazol-5-yl)cyclohexan-1-one (300 mg, 1.5 mmol) was dissolved in anhydrous tetrahydrofuran (10 mL). The reaction system was evacuated and purged with nitrogen, and then LiHMDS (1 M, 1.7 mL) was added dropwise at -78°C. After the dropwise addition was completed, the reaction mixture was stirred at -78°C for 2 hours. Then, N-phenylbis(trifluoromethanesulfonyl)imide (626 mg, 1.7 mmol) was dissolved in anhydrous tetrahydrofuran and then added dropwise to the reaction flask. The reaction system was slowly warmed to room temperature and then stirred for 16 hours.

After TLC monitoring indicated the disappearance of the starting material, the reaction mixture was quenched with saturated aqueous ammonium chloride solution, and then extracted with ethyl acetate. The organic phase was dried over anhydrous sodium sulfate, filtered, and concentrated under reduced pressure. The crude product was purified by reverse-phase chromatography (eluent: water (0.1% ammonium bicarbonate)/acetonitrile from 95:5 to 70:30) to obtain 4-(*N,N-*dimethylsulfamoyl)cyclohex-1-en-1-yl trifluoromethanesulfonate (320 mg).

¹H NMR (400 MHz, CDCl₃) δ 5.83 - 5.77 (m, 1H), 3.28 - 3.15 (m, 1H), 2.94 (s, 6H), 2.60 - 2.54 (m, 2H), 2.53 - 2.47 (m, 2H), 2.39 - 2.29 (m, 1H), 2.08 - 1.94 (m, 1H).

**Step G:** Compound 4-(*N,N*-dimethylsulfamoyl)cyclohex-1-en-1-yl trifluoromethanesulfonate (320 mg, yellow oil, crude product) and bis(pinacolato)diboron (317 mg, 1.2 mmol) were dissolved in 1,4-dioxane (10 mL). Then, potassium acetate (282 mg, 2.8 mmol) and [1,1'-bis(diphenylphosphino)ferrocene]dichloropalladium(II) (70 mg, 96.05 µmol) were added. The reaction system was purged with nitrogen, and then stirred at 100°C for 16 hours.

After LCMS monitoring indicated the disappearance of the starting material, the reaction mixture was used directly in the next step without further treatment.

MS (ESI) M/Z: 316.2 [M+H] ⁺.

**Step H:** Compound *N,N-*dimethyl-4-(4,4,5,5-tetramethyl-1,3,2-dioxaborolan-2-yl)cyclohex-3-ene-1-sulfonamide (crude product) and 2-(6-(4-(5-(benzyloxy)-6-methylpyrimidine-4-carbonyl)piperazin-1-yl)-2-bromo-5-ethyl-7-oxo-[1,2,4]triazolo[1,5-a]pyrimidin-4(7H)-yl)-*N*-(2-chloro-4-(trifluoromethyl)phenyl)acetamide (250 mg, 317.2 µmol) were dissolved in 1,4-dioxane (10 mL) and water (2 mL). Then, sodium carbonate (100 mg, 951.6 µmol) and [1,1'-bis(diphenylphosphino)ferrocene]dichloropalladium(II) (23 mg, 31.7 µmol) were added. The reaction system was purged with nitrogen, and then stirred at 80°C for 2 hours.

After LCMS monitoring indicated the disappearance of the starting material and the formation of the product, water was added to the reaction mixture, and the mixture was extracted with ethyl acetate (100 mL × 3) and then concentrated under reduced pressure to obtain a crude product. The crude product was purified by reverse-phase C18 column chromatography (eluent: water (0.1% ammonium bicarbonate)/acetonitrile from 95/5 to 70/30) to obtain the product 2-(6-(4-(5-(benzyloxy)-6-methylpyrimidine-4-formyl)piperazin-1-yl)-2-(4-(N,N-dimethylsulfamoyl)cyclohex-1-en-1-yl)-5-ethyl-7-oxo-[1,2,4]triazolo[1,5-a]pyrimidin-4(7H)-yl)-*N*-(2-chloro-4-(trifluoromethyl)phenyl)acetamide (80 mg).

MS (ESI) M/Z: 897.5 [M+H] ⁺.

**Step I:** Compound 2-(6-(4-(5-(benzyloxy)-6-methylpyrimidine-4-formyl)piperazin-1-yl)-2-(4-(*N,N*-dimethylsulfamoyl)cyclohex-1-en-1-yl)-5-ethyl-7-oxo-[1,2,4]triazolo[1,5-a]pyrimidin-4(7H)-yl)-N-(2-chloro-4-(trifluoromethyl)phenyl)acetamide (80 mg, 89.1 µmol) was dissolved in trifluoroacetic acid (3 mL), and the reaction mixture was stirred at 60°C for 1 hour.

After LCMS monitoring indicated the disappearance of the starting material, the reaction mixture was diluted with dichloromethane and then concentrated under reduced pressure. The resulting mixture was purified by preparative high-performance liquid chromatography. The purification conditions were as follows: chromatographic column: xbridge-C18 19 mm × 250 mm; mobile phase: water (containing 0.1% formic acid) and acetonitrile; flow rate: 20 mL/min; gradient: acetonitrile from 60% to 65% within 20 minutes; detection wavelength: 254 nm. The product was collected and lyophilized under reduced pressure to obtain the final product N-(2-chloro-4-(trifluoromethyl)phenyl)-2-(2-(4-(N,N-dimethylsulfamoyl)cyclohex-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide (23 mg).

MS (ESI) M/Z: 807.4 [M+H] ⁺.

¹H NMR (400 MHz, DMSO-d₆) δ 10.36 (s, 1H), 8.55 (s, 1H), 8.06 (d, *J* = 8.5 Hz, 1H), 8.00 - 7.93 (m, 1H), 7.72 (dd, *J* = 8.8, 2.2 Hz, 1H), 6.88 - 6.76 (m, 1H), 5.31 (s, 2H), 4.59 - 4.45 (m, 1H), 3.60 - 3.40 (m, 4H), 3.27 - 3.20 (m, 1H), 3.06 - 2.92 (m, 3H), 2.86 (s, 6H), 2.84 - 2.72 (m, 2H), 2.69 - 2.55 (m, 2H), 2.48 - 2.44 (m, 2H), 2.44 (s, 3H), 2.27 - 2.19 (m, 1H), 1.77 - 1.62 (m, 1H), 1.18 (t, *J =* 7.5 Hz, 3H).

### Example 193

### N-(3-Chloro-4-(trifluoromethyl)thiophen-2-yl)-2-(5-ethyl-6-(4-(5-hydroxy-6-methylpyrimidine-4-carbonyl)piperazin-1-yl)-7-oxo-2-(2,5,6,7-tetrahydrooxepin-4-yl)-[1,2,4]triazolo[1,5-a]pyrimidin-4(7H)-yl)acetamide

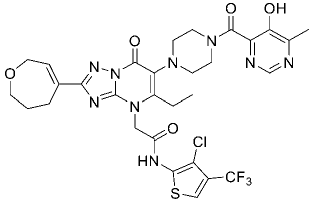

**The compound may also be synthesized according to the following steps:**

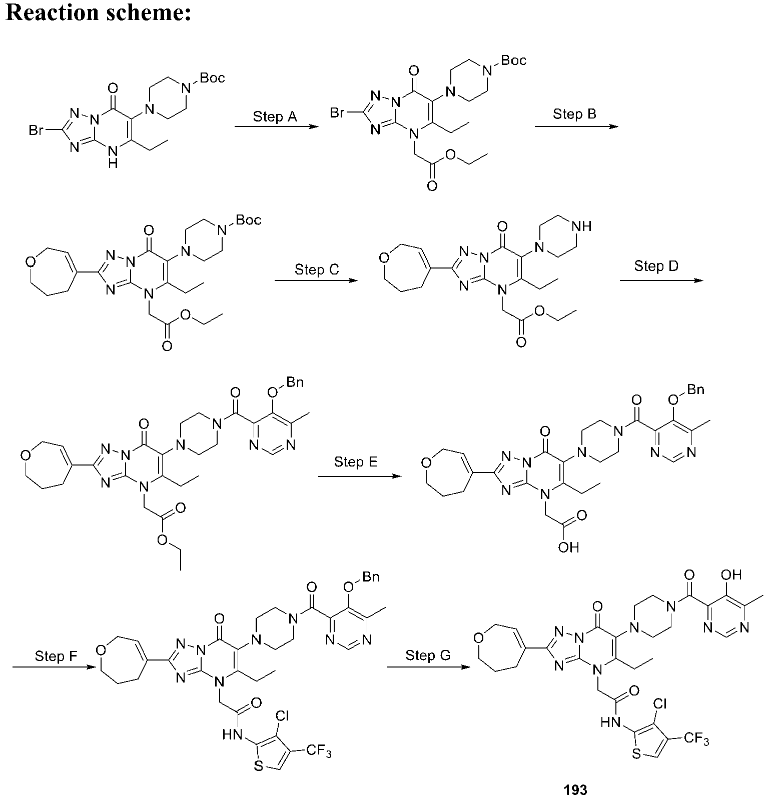

### Procedure:

**Step A:** Compound *tert*-butyl 4-(2-bromo-5-ethyl-7-oxo-4,7-dihydro-[1,2,4]triazolo[1,5-a]pyrimidin-6-yl)piperazine-1-carboxylate (20.0 g, 46.8 mmol) was dissolved in 1,4-dioxane (100 mL). Then, N,N-diisopropylethylamine (18.1 g, 140.4 mmol) and ethyl 2-bromoacetate (11.7 g, 70.1 mmol) were added, and the reaction mixture was stirred at 80°C overnight.

After LCMS monitoring indicated the disappearance of the starting material, water (200 mL) was added to the reaction system, and the mixture was extracted with ethyl acetate (300 mL × 2). The combined organic phases were first washed with saturated brine (50 mL × 3), then dried over anhydrous sodium sulfate, filtered, and finally concentrated under reduced pressure. The resulting residue was purified by silica gel column chromatography to obtain compound *tert*-butyl 4-(2-bromo-4-(2-ethoxy-2-oxoethyl)-5-ethyl-7-oxo-4,7-dihydro-[1,2,4]triazolo[1,5-a]pyrimidin-6-yl)piperazine-1-carboxylate (13.0 g).

MS (ESI) M/Z: 535.2 [M+Na] ⁺.

**Step B:** Compound *tert*-butyl 4-(2-bromo-4-(2-ethoxy-2-oxoethyl)-5-ethyl-7-oxo-4,7-dihydro-[1,2,4]triazolo[1,5-a]pyrimidin-6-yl)piperazine-1-carboxylate (344 mg, 669.3 µmol) and 4,4,5,5-tetramethyl-2-(2,5,6,7-tetrahydrooxepin-4-yl)-1,3,2-dioxaborolane (150 mg, 669.3 µmol) were dissolved in 1,4-dioxane (10 mL) and water (1 mL). Then, sodium carbonate (213 mg, 2.0 mmol) and [1,1'-bis(diphenylphosphino)ferrocene]dichloropalladium(II) (55 mg, 66.9 µmol) were added. The reaction system was purged with nitrogen, and then stirred at 100°C for 2 hours.

After LCMS monitoring indicated the disappearance of the starting material, water (50 mL) was added to the reaction system, and the mixture was extracted with ethyl acetate (80 mL × 3). The combined organic phases were first washed with saturated brine (10 mL × 3), then dried over anhydrous sodium sulfate, filtered, and finally concentrated under reduced pressure. The resulting residue was purified by silica gel column chromatography to obtain compound *tert*-butyl 4-(4-(2-ethoxy-2-oxoethyl)-5-ethyl-7-oxo-2-(2,5,6,7-tetrahydrooxepin-4-yl)-4,7-dihydro-[1,2,4]triazolo[1,5-a]pyrimidin-6-yl)piperazine-1-carboxylate (120 mg).

MS (ESI) M/Z: 475.3 [M-56+H] ⁺.

**Step C:** Compound *tert*-butyl 4-(4-(2-ethoxy-2-oxoethyl)-5-ethyl-7-oxo-2-(2,5,6,7-tetrahydrooxepin-4-yl)-4,7-dihydro-[1,2,4]triazolo[1,5-a]pyrimidin-6-yl)piperazine-1-carboxylate (120 mg, 226.2 µmol) was dissolved in dichloromethane (2 mL), and then trifluoroacetic acid (1 mL) was added. The reaction mixture was stirred at room temperature for 1 hour.

After LCMS monitoring indicated the disappearance of the starting material, the reaction mixture was diluted with dichloromethane and then concentrated under reduced pressure to obtain compound ethyl 2-(5-ethyl-7-oxo-6-(piperazin-1-yl)-2-(2,5,6,7-tetrahydrooxepin-4-yl)-[1,2,4]triazolo[1,5-a]pyrimidin-4(7H)-yl)acetate (100 mg, crude product). The crude product was used directly in the next step.

MS (ESI) M/Z: 431.3 [M+H] ⁺.

**Step D:** Compound ethyl 2-(5-ethyl-7-oxo-6-(piperazin-1-yl)-2-(2,5,6,7-tetrahydrooxepin-4-yl)-[1,2,4]triazolo[1,5-a]pyrimidin-4(7H)-yl)acetate (100 mg, crude product) and 5-benzyloxy-6-methylpyrimidine-4-carboxylic acid (55 mg, 225.3 µmol) were dissolved in acetonitrile (5 mL). Then, *N,N,N',N*'-tetramethylchloroformamidinium hexafluorophosphate (73 mg, 270.4 µmol) and *N*-methylimidazole (93 mg, 1.1 mmol) were added, and the reaction mixture was stirred at room temperature for 2 hours.

After LCMS monitoring indicated the disappearance of the starting material, water (20 mL) was added to the reaction system, and the mixture was extracted with ethyl acetate (40 mL × 2). The combined organic phases were first washed with saturated brine (10 mL × 2), then dried over anhydrous sodium sulfate, filtered, and finally concentrated under reduced pressure. The resulting residue was purified by silica gel column chromatography to obtain compound ethyl 2-(6-(5-(benzyloxy)-5-methylpyrimidine-4-carbonyl)-1-yl)-5-ethyl-7-oxo-2-(2,5,6,7-tetrahydrooxepin-4-yl)-[1,2,4]pyrimidin-4(7H)-yl)-[1,2,5-a]pyrimidin-4(7H)-yl)-acetate (90 mg).

MS (ESI) M/Z: 657.4 [M+H] ⁺.

**Step E:** Compound ethyl 2-(6-(5-(benzyloxy)-5-methylpyrimidine-4-carbonyl)-1-yl)-5-ethyl-7-oxo-2-(2,5,6,7-tetrahydrooxepin-4-yl)-[1,2,4]pyrimidin-4(7H)-yl)-[1,2,5-a]pyrimidin-4(7H)-yl)-acetate (90 mg, 137.0 µmol) was dissolved in methanol (2 mL), and then 2 M aqueous sodium hydroxide solution (2 mL) was added. The reaction mixture was stirred at room temperature for 1 hour.

After LCMS monitoring indicated the disappearance of the starting material, 1 N aqueous hydrochloric acid solution was added to the reaction system to adjust the pH to weakly acidic, and then the mixture was extracted with ethyl acetate (20 mL × 3). The combined organic phases were first washed with saturated brine (5 mL × 3), then dried over anhydrous sodium sulfate, filtered, and finally concentrated under reduced pressure. Compound 2-(6-(4-(5-(benzyloxy)-6-methylpyrimidine-4-carbonyl)piperazin-1-yl)-5-ethyl-7-oxo-2-(2,5,6,7-tetrahydrooxepin-4-yl)-[1,2,4]triazolo[1,5-a]pyrimidin-4(7H)-yl)acetic acid (60 mg, crude product used directly in the next step) was obtained.

MS (ESI) M/Z: 629.4 [M+H] ⁺.

**Step F:** Compound 2-(6-(4-(5-(benzyloxy)-6-methylpyrimidine-4-carbonyl)piperazin-1-yl)-5-ethyl-7-oxo-2-(2,5,6,7-tetrahydrooxepin-4-yl)-[1,2,4]triazolo[1,5-a]pyrimidin-4(7H)-yl)acetic acid (60 mg, crude product) and 3-chloro-4-trifluoromethylthiophen-2-amine hydrochloride (20 mg, 95.5 µmol) were dissolved in acetonitrile (4 mL). Then, *N,N,N',N'*-tetramethylchloroformamidinium hexafluorophosphate (54 mg, 190.9 µmol) and *N*-methylimidazole (40 mg, 477.2 µmol) were added, and the reaction mixture was stirred at room temperature for 16 hours.

After LCMS monitoring indicated the disappearance of the starting material, water (20 mL) was added to the reaction system, and the mixture was extracted with ethyl acetate (40 mL × 2). The combined organic phases were first washed with saturated brine (10 mL × 2), then dried over anhydrous sodium sulfate, filtered, and finally concentrated under reduced pressure. The resulting residue was purified by silica gel column chromatography (eluent: dichloromethane/methanol = 10/1) to obtain compound 2-(6-(4-(5-(benzyloxy)-6-methylpyrimidine-4-carbonyl)piperazin-1-yl)-5-ethyl-7-oxo-2-(2,5,6,7-tetrahydrooxepin-4-yl)-[1,2,4]triazolo[1,5-a]pyrimidin-4(7H)-yl)-*N*-(3-chloro-4-(trifluoromethyl)thiophen-2-yl)acetamide (30 mg).

MS (ESI) M/Z: 812.5 [M+H] ⁺.

**Step G:** Compound 2-(6-(4-(5-(benzyloxy)-6-methylpyrimidine-4-carbonyl)piperazin-1-yl)-5-ethyl-7-oxo-2-(2,5,6,7-tetrahydrooxepin-4-yl)-[1,2,4]triazolo[1,5-a]pyrimidin-4(7H)-yl)-*N*-(3-chloro-4-(trifluoromethyl)thiophen-2-yl)acetamide (30 mg, 36.9 µmol) was dissolved in trifluoroacetic acid (2 mL), and the reaction mixture was stirred at 60°C for 1 hour.

After LCMS monitoring indicated the disappearance of the starting material, the reaction mixture was diluted with dichloromethane and then concentrated under reduced pressure. The resulting mixture was purified by preparative high-performance liquid chromatography. The purification conditions were as follows: chromatographic column: xbridge-C18 21.2 mm × 150 mm; mobile phase: water (containing 0.1% ammonium bicarbonate) and acetonitrile; flow rate: 25 mL/min; gradient: acetonitrile from 30% to 50% within 15 minutes; detection wavelength: 254 nm. The product was collected and lyophilized under reduced pressure to obtain the final product *N*-(3-chloro-4-(trifluoromethyl)thiophen-2-yl)-2-(5-ethyl-6-(4-(5-hydroxy-6-methylpyrimidine-4-carbonyl)piperazin-1-yl)-7-oxo-2-(2,5,6,7-tetrahydrooxepin-4-yl)-[1,2,4]triazolo[1,5-a]pyrimidin-4(7H)-yl)acetamide (3.40 mg).

MS (ESI) M/Z: 722.4 [M+H] ⁺.

¹H NMR (400 MHz, DMSO-d₆) δ 11.59 (s, 1H), 10.22 (s, 1H), 8.58 (s, 1H), 7.89 (s, 1H), 6.87 (t, *J* = 4.7 Hz, 1H), 5.32 (s, 2H), 4.57 - 4.45 (m, 1H), 4.25 (d, *J* = 4.7 Hz, 2H), 3.80 (t, *J* = 5.8 Hz, 2H), 3.57 - 3.43 (m, 3H), 3.26 - 3.19 (m, 1H), 3.03 - 2.90 (m, 3H), 2.86 - 2.76 (m, 3H), 2.69 - 2.59 (m, 1H), 2.44 (s, 3H), 1.91 - 1.81 (m, 2H), 1.15 (t, *J* = 7.4 Hz, 3H).

### Example 169

### N-(3,5-Dichloro-4-(trifluoromethyl)thiophen-2-yl)-2-(2-(3,6-dihydro-2H-pyran-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide

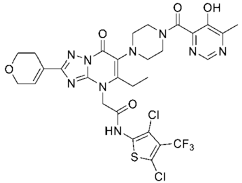

*N*-(3,5-Dichloro-4-(trifluoromethyl)thiophen-2-yl)-2-(2-(3,6-dihydro-2*H*-pyran-4-yl)-5-ethyl-6-(4-(5-hydroxy-6-methylpyrimidine-4-carbonyl)piperazin-1-yl)-7-oxo-**[1,2,4]triazolo[1,5-a]pyrimidin-4(7H)-yl)acetamide** (16.9 mg) **may also** be prepared with reference to the preparation method of **Example 129.**

MS (ESI) M/Z: 742.4 [M+H] ⁺.

¹H NMR (400 MHz, DMSO-d₆) δ 12.05 (s, 1H), 10.23 (s, 1H), 8.58 (s, 1H), 6.86 - 6.75 (m, 1H), 5.25 (s, 2H), 4.57 - 4.47 (m, 1H), 4.29 - 4.19 (m, 2H), 3.80 (t, *J* = 5.4 Hz, 2H), 3.57 - 3.43 (m, 3H), 3.28 - 3.20 (m, 1H), 3.04 - 2.88 (m, 3H), 2.87 - 2.78 (m, 1H), 2.70 - 2.60 (m, 1H), 2.50 (m, 2H), 2.45 (s, 3H), 1.15 (t, *J* = 7.4 Hz, 3H).

### Example 214

### 2-(2-(3,6-Dihydro-2H-pyran-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(7-(trifluoromethyl)benzofuran-4-yl)acetamide

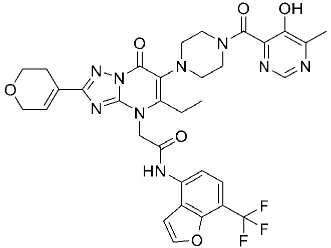

**The compound may also be synthesized according to the following steps:**

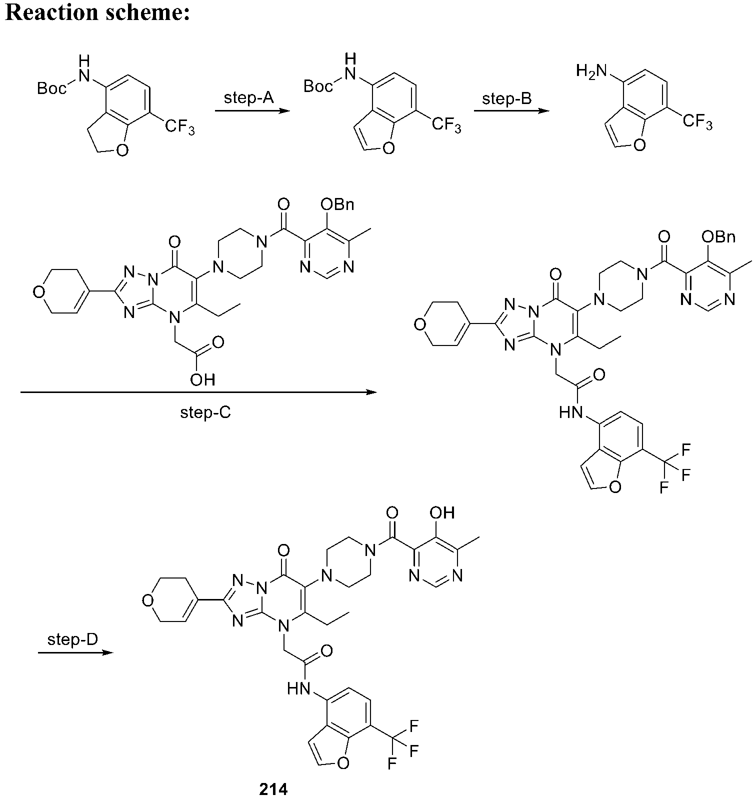

### Procedure:

**Step A:** In a microwave tube, *tert*-butyl (7-(trifluoromethyl)-2,3-dihydrobenzofuran-4-yl)carbamate (400 mg, 1.2 mmol) was dissolved in 1,4-dioxane (4 mL). 2,3-Dicyano-5,6-dichlorobenzoquinone (600 mg, 2.6 mmol) was added to the reaction system, and the reaction system was stirred at 100°C for 48 hours.

After LCMS monitoring indicated the disappearance of the starting material, water was added to the reaction mixture, and the mixture was extracted with ethyl acetate (100 mL × 3) and washed with saturated brine (30 mL × 3). The combined organic phases were dried over anhydrous sodium sulfate, filtered, and concentrated under reduced pressure. The resulting mixture was purified by silica gel column chromatography to obtain *tert*-butyl (7-(trifluoromethyl)benzofuran-4-yl)carbamate (300 mg).

MS (ESI) M/Z: 300.1 [M-H]⁻.

**Step B:** *tert*-Butyl (7-(trifluoromethyl)benzofuran-4-yl)carbamate (300 mg, 995.8 µmol) was dissolved in dichloromethane (3 mL), and then a solution of hydrochloric acid in dioxane (4 M, 3 mL) was added. The reaction mixture was stirred at room temperature for 2 hours.

After LCMS monitoring indicated the disappearance of the starting material, the reaction mixture was directly concentrated under reduced pressure to obtain 7-(trifluoromethyl)benzofuran-4-amine (200 mg).

MS (ESI) M/Z: 200.0 [M-H] ⁻.

**Step C:** 7-(Trifluoromethyl)benzofuran-4-amine (100 mg, crude product) and 2-(6-(4-(5-(benzyloxy)-6-methylpyrimidine-4-carbonyl)piperazin-1-yl)-2-(3,6-dihydro-2H-pyran-4-yl)-5-ethyl-7-oxo-[1,2,4]triazolo[1,5-a]pyrimidin-4(7H)-yl)acetic acid (150 mg, 244.0 µmol) were dissolved in acetonitrile (5 mL). *N,N,N',N'*-Tetramethylchloroformamidinium hexafluorophosphate (82 mg, 292.8 µmol) and *N*-methylimidazole (100 mg, 1.2 mmol) were added, and the reaction mixture was stirred at room temperature for 1 hour.

After LCMS monitoring indicated the disappearance of the starting material, water was added to the reaction mixture, and the mixture was extracted with ethyl acetate (100 mL × 3). The combined organic phases were washed with saturated brine (20 mL × 3), dried over anhydrous sodium sulfate, filtered, and concentrated under reduced pressure. The resulting mixture was purified by silica gel column chromatography to obtain 2-(6-(4-(5-(benzyloxy)-6-methylpyrimidine-4-carbonyl)piperazin-1-yl)-2-(3,6-dihydro-2H-pyran-4-yl)-5-ethyl-7-oxo-[1,2,4]triazolo[1,5-a]pyrimidin-4(7H)-yl)-*N*-(7-(trifluoromethyl)benzofuranyl)acetamide (100 mg).

MS (ESI) M/Z: 798.5 [M+H] ⁺.

**Step D:** 2-(6-(4-(5-(Benzyloxy)-6-methylpyrimidine-4-carbonyl)piperazin-1-yl)-2-(3,6-dihydro-2H-pyran-4-yl)-5-ethyl-7-oxo-[1,2,4]triazolo[1,5-a]pyrimidin-4(7H)-yl)-*N*-(7-(trifluoromethyl)benzofuranyl)acetamide (100 mg, 125.4 µmol) was dissolved in trifluoroacetic acid (3 mL), and the reaction system was stirred at 60°C for 1 hour.

After LCMS monitoring indicated the disappearance of the starting material, the reaction mixture was diluted with dichloromethane (60 mL) and then concentrated under reduced pressure. The resulting mixture was purified by preparative high-performance liquid chromatography. The purification conditions were as follows: chromatographic column: xbridge-C18 21.2 mm × 150 mm; mobile phase: water (containing 0.1% formic acid) and acetonitrile; flow rate: 25 mL/min; gradient: acetonitrile from 35% to 55% within 15 minutes; detection wavelength: 254 nm. The product was collected and lyophilized under reduced pressure to obtain 2-(2-(3,6-dihydro-2H-pyran-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-*N*-(7-(trifluoromethyl)benzofuran-4-yl)acetamide (14.91 mg).

MS (ESI) M/Z: 708.5 [M+H] ⁺.

¹H NMR (400 MHz, DMSO-d₆) δ 10.76 (s, 1H), 8.52 (s, 1H), 8.17 (d, *J* = 2.2 Hz, 1H), 7.96 (d, *J* = 8.0 Hz, 1H), 7.63 (d, *J* = 8.5 Hz, 1H), 7.38 (d, *J* = 2.3 Hz, 1H), 6.82 - 6.76 (m, 1H), 5.33 (s, 2H), 4.58 - 4.48 (m, 1H), 4.27 - 4.18 (m, 2H), 3.79 (t, *J =* 5.4 Hz, 2H), 3.57 - 3.44 (m, 3H), 3.26 - 3.21 (m, 1H), 3.07 - 2.93 (m, 3H), 2.87 - 2.77 (m, 1H), 2.69 - 2.61 (m, 1H), 2.50 (m, 2H), 2.43 (s, 3H), 1.19 (t, *J* = 7.4 Hz, 3H).

### Example 232

### N-(2-Chloro-5-(trifluoromethyl)thiophen-3-yl)-2-(5-ethyl-6-(4-(5-hydroxy-6-methylpyrimidine-4-carbonyl)piperazin-1-yl)-7-oxo-2-(2,5,6,7-tetrahydrooxepin-4-yl)-[1,2,4] triazolo [1,5-a] pyrimidin-4(7H)-yl)acetamide

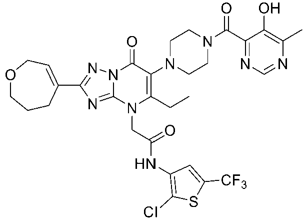

**The compound may also be synthesized according to the following steps:**

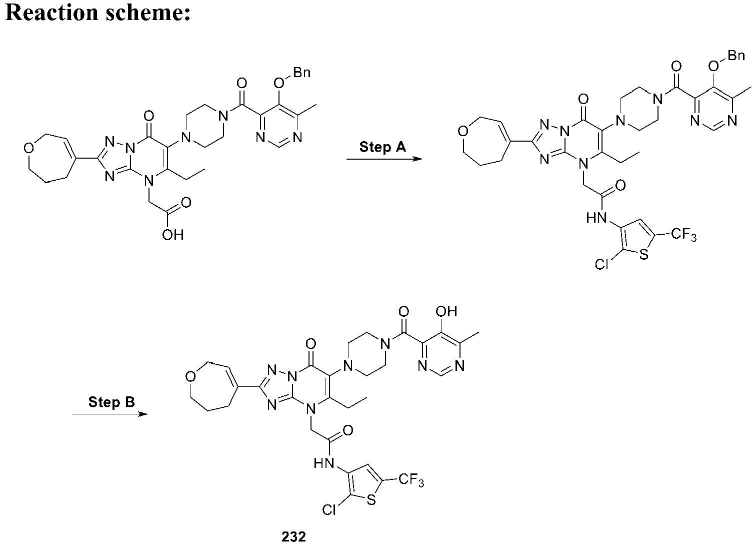

### Procedure:

**Step A:** 2-(6-(4-(5-(Benzyloxy)-6-methylpyrimidine-4-carbonyl)piperazin-1-yl)-5-ethyl-7-oxo-2-(2,5,6,7-tetrahydrooxepin-4-yl)-[1,2,4]triazolo[1,5-a]pyrimidin-4(7H)-yl)acetic acid (68 mg, 109.1 µmol) and 2-chloro-5-(trifluoromethyl)thiophen-3-amine (20 mg, 99.2 µmol) were dissolved in acetonitrile (2 mL), followed by the sequential addition of *N,N,N',N-*Tetramethylchloroformamidinium hexafluorophosphate (83 mg, 297.6 µmol) and *N-*methylimidazole (24 mg, 297.6 µmol). The reaction mixture was stirred at room temperature for 1 hour.

After LCMS monitoring indicated the disappearance of the starting material, water was added to the reaction mixture, and the mixture was extracted with ethyl acetate (50 mL × 3). The combined organic phases were dried over anhydrous sodium sulfate, filtered, and concentrated under reduced pressure. The resulting mixture was purified by Prep-TLC (developing solvent: dichloromethane:methanol = 15:1) to obtain 2-[6-[4-(5-benzyloxy-6-methylpyrimidine-4-carbonyl)piperazin-1-yl]-5-ethyl-7-oxo-2-(2,3,4,7-tetrahydrooxepin-5-yl)-[1,2,4]triazolo[1,5-a]pyrimidin-4-yl]-*N*-[2-chloro-5-(trifluoromethyl)-3-thienyl]acetamide (20 mg).

MS (ESI) M/Z: 812.4 [M+H] ⁺.

**Step B**: 2-[6-[4-(5-Benzyloxy-6-methylpyrimidine-4-carbonyl)piperazin-1-yl]-5-ethyl-7-oxo-2-(2,3,4,7-tetrahydrooxepin-5-yl)-[1,2,4]triazolo[1,5-a]pyrimidin-4-yl]-*N*-[2-chloro-5-(trifluoromethyl)-3-thienyl]acetamide (20 mg, 22.16 µmol) was dissolved in trifluoroacetic acid (3 mL), and the reaction mixture was stirred at 60°C for 1 hour.

After LCMS monitoring indicated the disappearance of the starting material, the reaction mixture was diluted with dichloromethane and then concentrated under reduced pressure. The resulting mixture was purified by preparative high-performance liquid chromatography. The purification conditions were as follows: chromatographic column: xbridge-C18 21.2 mm × 150 mm; mobile phase: water (containing 0.1% formic acid) and acetonitrile; flow rate: 25 mL/min; gradient: acetonitrile from 40% to 60% within 15 minutes; detection wavelength: 254 nm. *N*-[2-Chloro-5-(trifluoromethyl)-3-thienyl]-2-[5-ethyl-6-[4-(5-hydroxy-6-methylpyrimidine-4-carbonyl)piperazin-1-yl]-7-oxo-2-(2,3,4,7-tetrahydrooxepin-5-yl)-[1,2,4]triazolo[1,5-a]pyrimidin-4-yl]acetamide (3.77 mg) was obtained.

MS (ESI) M/Z: 722.4 [M+H] ⁺.

¹H NMR (400 MHz, DMSO-d₆) δ 10.35 (s, 1H), 8.47 (s, 1H), 7.99 - 7.92 (m, 1H), 6.89 (t, *J* = 4.6 Hz, 1H), 5.22 (s, 2H), 4.57 - 4.47 (m, 1H), 4.27 (d, *J* = 4.7 Hz, 2H), 3.81 (t, *J* = 5.8 Hz, 2H), 3.59 - 3.42 (m, 3H), 3.25 - 3.21 (m, 1H), 3.02 - 2.88 (m, 3H), 2.87 - 2.76 (m, 3H), 2.67 - 2.59 (m, 1H), 2.41 (s, 3H), 1.91 - 1.82 (m, 2H), 1.17 (t, *J* = 7.4 Hz, 3H).

### Example 122

### N-(2-Chloro-4-(trifluoromethyl)phenyl)-2-(5-ethyl-6-(4-(5-hydroxy-6-methylpyrimidine-4-formyl)piperazin-1-yl)-2-(4-(2-methyloxazol-5-yl)cyclohex-1-en-1-yl)-7-oxo-[1,2,4]triazolo[1,5-a]pyrimidin-4(7H)-yl)acetamide

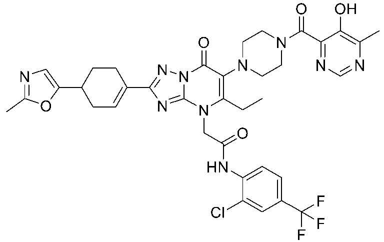

The product *N*-(2-chloro-4-(trifluoromethyl)phenyl)-2-(5-ethyl-6-(4-(5-hydroxy-6-methylpyrimidine-4-formyl)piperazin-1-yl)-2-(4-(2-methyloxazol-5-yl)cyclohex-1-en-1-yl)-7-oxo-[1,2,4]triazolo[1,5-a]pyrimidin-4(7H)-yl)acetamide (9.5 mg) may also be prepared with reference to the preparation method of **Example 65.**

MS (ESI) M/Z: 781.5 [M+H] ⁺.

¹H NMR (400 MHz, DMSO-*d*₆) δ 10.35 (s, 1H), 10.22 (s, 1H), 8.55 (s, 1H), 8.09 - 8.04 (m, 1H), 7.96 (d, *J* = 1.5 Hz, 1H), 7.72 (dd, *J* = 8.8, 1.6 Hz, 1H), 6.92 - 6.86 (m, 1H), 6.75 (s, 1H), 5.31 (s, 2H), 4.56 - 4.49 (m, 1H), 3.58 - 3.44 (m, 3H), 3.27 - 3.18 (m, 1H), 3.04 - 2.94 (m, 4H), 2.85 - 2.78 (m, 1H), 2.65 - 2.55 (m, 2H), 2.54 - 2.51 (m, 2H), 2.43 (s, 3H), 2.35 (s, 3H), 2.32 - 2.27 (m, 1H), 2.17 - 2.11 (m, 1H), 1.76 - 1.67 (m, 1H), 1.19 (t, *J* = 7.5 Hz, 3H).

### Example 156

### 4-(4-(2-((2-Chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)cyclohex-3-en-1-yl dimethylcarbamate

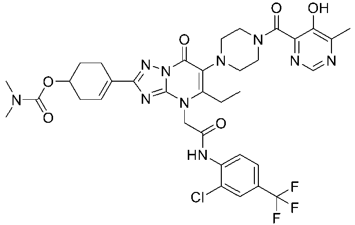

The product 4-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)cyclohex-3-en-1-yl dimethylcarbamate (5.63 mg) may also be prepared **with reference to the preparation method of Example 93.**

MS (ESI) M/Z: 787.4 [M+H] ⁺.

¹H NMR (400 MHz, DMSO-*d*₆) δ 10.40 (s, 1H), 8.39 (s, 1H), 8.05 (d, *J* = 8.6 Hz, 1H), 7.96 (d, *J* = 2.1 Hz, 1H), 7.71 (dd, *J* = 8.8, 2.1 Hz, 1H), 6.79 - 6.70 (m, 1H), 5.31 (s, 2H), 4.91 - 4.82 (m, 1H), 4.58 - 4.45 (m, 1H), 3.53 - 3.42 (m, 3H), 3.24 - 3.19 (m, 1H), 3.03 - 2.90 (m, 3H), 2.84 - 2.81 (m, 1H), 2.80 (s, 6H), 2.66 - 2.54 (m, 4H), 2.39 (s, 3H), 2.31 - 2.22 (m, 1H), 1.94 - 1.79 (m, 2H), 1.19 (t, *J* = 7.4 Hz, 3H).

### Example 115

### 2-(2-(4-(1H-Pyrazol-1-yl)cyclohex-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamide

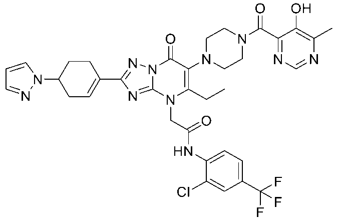

2-(2-(4-(1H-Pyrazol-1-yl)cyclohex-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-*N*-(2-chloro-4-(trifluoromethyl)phenyl)acetamide (22.69 mg) may also be prepared with reference to the preparation method of **Example 65.**

MS (ESI) M/Z: 766.5 [M+H] ⁺.

¹H NMR (400 MHz, DMSO-*d*₆) δ 10.36 (s, 1H), 8.53 (s, 1H), 8.06 (d, *J* = 8.6 Hz, 1H), 7.96 (d, *J* = 2.1 Hz, 1H), 7.79 (d, *J* = 2.3 Hz, 1H), 7.72 (dd, *J* = 8.8, 2.1 Hz, 1H), 7.45 (d, *J* = 1.8 Hz, 1H), 6.92 - 6.83 (m, 1H), 6.24 (t, *J* = 2.1 Hz, 1H), 5.32 (s, 2H), 4.58 - 4.44 (m, 2H), 3.59 - 3.43 (m, 3H), 3.25 - 3.19 (m, 1H), 3.05 - 2.92 (m, 3H), 2.87 - 2.78 (m, 1H), 2.75 - 2.53 (m, 5H), 2.43 (s, 3H), 2.22 - 2.07 (m, 2H), 1.19 (t, *J* = 7.2 Hz, 3H).

### Example 201

### N-(2-Chloro-4-(trifluoromethyl)phenyl)-2-(5-ethyl-6-(4-(5-hydroxy-6-methylpyrimidine-4-carbonyl)piperazin-1-yl)-7-oxo-2-(2,5,6,7-tetrahydrooxepin-3-yl)-[1,2,4]triazolo[1,5-a]pyrimidin-4(7H)-yl)acetamide

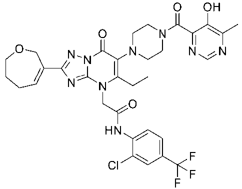

**The compound may also be synthesized according to the following steps:**

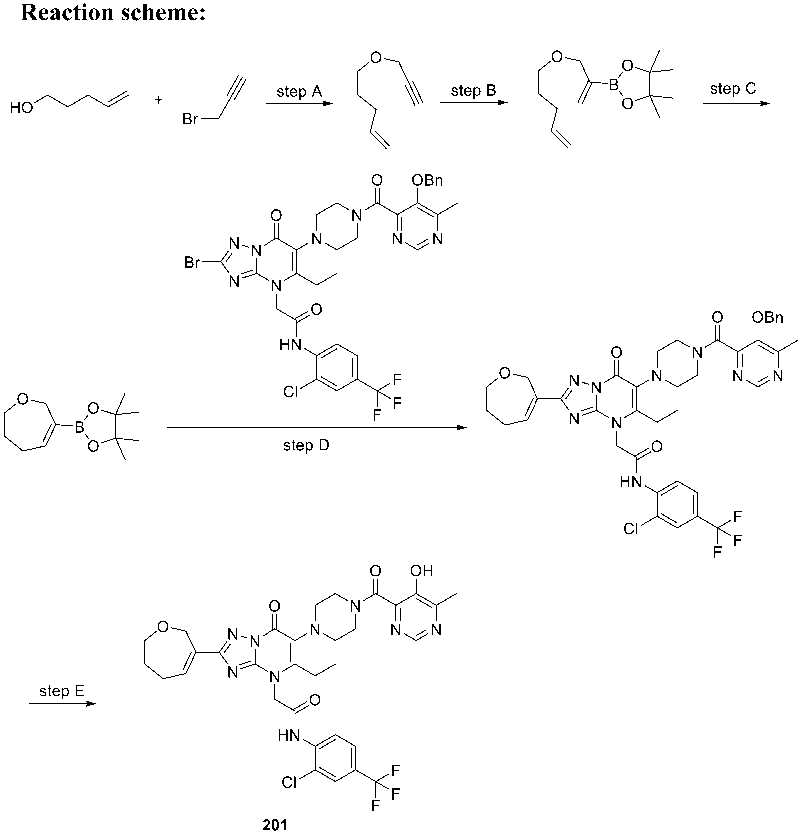

### Procedure:

**Step A:** Sodium hydride (60%, 947.4 mg, 23.7 mmol) was added to anhydrous *N,N-*dimethylformamide (20 mL), and the mixture was cooled to 0°C. 4-Penten-1-ol (1.7 g, 19.7 mmol) was dissolved in anhydrous *N,N*-dimethylformamide (5 mL), and the solution was added dropwise to the reaction system. The mixture was stirred at 0°C for 2 hours. Finally, 3-bromopropyne (7.04 g, 59.2 mmol) was added dropwise to the reaction system at 0°C, and then the mixture was warmed to room temperature and reacted overnight.

After TLC monitoring indicated the disappearance of the starting material, water (60 mL) was added to the reaction mixture, and the mixture was extracted twice with petroleum ether (60 mL). The combined organic phases were washed with saturated brine, dried over anhydrous sodium sulfate, and filtered. The filtrate was concentrated to dryness under reduced pressure at room temperature. The resulting residue was purified by silica gel column chromatography to obtain 5-(prop-2-yn-1-yloxy)pent-1-ene (720 mg).

¹H NMR (400 MHz, CDCl₃) δ 5.82 (m, 1H), 5.08 - 4.96 (m, 2H), 4.14 (d, *J* = 2.4 Hz, 2H), 3.53 (t, *J=* 6.5 Hz, 2H), 2.42 (t, *J* = 2.4 Hz, 1H), 2.14 (m, 2H), 1.71 (m, 2H).

**Step B:** 5-(Prop-2-yn-1-yloxy)pent-1-ene (720 mg, 5.81 mmol), cuprous chloride (64.8 mg, 0.66 mmol), bis(pinacolato)diboron (1.83 g, 7.21 mmol), tri-*tert*-butylphosphine tetrafluoroborate (228 mg, 0.79 mmol), and sodium *tert*-butoxide (188.6 mg, 1.97 mmol) were added to toluene (10 mL). The reaction system was purged three times with nitrogen, and methanol (419 mg, 13.1 mmol) was added dropwise to the reaction system. The reaction was carried out at room temperature for 2 hours.

After TLC monitoring indicated the disappearance of the starting material, the mixture was filtered through diatomite, rinsed with ethyl acetate, and concentrated to dryness under reduced pressure. The resulting residue was purified by silica gel column chromatography to obtain 2-(3-(but-3-en-1-oxy)prop-1-en-2-yl)-4,4,5,5-tetramethyl-1,3,2-dioxaborolane (640 mg).

**Step C:** 2-(3-(But-3-en-1-oxy)prop-1-en-2-yl)-4,4,5,5-tetramethyl-1,3,2-dioxaborolane (300 mg, 1.26 mmol) and Grubbs second-generation catalyst (53.5 mg, 0.063 mmol) were added to toluene (15 mL). The reaction system was purged five times with nitrogen and reacted at 90°C overnight.

After TLC monitoring indicated the disappearance of the starting material, the mixture was filtered through diatomite, rinsed with ethyl acetate, and concentrated to dryness under reduced pressure. The resulting residue was purified by silica gel column chromatography to obtain 4,4,5,5-tetramethyl-2-(2,5,6,7-tetrahydrooxepin-3-yl)-1,3,2-dioxaborolane (100 mg).

¹H NMR (400 MHz, CDCl₃) δ 6.86 - 6.56 (m, 1H), 4.32 - 4.21 (m, 2H), 3.85 (t,*J* = 5.7 Hz, 2H), 2.41 (m, 2H), 1.82 (m, 2H), 1.25 (s, 12H).

**Step D:** 2-(6-(4-(5-(Benzyloxy)-6-methylpyrimidine-4-carbonyl)piperazin-1-yl)-2-bromo-5-ethyl-7-oxo-[1,2,4]triazolo[1,5-a]pyrimidin-4(7H)-yl)-*N*-(2-chloro-4-(trifluoromethyl)phenyl)acetamide (319 mg, 0.41 mmol), 4,4,5,5-tetramethyl-2-(2,5,6,7-tetrahydrooxepin-3-yl)-1,3,2-dioxaborolane (100 mg, 0.45 mmol), anhydrous potassium carbonate (141.5 mg, 1.03 mmol), and [1,1'-bis(diphenylphosphino)ferrocene]dichloropalladium(II) dichloromethane complex (16.6 mg, 0.021 mmol) were added to 1,4-dioxane (8 mL) and water (1 mL). The reaction system was purged three times with nitrogen, and the reaction mixture was stirred at 100°C for 2 hours.

After TLC monitoring indicated the disappearance of the starting material, the mixture was cooled. Water (20 mL) was added, and the mixture was extracted twice with ethyl acetate (30 mL). The combined organic phases were washed with saturated brine (20 mL), dried over anhydrous sodium sulfate, filtered, and concentrated to dryness under reduced pressure. The resulting residue was purified by silica gel column chromatography to obtain 2-(6-(4-(5-(benzyloxy)-6-methylpyrimidine-4-carbonyl)piperazin-1-yl)-5-ethyl-7-oxo-2-(2,5,6,7-tetrahydrooxepin-3-yl)-[1,2,4]triazolo[1,5-a]pyrimidin-4(7H)-yl)-*N*-(2-chloro-4-(trifluoromethyl)phenyl)acetamide (180 mg).

MS (ESI) M/Z: 804.3 [M-H]⁻.

**Step E:** 2-(6-(4-(5-(Benzyloxy)-6-methylpyrimidine-4-carbonyl)piperazin-1-yl)-5-ethyl-7-oxo-2-(2,5,6,7-tetrahydrooxepin-3-yl)-[1,2,4]triazolo[1,5-a]pyrimidin-4(7H)-yl)-*N-*(2-chloro-4-(trifluoromethyl)phenyl)acetamide (180 mg, 0.22 mmol) was added to trifluoroacetic acid (4 mL), and the mixture was reacted at 50°C for 2 hours.

After LCMS monitoring indicated the disappearance of the starting material, the mixture was cooled. The mixture was concentrated to dryness under reduced pressure. The resulting residue was purified by preparative chromatography to obtain N-(2-chloro-4-(trifluoromethyl)phenyl)-2-(5-ethyl-6-(4-(5-hydroxy-6-methylpyrimidine-4-carbonyl)piperazin-1-yl)-7-oxo-2-(2,5,6,7-tetrahydrooxepin-3-yl)-[1,2,4]triazolo[1,5-a]pyrimidin-4(7H)-yl)acetamide (85.28 mg).

MS (ESI) M/Z: 716.1 [M+H] ⁺.

¹H NMR (400 MHz, DMSO-*d*₆) δ 10.37 (s, 1H), 10.24 (s, 1H), 8.58 (s, 1H), 8.06 (d, *J* = 8.6 Hz, 1H), 7.98 (s, 1H), 7.72 (d, *J* = 8.7 Hz, 1H), 7.05 (t, *J* = 5.8 Hz, 1H), 5.31 (s, 2H), 4.63 (s, 2H), 4.57 - 4.46 (m, 1H), 3.84 (t, *J* = 5.8 Hz, 2H), 3.55 - 3.42 (m, 3H), 3.27 - 3.17 (m, 1H), 3.05 - 2.91 (m, 3H), 2.85 - 2.76 (m, 1H), 2.68 - 2.59 (m, 1H), 2.48 - 2.45 (m, 2H), 2.44 (s, 3H), 1.87 - 1.74 (m, 2H), 1.18 (t, *J* = 7.4 Hz, 3H).

### Example 202

### N-(2-Chloro-4-(trifluoromethyl)phenyl)-2-(5-ethyl-6-(4-(5-hydroxy-6-methylpyrimidine-4-carbonyl)piperazin-1-yl)-7-oxo-2-(2,5,6,7-tetrahydrooxepin-4-yl-7,7-d₂)-[1,2,4]triazolo[1,5-a]pyrimidin-4(7H)-yl)acetamide

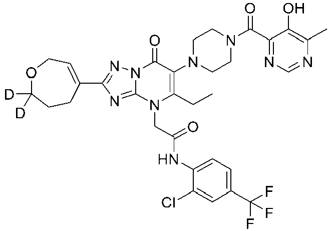

**The compound may also be synthesized according to the following steps:**

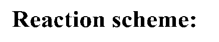

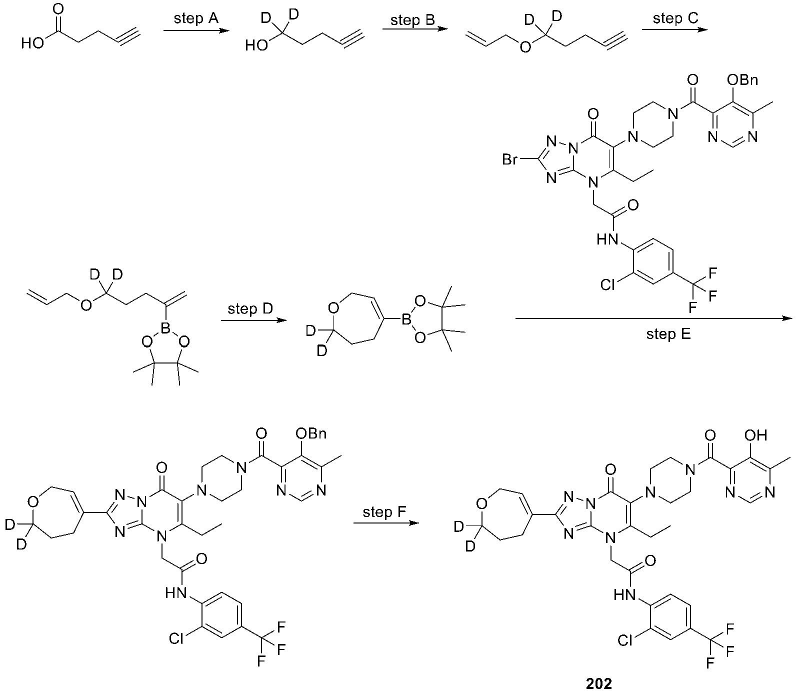

### Procedure:

**Step A:** Lithium aluminum deuteride (3.21 g, 76.45 mmol) was added to anhydrous tetrahydrofuran (50 mL), and the mixture was cooled to 0°C. 4-Pentynoic acid (5 g, 50.97 mmol) was dissolved in anhydrous tetrahydrofuran (10 mL), and the solution was added dropwise to the reaction system at 0°C. The mixture was then warmed to room temperature and reacted for 3 hours.

After TLC monitoring indicated the disappearance of the starting material, water (3.2 mL) was added, followed by an aqueous sodium hydroxide solution (15%, 3.2 mL), and finally water (9.6 mL) was added. The mixture was stirred for 0.5 hours. The mixture was filtered, rinsed with ethyl acetate, and the organic phase was washed with saturated brine (20 mL), dried over anhydrous sodium sulfate, and filtered. The filtrate was concentrated to dryness under reduced pressure to obtain crude 4-pentyn-1,1-d₂-1-ol (1.9 g).

**Step B:** Sodium hydride (1.06 g, 60%, 26.51 mmol) was added to anhydrous *N,N-*dimethylformamide (30 mL), and the mixture was cooled to 0°C. 4-Pentyn-1,1-d₂-1-ol (1.9 g, 22.09 mmol) was dissolved in anhydrous *N,N*-dimethylformamide (10 mL), and the solution was added dropwise to the reaction system. The mixture was stirred at 0°C for 2 hours. Finally, 3-bromopropene (8 g, 66.28 mmol) was added dropwise to the reaction system at 0°C, and then the mixture was warmed to room temperature and reacted overnight.

TLC monitoring indicated that the starting material was completely consumed. Water (60 mL) was added to the reaction mixture, and the mixture was extracted twice with petroleum ether (60 mL). The combined organic phases were washed with saturated brine, dried over anhydrous sodium sulfate, and filtered. The filtrate was concentrated to dryness under reduced pressure at room temperature. The resulting residue was purified by silica gel column chromatography to obtain 5-(allyloxy)pent-1-yne-5,5-d₂ (950 mg).

¹H NMR (400 MHz, CDCl₃) δ 5.99 - 5.86 (m, 1H), 5.31 - 5.25 (m, 1H), 5.22 - 5.15 (m, 1H), 3.98 (d, *J* = 5.6 Hz, 2H), 2.34 - 2.26 (m, 2H), 1.98 - 1.92 (m, 1H), 1.79 (t, *J*= 7.1 Hz, 2H).

**Step C:** 5-(Allyloxy)pent-1-yne-5,5-d₂ (950 mg, 7.54 mmol), cuprous chloride (74.6 mg, 0.75 mmol), bis(pinacolato)diboron (2.1 g, 8.3 mmol), tri-*tert-*butylphosphine tetrafluoroborate (262.4 mg, 0.9 mmol), and sodium *tert*-butoxide (217.2 mg, 2.26 mmol) were added to toluene (15 mL). The reaction system was purged three times with nitrogen, and methanol (482.6 mg, 15.1 mmol) was added dropwise to the reaction system. The reaction was carried out at room temperature for 2 hours.

TLC monitoring indicated that the starting material was completely consumed. The mixture was filtered through diatomite, rinsed with ethyl acetate, and concentrated to dryness under reduced pressure. The resulting residue was purified by silica gel column chromatography to obtain 2-(5-(allyloxy)pent-1-en-2-yl-5,5-d₂)-4,4,5,5-tetramethyl-1,3,2-dioxaborolane (1 g).

¹H NMR (400 MHz, CDCl₃) δ 6.72 - 6.47 (m, 1H), 6.00 - 5.83 (m, 1H), 5.55 - 5.35 (m, 1H), 5.30 - 5.24 (m, 1H), 5.19 - 5.13 (m, 1H), 3.95 (d, *J* = 5.4, 1.8 Hz, 2H), 2.27 - 2.17 (m, 2H), 1.74 - 1.66 (m, 2H), 1.27 (s, 12H).

**Step D:** 2-(5-(Allyloxy)pent-1-en-2-yl-5,5-d₂)-4,4,5,5-tetramethyl-1,3,2-dioxaborolane (500 mg, 1.97 mmol) and Grubbs second-generation catalyst (83.6 mg, 0.098 mmol) were added to toluene (25 mL). The reaction system was purged five times with nitrogen and reacted at 90°C overnight.

TLC monitoring indicated that the starting material was completely consumed. The mixture was filtered through diatomite, rinsed with ethyl acetate, and concentrated to dryness under reduced pressure to obtain crude 4,4,5,5-tetramethyl-2-(2,5,6,7-tetrahydrooxepin-4-yl-7,7-d₂)-1,3,2-dioxaborolane, which was used directly in the next step.

**Step E:** 2-(6-(4-(5-(Benzyloxy)-6-methylpyrimidine-4-carbonyl)piperazin-1-yl)-2-bromo-5-ethyl-7-oxo-[1,2,4]triazolo[1,5-a]pyrimidin-4(7H)-yl)-*N*-(2-chloro-4-(trifluoromethyl)phenyl)acetamide (300 mg, 0.38 mmol), crude 4,4,5,5-tetramethyl-2-(2,5,6,7-tetrahydrooxepin-4-yl-7,7-d₂)-1,3,2-dioxaborolane (128.8 mg, 0.57 mmol), anhydrous potassium carbonate (131.1 mg, 0.95 mmol), and [1,1'-bis(diphenylphosphino)ferrocene]dichloropalladium(II) dichloromethane complex (15.4 mg, 0.019 mmol) were added to 1,4-dioxane (8 mL) and water (1 mL). The reaction system was purged three times with nitrogen, and the reaction mixture was stirred at 100°C for 2 hours.

After TLC monitoring indicated the disappearance of the starting material, the mixture was cooled. Water (20 mL) was added, and the mixture was extracted twice with ethyl acetate (30 mL). The combined organic phases were washed with saturated brine (20 mL), dried over anhydrous sodium sulfate, filtered, and concentrated to dryness under reduced pressure. The resulting residue was purified by silica gel column chromatography to obtain 2-(6-(4-(5-(benzyloxy)-6-methylpyrimidine-4-carbonyl)piperazin-1-yl)-5-ethyl-7-oxo-2-(2,5,6,7-tetrahydrooxepin-4-yl-7,7-d₂)-[1,2,4]triazolo[1,5-a]pyrimidin-4(7H)-yl)-*N*-(2-chloro-4-(trifluoromethyl)phenyl)acetamide (75 mg).

MS (ESI) M/Z: 808.1 [M+H] ⁺.

**Step F:** 2-(6-(4-(5-(Benzyloxy)-6-methylpyrimidine-4-carbonyl)piperazin-1-yl)-5-ethyl-7-oxo-2-(2,5,6,7-tetrahydrooxepin-4-yl-7,7-d₂)-[1,2,4]triazolo[1,5-a]pyrimidin-4(7H)-yl)-N-(2-chloro-4-(trifluoromethyl)phenyl)acetamide (75 mg, 0.09 mmol) was added to trifluoroacetic acid (4 mL), and the reaction was carried out at 50°C for 2 hours.

After LCMS monitoring indicated the disappearance of the starting material, the mixture was cooled. The mixture was concentrated to dryness under reduced pressure. The resulting residue was purified by preparative chromatography to obtain *N*-(2-chloro-4-(trifluoromethyl)phenyl)-2-(5-ethyl-6-(4-(5-hydroxy-6-methylpyrimidine-4-carbonyl)piperazin-1-yl)-7-oxo-2-(2,5,6,7-tetrahydrooxepin-4-yl-7,7-d₂)-[1,2,4]triazolo[1,5-a]pyrimidin-4(7H)-yl)acetamide (11.34 mg).

MS (ESI) M/Z: 718.2 [M+H] ⁺.

¹H NMR (400 MHz, DMSO-*d*₆) δ 10.37 (s, 1H), 10.23 (s, 1H), 8.57 (s, 1H), 8.05 (d, *J* = 8.6 Hz, 1H), 7.97 (s, 1H), 7.72 (d, *J* = 8.7 Hz, 1H), 6.95 - 6.85 (m, 1H), 5.31 (s, 2H), 4.56 - 4.46 (m, 1H), 4.26 (d, *J* = 4.7 Hz, 2H), 3.56 - 3.44 (m, 3H), 3.26 - 3.20 (m, 1H), 3.05 - 2.91 (m, 3H), 2.87 - 2.76 (m, 3H), 2.68 - 2.59 (m, 1H), 2.44 (s, 3H), 1.90 - 1.77 (m, 2H), 1.18 (t, *J* = 7.4 Hz, 3H).

### Example 219

### N-(2-Chloro-4-(trifluoromethyl)phenyl)-2-(2-(3,6-dihydro-2H-pyran-4-yl)-5-ethyl-6-(4-(6-hydroxypyrazolo[1,5-a]pyridine-7-carbonyl)piperazin-1-yl)-7-oxo-[1,2,4]triazolo[1.5-a]pyrimidin-4(7H)-yl)acetamide

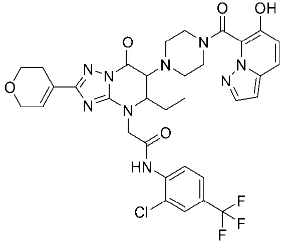

**The compound may also be synthesized according to the following steps:**

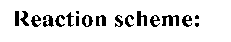

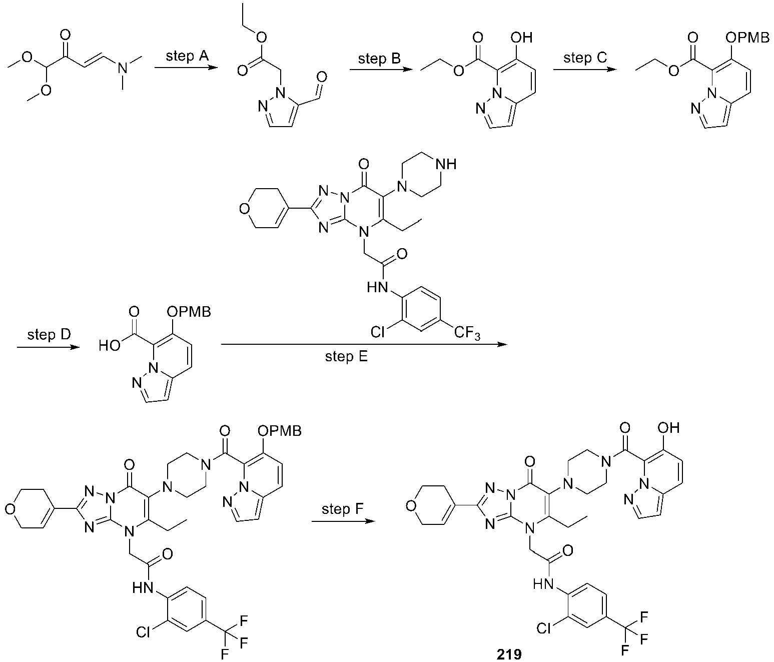

### Procedure:

**Step A:** (E)-4-(Dimethylamino)-1,1-dimethoxybut-3-en-2-one (2.0 g, 11.55 mmol) and ethyl aminoglycinate hydrochloride (1.96 g, 12.70 mmol) were dissolved in ethanol (40 mL). Triethylamine (1.52 g, 15.01 mmol) was added, and the reaction was carried out at 80°C overnight.

After TLC monitoring indicated the disappearance of the starting material, dilute hydrochloric acid (1 M, 60 mL) was added to the reaction mixture, and the mixture was stirred at room temperature for 1 hour. Ethyl acetate (40 mL × 2) was then added for extraction. The combined organic phases were washed with saturated brine and dried over anhydrous sodium sulfate, and concentrated to dryness under reduced pressure. The resulting residue was purified by silica gel column chromatography to obtain ethyl 2-(5-formyl-1H-pyrazol-1-yl)acetate (1.5 g).

¹H NMR (400 MHz, CDCl₃) δ 9.85 (s, 1H), 7.64 (d, *J* = 2.1 Hz, 1H), 6.99 (d, *J* = 2.1 Hz, 1H), 5.30 (s, 2H), 4.23 (q, *J* = 7.1 Hz, 2H), 1.28 (t, *J* = 7.2 Hz, 3H).

**Step B:** Ethyl 2-(bis(2,2,2-trifluoroethoxy)phosphoryl)acetate (3.29 g, 9.89 mmol) was dissolved in tetrahydrofuran (20 mL). Sodium hydride (395.6 mg, 60%, 9.89 mmol) was added at 0°C, followed by the addition of ethyl 2-(5-formyl-1H-pyrazol-1-yl)acetate (1.5 g, 8.24 mmol). The reaction was carried out at 0°C for 30 minutes. Sodium hydride (60%, 395.6 mg, 9.89 mmol) was added at 0°C, and the mixture was stirred at this temperature for 30 minutes.

After TLC monitoring indicated the disappearance of the starting material, an aqueous ammonium chloride solution was added to quench the reaction. Ethyl acetate (20 mL × 2) was then added for extraction, and the phases were separated. The combined organic phases were washed with saturated brine, dried over anhydrous sodium sulfate, and concentrated to dryness under reduced pressure. The resulting residue was purified by silica gel column chromatography to obtain ethyl 6-hydroxypyrazolo[1,5-a]pyridine-7-carboxylate (1.15 g).

MS (ESI) M/Z: 207.0 [M+H] ⁺.

¹H NMR (400 MHz, CDCl₃) δ 11.62 (s, 1H), 7.98 (d, *J* = 2.4 Hz, 1H), 7.66 (d, *J* = 9.4 Hz, 1H), 6.97 (d, *J* = 9.5 Hz, 1H), 6.61 (d, *J* = 2.4 Hz, 1H), 4.67 (q, *J* = 7.1 Hz, 2H), 1.54 (t, *J* = 7.0 Hz, 3H).

**Step C:** Ethyl 6-hydroxypyrazolo[1,5-a]pyridine-7-carboxylate (200 mg, 0.97 mmol) was dissolved in DMF (6 mL), then potassium carbonate (200.97 mg, 1.46 mmol) and *p-*methoxybenzyl chloride (167.25 mg, 1.07 mmol) were added, and the mixture was heated at 80°C and reacted for 2 hours.

After TLC monitoring indicated the disappearance of the starting material, the mixture was cooled. Water was added, and the mixture was extracted with ethyl acetate. The organic phase was washed with saturated brine, dried over anhydrous sodium sulfate, and concentrated to dryness under reduced pressure to obtain crude ethyl 6-((4-methoxybenzyl)oxy)pyrazolo[1,5-a]pyridine-7-carboxylate (0.97 mmol).

MS (ESI) M/Z: 327.1 [M+H] ⁺.

**Step D:** Crude ethyl 6-((4-methoxybenzyl)oxy)pyrazolo[1,5-a]pyridine-7-carboxylate (0.97 mmol) was dissolved in methanol (5 mL) and water (2 mL). Lithium hydroxide monohydrate (61.17 mg, 1.46 mmol) was added. The reaction was carried out at room temperature overnight.

After TLC monitoring indicated that the starting material was not completely consumed, additional lithium hydroxide monohydrate (40.74 mg, 0.97 mmol) was added. The reaction was carried out at 40°C for 1 hour.

After TLC monitoring indicated the disappearance of the starting material, water (5 mL) was added to the reaction mixture, and methanol was concentrated to dryness under reduced pressure. The resulting residue was extracted with methyl *tert*-butyl ether, and the phases were separated. The aqueous phase was adjusted to an acidic pH with dilute hydrochloric acid, resulting in the precipitation of a solid. The solid was filtered, and the filter cake was dried to obtain 6-((4-methoxybenzyl)oxy)pyrazolo[1,5-a]pyridine-7-carboxylic acid (106 mg).

MS (ESI) M/Z: 297.1 [M-H]⁻.

**Step E:** 6-((4-Methoxybenzyl)oxy)pyrazolo[1,5-a]pyridine-7-carboxylic acid (87.78 mg, 0.30 mmol) and *N*-(2-chloro-4-(trifluoromethyl)phenyl)-2-(2-(3,6-dihydro-2H-pyran-4-yl)-5-ethyl-7-oxo-6-(piperazin-1-yl)-[1,2,4]triazolo[1,5-a]pyrimidin-4(7H)-yl)acetamide trifluoroacetate (200 mg, 0.30 mmol) were dissolved in DMF (6 mL), and *N,N-*diisopropylethylamine (114 mg, 0.88 mmol) was added. HATU (134.4 mg, 0.35 mmol) was added portionwise at 0°C, and the reaction was carried out at room temperature overnight.

After TLC monitoring indicated the disappearance of the starting material, the mixture was cooled. Water (20 mL) was added, and the mixture was extracted twice with ethyl acetate (30 mL). The combined organic phases were washed with saturated brine (20 mL), dried over anhydrous sodium sulfate, filtered, and concentrated to dryness under reduced pressure. The resulting residue was purified by silica gel column chromatography to obtain *N*-(2-chloro-4-(trifluoromethyl)phenyl)-2-(2-(3,6-dihydro-2H-pyran-4-yl)-5-ethyl-6-(4-(6-((4-methoxybenzyl)oxy)pyrazolo[1,5-a]pyridine-7-carbonyl)piperazin-1-yl)-7-oxo-[1,2,4]triazolo[1.5-a]pyrimidin-4(7H)-yl)acetamide (80 mg).

MS (ESI) M/Z: 846.1 [M+H] ⁺.

**Step F:** *N*-(2-Chloro-4-(trifluoromethyl)phenyl)-2-(2-(3,6-dihydro-2H-pyran-4-yl)-5-ethyl-6-(4-(6-((4-methoxybenzyl)oxy)pyrazolo[1,5-a]pyridine-7-carbonyl)piperazin-1-yl)-7-oxo-[1,2,4]triazolo[1.5-a]pyrimidin-4(7H)-yl)acetamide (80 mg, 0.095 mmol) was dissolved in dichloromethane (4 mL), and trifluoroacetic acid (2 mL) was added. The reaction was carried out at room temperature for 2 hours.

After TLC monitoring indicated the disappearance of the starting material, the mixture was concentrated to dryness under reduced pressure to remove the solvent. The resulting residue was purified by preparative chromatography to obtain *N*-(2-chloro-4-(trifluoromethyl)phenyl)-2-(2-(3,6-dihydro-2H-pyran-4-yl)-5-ethyl-6-(4-(6-hydroxypyrazolo[1,5-a]pyridine-7-carbonyl)piperazin-1-yl)-7-oxo-[1,2,4]triazolo[1.5-a]pyrimidin-4(7H)-yl)acetamide (15.72 mg).

MS (ESI) M/Z: 724.2 [M-H]⁻.

¹H NMR (400 MHz, DMSO-*d*₆) δ 10.40 (s, 1H), 9.94 (s, 1H), 8.05 (d, *J* = 8.6 Hz, 1H), 7.96 (s, 1H), 7.91 - 7.81 (m, 1H), 7.71 (d, *J* = 8.8 Hz, 1H), 7.63 (d, *J* = 9.5 Hz, 1H), 7.16 - 7.00 (m, 1H), 6.83 (s, 1H), 6.66 - 6.51 (m, 1H), 5.32 (s, 2H), 4.65 - 4.52 (m, 1H), 4.33 - 4.14 (m, 2H), 3.81 (t, *J* = 5.6 Hz, 2H), 3.64 - 3.38 (m, 3H), 3.25 - 3.19 (m, 1H), 3.09 - 2.91 (m, 3H), 2.88 - 2.78 (m, 1H), 2.68 - 2.56 (m, 1H), 2.50 (m, 2H), 1.21 - 1.13 (m, 3H).

### Example 45

### 2-[2-[3,6-Dihydro-2H-pyran-4-yl]-5-ethyl-6-[4-(5-hydroxy-6-methylpyrimidine-4-carbonyl)piperazin-1-yl]-7-oxo-[1,2,4]triazolo[1,5-a]pyrimidin-4(7H)-yl]-N-[4-(trifluoromethyl)thiophen-2-yl] acetamide

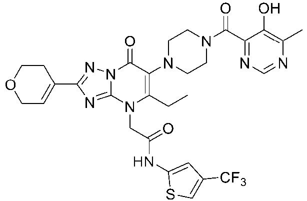

**The compound may also be synthesized according to the following steps:**

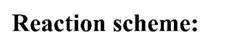

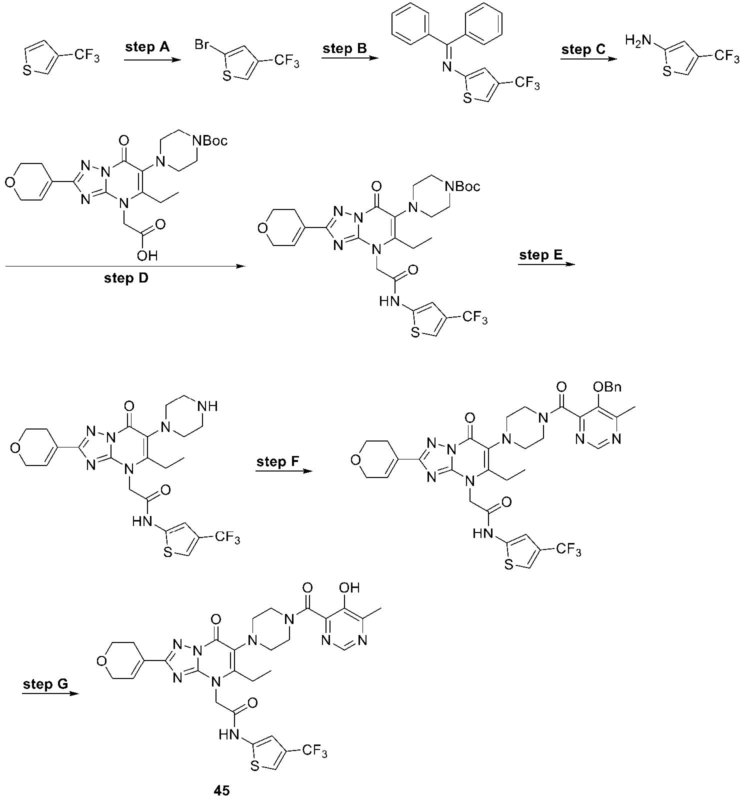

### Procedure:

**Step A:** 3-(Trifluoromethyl)thiophene (2.0 g, 13.2 mmol) was dissolved in glacial acetic acid (50 mL), and NBS (7.0 g, 39.4 mmol) was added at 0°C. The resulting mixture was stirred at 100°C for 8 hours.

After TLC monitoring indicated the disappearance of the starting material, an aqueous solution (300 mL) was added to the reaction mixture. The reaction mixture was extracted with n-pentane (300 mL × 3). The combined organic phases were dried over anhydrous sodium sulfate, filtered, and concentrated under reduced pressure. 2-Bromo-4-(trifluoromethyl)thiophene (2.0 g) was obtained.

¹H NMR (400 MHz, DMSO-*d*₆) δ 8.29 (s, 1H), 7.62 (d, *J* = 1.7 Hz, 1H).

**Step B:** 2-Bromo-4-(trifluoromethyl)thiophene (1.9 g, 8.2 mmol), diphenylmethanimine (1.8 g, 9.9 mmol), tris(dibenzylideneacetone)dipalladium (1.5 g, 1.6 mmol), 4,5-bis(diphenylphosphino)-9,9-dimethylxanthene (1.9 g, 3.3 mmol), and sodium *tert-*butoxide (2 g, 20.6 mmol) were sequentially added to 1,4-dioxane (20 mL). The reaction system was heated to 100°C under a nitrogen atmosphere and stirred for 2 hours.

After LCMS monitoring indicated the disappearance of the starting material, water was added to the reaction mixture, and the mixture was extracted with ethyl acetate (200 mL × 3). The combined organic phases were washed with saturated brine (50 mL × 3), dried over anhydrous sodium sulfate, filtered, and concentrated under reduced pressure. The resulting mixture was purified by silica gel column chromatography to obtain 1,1-diphenyl-*N*-[4-(trifluoromethyl)-2-thienyl]methanamine (1.5 g).

¹H NMR (400 MHz, DMSO-*d₆*) δ 7.89 (s, 1H), 7.73 - 7.66 (m, 2H), 7.62 (dd, *J* = 4.9, 1.8 Hz, 3H), 7.56 - 7.50 (m, 1H), 7.47 (t, *J* = 7.4 Hz, 2H), 7.34 (dd, *J* = 6.4, 3.1 Hz, 2H), 7.28 (d, *J* = 1.6 Hz, 1H).

**Step C:** 1,1-Diphenyl-*N*-[4-(trifluoromethyl)-2-thienyl]methanamine (1.2 g, 3.6 mmol) was dissolved in tetrahydrofuran (20 mL), and then concentrated hydrochloric acid (4 mL) was added thereto at 0°C. The reaction system was warmed to 25°C under a nitrogen atmosphere and stirred for 1 hour.

After LCMS monitoring indicated the disappearance of the starting material, the reaction mixture was basified to pH=8 with a 2M aqueous sodium hydroxide solution. The mixture was then extracted with dichloromethane (150 mL × 3). The combined organic phases were dried over anhydrous sodium sulfate, filtered, and concentrated under reduced pressure. The resulting mixture was purified by silica gel column chromatography to obtain 4-(trifluoromethyl)thiophen-2-amine (550 mg).

¹H NMR (400 MHz, DMSO-*d₆*) δ 6.97 (s, 1H), 6.00 (s, 1H), 5.93 (s, 2H).

**Step D:** 4-(Trifluoromethyl)thiophen-2-amine (100 mg, 0.6 mmol) and 2-[6-(4-*tert-*butoxycarbonylpiperazin-1-yl)-2-(3,6-dihydro-2*H*-pyran-4-yl)-5-ethyl-7-oxo-[1,2,4]triazolo[1,5-a]pyrimidin-4-yl]acetic acid (350 mg, 0.7 mmol) were dissolved in acetonitrile (10 mL). Then, *N*-methylimidazole (74 mg, 0.9 mmol) and *N,N,N',N-*tetramethylchloroformamidinium hexafluorophosphate (629 mg, 2.2 mmol) were added, and the reaction mixture was stirred at room temperature for 1 hour.

After LCMS monitoring indicated the disappearance of the starting material, water was added to the reaction mixture, and the mixture was extracted with ethyl acetate (80 mL × 3). The combined organic phases were washed with saturated brine (20 mL × 3), dried over anhydrous sodium sulfate, filtered, and concentrated under reduced pressure. The resulting mixture was purified by silica gel column chromatography to obtain 4-[2-(3,6-dihydro-2*H-*pyran-4-yl)-5-ethyl-7-oxo-4-[2-oxo-2-[[4-(trifluoromethyl)-2-thienyl]amino]ethyl]-[1,2,4]triazolo[1,5-*a*]pyrimidine (220 mg).

MS (ESI) M/Z: 582.2 [M-56+H] ⁺.

**Step E:** *tert-*Butyl 4-[2-(3,6-dihydro-2*H*-pyran-4-yl)-5-ethyl-7-oxo-4-[2-oxo-2-[[4-(trifluoromethyl)-2-thienyl]amino]ethyl]-[1,2,4]triazolo[1,5-*a*]pyrimidin-6-yl]piperazine-1-carboxylate (220 mg, 0.35 mmol) was dissolved in dichloromethane (10 mL), and then trifluoroacetic acid (3 mL) was added. The reaction mixture was stirred at 25°C for 1 hour.

After LCMS monitoring indicated the disappearance of the starting material, the reaction mixture was diluted with dichloromethane and then concentrated under reduced pressure to obtain 2-[2-(3,6-dihydro-2*H*-pyran-4-yl)-5-ethyl-7-oxo-6-piperazin-1-yl-[1,2,4]triazolo[1,5-a]pyrimidin-4-yl]-*N*-[4-(trifluoromethyl)-2-thienyl]acetamide (220 mg).

MS (ESI) M/Z: 538.2 [M+H]⁺.

**Step F:** 2-[2-(3,6-Dihydro-2H-pyran-4-yl)-5-ethyl-7-oxo-6-piperazin-1-yl-[1,2,4]triazolo[1,5-*a*]pyrimidin-4-yl]-*N*-[4-(trifluoromethyl)-2-thienyl]acetamide (110 mg, crude product) and 5-benzyloxy-6-methylpyrimidine-4-carboxylic acid (42 mg, 0.17 mmol) were dissolved in acetonitrile (8 mL). *N,N,N',N'*- Tetramethylchloroformamidinium hexafluorophosphate (50 mg, 0.17 mmol) and N-methylimidazole (75 mg, 0.9 mmol) were added at 0°C. The reaction mixture was stirred at 25°C for 1 hour.

After LCMS monitoring indicated the disappearance of the starting material, water was added to the reaction mixture, and the mixture was extracted with ethyl acetate (80 mL × 3). The combined organic phases were washed with saturated brine (20 mL × 3), dried over anhydrous sodium sulfate, filtered, and concentrated under reduced pressure. The resulting mixture was purified by silica gel column chromatography to obtain 2-[6-[4-(5-benzyloxy-6-methylpyrimidine-4-carbonyl)piperazin-1-yl]-2-(3,6-dihydro-2*H*-pyran-4-yl)-5-ethyl-7-oxo-[1,2,4]triazolo[1,5-a]pyrimidin-4-yl]-*N*-[4-(trifluoromethyl)-2-thienyl]acetamide (100 mg).

MS (ESI) M/Z: 764.3 [M+H] ⁺.

**Step G:** Compound 2-[6-[4-(5-benzyloxy-6-methylpyrimidine-4-carbonyl)piperazin-1-yl]-2-(3,6-dihydro-2*H*-pyran-4-yl)-5-ethyl-7-oxo-[1,2,4]triazolo[1,5-*a*]pyrimidin-4-yl]-*N-*[4-(trifluoromethyl)-2-thienyl]acetamide (90 mg, 0.12 mmol) was dissolved in trifluoroacetic acid (4 mL), and the reaction mixture was stirred at 60°C for 2 hours.

After LCMS monitoring indicated the disappearance of the starting material, the reaction mixture was diluted with dichloromethane and then concentrated under reduced pressure. The resulting mixture was purified by preparative high-performance liquid chromatography. The purification conditions were as follows: chromatographic column: xbridge-C18 21.2 mm × 150 mm; mobile phase: water (containing 0.1% formic acid) and acetonitrile; flow rate: 25 mL/min; gradient: acetonitrile from 40% to 70% within 20 minutes; detection wavelength: 254 nm. The product was collected and lyophilized under reduced pressure to obtain the product 2-[2-(3,6-dihydro-2*H*-pyran-4-yl)-5-ethyl-6-[4-(5-hydroxy-6-methylpyrimidine-4-carbonyl)piperazin-1-yl]-7-oxo-[1,2,4]triazolo[1,5-a]pyrimidin-4-yl]-N-[4-(trifluoromethyl)-2-thienyl]acetamide (24.5 mg).

MS (ESI) M/Z: 674.4 [M+H] ⁺.

¹H NMR (400 MHz, DMSO-*d*₆) δ 11.98 (s, 1H), 10.23 (s, 1H), 8.58 (s, 1H), 7.75 - 7.66 (m, 1H), 6.91 (d, *J=* 1.8 Hz, 1H), 6.83 - 6.76 (m, 1H), 5.22 (s, 2H), 4.58 - 4.47 (m, 1H), 4.30 - 4.18 (m, 2H), 3.80 (t, *J* = 5.4 Hz, 2H), 3.58 - 3.43 (m, 3H), 3.29 - 3.21 (m, 1H), 3.06 - 2.94 (m, 3H), 2.88 - 2.78 (m, 1H), 2.70 - 2.63 (m, 1H), 2.50 (m, 2H), 2.45 (s, 3H), 1.17 (t, *J* = 7.4 Hz, 3H).

### Example 128

### N-(5-Chloro-4-(trifluoromethyl)thiophen-2-yl)-2-(2-(3,6-dihydro-2H-pyran-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide

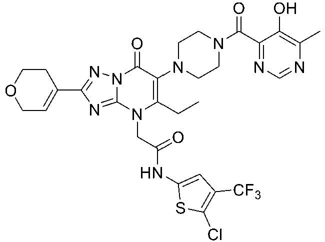

N-[5-Chloro-4-(trifluoromethyl)-2-thienyl]-2-[2-(3,6-dihydro-2H-pyran-4-yl)-5-ethyl-6-[4-(5-hydroxy-6-methylpyrimidine-4-carbonyl)piperazin-1-yl]-7-oxo-[1,2,4]triazolo[1,5-a]pyrimidin-4-yl]acetamide (2.2 mg) may also be prepared with reference to the preparation method of **Example 129.**

MS (ESI) M/Z: 708.4 [M+H] ⁺.

¹H NMR (400 MHz, DMSO-*d*₆) δ 8.47 (s, 1H), 6.83 - 6.74 (m, 1H), 6.51 (s, 1H), 5.08 (s, 2H), 4.59 - 4.49 (m, 1H), 4.25 - 4.22 (m, 2H), 3.79 (t, *J* = 5.4 Hz, 2H), 3.52 - 3.47 (m, 3H), 3.18 - 3.15 (m, 1H), 3.02 - 2.91 (m, 3H), 2.85 - 2.81 (m, 1H), 2.69 - 2.64 (m, 1H), 2.50 (m, 2H), 2.42 (s, 3H), 1.15 (t, *J* = 7.4 Hz, 3H).

### Example 190

### 2-(2-(3,6-Dihydro-2H-pyran-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(3-methyl-4-(trifluoromethyl)thiophen-2-yl)acetamide

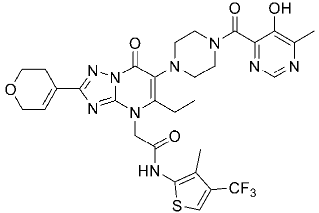

**The compound may also be synthesized according to the following steps:**

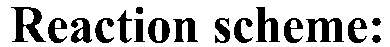

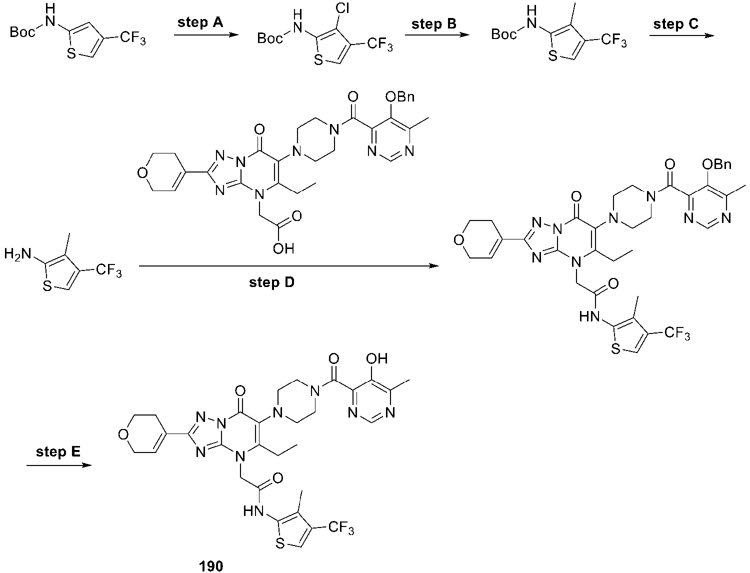

### Procedure:

**Step A:** *tert-*Butyl *N*-[4-(trifluoromethyl)-2-thienyl]carbamate (1.5 g, 5.6 mmol) was dissolved in acetonitrile (20 mL), and NCS (750 mg, 5.6 mmol) was added slowly with stirring at 0°C. The resulting mixture was stirred at 0°C for 8 hours.

After TLC monitoring indicated the disappearance of the starting material, water was added to the resulting mixture, and then the mixture was extracted with ethyl acetate (200 mL × 3). The combined organic phases were washed with saturated brine (30 mL × 2), dried over anhydrous sodium sulfate, and filtered. The filtrate was concentrated under reduced pressure. The resulting mixture was then purified by silica gel column chromatography to obtain *tert-*butyl *N*-[3-chloro-4-(trifluoromethyl)-2-thienyl]carbamate (1.0 g).

**Step B:** *tert*-Butyl *N*-[3-chloro-4-(trifluoromethyl)-2-thienyl]carbamate (200 mg, 0.7 mmol), methylboronic acid (400 mg, 6.6 mmol), X-phos Pd G2 (104 mg, 132.6 µmol), and cesium carbonate (540 mg, 1.7 mmol) were sequentially added to 1,4-dioxane (10 mL) and water (1 mL). The reaction system was heated to 100°C under a nitrogen atmosphere and stirred for 8 hours.

After LCMS monitoring indicated the disappearance of the starting material, water was added to the resulting mixture, and then the mixture was extracted with ethyl acetate (100 mL × 3). The combined organic phases were washed with saturated brine (20 mL × 2), dried over anhydrous sodium sulfate, and filtered. The filtrate was concentrated under reduced pressure. The resulting mixture was purified by silica gel column chromatography to obtain *tert*-butyl *N*-[3-methyl-4-(trifluoromethyl)-2-thienyl]carbamate (85 mg).

MS (ESI) M/Z: 280.1 [M-H]⁻.

**Step C:** At 0°C, to a stirred solution of *tert*-butyl *N*-[3-methyl-4-(trifluoromethyl)-2-thienyl]carbamate (80 mg, 0.3 mmol) was added HCl (2 mL, 4.0 M in 1,4-dioxane), and the reaction system was stirred at 25°C for 1 hour.

After LCMS monitoring indicated the disappearance of the starting material, the resulting mixture was concentrated under reduced pressure to obtain 3-methyl-4-(trifluoromethyl)thiophen-2-amine (80 mg). The crude product was used directly in the next step without further purification.

**Step D:** 3-Methyl-4-(trifluoromethyl)thiophen-2-amine (80 mg, crude product) and 2-[6-[4-(5-benzyloxy-6-methylpyrimidine-4-carbonyl)-2-(3,6-dihydro-2*H*-pyran-4-yl)-5-ethyl-7-oxo-[1,2,4]triazolo[1,5-a]pyrimidin-4-yl]acetic acid (271 mg, 0.4 mmol) were dissolved in acetonitrile (5 mL). Then, TCFH (372 mg, 1.3 mmol) and *N*-methylimidazole (181 mg, 2.2 mmol) were added. The reaction mixture was stirred at 25°C for 1 hour.

After LCMS monitoring indicated the disappearance of the starting material, water was added to the reaction mixture, and the mixture was extracted with dichloromethane (80 mL × 3). The combined organic phases were washed with saturated brine (10 mL × 3), dried over anhydrous sodium sulfate, filtered, and concentrated under reduced pressure. The resulting mixture was purified by silica gel column chromatography to obtain 2-[6-[4-(5-benzyloxy-6-methylpyrimidine-4-carbonyl)piperazin-1-yl]-2-(3,6-dihydro-2*H*-pyran-4-yl)-5-ethyl-7-oxo-[1,2,4]triazolo[1,5-*a*]pyrimidin-4-yl]-*N*-[3-methyl-4-(trifluoromethyl)-2-thienyl]acetamide (70 mg).

MS (ESI) M/Z: 778.4 [M+H]⁺.

**Step E:** Compound 2-[6-[4-(5-benzyloxy-6-methylpyrimidine-4-carbonyl)piperazin-1-yl]-2-(3,6-dihydro-2H-pyran-4-yl)-5-ethyl-7-oxo-[1,2,4]triazolo[1,5-*a*]pyrimidin-4-yl]-N-[3-methyl-4-(trifluoromethyl)-2-thienyl]acetamide (70 mg, 0.1 mmol) was dissolved in trifluoroacetic acid (4 mL), and the reaction mixture was stirred at 60°C for 1 hour.

After LCMS monitoring indicated the disappearance of the starting material, the reaction mixture was diluted with dichloromethane and then concentrated under reduced pressure. The resulting mixture was purified by preparative high-performance liquid chromatography. The purification conditions were as follows: chromatographic column: xbridge-C18 21.2 mm × 150 mm; mobile phase: water (containing 0.1% formic acid) and acetonitrile; flow rate: 25 mL/min; gradient: acetonitrile from 40% to 70% within 20 minutes; detection wavelength: 254 nm. The product was collected and lyophilized under reduced pressure to obtain the final product 2-[2-(3,6-dihydro-2*H*-pyran-4-yl)-5-ethyl-6-[4-(5-hydroxy-6-methylpyrimidine-4-carbonyl)piperazin-1-yl]-7-oxo-[1,2,4]triazolo[1,5-*a*]pyrimidin-4-yl]-*N*-[3-methyl-4-(trifluoromethyl)-2-thienyl]acetamide (48.1 mg).

MS (ESI) M/Z: 688.4 [M+H] ⁺.

¹H NMR (400 MHz, DMSO-*d*₆) δ 10.42 (s, 2H), 8.57 (s, 1H), 7.73 (s, 1H), 6.86 - 6.75 (m, 1H), 5.28 (s, 2H), 4.58 - 4.46 (m, 1H), 4.30 - 4.18 (m, 2H), 3.80 (t, *J* = 5.4 Hz, 2H), 3.57 - 3.45 (m, 3H), 3.28 - 3.20 (m, 1H), 3.03 - 2.91 (m, 3H), 2.86 - 2.78 (m, 1H), 2.70 - 2.61 (m, 1H), 2.50 (m, 2H), 2.44 (s, 3H), 2.26 (s, 3H), 1.16 (t, *J=* 7.4 Hz, 3H).

### Example 191

### N-(3-Cyclopropyl-4-(trifluoromethyl)thiophen-2-yl)-2-(2-(3,6-dihydro-2H-pyran-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide

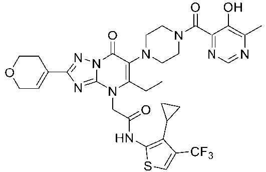

*N*-[3-Cyclopropyl-4-(trifluoromethyl)-2-thienyl]-2-[2-(3,6-dihydro-2H-pyran-4-yl)-5-ethyl-6-[4-(5-hydroxy-6-methylpyrimidine-4-carbonyl)piperazin-1-yl]-7-oxo-[1,2,4]triazolo[1,5-a]pyrimidin-4-yl]acetamide (52.72 mg) may also be prepared with reference to the preparation method of **Example 190.**

MS (ESI) M/Z: 714.4 [M+H] ⁺.

¹H NMR (400 MHz, DMSO-*d*₆) δ 10.30 (s, 2H), 8.56 (s, 1H), 7.71 (s, 1H), 6.92 - 6.74 (m, 1H), 5.30 (s, 2H), 4.60 - 4.41 (m, 1H), 4.32 - 4.14 (m, 2H), 3.80 (t, *J* = 5.4 Hz, 2H), 3.61 - 3.43 (m, 3H), 3.28 - 3.20 (m, 1H), 3.06 - 2.92 (m, 3H), 2.87 - 2.77 (m, 1H), 2.71 - 2.61 (m, 1H), 2.5 (m, 2H), 2.44 (s, 3H), 1.72 - 1.57 (m, 1H), 1.17 (t, *J* = 7.4 Hz, 3H), 1.03 - 0.88 (m, 2H), 0.71 - 0.57 (m, 2H).

### Example 297

### 2-(2-(3,6-Dihydro-2H-pyran-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(5-methyl-4-(trifluoromethyl)thiophen-2-yl)acetamide

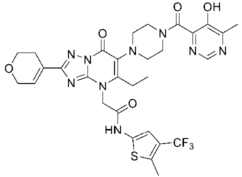

2-[2-(3,6-Dihydro-2*H*-pyran-4-yl)-5-ethyl-6-[4-(5-hydroxy-6-methylpyrimidine-4-carbonyl)piperazin-1-yl]-7-oxo-[1,2,4]triazolo[1,5-a]pyrimidin-4-yl]-*N*-[5-methyl-4-(trifluoromethyl)-2-thienyl]acetamide (12.3 mg) may also be prepared with reference to the preparation method of **Example 190.**

MS (ESI) M/Z: 688.4 [M+H] ⁺.

¹H NMR (400 MHz, DMSO-*d*₆) δ 11.52 (s, 1H), 8.48 (s, 1H), 6.86 - 6.76 (m, 1H), 6.72 (s, 1H), 5.19 (s, 2H), 4.60 - 4.45 (m, 1H), 4.29 - 4.15 (m, 2H), 3.79 (t, *J* = 5.4 Hz, 2H), 3.59 - 3.45 (m, 3H), 3.26 - 3.20 (m, 1H), 3.03 - 2.89 (m, 3H), 2.86 - 2.76 (m, 1H), 2.69 - 2.61 (m, 1H), 2.5 (m, 2H), 2.42 (s, 6H), 1.15 (t, *J* = 7.4 Hz, 3H).

### Example 298

### N-(5-Cyclopropyl-4-(trifluoromethyl)thiophen-2-yl)-2-(2-(3,6-dihydro-2H-pyran-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide

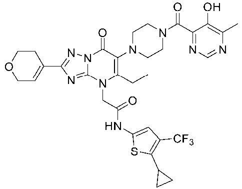

The product 2-[2-(3,6-dihydro-2*H*-pyran-4-yl)-5-ethyl-6-[4-(5-hydroxy-6-methylpyrimidine-4-carbonyl)piperazin-1-yl]-7-oxo-[1,2,4]triazolo[1,5-*a*]pyrimidin-4-yl]-*N-*[5-cyclopropyl-4-(trifluoromethyl)-2-thienyl]acetamide (16.9 mg) may also be prepared with reference to the preparation method of **Example 190.**

MS (ESI) M/Z: 714.4 [M+H] ⁺.

¹H NMR (400 MHz, DMSO-*d*₆) δ 11.74 (s, 1H), 10.36 (s, 1H), 8.54 (s, 1H), 6.78 (s, 1H), 6.70 (s, 1H), 5.17 (s, 2H), 4.58 - 4.45 (m, 1H), 4.29 - 4.18 (m, 2H), 3.79 (t, *J* = 5.3 Hz, 2H), 3.61 - 3.45 (m, 3H), 3.27 - 3.17 (m, 1H), 3.06 - 2.88 (m, 3H), 2.89 - 2.77 (m, 1H), 2.71 - 2.58 (m, 1H), 2.5 (m, 2H), 2.44 (s, 3H), 2.21 - 2.09 (m, 1H), 1.15 (t, *J* = 7.4 Hz, 3H), 1.08 - 0.98 (m, 2H), 0.72 - 0.61 (m, 2H).

### Example 258

### 2-(5-Ethyl-6-(4-(5-hydroxy-6-methylpyrimidine-4-carbonyl)piperazin-1-yl)-7-oxo-2-(2,5,6,7-tetrahydrooxepin-4-yl)-[1,2,4]triazolo[1,5-a]pyrimidin-4(7H)-yl)-N-(7-(trifluoromethyl)benzo[b]thiophen-4-yl)acetamide

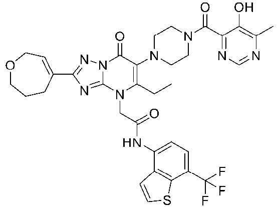

**The compound may also be synthesized according to the following steps:**

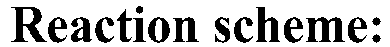

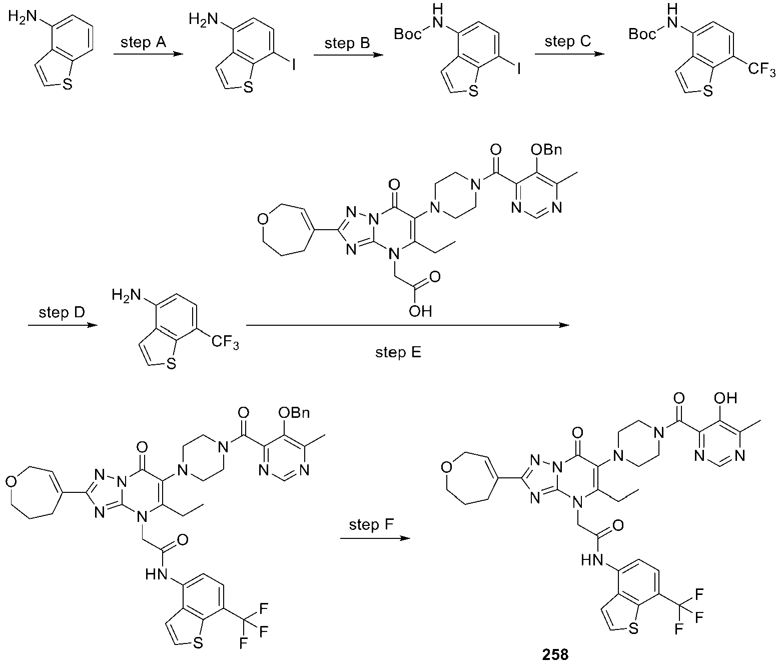

### Procedure:

**Step A:** Benzothiophen-4-amine (700 mg, 4.69 mmol) was dissolved in acetonitrile (10 mL), and then *N*-iodosuccinimide (1.06 g, 4.69 mmol) was added to the reaction system, and the reaction system was stirred at 0°C for 1 hour.

After LCMS monitoring indicated the disappearance of the starting material, the reaction mixture was quenched with water, then extracted with ethyl acetate (100 mL × 3), and washed with saturated brine (20 mL × 3). The combined organic phases were dried over anhydrous sodium sulfate, filtered, and concentrated under reduced pressure. The resulting mixture was purified by silica gel column chromatography to obtain 7-iodobenzothiophen-4-amine (650 mg).

MS (ESI) M/Z: 274.1 [M-H]⁻.

¹H NMR (400 MHz, DMSO-*d*₆) δ 7.86 (d, *J* = 5.6 Hz, 1H), 7.56 (d, *J* = 5.6 Hz, 1H), 7.33 (d, *J* = 8.1 Hz, 1H), 6.37 (d, *J* = 8.1 Hz, 1H), 5.86 (s, 2H).

**Step B:** 7-Iodobenzothiophen-4-amine (650 mg, 2.36 mmol) was dissolved in ethanol (10 mL), and then di-*tert*-butyl dicarbonate (1.55 g, 7.09 mmol) was added to the reaction system. The reaction system was stirred at 50°C under a nitrogen atmosphere for 12 hours.

After LCMS monitoring indicated the disappearance of the starting material, water was added to the reaction mixture, and the mixture was extracted with ethyl acetate (200 mL × 3) and washed with saturated brine (100 mL × 3). The combined organic phases were dried over anhydrous sodium sulfate, filtered, and concentrated under reduced pressure. The resulting mixture was purified by silica gel column chromatography to obtain *tert*-butyl (7-iodobenzothiophen-4-yl)carbamate (500 mg).

MS (ESI) M/Z: 374.0 [M-H]⁻.

**Step C:** *tert*-Butyl (7-iodobenzothiophen-4-yl)carbamate (250 mg, 666.27 µmol) was dissolved in *N,N*-dimethylformamide (3 mL), and then diphenyl(trifluoromethyl)sulfonium trifluoromethanesulfonate (593 mg, 1.47 mmol) and copper powder (127 mg, 2.00 mmol) were added to the reaction system. The reaction system was stirred at 60°C under a nitrogen atmosphere for 12 hours.

After LCMS monitoring indicated the disappearance of the starting material, water was added to the reaction mixture, and the mixture was extracted with ethyl acetate (100 mL × 3) and washed with saturated brine (100 mL × 3). The combined organic phases were dried over anhydrous sodium sulfate, filtered, and concentrated under reduced pressure. The resulting mixture was purified by silica gel column chromatography to obtain *tert*-butyl (7-(trifluoromethyl)benzothiophen-4-yl)carbamate (150 mg).

MS (ESI) M/Z: 316.0 [M-H]⁻.

**Step D:** *tert*-Butyl (7-(trifluoromethyl)benzothiophen-4-yl)carbamate (150 mg, 472.70 µmol) was dissolved in dichloromethane (3 mL), and then a solution of hydrochloric acid in 1,4-dioxane (4 M, 3 mL) was added. The reaction mixture was stirred at room temperature for 2 hours.

After LCMS monitoring indicated the disappearance of the starting material, the reaction mixture was directly concentrated under reduced pressure to obtain 7-(trifluoromethyl)benzothiophen-4-amine (100 mg).

MS (ESI) M/Z: 216.0 [M-H]⁻.

**Step E:** 7-(Trifluoromethyl)benzothiophen-4-amine (100 mg, crude product) and 2-(6-(4-(5-(benzyloxy)-6-methylpyrimidine-4-carbonyl)piperazin-1-yl)-5-ethyl-7-oxo-2-(2,5,6,7-tetrahydrooxepin-4-yl)-[1,2,4]triazolo[1,5-a]pyrimidin-4(7H)-yl)acetic acid (100 mg, 0.16 mmol) were dissolved in acetonitrile (5 mL). *N,N,N',N'-*Tetramethylchloroformamidinium hexafluorophosphate (89 mg, 0.32 mmol) and *N-*methylimidazole (65 mg, 0.79 mmol) were added, and the reaction mixture was stirred at room temperature for 1 hour.

After LCMS monitoring indicated the disappearance of the starting material, water was added to the reaction mixture, and the mixture was extracted with ethyl acetate (100 mL × 3). The combined organic phases were washed with saturated brine (20 mL × 3), dried over anhydrous sodium sulfate, filtered, and concentrated under reduced pressure. The resulting mixture was purified by silica gel column chromatography to obtain 2-(6-(4-(5-(benzyloxy)-6-methylpyrimidine-4-carbonyl)piperazin-1-yl)-5-ethyl-7-oxo-2-(2,5,6,7-tetrahydrooxepin-4-yl)-[1,2,4]triazolo[1,5-a]pyrimidin-4(7H)-yl)-N-(7-(trifluoromethyl)benzothiophen-4-yl)acetamide (100 mg).

MS (ESI) M/Z:826.2 [M-H]⁻.

**Step F:** 2-(6-(4-(5-(Benzyloxy)-6-methylpyrimidine-4-carbonyl)piperazin-1-yl)-5-ethyl-7-oxo-2-(2,5,6,7-tetrahydrooxepin-4-yl)-[1,2,4]triazolo[1,5-a]pyrimidin-4(7H)-yl)-*N-*(7-(trifluoromethyl)benzothiophen-4-yl)acetamide (100 mg, 120.79 µmol) was dissolved in trifluoroacetic acid (3 mL). The reaction system was stirred at 60°C for 1 hour.

After LCMS monitoring indicated the disappearance of the starting material, the reaction mixture was diluted with dichloromethane (60 mL) and then concentrated under reduced pressure. The resulting mixture was purified by preparative high-performance liquid chromatography. The purification conditions were as follows: chromatographic column: xbridge-C18 21.2 mm × 150 mm; mobile phase: water (containing 0.1% ammonia water) and acetonitrile; flow rate: 25 mL/min; gradient: acetonitrile from 35% to 55% within 15 minutes; detection wavelength: 254 nm. The product was collected and lyophilized under reduced pressure to obtain 2-(5-ethyl-6-(4-(5-hydroxy-6-methylpyrimidine-4-carbonyl)piperazin-1-yl)-7-oxo-2-(2,5,6,7-tetrahydrooxepin-4-yl)-[1,2,4]triazolo[1,5-a]pyrimidin-4(7H)-yl)-N-(7-(trifluoromethyl)benzo[b]thiophen-4-yl)acetamide (18.74 mg).

MS (ESI) M/Z: 738.4 [M+H] ⁺.

¹H NMR (400 MHz, DMSO-*d*₆) δ 10.66 (s, 1H), 8.61 - 8.52 (m, 1H), 8.07 - 7.98 (m, 2H), 7.93 (d, *J* = 5.7 Hz, 1H), 7.77 (d, *J* = 8.3 Hz, 1H), 6.89 (t, *J* = 4.7 Hz, 1H), 5.35 (s, 2H), 4.58 - 4.47 (m, 1H), 4.25 (d, *J* = 4.7 Hz, 2H), 3.80 (t, *J* = 5.8 Hz, 2H), 3.61 - 3.44 (m, 3H), 3.27 - 3.19 (m, 1H), 3.09 - 2.93 (m, 3H), 2.90 - 2.77 (m, 3H), 2.68 - 2.65 (m, 1H), 2.44 (s, 3H), 1.93 - 1.77 (m, 2H), 1.20 (t, *J* = 7.4 Hz, 3H).

### Example 264

### N-(2-Cyclopropyl-5-(trifluoromethyl)thiophen-3-yl)-2-(5-ethyl-6-(4-(5-hydroxy-6-methylpyrimidine-4-carbonyl)piperazin-1-yl)-7-oxo-2-(2,5,6,7-tetrahydrooxepin-4-yl)-[1,2,4]triazolo[1,5-a]pyrimidin-4(7H)-yl)acetamide

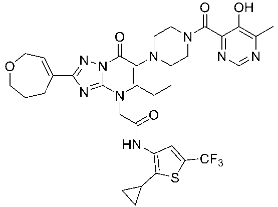

*N*-[2-Cyclopropyl-5-(trifluoromethyl)thiophen-3-yl]-2-[5-ethyl-6-(4-(5-hydroxy-6-methylpyrimidine-4-carbonyl)piperazin-1-yl)-7-oxo-2-(2,5,6,7-tetrahydrooxepin-4-yl)-[1,2,4]triazolo[1,5-a]pyrimidin-4(7H)-yl]acetamide (25.38 mg) may also be prepared with reference to the preparation method of **Example 190.**

MS (ESI) M/Z: 728.4 [M+H] ⁺.

¹H NMR (400 MHz, DMSO-*d*₆) δ 10.33 (s, 1H), 10.16 (s, 1H), 8.64 - 8.44 (m, 1H), 7.77 - 7.61 (m, 1H), 6.89 (t, *J* = 4.7 Hz, 1H), 5.18 (s, 2H), 4.66 - 4.43 (m, 1H), 4.27 (d, *J* = 4.7 Hz, 2H), 3.81 (t, *J* = 5.8 Hz, 2H), 3.58 - 3.43 (m, 3H), 3.26 - 3.18 (m, 1H), 3.11 - 2.90 (m, 3H), 2.88 - 2.72 (m, 3H), 2.69 - 2.56 (m, 1H), 2.43 (s, 3H), 2.28 - 2.17 (m, 1H), 1.92 - 1.82 (m, 2H), 1.18 (t, *J* = 7.4 Hz, 3H), 1.14 - 1.06 (m, 2H), 0.78 - 0.68 (m, 2H).

### Example 163

### N-(2-Chloro-4-(trifluoromethyl)phenyl)-2-(5-ethyl-6-(4-(5-hydroxy-6-methylpyrimidine-4-carbonyl)piperazin-1-yl)-2-(2-methyl-1-oxo-2-azaspiro[4.5]dec-7-en-8-yl)-7-oxo-[1,2,4]triazolo[1,5-a]pyrimidin-4(7H)-yl)acetamide

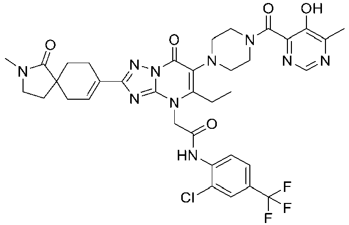

*N*-[2-Chloro-4-(trifluoromethyl)phenyl]-2-[5-ethyl-6-[4-(5-hydroxy-6-methylpyrimidine-4-carbonyl)piperazin-1-yl]-2-(2-methyl-1-oxo-2-azaspiro[4.5]dec-7-en-8-yl)-7-oxo-[1,2,4]triazolo[1,5-*a*]pyrimidin-4-yl]acetamide (8.6 mg) may also be prepared with reference to the preparation method of **Example 66.**

MS (ESI) M/Z: 783.3 [M+H] ⁺.

¹H NMR (400 MHz, DMSO-*d*₆) δ 9.82 (s, 1H), 8.49 (s, 1H), 8.07 (d, *J* = 8.5 Hz, 1H), 7.96 (s, 1H), 7.76 - 7.63 (m, 1H), 6.90 - 6.77 (m, 1H), 5.31 (s, 2H), 4.52 (d, *J* = 11.9 Hz, 1H), 3.53 - 3.45 (m, 3H), 3.24 - 3.16 (m, 3H), 3.00 - 2.91 (m, 3H), 2.81 - 2.75 (s, 4H), 2.68 - 2.64 (m, 2H), 2.42 (s, 3H), 2.38 - 2.33 (m, 2H), 2.10 - 2.05 (m, 1H), 1.93 - 1.86 (m, 1H), 1.75 - 1.68 (m, 2H), 1.60 -1.55 (m, 1H), 1.18 (t, *J* = 7.4 Hz, 3H).

### Example 277

### N-(2-Chloro-5-(trifluoromethyl)thiophen-3-yl)-2-(5-ethyl-6-(4-(5-hydroxy-6-methylpyrimidine-4-carbonyl)piperazin-1-yl)-7-oxo-2-(2,5,6,7-tetrahydrooxepin-4-yl-7,7-d₂)-[1,2,4]triazolo[1,5-a]pyrimidin-4(7H)-yl)acetamide

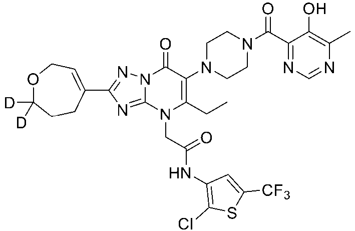

**The compound may also be synthesized according to the following steps:**

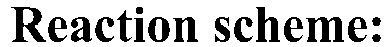

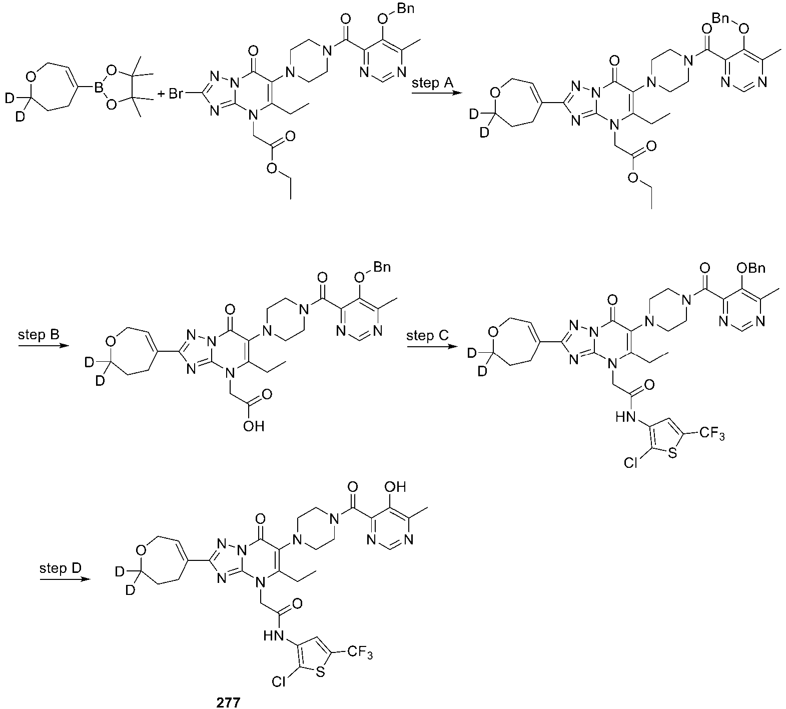

### Procedure:

**Step A:** 4,4,5,5-Tetramethyl-2-(2,5,6,7-tetrahydrooxepin-4-yl-7,7-d₂)-1,3,2-dioxaborolane (200 mg, 0.884 mmol) and ethyl 2-(6-(4-(5-(benzyloxy)-6-methylpyrimidine-4-carbonyl)piperazin-1-yl)-2-bromo-5-ethyl-7-oxo-[1,2,4]triazolo[1,5-a]pyrimidin-4(7H)-yl)acetate (100 mg, 0.159 mmol) were dissolved in 1,4-dioxane (5 mL) and water (1 mL). [1,1'-Bis(diphenylphosphino)ferrocene]dichloropalladium(II) (25 mg, 0.031 mmol) and sodium carbonate (99.4 mg, 0.938 mmol) were added, and the reaction mixture was stirred at 80°C under a nitrogen atmosphere for 3 hours.

After LCMS monitoring indicated the disappearance of the starting material, water was added to the reaction mixture, and the mixture was extracted with ethyl acetate (100 mL × 3). The combined organic phases were washed with saturated brine (20 mL × 3), dried over anhydrous sodium sulfate, filtered, and concentrated under reduced pressure. The resulting mixture was purified by C18 reverse-phase column chromatography and lyophilized to obtain ethyl 2-(6-(4-(5-(benzyloxy)-6-methylpyrimidine-4-carbonyl)piperazin-1-yl)-5-ethyl-7-oxo-2-(2,5,6,7-tetrahydrooxepin-4-yl-7,7-d₂)-[1,2,4]triazolo[1,5-a]pyrimidin-4(7H)-yl)acetate (160 mg).

MS (ESI) M/Z: 659.4 [M+H] ⁺.

**Step B:** Ethyl 2-(6-(4-(5-(benzyloxy)-6-methylpyrimidine-4-carbonyl)piperazin-1-yl)-5-ethyl-7-oxo-2-(2,5,6,7-tetrahydrooxepin-4-yl-7,7-d₂)-[1,2,4]triazolo[1,5-a]pyrimidin-4(7H)-yl)acetate (160 mg, 0.242 mmol) was dissolved in methanol (3 mL) and sodium hydroxide solution (3 mL, 2 N aqueous solution), and the reaction mixture was stirred at room temperature for 1 hour.

After LCMS monitoring indicated the disappearance of the starting material, an aqueous hydrochloric acid solution (2 M) was added to the reaction mixture to adjust the pH to 3, and then the mixture was extracted with ethyl acetate (100 mL × 3). The combined organic phases were washed with saturated brine (20 mL × 3), dried over anhydrous sodium sulfate, filtered, and concentrated under reduced pressure to obtain 2-(6-(4-(5-(benzyloxy)-6-methylpyrimidine-4-carbonyl)piperazin-1-yl)-5-ethyl-7-oxo-2-(2,5,6,7-tetrahydrooxepin-4-yl-7,7-d₂)-[1,2,4]triazolo[1,5-a]pyrimidin-4(7H)-yl)acetic acid (120 mg).

MS (ESI) M/Z: 631.4 [M+H] ⁺.

**Step C:** 2-(6-(4-(5-(Benzyloxy)-6-methylpyrimidine-4-carbonyl)piperazin-1-yl)-5-ethyl-7-oxo-2-(2,5,6,7-tetrahydrooxepin-4-yl-7,7-d₂)-[1,2,4]triazolo[1,5-a]pyrimidin-4(7H)-yl)acetic acid (100 mg, 0.159 mmol) and 2-chloro-5-(trifluoromethyl)thiophen-3-amine (32 mg, 0.159 mmol) were dissolved in acetonitrile (5 mL). *N,N,N',N'-*Tetramethylchloroformamidinium hexafluorophosphate (111 mg, 0.396 mmol) and N-methylimidazole (65 mg, 0.793 mmol) were added, and the reaction mixture was stirred at room temperature for 12 hours.

After LCMS monitoring indicated the disappearance of the starting material, water was added to the reaction mixture, and the mixture was extracted with ethyl acetate (100 mL × 3). The combined organic phases were washed with saturated brine (20 mL × 3), dried over anhydrous sodium sulfate, filtered, and concentrated under reduced pressure. The resulting mixture was purified by silica gel column chromatography to obtain 2-(6-(4-(5-(benzyloxy)-6-methylpyrimidine-4-carbonyl)piperazin-1-yl)-5-ethyl-7-oxo-2-(2,5,6,7-tetrahydrooxepin-4-yl-7,7-d₂)-[1,2,4]triazolo[1,5-a]pyrimidin-4(7H)-yl)-*N*-(2-chloro-5-(trifluoromethyl)thiophen-3-yl)acetamide (70 mg).

MS (ESI) M/Z: 814.8 [M+H] ⁺.

**Step D:** 2-(6-(4-(5-(Benzyloxy)-6-methylpyrimidine-4-carbonyl)piperazin-1-yl)-5-ethyl-7-oxo-2-(2,5,6,7-tetrahydrooxepin-4-yl-7,7-d₂)-[1,2,4]triazolo[1,5-a]pyrimidin-4(7H)-yl)-N-(2-chloro-5-(trifluoromethyl)thiophen-3-yl)acetamide (70 mg, 86.0 µmol) was dissolved in trifluoroacetic acid (3 mL). The reaction system was stirred at 60°C for 1 hour.

After LCMS monitoring indicated the disappearance of the starting material, the reaction mixture was diluted with dichloromethane (60 mL) and then concentrated under reduced pressure. The resulting mixture was purified by preparative high-performance liquid chromatography. The purification conditions were as follows: chromatographic column: xbridge-C18 21.2 mm × 150 mm; mobile phase: water (containing 0.1% ammonia water) and acetonitrile; flow rate: 25 mL/min; gradient: acetonitrile from 35% to 55% within 15 minutes; detection wavelength: 254 nm. The product was collected and lyophilized under reduced pressure to obtain N-(2-chloro-5-(trifluoromethyl)thiophen-3-yl)-2-(5-ethyl-6-(4-(5-hydroxy-6-methylpyrimidine-4-carbonyl)piperazin-1-yl)-7-oxo-2-(2,5,6,7-tetrahydrooxepin-4-yl-7,7-d₂)-[1,2,4]triazolo[1,5-a]pyrimidin-4(7H)-yl)acetamide (44.15 mg).

MS (ESI) M/Z: 724.3 [M+H] ⁺.

¹H NMR (400 MHz, DMSO-*d*₆) δ 10.55 (s, 1H), 10.20 (s, 1H), 8.57 (s, 1H), 7.97 - 7.91 (m, 1H), 6.89 (t, *J* = 4.7 Hz, 1H), 5.23 (s, 2H), 4.57 - 4.44 (m, 1H), 4.27 (d, *J* = 4.7 Hz, 2H), 3.57 - 3.41 (m, 3H), 3.27 - 3.18 (m, 1H), 3.04 - 2.90 (m, 3H), 2.88 - 2.75 (m, 3H), 2.67 - 2.59 (m, 1H), 2.44 (s, 3H), 1.90 - 1.82 (m, 2H), 1.17 (t, *J* = 7.4 Hz, 3H).

### Example 263

### 2-(5-Ethyl-6-(4-(5-hydroxy-6-methylpyrimidine-4-carbonyl)piperazin-1-yl)-7-oxo-2-(2,5,6,7-tetrahydrooxepin-4-yl)-[1,2,4]triazolo[1,5-a]pyrimidin-4(7H)-yl)-N-(2-methyl-5-(trifluoromethyl)thiophen-3-yl)acetamide

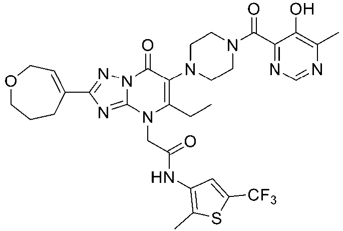

The product 2-(5-ethyl-6-(4-(5-hydroxy-6-methylpyrimidine-4-carbonyl)piperazin-1-yl)-7-oxo-2-(2,5,6,7-tetrahydrooxepin-4-yl)-[1,2,4]triazolo[1,5-a]pyrimidin-4(7H)-yl)-*N*-(2-methyl-5-(trifluoromethyl)thiophen-3-yl)acetamide (15.0 mg) may also be prepared with reference to the preparation method of **Example 190.**

MS (ESI) M/Z: 702.4 [M+H] ⁺.

¹H NMR (400 MHz, DMSO-*d*₆) δ 10.24 (s, 1H), 8.58 (s, 1H), 7.72 (s, 1H), 6.90 (t, *J* = 4.6 Hz, 1H), 5.16 (s, 2H), 4.53 (d, *J=* 12.3 Hz, 1H), 4.27 (d, *J* = 4.6 Hz, 2H), 3.82 (t, *J* = 4.6 Hz, 2H), 3.54 - 3.46 (m, 3H), 3.28 - 3.22 (m, 1H), 3.02 - 2.96 (m, 3H), 2.86 - 2.80 (m, 3H), 2.68 - 2.65 (m, 1H), 2.44 (s, 3H), 2.41 (s, 3H), 1.90 - 1.84 (m, 2H), 1.18 (t, *J* = 7.4 Hz, 3H).

### Example 284

### 2-(5-Ethyl-6-(4-(5-hydroxy-6-methylpyrimidine-4-carbonyl)piperazin-1-yl)-7-oxo-2-(2,5,6,7-tetrahydrooxepin-4-yl-7,7-D₂)-[1,2,4]triazolo[1,5-a]pyrimidin-4(7H)-yl)-N-(3-methyl-4-(trifluoromethyl)thiophen-2-yl)acetamide

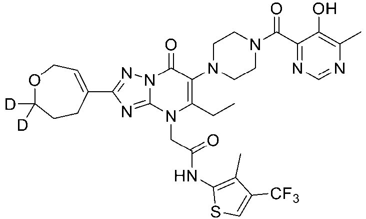

**The compound may also be synthesized according to the following steps:**

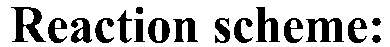

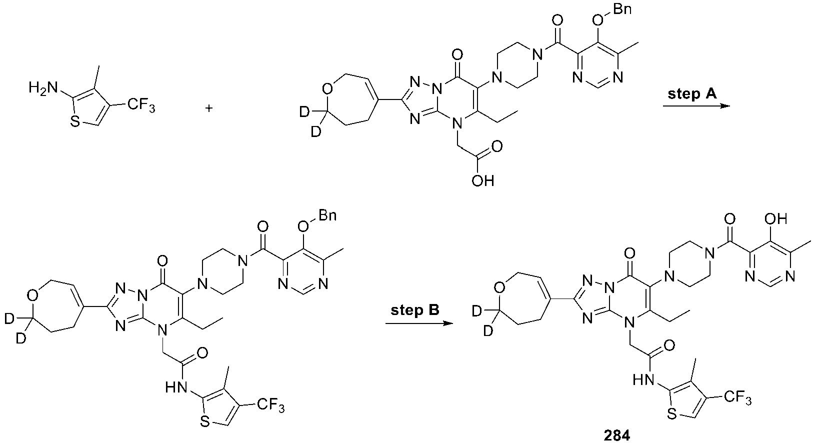

### Procedure:

**Step A:** 3-Methyl-4-(trifluoromethyl)thiophen-2-amine (18 mg, crude product) and 2-(6-(4-(5-(benzyloxy)-6-methylpyrimidine-4-carbonyl)piperazin-1-yl)-5-ethyl-7-oxo-2-(2,5,6,7-tetrahydrooxepin-4-yl-7,7-d₂)-[1,2,4]triazolo[1,5-a]pyrimidin-4(7H)-yl)acetic acid (60 mg, 95.1 µmol) were dissolved in acetonitrile (8 mL). *N,N,N',N-*Tetramethylchloroformamidinium hexafluorophosphate (80 mg, 0.29 mmol) and N-methylimidazole (39 mg, 0.48 mmol) were added at 0°C. The reaction mixture was stirred at 25°C for 1 hour.

After LCMS monitoring indicated the disappearance of the starting material, water was added to the reaction mixture, and the mixture was extracted with ethyl acetate (10 mL × 3). The combined organic phases were washed with saturated brine (10 mL × 3), dried over anhydrous sodium sulfate, filtered, and concentrated under reduced pressure. The resulting mixture was purified by silica gel column chromatography to obtain 2-(6-(4-(5-(benzyloxy)-6-methylpyrimidine-4-carbonyl)piperazin-1-yl)-5-ethyl-7-oxo-2-(2,5,6,7-tetrahydrooxepin-4-yl-7,7-d₂)-[1,2,4]triazolo[1,5-a]pyrimidin-4(7H)-yl)-N-(3-methyl-4-(trifluoromethyl)thiophen-2-yl)acetamide (50 mg).

MS (ESI) M/Z: 794.5 [M+H] ⁺.

**Step B:** Compound 2-(6-(4-(5-(benzyloxy)-6-methylpyrimidine-4-carbonyl)piperazin-1-yl)-5-ethyl-7-oxo-2-(2,5,6,7-tetrahydrooxepin-4-yl-7,7-d₂)-[1,2,4]triazolo[1,5-a]pyrimidin-4(7H)-yl)-*N*-(3-methyl-4-(trifluoromethyl)thiophen-2-yl)acetamide (50 mg, 63 µmol) was dissolved in trifluoroacetic acid (4 mL), and the reaction mixture was stirred at 60°C for 2 hours.

After LCMS monitoring indicated the disappearance of the starting material, the reaction mixture was diluted with dichloromethane and then concentrated under reduced pressure. The resulting mixture was purified by preparative high-performance liquid chromatography. The purification conditions were as follows: chromatographic column: xbridge-C18 21.2 mm × 150 mm; mobile phase: water (containing 0.1% formic acid) and acetonitrile; flow rate: 25 mL/min; gradient: acetonitrile from 40% to 70% within 20 minutes; detection wavelength: 254 nm. The product was collected and lyophilized under reduced pressure to obtain the product 2-(5-ethyl-6-(4-(5-hydroxy-6-methylpyrimidine-4-carbonyl)piperazin-1-yl)-7-oxo-2-(2,5,6,7-tetrahydrooxepin-4-yl-7,7-D₂)-[1,2,4]triazolo[1,5-*a*]pyrimidin-4(7*H*)-yl)-*N*-(3-methyl-4-(trifluoromethyl)thiophen-2-yl)acetamide (22.5 mg).

MS (ESI) M/Z: 704.4 [M+H] ⁺.

¹H NMR (400 MHz, DMSO-*d*₆) δ 10.50 (s, 2H), 8.56 (s, 1H), 7.73 (s, 1H), 6.90 - 6.83 (m, 1H), 5.27 (s, 2H), 4.55 - 4.49 (m, 1H), 4.27 - 4.23 (m, 2H), 3.54 - 3.48 (m, 3H), 3.27 - 3.21 (m, 1H), 3.00 - 2.93 (m, 3H), 2.84 - 2.78 (m, 3H), 2.68 - 2.63 (m, 1H), 2.44 (s, 3H), 2.25 (s, 3H), 1.86 - 1.82 (m, 2H), 1.16 (t, *J* = 7.2 Hz, 3H).

### Example 293

### 2-(5-Ethyl-6-((1S,6S)-5-(5-hydroxy-6-methylpyrimidine-4-carbonyl)-2,5-diazabicyclo [4.2.0]octan-2-yl)-7-oxo-2-(2,5,6,7-tetrahydrooxepin-4-yl-7,7-D₂)-[1,2,4]triazolo[1,5-a]pyrimidin-4(7H)-yl)-N-(3-methyl-4-(trifluoromethyl)thiophen-2-yl)acetamide

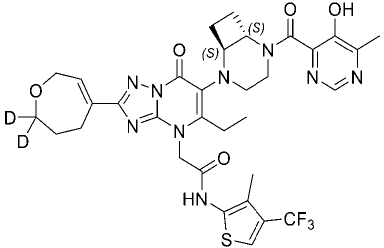

**The compound may also be synthesized according to the following steps:**

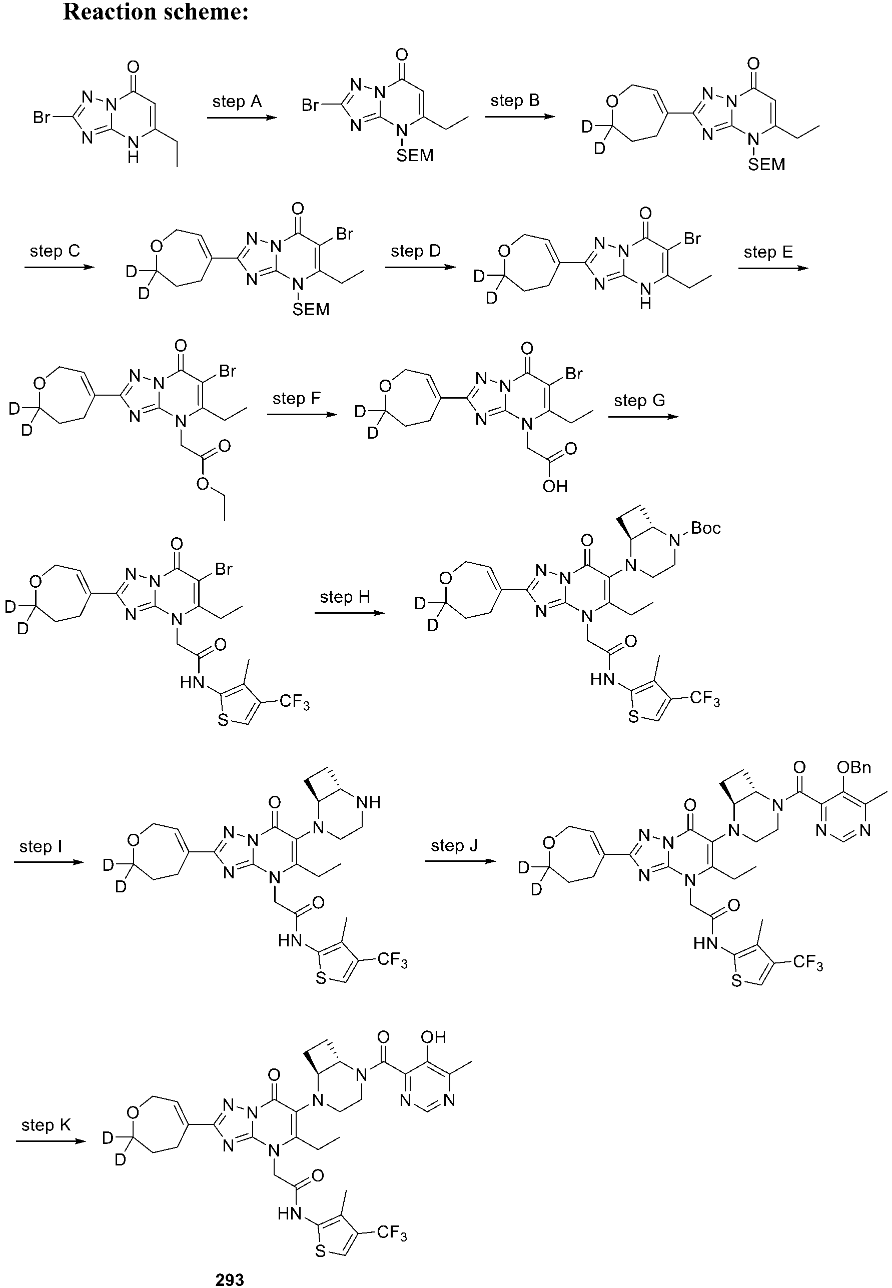

### Procedure:

**Step A:** Compound 2-bromo-5-ethyl-[1,2,4]triazolo[1,5-a]pyrimidin-7(4H)-one (2.0 g, 8.2 mmol) was dissolved in *N,N-*dimethylformamide (20 mL). Sodium hydride (0.3 g, 12.4 mmol) was then added in an ice bath, and the reaction mixture was stirred at 0°C for 30 minutes. Then, 2-(trimethylsilyl)ethoxymethyl chloride (1.5 g, 9.0 mmol) was slowly added dropwise to the reaction system. After the dropwise addition was completed, the reaction mixture was stirred from 0°C to room temperature for 2 hours.

After LCMS monitoring indicated the disappearance of the starting material, saturated aqueous ammonium chloride solution was added to the reaction system to quench the reaction. The mixture was then extracted with ethyl acetate (300 mL × 2). The combined organic phases were first washed with saturated brine (50 mL × 3), then dried over anhydrous sodium sulfate, filtered, and finally concentrated under reduced pressure. The resulting residue was purified by silica gel column chromatography to obtain compound 2-bromo-5-ethyl-4-((2-(trimethylsilyl)ethoxy)methyl)-[1,2,4]triazolo[1,5-a]pyrimidin-7(4H)-one (1.6 g).

MS (ESI) M/Z: 373.2 [M+H] ⁺.

**Step B:** Compound 2-bromo-5-ethyl-4-((2-(trimethylsilyl)ethoxy)methyl)-[1,2,4]triazolo[1,5-a]pyrimidin-7(4H)-one (1.6 g, 4.3 mmol) and 4,4,5,5-tetramethyl-2-(2,5,6,7-tetrahydrooxepin-4-yl-7,7-D₂)-1,3,2-dioxaborolane (1.9 g, 8.6 mmol) were dissolved in 1,4-dioxane (20 mL) and water (4 mL). Then, sodium carbonate (1.36 g, 12.8 mmol) and [1,1'-bis(diphenylphosphino)ferrocene]dichloropalladium(II) dichloromethane complex (350 mg, 428.8 µmol) were added. The reaction system was purged with nitrogen and then stirred at 100°C overnight.

After LCMS monitoring indicated the disappearance of the starting material, water (150 mL) was added to the reaction system, and the mixture was extracted with ethyl acetate (200 mL × 3). The combined organic phases were first washed with saturated brine (30 mL × 3), then dried over anhydrous sodium sulfate, filtered, and finally concentrated under reduced pressure. The resulting residue was purified by silica gel column chromatography to obtain compound 5-ethyl-2-(2,5,6,7-tetrahydrooxepin-4-yl-7,7-D₂)-4-((2-(trimethylsilyl)ethoxy)methyl)-[1,2,4]triazolo[1,5-a]pyrimidin-7(4H)-one (1.3 g).

MS (ESI) M/Z: 393.4 [M+H]⁺.

**Step C:** Compound 5-ethyl-2-(2,5,6,7-tetrahydrooxepin-4-yl-7,7-D₂)-4-((2-(trimethylsilyl)ethoxy)methyl)-[1,2,4]triazolo[1,5-a]pyrimidin-7(4H)-one (1.3 g, 3.3 mmol) was dissolved in acetonitrile (15 mL). Then, *N*-bromosuccinimide (587 mg, 3.3 mmol) was slowly added in an ice bath. The reaction system was stirred from 0°C to room temperature overnight.

After LCMS monitoring indicated the disappearance of the starting material, water (100 mL) was added to the reaction system, and the mixture was extracted with ethyl acetate (150 mL × 3). The combined organic phases were first washed with saturated brine (30 mL × 3), then dried over anhydrous sodium sulfate, filtered, and finally concentrated under reduced pressure. The resulting residue was purified by silica gel column chromatography to obtain compound 6-bromo-5-ethyl-2-(2,5,6,7-tetrahydrooxepin-4-yl-7,7-D₂)-4-((2-(trimethylsilyl)ethoxy)methyl)-[1,2,4]triazolo[1,5-a]pyrimidin-7(4H)-one (800 mg).

MS (ESI) M/Z: 471.3 [M+H]⁺.

**Step D:** Compound 6-bromo-5-ethyl-2-(2,5,6,7-tetrahydrooxepin-4-yl-7,7-D₂)-4-((2-(trimethylsilyl)ethoxy)methyl)-[1,2,4]triazolo[1,5-a]pyrimidin-7(4H)-one (800 mg, 1.7 mmol) was dissolved in dimethyl sulfoxide (5 mL). Then, silver tetrafluoroborate (330 mg, 1.7 mmol) was added, and the reaction mixture was stirred at 120°C for 1 hour.

LCMS monitoring indicated that the starting material had disappeared and the reaction was complete. The reaction mixture was used directly in the next step without further treatment.

MS (ESI) M/Z: 339.0 [M-H]⁻.

**Step E:** *N,N*-Diisopropylethylamine (660 mg, 5.1 mmol) was added to the reaction mixture from the previous step. Then, ethyl bromoacetate (567 mg, 3.4 mmol) was added, and the reaction system was stirred at 85°C for 2 hours.

After LCMS monitoring indicated the disappearance of the starting material, water (100 mL) was added to the reaction system, and the mixture was extracted with ethyl acetate (250 mL × 2). The combined organic phases were first washed with saturated brine (50 mL × 2), then dried over anhydrous sodium sulfate, filtered, and finally concentrated under reduced pressure. The resulting residue was purified by reverse-phase C18 chromatography (eluent: water_{(0.1% formic acid)}/acetonitrile from 98:2 to 40:60) to obtain compound ethyl 2-(6-bromo-5-ethyl-7-oxo-2-(2,5,6,7-tetrahydrooxepin-4-yl-7,7-D₂)-[1,2,4]triazolo[1,5-a]pyrimidin-4(7H)-yl)acetate (300 mg).

MS (ESI) M/Z: 427.2 [M+H]⁺.

**Step F:** Compound ethyl 2-(6-bromo-5-ethyl-7-oxo-2-(2,5,6,7-tetrahydrooxepin-4-yl-7,7-D₂)-[1,2,4]triazolo[1,5-a]pyrimidin-4(7H)-yl)acetate (300 mg, 702.1 µmol) was dissolved in methanol (2 mL), and then 2 M aqueous sodium hydroxide solution (2 mL) was added. The reaction mixture was stirred at room temperature for 1 hour.

After LCMS monitoring indicated the disappearance of the starting material, 1 N aqueous hydrochloric acid solution was added to the reaction system to adjust the pH to weakly acidic, and then the mixture was extracted with ethyl acetate (100 mL × 3). The combined organic phases were first washed with saturated brine (15 mL × 3), then dried over anhydrous sodium sulfate, filtered, and finally concentrated under reduced pressure to obtain compound 2-(6-bromo-5-ethyl-7-oxo-2-(2,5,6,7-tetrahydrooxepin-4-yl-7,7-D₂)-[1,2,4]triazolo[1,5-a]pyrimidin-4(7H)-yl)acetic acid (250 mg).

MS (ESI) M/Z: 399.2 [M+H] ⁺.

**Step G:** Compound 2-(6-bromo-5-ethyl-7-oxo-2-(2,5,6,7-tetrahydrooxepin-4-yl-7,7-D₂)-[1,2,4]triazolo[1,5-a]pyrimidin-4(7H)-yl)acetic acid (250 mg, 626.2 µmol) and 3-methyl-4-trifluoromethylthiophen-2-amine hydrochloride (200 mg, 919.0 µmol) were dissolved in acetonitrile (5 mL). Then, *N,N,N',N'*-tetramethylchloroformamidinium hexafluorophosphate (350 mg, 1.25 mmol) and *N*-methylimidazole (260 mg, 3.14 mmol) were added. The reaction mixture was stirred at room temperature for 16 hours.

After LCMS monitoring indicated the disappearance of the starting material, water (30 mL) was added to the reaction system, and the mixture was extracted with ethyl acetate (100 mL × 2). The combined organic phases were first washed with saturated brine (10 mL × 2), then dried over anhydrous sodium sulfate, filtered, and finally concentrated under reduced pressure. The resulting residue was purified by reverse-phase C18 column chromatography (eluent: water_{(0.1% formic acid)}/acetonitrile from 98:2 to 45:55) to obtain compound 2-(6-bromo-5-ethyl-7-oxo-2-(2,5,6,7-tetrahydrooxepin-4-yl-7,7-D₂)-[1,2,4]triazolo[1,5-a]pyrimidin-4(7H)-yl)-*N*-(3-methyl-4-(trifluoromethyl)thiophen-2-yl)acetamide (240 mg).

MS (ESI) M/Z: 560.1 [M-H]⁻.

**Step H:** Compound 2-(6-bromo-5-ethyl-7-oxo-2-(2,5,6,7-tetrahydrooxepin-4-yl-7,7-D₂)-[1,2,4]triazolo[1,5-a]pyrimidin-4(7H)-yl)-*N*-(3-methyl-4-(trifluoromethyl)thiophen-2-yl)acetamide (80 mg, 142.3 µmol) and *tert*-butyl (1S,6S)-2,5-diazabicyclo[4.2.0]octane-2-carboxylate (150 mg, 711.2 µmol) were dissolved in ultra-dry dimethyl sulfoxide (5 mL). Then, silver tetrafluoroborate (30 mg, 154.1 µmol) and diisopropylamine (15 mg, 142.3 µmol) were added. The reaction system was stirred at 125°C under a nitrogen atmosphere for 16 hours.

LCMS monitoring indicated the formation of the product. Water (20 mL) was added to the reaction system, and the mixture was extracted with ethyl acetate (60 mL × 3). The combined organic phases were first washed with saturated brine (10 mL × 3), then dried over anhydrous sodium sulfate, filtered, and finally concentrated under reduced pressure. The resulting residue was purified by reverse-phase C18 column chromatography (eluent: water_{(0.1% formic acid)}/acetonitrile from 98:2 to 50:50) to obtain *tert*-butyl (1S,6S)-5-(5-ethyl-4-(2-((3-methyl-4-(trifluoromethyl)thiophen-2-yl)amino)-2-oxoethyl)-7-oxo-2-(2,5,6,7-tetrahydrooxepin-4-yl-7,7-D₂)-4,7-dihydro-[1,2,4]triazolo[1,5-a]pyrimidin-6-yl)-2,5-diazabicyclo[4.2.0]octane-2-carboxylate (40 mg).

MS (ESI) M/Z: 692.3 [M-H]⁻.

**Step I:** Compound *tert*-butyl (1S,6S)-5-(5-ethyl-4-(2-((3-methyl-4-(trifluoromethyl)thiophen-2-yl)amino)-2-oxoethyl)-7-oxo-2-(2,5,6,7-tetrahydrooxepin-4-yl-7,7-D₂)-4,7-dihydro-[1,2,4]triazolo[1,5-a]pyrimidin-6-yl)-2,5-diazabicyclo[4.2.0]octane-2-carboxylate (40 mg, 57.66 µmol) was dissolved in dichloromethane (2.0 mL), and then trifluoroacetic acid (0.5 mL) was added. The reaction mixture was stirred at room temperature for 1 hour.

LCMS monitoring indicated the formation of the product. The reaction mixture was diluted with dichloromethane and then concentrated under reduced pressure to obtain 2-(6-((1S,6S)-2,5-diazabicyclo[4.2.0]octan-2-yl)-5-ethyl-7-oxo-2-(2,5,6,7-tetrahydrooxepin-4-yl-7,7-D₂)-[1,2,4]triazolo[1,5-a]pyrimidin-4(7H)-yl)-N-(3-methyl-4-(trifluoromethyl)thiophen-2-yl)acetamide (40 mg, the crude product was used directly in the next step).

MS (ESI) M/Z: 592.3 [M-H]⁻.

**Step J:** Compound 2-(6-((1S,6S)-2,5-diazabicyclo[4.2.0]octan-2-yl)-5-ethyl-7-oxo-2-(2,5,6,7-tetrahydrooxepin-4-yl-7,7-D₂)-[1,2,4]triazolo[1,5-a]pyrimidin-4(7H)-yl)-*N*-(3-methyl-4-(trifluoromethyl)thiophen-2-yl)acetamide (40 mg, crude product) and 5-benzyloxy-6-methylpyrimidine-4-carboxylic acid (33 mg, 134.8 µmol) were dissolved in acetonitrile (3 mL). Then, *N,N,N',N*'-tetramethylchloroformamidinium hexafluorophosphate (38 mg, 134.8 µmol) and *N*-methylimidazole (30 mg, 365.4 µmol) were added. The reaction mixture was stirred at room temperature for 1 hour.

After LCMS monitoring indicated the disappearance of the starting material, water (20 mL) was added to the reaction system, and the mixture was extracted with ethyl acetate (50 mL × 2). The combined organic phases were first washed with saturated brine (10 mL × 2), then dried over anhydrous sodium sulfate, filtered, and finally concentrated under reduced pressure. The resulting residue was purified by reverse-phase C18 column chromatography (eluent: water_{(0.1% formic acid)}/acetonitrile from 98:2 to 60:40) to obtain 2-(6-((1S,6S)-5-(5-(benzyloxy)-6-methylpyrimidine-4-carbonyl)-2,5-diazabicyclo[4.2.0]octan-2-yl)-5-ethyl-7-oxo-2-(2,5,6,7-tetrahydrooxepin-4-yl-7,7-D₂)-[1,2,4]triazolo[1,5-a]pyrimidin-4(7H)-yl)-N-(3-methyl-4-(trifluoromethyl)thiophen-2-yl)acetamide (6 mg).

MS (ESI) M/Z: 820.6 [M+H] ⁺.

**Step K:** Compound 2-(6-((1S,6S)-5-(5-(benzyloxy)-6-methylpyrimidine-4-carbonyl)-2,5-diazabicyclo[4.2.0]octan-2-yl)-5-ethyl-7-oxo-2-(2,5,6,7-tetrahydrooxepin-4-yl-7,7-D₂)-[1,2,4]triazolo[1,5-a]pyrimidin-4(7H)-yl)-*N*-(3-methyl-4-(trifluoromethyl)thiophen-2-yl)acetamide (6 mg, 7.3 µmol) was dissolved in trifluoroacetic acid (2 mL), and the reaction mixture was stirred at 60°C for 1 hour.

After LCMS monitoring indicated the disappearance of the starting material, the reaction mixture was diluted with dichloromethane and then concentrated under reduced pressure. The resulting mixture was purified by preparative high-performance liquid chromatography. The purification conditions were as follows: chromatographic column: xbridge-C18 21.2 mm × 150 mm; mobile phase: water (containing 0.1% ammonia water) and acetonitrile; flow rate: 25 mL/min; gradient: acetonitrile from 30% to 50% within 15 minutes; detection wavelength: 254 nm. The product was collected and lyophilized under reduced pressure to obtain the final product 2-(5-ethyl-6-((1S,6S)-5-(5-hydroxy-6-methylpyrimidine-4-carbonyl)-2,5-diazabicyclo[4.2.0]octan-2-yl)-7-oxo-2-(2,5,6,7-tetrahydrooxepin-4-yl-7,7-D₂)-[1,2,4]triazolo[1,5-a]pyrimidin-4(7H)-yl)-N-(3-methyl-4-(trifluoromethyl)thiophen-2-yl)acetamide (0.5 mg).

MS (ESI) M/Z: 730.4 [M+H] ⁺.

¹H NMR (400 MHz, DMSO-*d*₆) δ 10.91 (s, 1H), 10.41 (s, 1H), 8.55 (s, 1H), 7.73 (s, 1H), 6.87 (t, *J* = 4.7 Hz, 1H), 5.26 (s, 2H), 4.47 - 4.36 (m, 1H), 4.26 (d, *J* = 4.5 Hz, 2H), 3.71 - 3.56 (m, 1H), 3.51 - 3.32 (m, 3H), 3.28 - 3.21 (m, 1H), 3.05 - 2.89 (m, 2H), 2.89 - 2.79 (m, 2H), 2.53 - 2.51 (m, 1H), 2.43 (s, 3H), 2.25 (s, 3H), 1.90 - 1.82 (m, 2H), 1.71 - 1.59 (m, 1H), 1.42 - 1.27 (m, 2H), 1.17 (t, *J* = 7.4 Hz, 3H).

### Example 302

### N-(2-Chloro-4-(trifluoromethyl)phenyl)-2-(5-ethyl-6-((1S,6S)-5-(5-hydroxy-6-methylpyrimidine-4-carbonyl)-2,5-diazabicyclo[4.2.0]octan-2-yl)-7-oxo-2-(2,5,6,7-tetrahydrooxepin-4-yl-7,7-d₂)-[1,2,4]triazolo[1,5-a]pyrimidin-4(7H)-yl)acetamide

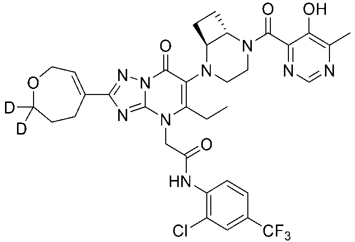

**The compound may also be synthesized according to the following steps:**

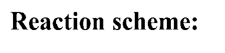

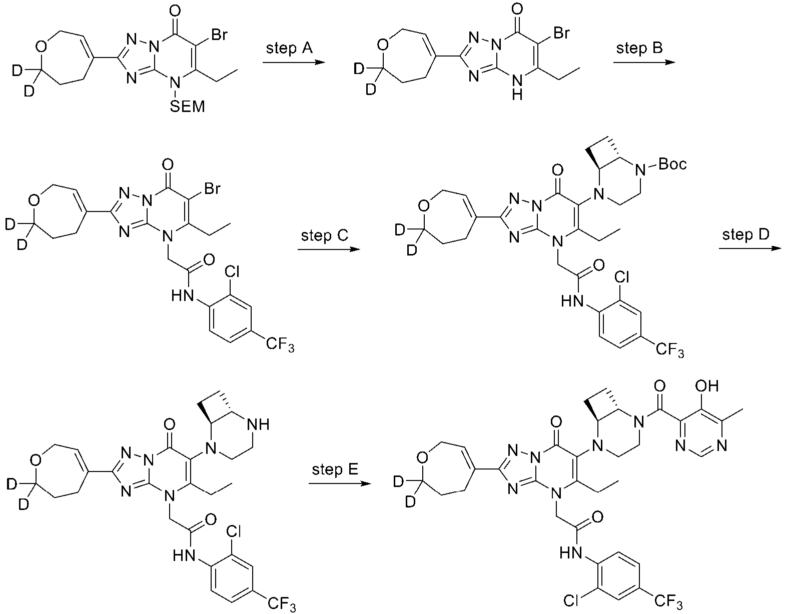

### Procedure:

**Step A:** Compound 6-bromo-5-ethyl-2-(2,5,6,7-tetrahydrooxepin-4-yl-7,7-d₂)-4-((2-(trimethylsilyl)ethoxy)methyl)-[1,2,4]triazolo[1,5-a]pyrimidin-7(4H)-one (500 mg, 1.1 mmol) was dissolved in dimethyl sulfoxide (5 mL). Then, silver tetrafluoroborate (206 mg, 1.1 mmol) was added, and the reaction mixture was stirred at 120°C for 1 hour.

LCMS monitoring indicated that the starting material had disappeared and the reaction was complete. The reaction mixture was used directly in the next step without further treatment.

MS (ESI) M/Z: 341.3 [M+H] ⁺.

**Step B:** *N,N*-Diisopropylethylamine (274 mg, 2.1 mmol) was added to the reaction mixture from the previous step. Then, *N*-(2-chloro-4-(trifluoromethyl)phenyl)-2-iodoacetamide (462.6 mg, 1.3 mmol) was added, and the reaction system was stirred at 85°C for 2 hours.

After LCMS monitoring indicated the disappearance of the starting material, water (100 mL) was added to the reaction system, and the mixture was extracted with ethyl acetate (250 mL × 2). The combined organic phases were first washed with saturated brine (50 mL × 2), then dried over anhydrous sodium sulfate, filtered, and finally concentrated under reduced pressure. The resulting residue was purified by reverse-phase C18 chromatography (eluent: water_{(0.1% formic acid)}/acetonitrile from 98:2 to 40:60) to obtain 2-(6-bromo-5-ethyl-7-oxo-2-(2,5,6,7-tetrahydrooxepin-4-yl-7,7-d₂)-[1,2,4]triazolo[1,5-a]pyrimidin-4(7H)-yl)-*N*-(2-chloro-4-(trifluoromethyl)phenyl)acetamide (380 mg).

MS (ESI) M/Z: 576.2 [M+H] ⁺.

**Step C:** Compound 2-(6-bromo-5-ethyl-7-oxo-2-(2,5,6,7-tetrahydrooxepin-4-yl-7,7-d₂)-[1,2,4]triazolo[1,5-a]pyrimidin-4(7H)-yl)-*N-*(2-chloro-4-(trifluoromethyl)phenyl)acetamide (380 mg, 658.8 µmol) and *tert*-butyl (1S,6S)-2,5-diazabicyclo[4.2.0]octane-2-carboxylate (699.3 mg, 3.29 mmol) were dissolved in ultra-dry dimethyl sulfoxide (4 mL). Then, silver tetrafluoroborate (142 mg, 724.7 µmol) and diisopropylamine (74 mg, 724.7 µmol) were added. The reaction system was stirred at 125°C under a nitrogen atmosphere for 16 hours.

LCMS monitoring indicated the formation of the product. Water (20 mL) was added to the reaction system, and the mixture was extracted with ethyl acetate (60 mL × 3). The combined organic phases were first washed with saturated brine (10 mL × 3), then dried over anhydrous sodium sulfate, filtered, and finally concentrated under reduced pressure. The resulting residue was purified by reverse-phase C18 column chromatography (eluent: water_{(0.1% formic acid)}/acetonitrile from 98:2 to 50:50) to obtain compound *tert*-butyl (1S,6S)-5-(4-(2-((2-chloro-4-(trifluoromethyl)phenyl)amino)-2-oxoethyl)-5-ethyl-7-oxo-2-(2,5,6,7-tetrahydrooxepin-4-yl-7,7-d₂)-4,7-dihydro-[1,2,4]triazolo[1,5-a]pyrimidin-6-yl)-2,5-diazabicyclo[4.2.0]octane-2-carboxylate (170 mg).

MS (ESI) M/Z: 706.3 [M-H]⁻.

**Step D:** tert-Butyl (1S,6S)-5-(4-(2-((2-chloro-4-(trifluoromethyl)phenyl)amino)-2-oxoethyl)-5-ethyl-7-oxo-2-(2,5,6,7-tetrahydrooxepin-4-yl-7,7-d₂)-4,7-dihydro-[1,2,4]triazolo[1,5-a]pyrimidin-6-yl)-2,5-diazabicyclo[4.2.0]octane-2-carboxylate (80 mg, 112.9 µmol) was dissolved in dichloromethane (3 mL), and then trifluoroacetic acid (1 mL) was added. The reaction was stirred at room temperature for 1 hour.

LCMS monitoring indicated the disappearance of the starting material. The mixture was diluted with dichloromethane and then concentrated under reduced pressure to obtain 2-(6-((l S,6S)-2,5-diazabicyclo[4.2.0]octan-2-yl)-5-ethyl-7-oxo-2-(2,5,6,7-tetrahydrooxepin-4-yl-7,7-d₂)-[1,2,4]triazolo[1,5-a]pyrimidin-4(7H)-yl)-N-(2-chloro-4-(trifluoromethyl)phenyl)acetamide (50 mg).

MS (ESI) M/Z: 608.3 [M+H] ⁺.

**Step E:** 2-(6-((1S,6S)-2,5-Diazabicyclo[4.2.0]octan-2-yl)-5-ethyl-7-oxo-2-(2,5,6,7-tetrahydrooxepin-4-yl-7,7-d₂)-[1,2,4]triazolo[1,5-a]pyrimidin-4(7H)-yl)-*N*-(2-chloro-4-(trifluoromethyl)phenyl)acetamide (50 mg, 82.2 µmol) and 5-hydroxy-6-methylpyrimidine-4-carboxylic acid (13.2 mg, 85.8 µmol) were dissolved in acetonitrile (5 mL). Then, *N,N,N',N'-*tetramethylchloroformamidinium hexafluorophosphate (58 mg, 205.6 µmol) and *N-*methylimidazole (17 mg, 205.6 mmol) were added. The reaction mixture was stirred at room temperature for 16 hours.

After TLC monitoring indicated the disappearance of the starting material, water (30 mL) was added to the reaction system, and the mixture was extracted with ethyl acetate (100 mL × 2). The combined organic phases were first washed with saturated brine (10 mL × 2), then dried over anhydrous sodium sulfate, filtered, and finally concentrated under reduced pressure to obtain a crude product. The crude product was then dissolved in trifluoroacetic acid (5 mL), and the mixture was heated to 60°C and reacted for 1 hour.

LCMS monitoring indicated the disappearance of the starting material. The reaction mixture was diluted with dichloromethane and then concentrated under reduced pressure. The resulting mixture was purified by preparative high-performance liquid chromatography under the following purification conditions: chromatographic column: xbridge-C18 21.2 mm × 150 mm; mobile phase: water (containing 0.1% ammonia water) and acetonitrile; flow rate: 25 mL/min; gradient: acetonitrile from 30% to 50% within 15 minutes; detection wavelength: 254 nm. The product was collected and lyophilized under reduced pressure to obtain N-(2-chloro-4-(trifluoromethyl)phenyl)-2-(5-ethyl-6-((1S,6S)-5-(5-hydroxy-6-methylpyrimidine-4-carbonyl)-2,5-diazabicyclo[4.2.0]octan-2-yl)-7-oxo-2-(2,5,6,7-tetrahydrooxepin-4-yl-7,7-d₂)-[1,2,4]triazolo[1,5-a]pyrimidin-4(7H)-yl)acetamide (22.05 mg).

MS (ESI) M/Z: 744.4 [M+H] ⁺.

¹H NMR (400 MHz, DMSO-*d*₆) δ 10.78 (s, 1H), 10.37 (s, 1H), 8.50 (s, 1H), 8.04 (d, *J* = 8.5 Hz, 1H), 7.96 (d, *J =* 2.2 Hz, 1H), 7.71 (dd, *J =* 8.8, 2.1 Hz, 1H), 6.91 (t, *J =* 4.7 Hz, 1H), 5.30 (s, 2H), 4.48 - 4.36 (m, 1H), 4.26 (d, *J* = 4.6 Hz, 2H), 3.68 - 3.56 (m, 1H), 3.48 - 3.36 (m, 3H), 3.27 - 3.22 (m, 1H), 3.10 - 2.91 (m, 2H), 2.87 - 2.80 (m, 2H), 2.50 (m, 1H), 2.42 (s, 3H), 1.90 - 1.81 (m, 2H), 1.67 - 1.55 (m, 1H), 1.46 - 1.29 (m, 2H), 1.19 (t, *J* = 7.4 Hz, 3H).

### Example 303

### N-(2-Chloro-4-(trifluoromethyl)phenyl)-6-((1S,6S)-5-(5-hydroxy-6-methylpyrimidine-4-carbonyl)-2,5-diazabicyclo[4.2.0]octan-2-yl)-7-methyl-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxamide

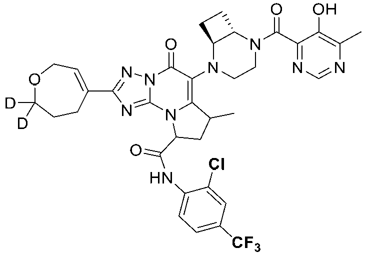

**The compound may also be synthesized according to the following steps:**

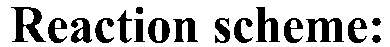

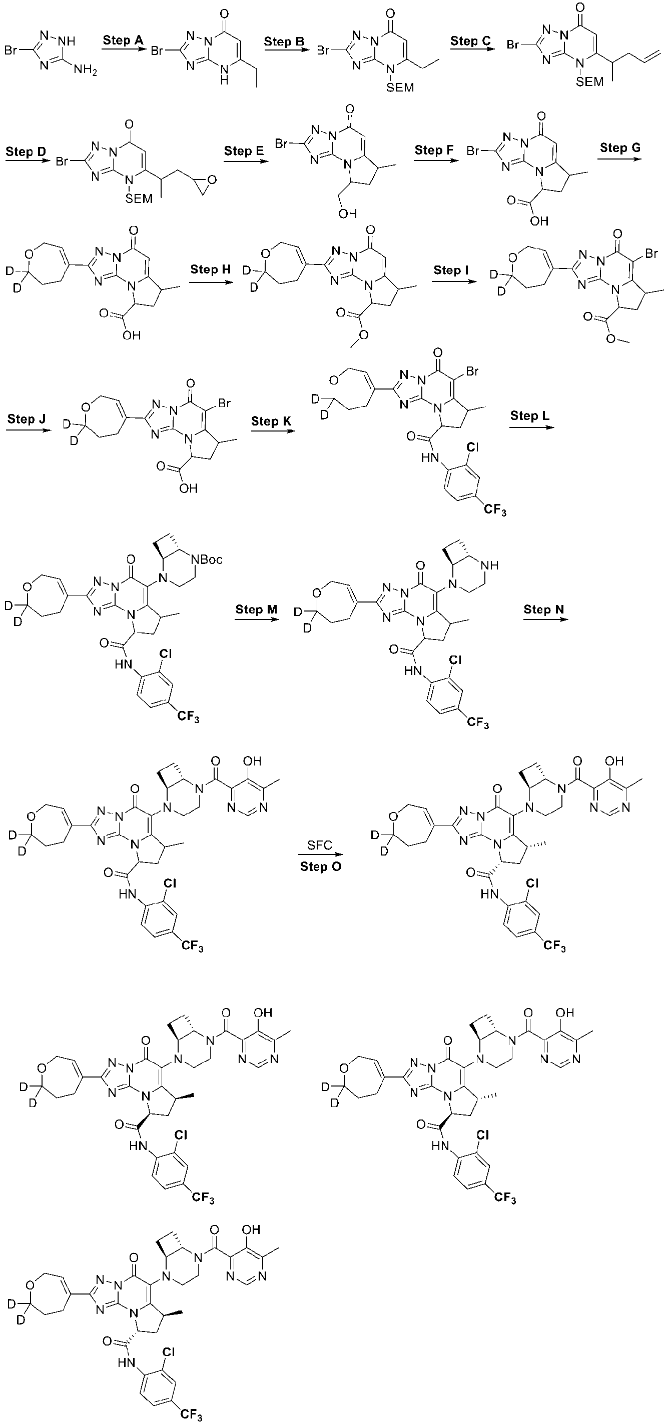

### Procedure:

**Step A:** 5-Bromo-1H-1,2,4-3-amino-1,2,4-triazole (150 g, 920.38 mmol) and ethyl propionylacetate (199 g, 1.38 mol) were dissolved in acetic acid (500 mL). The reaction system was heated to 110°C and stirred for 48 hours.

After LCMS monitoring indicated the disappearance of the starting material and the formation of the product, the reaction mixture was cooled to room temperature, and the product precipitated. The mixture was filtered under reduced pressure, and the solid was collected. The solid was added to petroleum ether (1 L) and stirred for 1 hour. The mixture was filtered under reduced pressure to obtain the product 2-bromo-5-ethyl-[1,2,4]triazolo[1,5-a]pyrimidin-7(4H)-one (150 g).

MS (ESI) M/Z: 243.1 [M+H] ⁺.

**Step B:** Sodium hydride (40 g, 987.4 mmol, 60%) was dissolved in *N,N-*dimethylformamide (1 L). Then, 2-bromo-5-ethyl-[1,2,4]triazolo[1,5-a]pyrimidin-7(4H)-one (160 g, 658.3 mmol) was slowly added portionwise in an ice-water bath. The mixture was stirred at 0°C for 1 hour. 2-(Trimethylsilyl)ethoxymethyl chloride (132 g, 789.9 mmol) was added. After the dropwise addition was completed, the mixture was warmed to room temperature and stirred for 1 hour.

After LCMS monitoring indicated the disappearance of the starting material and the formation of the product, the reaction was quenched with saturated aqueous ammonium chloride solution, and then extracted with methyl *tert*-butyl ether (700 mL×3). The combined organic phases were washed with saturated brine (500 mL×3), dried, filtered, and concentrated under reduced pressure to obtain a crude product. The crude product was slurried with petroleum ether to obtain 2-bromo-5-ethyl-4-(2-trimethylsilylethoxymethyl)-[1,2,4]triazolo[1,5-a]pyrimidin-7-one (130 g).

MS (ESI) M/Z: 315.2 [M-58+H] ⁺.

**Step C:** Compound 2-bromo-5-ethyl-4-(2-trimethylsilylethoxymethyl)-[1,2,4]triazolo[1,5-a]pyrimidin-7-one (130 g, 348.2 mmol) was dissolved in tetrahydrofuran (1 L). Then, sodium bis(trimethylsilyl)amide (1 M, 417.9 mmol, 418 mL) was slowly added at -78°C, and the mixture was stirred at -78°C for 1 hour. Subsequently, 3-bromopropene (51 g, 417.9 mmol) was slowly added dropwise to the reaction system. After the dropwise addition was completed, the reaction was transferred to an ice-water bath and stirred, allowing the temperature to rise naturally, for 1 hour.

After LCMS monitoring indicated the disappearance of the starting material, saturated aqueous ammonium chloride solution was added to the reaction system to quench the reaction. The mixture was extracted with ethyl acetate (1000 mL × 2). The combined organic phases were first washed with saturated brine (300 mL × 3), then dried over anhydrous sodium sulfate, filtered, and concentrated under reduced pressure to obtain compound 2-bromo-5-(1-methylbut-3-enyl)-4-(2-trimethylsilylethoxymethyl)-[1,2,4]triazolo[1,5-a]pyrimidin-7-one (130 g, the crude product was used directly in the next step).

MS (ESI) M/Z: 355.3 [M-58+H] ⁺.

**Step D:** 2-Bromo-5-(pent-4-en-2-yl)-4-((2-(trimethylsilyl)ethoxy)methyl)-[1,2,4]triazolo[1,5-a]pyrimidin-7(4H)-one (130 g, crude product) was dissolved in dichloromethane (1 L). Then, *m*-chloroperoxybenzoic acid (109 g, 629.0 mmol) was slowly added in an ice-water bath. The reaction system was stirred at room temperature overnight.

After LCMS monitoring indicated the disappearance of the starting material, the reaction mixture was cooled, then filtered through diatomite. The filtrate was quenched with aqueous sodium thiosulfate solution. The mixture was extracted with dichloromethane. The combined organic phases were first washed with saturated brine (300 mL × 2), then dried over anhydrous sodium sulfate, filtered, and finally concentrated under reduced pressure. The resulting residue was purified by silica gel column chromatography to obtain compound 2-bromo-5-(1-(oxiran-2-yl)propan-2-yl)-4-((2-(trimethylsilyl)ethoxy)methyl)-[1,2,4]triazolo[1,5-a]pyrimidin-7(4H)-one (100 g).

MS (ESI) M/Z: 371.2 [M-58+H] ⁺.

¹H NMR (400 MHz, DMSO-*d*₆) δ 6.22 (d, *J* = 16.3 Hz, 1H), 5.76 - 5.67 (m, 2H), 3.72 - 3.68 (m, 2H), 3.34 - 3.32 (m, 1H), 3.06 - 2.95 (m, 1H), 2.75 - 2.68 (m, 1H), 2.53 - 2.48 (m, 1H), 1.98 - 1.65 (m, 2H), 1.38 - 1.35 (m, 3H), 0.97 - 0.91 (m, 2H), 0.00 (s, 9H).

**Step E:** Under a nitrogen atmosphere, 2-bromo-5-(1-(oxiran-2-yl)propan-2-yl)-4-((2-(trimethylsilyl)ethoxy)methyl)-[1,2,4]triazolo[1,5-a]pyrimidin-7(4H)-one (13 g, 30.28 mmol) was dissolved in DMSO (40 mL). Then, silver tetrafluoroborate (7.07 g, 36.33 mmol) was added. Subsequently, the reaction system was transferred to an oil bath at 120°C and stirred for one hour.

After LCMS monitoring indicated the disappearance of the starting material, the reaction mixture was cooled, then filtered through diatomite. The filtrate was directly purified by reverse-phase C18 column chromatography to obtain 2-bromo-9-hydroxymethyl-7-methyl-8,9-dihydropyrrolo[1,2-c][1,2,4]triazolo[1,5-a]pyrimidin-5(7H)-one (4.5 g, 15.04 mmol).

MS (ESI) M/Z: 299.3 [M+H] ⁺.

**Step F:** 2-Bromo-9-hydroxymethyl-7-methyl-8,9-dihydropyrrolo[1,2-c][1,2,4]triazolo[1,5-a]pyrimidin-5(7H)-one (4.5 g, 15.04 mmol) was dissolved in acetonitrile (20 mL), and then saturated aqueous potassium dihydrogen phosphate solution (20 mL) was added. The reaction mixture was cooled to 0°C, and then TEMPO (4.7 g, 30.09 mmol) and sodium chlorite (2.7 g, 30.09 mmol) were added. Finally, 10% aqueous sodium hypochlorite solution (30 mL) was slowly added dropwise. After the dropwise addition was completed, the reaction mixture was stirred at room temperature for 3 hours.

After LCMS monitoring indicated the disappearance of the starting material and the formation of the product, sodium bicarbonate was added to the reaction mixture and stirred for 5 minutes. Then, the reaction mixture was extracted with ethyl acetate, and the product was in the aqueous phase. Subsequently, dilute hydrochloric acid was added to the aqueous phase to adjust the pH to acidic, and the mixture was extracted three times with ethyl acetate. The combined organic phases were dried over anhydrous sodium sulfate, filtered, and concentrated under reduced pressure to obtain 2-bromo-7-methyl-5-oxo-5,7,8,9-tetrahydropyrrolo[1,2-c][1,2,4]triazolo[1,5-a]pyrimidine-9-carboxylic acid (3.0 g).

MS (ESI) M/Z: 313.1 [M+H] ⁺.

**Step G:** Compound 2-bromo-7-methyl-5-oxo-5,7,8,9-tetrahydropyrrolo[1,2-c][1,2,4]triazolo[1,5-a]pyrimidine-9-carboxylic acid (1.5 g, 4.79 mmol) and 4,4,5,5-tetramethyl-2-(2,5,6,7-tetrahydrooxepin-4-yl-7,7-D₂)-1,3,2-dioxaborolane (2.2 g, 9.6 mmol) were dissolved in 1,4-dioxane (16 mL) and water (2 mL). Then, potassium carbonate (2.0 g, 14.5 mmol) and [1,1'-bis(diphenylphosphino)ferrocene]dichloropalladium(II) (350 mg, 478.3 µmol) were added. The reaction system was purged with nitrogen and then stirred at 115°C for 3 hours.

After LCMS monitoring indicated the disappearance of the starting material, the reaction system was filtered through diatomite. Then, water (200 mL) was added to the filtrate, and the mixture was extracted with ethyl acetate (200 mL × 2). The product was in the aqueous phase. The aqueous phase was adjusted to a weakly acidic pH with dilute hydrochloric acid, and then extracted with ethyl acetate (200 mL × 2). The organic phase was dried over anhydrous sodium sulfate, filtered, and finally concentrated under reduced pressure to obtain compound 7-methyl-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxylic acid (720 mg).

MS (ESI) M/Z: 333.3 [M+H] ⁺.

**Step H:** 7-Methyl-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxylic acid (720 mg, 2.17 mmol) was dissolved in DMF (10 mL), and then potassium carbonate (516 mg, 3.73 mmol) and iodomethane (530 mg, 3.73 mmol) were added. The reaction mixture was stirred at room temperature for 2 hours.

After LCMS monitoring indicated the disappearance of the starting material, the reaction system was first filtered, and then dilute hydrochloric acid was added to adjust the pH to neutral. Water (200 mL) was added to the reaction system, and the mixture was extracted with ethyl acetate (300 mL × 3). The combined organic phases were first washed with saturated brine (30 mL × 3), then dried over anhydrous sodium sulfate, filtered, and finally concentrated under reduced pressure. The resulting residue was purified by silica gel column chromatography to obtain compound methyl 7-methyl-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxylate (720 mg).

MS (ESI) M/Z: 347.4 [M+H] ⁺.

**Step I:** Compound methyl 7-methyl-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxylate (700 mg, 2.02 mmol) was dissolved in acetonitrile (15 mL), and then NBS (396 mg, 2.22 mmol) was added in an ice bath. The reaction mixture was naturally warmed and stirred overnight.

After LCMS monitoring indicated the disappearance of the starting material, the reaction system was quenched with sodium thiosulfate. Water (100 mL) was then added to the reaction system, and the mixture was extracted with ethyl acetate (150 mL × 3). The combined organic phases were first washed with saturated brine (30 mL × 3), then dried over anhydrous sodium sulfate, filtered, and finally concentrated under reduced pressure. The resulting residue was purified by silica gel column chromatography to obtain compound methyl 6-bromo-7-methyl-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxylate (620 mg).

MS (ESI) M/Z: 425.2 [M+H] ⁺.

**Step J:** Compound methyl 6-bromo-7-methyl-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxylate (620 mg, 1.46 mmol) was dissolved in methanol (3 mL), and then 2 M aqueous sodium hydroxide solution (3 mL) was added. The reaction mixture was stirred at room temperature for 1 hour.

After LCMS monitoring indicated the disappearance of the starting material, the reaction system was adjusted to acidic pH with dilute hydrochloric acid. Water (30 mL) was added to the reaction mixture, and the mixture was extracted with ethyl acetate (100 mL × 3). The combined organic phases were first washed with saturated brine (20 mL × 3), then dried over anhydrous sodium sulfate, filtered, and finally concentrated under reduced pressure to obtain 6-bromo-7-methyl-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxylic acid (550 mg).

MS (ESI) M/Z: 411.2 [M+H] ⁺.

**Step K:** 2-Chloro-4-(trifluoromethyl)aniline (518 mg, 2.66 mmol) and 6-bromo-7-methyl-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxylic acid (550 mg, 1.33 mmol) were dissolved in acetonitrile (15 mL), and then TCFH (1.1 g, 3.99 mmol) and *N*-methylimidazole (545 mg, 6.65 mmol) were added. The reaction mixture was stirred at 25°C for 3 hour.

After LCMS monitoring indicated the disappearance of the starting material, water was added to the reaction mixture, and the mixture was extracted with dichloromethane (50 mL × 3). The combined organic phases were washed with saturated brine (10 mL × 3), dried over anhydrous sodium sulfate, filtered, and concentrated under reduced pressure. The resulting mixture was purified by silica gel column chromatography to obtain 6-bromo-*N*-(2-chloro-4-(trifluoromethyl)phenyl)-7-methyl-5-oxo-2-(2,5,6,7-tetrahydrooxepin-4-yl-7,7-d₂)-*5,7,8,9-tetrahydropyrrolo[1,2-c][1,2,4]triazolo[1,5-a]pyrimidine-9-carboxamide* (280 mg).

MS (ESI) M/Z: 588.2 [M+H] ⁺.

**Step L:** In a microwave tube, 6-bromo-N-(2-chloro-4-(trifluoromethyl)phenyl)-7-methyl-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (280 mg, 476.1 µmol) and *tert*-butyl (1S,6S)-2,5-diazabicyclo[4.2.0]octane-2-carboxylate (560 mg, 2.6 mmol) were dissolved in DMSO (3 mL). Then, the reaction mixture was stirred at 120°C for 1 hour.

LCMS monitoring indicated 40% product. The reaction mixture was directly purified by reverse-phase C18 column chromatography to obtain compound *tert*-butyl (1S,6S)-5-(9-((2-chloro-4-(trifluoromethyl)phenyl)carbamoyl)-7-methyl-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidin-6-yl)-2,5-diazabicyclo[4.2.0]octane-2-carboxylate (120 mg).

MS (ESI) M/Z: 720.7 [M+H] ⁺.

**Step M:** *tert-*Butyl (1S,6S)-5-(9-((2-chloro-4-(trifluoromethyl)phenyl)carbamoyl)-7-methyl-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidin-6-yl)-2,5-diazabicyclo[4.2.0]octane-2-carboxylate (120 mg, 166.9 µmol) was dissolved in dichloromethane (3 mL), and then trifluoroacetic acid (1 mL) was added to the above system. The reaction mixture was stirred at room temperature for 1 hour.

After LCMS monitoring indicated the disappearance of the starting material, the resulting mixture was diluted with dichloromethane and then concentrated under reduced pressure to obtain 6-((1S,6S)-2,5-diazabicyclo[4.2.0]octan-2-yl)-N-(2-chloro-4-(trifluoromethyl)phenyl)-7-methyl-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (120 mg). The crude product was used directly in the next step without further purification.

MS (ESI) M/Z: 620.6 [M+H] ⁺.

**Step N:** Compound 5-hydroxy-6-methylpyrimidine-4-carboxylic acid (30 mg, 194.8 µmol) was dissolved in DMF (2 mL). EDCI (45 mg, 233.7 µmol) and HOAT (32 mg, 233.7 µmol) were added at 0°C. The reaction mixture was stirred at 0°C for 30 minutes and for later use. 6-((1S,6S)-2,5-Diazabicyclo[4.2.0]octan-2-yl)-N-(2-chloro-4-(trifluoromethyl)phenyl)-7-methyl-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (120 mg, crude product) was dissolved in DMF (2 mL), and then DIEA was added at 0°C until the system became basic. The previously prepared solution was added dropwise to the reaction system. After the dropwise addition was completed, the mixture was stirred at 0°C for 10 minutes.

After LCMS monitoring indicated the disappearance of the starting material, the reaction mixture was directly purified by reverse-phase C18 column chromatography to obtain compound *N*-(2-chloro-4-(trifluoromethyl)phenyl)-6-((1S,6S)-5-(5-hydroxy-6-methylpyrimidine-4-carbonyl)-2,5-diazabicyclo[4.2.0]octan-2-yl)-7-methyl-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxamide (100 mg).

MS (ESI) M/Z: 756.4 [M+H] ⁺.

**Step O:** *N*-(2-Chloro-4-(trifluoromethyl)phenyl)-6-((1S,6S)-5-(5-hydroxy-6-methylpyrimidine-4-carbonyl)-2,5-diazabicyclo[4.2.0]octan-2-yl)-7-methyl-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxamide (100 mg, 132.6 µmol) was separated by preparative SFC under the following separation conditions: preparative column: DAICEL CHIRALPAK^{®}OD-10, 25 * 250 mm 10 m; mobile phase: supercritical CO₂/ MeOH+(0.05%) *N*H₃·MeOH; flow rate: 80 mL/min; gradient: 78:22; detection wavelength: 214 nm/254 nm. The product was collected and lyophilized under reduced pressure to obtain mixture 1 (35 mg, retention time: 4.34 min), mixture 2 (23 mg, retention time: 5.89 min), and single-configuration product 303-P3 (7.49 mg, retention time: 4.52 min).

Mixture 1 (35 mg) was separated by preparative SFC under the following separation conditions: preparative column: DAICEL CHIRALPAK^{®}OJ-10, 25*250 mm 10 µm; mobile phase: supercritical CO₂/ IPA+(0.05%) *N*H₃·MeOH; flow rate: 80 mL/min; gradient: 70:30; detection wavelength: 214 nm/254 nm. The product was collected and lyophilized under reduced pressure to obtain single-configuration product 303-P1 (14.54 mg, retention time: 3.66 min) and single-configuration product 303-P2 (11.64 mg, retention time: 4.35 min).

Mixture 2 (23 mg) was separated by preparative SFC under the following separation conditions: preparative column: DAICEL CHIRALPAK^{®}OD-10, 25*250 mm 10 µm; mobile phase: supercritical CO₂/ MeOH+(0.05%) NH₃·MeOH; flow rate: 80 mL/min; gradient: 60:40; detection wavelength: 214 nm/254 nm. The product was collected and lyophilized under reduced pressure to obtain single-configuration product 303-P4 (8.36 mg, retention time: 5.89 min).

### 303-P1:

MS (ESI) M/Z: 756.6 [M+H] ⁺.

¹H NMR (400 MHz, DMSO-*d*₆) δ 10.71 - 10.13 (m, 2H), 8.51 (s, 1H), 8.07 - 7.85 (m, 2H), 7.80 - 7.69 (m, 1H), 6.85 (t, *J =* 4.7 Hz, 1H), 5.59 (dd, *J =* 8.6, 5.0 Hz, 1H), 4.47 - 4.31 (m, 1H), 4.25 (d, *J =* 4.7 Hz, 2H), 4.15 - 3.42 (m, 3H), 3.40 - 3.33 (m, 2H), 3.24 - 3.15 (m, 1H), 2.90 - 2.77 (m, 2H), 2.62 - 2.55 (m, 1H), 2.45 - 2.35 (m, 4H), 1.89 - 1.80 (m, 2H), 1.69 - 1.61 (m, 1H), 1.55 (d, *J =* 7.0 Hz, 3H), 1.48 - 1.31 (m, 2H), 1.26 - 1.13 (m, 1H).

### 303-P2:

MS (ESI) M/Z: 756.4 [M+H] ⁺.

¹H NMR (400 MHz, DMSO-d₆) δ 11.11 - 10.06 (m, 2H), 8.43 (s, 1H), 8.04 - 7.85 (m, 2H), 7.79 - 7.64 (m, 1H), 6.85 (t, *J =* 4.7 Hz, 1H), 5.59 (dd, *J =* 8.7, 4.4 Hz, 1H), 4.46 - 4.32 (m, 1H), 4.25 (d, *J =* 4.7 Hz, 2H), 3.72 - 3.63 (m, 1H), 3.57 - 3.38 (m, 4H), 3.27 - 3.22 (m, 1H), 2.90 - 2.76 (m, 2H), 2.61 - 2.56 (m, 1H), 2.42 - 2.34 (m, 4H), 1.90 - 1.79 (m, 2H), 1.67 - 1.58 (m, 1H), 1.53 (d, *J =* 7.0 Hz, 3H), 1.43 - 1.28 (m, 2H), 1.21 - 1.11 (m, 1H).

### 303-P3:

MS (ESI) M/Z: 756.2 [M+H] ⁺.

¹H NMR (400 MHz, DMSO-*d*₆) δ 10.68 - 9.91 (m, 2H), 8.56 (s, 1H), 8.05 - 7.94 (m, 2H), 7.83 - 7.72 (m, 1H), 6.90 (t, *J =* 4.7 Hz, 1H), 5.55 (dd, *J =* 10.4, 2.3 Hz, 1H), 4.50 - 4.35 (m, 1H), 4.30 (d, *J =* 4.7 Hz, 2H), 4.19 - 3.45 (m, 3H), 3.29 - 3.18 (m, 2H), 3.09 - 2.98 (m, 1H), 2.93 - 2.83 (m, 2H), 2.57 - 2.56 (m, 1H), 2.46 (s, 3H), 2.21 - 2.14 (m, 1H), 1.93 - 1.84 (m, 2H), 1.77 - 1.67 (m, 1H), 1.50 (d, *J =* 7.3 Hz, 3H), 1.47 - 1.35 (m, 2H), 1.26 - 1.17 (m, 1H).

### 303-P4:

MS (ESI) M/Z: 756.4 [M+H] ⁺.

¹H NMR (400 MHz, DMSO-*d*₆) δ 10.74 - 9.75 (m, 2H), 8.49 (s, 1H), 8.06 - 7.91 (m, 2H), 7.82 - 7.67 (m, 1H), 6.87 (t, *J =* 4.7 Hz, 1H), 5.60 - 5.45 (m, 1H), 4.48 - 4.33 (m, 1H), 4.26 (d, *J =* 4.8 Hz, 2H), 3.79 - 3.67 (m, 1H), 3.58 - 3.36 (m, 4H), 3.08 - 2.93 (m, 1H), 2.90 - 2.77 (m, 2H), 2.54 - 2.52 (m, 1H), 2.41 (s, 3H), 2.14 - 2.05 (m, 1H), 1.90 - 1.80 (m, 2H), 1.67 - 1.55 (m, 1H), 1.43 (d, *J =* 7.3 Hz, 3H), 1.41 - 1.30 (m, 2H), 1.21 - 1.12 (m, 1H).

Approximately 10.0 mg of compound **303-P4** was weighed, dissolved in methanol (1.0 mL, analytical grade), and sonicated at room temperature for 2 minutes. Using the diffusion method, n-hexane (10 mL, analytical grade) was used as the poor solvent. After standing at room temperature for 15 days, colorless block-shaped crystals were obtained.

### X-ray single-crystal diffractometer parameters:

| | | | |
|---|---|---|---|
| Source: | Cu target | X-ray: | Cu-K (= 1.54178 Å) |
| Detector: | CMOS area detector | Resolution: | 0.86 Å |
| Current and voltage: | 50 kV, 1.2 mA | Exposure time: | 10 s |
| Distance from area detector to sample: | 40 mm | Test temperature: | 170(2) K |

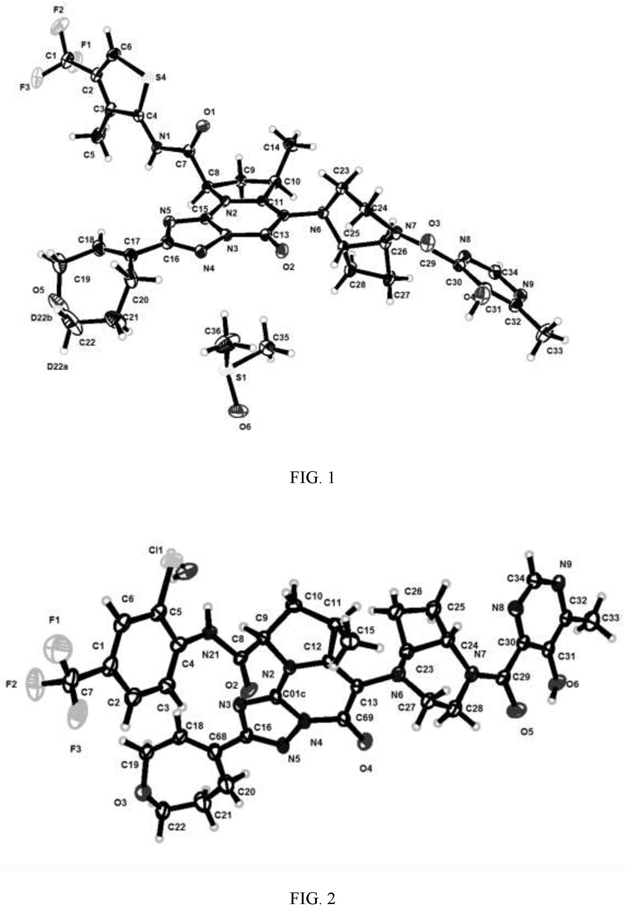

After the diffraction data were integrated and reduced using the SAINT program, empirical absorption correction was applied to the data using the SADABS program. The single-crystal structure was solved by direct methods using SHELXT2014, and the structure was refined by the least-squares method. Hydrogen atoms were treated isotropically during refinement. Hydrogen atoms on C-H were placed in calculated positions and refined using a riding model. The Flack constant was 0.13 (3), allowing the absolute configuration to be determined. In the structure, the configurations of C23 and C24 were determined to be S-configuration, and C9 and C11 were determined to be R-configuration (as shown in FIG. 2). That is, the structure of compound **303-P4** was

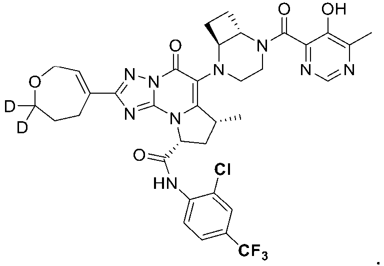

The crystallographic data for the monohydrate of 303-P4 are as follows:

| | |
|---|---|
| C₃₅H₃₅ClF₃N₉O₅·H₂O | *F*(000) = 1608 |
| *Mᵣ* = 772.18 | *D*ₓ = 1.421 Mg m⁻³ |
| Monoclinic, *P*2₁ | Cu *K*α radiation, λ = 1.54178 Å |
| *a* = 14.4250 (8) Å | Cell parameters from 2512 reflections |
| *b* = 19.2839 (10) Å | θ = 4.1-60.8° |
| *c* = 14.1565 (9) Å | µ = 1.58 mm⁻¹ |
| β = 113.585 (4)° | *T =* 170 K |
| *V =* 3609.0 (4) Å³ | Block, colourless |
| *Z* = 4 | 0.09 × 0.06 × 0.04 mm |

### Example 295

### 6-((1S,6S)-5-(5-Hydroxy-6-methylpyrimidine-4-carbonyl)-2,5-diazabicyclo[4.2.0loctan-2-yl)-7-methyl-N-(3-methyl-4-(trifluoromethyl)thiophen-2-yl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide

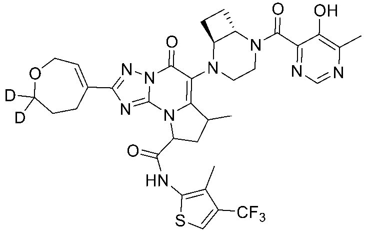

**The compound may also be synthesized according to the following steps:**

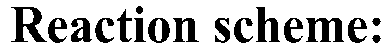

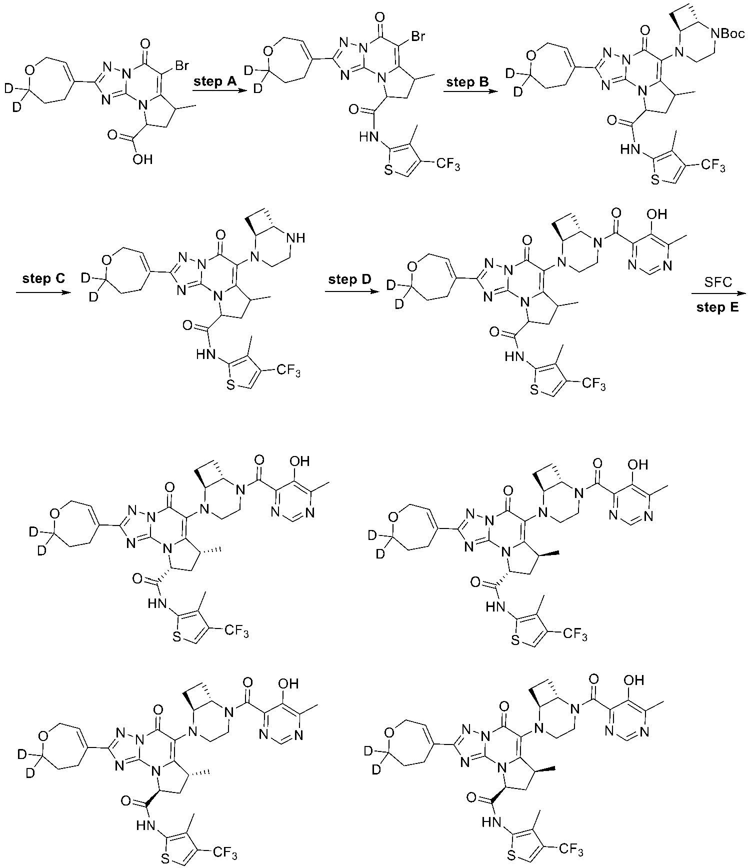

### Procedure:

**Step A:** 3-Methyl-4-trifluoromethylthiophen-2-amine (661 mg, 3.65 mmol) and 6-bromo-7-methyl-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxylic acid (1.5 g, 3.65 mmol) were dissolved in acetonitrile (20 mL), and then TCFH (3.1 g, 10.9 mmol) and *N-*methylimidazole (1.5 g, 18.2 mmol) were added. The reaction mixture was stirred at 25°C for 1 hour.

After LCMS monitoring indicated the disappearance of the starting material, water was added to the reaction mixture, and the mixture was extracted with dichloromethane (300 mL × 3). The combined organic phases were washed with saturated brine (100 mL × 3), dried over anhydrous sodium sulfate, filtered, and concentrated under reduced pressure. The resulting mixture was purified by silica gel column chromatography to obtain 6-bromo-7-methyl-*N*-(3-methyl-4-(trifluoromethyl)thiophen-2-yl)-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxamide (1.0 g).

MS (ESI) M/Z: 574.5 [M+H] ⁺.

**Step B:** 6-Bromo-7-methyl-*N*-(3-methyl-4-(trifluoromethyl)thiophen-2-yl)-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxamide (1.0 g, 1.7 mmol) and *tert-*butyl (1S,6S)-2,5-diazabicyclo[4.2.0]octane-2-carboxylate (1.85 g, 8.5 mmol) were dissolved in DMSO (5 mL). The reaction mixture was stirred at 120°C for 1 hour.

After LCMS monitoring indicated the disappearance of the starting material, the resulting mixture was purified by C18 silica gel column chromatography to obtain *tert-*butyl (1S,6S)-5-(7-methyl-9-((3-methyl-4-(trifluoromethyl)thiophen-2-yl)carbamoyl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidin-6-yl)-2,5-diazabicyclo[4.2.0]octane-2-carboxylate (400 mg).

MS (ESI) M/Z: 650.7 [M-56+H] ⁺.

**Step C:** *tert*-Butyl (1S,6S)-5-(7-methyl-9-((3-methyl-4-(trifluoromethyl)thiophen-2-yl)carbamoyl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidin-6-yl)-2,5-diazabicyclo[4.2.0]octane-2-carboxylate (400 mg) was dissolved in dichloromethane (5 mL), and then trifluoroacetic acid (2 mL) was added. The reaction mixture was stirred at room temperature for 1 hour.

After LCMS monitoring indicated the disappearance of the starting material, the reaction mixture was diluted with dichloromethane, and the resulting mixture was concentrated under reduced pressure to obtain 6-((1S,6S)-2,5-diazabicyclo[4.2.0]octan-2-yl)-7-methyl-*N-*(3-methyl-4-(trifluoromethyl)thiophen-2-yl)-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxamide (320 mg, crude). The crude product was used directly in the next step without further purification.

MS (ESI) M/Z: 606.4 [M+H] ⁺.

**Step D:** Compound 5-hydroxy-6-methylpyrimidine-4-carboxylic acid (84 mg, 544.9 µmol) was dissolved in DMF (3 mL). EDCI (142 mg, 743 µmol) and HOAT (94 mg, 693.4 µmol) were added portionwise at 0°C. The reaction mixture was stirred at 0°C for 30 minutes for later use. 6-((1S,6S)-2,5-Diazabicyclo[4.2.0]octan-2-yl)-7-methyl-*N*-(3-methyl-4-(trifluoromethyl)thiophen-2-yl)-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxamide (320 mg, crude product) was dissolved in DMF (3 mL), and DIEA was added to adjust the pH to basic. Then, previously prepared solution was slowly added dropwise to the reaction system. After the dropwise addition was completed, the reaction mixture was stirred at 0°C for 20 minutes.

After LCMS monitoring indicated the disappearance of the starting material, the reaction mixture was directly purified by reverse-phase C18 column chromatography to obtain compound 6-((1*S*,6*S*)-5-(5-hydroxy-6-methylpyrimidine-4-carbonyl)-2,5-diazabicyclo[4.2.0]octan-2-yl)-7-methyl-*N*-(3-methyl-4-(trifluoromethyl)thiophen-2-yl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (220 mg).

MS (ESI) M/Z: 742.7 [M+H] ⁺.

**Step E:** 6-((1*S*,6*S*)-5-(5-Hydroxy-6-methylpyrimidine-4-carbonyl)-2,5-diazabicyclo[4.2.0]octan-2-yl)-7-methyl-*N*-(3-methyl-4-(trifluoromethyl)thiophen-2-yl)-5-oxo-2-(2,5,6,7-tetrahydrooxepin-4-yl-7,7-d₂)-5,7,8,9-tetrahydropyrrolo[1,2-*c*][1,2,4]triazolo[1,5-*a*]pyrimidine-9-carboxamide (220 mg, 0.3 mmol) was separated by preparative SFC under the following separation conditions: preparative column: DAICEL CHIRALPAK^{®} OD-10, 25 * 250 mm, 10 m; mobile phase: supercritical CO₂/ MeOH + (0.05%) *N*H₃·MeOH; flow rate: 90 mL/min; gradient: 50:50; detection wavelength: 214 nm/254 nm. The product was collected and lyophilized under reduced pressure to obtain mixture 1 (140 mg, mixture with retention times of 4.36 min, 4.47 min, and 4.89 min) and single-configuration product 295-P4 (26.36 mg, retention time: 8.49 min).

Mixture 1 (140 mg) was separated by preparative SFC under the following separation conditions: preparative column: DAICEL CHIRALPAK^{®}OD-10, 25*250 mm 10 µm; mobile phase: supercritical CO₂/ MeOH+(0.05%) *N*H₃·MeOH; flow rate: 80 mL/min; gradient: 70:30; detection wavelength: 214 nm/254 nm. The product was collected and lyophilized under reduced pressure to obtain mixture 2 (90 mg, mixture with retention times of 4.37 min and 4.47 min) and single-configuration product 295-P3 (46.22 mg, retention time: 4.90 min).

Mixture 2 (90 mg) was separated by preparative SFC under the following separation conditions: preparative column: DAICEL CHIRALPAK^{®} OD-10, 25*250 mm, 10 µm; mobile phase: supercritical CO₂/ IPA + (0.05%) NH₃·MeOH; flow rate: 80 mL/min; gradient: 70:30; detection wavelength: 214 nm/254 nm. The product was collected and lyophilized under reduced pressure to obtain single-configuration product 295-P1 (37.42 mg, retention time: 4.55 min) and single-configuration product 295-P2 (23.44 mg, retention time: 4.85 min).

### 295-P1:

MS (ESI) M/Z: 742.4 [M+H] ⁺

¹H NMR (400 MHz, DMSO-d6) δ 11.08 (s, 1H), 10.45 (s, 1H), 8.62 (s, 1H), 7.81 (s, 1H), 6.86 (t, *J =* 4.6 Hz, 1H), 5.65 - 5.51 (m, 1H), 4.59 - 4.40 (m, 1H), 4.29 (d, *J* = 4.7 Hz, 2H), 3.77 - 3.68 (m, 1H), 3.60 - 3.51 (m, 4H), 3.37 (m, 1H), 2.92 - 2.85 (m, 2H), 2.66 - 2.58 (m, 1H), 2.49 (s, 3H), 2.45 - 2.40 (m, 1H), 2.30 (s, 3H), 1.95 - 1.85 (m, 2H), 1.74 - 1.68 (m, 1H), 1.58 (d, *J =* 6.9 Hz, 3H), 1.52 - 1.38 (m, 2H), 1.28 - 1.19 (m, 1H).

### 295-P2:

MS (ESI) M/Z: 742.4 [M+H] ⁺.

¹H NMR (400 MHz, DMSO-d₆) δ 11.02 (s, 1H), 10.23 (s, 1H), 8.55 (s, 1H), 7.75 (s, 1H), 6.82 (t, *J =* 4.5 Hz, 1H), 5.54 - 5.39 (m, 1H), 4.44 - 4.34 (m, 1H), 4.24 (d, *J =* 4.5 Hz, 2H), 4.16 - 3.39 (m, 3H), 3.26 - 3.14 (m, 2H), 3.03 - 2.92 (m, 1H), 2.88 - 2.80 (m, 2H), 2.54 - 2.52 (m, 1H), 2.44 (s, 3H), 2.26 (s, 3H), 2.12 - 2.04 (m, 1H), 1.90 - 1.81 (m, 2H), 1.76 - 1.65 (m, 1H), 1.51 - 1.31 (m, 5H), 1.23 - 1.14 (m, 1H).

### 295-P3:

MS (ESI) M/Z: 742.4 [M+H] ⁺.

¹H NMR (400 MHz, DMSO-*d*₆) δ 11.32 - 10.04 (m, 2H), 8.54 (s, 1H), 7.76 (s, 1H), 6.80 (t, *J =* 4.6 Hz, 1H), 5.53 (dd, *J =* 8.5, 4.7 Hz, 1H), 4.43 - 4.32 (m, 1H), 4.24 (d, *J =* 4.6 Hz, 2H), 4.15 - 3.42 (m, 3H), 3.41 - 3.35 (m, 1H), 3.26 - 3.12 (m, 2H), 2.85 - 2.79 (m, 2H), 2.60 - 2.53 (m, 1H), 2.43 (s, 3H), 2.41 - 2.36 (m, 1H), 2.24 (s, 3H), 1.87 - 1.81 (m, 2H), 1.69 - 1.61 (m, 1H), 1.55 (d, *J =* 6.8 Hz, 3H), 1.48 - 1.32 (m, 2H), 1.23 - 1.09 (m, 1H).

### 295-P4:

MS (ESI) M/Z: 742.3 [M+H] ⁺.

¹H NMR (400 MHz, DMSO-*d*₆) δ 11.45 - 10.03 (m, 2H), 8.61 (s, 1H), 7.83 (s, 1H), 6.88 (t, *J* = 4.6 Hz, 1H), 5.65 - 5.44 (m, 1H), 4.57 - 4.37 (m, 1H), 4.30 (d, *J =* 4.5 Hz, 2H), 3.83 - 3.74 (m, 1H), 3.67 - 3.43 (m, 4H), 3.12 - 2.97 (m, 1H), 2.93 - 2.82 (m, 2H), 2.61 - 2.58 (m, 1H), 2.49 (s, 3H), 2.32 (s, 3H), 2.15 - 2.09 (m, 1H), 1.94 - 1.87 (m, 2H), 1.73 - 1.65 (m, 1H), 1.54 - 1.38 (m, 5H), 1.27 - 1.16 (m, 1H).

Approximately 30.0 mg of compound 295-P4 was weighed, dissolved in methanol (3.0 mL, AR), sonicated at room temperature for 2 minutes, then 1 mL of dimethyl sulfoxide was added, and the mixture was allowed to stand at 45°C for 5 days to obtain block-shaped yellow crystals.

### X-ray single-crystal diffractometer parameters:

| | | | |
|---|---|---|---|
| Source: Cu | target | X-ray: | Cu-K (= 1.54178 Å) |
| Detector: | CMOS area detector | Resolution: | 0.86 Å |
| Current and voltage: | 50 kV, 1.2 mA | Exposure time: | 3 s |
| Distance from area detector to sample: | 40 mm | Test temperature: | 170(2) K |

After the diffraction data were integrated and reduced using the SAINT program, empirical absorption correction was applied to the data using the SADABS program. The single-crystal structure was solved by direct methods using SHELXT2014, and the structure was refined by the least-squares method. Hydrogen atoms were treated isotropically during refinement. Hydrogen atoms on C-H were placed in calculated positions and refined using a riding model. The Flack constant was 0.061 (14), allowing the absolute configuration to be determined. In the structure, the configurations of C25 and C26 were determined to be S-configuration, and C8 and C10 were determined to be R-configuration (as shown in FIG. 1). That is, the structure of compound **295-P4** was

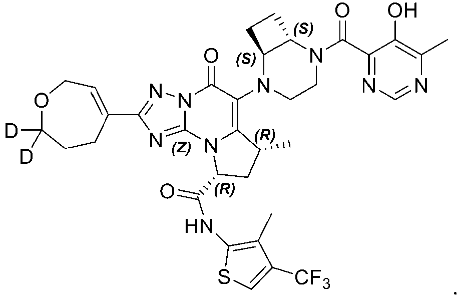

The crystallographic data for the DMSO solvate of 295-P4 are as follows:

| | |
|---|---|
| C₃₄H₃₄D₂F₃N₉O₅S·C₂H₆OS | *D*ₓ = 1.399 Mg m⁻³ |
| *Mᵣ* = 819.92 | Cu *K*α radiation, λ = 1.54178 Å |
| Orthorhombic, *P*2₁2₁2₁ | Cell parameters from 9935 reflections |
| *a* = 11.3002 (3) Å | θ = 3.5-63.8° |
| *b* = 15.9609 (4) Å | µ = 1.86 mm⁻¹ |
| *c* = 21.5818 (6) Å | *T =* 150 K |
| *V* = 3892.52 (18) Å³ | Block, colourless |
| *Z =* 4 | 0.15 × 0.06 × 0.05 mm |
| *F*(000) = 1712 | |

.

### Example 306

### 6-((R)-4-(5-Hydroxy-6-methylpyrimidine-4-carbonyl)-3-methylpiperazin-1-yl)-7-methyl-N-(3-methyl-4-(trifluoromethyl)thiophen-2-yl)-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxamide

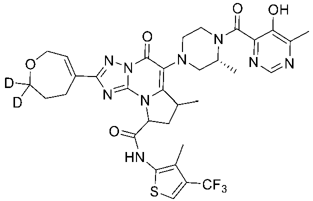

**The compound may also be synthesized according to the following steps:**

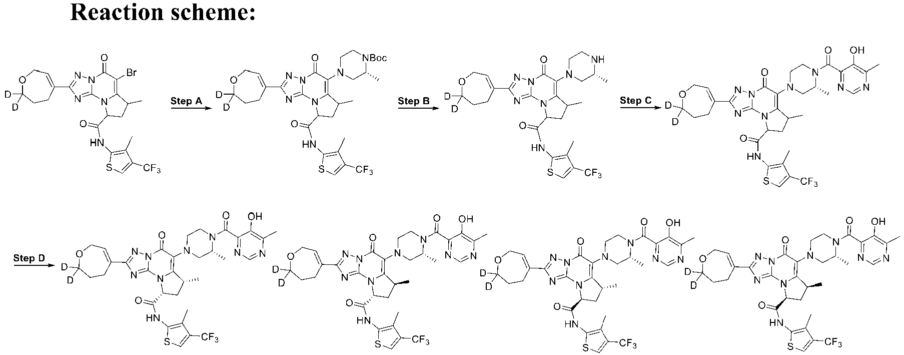

### Procedure:

**Step A:** In a microwave tube, 6-bromo-7-methyl-*N*-(3-methyl-4-(trifluoromethyl)thiophen-2-yl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (500 mg, 870.46 µmol) and (R)-*tert*-butyl 2-methylpiperazine-1-carboxylate (1 g, 4.99 mmol) were dissolved in DMF (5 mL). Then, *N,N*-diisopropylethylamine (225 mg, 1.7 mmol) was added, and the reaction mixture was stirred at 110°C for 3 hours.

LCMS monitoring indicated the disappearance of the starting material, and the reaction mixture was directly purified by reverse-phase C18 column chromatography to obtain compound *tert-*butyl (2R)-2-methyl-4-(7-methyl-9-((3-methyl-4-(trifluoromethyl)thiophen-2-yl)carbamoyl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidin-6-yl)piperazine-1-carboxylate (180 mg).

MS (ESI) M/Z: 638.6 [M-56+H] ⁺

**Step B:** *tert*-Butyl (2R)-2-methyl-4-(7-methyl-9-((3-methyl-4-(trifluoromethyl)thiophen-2-yl)carbamoyl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidin-6-yl)piperazine-1-carboxylate (180 mg, 259.45 µmol) was dissolved in dichloromethane (3 mL), and then trifluoroacetic acid (1 mL) was added to the above system. The reaction mixture was stirred at room temperature for 1 hour.

After LCMS monitoring indicated the disappearance of the starting material, the resulting mixture was diluted with dichloromethane and then concentrated under reduced pressure to obtain 7-methyl-*N*-(3-methyl-4-(trifluoromethyl)thiophen-2-yl)-6-((R)-3-methylpiperazin-1-yl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (150 mg, crude product). The crude product was used directly in the next step without further purification.

MS (ESI) M/Z: 594.6 [M+H] ⁺.

**Step C:** Compound 5-hydroxy-6-methylpyrimidine-4-carboxylic acid (41 mg, 266 µmol) was dissolved in DMF (2 mL). EDCI (58 mg, 303.20 µmol) and HOAT (41 mg, 303.20 µmol) were added at 0°C. The reaction mixture was stirred at 0°C for 30 minutes for later use. 7-Methyl-*N*-(3-methyl-4-(trifluoromethyl)thiophen-2-yl)-6-((R)-3-methylpiperazin-1-yl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (150 mg, crude product) was dissolved in DMF (2 mL), and then DIEA was added at 0°C until the system became basic. The previously prepared solution was added dropwise to the reaction system. After the dropwise addition was completed, the mixture was stirred at 0°C for 10 minutes.

After LCMS monitoring indicated the disappearance of the starting material, the reaction mixture was directly purified by reverse-phase C18 column chromatography to obtain 6-((R)-4-(5-hydroxy-6-methylpyrimidine-4-carbonyl)-3-methylpiperazin-1-yl)-7-methyl-*N-*(3-methyl-4-(trifluoromethyl)thiophen-2-yl)-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxamide (150 mg).

MS (ESI) M/Z: 730.4 [M+H] ⁺.

**Step D:** 6-((R)-4-(5-Hydroxy-6-methylpyrimidine-4-carbonyl)-3-methylpiperazin-1-yl)-7-methyl-*N*-(3-methyl-4-(trifluoromethyl)thiophen-2-yl)-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxamide (150 mg, 205.54 µmol) was separated by preparative SFC under the following separation conditions: preparative column: DAICEL CHIRALPAK^{®} OD-10, 25 * 250 mm, 10 m; mobile phase: supercritical CO₂/ MeOH + (0.05%) *N*H₃·MeOH; flow rate: 90 mL/min; gradient: 50:50; detection wavelength: 214 nm/254 nm. The product was collected and lyophilized under reduced pressure to obtain mixture 1 (50 mg, mixture with retention times of 4.04 min and 4.36 min), single-configuration product **306-P3** (21.37 mg, retention time: 5.59 min), and single-configuration product **306-P4** (20.38 mg, retention time: 9.66 min).

Mixture 1 (50 mg) was separated by preparative SFC under the following separation conditions: preparative column: DAICEL CHIRALPAK^{®}AS-10, 25*250 mm 10 m; mobile phase: supercritical CO₂/ MeOH+(0.05%) NH₃·M_{E}OH; flow rate: 60 mL/min; gradient: 70:30; detection wavelength: 214 nm/254 nm; the product was collected and lyophilized under reduced pressure to obtain single-configuration product **306-P1** (23.91 mg, retention time: 3.16 min) and single-configuration product **306-P2**(11.97 mg, retention time: 3.63 min).

### 306-P1

MS (ESI) M/Z: 730.4 [M+H] ⁺.

¹H NMR (400 MHz, DMSO-d₆) δ 11.00 (s, 1H), 10.15 (s, 1H), 8.58 (s, 1H), 7.78 (s, 1H), 6.80 (t, *J =* 4.7 Hz, 1H), 5.59 - 5.51 (m, 1H), 4.83 - 4.36 (m, 1H), 4.28 - 4.19 (m, 2H), 3.73 - 3.53 (m, 3H), 3.45 - 3.33 (m, 1H), 3.22 - 3.10 (m, 1H), 2.86 - 2.80 (m, 2H), 2.78 - 2.64 (m, 1H), 2.61 - 2.52 (m, 2H), 2.45 (s, 3H), 2.42 - 2.36 (m, 1H), 2.25 (s, 3H), 1.88 - 1.79 (m, 2H), 1.58 - 1.46 (m, 3H), 1.44 - 1.35 (m, 3H).

### 306-P2

MS (ESI) M/Z: 730.4 [M+H] ⁺.

¹H NMR (400 MHz, DMSO-d₆) δ 11.03 (s, 1H), 10.17 (s, 1H), 8.57 (s, 1H), 7.76 (s, 1H), 6.83 (t, *J =* 4.5 Hz, 1H), 5.56 - 5.43 (m, 1H), 4.82 - 4.37 (m, 1H), 4.31 - 4.18 (m, 2H), 3.87 - 3.65 (m, 3H), 3.49 - 3.36 (m, 1H), 3.28 - 3.18 (m, 1H), 3.01 - 2.91 (m, 1H), 2.87 - 2.82 (m, 2H), 2.54 - 2.52 (m, 2H), 2.45 (s, 3H), 2.26 (s, 3H), 2.16 - 2.08 (m, 1H), 1.90 - 1.82 (m, 2H), 1.44 - 1.32 (m, 6H).

### 306-P3

MS (ESI) M/Z: 730.4 [M+H] ⁺.

¹H NMR (400 MHz, DMSO-d₆) δ 10.98 (s, 1H), 10.17 (s, 1H), 8.58 (s, 1H), 7.80 (s, 1H), 6.77 (t, *J =* 4.6 Hz, 1H), 5.60 - 5.52 (m, 1H), 4.82 - 4.35 (m, 1H), 4.26 - 4.16 (m, 2H), 3.95 - 3.66 (m, 3H), 3.51 - 3.37 (m, 1H), 3.29 - 3.19 (m, 1H), 2.98 - 2.87 (m, 1H), 2.86 - 2.78 (m, 2H), 2.62 - 2.52 (m, 2H), 2.45 (s, 3H), 2.41 - 2.36 (m, 1H), 2.25 (s, 3H), 1.88 - 1.79 (m, 2H), 1.49 - 1.36 (m, 6H).

### 306-P4

MS (ESI) M/Z: 730.4 [M+H] ⁺.

¹H NMR (400 MHz, DMSO-d₆) δ 11.04 (s, 1H), 10.15 (s, 1H), 8.58 (s, 1H), 7.78 (s, 1H), 6.83 (t, *J =* 4.6 Hz, 1H), 5.55 - 5.45 (m, 1H), 4.83 - 4.37 (m, 1H), 4.30 - 4.20 (m, 2H), 3.70 - 3.51 (m, 3H), 3.45 - 3.33 (m, 1H), 3.24 - 3.10 (m, 1H), 3.07 - 2.93 (m, 1H), 2.88 - 2.80 (m, 2H), 2.58 - 2.53 (m, 2H), 2.45 (s, 3H), 2.27 (s, 3H), 2.14 - 2.07 (m, 1H), 1.89 - 1.80 (m, 2H), 1.48 - 1.34 (m, 6H).

### Example 307

### 6-(4-(5-Hydroxy-6-methylpyrimidine-4-carbonyl)piperazin-1-yl)-7-methyl-N-(3-methyl-4-(trifluoromethyl)thiophen-2-yl)-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxamide

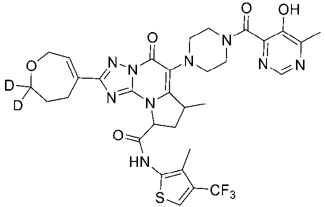

**The compound may also be synthesized according to the following steps:**

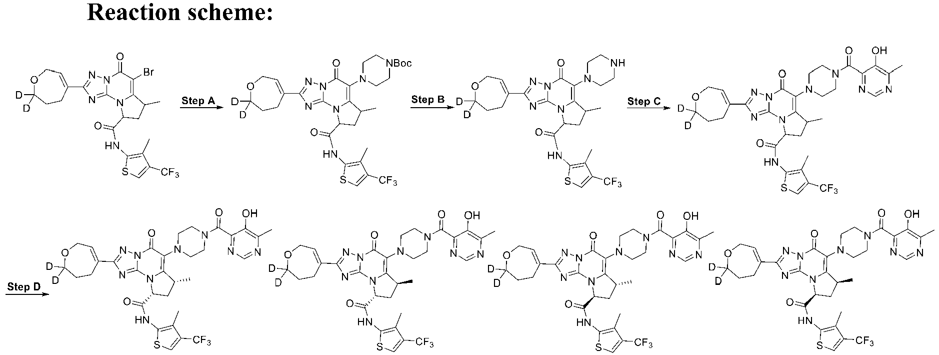

### Procedure:

**Step A:** In a microwave tube, 6-bromo-7-methyl-N-(3-methyl-4-(trifluoromethyl)thiophen-2-yl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (500 mg, 870.5 µmol) and *tert-*butyl piperazine-1-carboxylate (930 mg, 5.0 mmol) were dissolved in ultra-dry DMF (5 mL), and then N,N-diisopropylethylamine (225 mg, 1.7 mmol) was added. The reaction mixture was stirred at 110°C for 3 hours.

After LCMS monitoring indicated the disappearance of the starting material and the formation of the product, the reaction mixture was directly purified by reverse-phase C18 column chromatography to obtain *tert-*butyl 4-(7-methyl-9-((3-methyl-4-(trifluoromethyl)thiophen-2-yl)carbamoyl)-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidin-6-yl)piperazine-1-carboxylate (240 mg).

MS (ESI) M/Z: 580.7 [M-100+H] ⁺.

**Step B:** *tert*-Butyl 4-(7-methyl-9-((3-methyl-4-(trifluoromethyl)thiophen-2-yl)carbamoyl)-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidin-6-yl)piperazine-1-carboxylate (240 mg, 353.1 µmol) was dissolved in dichloromethane (3 mL), and then trifluoroacetic acid (1 mL) was added to the above system. The reaction mixture was stirred at room temperature for 1 hour.

After LCMS monitoring indicated the disappearance of the starting material, the resulting mixture was diluted with dichloromethane and then concentrated under reduced pressure to obtain 7-methyl-*N*-(3-methyl-4-(trifluoromethyl)thiophen-2-yl)-5-oxo-6-(piperazin-1-yl)-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (204 mg). The crude product was used directly in the next step without further purification.

MS (ESI) M/Z: 580.6 [M+H] ⁺.

**Step C:** Compound 5-hydroxy-6-methylpyrimidine-4-carboxylic acid (65 mg, 422.3 µmol) was dissolved in DMF (2 mL). EDCI (81 mg, 422.3 µmol) and HOAT (58 mg, 422.3 µmol) were added at 0°C. The reaction mixture was stirred at 0°C for 30 minutes for later use. 7-Methyl-*N*-(3-methyl-4-(trifluoromethyl)thiophen-2-yl)-5-oxo-6-(piperazin-1-yl)-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (204 mg, crude product) was dissolved in DMF (2 mL), and then DIEA was added at 0°C until the system became basic. The previously prepared solution was added dropwise to the reaction system. After the dropwise addition was completed, the mixture was stirred at 0°C for 10 minutes.

After LCMS monitoring indicated the disappearance of the starting material, the reaction mixture was directly purified by reverse-phase C18 column chromatography to obtain 6-(4-(5-hydroxy-6-methylpyrimidine-4-carbonyl)piperazin-1-yl)-7-methyl-*N*-(3-methyl-4-(trifluoromethyl)thiophen-2-yl)-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxamide (150 mg).

MS (ESI) M/Z: 716.3 [M+H] ⁺.

**Step D:** 6-(4-(5-Hydroxy-6-methylpyrimidine-4-carbonyl)piperazin-1-yl)-7-methyl-*N*-(3-methyl-4-(trifluoromethyl)thiophen-2-yl)-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxamide (150 mg) was separated by preparative SFC under the following separation conditions: preparative column: DAICEL CHIRALPAK^{®}OD-10, 25*250 mm 10 µm; mobile phase: supercritical CO₂/ MeOH(80%) + ACN(20%) + (0.05%)NH₃·MeOH; flow rate: 80 mL/min; gradient: 50:50; detection wavelength: 214 nm/254 nm. The product was collected and lyophilized under reduced pressure to obtain mixture 1 (50 mg, mixture with retention times of 1.04 min and 1.14 min), single-configuration product **307-P3** (22.08 mg, retention time: 3.07 min), and single-configuration product **307-P4** (20.84 mg, retention time: 10.03 min).

Mixture 1 (50 mg) was separated by preparative SFC under the following separation conditions: preparative column: DAICEL CHIRALPAK^{®}AS-10, 25*250 mm 10 m; mobile phase: supercritical CO₂/ IPA+(0.05%) *N*H₃·MeOH; flow rate: 60 mL/min; gradient: 70:30; detection wavelength: 214 nm/254 nm. The product was collected and lyophilized under reduced pressure to obtain single-configuration product **307-P1** (11.06 mg, retention time: 3.75 min) and single-configuration product **307-P2** (16.08 mg, retention time: 4.10 min).

### 307-P1

MS (ESI) M/Z: 716.4 [M+H] ⁺.

¹H NMR (400 MHz, DMSO-d₆) δ 10.95 (s, 1H), 8.37 (s, 1H), 7.71 (s, 1H), 6.80 (t, *J* = 4.6 Hz, 1H), 5.52 (dd, *J =* 8.4, 4.8 Hz, 1H), 4.24 (d, *J =* 4.7 Hz, 2H), 3.73 - 3.66 (m, 2H), 2.83 (d, *J =* 8.8 Hz, 3H), 2.57 - 2.53 (m, 4H), 2.41 - 2.30 (m, 6H), 2.24 (s, 1H), 2.23 (s, 3H), 1.84 (dd, *J =* 6.9*,* 2.8 Hz, 2H), 1.50 (d, *J =* 6.9 Hz, 3H).

### 307-P2

MS (ESI) M/Z: 716.4 [M+H] ⁺.

¹H NMR (400 MHz, DMSO-d₆) δ 10.79 (s, 2H), 8.50 (s, 1H), 7.70 (s, 1H), 6.86 (t, *J* = 4.6 Hz, 1H), 5.56 - 5.38 (m, 1H), 4.73 - 4.37 (m, 1H), 4.30 - 4.27 (m, 2H), 3.78 - 3.65 (m, 2H), 3.04 - 2.92 (m, 2H), 2.91 - 2.84 (m, 3H), 2.61 - 2.56 (m, 4H), 2.45 (s, 3H), 2.27 (s, 3H), 2.15 - 2.08 (m, 1H), 1.92 - 1.85 (m, 2H), 1.42 (d, *J =* 7.2 Hz, 3H).

### 307-P3

MS (ESI) M/Z: 716.4 [M+H] ⁺.

¹H NMR (400 MHz, DMSO-d₆) δ 10.96 (s, 1H), 8.48 (s, 1H), 7.72 (s, 1H), 6.81 (s, 1H), 5.56 - 5.40 (m, 1H), 4.72 - 4.29 (m, 1H), 4.27 - 4.21 (m, 2H), 3.79 - 3.63 (m, 2H), 3.02 - 2.90 (m, 2H), 2.87 - 2.75 (m, 3H), 2.56 - 2.52 (m, 4H), 2.42 (s, 3H), 2.25 (s, 3H), 2.12 - 2.03 (m, 1H), 1.88 - 1.81 (m, 2H), 1.39 (d, *J =* 7.2 Hz, 3H).

### 307-P4

MS (ESI) M/Z: 716.4 [M+H] ⁺.

¹H NMR (400 MHz, DMSO-d₆) δ 11.36 - 10.11 (m, 2H), 8.53 (s, 1H), 7.74 (s, 1H), 6.80 (t, *J =* 4.6 Hz, 1H), 5.62 - 5.45 (m, 1H), 4.88 - 4.28 (m, 1H), 4.26 - 4.19 (m, 2H), 3.78 - 3.60 (m, 2H), 3.17 - 3.09 (m, 1H), 2.88 - 2.73 (m, 3H), 2.61 - 2.51 (m, 4H), 2.43 (s, 3H), 2.41 - 2.30 (m, 2H), 2.24 (s, 3H), 1.88 - 1.81 (m, 2H), 1.50 (d, *J =* 6.9 Hz, 3H).

### Example 296

### N-(2-Chloro-5-(trifluoromethyl)thiophen-3-yl)-6-((1S,6S)-5-(5-hydroxy-6-methylpyrimidine-4-carbonyl)-2,5-diazabicyclo[4.2.0]octan-2-yl)-7-methyl-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide

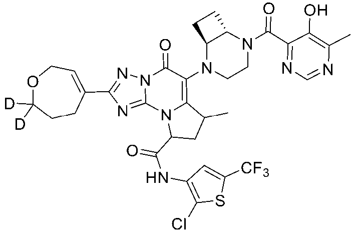

**The compound may also be synthesized according to the following steps:**

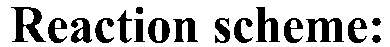

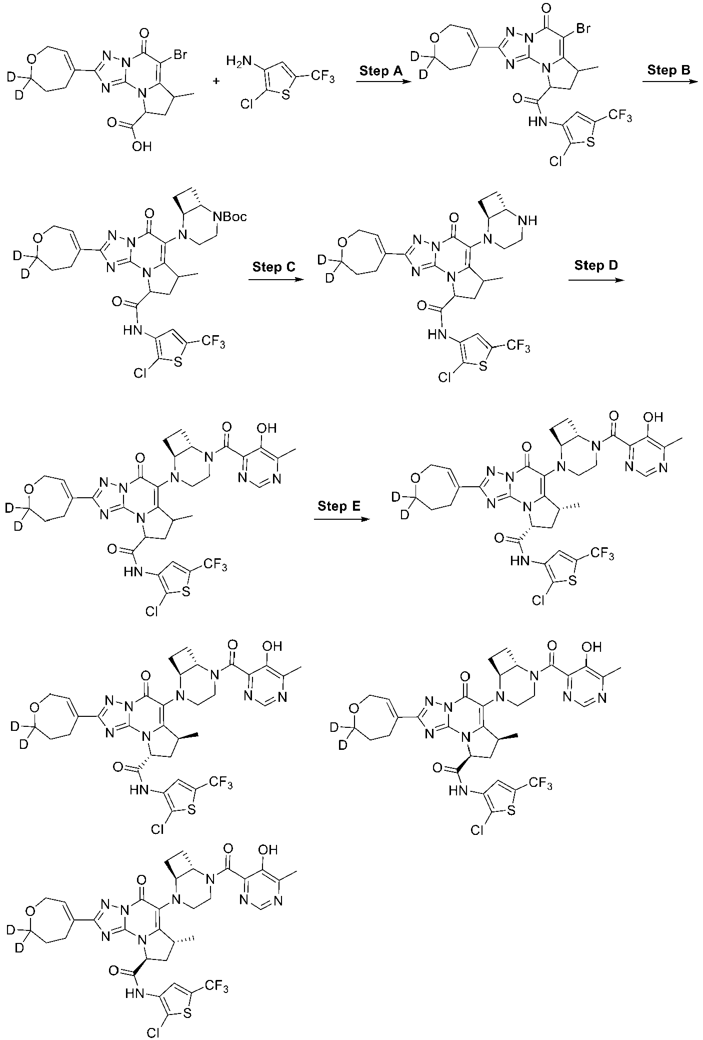

### Procedure:

**Step A:** 6-Bromo-7-methyl-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxylic acid (778 mg, 1.9 mmol) and 2-chloro-5-(trifluoromethyl)thiophen-3-amine hydrochloride (500 mg, 2.1 mmol) were dissolved in acetonitrile (20 mL). *N*-Methylimidazole (863 mg, 10.50 mmol) and *N,N,N',N'-*tetramethylchloroformamidinium hexafluorophosphate (1.8 g, 6.30 mmol) were added in an ice-water bath, and the reaction was carried out at room temperature for 16 hours.

After LCMS monitoring indicated the disappearance of the starting material, the reaction was quenched with water, extracted with ethyl acetate (100 mL × 3), and concentrated under reduced pressure. The resulting mixture was purified by column chromatography to obtain 6-bromo-*N*-(2-chloro-5-(trifluoromethyl)thiophen-3-yl)-7-methyl-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (180 mg).

MS (ESI) M/Z: 594.4 [M+H] ⁺.

**Step B:** 6-Bromo-*N*-(2-chloro-5-(trifluoromethyl)thiophen-3-yl)-7-methyl-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxamide (170 mg, 285.8 µmol) and *tert-*butyl (1S,6S)-2,5-diazabicyclo[4.2.0]octane-2-carboxylate (340 mg, 1.6 mmol) were dissolved in *N,N-*dimethylformamide (4 mL). *N,N*-Diisopropylethylamine (74 mg, 571.6 µmol) was added, and the mixture was stirred at 110°C for 2 hours.

LCMS monitoring indicated the disappearance of the starting material. The reaction mixture was subjected to column chromatography to obtain *tert-*butyl (1S,6S)-5-(9-((2-chloro-5-(trifluoromethyl)thiophen-3-yl)carbamoyl)-7-methyl-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidin-6-yl)-2,5-diazabicyclo[4.2.0]octane-2-carboxylate (90 mg).

MS (ESI) M/Z: 726.2 [M+H] ⁺.

**Step C:** *tert-*Butyl (1S,6S)-5-(9-((2-chloro-5-(trifluoromethyl)thiophen-3-yl)carbamoyl)-7-methyl-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidin-6-yl)-2,5-diazabicyclo[4.2.0]octane-2-carboxylate (140 mg, 192.8 µmol) was dissolved in dichloromethane (2 mL), and trifluoroacetic acid (1 mL) was added in an ice-water bath. The mixture was stirred at room temperature for 1 hour.

LCMS monitoring indicated the disappearance of the starting material. The reaction mixture was diluted with dichloromethane and then concentrated under reduced pressure to obtain the crude product 6-((1S,6S)-2,5-diazabicyclo[4.2.0]octan-2-yl)-*N*-(2-chloro-5-(trifluoromethyl)thiophen-3-yl)-7-methyl-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (110 mg).

MS (ESI) M/Z: 626.8 [M+H] ⁺.

**Step D:** 5-Hydroxy-6-methylpyrimidine-4-carboxylic acid (59 mg, 383.3 µmol), 1-(3-dimethylaminopropyl)-3-ethylcarbodiimide hydrochloride (77 mg, 402.5 µmol), and *N-*hydroxy-7-azabenzotriazole (55 mg, 402.5 µmol) were dissolved in *N,N*-dimethylformamide (2 mL) and stirred in an ice-water bath for 0.5 hours for later use. 6-((1S,6S)-2,5-Diazabicyclo[4.2.0]octan-2-yl)-*N*-(2-chloro-5-(trifluoromethyl)thiophen-3-yl)-7-methyl-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (120 mg, 191.7 µmol) was dissolved in *N*,*N-*dimethylformamide (2 mL), and *N,N*-diisopropylethylamine (124 mg, 958.3 µmol) was added to make the system basic. The previously prepared solution was added dropwise to the reaction mixture, and the mixture was stirred for ten minutes.

LCMS monitoring indicated the disappearance of the starting material. The reaction mixture was subjected to column chromatography to obtain *N*-(2-chloro-5-(trifluoromethyl)thiophen-3-yl)-6-((1S,6S)-5-(5-hydroxy-6-methylpyrimidine-4-carbonyl)-2,5-diazabicyclo[4.2.0]octan-2-yl)-7-methyl-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (110 mg).

MS (ESI) M/Z: 762.4 [M+H] ⁺.

**Step E:** *N*-(2-Chloro-5-(trifluoromethyl)thiophen-3-yl)-6-((1S,6S)-5-(5-hydroxy-6-methylpyrimidine-4-carbonyl)-2,5-diazabicyclo[4.2.0]octan-2-yl)-7-methyl-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (110 mg, 144.3 µmol) was separated by preparative SFC under the following separation conditions: preparative column: DAICEL CHIRALPAK^{®}OD-10, 25 * 250 mm 10 m; mobile phase: supercritical CO₂/ MeOH+(0.05%) *N*H₃·MeOH; flow rate: 70 mL/min; gradient: 50:50; detection wavelength: 214 nm/254 nm. The product was collected and lyophilized under reduced pressure to obtain mixture 1 (80 mg, mixture with retention times of 4.34 min, 4.44 min, and 4.59 min) and single-configuration product **296-P4** (17.18 mg, retention time: 6.58 min).

Mixture 1 (80 mg) was separated by preparative SFC under the following separation conditions: preparative column: DAICEL CHIRALPAK^{®} OD-10, 25*250 mm 10 m; mobile phase: supercritical CO₂/ IPA+(0.05%) *N*H₃·MeOH; flow rate: 60 mL/min; gradient: 60:40; detection wavelength: 214 nm/254 nm. The product was collected and lyophilized under reduced pressure to obtain mixture 2 (50 mg, retention time: 5.05 min), and single-configuration product **296-P1** (17.36 mg, retention time: 4.58 min)

Mixture 2 (50 mg) was separated by preparative SFC under the following separation conditions: preparative column: DAICEL CHIRALPAK^{®}AS-10, 25*250 mm 10 m; mobile phase: supercritical CO₂/ IPA+(0.05%) *N*H₃·MeOH; flow rate: 60 mL/min; gradient: 60:40; detection wavelength: 214 nm/254 nm. Retention times: 3.63 min and 4.27 min. The product was collected and lyophilized under reduced pressure to obtain single-configuration product **296-P2** (12.9 mg, retention time: 3.65 min) and single-configuration product **296-P3** (10.17 mg, retention time: 4.31 min).

### 296-P1

MS (ESI) M/Z: 762.4 [M+H] ⁺.

¹H NMR (400 MHz, DMSO-d₆) δ 10.74 (s, 1H), 8.44 (s, 1H), 7.92 (s, 1H), 6.84 (t, *J* = 4.6 Hz, 1H), 5.53 - 5.47 (m, 1H), 4.45 - 4.36 (m, 1H), 4.25 (d, *J =* 5.7 Hz, 2H), 3.70 - 3.63 (m, 1H), 3.54 - 3.39 (m, 4H), 3.28 - 3.24 (m, 1H), 2.86 - 2.81 (m, 2H), 2.62 - 2.55 (m, 1H), 2.39 (s, 3H), 2.37 - 2.33 (m, 1H), 1.88 - 1.82 (m, 2H), 1.67 - 1.59 (m, 1H), 1.52 (d, *J =* 6.8 Hz, 3H), 1.43 - 1.30 (m, 2H), 1.23 - 1.11 (m, 1H).

### 296-P2

MS (ESI) M/Z: 762.4 [M+H] ⁺.

¹H NMR (400 MHz, DMSO-d₆) δ 10.73 (s, 1H), 8.52 (s, 1H), 7.92 (s, 1H), 6.84 (t, *J* = 4.6 Hz, 1H), 5.54 - 5.47 (m, 1H), 4.42 - 4.33 (m, 1H), 4.28 - 4.22 (m, 2H), 4.15 - 3.44 (m, 3H), 3.28 - 3.03 (m, 3H), 2.86 - 2.79 (m, 2H), 2.62 - 2.54 (m, 1H), 2.43 (s, 3H), 2.41 - 2.35 (m, 1H), 1.87 - 1.81 (m, 2H), 1.69 - 1.60 (m, 1H), 1.55 (d, *J =* 6.8 Hz, 3H), 1.49 - 1.31 (m, 2H), 1.25 - 1.11 (m, 1H).

### 296-P3

MS (ESI) M/Z: 762.4 [M+H] ⁺.

¹H NMR (400 MHz, DMSO-d₆) δ 10.77 (s, 1H), 8.23 (s, 1H), 7.95 (s, 1H), 6.84 (t, *J* = 4.5 Hz, 1H), 5.52 - 5.30 (m, 1H), 4.50 - 4.31 (m, 1H), 4.30 - 4.20 (m, 2H), 4.11 - 3.53 (m, 3H), 3.21 - 2.91 (m, 3H), 2.88 - 2.78 (m, 2H), 2.55 - 2.52 (m, 1H), 2.33 (s, 3H), 2.15 - 2.05 (m, 1H), 1.89 - 1.80 (m, 2H), 1.74 - 1.61 (m, 1H), 1.54 - 1.32 (m, 5H), 1.24 - 1.14 (m, 1H).

### 296-P4

MS (ESI) M/Z: 762.4 [M+H] ⁺.

¹H NMR (400 MHz, DMSO-*d*₆) δ 10.74 (s, 1H), 10.37 (s, 1H), 8.58 (s, 1H), 7.93 (s, 1H), 6.85 (t, *J =* 4.6 Hz, 1H), 5.48 - 5.40 (m, 1H), 4.47 - 4.37 (m, 1H), 4.29 - 4.21 (m, 2H), 3.76 - 3.68 (m, 1H), 3.57 - 3.36 (m, 4H), 3.05 - 2.90 (m, 1H), 2.88 - 2.77 (m, 2H), 2.54 - 2.53 (m, 1H), 2.43 (s, 3H), 2.11 - 2.05 (m, 1H), 1.88 - 1.83 (m, 2H), 1.67 - 1.59 (m, 1H), 1.51 - 1.30 (m, 5H), 1.23 - 1.10 (m, 1H).

### Example 322

### 6-((1S,6S)-5-(5-Hydroxy-6-methylpyrimidine-4-carbonyl)-2,5-diazabicyclo[4.2.0loctan-2-yl)-7-methyl-N-(4-methyl-5-(trifluoromethyl)thiophen-3-yl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide

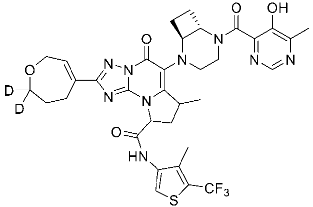

**The compound may also be synthesized according to the following steps:**

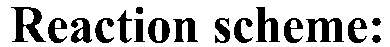

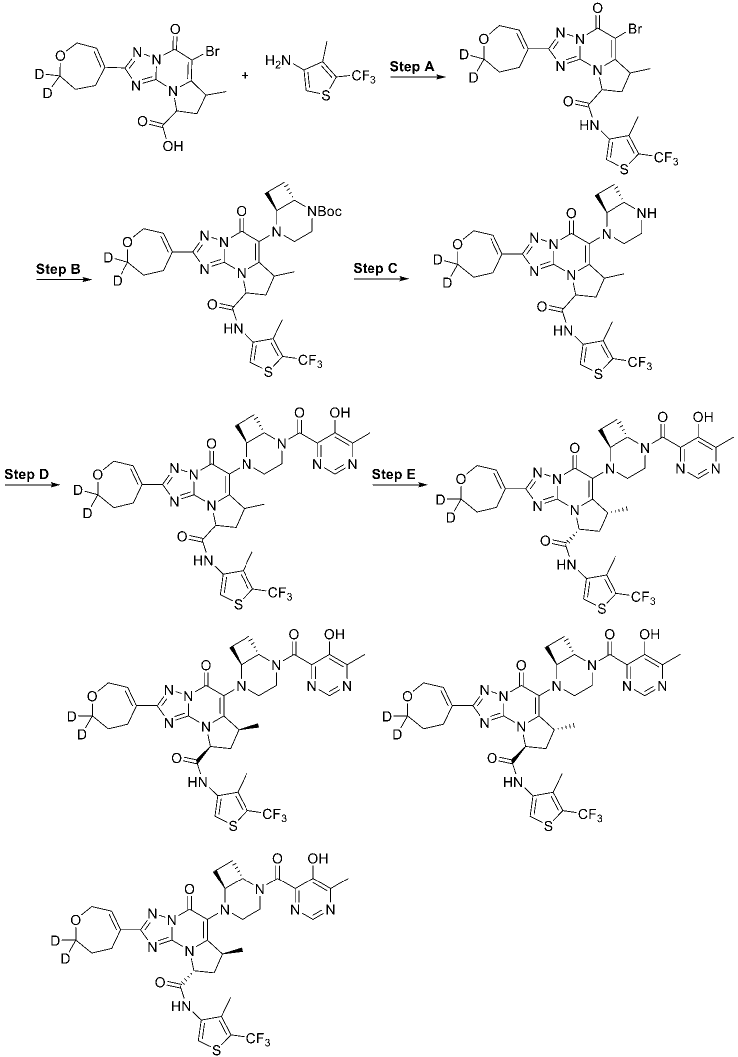

### Procedure:

**Step A:** 4-Methyl-5-trifluoromethylthiophen-3-amine (264 mg, 1.5 mmol) and 6-bromo-7-methyl-5-oxo-2-(2,5,6,7-tetrahydrooxepin-4-yl-7,7-D₂)-5,7,8,9-tetrahydropyrrolo[1,2-c][1,2,4]triazolo[1,5-a]pyrimidine-9-carboxylic acid (500 mg, 1.2 mmol) were dissolved in acetonitrile (10 mL), followed by the addition of TCFH (1.0 g, 3.7 mmol) and NMI (500 mg, 6.1 mmol). The reaction mixture was stirred at 25°C for 1 hour.

After LCMS monitoring indicated the disappearance of the starting material, water was added to the reaction mixture, and the mixture was extracted with dichloromethane (100 mL × 3). The combined organic phases were washed with saturated brine (50 mL × 3), dried over anhydrous sodium sulfate, filtered, and concentrated under reduced pressure. The resulting mixture was purified by silica gel column chromatography to obtain 6-bromo-7-methyl-*N*-(4-methyl-5-(trifluoromethyl)thiophen-3-yl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (320 mg).

MS (ESI) M/Z: 574.7 [M+H] ⁺.

**Step B:** 6-Bromo-7-methyl-*N*-(4-methyl-5-(trifluoromethyl)thiophen-3-yl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (320 mg, 0.6 mmol), *tert-*butyl (1S,6S)-2,5-diazabicyclo[4.2.0]octane-2-carboxylate (590 mg, 2.8 mmol), and DIEA (140 mg, 1.1 mmol) were dissolved in DMF (5 mL). The reaction mixture was stirred at 110°C for 1 hour.

After LCMS monitoring indicated the disappearance of the starting material, the resulting mixture was purified by C18 silica gel column chromatography to obtain *tert-*butyl (1S,6S)-5-(7-methyl-9-((4-methyl-5-(trifluoromethyl)thiophen-3-yl)carbamoyl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidin-6-yl)-2,5-diazabicyclo[4.2.0]octane-2-carboxylate (140 mg).

MS (ESI) M/Z: 650.9 [M-56+H] ⁺.

**Step C:** *tert-*Butyl (1S,6S)-5-(7-methyl-9-((4-methyl-5-(trifluoromethyl)thiophen-3-yl)carbamoyl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidin-6-yl)-2,5-diazabicyclo[4.2.0]octane-2-carboxylate (140 mg, 0.20 mmol) was dissolved in dichloromethane (5 mL), and then trifluoroacetic acid (2 mL) was added. The reaction mixture was stirred at room temperature for 1 hour.

After LCMS monitoring indicated the disappearance of the starting material, the reaction mixture was diluted with dichloromethane, and the resulting mixture was concentrated under reduced pressure to obtain 6-((1S,6S)-2,5-diazabicyclo[4.2.0]octan-2-yl)-7-methyl-*N-*(4-methyl-5-(trifluoromethyl)thiophen-3-yl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (120 mg). The crude product was used directly in the next step without further purification.

MS (ESI) M/Z: 606.9 [M+H] ⁺.

**Step D:** Compound 5-hydroxy-6-methylpyrimidine-4-carboxylic acid (37 mg, 238.0 µmol) was dissolved in DMF (3 mL). EDCI (57 mg, 297.0 µmol) and HOAT (40 mg, 297.0 µmol) were added portionwise at 0°C. The reaction mixture was stirred at 0°C for 30 minutes for later use. 6-((1S,6S)-2,5-Diazabicyclo[4.2.0]octan-2-yl)-7-methyl-*N*-(4-methyl-5-(trifluoromethyl)thiophen-3-yl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (120 mg, crude product) was dissolved in DMF (3 mL), and DIEA was added to adjust the pH to basic. Then, previously prepared solution was slowly added dropwise to the reaction system. After the dropwise addition was completed, the reaction mixture was stirred at 0°C for 20 minutes.

After LCMS monitoring indicated the disappearance of the starting material, the reaction mixture was directly purified by reverse-phase C18 column chromatography to obtain compound 6-((1S,6S)-5-(5-hydroxy-6-methylpyrimidine-4-carbonyl)-2,5-diazabicyclo[4.2.0]octan-2-yl)-7-methyl-*N*-(4-methyl-5-(trifluoromethyl)thiophen-3-yl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidine-9-carboxamide (120 mg).

MS (ESI) M/Z: 742.5 [M+H] ⁺.

**Step E:** 6-((1S,6S)-5-(5-Hydroxy-6-methylpyrimidine-4-carbonyl)-2,5-diazabicyclo[4.2.0]octan-2-yl)-7-methyl-*N*-(4-methyl-5-(trifluoromethyl)thiophen-3-yl)-5-oxo-2-(2,5,6,7-tetrahydrooxepin-4-yl-7,7-d₂)-5,7,8,9-tetrahydropyrrolo[1,2-*c*][1,2,4]triazolo[1,5-a]pyrimidine-9-carboxamide (220 mg, 0.17 mmol) was separated by preparative SFC under the following separation conditions: preparative column: DAICEL CHIRALPAK^{®} OD-10, 25 * 250 mm, 10 m; mobile phase: supercritical CO₂/ MeOH+(0.05%) NH₃·MeOH; flow rate: 70 mL/min; gradient: 50:50; detection wavelength: 214 nm/254 nm. The product was collected and lyophilized under reduced pressure to obtain mixture 1 (80 mg, mixture with retention times of 4.33 min and 4.48 min), single-configuration product **322-P3** (22.97 mg, retention time: 4.97 min), and single-configuration product **322-P4** (10.89 mg, retention time: 8.90 min).

Mixture 1 (80 mg) was separated by preparative SFC under the following separation conditions: preparative column: DAICEL CHIRALPAK^{®} AS-10, 25*250 mm, 10 µm; mobile phase: supercritical CO₂/ IPA+(0.05%) *N*H₃·MeOH; flow rate: 60 mL/min; gradient: 70:30; detection wavelength: 214 nm/254 nm; the product was collected and lyophilized under reduced pressure to obtain single-configuration product **322-P1** (15.45 mg, retention time: 3.86 min) and single-configuration product **322-P2** (12.67 mg, retention time: 4.23 min).

### 322-P1

MS (ESI) M/Z: 742.4 [M+H] ⁺

¹H NMR (400 MHz, DMSO-d₆) δ 10.26 (s, 1H), 8.51 (s, 1H), 7.84 (s, 1H), 6.83 (t, *J* = 4.6 Hz, 1H), 5.54 - 5.45 (m, 1H), 4.48 - 4.32 (m, 1H), 4.31 - 4.20 (m, 2H), 3.73 - 3.63 (m, 1H), 3.56 - 3.38 (m, 4H), 3.28 - 3.24 (m, 1H), 2.88 - 2.80 (m, 2H), 2.62 - 2.54 (m, 1H), 2.42 (s, 3H), 2.36 - 2.32 (m, 1H), 2.28 (s, 3H), 1.89 - 1.82 (m, 2H), 1.67 - 1.59 (m, 1H), 1.53 (d, *J* = 6.8 Hz, 3H), 1.46 - 1.29 (m, 2H), 1.22 - 1.11 (m, 1H).

### 322-P2

MS (ESI) M/Z: 742.4 [M+H] ⁺.

¹H NMR (400 MHz, DMSO-d₆) δ 10.34 (s, 1H), 8.53 (s, 1H), 7.94 (s, 1H), 6.92 - 6.88 (m, 1H), 5.51 - 5.43 (m, 1H), 4.52 - 4.38 (m, 1H), 4.36 - 4.26 (m, 2H), 4.18 - 3.50 (m, 3H), 3.31 - 3.18 (m, 2H), 3.10 - 2.97 (m, 1H), 2.96 - 2.84 (m, 2H), 2.65 - 2.59 (m, 1H), 2.47 (s, 3H), 2.36 (s, 3H), 2.19 - 2.11 (m, 1H), 1.95 - 1.86 (m, 2H), 1.80 - 1.69 (m, 1H), 1.51 (d, *J =* 7.1 Hz, 3H), 1.48 - 1.35 (m, 2H), 1.28 - 1.19 (m, 1H).

### 322-P3

MS (ESI) M/Z: 742.4 [M+H] ⁺.

¹H NMR (400 MHz, DMSO-d₆) δ 10.26 (s, 1H), 8.48 (s, 1H), 7.85 (s, 1H), 6.83 (t, *J* = 4.6 Hz, 1H), 5.53 - 5.43 (m, 1H), 4.47 - 4.30 (m, 1H), 4.29 - 4.21 (m, 2H), 4.13 - 3.42 (m, 3H), 3.27 - 3.05 (m, 3H), 2.88 - 2.79 (m, 2H), 2.62 - 2.54 (m, 1H), 2.41 (s, 3H), 2.38 - 2.32 (m, 1H), 2.28 (s, 3H), 1.90 - 1.81 (m, 2H), 1.69 - 1.60 (m, 1H), 1.55 (d, *J =* 6.7 Hz, 3H), 1.49 - 1.31 (m, 2H), 1.28 - 1.13 (m, 1H).

### 322-P4

MS (ESI) M/Z: 742.4 [M+H] ⁺.

¹H NMR (400 MHz, DMSO-d₆) δ 10.36 (s, 1H), 10.27 (s, 1H), 8.57 (s, 1H), 7.87 (s, 1H), 6.85 (t, *J =* 4.6 Hz, 1H), 5.49 - 5.38 (m, 1H), 4.50 - 4.38 (m, 1H), 4.30 - 4.19 (m, 2H), 3.78 - 3.68 (m, 1H), 3.62 - 3.36 (m, 4H), 3.06 - 2.92 (m, 1H), 2.91 - 2.79 (m, 2H), 2.55 - 2.53 (m, 1H), 2.44 (s, 3H), 2.29 (s, 3H), 2.11 - 2.02 (m, 1H), 1.91 - 1.81 (m, 2H), 1.70 - 1.59 (m, 1H), 1.52 - 1.30 (m, 5H), 1.22 - 1.12 (m, 1H).

### Example 318

### 2-(5-Ethyl-6-((1S,6S)-5-(5-hydroxy-6-methylpyrimidine-4-carbonyl)-2,5-diazabicyclo [4.2.0]octan-2-yl)-7-oxo-2-(2,5,6,7-tetrahydrooxepin-4-yl-7,7-D₂)-[1,2,4]triazolo[1,5-a]pyrimidin-4(7H)-yl)-N-(4-methyl-5-(trifluoromethyl)thiophen-3-yl)acetamide

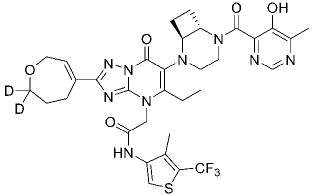

**The compound may also be synthesized according to the following steps:**

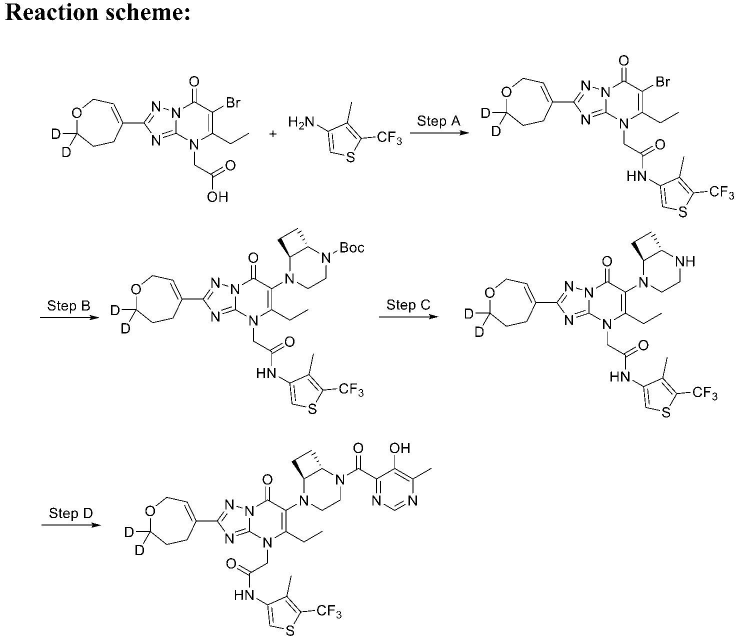

### Procedure:

**Step A:** 2-(6-Bromo-5-ethyl-7-oxo-2-(2,5,6,7-tetrahydrooxepin-4-yl-7,7-D₂)-[1,2,4]triazolo[1,5-a]pyrimidin-4(7H)-yl)acetic acid (370 mg, 926.8 µmol) and 4-methyl-5-trifluoromethylthiophen-3-amine hydrochloride (202 mg, 926.8 µmol) were dissolved in acetonitrile (10 mL). *N*-Methylimidazole (381 mg, 4.6 mmol) and *N,N,N',N'-*tetramethylchloroformamidinium hexafluorophosphate (780 mg, 2.8 mmol) were added sequentially, and the reaction mixture was stirred at room temperature for 1 hour.

After LCMS monitoring indicated the disappearance of the starting material, water was added to the reaction mixture, and the mixture was extracted with ethyl acetate (150 mL × 3). The combined organic phases were concentrated under reduced pressure, and the resulting mixture was subjected to column chromatography to obtain 2-(6-bromo-5-ethyl-7-oxo-2-(2,5,6,7-tetrahydrooxepin-4-yl-7,7-D₂)-[1,2,4]triazolo[1,5-a]pyrimidin-4(7H)-yl)-*N*-(4-methyl-5-(trifluoromethyl)thiophen-3-yl)acetamide (300 mg).

MS (ESI) M/Z: 562.7 [M+H] ⁺.

**Step B:** 2-(6-Bromo-5-ethyl-7-oxo-2-(2,5,6,7-tetrahydrooxepin-4-yl-7,7-D₂)-[1,2,4]triazolo[1,5-a]pyrimidin-4(7H)-yl)-*N*-(4-methyl-5-(trifluoromethyl)thiophen-3-yl)acetamide (330 mg, 586.8 µmol) and *tert-*butyl (1S,6S)-2,5-diazabicyclo[4.2.0]octane-2-carboxylate (623 mg, 2.9 mmol) were dissolved in dimethyl sulfoxide (5 mL). 2,6-Diisopropylaniline (104 mg, 586.8 µmol) and silver tetrafluoroborate (114 mg, 586.8 µmol) were added sequentially, and the reaction was carried out in a sealed tube at 125°C for 16 hours.

LCMS monitoring indicated the disappearance of the starting material, and the reaction mixture was filtered. The filtrate was purified by column chromatography to obtain *tert-*butyl (1S,6S)-5-(5-ethyl-4-(2-((4-methyl-5-(trifluoromethyl)thiophen-3-yl)amino)-2-oxoethyl)-7-oxo-2-(2,5,6,7-tetrahydrooxepin-4-yl-7,7-D₂)-4,7-dihydro-[1,2,4]triazolo[1,5-a]pyrimidin-6-yl)-2,5-diazabicyclo[4.2.0]octane-2-carboxylate (160 mg).

MS (ESI) M/Z: 694.9 [M+H] ⁺.

**Step C:** *tert*-Butyl (1S,6S)-5-(5-ethyl-4-(2-((4-methyl-5-(trifluoromethyl)thiophen-3-yl)amino)-2-oxoethyl)-7-oxo-2-(2,5,6,7-tetrahydrooxepin-4-yl-7,7-D₂)-4,7-dihydro-[1,2,4]triazolo[1,5-a]pyrimidin-6-yl)-2,5-diazabicyclo[4.2.0]octane-2-carboxylate (165 mg, 237.8 µmol) was dissolved in dichloromethane (3 mL), and then trifluoroacetic acid (1 mL) was added. The reaction was carried out at room temperature for 1 hour.

LCMS monitoring indicated the disappearance of the starting material, and the reaction mixture was diluted with dichloromethane and then concentrated under reduced pressure to obtain 2-(6-((1S,6S)-2,5-diazabicyclo[4.2.0]octan-2-yl)-5-ethyl-7-oxo-2-(2,5,6,7-tetrahydrooxepin-4-yl-7,7-d₂)-[1,2,4]triazolo[1,5-a]pyrimidin-4(7H)-yl)-*N*-(4-methyl-5-(trifluoromethyl)thiophen-3-yl)acetamide (140 mg).

MS (ESI) M/Z: 594.9 [M+H] ⁺.

**Step D:** 5-Hydroxy-6-methylpyrimidine-4-carboxylic acid (73 mg, 471.65 µmol), *N-*hydroxy-7-azabenzotriazole (68 mg, 495.2 µmol), and 1-(3-dimethylaminopropyl)-3-ethylcarbodiimide hydrochloride (95 mg, 495.2 µmol) were dissolved in *N,N-*dimethylformamide (2 mL) and stirred in an ice-water bath for 0.5 hour for later use. 2-(6-((1S,6S)-2,5-Diazabicyclo[4.2.0]octan-2-yl)-5-ethyl-7-oxo-2-(2,5,6,7-tetrahydrooxepin-4-yl-7,7-d₂)-[1,2,4]triazolo[1,5-a]pyrimidin-4(7H)-yl)-*N*-(4-methyl-5-(trifluoromethyl)thiophen-3-yl)acetamide (140 mg, 235.8 µmol) and *N*,*N*-diisopropylethylamine (153 mg, 1.2 mmol) were dissolved in *N,N*-dimethylformamide (2 mL). The previously prepared solution was added dropwise to the reaction mixture, and the reaction was carried out at room temperature for 10 minutes.

LCMS monitoring indicated the disappearance of the starting material. The mixture was filtered, and the filtrate was purified by C18 (0.1% formic acid in water/acetonitrile = 40/60) to obtain 2-(5-ethyl-6-((1S,6S)-5-(5-hydroxy-6-methylpyrimidine-4-carbonyl)-2,5-diazabicyclo[4.2.0]octan-2-yl)-7-oxo-2-(2,5,6,7-tetrahydrooxepin-4-yl-7,7-D₂)-[1,2,4]triazolo[1,5-a]pyrimidin-4(7H)-yl)-*N*-(4-methyl-5-(trifluoromethyl)thiophen-3-yl)acetamide (93.17 mg).

MS (ESI) M/Z: 730.4 [M+H] ⁺.

¹H NMR (400 MHz, DMSO-*d*₆) δ 10.33 (s, 1H), 10.16 (s, 1H), 8.57 (s, 1H), 7.86 (s, 1H), 6.89 (t, *J =* 4.7 Hz, 1H), 5.22 (s, 2H), 4.53 - 4.36 (m, 1H), 4.31 - 4.23 (m, 2H), 3.70 - 3.54 (m, 1H), 3.50 - 3.35 (m, 3H), 3.27 - 3.16 (m, 1H), 3.07 - 2.89 (m, 2H), 2.89 - 2.79 (m, 2H), 2.54 - 2.52 (m, 1H), 2.44 (s, 3H), 2.30 (s, 3H), 1.91 - 1.81 (m, 2H), 1.70 - 1.58 (m, 1H), 1.44 - 1.29 (m, 2H), 1.19 (t, *J =* 7.4 Hz, 3H).

### Example 361:

### 6-((1S,6S)-5-(5-Hydroxy-6-methylpyrimidine-4-carbonyl)-2,5- diazabicyclo[4.2.0]octan-2-yl)-7-methyl-N-(3-methyl-4-(trifluoromethyl)thiophen-2-yl)-

### 5-oxo-2-(2,5,6,7-tetrahydrooxepin-4-yl)-5,7,8,9-tetrahydropyrrolo[1,2-c][1,2,4]triazolo[1,5-a]pyrimidine-9-carboxamide

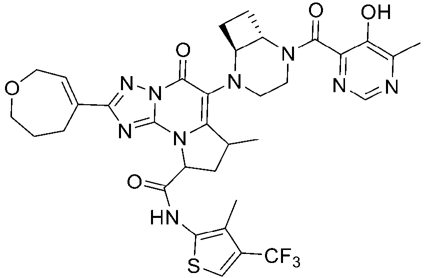

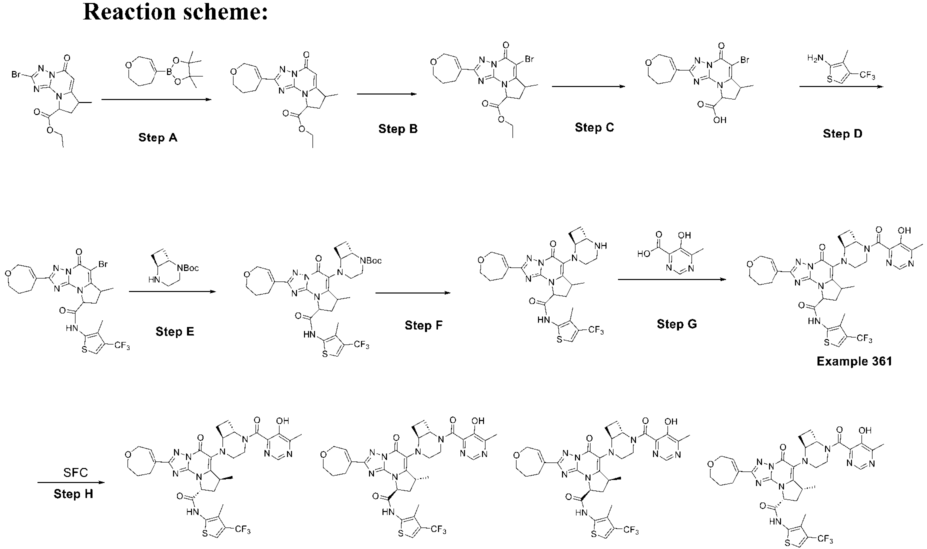

### Procedure:

**Step A:** Compound ethyl 4-bromo-10-methyl-7-oxo-1,3,5,6-tetraazatricyclo[7.3.0.0^{2,6}]dodeca-2,4,8-triene-12-carboxylate (5.6 g, 16.4 mmol) and 4,4,5,5-tetramethyl-2-(2,3,4,7-tetrahydrooxepin-5-yl)-1,3,2-dioxaborolane (4.8 g, 21.3 mmol) were dissolved in 1,4-dioxane (60 mL) and water (10 mL). Then potassium phosphate (8.7 g, 41.0 mmol) and [1,1'-bis(diphenylphosphino)ferrocene]dichloropalladium(II) (1.2 g, 1.6 mmol) were added. The reaction system was purged with nitrogen and then stirred at 100°C for 8 hours.

After LCMS monitoring indicated the disappearance of the starting material, the reaction mixture was filtered through diatomite, concentrated under reduced pressure to remove dioxane, then water (100 mL) was added and the mixture was extracted with ethyl acetate (200 mL × 2). The combined organic phases were dried over anhydrous sodium sulfate, filtered, and concentrated under reduced pressure. The resulting residue was purified by normal-phase silica gel column chromatography to obtain compound 10-methyl-7-oxo-4-(2,3,4,7-tetrahydrooxepin-5-yl)-1,3,5,6-tetraazatricyclo[7.3.0.0^{2,6}]dodeca-2,4,8-triene-12-carboxylate (4.0 g).

MS (ESI) M/Z: 359.4 [M+H] ⁺.

**Step B:** Compound 10-methyl-7-oxo-4-(2,3,4,7-tetrahydrooxepin-5-yl)-1,3,5,6-tetraazatricyclo[7.3 .0.0^{2,6}]dodeca-2,4,8-triene-12-carboxylate (4.0 g, 11.1 mmol) was dissolved in acetonitrile (50 mL), then N-bromosuccinimide (1.9 g, 11.1 mmol) was added in an ice bath. The reaction mixture was naturally warmed to room temperature and stirred overnight.

After LCMS monitoring indicated the disappearance of the starting material, the reaction system was quenched with aqueous sodium thiosulfate solution, then the mixture was extracted with ethyl acetate (500 mL × 2). The combined organic phases were washed with saturated brine (100 mL × 3), then dried over anhydrous sodium sulfate, filtered, and finally concentrated under reduced pressure to obtain 8-bromo-10-methyl-7-oxo-4-(2,3,4,7-tetrahydrooxepin-5-yl)-1,3,5,6-tetraazatricyclo[7.3.0.0^{2,6}]dodeca-2,4,8-triene-12-carboxylate (3.6 g, the crude product was used directly in the next step).

MS (ESI) M/Z: 437.3 [M+H] ⁺.

**Step C:** Compound 8-bromo-10-methyl-7-oxo-4-(2,3,4,7-tetrahydrooxepin-5-yl)-1,3,5,6-tetraazatricyclo[7.3.0.0^{2,6}]dodeca-2,4,8-triene-12-carboxylate (3.6 g, crude product) was dissolved in methanol (20.0 mL) and tetrahydrofuran (60.0 mL), then 2 M aqueous sodium hydroxide solution (20.0 mL) was added. The reaction mixture was stirred at room temperature for 1 hour.

After LCMS monitoring indicated the disappearance of the starting material, the reaction mixture was first concentrated under reduced pressure to remove methanol and tetrahydrofuran. Then water (150 mL) was added to the reaction mixture, and the mixture was extracted with ethyl acetate (200 mL). The product was in the aqueous phase. The aqueous phase was adjusted to pH 3-4 with dilute hydrochloric acid, and then extracted with ethyl acetate (200 mL × 3). The combined organic phases were first washed with saturated brine (100 mL × 3), then dried over anhydrous sodium sulfate, filtered, and finally concentrated under reduced pressure to obtain 8-bromo-10-methyl-7-oxo-4-(2,3,4,7-tetrahydrooxepin-5-yl)-1,3,5,6-tetraazatricyclo[7.3.0.0^{2,6}]dodeca-2,4,8-triene-12-carboxylic acid (3.2 g).

MS (ESI) M/Z: 409.2 [M+H] ⁺.

**Step D:** 8-Bromo-10-methyl-7-oxo-4-(2,3,4,7-tetrahydrooxepin-5-yl)-1,3,5,6-tetraazatricyclo[7.3.0.0^{2,6}]dodeca-2,4,8-triene-12-carboxylic acid (1.6 g, 3.9 mmol) and 3-methyl-4-(trifluoromethyl)thiophen-2-amine hydrochloride (1.7 g, 7.8 mmol) were dissolved in N,N-dimethylformamide (15.0 mL), then N,N-diisopropylethylamine (2.5 g, 19.6 mmol) was added. The reaction system was purged with nitrogen and then stirred at 0°C, then 2-(7-azabenzotriazol-1-yl)-*N,N,N',N*'-tetramethyluronium hexafluorophosphate (3.0 g, 7.8 mmol) was added. The reaction mixture was stirred at 0°C to room temperature for 2 hours.

After LCMS monitoring indicated the disappearance of the starting material, ethyl acetate (200 L) and aqueous hydrochloric acid (0.5 N, 100 mL) were added to the reaction mixture and stirred for 5 minutes. Then the phases were separated, and the organic phase was collected. The organic phase was washed with saturated aqueous sodium bicarbonate solution (100 mL), and the phases were separated. Then the organic phase was washed with saturated brine (100 mL × 3), then dried over anhydrous sodium sulfate, filtered, and concentrated under reduced pressure. The resulting mixture was purified by silica gel column chromatography to obtain 6-bromo-7-methyl-*N*-(3-methyl-4-(trifluoromethyl)thiophen-2-yl)-5-oxo-2-(2,5,6,7-tetrahydrooxepin-4-yl)-5,7,8,9-tetrahydropyrrolo[1,2-c][1,2,4]triazolo[1,5-a]pyrimidine-9-carboxamide (1.2 g).

MS (ESI) M/Z: 572.3 [M+H] ⁺.

**Step E:** In a microwave tube, 6-bromo-7-methyl-N-(3-methyl-4-(trifluoromethyl)thiophen-2-yl)-5-oxo-2-(2,5,6,7-tetrahydrooxepin-4-yl)-5,7,8,9-tetrahydropyrrolo[1,2-c][1,2,4]triazolo[1,5-a]pyrimidine-9-carboxamide (1.2 g, 2.1 mmol) and *tert-*butyl (1S,6S)-2,5-diazabicyclo[4.2.0]octane-2-carboxylate (1.3 g, 6.3 mmol) were dissolved in acetonitrile (2.5 mL), and then potassium acetate (617.0 mg, 6.2 mmol) was added. The reaction mixture was stirred at 90°C for 8 hour.

LCMS monitoring indicated completion, Water (50 mL) was added to the reaction mixture, and then the mixture was extracted with ethyl acetate (50 mL × 3). The organic phase was dried, filtered, and concentrated under reduced pressure to obtain a crude product. The crude product was directly purified by reverse-phase C18 column chromatography (eluent: water (0.1% formic acid) / acetonitrile from 98/2 to 20/80) to obtain compound *tert-*butyl (1S,6S)-5-(7-methyl-9-((3-methyl-4-(trifluoromethyl)thiophen-2-yl)carbamoyl)-5-oxo-2-(2,5,6,7-tetrahydrooxepin-4-yl)-5,7,8,9-tetrahydropyrrolo[1,2-c][1,2,4]triazolo[1,5-a]pyrimidin-6-yl)-2,5-diazabicyclo[4.2.0]octane-2-carboxylate (800 mg).

MS (ESI) M/Z: 648.5 [M-56+H]⁻.

**Step F:** *tert*-Butyl (1S,6S)-5-(7-methyl-9-((3-methyl-4-(trifluoromethyl)thiophen-2-yl)carbamoyl)-5-oxo-2-(2,5,6,7-tetrahydrooxepin-4-yl-7,7-d₂)-5,7,8,9-tetrahydropyrrolo[1,2-c][1,2,4]triazolo[1,5-a]pyrimidin-6-yl)-2,5-diazabicyclo[4.2.0]octane-2-carboxylate (800 mg, 1.14 mmol) was dissolved in dichloromethane (6 mL), and then trifluoroacetic acid (3 mL) was added. The reaction mixture was stirred at room temperature for 1 hour.

After LCMS monitoring indicated the disappearance of the starting material, the reaction mixture was diluted with dichloromethane, and the resulting mixture was concentrated under reduced pressure to obtain 6-((1S,6S)-2,5-diazabicyclo[4.2.0]octan-2-yl)-7-methyl-N-(3-methyl-4-(trifluoromethyl)thiophen-2-yl)-5-oxo-2-(2,5,6,7-tetrahydrooxepin-4-yl)-5,7,8,9-tetrahydropyrrolo[1,2-c][1,2,4]triazolo[1,5-a]pyrimidine-9-carboxamide (686.0 mg). The crude product was used directly in the next step without further purification.

MS (ESI) M/Z: 604.4 [M+H] ⁺.

**Step G:** Compound 5-hydroxy-6-methylpyrimidine-4-carboxylic acid (196.3 mg, 1.3 mmol) was dissolved in DMF (10 mL). EDCI (266.4 mg, 1.4 mmol) and HOAT (189.1 mg, 1.4 mmol) were added portionwise at 0°C, and the reaction mixture was stirred at 0°C for 30 minutes for later use. 6-((1S,6S)-2,5-Diazabicyclo[4.2.0]octan-2-yl)-7-methyl-*N*-(3-methyl-4-(trifluoromethyl)thiophen-2-yl)-5-oxo-2-(2,5,6,7-tetrahydrooxepin-4-yl)-5,7,8,9-tetrahydropyrrolo[1,2-c][1,2,4]triazolo[1,5-a]pyrimidine-9-carboxamide (686.0 mg, crude product) was dissolved in DMF (5 mL), and DIEA was added to adjust the pH to basic. Then, previously prepared solution was slowly added dropwise to the reaction system. After the dropwise addition was completed, the reaction mixture was stirred at 0°C for 10 minutes.

After LCMS monitoring indicated the disappearance of the starting material, the reaction mixture was directly purified by reverse-phase C18 column chromatography (eluent: water (0.1% formic acid) / acetonitrile = 60/40) to obtain compound 6-((1*S*,6*S*)-5-(5-hydroxy-6-methylpyrimidine-4-carbonyl)-2,5-diazabicyclo[4.2.0]octan-2-yl)-7-methyl-*N*-(3-methyl-4-(trifluoromethyl)thiophen-2-yl)-5-oxo-2-(2,5,6,7-tetrahydrooxepin-4-yl)-5,7,8,9-tetrahydropyrrolo[1,2-*c*][1,2,4]triazolo[1,5-*a*]pyrimidine-9-carboxamide (820.0 mg).

MS (ESI) M/Z: 740.6 [M+H] ⁺.

**Step H:** 6-((1*S*,6*S*)-5-(5-Hydroxy-6-methylpyrimidine-4-carbonyl)-2,5-diazabicyclo[4.2.0]octan-2-yl)-7-methyl-*N*-(3-methyl-4-(trifluoromethyl)thiophen-2-yl)-5-oxo-2-(2,5,6,7-tetrahydrooxepin-4-yl)-5,7,8,9-tetrahydropyrrolo[1,2-*c*][1,2,4]triazolo[1,5-*a*]pyrimidine-9-carboxamide (540.0 mg, 0.3 mmol) was separated by preparative SFC under the following separation conditions: preparative column: DAICEL CHIRALPAK^{®} OJ-10, 25 * 250 mm 10 m; mobile phase: supercritical CO₂/ MeOH+(0.05%) NH₃·MeOH; flow rate: 90 mL/min; gradient: 45:55; detection wavelength: 214 nm/254 nm. The product was collected and lyophilized under reduced pressure to obtain mixture 1 (240 mg), single-configuration product 361-P3 (77.31 mg, retention time: 4.951 min), and single-configuration product 361-P4 (80.66 mg, retention time: 8.502 min).

Mixture 1 (240 mg) was separated by preparative SFC under the following separation conditions: preparative column: DAICEL CHIRALPAK^{®} OD-3, 3.0*100 mm 3 µm; mobile phase: supercritical CO₂/ IPA+(0.05%)DEA; flow rate: 1.5 mL/min; detection wavelength: 214 nm/254 nm. The product was collected and lyophilized under reduced pressure to obtain single-configuration product 361-P1 (58.5 mg, retention time: 4.583 min) and single-configuration product 361-P2 (58.66 mg, retention time: 4.887 min).

361-P1 MS (ESI) M/Z: 740.5 [M+H]⁺

¹H NMR (400 MHz, DMSO-d₆) δ 10.99 (s, 1H), 10.36 (s, 1H), 8.52 (s, 1H), 7.73 (s, 1H), 6.77 (t, *J =* 4.6 Hz, 1H), 5.50 (dd, *J =* 8.8, 4.4 Hz, 1H), 4.37 (d, *J =* 9.6 Hz, 1H), 4.20 (d, *J =* 4.7 Hz, 2H), 3.76 (t, *J =* 5.9 Hz, 2H), 3.69 - 3.59 (m, 1H), 3.50 - 3.42 (m, 4H), 3.25 - 3.17 (m, 1H), 2.79 (d, *J =* 9.3 Hz, 2H), 2.55 - 2.46 (m, 1H), 2.40 (s, 3H), 2.37 - 2.26 (m, 1H), 2.21 (s, 3H), 1.90 - 1.75 (m, 2H), 1.66 - 1.59 (m,1H), 1.48 (d, *J=* 6.9 Hz, 3H), 1.40 - 1.28 (m, 2H), 1.24 - 1.08 (m, 1H).

361-P2 MS (ESI) M/Z: 740.5 [M+H] ⁺.

¹H NMR (400 MHz, DMSO-d₆) δ 10.11 - 10.47 (m, 2H), 8.55 (s, 1H), 7.77 (s, 1H), 6.82 (t, *J =* 4.6 Hz, 1H), 5.48 (dd, *J =* 10.2, 1.8 Hz, 1H), 4.39 (d, *J =* 9.3 Hz, 1H), 4.24 (d, *J =* 4.6 Hz, 2H), 3.80 (t, *J =* 5.9 Hz, 2H), 3.73 - 3.64 (m, 2H), 3.46 (d, *J =* 31.4 Hz, 1H), 3.29 - 3.12 (m, 2H), 3.01 - 2.93 (m, 1H), 2.87 - 2.79 (m, 2H), 2.54 - 2.52 (m, 1H), 2.44 (s, 3H), 2.27 (s, 3H), 2.09 (d, *J =* 13.5 Hz, 1H), 1.95 - 1.79 (m, 2H), 1.78 - 1.64 (m, 1H), 1.45 - 1.35 (m, 5H), 1.24 - 1.15 (m, 1H).

361-P3 MS (ESI) M/Z: 740.5 [M+H] ⁺.

¹H NMR (400 MHz, DMSO-d₆) δ 10.87 - 10.21 (m, 2H), 8.55 (s, 1H), 7.77 (s, 1H), 6.81 (t, *J =* 4.6 Hz, 1H), 5.54 (dd, *J =* 8.6, 4.7 Hz, 1H), 4.37 (d, *J =* 14.4 Hz, 1H), 4.24 (d, *J* = 4.6 Hz, 2H), 4.18 - 3.83 (m, 1H), 3.80 (t, *J =* 5.8 Hz, 2H), 3.76 - 3.59 (m, 2H), 3.55 - 3.40 (m, 1H), 3.18 (d, *J =* 13.5 Hz, 2H), 2.82 (d, *J =* 6.0 Hz, 2H), 2.58 - 2.55 (m, 1H), 2.44 (s, 3H), 2.42 - 2.31 (m, 1H), 2.25 (s, 3H), 1.90 - 1.81 (m, 2H), 1.71 - 1.60 (m, 1H), 1.55 (d, *J =* 6.8 Hz, 3H), 1.50 - 1.30 (m, 2H), 1.27 - 1.11 (m, 1H).

361-P4MS (ESI) M/Z: 740.5 [M+H]⁺.

¹H NMR (400 MHz, DMSO-*d*₆) δ 11.32-10.18 (m, 2H), 8.56 (s, 1H), 7.77 (s, 1H), 6.81 (t, *J =* 4.6 Hz, 1H), 5.50 (dd, *J =* 8.6, 4.7 Hz, 1H), 4.54 - 4.36 (m, 1H), 4.24 (d, *J =* 4.6 Hz, 2H), 3.80 (t, *J =* 5.8 Hz, 2H), 3.77 - 3.65 (m, 1H), 3.63 - 3.34 (m, 4H), 3.06 - 2.91 (m, 1H), 2.89 - 2.80 (m, 2H), 2.54 - 2.51 (m, 1H), 2.44 (s, 3H), 2.27 (s, 3H), 2.12 - 2.01 (m, 1H), 1.93 - 1.80 (m, 2H), 1.71 - 1.55 (m, 1H), 1.51 - 1.30 (m, 5H), 1.28 - 1.11 (m, 1H).

| Example | MS (ESI) M/Z: | ¹H NMR |
|---|---|---|
| 7 | 640.5 [M+H] ⁺ | ¹H NMR (400 MHz, DMSO-d₆) δ 11.17 (s, 1H), 10.27 (s, 1H), 8.58 (s, 1H), 8.50 (d, *J =* 7.3 Hz, 1H), 7.91 (s, 1H), 7.85 (s, 1H), 7.47 (s, 1H), 7.06 (d, *J =* 7.3 Hz, 1H), 6.81 (s, 1H), 5.24 (s, 2H), 4.58 - 4.45 (m, 1H), 4.28 - 4.20 (m, 2H), 3.83 - 3.75 (m, 2H), 3.55 - 3.46 (m, 3H), 3.29 - 3.20 (m, 2H), 3.04 - 2.92 (m, 3H), 2.87 - 2.78 (m, 1H), 2.71 - 2.59 (m, 2H), 2.45 (s, 3H), 1.18 - 1.17 (m, 3H). |
| 15 | 710.4 [M+H] ⁺ | ¹H NMR (400 MHz, DMSO-d₆) δ 10.38 (s, 1H), 8.51 - 8.32 (m, 1H), 7.80 (d, *J =* 8.4 Hz, 1H), 7.62 (d, *J =* 8.5 Hz, 1H), 6.84 - 6.76 (m, 1H), 5.22 (s, 2H), 5.15 (s, 2H), 5.05 (s, 2H), 4.58 - 4.45 (m, 1H), 4.28 - 4.21 (m, 2H), 3.80 (t, *J =* 5.4 Hz, 2H), 3.64 - 3.43 (m, 3H), 3.26 - 3.23 (m, 1H), 3.04 - 2.92 (m, 3H), 2.86 - 2.75 (m, 1H), 2.69 - 2.61 (m, 1H), 2.5 (m, 2H), 2.41 (s, 3H), 1.18 (t, *J* = 7.4 Hz, 3H). |
| 17 | 769.3 [M+H] ⁺. | ¹H NMR (400 MHz, DMSO-*d*₆) δ 10.41 (s, 1H), 10.24 (s, 1H), 8.58 (s, 1H), 8.08 - 8.04 (m, 1H), 7.98 (s, 1H), 7.72 (d, *J =* 8.7 Hz, 1H), 6.82 (s, 1H), 5.33 (s, 2H), 4.57 - 4.47 (m, 1H), 4.47 - 4.37 (m, 1H), 4.20 - 4.09 (m, 1H), 3.93 - 3.80 (m, 1H), 3.76 - 3.61 (m, 1H), 3.56 - 3.39 (m, 3H), 3.26 - 3.22 (m, 1H), 3.06 - 2.91 (m, 3H), 2.87 - 2.76 (m, 1H), 2.70 - 2.59 (m, 2H), 2.44 (s, 3H), 2.11 - 1.90 (m, 2H), 1.20 - 1.17 (m, 3H), 0.87 - 0.83 (m, 2H), 0.80 - 0.76 (m, 2H). |
| 19 | 755.4 [M-H] ⁻. | ¹H NMR (400 MHz, DMSO-*d*₆) δ 10.39 (s, 1H), 10.23 (s, 1H), 8.57 (s, 1H), 8.06 (dd, *J =* 8.6, 3.0 Hz, 1H), 7.98 (s, 1H), 7.72 (d, *J = 8.6* Hz, 1H), 6.80 (d, *J =* 10.8 Hz, 1H), 5.32 (s, 2H), 4.52 (d, *J* = 12.4 Hz, 1H), 4.17 (d, *J =* 20.2 Hz, 2H), 3.69 - 3.58 (m, 2H), 3.54 - 3.44 (m, 3H), 3.27 - 3.21 (m, 1H), 3.04 - 2.93 (m, 3H), 2.81 (d, *J =* 11.2 Hz, 1H), 2.67 - 2.54 (m, 3H), 2.44 (s, 3H), 2.39 - 2.30 (m, 2H), 1.17 (t, *J =* 3.3 Hz, 3H), 1.01 (t, *J =* 7.2 Hz, 3H). |
| 20 | 779.1 [M+H] ⁺ | ¹H NMR (400 MHz, DMSO-d₆) δ 10.41 (s, 1H), 10.24 (s, 1H), 8.58 (s, 1H), 8.06 (d, *J =* 8.6 Hz, 1H), 7.98 (s, 1H), 7.72 (d, *J =* 8.8 Hz, 1H), 6.84 (s, 1H), 5.33 (s, 2H), 4.58 - 4.44 (m, 1H), 3.93 (s, 2H), 3.55 - 3.44 (m, 3H), 3.39 - 3.38 (m, 1H), 3.29 - 3.20 (m, 2H), 3.04 - 2.97 (m, 3H), 2.94 (s, 3H), 2.85 - 2.78 (m, 1H), 2.70 - 2.62 (m, 3H), 2.44 (s, 3H), 1.19 (t, *J =* 7.4 Hz, 3H) |
| 21 | 716.2 [M+H]⁺ | ¹H NMR (400 MHz, DMSO-d₆) δ 10.36 (s, 1H), 10.23 (s, 1H), 8.58 (s, 1H), 8.06 (d, *J =* 8.6 Hz, 1H), 8.01 - 7.92 (m, 1H), 7.75 - 7.66 (m, 1H), 7.14 - 7.08 (m, 1H), 5.31 (s, 2H), 4.52 (d, *J=* 12.6 Hz, 1H), 3.72 - 3.60 (m, 4H), 3.52 - 3.47 (m, 3H), 3.24 - 3.17 (m, 1H), 3.04 - 2.94 (m, 3H), 2.93 - 2.88 (m, 2H), 2.86 - 2.76 (m, 1H), 2.68 - 2.58 (m, 1H), 2.56 - 2.51 (m, 2H), 2.44 (s, 3H), 1.18 (t, *J =* 7.4 Hz, 3H). |
| 38 | 728.3 [M+H] ⁺ | ¹H NMR (400 MHz, DMSO-*d*₆) δ 11.84 (s, 1H), 10.38 (s, 1H), 8.63 (s, 1H), 8.05 (d, *J =* 8.7 Hz, 1H), 7.97 (s, 1H), 7.72 (d, *J =* 8.7 Hz, 1H), 6.83 (s, 1H), 5.30 (s, 2H), 4.31 - 4.20 (m, 2H), 4.13 - 4.03 (m, 1H), 3.99 - 3.87 (m, 2H), 3.84 - 3.76 (m, 2H), 3.75 - 3.66 (m, 1H), 3.64 - 3.41 (m, 2H), 3.11 - 2.94 (m, 3H), 2.93 - 2.82 (m, 3H), 2.50 - 2.50 (m, 2H), 2.45 (s, 3H), 1.20 - 1.15 (m, 3H). |
| 39 | 711.0 [M+H] ⁺ | ¹H NMR (400 MHz, DMSO-d₆) δ 13.82 (s, 1H), 10.39 (s, 1H), 8.34 - 8.24 (m, 2H), 8.07 (d, *J =* 8.5 Hz, 1H), 7.98 (s, 1H), 7.90 (d, *J =* 5.5 Hz, 1H), 7.72 (d, *J =* 8.6 Hz, 1H), 6.83 (s, 1H), 5.33 (s, 2H), 4.68 (d, *J =* 12.4 Hz, 1H), 4.31 - 4.16 (m, 3H), 3.80 (m, 2H), 3.65 - 3.48 (m, 2H), 3.40 - 3.35 (m, 1H), 3.17 - 2.96 (m, 3H), 2.88 (m, 1H), 2.69 (m, 1H), 2.50 (m, 2H), 1.21 (t, *J =* 7.6 Hz, 3H). |
| 40 | 742.4 [M+H] ⁺ | ¹H NMR (400 MHz, DMSO-d₆) δ 12.64 (s, 1H), 10.34 (s, 1H), 8.74 - 8.33 (m, 1H), 8.06 (d, *J =* 8.5 Hz, 1H), 7.96 (d, *J =* 2.1 Hz, 1H), 7.72 (dd, *J =* 8.7, 2.1 Hz, 1H), 6.83 (s, 1H), 5.31 (s, 2H), 4.52 (d, *J =* 60.1 Hz, 2H), 4.29 - 4.20 (m, 2H), 3.93 (d, *J =* 45.6 Hz, 2H), 3.81 (t, *J =* 5.4 Hz, 2H), 3.42 - 3.35 (m, 2H), 3.01 - 2.88 (m, 2H), 2.75 - 2.66 (m, 2H), 2.50 (m, 2H), 2.43 (s, 3H), 2.06 - 1.92 (m, 2H), 1.85 - 1.68 (m, 2H), 1.18 (t, *J =* 7.4 Hz, 3H). |
| 76 | 727.1 [M+H]⁺ | ¹H NMR (400 MHz, DMSO-d₆) δ 8.29 (s, 1H), 8.20 (d, *J=* 8.1 Hz, 1H), 8.14 (s, 1H), 7.95 (d, *J =* 7.7 Hz, 1H), 6.83 (s, 1H), 5.89 (s, 2H), 4.50 (d, *J =* 12.1 Hz, 1H), 4.25 (s, 2H), 3.81 (t, *J =* 5.2 Hz, 2H), 3.45 (dd, *J =* 27.1, 11.7 Hz, 4H), 3.30 (d, *J =* 12.0 Hz, 1H), 3.18 - 2.96 (m, 4H), 2.84 (d, *J =* 10.5 Hz, 1H), 2.66 (d, *J =* 10.2 Hz, 1H), 2.37 (s, 3H), 1.21 (m, 3H). |
| 78 | 704.4 [M+H] ⁺ | 1H NMR (400 MHz, DMSO-d₆) δ 10.32 (s, 2H), 8.43 (d, *J =* 43.5 Hz, 1H), 8.05 (t, *J =* 8.1 Hz, 1H), 7.96 (s, 1H), 7.71 (t, *J =* 7.2 Hz, 1H), 6.83 (s, 1H), 5.28 (d, *J =* 9.2 Hz, 2H), 4.31 - 4.21 (m, 2H), 3.81 (t, *J =* 5.4 Hz, 2H), 3.72 - 3.47 (m, 1H), 3.39 (m, 1H), 3.29 - 3.05 (m, 3H), 3.02 (s, 2H), 2.93 - 2.74 (m, 4H), 2.56 (s, 3H), 2.39 (d, *J =* 16.8 Hz, 3H), 1.10 (m, 3H). |
| 79 | 694.4 [M+H] ⁺ | ¹H NMR (400 MHz, DMSO-d₆) δ 10.30 (s, 1H), 8.55 (s, 1H), 8.06 (d, *J =* 8.5 Hz, 1H), 7.67 (s, 1H), 7.53 (d, *J =* 8.5 Hz, 1H), 6.81 (s, 1H), 5.34 (s, 2H), 4.53 (d, *J =* 12.1 Hz, 1H), 4.42 (t, *J =* 8.4 Hz, 2H), 4.27 - 4.18 (m, 2H), 3.79 (t, *J =* 5.3 Hz, 2H), 3.55 - 3.47 (m, 3H), 3.37 - 3.22 (m , 5H), 3.02 - 2.96 (m, 3H), 2.83 (m, 1H), 2.66 (m, 1H), 2.44 (s, 3H), 1.17 (t, *J =* 7.4 Hz, 3H). |
| 80 | 694.5 [M+H] ⁺ | ¹H NMR (400 MHz, DMSO-d₆) δ 10.22 (s, 1H), 8.57 (s, 1H), 7.83 - 7.78 (m, 1H), 7.75 - 7.70 (m, 1H), 7.67 - 7.61 (m, 1H), 6.84 - 6.77 (m, 1H), 5.25 (s, 2H), 5.17 (d, *J =* 6.4 Hz, 2H), 4.78 (s, 2H), 4.57 - 4.47 (m, 1H), 4.26 - 4.18 (m, 2H), 3.82 - 3.76 (m, 2H), 3.58 - 3.45 (m, 3H), 3.29 - 3.22 (m, 1H), 3.04 - 2.89 (m, 3H), 2.85 - 2.79 (m, 1H), 2.67 - 2.61 (m, 1H), 2.50 (m, 2H), 2.44 (s, 3H), 1.17 (t, *J =* 7.4 Hz, 3H). |
| 81 | 714.5 [M+H] ⁺ | ¹H NMR (400 MHz, DMSO-*d*₆) δ 8.60 (s, 1H), 8.05 (d, *J =* 8.6 Hz, 1H), 7.92 (s, 1H), 7.68 (d, *J =* 8.9 Hz, 1H), 6.83 (s, 1H), 5.26 (s, 2H), 4.95 (s, 2H), 4.37 (s, 2H), 4.27 - 4.25 (m, 2H), 4.17 - 4.11 (m, 4H), 3.81 (t, *J =* 5.4 Hz, 2H), 2.91 (m, 2H), 2.50 (m, 2H), 2.42 (s, 3H), 1.15 (t, *J =* 7.4 Hz, 3H). |
| 82 | 742.4 [M+H] ⁺ | ¹H NMR (400 MHz, DMSO-*d*₆) δ 10.35 (s, 1H), 10.11 (s, 1H), 8.54 (s, 1H), 8.05 (d, *J =* 8.6 Hz, 1H), 7.96 (d, *J =* 2.1 Hz, 1H), 7.71 (dd, *J =* 8.7, 2.1 Hz, 1H), 6.82 (s, 1H), 5.28 (s, 2H), 4.25 (d, *J =* 2.8 Hz, 2H), 3.81 (t, *J =* 5.4 Hz, 2H), 3.78 - 3.72 (m, 4H), 3.67 - 3.56 (m, 2H), 3.26 - 3.18 (m, 2H), 2.98 - 2.85 (m, 2H), 2.50 (m, 2H), 2.43 (s, 3H), 2.01 - 1.90 (m, 2H), 1.89 - 1.80 (m, 2H), 1.16 (t, *J =* 7.4 Hz, 3H). |
| 88 | 675.4 [M+H] ⁺ | ¹H NMR (400 MHz, DMSO-d₆) δ 13.28 (s, 1H), 10.23 (s, 1H), 8.59 (s, 1H), 8.17 (s, 1H), 6.86 - 6.71 (m, 1H), 5.33 (s, 2H), 4.59 - 4.48 (m, 1H), 4.29 - 4.19 (m, 2H), 3.79 (t, *J =* 5.4 Hz, 2H), 3.54 - 3.44 (m, 3H), 3.31 - 3.20 (m, 1H), 3.06 - 2.95 (m, 3H), 2.86 - 2.78 (m, 1H), 2.70 - 2.62 (m, 1H), 2.50 (m, 2H), 2.45 (s, 3H), 1.16 (t, *J =* 7.4 Hz, 3H). |
| 89 | 675.4 [M+H] ⁺ | ¹H NMR (400 MHz, DMSO-d₆) δ 12.56 (s, 1H), 10.22 (s, 1H), 8.58 (s, 1H), 7.85 (s, 1H), 6.77 (s, 1H), 5.28 (s, 2H), 4.53 (d, *J =* 12.7 Hz, 1H), 4.23 (s, 2H), 3.79 (t, *J =* 5.3 Hz, 2H), 3.50 (q, *J =* 11.8, 10.2 Hz, 3H), 3.31 - 3.20 (m, 1H), 3.00 (t, *J =* 10.6 Hz, 3H), 2.83 (m, 1H), 2.65 (m, 1H), 2.50 (m, 2H), 2.44 (s, 3H), 1.15 (t, *J* = 7.4 Hz, 3H). |
| 91 | 716.3 [M+H] ⁺ | ¹H NMR (400 MHz, DMSO-d₆) δ 12.55 (s, 1H), 10.39 (s, 1H), 9.41 (s, 1H), 8.65 (s, 1H), 8.07 (d, *J =* 8.7 Hz, 1H), 7.98 (s, 1H), 7.73 (d, *J =* 8.7 Hz, 1H), 6.83 (s, 1H), 5.32 (s, 2H), 4.26 (d, *J =* 3.6 Hz, 2H), 3.98 - 3.85 (m, 1H), 3.84 - 3.74 (m, 2H), 3.53 - 3.46 (m, 2H), 3.03 - 2.90 (m, 2H), 2.84 - 2.74 (m, 2H), 2.51 - 2.51 (m, 2H), 2.45 (s, 3H), 2.02 - 1.81 (m, 4H), 1.20 (t, *J =* 7.5 Hz, 3H). |
| 96 | 716.1 [M+H] ⁺ | ¹H NMR (400 MHz, DMSO-d₆) δ 10.41 (s, 2H), 8.55 (s, 1H), 8.04 (d, *J =* 8.6 Hz, 1H), 7.98 (s, 1H), 7.73 (d, *J =* 8.7 Hz, 1H), 6.21 (s, 1H), 5.30 (s, 2H), 4.59 - 4.46 (m, 1H), 3.68 (t, *J =* 5.4 Hz, 2H), 3.56 (t, *J =* 5.6 Hz, 2H), 3.51 - 3.45 (m, 3H), 3.26 - 3.21 (m, 1H), 3.06 - 2.94 (m, 5H), 2.85 - 2.77 (m, 1H), 2.67 - 2.62 (m, 1H), 2.43 (s, 3H), 2.40 - 2.33 (m, 2H), 1.18 (t, 3H). |
| 97 | 716.4 [M-H] ⁻ | ¹H NMR (400 MHz, DMSO-*d*₆) δ 10.35 (s, 1H), 10.23 (s, 1H), 8.58 (s, 1H), 8.04 (d, *J =* 8.5 Hz, 1H), 7.98 (s, 1H), 7.77 - 7.66 (m, 1H), 5.30 (s, 2H), 4.57 - 4.46 (m, 1H), 3.82 - 3.71 (m, 2H), 3.57 - 3.42 (m, 3H), 3.26 - 3.15 (m, 3H), 3.05 - 2.89 (m, 3H), 2.85 - 2.76 (m, 1H), 2.65 - 2.58 (m, 3H), 2.44 (s, 3H), 2.05 - 1.85 (m, 1H), 1.58 - 1.50 (m, 2H), 1.27 - 1.23 (m, 2H), 1.16 (t*, J* = 7.4 Hz, 3H). |
| 98 | 700.3 [M+H] ⁺ | ¹H NMR (400 MHz, DMSO-*d*₆) δ 10.38 (s, 1H), 10.24 (s, 1H), 8.58 (s, 1H), 8.09 (d, *J =* 8.6 Hz, 1H), 7.98 (s, 1H), 7.72 (d, *J =* 8.2 Hz, 1H), 5.33 (s, 2H), 4.81 (m, 2H), 4.63 (m, 2H), 4.57 - 4.46 (m, 1H), 4.29 - 4.15 (m, 1H), 3.58 - 3.41 (m, 3H), 3.29 - 3.17 (m, 1H), 3.01 (m, 3H), 2.82 (m, 1H), 2.64 (m, 1H), 2.45 (s, 3H), 1.22 - 1.14 (m, 3H). |
| 99 | 684.1 [M+H] ⁺ | ¹H NMR (400 MHz, DMSO-*d*₆) δ 10.37 (s, 1H), 10.24 (s, 1H), 8.57 (s, 1H), 8.09 (d, *J =* 8.6 Hz, 1H), 7.98 (s, 1H), 7.72 (d, *J =* 8.6 Hz, 1H), 5.30 (s, 2H), 4.57 - 4.44 (m, 1H), 3.51 - 3.43 (m, 3H), 3.27 - 3.21 (m, 1H), 3.04 - 2.93 (m, 3H), 2.84 - 2.78 (m, 1H), 2.65 - 2.61 (m, 1H), 2.44 (s, 3H), 1.68 - 1.60 (m, 1H), 1.17 (t, 3H), 0.97 - 0.92 (m, 2H), 0.84 - 0.81 (m, 2H). |
| 100 | 720.2 [M+H] ⁺ | ¹H NMR (400 MHz, DMSO-*d*₆) δ 10.36 (s, 1H), 10.23 (s, 1H), 8.57 (s, 1H), 8.08 (d, *J =* 8.7 Hz, 1H), 7.98 (d, *J =* 2.2 Hz, 1H), 7.72 (d, *J =* 8.8 Hz, 1H), 5.24 (s, 2H), 4.94 (m, 1H), 4.51 (m, 1H), 3.82 (m, 2H), 3.54 - 3.43 (m, 5H), 3.29 - 3.21 (m, 1H), 3.00 - 2.93 (m, 3H), 2.80 (m, 1H), 2.62 (m, 1H), 2.44 (s, 3H), 2.05 (m, 2H), 1.66 (m, 2H), 1.20 - 1.12 (m, 3H). |
| 101 | 731.3 [M-H] ⁻ | ¹H NMR (400 MHz, DMSO-d₆) δ 10.39 (s, 2H), 8.54 (s, 1H), 8.07 (d, *J =* 8.5 Hz, 1H), 7.98 (s, 1H), 7.73 (d, *J =* 8.6 Hz, 1H), 5.35 (s, 2H), 4.60 - 4.45 (m, 1H), 3.65 (m, 4H), 3.57 - 3.44 (m, 7H), 3.28 - 3.19 (m, 1H), 3.02 (m, 3H), 2.82 (m, 1H), 2.64 (m, 1H), 2.43 (s, 3H), 1.20 - 1.11 (m, 3H) |
| 102 | 719.3 [M+H] ⁺ | ¹H NMR (400 MHz, DMSO-d₆) δ 10.45 (s, 1H), 10.26 (s, 1H), 8.57 (s, 1H), 8.07 (d, *J =* 8.5 Hz, 1H), 7.97 (s, 1H), 7.72 (d, *J =* 8.5 Hz, 1H), 6.79 (d, *J =* 7.9 Hz, 1H), 5.19 (s, 2H), 4.53 - 4.45 (m, 1H), 3.87 - 3.78 (m, 2H), 3.63 - 3.57 (m, 1H), 3.53 - 3.40 (m, 5H), 3.24 - 3.17 (m, 1H), 2.98 - 2.86 (m, 3H), 2.80 - 2.75 (m, 1H), 2.61 - 2.58 (m, 1H), 2.45 (s, 3H), 1.87 - 1.82 (m, 2H), 1.46 - 1.43 (m, 2H), 1.20 - 1.13 (m, 3H). |
| 105 | 688.4 [M+H] ⁺ | ¹H NMR (400 MHz, DMSO-d₆) δ 10.35 (s, 1H), 8.44 (s, 1H), 8.06 (d, *J =* 8.6 Hz, 1H), 7.96 (d, *J =* 2.1 Hz, 1H), 7.71 (dd, *J =* 8.7, 2.1 Hz, 1H), 6.86 - 6.80 (m, 1H), 5.31 (s, 2H), 4.30 - 4.22 (m, 2H), 3.94 (s, 2H), 3.81 (t, *J =* 5.4 Hz, 2H), 3.65 - 3.58 (m, 2H), 2.97 - 2.88 (m, 4H), 2.76 - 2.69 (m, 2H), 2.50 (m, 2H), 2.48 - 2.41 (m, 2H), 2.36 (s, 3H), 1.18 (t, *J =* 7.4 Hz, 3H). |
| 108 | 750.2 [M+H] ⁺ | ¹H NMR (400 MHz, DMSO-d₆) δ 10.40 (s, 2H), 8.53 (s, 1H), 8.06 (d, *J =* 8.8 Hz, 2H), 7.98 (s, 1H), 7.73 (d, *J =* 8.6 Hz, 1H), 7.21 (s, 1H), 6.90 (s, 2H), 6.82 (s, 1H), 5.36 (s, 2H), 4.89 (s, 2H), 4.53 (m, 1H), 3.53 - 3.46 (m, 3H), 3.26 - 3.24 (m, 1H), 3.04 - 2.98 (m, 3H), 2.86 - 2.82 (m, 1H), 2.67 - 2.65 (m, 1H), 2.43 (s, 3H), 1.25 - 1.18 (m, 3H). |
| 111 | 740.4 [M+H] ⁺ | ¹H NMR (400 MHz, DMSO-d₆) δ 10.35 (s, 1H), 10.23 (s, 1H), 8.58 (s, 1H), 8.05 (d, *J =* 8.6 Hz, 1H), 7.96 (d, *J =* 2.1 Hz, 1H), 7.72 (dd, *J =* 8.8, 2.1 Hz, 1H), 6.83 - 6.76 (m, 1H), 5.31 (s, 2H), 4.56 - 4.51 (m, 1H), 3.90 (s, 2H), 3.56 - 3.43 (m, 3H), 3.30 - 3.20 (m, 3H), 3.05 - 2.92 (m, 3H), 2.84 - 2.79 (m, 1H), 2.78 - 2.72 (m, 2H), 2.68 - 2.57 (m, 3H), 2.44 (s, 3H), 1.19 (t, *J =* 7.4 Hz, 3H). |
| 116 | 724.5 [M+H] ⁺ | ¹H NMR (400 MHz, DMSO-d₆) δ 10.18 (s, 1H), 8.51 (s, 1H), 8.09 (d, *J =* 8.6 Hz, 1H), 7.96 (d, *J =* 2.1 Hz, 1H), 7.84 (d, *J =* 2.3 Hz, 1H), 7.71 (dd, *J =* 8.9, 2.1 Hz, 1H), 6.68 (d, *J =* 2.3 Hz, 1H), 5.34 (s, 2H), 4.58 - 4.45 (m, 1H), 3.90 (s, 3H), 3.53 - 3.44 (m, 3H), 3.26 - 3.21 (m, 1H), 3.06 - 2.93 (m, 3H), 2.87 - 2.79 (m, 1H), 2.67 - 2.60 (m, 1H), 2.42 (s, 3H), 1.19 (t, *J =* 7.4 Hz, 3H). |
| 119 | 709.3 [M+H] ⁺ | ¹HNMR (400 MHz, DMSO-d₆) δ 12.34 (s, 1H), 10.22 (s, 1H), 8.58 (s, 1H), 6.78 (s, 1H), 5.37 (s, 2H), 4.52 (d, *J =* 13.1 Hz, 1H), 4.29 - 4.19 (m, 2H), 3.79 (t, *J =* 5.3 Hz, 2H), 3.55- 3.49 (m, 3H), 3.27 - 3.19 (m, 1H), 3.02 - 2.92 (m, 3H), 2.86 - 2.77 (m, 1H), 2.68 - 2.2.62 (m, 1H), 2.50 (m, 2H), 2.44 (s, 3H), 1.15 (t, *J =* 7.4 Hz, 3H). |
| 138 | 710.0 [M-H] ⁻ | ¹H NMR (400 MHz, DMSO-d₆) δ 10.36 (s, 1H), 10.24 (s, 1H), 8.58 (s, 1H), 8.10 (d, *J =* 8.6 Hz, 1H), 7.98 (d, *J* = 2.2 Hz, 1H), 7.72 (dd, *J* = 8.9, 2.2 Hz, 1H), 5.32 (s, 2H), 4.52 (m, 1H), 3.54 - 3.44 (m, 3H), 3.30 - 3.18 (m, 1H), 3.04 - 2.86 (m, 4H), 2.81 (m, 1H), 2.64 (m, 1H), 2.45 (s, 3H), 2.06 - 1.95 (m, 2H), 1.76 - 1.51 (m, 6H), 1.20 - 1.15 (m, 3H). |
| 153 | 819.3 [M+H] ⁺ | ¹H NMR (400 MHz, DMSO-d₆) δ 10.40 (s, 1H), 10.28 (s, 1H), 8.57 (s, 1H), 8.06 (d, *J =* 8.7 Hz, 1H), 7.97 (s, 1H), 7.72 (d, *J* = 8.7 Hz, 1H), 6.96 - 6.76 (m, 1H), 5.32 (s, 2H), 4.80 - 4.62 (m, 2H), 4.59 - 4.43 (m, 1H), 4.39 - 4.17 (m, 2H), 3.59 - 3.44 (m, 3H), 3.28 - 3.19 (m, 1H), 3.08 - 2.91 (m, 3H), 2.85 - 2.77 (m, 1H), 2.70 - 2.57 (m, 3H), 2.44 (s, 3H), 2.40 - 2.26 (m, 3H), 2.02 - 1.88 (m, 1H), 1.65 - 1.48 (m, 1H), 1.17 (t*, J =* 7.5 Hz, 3H). |
| 157 | 828.6 [M+H] ⁺ | ¹H NMR (400 MHz, DMSO-d₆) δ 10.36 (s, 1H), 8.56 (s, 1H), 8.07 (d, *J =* 8.6 Hz, 1H), 7.96 (s, 1H), 7.75 - 7.68 (m, 1H), 6.85 - 6.77 (m, 1H), 5.33 (s, 2H), 4.58 - 4.48 (m, 1H), 3.77 - 3.71 (m, 2H), 3.66 - 3.58 (m, 2H), 3.55 - 3.47 (m, 3H), 3.27 - 3.25 (m, 1H), 3.22 - 3.18 (m, 2H), 3.05 - 2.93 (m, 3H), 2.87 - 2.78 (m, 1H), 2.69 - 2.61 (m, 1H), 2.61 - 2.54 (m, 2H), 2.45 (s, 3H), 1.25 - 1.16 (m, 15H). |
| 158 | 798.5 [M+H] ⁺ | ¹H NMR (400 MHz, DMSO-d₆) δ 10.36 (s, 1H), 8.53 (s, 1H), 8.06 (d, *J =* 8.6 Hz, 1H), 7.98 - 7.94 (m, 1H), 7.75 - 7.66 (m, 1H), 6.82 - 6.75 (m, 1H), 5.32 (s, 2H), 4.58 - 4.46 (m, 1H), 3.96 - 3.86 (m, 2H), 3.56 - 3.45 (m, 3H), 3.38 (t, *J =* 5.5 Hz, 2H), 3.30 - 3.27 (m, 4H), 3.26 - 3.20 (m, 1H), 3.04 - 2.92 (m, 3H), 2.86 - 2.78 (m, 1H), 2.69 - 2.62 (m, 1H), 2.59 - 2.54 (m, 2H), 2.43 (s, 3H), 1.79 - 1.71 (m, 4H), 1.19 (t, *J =* 7.4 Hz, 3H). |
| 159 | 820.5 [M+H] ⁺ | ¹H NMR (400 MHz, DMSO-d₆) δ 10.36 (s, 1H), 8.52 (s, 1H), 8.06 (d, *J =* 8.6 Hz, 1H), 7.96 (d, *J =* 2.1 Hz, 1H), 7.71 (dd, *J =* 8.7, 2.1 Hz, 1H), 6.82 - 6.70 (m, 1H), 5.32 (s, 2H), 4.58 - 4.47 (m, 1H), 4.36 (t, *J =* 13.0 Hz, 4H), 4.06 - 3.95 (m, 2H), 3.57 - 3.49 (m, 3H), 3.48 - 3.45 (m, 2H), 3.25 - 3.22 (m, 1H), 3.02 - 2.92 (m, 3H), 2.86 - 2.77 (m, 1H), 2.69 - 2.61 (m, 1H), 2.58 - 2.53 (m, 2H), 2.43 (s, 3H), 1.19 (t, *J=* 7.4 Hz, 3H). |
| 160 | 797.4 [M+H] ⁺ | ¹H NMR (400 MHz, DMSO-d₆) δ 10.36 (s, 1H), 8.53 (s, 1H), 8.06 (d, *J =* 8.6 Hz, 1H), 7.96 (d, *J* = 2.1 Hz, 1H), 7.71 (dd,*J* = 8.7, 2.1 Hz, 1H), 7.37 (s, 1H), 6.94 - 6.86 (m, 1H), 5.32 (s, 2H), 4.59 - 4.47 (m, 1H), 3.59 - 3.43 (m, 3H), 3.25 - 3.18 (m, 1H), 3.06 - 2.93 (m, 3H), 2.87 - 2.79 (m, 1H), 2.76 - 2.60 (m, 3H), 2.59 - 2.51 (m, 3H), 2.43 (s, 3H), 2.40 (s, 3H), 2.27 - 2.15 (m, 1H), 1.90 - 1.77 (m, 1H), 1.19 (t, *J =* 7.4 Hz, 3H). |
| 161 | 797.4 [M+H] ⁺ | ¹H NMR (400 MHz, DMSO-d₆) δ 10.36 (s, 1H), 8.56 - 8.30 (m, 1H), 8.06 (d, *J =* 8.6 Hz, 1H), 7.96 (d, *J =* 2.1 Hz, 1H), 7.71 (dd, *J* = 8.7, 2.1 Hz, 1H), 7.42 (s, 1H), 6.89 (s, 1H), 5.32 (s, 2H), 4.57 - 4.46 (m, 1H), 3.58 - 3.43 (m, 3H), 3.22 - 3.18 (m, 1H), 3.06 - 2.90 (m, 3H), 2.86 - 2.76 (m, 1H), 2.70 - 2.60 (m, 3H), 2.59 (s, 3H), 2.56 - 2.52 (m, 1H), 2.42 (s, 3H), 2.37 - 2.26 (m, 2H), 2.19 - 2.07 (m, 1H), 1.83 - 1.68 (m, 1H), 1.19 (t, *J* = 7.4 Hz, 3H). |
| 172 | 798.5 [M+H] ⁺ | ¹H NMR (400 MHz, CDCl₃) δ 12.24 (s, 1H), 9.09 (s, 1H), 8.48 (d, *J* = 8.7 Hz, 1H), 7.63 (s, 1H), 7.53 (d, *J =* 10.0 Hz, 1H), 6.94 (s, 1H), 5.10 (s, 2H), 4.79 (d, *J =* 9.7 Hz, 1H), 3.80 (d, *J =* 17.4 Hz, 3H), 3.55 - 3.37 (m, 2H), 3.19 - 3.10 (m, 3H), 2.93 - 2.84 (m, 3H), 2.69 (s, 3H), 2.57 (d, *J =* 23.0 Hz, 2H), 2.27 - 2.22 (m, 1H), 2.02 (d, *J =* 14.4 Hz, 2H), 1.91 - 1.89 (m, 2H), 1.74 (d, *J =* 10.3 Hz, 3H), 1.57 - 1.49 (m, 1H), 1.37 -1.23 (m, 8H). |
| 173 | 798.5 [M+H] ⁺ | ¹H NMR (400 MHz, DMSO-*d*₆) δ 10.37 (s, 1H), 8.41 (d, *J =* 54.7 Hz, 1H), 8.06 (d, *J =* 8.6 Hz, 1H), 7.97 (d, *J =* 2.1 Hz, 1H), 7.71 (dd, *J =* 8.8, 2.1 Hz, 1H), 6.77 - 6.70 (m, 1H), 5.31 (s, 2H), 4.58 - 4.44 (m, 1H), 3.62 - 3.43 (m, 6H), 3.20 - 3.16 (m, 1H), 3.04 - 2.93 (m, 3H), 2.85 - 2.75 (m, 1H), 2.65 - 2.59 (m, 1H), 2.55 (d, *J* = 3.9 Hz, 2H), 2.47 - 2.44 (m, 1H), 2.41 (s, 3H), 2.20 - 1.90 (m, 4H), 1.71 - 1.61 (m, 3H), 1.60 - 1.36 (m, 5H), 1.18 (t, *J =* 7.4 Hz, 3H). |
| 181 | 726.4 [M+H] ⁺ | ¹H NMR (400 MHz, DMSO-d₆) δ 10.11 (s, 1H), 9.97 (s, 1H), 8.34 (s, 1H), 7.83 (d, *J =* 8.6 Hz, 1H), 7.73 (d, *J =* 2.1 Hz, 1H), 7.48 (dd, *J =* 8.9, 2.2 Hz, 1H), 6.69 - 6.61 (m, 1H), 5.08 (s, 2H), 4.35 - 4.21 (m, 1H), 3.36 - 3.17 (m, 3H), 3.04 - 2.96 (m, 1H), 2.84 - 2.70 (m, 3H), 2.62 - 2.54 (m, 1H), 2.46 - 2.38 (m, 1H), 2.21 (s, 3H), 1.92 - 1.83 (m, 3H), 1.72 - 1.64 (m, 1H), 1.26 (t, *J =* 6.1 Hz, 2H), 0.96 (t, *J =* 7.5 Hz, 3H), 0.16 - 0.05 (m, 4H). |
| 183 | 710.4 [M+H] ⁺ | ¹H NMR (400 MHz, DMSO-d₆) δ 10.22 (s, 1H), 8.55 (s, 1H), 7.42 - 7.30 (m, 2H), 6.85 - 6.77 (m, 1H), 5.24 (s, 2H), 4.72 (t, *J =* 8.8 Hz, 2H), 4.58 - 4.47 (m, 1H), 4.29 - 4.21 (m, 2H), 3.80 (t, *J =* 5.5 Hz, 2H), 3.59 - 3.42 (m, 3H), 3.27 - 3.18 (m, 3H), 3.04 - 2.91 (m, 3H), 2.87 - 2.77 (m, 1H), 2.69 - 2.57 (m, 1H), 2.50 (m, 2H), 2.44 (s, 3H), 1.18 (t, *J =* 7.4 Hz, 3H). |
| 184 | 710.2 [M+H]⁺ | ¹H NMR (400 MHz, DMSO-d₆) δ 10.35 (s, 1H), 8.56 (s, 1H), 7.92 (d, *J* = 8.5 Hz, 1H), 7.12 (d, *J =* 8.7 Hz, 1H), 6.81 (s, 1H), 5.26 (s, 2H), 4.75 (t, *J =* 8.8 Hz, 2H), 4.52 (d, *J =* 12.6 Hz, 1H), 4.25 (d, *J =* 2.4 Hz, 2H), 3.80 (dd, *J =* 7.1, 3.5 Hz, 2H), 3.59 - 3.54 - 3.44 (m, 3H), 3.42 - 3.37 (m, 2H), 3.23 - 3.16 (m, 1H), 3.04 - 2.90 (m, 3H), 2.81 (m, 1H), 2.65 (m, 1H), 2.5 (m, 2H), 2.44 (s, 3H), 1.16 (t*, J =* 7.4 Hz, 3H). |

### Bioassay Examples

### I. In vitro WRN enzymatic activity assay

This assay was used to establish an ATPase assay to measure the DNA-dependent ATP hydrolysis activity of WRN helicase. The assay was also used to evaluate the inhibitory properties of the compounds of the present disclosure on DNA-dependent WRN enzymatic activity.

### 1. Experimental materials

| Reagent | Manufacturer | Catalog No. |
|---|---|---|
| WRN protein (AA N517-P1238) | ICE | |
| ssDNA substrate | Sangon Biotech | |
| ATP, 100 mM Solution | Sangon Biotech | R0441 |
| ADP-Glo assay kit | Promega | V9102 |
| Pluronic F-127* | Sangon Biotech | A600750 |

### 2. Experimental methods

1) Compounds were serially diluted with DMSO in a 384 PP plate compound dilution plate (ensuring a starting concentration of 10 µM in the final reaction system, 9 concentrations, 1:3 serial dilution).
2) 0.1 µL of compound was transferred to a 384 reaction microplate (Greiner 784075) using Echo, ensuring a final DMSO content of 1% (in duplicate).
3) 2.5 µL of WRN enzyme solution was added to each well of the 384 reaction microplate, ensuring a final WRN enzyme concentration of 10 nM in the reaction system, and incubated at 25°C for 3 hours. The reaction buffer was 300 mM ATP, 30 mM Tris pH 7.5, 2 mM MgCl₂, 0.02% BSA, 50 mM NaCl, 0.1% pluronic F-127*.
4) 2.5 µL of single-stranded DNA substrate was added to each well, with the dilution buffer being the same as above, to a final substrate concentration of 0.2 nM, and incubated at 25°C for 30 minutes.
5) 5 µL of ADP-Glo reagent was added to the reaction system, followed by incubation at room temperature for 1 hour to remove the excess ATP from the reaction.
6) 10 µL of ATP Detection Reagent was added to the reaction system, followed by incubation at room temperature for 1 hour.
7) The chemiluminescent signal was measured using an EnVision microplate reader (PerkinElmer), and the inhibition rate of WRN enzymatic activity in each well was calculated.
8) Data analysis was performed using GraphPad Prism 8 software to calculate the IC₅₀ (nM) of the compounds of the present disclosure.

### 3. Experimental results

**Table 1**

| Example | ADP-Glo |
|---|---|
| 1 | 4.84 |
| 15 | 7.87 |
| 17 | 11.51 |
| 18 | 10.16 |
| 19 | 10.07 |
| 20 | 8.12 |
| 21 | 4.20 |
| 33 | 20.30 |
| 39 | 23.46 |
| 40 | 17.69 |
| 44 | 5.41 |
| 45 | 3.42 |
| 60 | 5.21 |
| 61 | 9.31 |
| 65 | 9.22 |
| 66 | 6.63 |
| 89 | 16.79 |
| 90 | 6.49 |
| 92 | 11.80 |
| 93 | 6.90 |
| 94 | 0.84 |
| 94-peak 1 | 4.96 |
| 94-peak 2 | 3.59 |
| 95 | 1.82 |
| 99 | 34.99 |
| 109 | 2.33 |
| 110 | 4.51 |
| 111 | 4.93 |
| 114 | 4.71 |
| 115 | 2.80 |
| 116 | 31.19 |
| 121 | 6.39 |
| 122 | 1.80 |
| 125 | 3.05 |
| 126 | 2.00 |
| 127 | 4.70 |
| 128 | 3.70 |
| 129 | 8.69 |
| 130 | 5.20 |
| 131 | 5.23 |
| 133 | 7.00 |
| 136 | 3.20 |
| 139 | 18.40 |
| 140 | 7.65 |
| 141 | 6.82 |
| 142 | 3.90 |
| 144 | 4.80 |
| 145 | 25.10 |
| 146 | 5.50 |
| 147 | 5.38 |
| 152-peak 1 | 3.22 |
| 152-peak 2 | 4.00 |
| 153 | 4.81 |
| 154 | 2.30 |
| 155 | 1.90 |
| 156 | 2.70 |
| 157 | 6.86 |
| 158 | 5.52 |
| 159 | 4.70 |
| 160 | 5.30 |
| 161 | 6.30 |
| 162 | 4.30 |
| 163 | 2.03 |
| 164 | 1.80 |
| 166 | 2.80 |
| 167 | 4.30 |
| 168 | 2.90 |
| 172 | 6.90 |
| 173 | 21.00 |
| 174 | 6.90 |
| 175 | 4.00 |
| 176 | 5.30 |
| 177 | 2.30 |
| 179 | 5.57 |
| 181 | 8.40 |
| 183 | 5.10 |
| 187 | 4.60 |
| 188 | 2.60 |
| 190 | 4.39 |
| 191 | 18.70 |
| 193 | 2.32 |
| 194 | 14.00 |
| 201 | 2.76 |
| 202 | 3.36 |
| 203 | 2.16 |
| 204 | 3.26 |
| 205 | 5.56 |
| 214 | 0.63 |
| 215 | 5.44 |
| 219 | 2.51 |
| 222 | 2.04 |
| 227 | 81.46 |
| 228 | 4.24 |
| 232 | 2.24 |
| 233 | 9.46 |
| 237 | 4.49 |
| 258 | 0.83 |
| 259 | 1.09 |
| 262 | 4.67 |
| 263 | 1.93 |
| 266 | 1.66 |
| 267 | 3.62 |
| 275 | 4.70 |
| 277 | 1.87 |
| 279 | 3.15 |
| 284 | 1.74 |
| 292 | 2.01 |
| 293 | 1.06 |
| 295-P3 | 2.53 |
| 295-P4 | 2.66 |
| 296-P2 | 0.98 |
| 296-P4 | 0.64 |
| 298 | 2.51 |
| 299 | 2.38 |
| 301 | 1.55 |
| 302 | 1.38 |
| 303-P1 | 2.19 |
| 303-P4 | 2.15 |
| 306-P3 | 2.49 |
| 306-P4 | 1.12 |
| 307-P3 | 2.29 |
| 307-P4 | 1.13 |
| 318 | 1.17 |
| 322-P3 | 1.02 |
| 322-P4 | 1.09 |
| 351 | 1.64 |
| 352 | 2.31 |
| 353 | 1.67 |
| 361-P4 | 1.16 |

The compounds of the examples of the present disclosure have good inhibitory activity against DNA-dependent WRN enzyme. The inhibitory activity IC₅₀ of the compounds of the present disclosure is less than 100 nM, the inhibitory activity IC₅₀ of some compounds is less than 50 nM, the inhibitory activity IC₅₀ of some compounds is less than 20 nM, and the inhibitory activity IC₅₀ of some compounds is less than 10 nM.

### II. Cell proliferation inhibition assay

In this assay, the inhibitory effects of the compounds on the proliferation of the relevant MSI/MSS cell lines was detected by measuring the intracellular ATP content using the chemiluminescence method (CellTiter-Glo), and the IC₅₀ values of the compounds for inhibiting the proliferation of the above cell lines were obtained.

### 1. Experimental materials

RPMI-1640 medium, DMEM medium, McCoy's 5a medium, Leibovitz's L-15 medium, fetal bovine serum (FBS), 100X Pen/Strep, and GlutaMAX-I Supplement were purchased from GIBCO; RKO, SW48, HCT116, LoVo, RL95-2, IM95 (MSI), and SW620 (MSS) cell lines were purchased from Nanjing Cobioer Biotechnology Co., Ltd.; the HCT116_WRN-KO cell line was provided by ICE; Cell Titer-Glo luminescent cell viability assay reagent was purchased from Promega.

### 2. Experimental methods

1) Day 0: Cells were seeded into a 384-well culture plate at 50 µL per well and incubated overnight in an incubator (37°C, 5% CO₂).
2) Day 1: 100 nL of cell culture medium containing serially diluted test compounds (starting concentration of 10 µM, 9 concentrations, 1:3 serial dilution) was added to the cells in the culture plate using D300e (TECAN), with a final DMSO concentration of 0.2%. The culture plate was incubated in a cell culture incubator for 120 hours (37°C, 5% CO₂), and a blank control was set by adding 100 nL of DMSO to each well.
3) Day 5: 20 µL of Cell Titer-Glo reagent was added to each well, shaken at 500 rpm for 10 minutes, and incubated at room temperature in the dark for 10 minutes to stabilize the luminescent signal. The luminescent signal was then detected using an Envision microplate reader (PerkinElmer), and data analysis was performed using GraphPad Prism 8 software to calculate the IC₅₀ (nM) of the compounds.

### 3. Experimental results

**Table 2**

| Example | SW48 (CRC, MSI) | SW620 (CRC, MSS) | HCT116 (WRN-KO) |
|---|---|---|---|
| 17 | 37.00 | - | - |
| 18 | 25.00 | - | - |
| 19 | 30.00 | >10000 | >10000 |
| 20 | 50.88 | >10000 | - |
| 21 | 45.6 | >10000 | - |
| 60 | 50.57 | >10000 | - |
| 61 | 43.29 | >10000 | - |
| 66 | 50.79 | >10000 | - |
| 92 | 52.50 | >10000 | - |
| 93 | 38.04 | >10000 | - |
| 94 | 27.74 | >10000 | - |
| 94 | 25.80 | >10000 | - |
| 94-peak 1 | 30.47 | >10000 | - |
| 94-peak 2 | 52.17 | >10000 | - |
| 110 | 54.73 | >10000 | - |
| 114 | 59.61 | >10000 | - |
| 114 | 47.06 | >10000 | - |
| 115 | 33.40 | >10000 | - |
| 121 | 77.46 | >10000 | - |
| 125 | 52.05 | >10000 | - |
| 126 | 53.10 | >10000 | - |
| 127 | 50.31 | >10000 | - |
| 129 | 72.52 | >10000 | - |
| 130 | 82.70 | >10000 | - |
| 140 | 51.79 | >10000 | - |
| 141 | 32.21 | >10000 | - |
| 142 | 79.00 | >10000 | - |
| 152-peak 1 | 30.57 | >10000 | - |
| 152-peak 2 | 76.30 | >10000 | - |
| 153 | 59.79 | >10000 | - |
| 154 | 28.40 | >10000 | - |
| 155 | 42.50 | >10000 | - |
| 156 | 43.60 | >10000 | - |
| 157 | 48.33 | >10000 | - |
| 158 | 38.70 | >10000 | - |
| 159 | 45.20 | >10000 | - |
| 160 | 92.70 | >10000 | - |
| 162 | 46.10 | >10000 | - |
| 163 | 33.55 | >10000 | - |
| 164 | 17.00 | >10000 | - |
| 166 | 70.30 | >10000 | - |
| 167 | 53.70 | >10000 | - |
| 168 | 69.80 | >10000 | - |
| 172 | 78.20 | >10000 | - |
| 175 | 42.90 | >10000 | - |
| 177 | 58.90 | >10000 | - |
| 187 | 76.30 | >10000 | - |
| 188 | 16.00 | >10000 | - |
| 190 | 43.43 | >10000 | - |
| 193 | 46.48 | >10000 | - |
| 194 | 49.80 | 8091 | - |
| 202 | 34.06 | >10000 | - |
| 203 | 42.86 | >10000 | - |
| 214 | 44.30 | >10000 | - |
| 228 | 26.98 | >10000 | - |
| 232 | 48.79 | >10000 | - |
| 258 | 26.82 | >10000 | - |
| 259 | 48.48 | >10000 | - |
| 267 | 76.02 | >10000 | - |
| 275 | 49.50 | >10000 | - |
| 279 | 44.04 | >10000 | - |
| 284 | 32.60 | >10000 | - |
| 292 | 48.55 | > 10000 | - |
| 293 | 10.00 | > 10000 | - |
| 295 | 18.00 | - | - |
| 295-P3 | 6.89 | >10000 | - |
| 295-P4 | 7.29 | > 10000 | - |
| 296-P2 | 17.04 | >10000 | - |
| 296-P4 | 8.95 | >10000 | - |
| 299 | 68.25 | > 10000 | - |
| 301 | 67.77 | >10000 | - |
| 302 | 7.35 | >10000 | - |
| 303-P1 | 4.8 | > 10000 | - |
| 303-P4 | 4.47 | > 10000 | - |
| 306-P4 | 18.43 | > 10000 | - |
| 307-P4 | 9.33 | > 10000 | - |
| 318 | 17.40 | >10000 | - |
| 322-P3 | 14.20 | >10000 | - |
| 322-P4 | 8.20 | >10000 | - |
| 342 | 477.00 | - | - |
| 344 | 829.00 | - | - |
| 345 | 107.00 | - | - |
| 348 | 95.00 | - | - |
| 351 | 83.19 | >10000 | - |
| 371 | 441.00 | - | - |
| 373 | 134.50 | - | - |

The compounds of the examples of the present disclosure have good inhibitory activity on cells and are selective.

### III. Whole-cell manual patch clamp assay

In this assay, the electrophysiological whole-cell manual voltage clamp method was used to test the effect of the compounds on the hERG potassium channel (human Ether-a-go-go Related Gene potassium channel) current.

### Experimental materials:

Cisapride, sodium chloride, potassium chloride, magnesium chloride, calcium chloride, glucose, HEPES, EGTA, and DMSO were purchased from Sigma; the CHO-hERG cell line (Chinese Hamster Ovary) (Chinese hamster ovary cells stably expressing the hERG channel) was constructed internally by the Ion Channel Research Platform of the Shanghai Institute of Materia Medica, Chinese Academy of Sciences.

### Experimental methods

Cell culture and treatment: CHO cells stably expressing hERG were cultured in 35-mm-diameter cell culture dishes, placed in an incubator at 37°C with 5% CO₂, and passaged every 48 hours at a ratio of 1:5. The culture medium formulation: 90% F12 (Invitrogen), 10% fetal bovine serum (Gibco), 100 g/mL G418 (Invitrogen), and 100 µg/mL Hygromycin B (Invitrogen). On the day of the experiment, the cell culture medium was aspirated, the cells were rinsed once with extracellular fluid, and then 0.25% Trypsin-EDTA (Invitrogen) solution was added, followed by digestion at room temperature for 3-5 minutes. The digestion solution was aspirated, and the cells were resuspended in extracellular fluid, and then transferred to experimental dishes for electrophysiological recording for later use.

Compound preparation: On the day of testing, the compounds were prepared as 20 mM stock solutions in DMSO. The stock solutions were then subjected to 3-fold serial dilutions with DMSO and subsequently diluted with extracellular solution to obtain the desired final test concentrations. Preparation of positive control compound cisapride: 10 µL of 150 µM cisapride DMSO stock solution was taken and added to 4990 µL of extracellular fluid, diluted 500-fold to obtain the desired final test concentration of 300 nM. The DMSO content in the final test concentration did not exceed 0.2%, and this concentration of DMSO had no effect on the hERG potassium channel.

Electrophysiological recording process: CHO cells stably expressing the hERG potassium channel were used to record hERG potassium channel currents at room temperature using the whole-cell voltage-clamp technique. Glass microelectrodes were fabricated from glass electrode blanks (BF150-86-10, Sutter) using a puller. After filling with electrode internal solution, the tip resistance was approximately 2-5 MΩ. The glass microelectrodes were inserted into the amplifier headstage to connect to the patch-clamp amplifier. The clamping voltage and data recording were controlled and recorded by pClamp software via a computer, with a sampling frequency of 10 kHz and a filter frequency of 2 kHz. After obtaining the whole-cell recording, the cells were clamped at -100 mV. The hERG potassium current (I hERG) was elicited by a step voltage protocol: a 2-second depolarizing voltage from -100 mV to +20 mV, followed by repolarization to -50 mV for 1 second, and then returned to - 100 mV. This voltage stimulus was applied every 5 seconds. After the hERG potassium current was confirmed to be stable (1 minute), the drug administration process was initiated. Each test concentration of the compound was applied for at least 1 minute until a steady-state effect was achieved, or for a maximum of 3 minutes. At least two cells were tested for each concentration (n ≥ 2).

Data analysis and processing were conducted using pClamp, GraphPad Prism 8, and Excel software. The degree of inhibition of hERG potassium current (peak of hERG tail current evoked at -50 mV) by different compound concentrations was calculated using the following formula: Inhibition % = [1 - (I / Io)] × 100%, wherein Inhibition % represents the percentage inhibition of hERG potassium current by the compound, and I and Io represent the amplitudes of hERG potassium current after and before drug administration, respectively.

The IC₅₀ of the compound was calculated by fitting using GraphPad Prism 8 software with the following formula: Y = Bottom + (Top - Bottom) / (1 + 10^((LogIC₅₀ - X) * HillSlope))
where X represents the Log value of the detected concentration of the test article, Y represents the percentage inhibition at the corresponding concentration, and Bottom and Top represent the minimum and maximum percentage inhibition, respectively.

IC₅₀ values of some compounds of the present disclosure for inhibition of hERG potassium channel current.

**Table 3**

| Example No. | IC₅₀ (µM) |
|---|---|
| Control molecule 2 | 32.9 |
| 295-P4 | >100 |
| 303-P4 | 62.1 |

The compounds of the examples of the present disclosure exhibit low inhibition rates on the hERG channel and have high safety.

### IV. Liver microsomal stability of the compounds of the present disclosure

The purpose of this test was to study the *in vitro* metabolic stability of the test articles in liver microsomes from different species. The concentration of the parent drug in the incubation system was detected using LC/MS/MS, and the intrinsic clearance rate of the test compound in the microsomal system was calculated to evaluate its stability.

### 1. Experimental materials

Human, monkey, dog, rat, and mouse liver microsomes were purchased from BD Gentest at a concentration of 20 mg/mL; potassium phosphate buffer, 100 mM; NADPH (nicotinamide adenine dinucleotide phosphate), 1 mM; stop solution, acetonitrile (containing 100 nM alprazolam, 200 nM caffeine, and 100 nM tolbutamide as internal standards).

### 2. Experimental methods

1) Liver microsomes were diluted with 100 mM potassium phosphate buffer to an appropriate concentration of microsome working solution. A certain amount of NADPH was added to the above microsome working solution, and then an appropriate amount of the test article was added to initiate the reaction, so that the final incubation system had a compound concentration of 1 µM, and the concentrations of NADPH and liver microsomes were 1 mM and 0.5 mg/mL, respectively.
2) At time points of 0.5, 5, 15, 30, and 60 minutes, 30 µL of the incubation sample was taken. 150 µL of acetonitrile containing internal standard was added to terminate the reaction, and the mixture was vortexed for 10 minutes.
3) Subsequently, the mixture was centrifuged at 3220 g for 40 minutes for protein precipitation. 100 µL of the supernatant was transferred to a sample injection plate, and 100 µL of pure water was added and mixed well for UPLC-MS/MS analysis.
4) Data analysis was performed using Microsoft Excel software. The *in vitro* half-life (t_{1/2}) and *in vitro* clearance rate of the parent drug were calculated by linear fitting of the natural logarithm of the percentage of parent drug eliminated versus time.

Conclusion: The compounds of the present disclosure exhibit good metabolic stability in liver microsomes.

### V. Evaluation of hepatocyte stability of the compounds of the present disclosure

The purpose of this test was to study the *in vitro* metabolic stability of the test articles in hepatocytes from different species. The concentration of the parent drug in the incubation system was detected using LC/MS/MS, and the intrinsic clearance rate of the test compound in the hepatocyte system was calculated to evaluate its stability.

### 1. Experimental materials

Human, dog, rat, and mouse hepatocytes were purchased from IVT, and monkey hepatocytes were purchased from RILD. Stop solution, acetonitrile (containing 100 nM alprazolam, 200 nM caffeine, and 100 nM tolbutamide as internal standards).

### 2. Experimental methods

1) The hepatocyte culture medium (William's E medium supplemented with GlutaMAX) and the hepatocyte thawing medium were placed in a 37°C water bath and heated for at least 15 minutes before use. The hepatocytes were transferred from liquid nitrogen, placed in a 37°C water bath, and the vial was gently shaken for 2 minutes to thaw the cells. After thawing was completed, the hepatocytes were transferred to a biosafety cabinet. The hepatocytes were transferred to a 50 mL centrifuge tube containing the thawing medium. The 50 mL centrifuge tube was placed in a centrifuge and centrifuged at 100 g for 10 minutes. The thawing medium was aspirated, and the hepatocytes were resuspended in sufficient culture medium. The cells were counted, and the viable cell density was determined. The cells were diluted with culture medium to a working cell density of 0.5×10⁶ viable cells/mL.
2) 198 µL of hepatocytes were transferred to each well of a 96-well uncoated plate. The plate was placed in an incubator, and the hepatocytes were pre-warmed for 10 minutes. 2 µL of 100 µM test compound or positive control was transferred to the corresponding wells of the 96-well plate to initiate the reaction.
3) At time points of 0.5, 15, 30, 60, 90, and 120 minutes, 25 µL of the incubation sample was taken and mixed with 300 µL of acetonitrile containing internal standards to terminate the reaction. The mixture was vortexed for 5 minutes. The samples were centrifuged at 3,220 g for 45 minutes. 100 µL of the supernatant was transferred to a sample injection plate, and 100 µL of pure water was added and mixed well for UPLC-MS/MS analysis.
4) Data analysis was performed using Microsoft Excel software. The *in vitro* half-life (t_{1/2}) and *in vitro* clearance rate of the parent drug were calculated by linear fitting of the natural logarithm of the percentage of parent drug eliminated versus time.

Conclusion: The compounds of the present disclosure exhibit excellent metabolic stability in hepatocytes.

### VI. Evaluation of the plasma protein binding rate of the compounds of the present disclosure

### 1. Experimental purpose

The purpose of this test was to evaluate the protein binding rate of the test drug in plasma of human, monkey, dog, rat and mouse using the equilibrium dialysis method.

### 2. Experimental materials

Plasma (EDTA-K2 anticoagulated) was stored in a -80°C freezer. Stop solution, acetonitrile (containing 100 nM alprazolam, 200 nM caffeine, and 100 nM tolbutamide as internal standards).

### 3. Experimental methods

The test article and the control compound ketoconazole were prepared as high-concentration DMSO stock solutions and diluted to 1 mM working solution, with a final test concentration of 5 µM.

NaH₂PO₄ (12.0 g) and NaCl (8.77 g) were weighed and dissolved in 1 L of water to prepare an alkaline solution. Na₂HPO₄ (14.2 g) and NaCl (8.77 g) were weighed and dissolved in 1 L of water to prepare an acidic solution. The alkaline solution and the acidic solution were titrated to pH 7.4.

The temperatures of the water bath and incubator were pre-adjusted to 37°C. The plasma was removed from -80°C, rapidly thawed in a 37°C water bath, and centrifuged at 2000 g for 5 minutes at room temperature. The clot was removed, and the supernatant was transferred to a new centrifuge tube. The pH of the plasma was tested and recorded. (Note: a. Only plasma with a pH range of 7.2-7.8 was used. b. Only plasma that had been thawed no more than twice after receipt was used.)

The equilibrium dialysis membrane was soaked in pure water for 60 minutes, then in 20% ethanol for 20 minutes, and finally in dialysis buffer for 20 minutes. The soaked dialysis membrane was then spread flat on a clean HTDialysis clamping device, and the dialysis device was assembled according to the product manual.

3 µL of the working solution of the test drug and the control drug was taken and added to 597 µL of pre-incubated plasma, and mixed thoroughly. The final organic solvent content in the incubation system was 0.5%. Immediately after mixing, 50 µL of the incubation system was transferred to a new 96-well plate as the time-0 sample, which was processed in the same manner as the incubated samples.

120 µL of the incubation system was added to each well of the dialysis device, and an equal volume of dialysis buffer (PBS) was added to the opposite side. All samples were prepared in duplicate. The dialysis plate was sealed and placed in an incubator at 37°C with 5% CO₂, and equilibrated at 100 rpm for 6 hours. After incubation, 50 µL of sample was collected from both side of the dialysis plate for processing.

The remaining incubation system was also incubated under the same conditions for 6 hours. After incubation, 50 µL of the incubation system was transferred for processing to test the stability of the test drug. The stability samples were processed in the same manner as the dialysis incubation samples.

50 µL of blank plasma was added to the collected buffer sample, and an equal volume of blank buffer was similarly added to the collected plasma sample. After thorough mixing, 400 µL of stop solution (acetonitrile containing internal standards) was added. All samples were vortexed for 10 minutes and then centrifuged at 3220 g for 30 minutes to precipitate proteins. 120 µL of the supernatant was transferred to a sample injection plate, and 120 µL of pure water was added and mixed well for UPLC-MS/MS analysis.

### 4.Data analysis

All data calculations were performed using Microsoft Excel software. The concentrations of the drug in free samples and plasma samples were determined. The calculation formula was as follows: 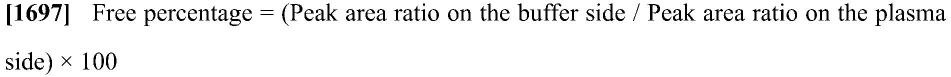 Free percentage = (Peak area ratio on the buffer side / Peak area ratio on the plasma side) × 100$Bound percentage = 100 - Free percentage$ 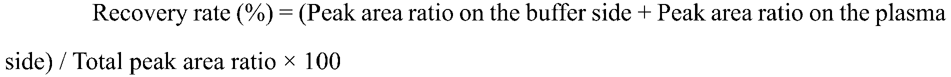 Recovery rate (%) = (Peak area ratio on the buffer side + Peak area ratio on the plasma side) / Total peak area ratio × 100$Remaining percentage = peak area ratio at 6 h / peak area ratio at 0 h \times 100$

**Table 4 Results of plasma protein binding rate of the compounds of the present disclosure in plasma**

| **Compound** | **Species** | Bound percentage | Free percentage | Recovery rate |
|---|---|---|---|---|
| | Human | 97.47 | 2.53 | 94.57 |
| 295-P4 | Monkey | 96.40 | 3.60 | 92.31 |
| | Dog | 91.91 | 8.09 | 90.05 |
| | Rat | 87.87 | 12.13 | 93.61 |
| | Mouse | 91.64 | 8.36 | 95.42 |

### VII. In vitro evaluation of the PXR activation potential of the test article in stably transfected DPX2 cells

### 1. Experimental purpose

The purpose of this study was to evaluate the potential of the test article to activate PXR *in vitro* and thereby induce drug-metabolizing enzymes.

### 2. Experimental materials

DPX2 cells (HepG2 cell line stably transfected with PXR and a luciferase-linked CYP3A4 promoter reporter gene) were purchased from Puracyp Inc (Carlsbad, CA); fetal bovine serum was purchased from Acantor.

### 3. Experimental methods

1) DPX2 cells were cultured using a culture medium containing 10% fetal bovine serum (FBS).
2) DPX2 cells were cultured in T-75 culture flask in a cell culture incubator at 37°C, 5% CO₂, and 95% relative humidity. When the cells reached 80-90% confluence, they were digested.
3) The culture medium was aspirated from the T-75 culture flask, and the cells were washed with 10 mL of PBS. The PBS was then aspirated, and 3-5 mL of trypsin was added. After incubation at 37°C for approximately 5 minutes, or until cell detachment and floating were observed, an excess volume of serum-containing culture medium was added to inactivate the trypsin.
4) The digested cells were transferred to a 50 mL centrifuge tube and centrifuged for 5 minutes (150 g) to pellet the cells. The cells were resuspended in seeding medium to a density of 3.2* 10⁵ cells/mL, and then 25 µL of the cell suspension was seeded into a 384-well cell culture plate. The 384-well plate was placed in a cell culture incubator, and after 24 hours, the PXR activation experiment was prepared.
5) DMSO stock solutions of the test compound, the positive control compound (rifampicin), and the negative control compound (propranolol) were prepared using Tecan at 1000-fold the incubation concentration. The final incubation concentration of the positive control compound (rifampicin) was 1 µM, 10 µM, or 20 µM.The final incubation concentration of the negative control compound (propranolol) was 10 µM. The final incubation concentration of the test compound was provided by the client. The final concentration of DMSO in the incubation solution was 0.1%.
6) The 384-well plate was removed from the cell culture incubator, and 25 nL of DMSO stock solutions containing the test compound and the control compounds were dispensed into the corresponding wells using Echo, with each experimental condition maintained in triplicate. The 384-well plate was returned to the cell culture incubator and incubated for 24 or 48 hours.
7) Before the start of the experiment, cell morphology and monolayer integrity were checked to ensure that the cell monolayer was of acceptable quality. After 24 or 48 hours of cell treatment, quantitative determination of PXR activation was performed.
8) The CellTiter Fluor^{™} cell viability assay kit and the One-Glo luciferase assay kit were brought to room temperature. GF-AFC substrate (10 µL) was transferred into the assay buffer (10 mL), and then 10 mL of PBS was added for 1:1 dilution to obtain the cell viability assay system. The One-Glo luciferase assay buffer was added to the One-Glo luciferase assay substrate to obtain the luciferase assay system.
9) The 384-well plate was removed from the cell culture incubator, and the culture medium was aspirated. 25 µL of the prepared cell viability assay system was gently added to each well, and then the plate was incubated at 37°C in the cell culture incubator for 30 minutes.
10) The 384-well plate was removed from the cell culture incubator, and the fluorescence value of each well was read using a microplate reader in fluorescence mode with excitation at 400 nm and emission at 505 nm. Subsequently, 25 µL of the prepared luciferase assay system was gently added to each well. After incubation with vortex shaking at room temperature for 5 minutes, the luminescence value of each well was read using a microplate reader.

### 4. Data analysis

### 1) Cell viability

The percentage of cell viability was calculated using the following formula: Cell viability (%) = Iₛₐₘₚₗₑ/I_{vehicle} × 100

In the formula: Iₛₐₘₚₗₑ is the average fluorescence intensity value in the test well; I_{vehicle} is the average fluorescence intensity value of cells treated with 0.1% DMSO.

### 2) Calculation of fold activation

All calculations were performed using Microsoft Excel software. The normalized luciferase activity was determined by RLU/RFU, wherein RLU represents the average of three parallel relative light units for each test compound at each dose, and RFU represents the average of three parallel relative fluorescence units for each test compound at each dose. The RLU and RFU of the vehicle were the averages of three parallel samples of cells treated with 0.1% DMSO. The fold activation at the mRNA level was calculated using the following formula: $Fold activation = \left({RLU}_{sample}/{RFU}_{sample}\right) / \left({RLU}_{vehicle}/{RFU}_{vehicle}\right)$

### 3) Calculation of the percentage of positive control

The percentage of positive control (%) was calculated using the following formula: 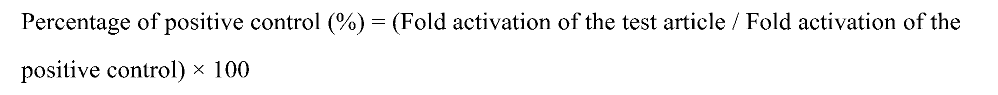 Percentage of positive control (%) = (Fold activation of the test article / Fold activation of the positive control) × 100

Fold activation values of some compounds of the present disclosure on PXR at 10 µM *in vitro.*

**Table 5**

| Example No. | Fold activation |
|---|---|
| 295-P4 | 1.44 |

Conclusion: The compounds of the present disclosure did not exhibit activation effect, indicating no significant activation effect on PXR.

### VIII. Pharmacokinetic determination of the compounds of the present disclosure in mice, rats, dogs, and monkeys in vivo

Using mice, rats, dogs, and monkeys as test animals, the compounds of the present disclosure were studied after intravenous bolus injection and oral administration. Plasma samples were collected at specific time points, and the concentrations of the compounds in plasma were detected by LC-MS/MS. PK parameters were calculated to reflect the pharmacokinetic behavior of the compounds of the present disclosure in mouse plasma *in vivo.*

### 1. Experimental scheme

### 1.1 Test drug:

Compounds of the present disclosure.

### 1.2 Test animals

Mice, balb/c nude, female, supplied by Jiangsu GemPharmatech Co., Ltd.

Mice, NOD SCID, female, supplied by Jiangsu GemPharmatech Co., Ltd.

Rats, SD, male, supplied by Jiangsu GemPharmatech Co., Ltd.

Beagle dogs, male, Beijing Marshall Biotechnology Co.,Ltd.

Cynomolgus monkeys, male, Beijing Anikeeper Biotech Co., Ltd.

### 1.3 Administration

Administration information for mouse: Both the IV (intravenous bolus injection) and PO (oral administration) groups consisted of 3 mice. The IV administration dose for mice was 1 mg/kg, with an administration volume of 5 mL/kg; the PO administration dose for mice was 10 mg/kg, with an administration volume of 10 mL/kg; the PO administration dose for mice was 20 mg/kg, with an administration volume of 20 mL/kg; and the dosing vehicle was 10 vol% DMSO/20 vol% Solutol/70 vol% saline.

Administration information for rat: The IV administration dose was 1 mg/kg, with an administration volume of 2 mL/kg; the PO administration dose was 5 mg/kg, with an administration volume of 10 mL/kg; and the vehicle was 10 vol% DMSO/20 vol% Solutol/70 vol% Saline.

Administration information for dog: The IV administration dose was 0.5 mg/kg, with an administration volume of 0.5 mL/kg; the IV administration dose was 1 mg/kg, with an administration volume of 0.5 mL/kg; the PO administration dose was 1 mg/kg, with an administration volume of 2 mL/kg; and the vehicle was 10 vol% DMSO/20 vol% Solutol/70 vol% Saline.

Administration information for monkey: The IV administration dose was 0.5 mg/kg, with an administration volume of 0.5 mL/kg; the PO administration dose was 5 mg/kg, with an administration volume of 3 mL/kg, and the vehicle was 10 vol% DMSO/20 vol% Solutol/70 vol% Saline.

### 1.4 Experimental equipment

The centrifuge was purchased from Eppendorf, and the pipette was purchased from Eppendorf.

### 1.5 Sample collection

After administration to mice, rats, dogs, and monkeys, blood was collected from the vein at 0.0833 (IV), 0.25, 0.5, 1, 2, 4, 8, and 24 hours, with volumes of 0.025, 0.2, 0.5, and 0.6 mL, respectively, placed into EDTA-K2 tubes, and centrifuged at 4°C and 2000 g for 10 min to separate plasma, which was stored at -80°C.

### 1.6 Sample processing

Mouse plasma sample processing:
1) 10 µL of plasma sample was added to 200 µL of acetonitrile for precipitation, vortexed to mix, and then centrifuged for 15 minutes.
2) The treated supernatant was diluted with water, and the concentration of the test compound was analyzed by LC/MS/MS.

Rat plasma sample processing:
1) 50 µL of plasma sample was added to 200 µL of acetonitrile for precipitation, vortexed to mix, and then centrifuged for 15 minutes.
2) The treated supernatant was diluted with water, and the concentration of the test compound was analyzed by LC/MS/MS.

Dog plasma sample processing:
1) 50 µL of plasma sample was added to 200 µL of acetonitrile for precipitation, vortexed to mix, and then centrifuged for 15 minutes.
2) The treated supernatant was diluted with water, and the concentration of the test compound was analyzed by LC/MS/MS.

Cynomolgus monkey plasma sample processing:
1) 50 µL of plasma sample was added to 200 µL of acetonitrile for precipitation, vortexed to mix, and then centrifuged for 15 minutes.
2) The treated supernatant was diluted with water, and the concentration of the test compound was analyzed by LC/MS/MS.

### 2. Experimental results

Pharmacokinetic parameters were calculated using WinNonlin 6.1, and the pharmacokinetic parameters are shown in the table below. Herein, Cₘₐₓ represents the maximum plasma concentration; CL represents clearance; Vss represents the volume of distribution at steady state; T_{1/2} represents the terminal elimination half-life; MRTInf represents the mean residence time; AUC represents the area under the plasma concentration-time curve; and F represents bioavailability.

**Table 6 Pharmacokinetic parameters of some compounds of the present disclosure after intravenous injection and oral administration in balb/c nude mice**

| Example No. | Route of administration | Dose | Cₘₐₓ | CL | T_{1/2} | AUC | F |
|---|---|---|---|---|---|---|---|
| | | (mg/kg) | (*ng*/*mL*) | (mL/h r/kg) | (hr) | (*h*ng*/ *mL*) | (%) |
| Control molecule 3 | IV | 1 | / | 1.16 | 1.16 | 1122 | / |
| | PO | 10 | 2473 | / | / | 4648 | 41.7 |
| 201 | IV | 1 | / | 0.84 | 1.34 | 1201 | / |
| | PO | 10 | 1264 | / | / | 4201 | 33.8 |
| 202 | IV | 1 | / | 0.47 | 1.33 | 2144 | / |
| | PO | 10 | 5407 | / | / | 11178 | 53.0 |
| 293 | IV | 1 | / | 0.68 | 1.12 | 1503, | / |
| | PO | 10 | 4657 | / | 1.60 | 10198 | 67.9 |
| 295-P4 | IV | 1 | / | 0.61 | 1.27 | 1652 | / |
| | PO | 10 | 3543 | / | 1.42 | 8266 | 51 |
| 296-P4 | IV | 1 | / | 0.59 | 1.22 | 1767 | / |
| | PO | 10 | 3010 | / | / | 6410 | 36.0 |
| 303-P4 | IV | 1 | / | 0.30 | 2.17 | 3528 | / |
| | PO | 10 | 4923 | / | 2.14 | 22224 | 60.8 |
| 322-P4 | IV | 1 | / | 0.63 | 1.37 | 1586 | / |
| | PO | 10 | 3257 | / | / | 8253 | 52.0 |
| 351 | IV | 1 | / | 0.76 | 1.03 | 1349 | / |
| | PO | 10 | 1153 | / | / | 1358 | 10.1 |

**Table 7 Pharmacokinetic parameters of some compounds of the present disclosure after intravenous injection and oral administration in NOD SCID mice**

| Example No. | Route of administration | Dose (mg/kg) | Cₘₐₓ (*ng*/*mL*) | AUC (*h*ng*/*mL*) |
|---|---|---|---|---|
| Control molecule 1 | PO | 20 | 7557 | 24247 |
| 202 | PO | 20 | 8547 | 48526 |

**Table 8 Pharmacokinetic parameters of some compounds of the present disclosure after oral administration to dogs**

| Example No. | Route of administration | Dose | Cₘₐₓ | CL | T_{1/2} | AUC | F |
|---|---|---|---|---|---|---|---|
| | | (mg/kg) | (*nM*) | (mL/hr/kg) | (hr) | (*hr*nM*) | (%) |
| 293 | IV | 0.5 | / | 0.36 | 4.05 | 1365 | / |
| | PO | 1 | 684 | / | 4.43 | 3388 | 127 |
| 295-P4 | IV | 0.5 | / | 0.46 | 2.89 | 1345 | / |
| | PO | 1 | 705 | / | 3.72 | 4199 | 111 |
| 303-P4 | IV | 0.5 | 0.34 | / | 6.49 | 1475 | / |
| | PO | 1 | 421 | / | 7.61 | 3097 | 107 |

| | | | | | | | |
|---|---|---|---|---|---|---|---|
| Note: "/" indicates not determined Results: As can be seen from the above table, the compounds of the present disclosure exhibit good pharmacokinetic properties *in vivo.* | | | | | | | |

### IX. In vivo pharmacodynamic study of the compounds in the SW48 xenograft tumor model

### 1. Experimental objective

To evaluate the *in vivo* efficacy of the compounds in a human colorectal cancer SW48 cell subcutaneous xenograft tumor mouse model.

### 2. Experimental materials

### 2.1 Experimental animals and housing environment

Species: Mouse
Strain: NU/NU mouse
Age and body weight: 6-8 weeks of age, weighing 20-24 g
Sex: Female
Quantity: 110 (including remaining mice from grouping)
Supplier: Beijing Vital River Laboratory Animal Technology Co., Ltd.
Animal certificate: 110011241105714757

### 2.2 Compounds

Compounds in the examples of the present disclosure.

### 3. Experimental methods and procedures

### 3.1 Cell culture

Human colon cancer SW48 cells (supplier: ECACC; catalog No.: 89012702) were cultured *in vitro* as a monolayer. The culture conditions were as follows: L-15 medium (supplier: Gibco; catalog No.: 11415-064) supplemented with 2 mM glutamine (supplier: Gibco; catalog No.: 25030-081), 10% fetal bovine serum (supplier: Excell; catalog No.: FSP500), and 1% antibiotics (supplier: Gibco; catalog No.: 15240-062) in a cell culture incubator at 37°C with 5% CO₂. Routine passaging was performed by digestion twice a week. When the cell confluence reached 80% to 90% and the cell number met the requirement, the cells were harvested, counted, and inoculated.

### 3.2 Tumor cell inoculation and grouping

A 0.1 mL cell suspension containing 3.0*10⁶ SW48 cells (the cells were suspended in medium L15:Matrigel =1:1) was subcutaneously inoculated into the right dorsal flank of each mouse. On Day 7 after inoculation, animals with uniform and appropriate tumor sizes were selected for enrollment, with the mean tumor volume reaching 117 mm³ (range: 89 mm³ - 161 mm³). The animals were assigned to groups according to tumor volume using a randomized block design, and dosing was initiated.

### Grouping and administration regimen of experimental animals

| **Group** | **N^{a}** | **Example** | **Dose (mg/kg)** | **Administration volume parameter (µL/g)^{b}** | **Route of administration** | **Cycle of administration** |
|---|---|---|---|---|---|---|
| 1 | 6 | Vehicle^{c} | - | 10 | p.o. | QD x 22D |
| 3 | 6 | 293 | 1 | 10 | p.o. | QD x 27D |
| 4 | 6 | 293 | 3 | 10 | p.o. | QD x 29D |
| 5 | 6 | 293 | 10 | 10 | p.o. | QD x 29D |
| 6 | 6 | 295-P4 | 3 | 10 | p.o. | QD x 29D |
| 7 | 6 | 295-P4 | 10 | 10 | p.o. | QD x 29D |
| 8 | 6 | 303-P4 | 0.3 | 10 | p.o. | QD x 29D |
| 9 | 6 | 303-P4 | 1 | 10 | p.o. | QD x 29D |
| 10 | 6 | 303-P4 | 3 | 10 | p.o. | QD x 29D |

| | | | | | | |
|---|---|---|---|---|---|---|
| Note: a. N: Number of mice per group; b. Administration volume: administered at 10 µL/g based on mouse body weight; if body weight loss exceeded 15%, the dosing regimen was adjusted accordingly; c. Blank control vehicle: 10% DMSO + 20% Solutol + 70% Saline. | | | | | | |

### 3.3 Preparation of test articles

| **Compound** | **Dose (mg/kg)** | **Preparation method** | **Concentration (mg/mL)** | **Storage condition** |
|---|---|---|---|---|
| Vehicle Control | -- | 10%DMSO + 20%Solutol + 70%Saline | -- | 2-8°C |
| 293 | 10 | Vehicle: 10% DMSO + 20% Solutol + 70% Saline | 1 | 2-8°C, prepared twice a week |
| | | 6.22 mg of compound 293 (purity: 96.50%) powder was weighed, added with 0.600 mL of DMSO, and dissolved by ultrasonic vibration. Subsequently, 1.200 mL of Solutol and 4.202 mL of Saline were added sequentially, and the mixture was shaken to homogeneity, yielding a clear solution. | | |
| 293 | 3 | Vehicle: 10% DMSO + 20% Solutol + 70% Saline | 0.3 | 2-8°C, prepared twice a week |
| | | 2.160 mL of 1 mg/mL solution of compound 293 was aspirated, and 5.040 mL of vehicle (10% DMSO + 20% Solutol + 70% Saline) was added. The mixture was shaken to homogeneity, yielding a clear solution. | | |
| 293 | 1 | Vehicle: 10% DMSO + 20% Solutol + 70% Saline | 0.1 | 2-8°C, prepared twice a week |
| | | 0.720 mL of 1 mg/mL solution of compound 293 was aspirated, and 6.480 mL of vehicle (10% DMSO + 20% Solutol + 70% Saline) was added. The mixture was shaken to homogeneity, yielding a clear solution. | | |
| 295-P4 | 3 | Vehicle: 10% DMSO + 20% Solutol + 70% Saline | 0.3 | 2-8°C, prepared twice a week |
| | | 2.160 mL of 1 mg/mL solution of compound 295-P4 was aspirated, and 5.040 mL of vehicle (10% DMSO + 20% Solutol + 70% Saline) was added. The mixture was shaken to homogeneity, yielding a clear solution. | | |
| 295-P4 | 10 | Vehicle: 10% DMSO + 20% Solutol + 70% Saline | 1 | 2-8°C, prepared twice a week |
| | | 6.22 mg of compound 295-P4 (purity: 97.18%) powder was weighed, added with 0.604 mL of DMSO, and dissolved by ultrasonic vibration. Subsequently, 1.209 mL of Solutol and 4.231 mL of Saline were added sequentially, and the mixture was shaken to homogeneity, yielding a clear solution. | | |
| 303-P4 | 3 | Vehicle: 10% DMSO + 20% Solutol + 70% Saline | 0.3 | 2-8°C, prepared twice a week |
| | | 3.21 mg of compound 303-P4 (purity: 94.30%) powder was weighed, added with 1.009 mL of DMSO, and dissolved by ultrasonic vibration. Subsequently, 2.018 mL of Solutol and 7.063 mL of Saline were added sequentially, and the mixture was shaken to homogeneity, yielding a clear solution. | | |
| 303-P4 | 1 | Vehicle: 10% DMSO + 20% Solutol + 70% Saline | 0.1 | 2-8°C, prepared twice a week |
| | | 2.400 mL of a 0.3 mg/mL solution of compound 303-P4 was aspirated, and 4.800 mL of vehicle (10% DMSO + 20% Solutol + 70% Saline) was added. The mixture was shaken to homogeneity, yielding a clear solution. | | |
| 303-P4 | 0.3 | Vehicle: 10% DMSO + 20% Solutol + 70% Saline | 0.03 | 2-8°C, prepared twice a week |
| | | 0.720 mL of a 0.3 mg/mL solution of compound 303-P4 was aspirated, and 6.480 mL of vehicle (10% DMSO + 20% Solutol + 70% Saline) was added. The mixture was shaken to homogeneity, yielding a clear solution. | | |

### 3.4 Daily observation of experimental animals

The health status and mortality of the animals were monitored daily. Routine inspections included observing the effects of tumor growth and drug treatment on the animals' daily behavioral performance, such as behavioral activity, food and water intake (visual inspection only), body weight changes (body weight was measured three times per week), appearance signs, or other abnormal conditions. Based on the number of animals in each group, the number of animal deaths and side effects within the group were recorded.

### 3.5 Tumor measurement and experimental indicators

Tumor diameters were measured three times per week using a vernier caliper. The formula for calculating tumor volume was: V = 0.5a × b², wherein a and b represent the long and short diameters of the tumor, respectively.

The anti-tumor efficacy of the compounds was evaluated by the tumor growth inhibition rate TGI (%). The TGI (%) was calculated as: TGI (%) = [1 - (mean tumor volume of a treatment group at the end of administration - mean tumor volume of the treatment group at the start of administration) / (mean tumor volume of the vehicle control group at the end of treatment - mean tumor volume of the vehicle control group at the start of treatment)] × 100%.

### 4. Experimental results and analysis

**Table 9**

| Example | **Group** *(N=6)* | **TGI** *(%) Day21* |
|---|---|---|
| | Vehicle | - |
| Example 293 | 1 mg/kg | 33.96 |
| Example 293 | 3 mg/kg | 74.46 |
| Example 293 | 10 mg/kg | 101.85 |
| Example 295-P4 | 3 mg/kg | 101.70 |
| Example 295-P4 | 10 mg/kg | 102.37 |
| Example 303-P4 | 0.3 mg/kg | 59.30 |
| Example 303-P4 | 1 mg/kg | 101.86 |
| Example 303-P4 | 3 mg/kg | 103.09 |

The experimental results showed that Example 293 at 10 mg/kg, Example 295-P4 at doses of 3 mg/kg and 10 mg/kg, and Example 303-P4 at doses of 1 mg/kg and 3 mg/kg all exhibited significant anti-tumor effects. During the treatment period, all treatment groups were well tolerated without significant body weight loss.

### IX. In vivo pharmacodynamic study of the compounds in the Ishikawa xenograft tumor model

### 1. Experimental objective

To evaluate the *in vivo* efficacy of the compounds in a human endometrial Ishikawa cell subcutaneous xenograft tumor mouse model.

### 2. Experimental materials

### 2.1 Experimental animals and housing environment

Species: Mouse
Strain: Balb/c Nude mouse
Age and body weight: 6-8 weeks of age, weighing 20-24 g
Sex: Female
Quantity: 115 (including remaining mice from grouping)
Supplier: Zhejiang Vital River Laboratory Animal Technology Co., Ltd.
Animal certificate: 20240822Abzz0619000609

### 2.2 Compound information

Compounds in the examples of the present disclosure.

### 3. Experimental methods and procedures

### 3.1 Cell culture

Human endometrial cancer Ishikawa cells (ECACC, 99040201) were cultured *in vitro* under the following conditions: DMEM medium supplemented with 10% fetal bovine serum, 1% antibiotics, 2 mM glutamine, 1% non-essential amino acids, 1 mM sodium pyruvate, and 1.5 g/L sodium bicarbonate, at 37°C in a 5% CO₂ cell incubator. Routine passaging was performed by centrifugation twice a week. When the cell confluence reached 80% to 90% and the cell number met the requirement, the cells were harvested, counted, and inoculated.

### 3.2 Tumor cell inoculation and grouping

A 0.2 mL cell suspension containing 1.0*10⁷ Ishikawa cells (the cells were suspended in medium DMEM:Matrigel = 1:1) was subcutaneously inoculated into the right dorsal flank of each mouse. On Day 15 after inoculation, 48 animals with uniform and appropriate tumor sizes were selected for enrollment, with the mean tumor volume reaching 125 mm³ (range: 101 mm³ - 159 mm³). The animals were assigned to groups according to tumor volume using a randomized block design (Table 10), and dosing was initiated.

**Table 10. Animal grouping and dosing regimen for pharmacodynamic study**

| **Group** | **Number of cases^{a}** | **Treatment** | **Dose (mg/kg)** | **Administration volume^{b} (mL/kg)** | **Route of administration** | **Frequency of administration** |
|---|---|---|---|---|---|---|
| **1** | 6 | Vehicle Control^{c} | - | 10 | p.o. | QD x 28D |
| **5** | 6 | 295-P4 | 0.3 | 10 | p.o. | QD x 28D |
| **6** | 6 | 295-P4 | 1 | 10 | p.o. | QD x 28D |
| **7** | 6 | 295-P4 | 3 | 10 | p.o. | QD x 28D |
| **8** | 6 | 295-P4 | 10 | 10 | p.o. | QD x 28D |

| | | | | | | |
|---|---|---|---|---|---|---|
| Note: d. N: Number of mice per group; e. Administration volume: administered at 10 µL/g based on mouse body weight; if body weight loss exceeded 15%, the dosing regimen was adjusted accordingly; f. Blank control vehicle: 10% DMSO + 20% Solutol + 70% Saline. | | | | | | |

### 3.3 Preparation of test articles

**Table 11. Preparation methods of test articles**

| **Compound** | **Dose (mg/kg)** | **Preparation method** | **Concentration (mg/mL)** | **Storage condition** |
|---|---|---|---|---|
| Vehicle Control | -- | 10% DMSO + 20% Solutol HS-15 + 70% Saline | -- | 2-8°C Prepared every three days |
| | | 3.000 mL of DMSO, 6.000 mL of Solutol HS-15, and 21.000 mL of Saline were respectively pipetted, and thoroughly mixed to obtain a clear solution. Solutol HS-15 was melted in advance. | | |
| 295-P4 | 10 | Vehicle: 10% DMSO + 20% Solutol HS-15 + 70% Saline | 1 | 2-8°C Prepared every three days |
| | | 11.44 mg of 295-P4 powder was weighed and dissolved in 1.098 mL of DMSO by sonication. Subsequently, 2.196 mL of Solutol HS-15 and 7.684 mL of Saline were added sequentially, and the mixture was sonicated to mix thoroughly, yielding a clear solution. | | |
| 295-P4 | 3 | Vehicle: 10% DMSO + 20% Solutol HS-15 + 70% Saline | 0.3 | 2-8°C Prepared every three days |
| | | 2.160 mL of 1 mg/mL 295-P4 solution was pipetted, 5.040 mL of vehicle was added, and the mixture was shaken to homogeneity, yielding a clear solution. | | |
| 295-P4 | 1 | Vehicle: 10% DMSO + 20% Solutol HS-15 + 70% Saline | 0.1 | 2-8°C Prepared every three days |
| | | 0.720 mL of 1 mg/mL 295-P4 solution was pipetted, 6.480 mL of vehicle was added, and the mixture was shaken to homogeneity, yielding a clear solution. | | |
| 295-P4 | 0.3 | Vehicle: 10% DMSO + 20% Solutol HS-15 + 70% Saline | 0.03 | 2-8°C Prepared every three days |
| | | 0.216 mL of 1 mg/mL 295-P4 solution was pipetted, 6.984 mL of vehicle was added, and the mixture was shaken to homogeneity, yielding a clear solution. | | |

### 3.4 Daily observation of experimental animals

The health status and mortality of the animals were monitored daily. Routine inspections included observing the effects of tumor growth and drug treatment on the animals' daily behavioral performance, such as behavioral activity, food and water intake (visual inspection only), body weight changes (body weight was measured three times per week), appearance signs, or other abnormal conditions. Based on the number of animals in each group, the number of animal deaths and side effects within the group were recorded.

### 3.5 Tumor measurement and experimental indicators

Tumor diameters were measured twice per week using a vernier caliper. The formula for calculating tumor volume was: V = 0.5a × b², wherein a and b represent the long and short diameters of the tumor, respectively.

The anti-tumor efficacy of the compounds was evaluated by the tumor growth inhibition rate TGI (%). The TGI (%) was calculated as: TGI (%) = [1 - (mean tumor volume of a treatment group at the end of administration - mean tumor volume of the treatment group at the start of administration) / (mean tumor volume of the vehicle control group at the end of treatment - mean tumor volume of the vehicle control group at the start of treatment)] × 100%.

At the end of the experiment, tumors were excised from the mice, weighed, and the tumor inhibition rate was calculated. The tumor weight inhibition rate (IR_{TW}) % = (1 - T_{weight} / C_{weight}) × 100%, where C_{weight} represents the mean tumor weight of the vehicle control group, and T_{weight} represents the mean tumor weight of each treatment group.

The experimental results showed that Example 295-P4 exhibited significant anti-tumor effects at doses of 1 mg/kg, 3 mg/kg, and 10 mg/kg. During the treatment period, all treatment groups were well tolerated without significant body weight loss.

The structures of the control molecules involved in the full text are as follows.

| Molecule | Control molecule 1 (i.e., HRO761) | Control molecule 2 | Control molecule 3 |
|---|---|---|---|
| Source | WO2022249060 | WO2024079623 | WO2022249060 |
| Structure | 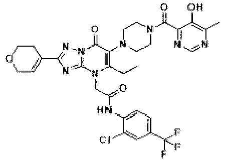 | 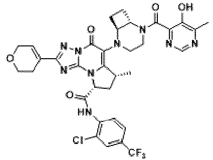 | 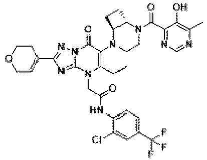 |

.

## Claims

1. A compound of formula (IC), a stereoisomer thereof, or a pharmaceutically acceptable salt thereof: 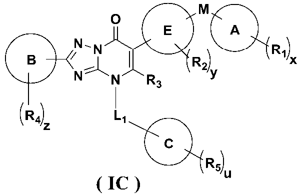 wherein
M is selected from the group consisting of a bond, CO, -NHCO-, -C(R₆₋₁R₆₋₂)-, and 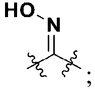
ring A is selected from the group consisting of C₆₋₁₄ aryl and 5- to 14-membered heteroaryl;
ring B is selected from the group consisting of C₃₋₁₂ cycloalkyl, C₃₋₁₂ cycloalkenyl, 3- to 12-membered heterocycloalkyl, 3- to 12-membered heterocycloalkenyl, C₆₋₁₄ aryl, and 5- to 14-membered heteroaryl; or, ring B is absent, and R₄ is directly attached to the parent structure;
ring C is selected from the group consisting of C₃₋₁₂ cycloalkyl, C₆₋₁₄ aryl, and 5- to 14-membered heteroaryl;
ring E is selected from the group consisting of C₃₋₁₂ cycloalkylene, C₃₋₁₂ cycloalkenylene, 3- to 12-membered heterocycloalkylene, 3- to 12-membered heterocycloalkenylene, C₆₋₁₄ arylene, and 5- to 14-membered heteroarylene; alternatively, ring E is directly attached to the parent to form 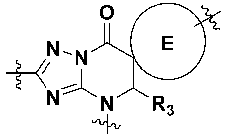 or 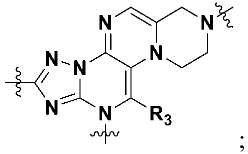
L₁ is selected from the group consisting of a bond, -O-, -S-, imino, C₁₋₆ alkylene, -CONH-, -CO-C₁₋₆ alkylene-NH-, -C₁₋₆ alkylene-CONH-, -C₁₋₆ alkylene-CONH-C₁₋₆ alkylene, -C₁₋₆ alkylene-SO₂NH-, -C₁₋₆ alkylene-NHCONH-, -C₁₋₆ alkylene-NHCO-, -C₁₋₆ alkylene-NHSO₂-, -C₁₋₆ alkylene-CO-, -C₁₋₆ alkylene-C(=N-OH)NH-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -C₁₋₆ alkylene-3- to 8-membered heterocycloalkylene-, -C₁₋₆ alkylene-3- to 8-membered heterocycloalkenylene-, -C₁₋₆ alkylene-C₆₋₁₄ arylene-, C₆₋₁₄ arylene, -C₁₋₆ alkylene-5- to 14-membered heteroarylene-, and 5- to 14-membered heteroarylene, wherein the imino, C₁₋₆ alkylene, -CONH-, -CO-C₁₋₆ alkylene-NH-, -C₁₋₆ alkylene-CONH-, -C₁₋₆ alkylene-CONH-C₁₋₆ alkylene, -C₁₋₆ alkylene-SO₂NH-, -C₁₋₆ alkylene-NHCONH-, -C₁₋₆ alkylene-NHCO-, -C₁₋₆ alkylene-NHSO₂-, -C₁₋₆ alkylene-CO-, C₂₋₆ alkenylene, C₂₋₆ alkynylene, C₁₋₆ deuterated alkylene, C₁₋₆ haloalkylene, C₁₋₆ hydroxyalkylene, C₁₋₆ alkyleneoxy, C₁₋₆ alkylenethio, C₁₋₆ haloalkyleneoxy, C₃₋₈ cycloalkylene, 3- to 8-membered heterocycloalkylene, 3- to 8-membered heterocycloalkenylene, -C₁₋₆ alkylene-3- to 8-membered heterocycloalkylene-, -C₁₋₆ alkylene-3- to 8-membered heterocycloalkenylene-, - C₁₋₆ alkylene-C₆₋₁₄ arylene-, C₆₋₁₄ arylene, -C₁₋₆ alkylene-5- to 14-membered heteroarylene-, or 5- to 14-membered heteroarylene is optionally further substituted with one or more substituents selected from the group consisting of deuterium, halogen, amino, hydroxyl, cyano, oxo, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5-to 14-membered heteroaryl;
alternatively, L₁, R₃, and the parent form a moiety 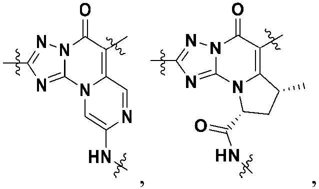 or 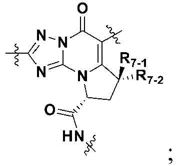
R₁ is independently selected from the group consisting of hydrogen, deuterium, halogen, amino, hydroxyl, mercapto, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₁ₐ; the R₁ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₂ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₂ₐ; the R₂ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
alternatively, two R₂ are connected to each other to form a C₃₋₈ cycloalkyl or a 3- to 8-membered heterocycloalkyl, wherein the C₃₋₈ cycloalkyl or 3- to 8-membered heterocycloalkyl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
alternatively, one R₂ and R₃ are connected to form a 5- to 8-membered heterocycloalkenyl, wherein the 5- to 8-membered heterocycloalkenyl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₃ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₃ₐ; the R₃ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
Li and R₃ are connected to form a 3- to 8-membered heterocycloalkenyl or a 5- to 10-membered heteroaryl; wherein the 3- to 8-membered heterocycloalkenyl or 5- to 10-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, 5- to 14-membered heteroaryl, -COR₄₋₁, -COOR₄₋₁, - OC(=O)R₄₋₁, -SO₂R₄₋₂, and -OR₄₋₃, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₄ₐ; the R₄ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl; alternatively, two R₄ₐ are connected to form a 3- to 8-membered heterocycloalkyl or a 3- to 8-membered heterocycloalkenyl;
R₄₋₁ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄₋₂ is selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₄₋₃ is selected from the group consisting of hydrogen, deuterium, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₃₋₈ cycloalkyl, 3- to 8-membered heterocycloalkyl, C₆₋₁₄ aryl, and 5- to 14-membered heteroaryl, wherein the C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₅ is independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, -SO₂-C₁₋₆ alkyl, -SF₅, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₅ₐ; the R₅ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
R₆₋₁ and R₆₋₂ are each independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more R₅ₐ; the R₅ₐ is independently selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, and 5- to 14-membered heteroaryl, wherein the amino, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, C₁₋₆ haloalkoxy, C₃₋₈ cycloalkyl, 3- to 8-membered heterocycloalkyl, C₆₋₁₄ aryl, or 5- to 14-membered heteroaryl is optionally further substituted with one or more substituents selected from the group consisting of deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3- to 8-membered heterocycloalkyl, C₆₋₁₄ aryl, and 5- to 14-membered heteroaryl;
alternatively, R₆₋₁ and R₆₋₂, together with the carbon atom to which they are attached, form a C₃₋₈ cycloalkyl or a 3- to 8-membered heterocycloalkyl;
R₇₋₁ and R₇₋₂ are each independently selected from the group consisting of hydrogen, deuterium, halogen, amino, hydroxyl, cyano, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ alkylthio, and C₁₋₆ haloalkoxy;
alternatively, R₇₋₁ and R₇₋₂ are connected to form a C₃₋₈ cycloalkyl;
x is selected from the group consisting of 0, 1, 2, and 3;
y is selected from the group consisting of 0, 1, 2, 3, and 4;
z is selected from the group consisting of 0, 1, 2, 3, and 4;
u is selected from the group consisting of 0, 1, 2, 3, and 4.

2. The compound, the stereoisomer thereof, or the pharmaceutically acceptable salt thereof according to claim 1, wherein ring A is selected from the group consisting of 5- to 6-membered heteroaryl, 5- to 6-membered heteroaryl-fused C₃₋₆ cycloalkenyl, 5- to 6-membered heteroaryl-fused 5- to 6-membered heteroaryl, 5- to 6-membered heteroaryl-fused 3- to 6-membered heterocycloalkenyl, and 5- to 6-membered heteroaryl-fused phenyl;
preferably, ring A is selected from the group consisting of

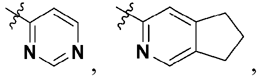

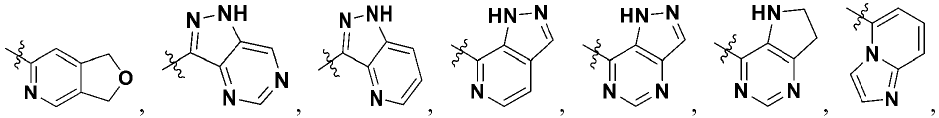

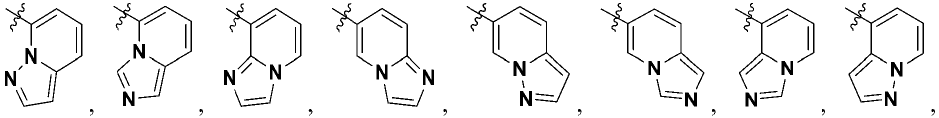

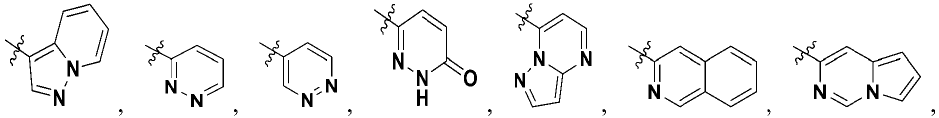

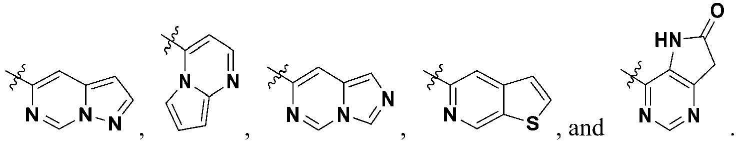

3. The compound, the stereoisomer thereof, or the pharmaceutically acceptable salt thereof according to claim 1 or 2, wherein ring B is selected from the group consisting of C₃₋₁₀ cycloalkenyl-spiro-3- to 10-membered heterocycloalkyl, 3- to 10-membered heterocycloalkenyl, 5- to 6-membered heteroaryl-fused 3- to 10-membered heterocycloalkenyl, 3- to 10-membered heterocycloalkenyl-fused 5- to 6-membered heteroaryl, 3- to 10-membered heterocycloalkenyl-spiro-3- to 10-membered heterocycloalkyl, 5- to 6-membered heteroaryl-fused 5- to 6-membered heteroaryl, benzo-fused C₃₋₁₀ cycloalkenyl, benzo-fused 3- to 10-membered heterocycloalkenyl, C₃₋₁₀ cycloalkenyl, 3- to 10-membered heterocycloalkenyl-fused phenyl, 5- to 6-membered heteroaryl, 3- to 10-membered heterocycloalkenyl-spiro-C₃₋₁₀ cycloalkyl, 3- to 10-membered heterocycloalkenyl-fused 3- to 10-membered heterocycloalkyl, benzo-fused 5- to 6-membered heteroaryl, C₃₋₁₀ cycloalkenyl-spiro-C₃₋₁₀ cycloalkyl, 3- to 10-membered heterocycloalkenyl-fused C₃₋₁₀ cycloalkyl, and 3- to 10-membered heterocycloalkyl;
preferably, ring B is selected from the group consisting of

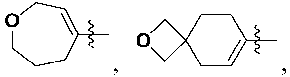

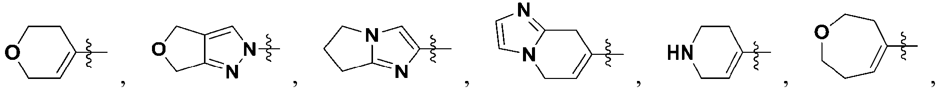

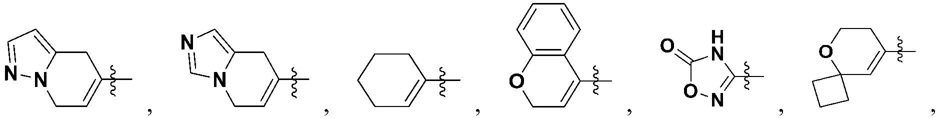

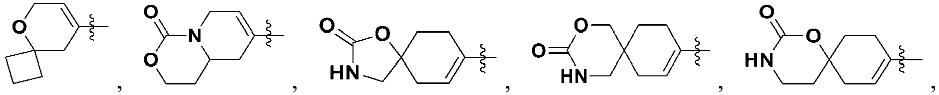

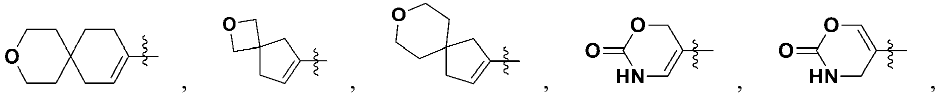

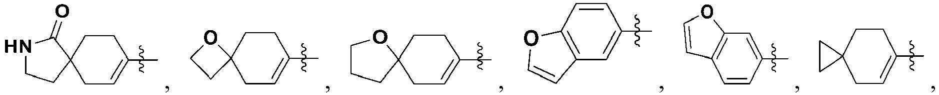

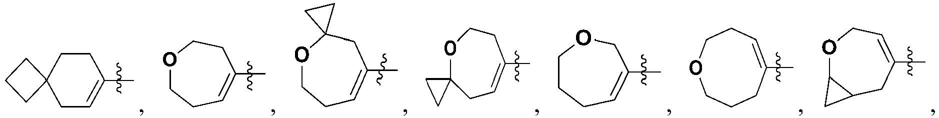

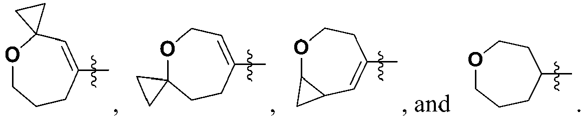

4. The compound, the stereoisomer thereof, or the pharmaceutically acceptable salt thereof according to any one of claims 1 to 3, wherein ring C is selected from the group consisting of C₆₋₁₀ aryl, 5- to 6-membered heteroaryl, 5- to 6-membered heteroaryl-fused 5- to 6-membered heteroaryl, benzo-fused 5- to 6-membered heteroaryl, benzo-fused 3- to 6-membered heterocycloalkenyl, 5- to 6-membered heteroaryl-fused phenyl, 3- to 6-membered heterocycloalkenyl-fused phenyl, 5- to 6-membered heteroaryl-fused C₃₋₆ cycloalkenyl, and C₃₋₈ cycloalkyl;
preferably, ring C is selected from the group consisting of 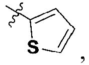 phenyl, 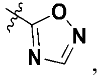 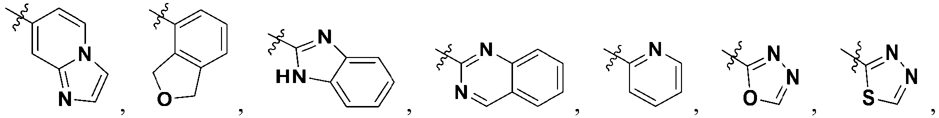 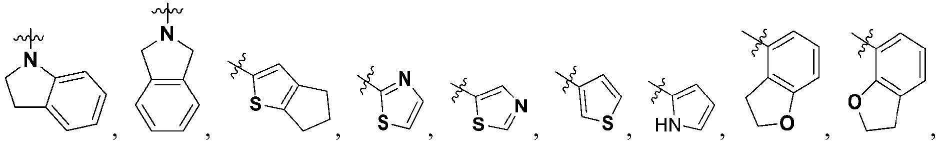 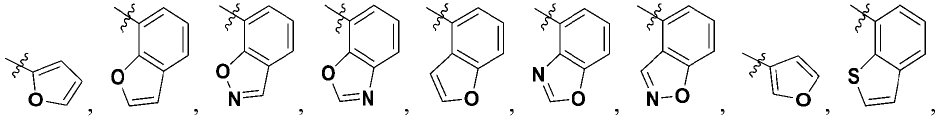 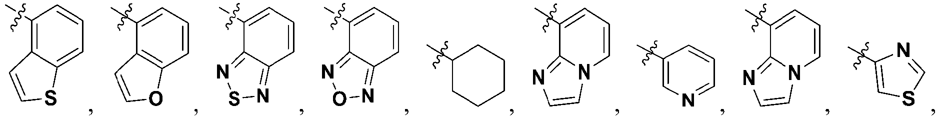 and

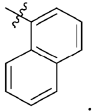

5. The compound, the stereoisomer thereof, or the pharmaceutically acceptable salt thereof according to any one of claims 1 to 4, wherein ring E is selected from the group consisting of 5- to 6-membered heterocycloalkylene, 4- to 6-membered heterocycloalkylene-spiro-4- to 6-membered heterocycloalkylene, 5- to 6-membered heterocycloalkylene-fused 5-to 6-membered heterocycloalkylene, 5- to 6-membered heteroaryl-fused 5- to 6-membered heterocycloalkenyl, and 5- to 6-membered heterocycloalkylene-fused C₃₋₆ cycloalkylene;
preferably, ring E is selected from the group consisting of 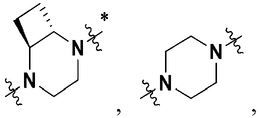 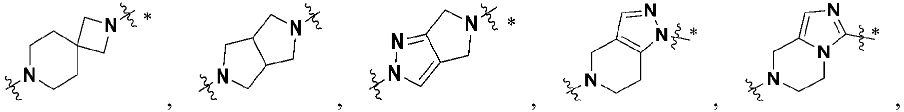 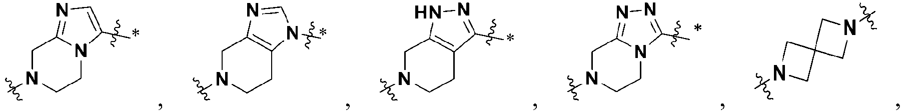 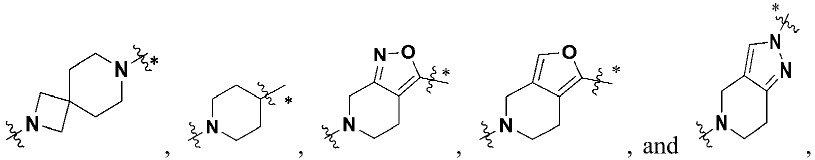 wherein the end with * is connected to M;
alternatively, ring E is directly connected to the parent to form

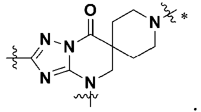

6. The compound, the stereoisomer thereof, or the pharmaceutically acceptable salt thereof according to any one of claims 1, 3, and 4, wherein the compound is represented by formula (XVI), 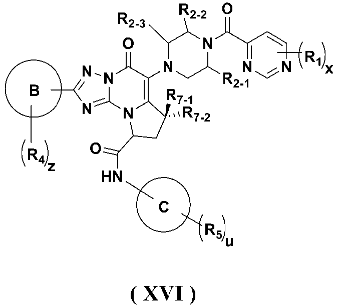
wherein R₂₋₁, R₂₋₂, and R₂₋₃ are each independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, and C₁₋₃ haloalkoxy; or, R₂₋₂ and R₂₋₃ are connected to form a C₃₋₆ cycloalkyl or a 3- to 6-membered heterocycloalkyl, wherein the C₃₋₆ cycloalkyl and 3- to 6-membered heterocycloalkyl are optionally further substituted with one or more substituents selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, and C₁₋₃ haloalkoxy;
R₇₋₁ and R₇₋₂ are each independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, and C₁₋₃ haloalkoxy;
alternatively, R₇₋₁ and R₇₋₂ are connected to form a C₃₋₆ cycloalkyl.

7. The compound, the stereoisomer thereof, or the pharmaceutically acceptable salt thereof according to claim 6, wherein the compound is represented by formula (XVI),

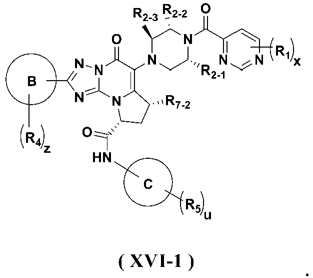

8. The compound, the stereoisomer thereof, or the pharmaceutically acceptable salt thereof according to claim 6 or 7, wherein
the structural moiety 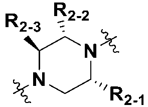 is

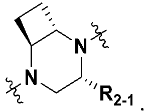

9. The compound, the stereoisomer thereof, or the pharmaceutically acceptable salt thereof according to any one of claims 6 to 8, wherein 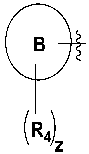 is 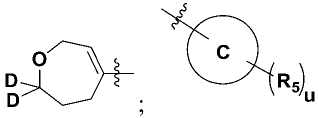 is 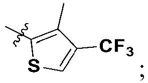 or, 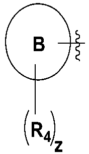 is 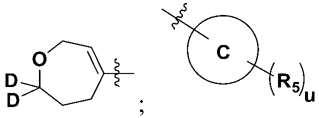 is 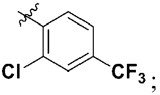 or, 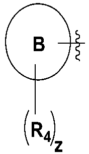 is 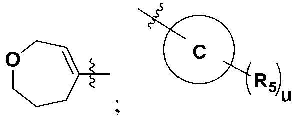 is 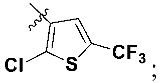 or, 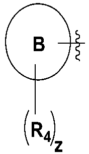 is 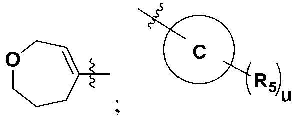 is

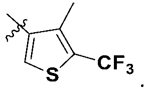

10. The compound, the stereoisomer thereof, or the pharmaceutically acceptable salt thereof according to claim 1, wherein the compound is represented by formula (II), 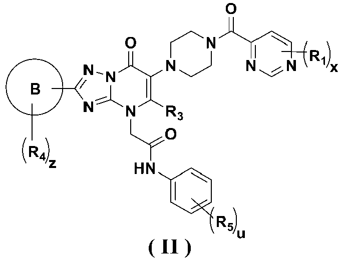 wherein
ring B is selected from the group consisting of C₃₋₁₀ cycloalkenyl-spiro-3- to 10-membered heterocycloalkyl, 5- to 6-membered heteroaryl-fused 3- to 10-membered heterocycloalkenyl, 3- to 10-membered heterocycloalkenyl-fused 5- to 6-membered heteroaryl, 3- to 10-membered heterocycloalkenyl-spiro-3- to 10-membered heterocycloalkyl, 5- to 6-membered heteroaryl-fused 5- to 6-membered heteroaryl, 7- to 10-membered heterocycloalkenyl, C₃₋₁₀ cycloalkenyl, 3- to 10-membered heterocycloalkenyl-fused phenyl, 5- to 6-membered heteroaryl, 3- to 10-membered heterocycloalkenyl-spiro-C₃₋₁₀ cycloalkyl, 3-to 10-membered heterocycloalkenyl-fused 3- to 10-membered heterocycloalkyl, 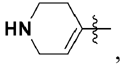 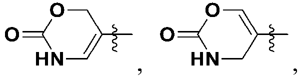 benzo-fused 5- to 6-membered heteroaryl, C₃₋₁₀ cycloalkenyl-spiro-C₃₋₁₀ cycloalkyl, 3- to 10-membered heterocycloalkenyl-fused C₃₋₁₀ cycloalkyl, and 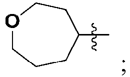 preferably 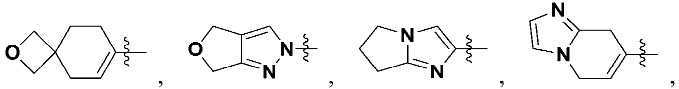 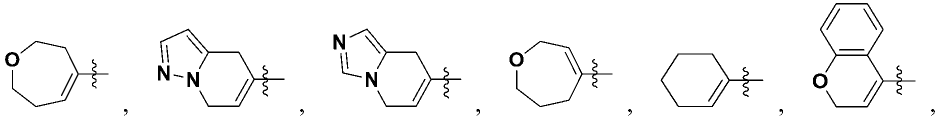 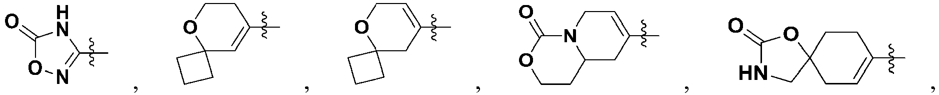 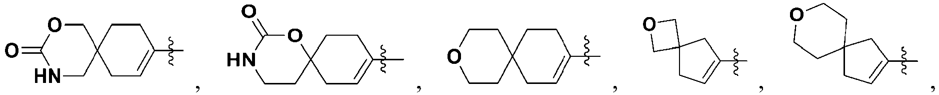 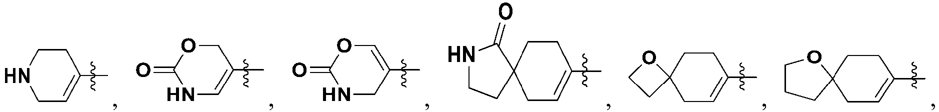 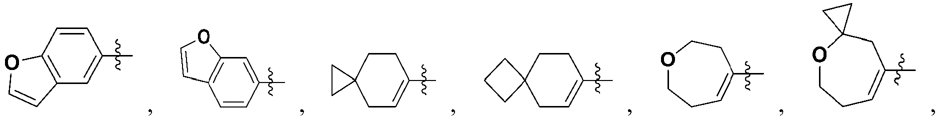 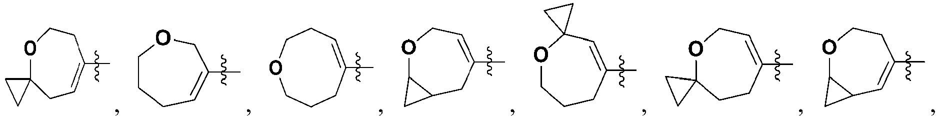 or

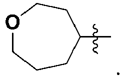

11. The compound, the stereoisomer thereof, or the pharmaceutically acceptable salt thereof according to claim 1, wherein the compound is represented by formula (VI), 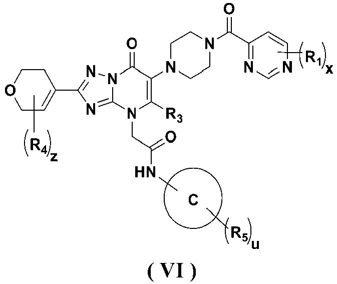 wherein
ring C is selected from the group consisting of 5-membered heteroaryl, 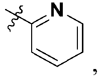 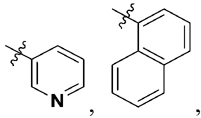 5- to 6-membered heteroaryl-fused 5- to 6-membered heteroaryl, benzo-fused 5- to 6-membered heteroaryl, benzo-fused 3- to 6-membered heterocycloalkenyl, 3- to 6-membered heterocycloalkenyl-fused phenyl, 5- to 6-membered heteroaryl-fused C₃₋₆ cycloalkenyl, and C₃₋₈ cycloalkyl; preferably, ring C is selected from the group consisting of

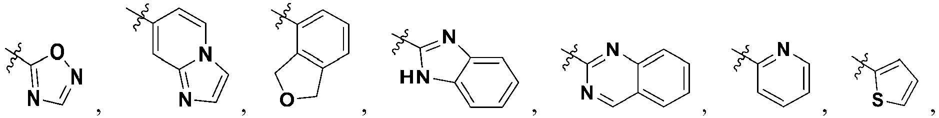

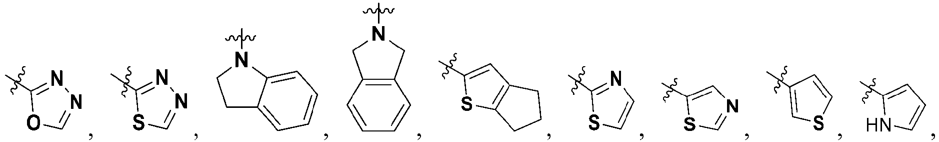

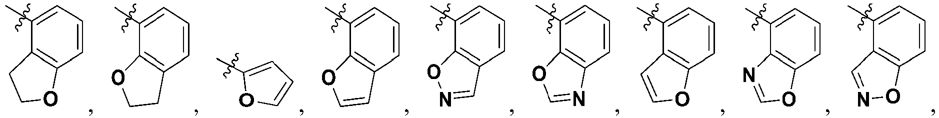

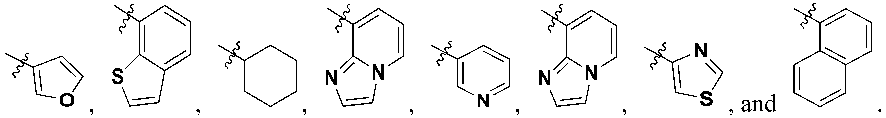

12. The compound, the stereoisomer thereof, or the pharmaceutically acceptable salt thereof according to any one of claims 1, 3, and 4, wherein the compound is represented by formula (XV),

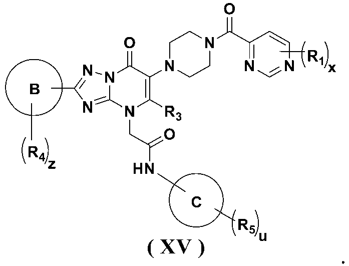

13. The compound, the stereoisomer thereof, or the pharmaceutically acceptable salt thereof according to any one of claims 1 to 12, wherein
R₁ is independently selected from the group consisting of hydrogen, deuterium, halogen, amino, hydroxyl, mercapto, cyano, C₁₋₃ alkyl, C₂₋₄ alkenyl, C₂₋₄ alkynyl, C₁₋₃ deuterated alkyl, C₁₋₃ haloalkyl, C₁₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₁ₐ; the R₁ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
preferably, R₁ is independently selected from the group consisting of hydroxyl, methyl, hydrogen, fluorine, 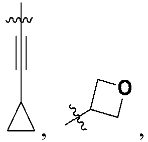 cyclopropyl, trifluoromethyl, mercapto, -CH₂OH, -CD₃, and -N(CH₃)₂.

14. The compound, the stereoisomer thereof, or the pharmaceutically acceptable salt thereof according to any one of claims 1 to 13, wherein
R₂ is independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₂ₐ; the R₂ₐ is independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
alternatively, two R₂ are connected to each other to form a C₃₋₆ cycloalkyl or a 3- to 6-membered heterocycloalkyl, wherein the C₃₋₆ cycloalkyl or 3- to 6-membered heterocycloalkyl is optionally further substituted with deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl;
alternatively, one R₂ and R₃ are connected to form 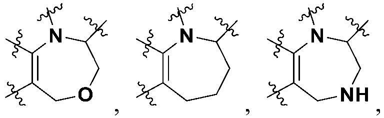 or 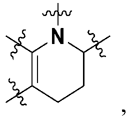 wherein the 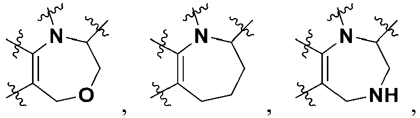 or 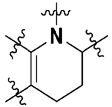 is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
preferably, R₂ is independently hydrogen and methyl.

15. The compound, the stereoisomer thereof, or the pharmaceutically acceptable salt thereof according to any one of claims 1 to 14, wherein
R₃ is selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₃ₐ; the R₃ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
L₁ and R₃ are connected to form a 3- to 8-membered heterocycloalkenyl or a 5- to 6-membered heteroaryl; wherein the 3- to 8-membered heterocycloalkenyl or 5- to 10-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 10-membered heteroaryl;
R₃ is selected from the group consisting of ethyl, trifluoromethoxy, vinyl, ethynyl, cyano, and

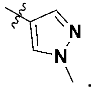

16. The compound, the stereoisomer thereof, or the pharmaceutically acceptable salt thereof according to any one of claims 1 to 15, wherein
R₄ is independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, 5- to 6-membered heteroaryl, -COR₄₋₁, -COOR₄₋₁, - OC(=O)R₄₋₁, -SO₂R₄₋₂, and -OR₄₋₃, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₄ₐ; the R₄ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl; or, two R₄ₐ are connected to form a 3- to 6-membered heterocycloalkyl;
R₄₋₁ is selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
R₄₋₂ is selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
R₄₋₃ is selected from the group consisting of hydrogen, deuterium, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₃₋₆ cycloalkyl, 3- to 6-membered heterocycloalkyl, C₆₋₁₀ aryl, and 5- to 6-membered heteroaryl, wherein the C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
preferably, R₄ is independently selected from the group consisting of hydrogen, methyl, 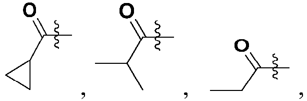 -SO₂CH₃, 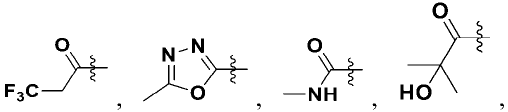 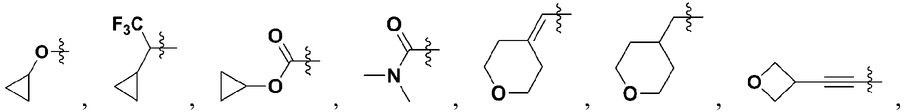 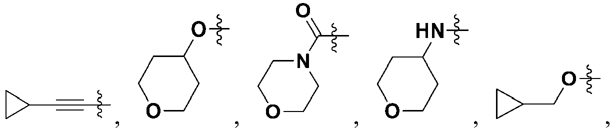 -CH₂CN, 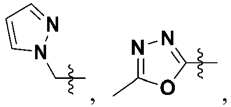 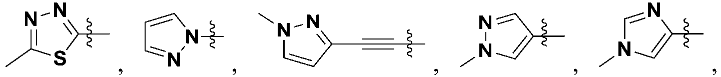 phenyl, 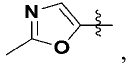 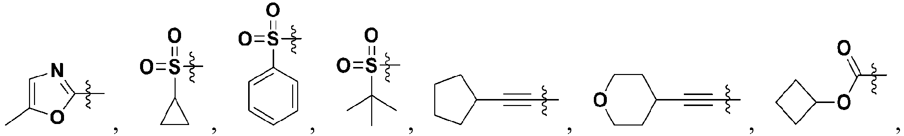 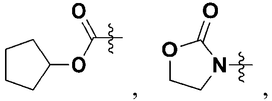 cyclopropyl, 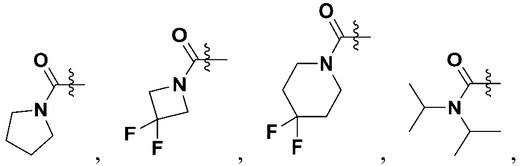 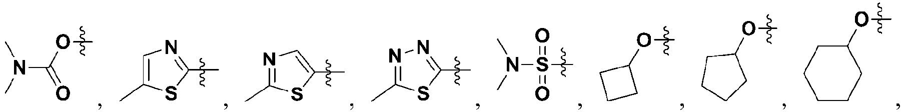 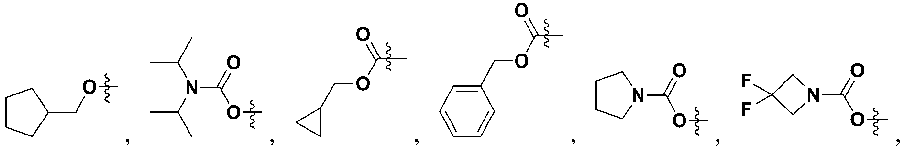 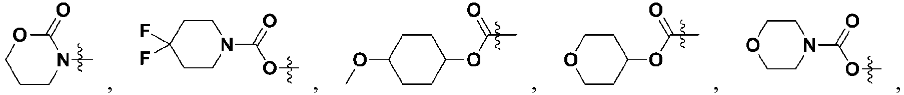 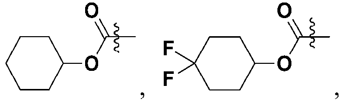 fluorine, and deuterium.

17. The compound, the stereoisomer thereof, or the pharmaceutically acceptable salt thereof according to any one of claims 1 to 16, wherein
R₅ is independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, -SO₂-C₁₋₃ alkyl, -SF₅, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₅ₐ; the R₅ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
preferably, R₅ is independently selected from the group consisting of hydrogen, chlorine, trifluoromethyl, fluorine, methyl, cyclopropyl, 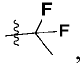 -SF₅, ethyl, and -SO₂CH₃.

18. The compound, the stereoisomer thereof, or the pharmaceutically acceptable salt thereof according to any one of claims 1 to 17, wherein
L₁ is selected from the group consisting of a bond, -O-, -S-, imino, C₁₋₃ alkylene, -CONH-, -CO-C₁₋₃ alkylene-NH-, -C₁₋₃ alkylene-CONH-, -C₁₋₃ alkylene-CONH-C₁₋₃ alkylene, -C₁₋₃ alkylene-SO₂NH-, -C₁₋₃ alkylene-SO₂NH-, -C₁₋₃ alkylene-NHCONH-, -C₁₋₃ alkylene-NHCO-, -C₁₋₃ alkylene-NHSO₂-, -C₁₋₃ alkylene-CO-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃ haloalkyleneoxy, C₃₋₈ cycloalkylene, 3- to 8-membered heterocycloalkylene, 3- to 8-membered heterocycloalkenylene, -C₁₋₃ alkylene-3- to 8-membered heterocycloalkylene-, -C₁₋₃ alkylene-3- to 8-membered heterocycloalkenylene-, -C₁₋₃ alkylene-C₆₋₁₀ arylene-, C₆₋₁₀ arylene, -C₁₋₃ alkylene-5- to 6-membered heteroarylene-, and 5- to 6-membered heteroarylene, wherein the imino, C₁₋₃ alkylene, -CONH-, -CO-C₁₋₃ alkylene-NH-, -C₁₋₃ alkylene-CONH-, -C₁₋₃ alkylene-CONH-C₁₋₃ alkylene-C₁₋₃ alkylene-SO₂NH-, -C₁₋₃ alkylene-SO₂NH-, -C₁₋₃ alkylene-NHCONH-, -C₁₋₃ alkylene-NHCO-, -C₁₋₃ alkylene-NHSO₂-, -C₁₋₃ alkylene-CO-, -C₁₋₃ alkylene-C(=N-OH)NH-, C₂₋₄ alkenylene, C₂₋₄ alkynylene, C₁₋₃ deuterated alkylene, C₁₋₃ haloalkylene, C₁₋₃ hydroxyalkylene, C₁₋₃ alkyleneoxy, C₁₋₃ alkylenethio, C₁₋₃ haloalkyleneoxy, C₃₋₈ cycloalkylene, 3- to 8-membered heterocycloalkylene, 3- to 8-membered heterocycloalkenylene, -C₁₋₃ alkylene-3- to 8-membered heterocycloalkylene-, -C₁₋₃ alkylene-3- to 8-membered heterocycloalkenylene-, -C₁₋₃ alkylene-C₆₋₁₀ arylene-, C₆₋₁₀ arylene, -C₁₋₃ alkylene-5- to 6-membered heteroarylene-, or 5- to 6-membered heteroarylene is optionally further substituted with one or more substituents selected from the group consisting of deuterium, halogen, amino, hydroxyl, cyano, oxo, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
more preferably, L₁ is selected from the group consisting of -CH₂-, -CH₂CONH-*, - CH₂SO₂NH-*, -COCH₂NH-*, -CONH-*, -CH₂CO-*, -CH₂CON(CH₃)-*, -CH₂CONHCH₂-*, - CH₂CH₂CONH-*, 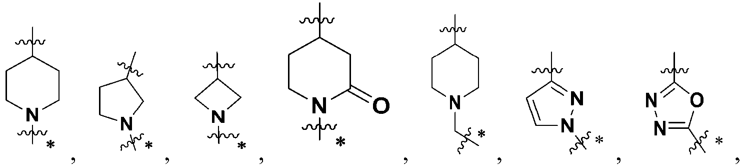 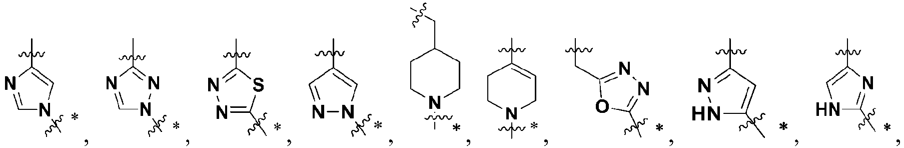 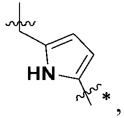 and -CH₂-C(=N-OH)NH-*, wherein the side with * is connected to ring C.

19. The compound, the stereoisomer thereof, or the pharmaceutically acceptable salt thereof according to any one of claims 1 to 18, wherein
R₆₋₁ and R₆₋₂ are each independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more R₅ₐ; the R₅ₐ is independently selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, and 5- to 6-membered heteroaryl, wherein the amino, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₃₋₆ cycloalkyl, 3- to 6-membered heterocycloalkyl, C₆₋₁₀ aryl, or 5- to 6-membered heteroaryl is optionally further substituted with one or more substituents selected from the group consisting of deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3- to 6-membered heterocycloalkyl, C₆₋₁₀ aryl, and 5- to 6-membered heteroaryl;
alternatively, R₆₋₁ and R₆₋₂, together with the carbon atom to which they are attached, form a C₃₋₆ cycloalkyl or a 3- to 6-membered heterocycloalkyl;
preferably, R₆₋₁ is selected from the group consisting of hydrogen, cyano, and trifluoromethyl; R₆₋₂ is hydrogen;
or preferably, R₆₋₁, R₆₋₂, together with the carbon atom to which they are attached, form 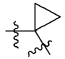 or

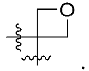

20. The compound, the stereoisomer thereof, or the pharmaceutically acceptable salt thereof according to any one of claims 1 to 19, wherein
R₇₋₁ and R₇₋₂ are each independently selected from the group consisting of hydrogen, deuterium, halogen, amino, hydroxyl, cyano, C₁₋₃ alkyl, C₂₋₄ alkenyl, C₂₋₄ alkynyl, C₁₋₃ deuterated alkyl, C₁₋₃ haloalkyl, C₁₋₃ hydroxyalkyl, C₁₋₃ alkoxy, C₁₋₃ alkylthio, and C₁₋₃ haloalkoxy; preferably, R₇₋₁ is selected from the group consisting of hydrogen and methyl; R₇₋₂ is methyl;
alternatively, R₇₋₁ and R₇₋₂ are connected to form a C₃₋₆ cycloalkyl; preferably, R₇₋₁ and R₇₋₂ are connected to form a cyclopropyl.

21. The compound, the stereoisomer thereof, or the pharmaceutically acceptable salt thereof according to any one of claims 1 to 20, wherein the compound is as shown below:

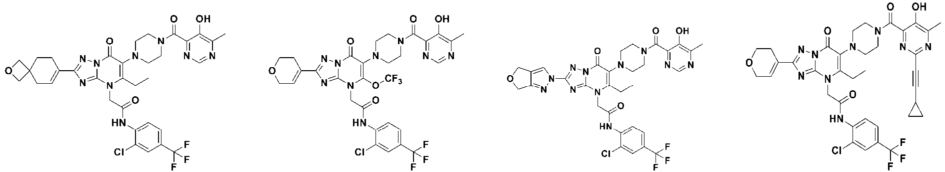

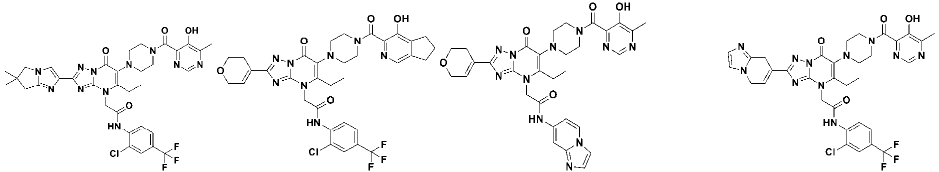

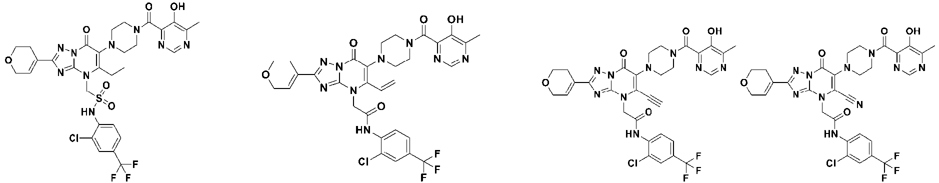

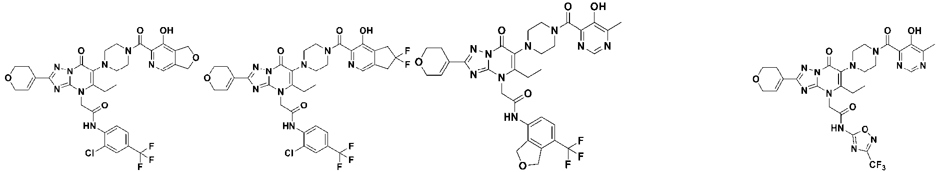

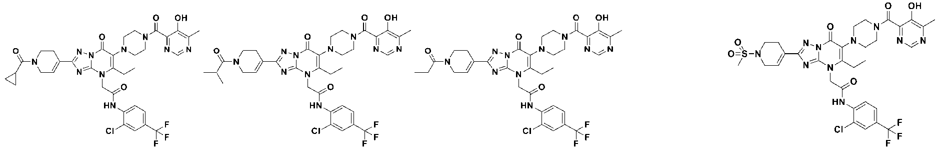

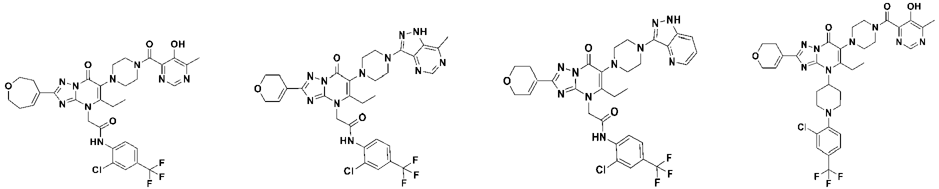

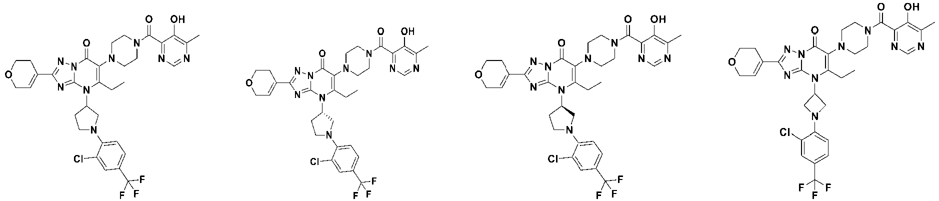

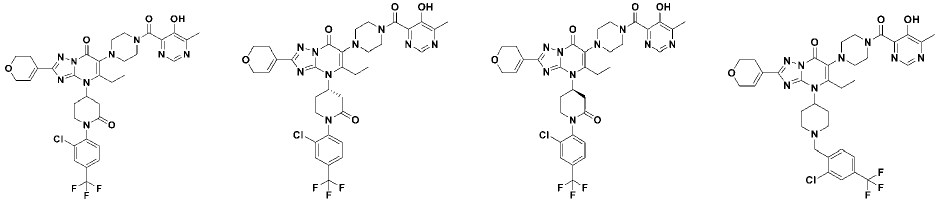

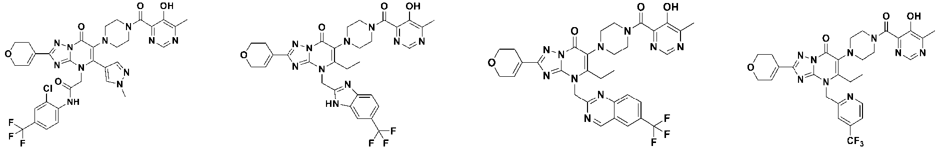

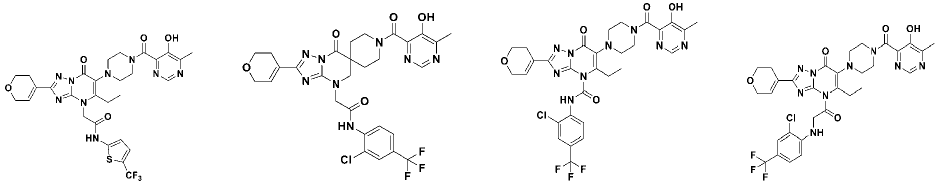

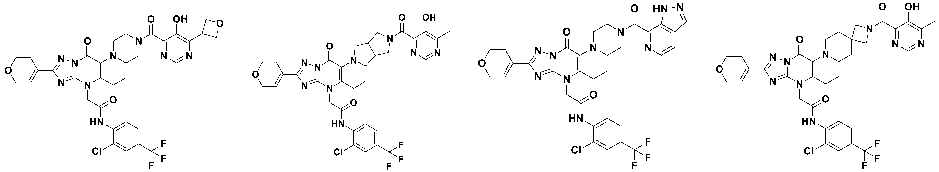

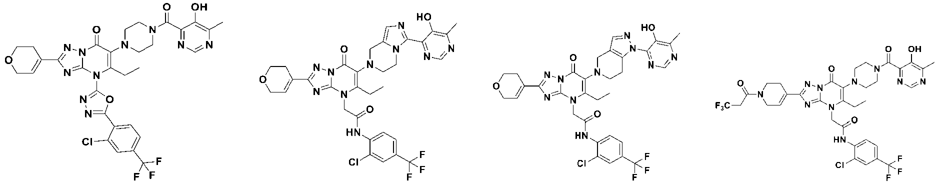

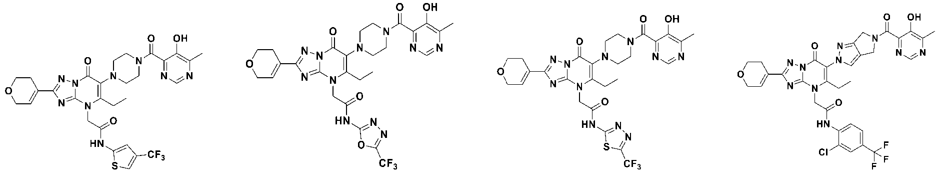

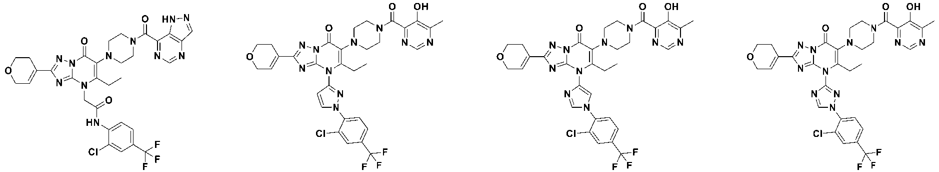

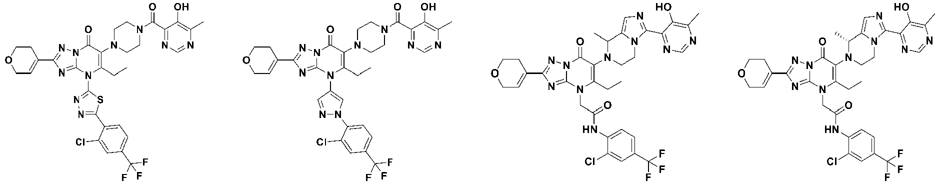

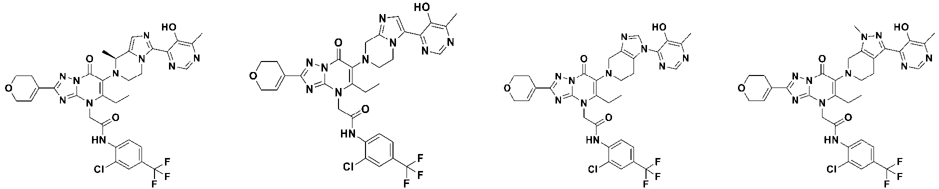

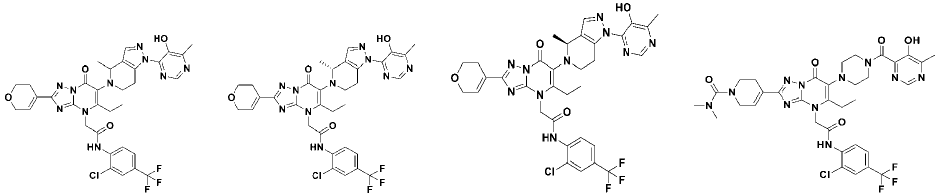

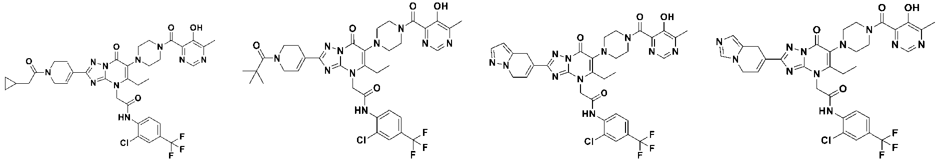

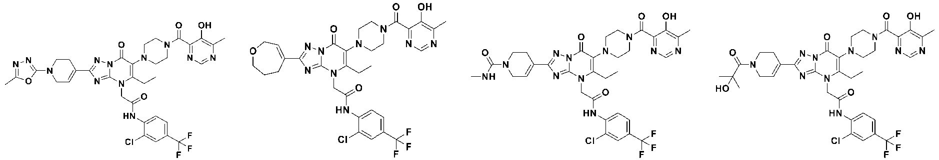

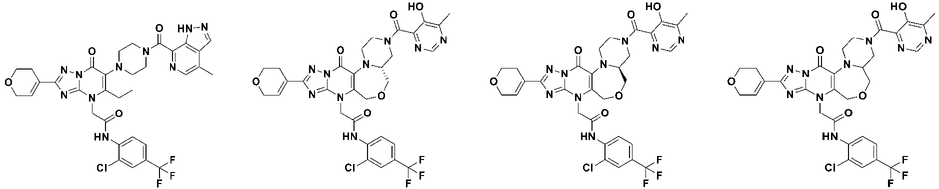

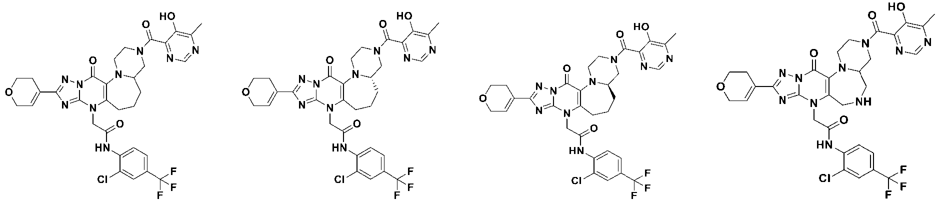

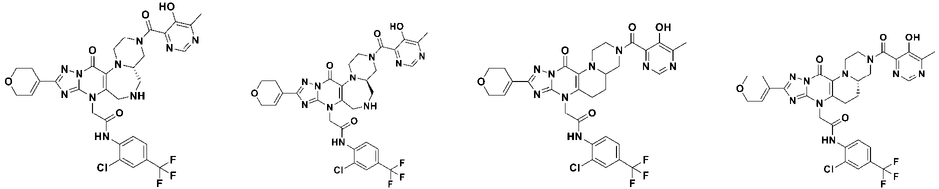

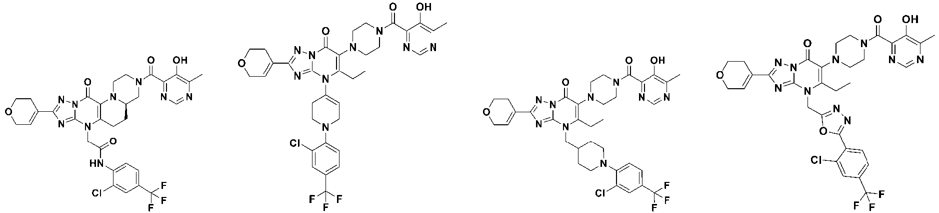

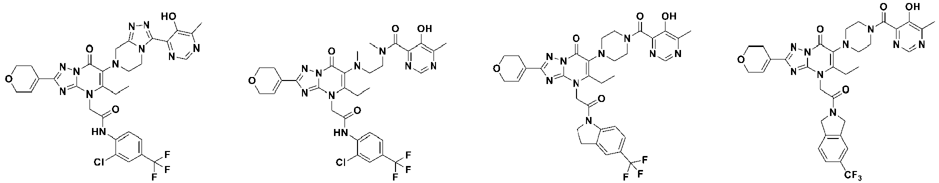

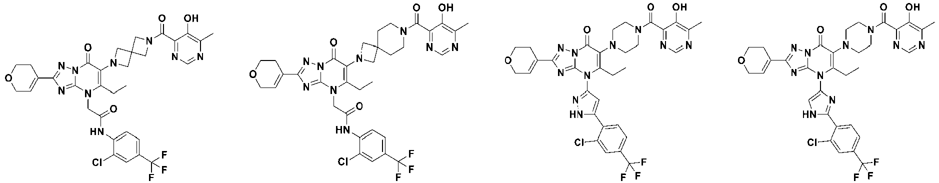

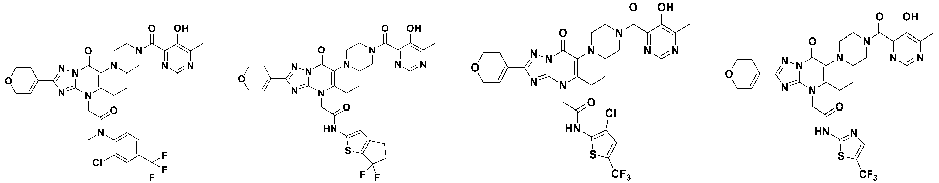

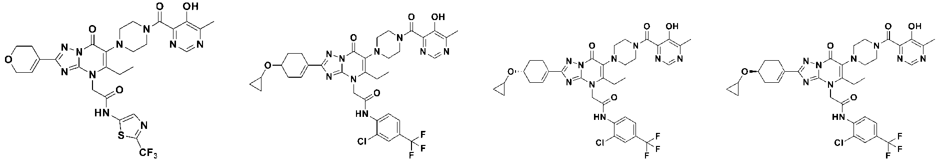

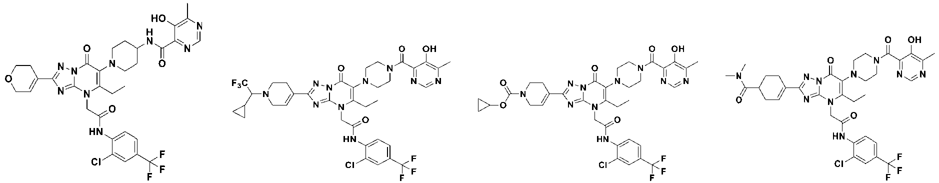

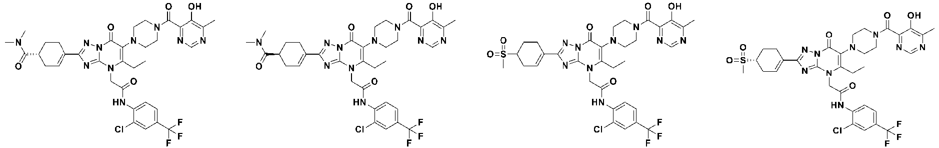

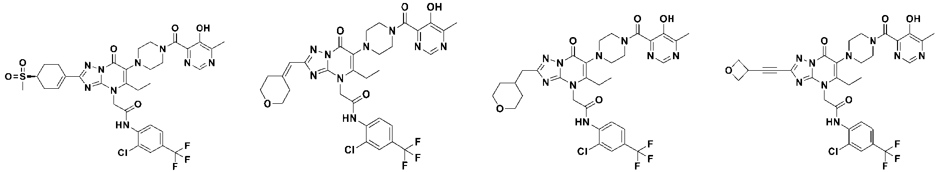

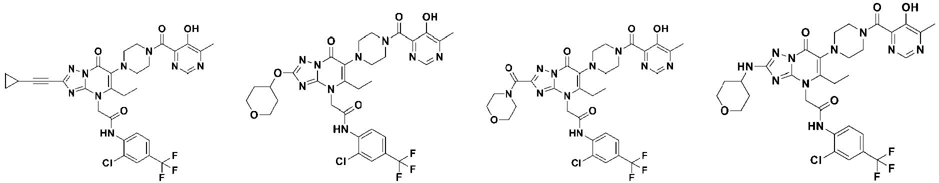

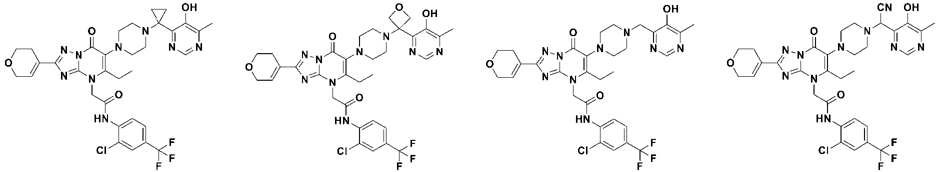

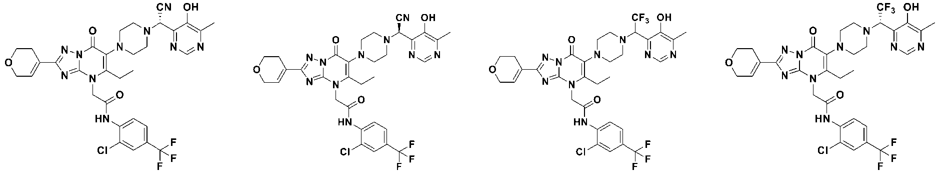

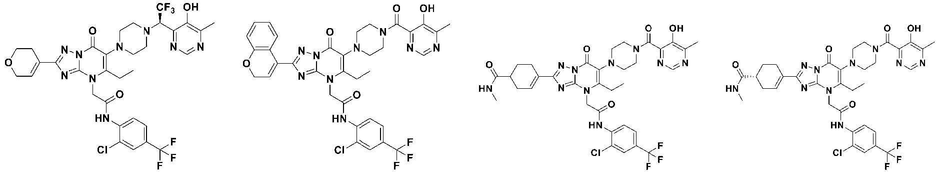

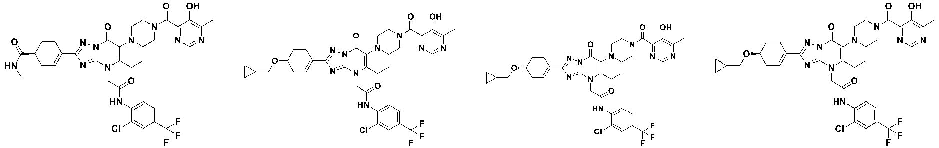

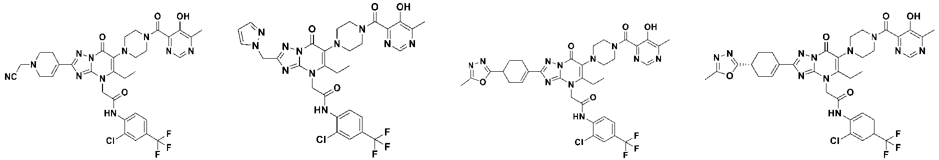

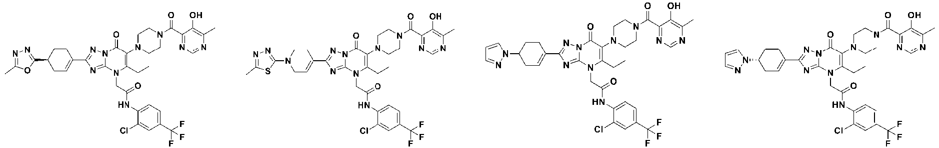

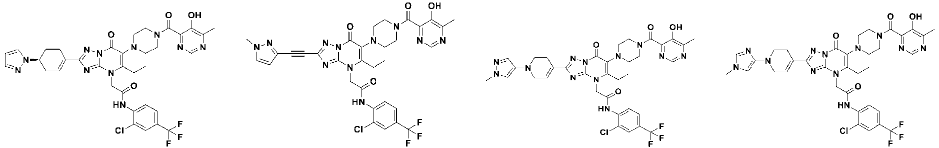

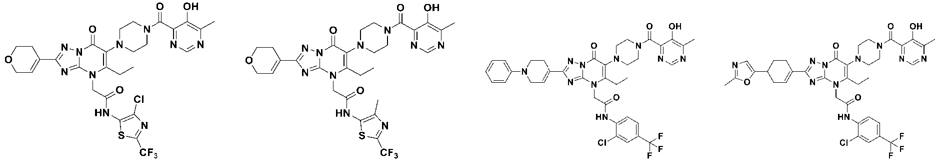

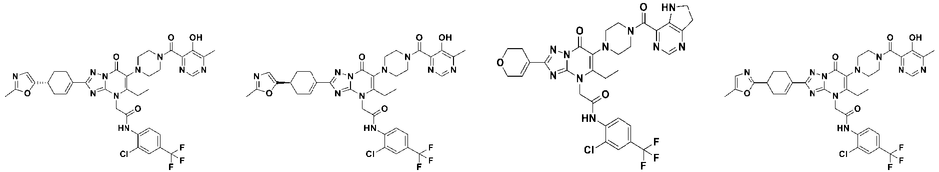

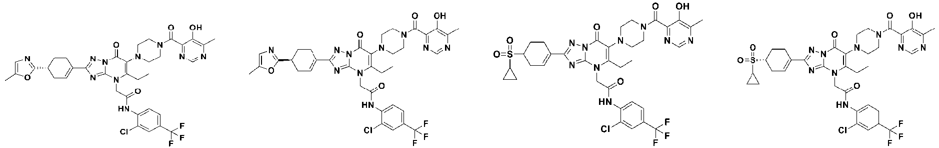

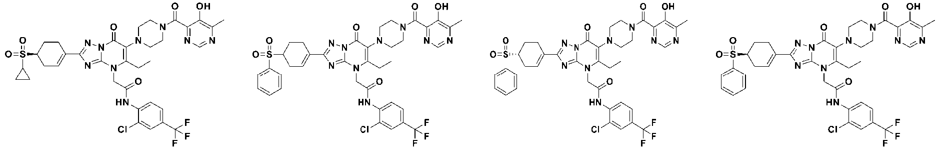

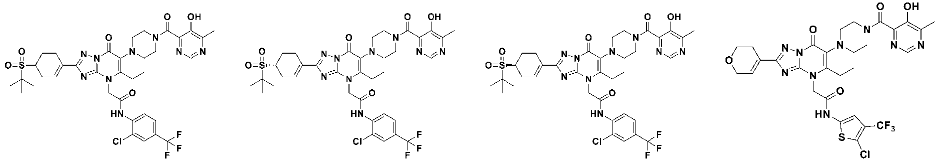

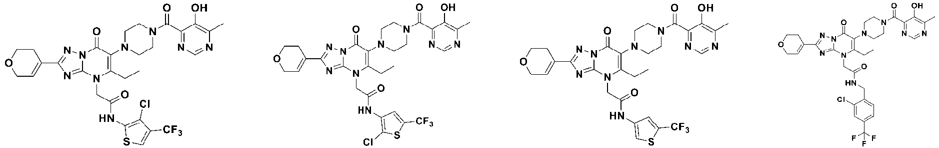

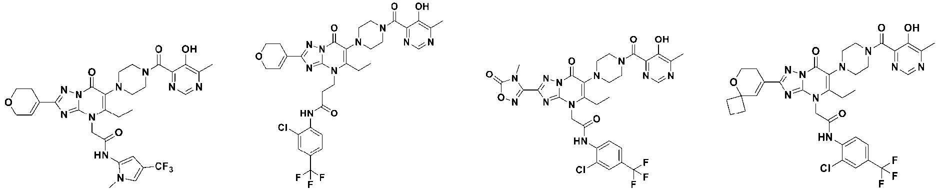

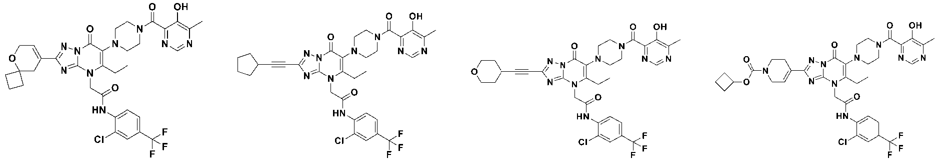

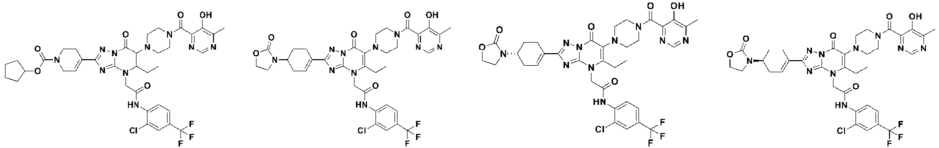

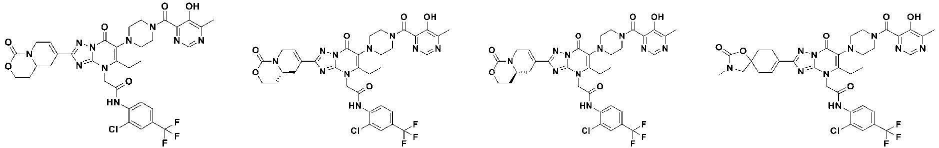

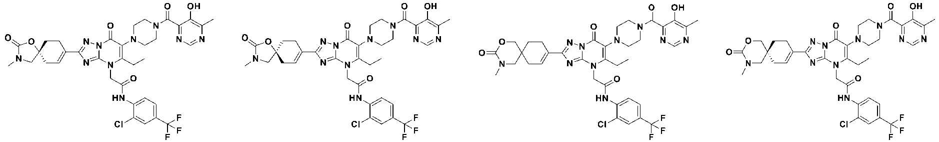

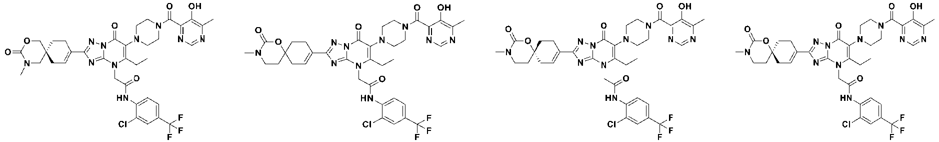

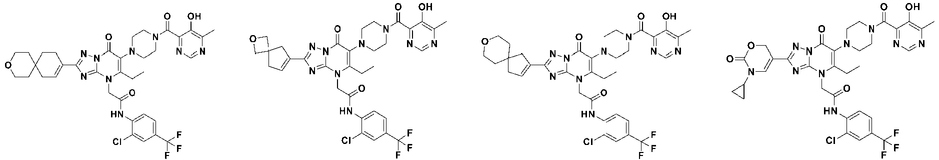

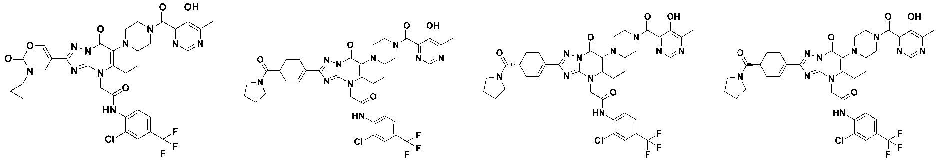

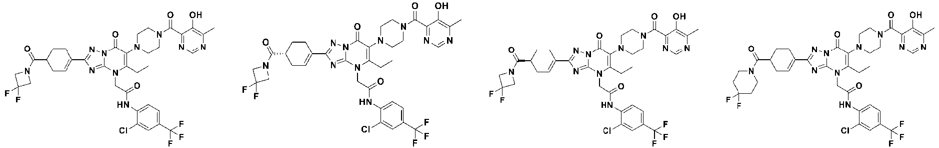

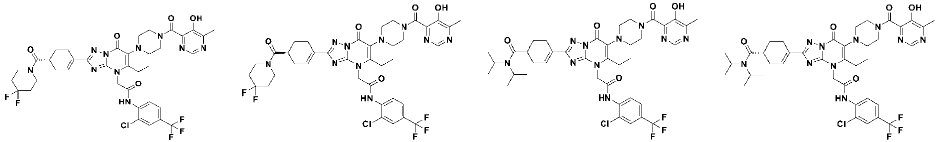

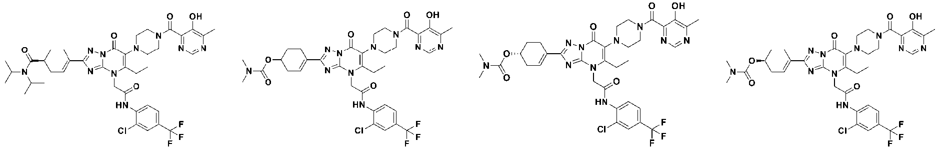

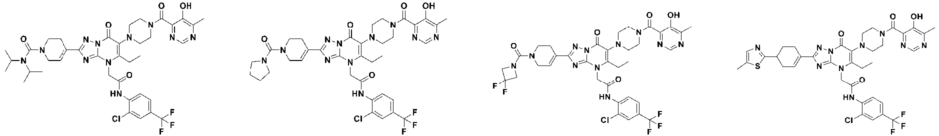

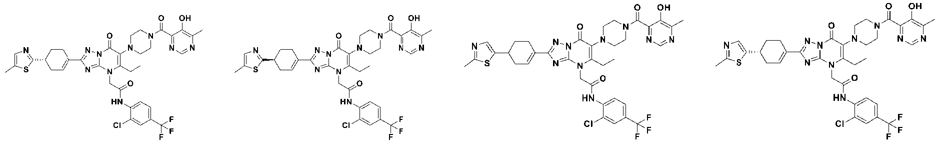

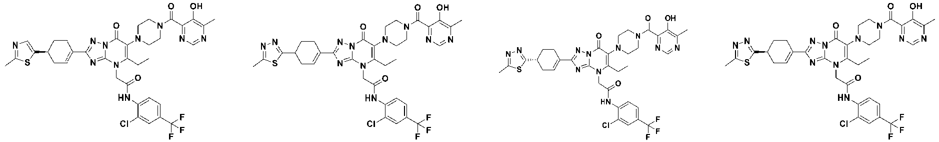

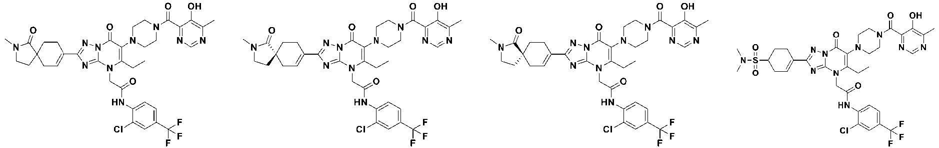

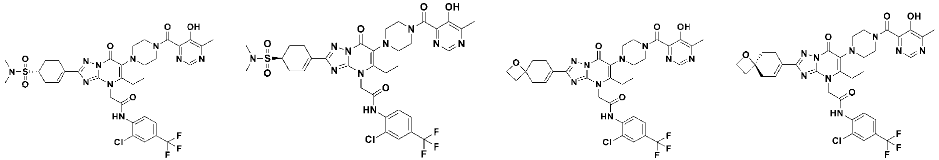

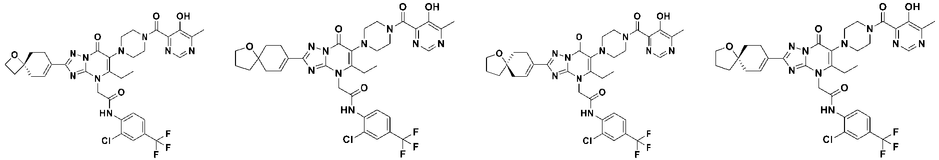

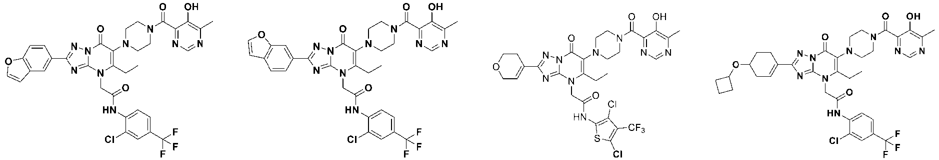

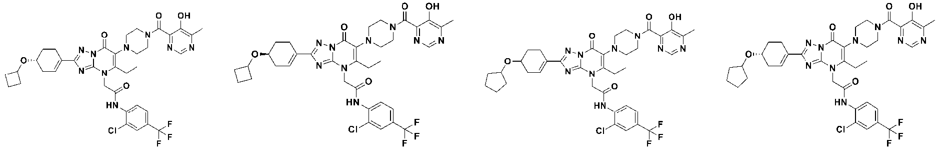

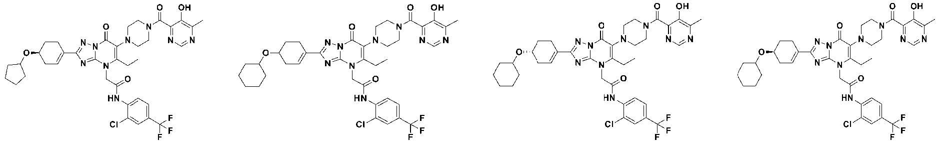

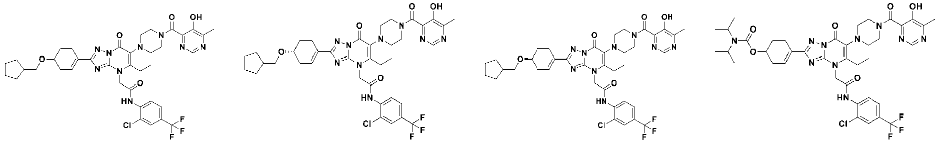

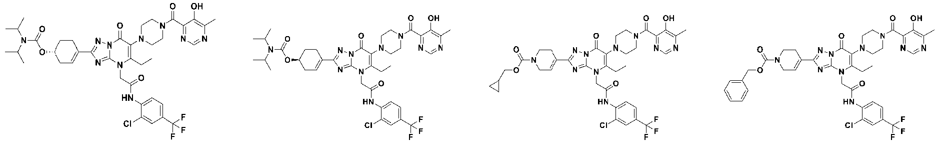

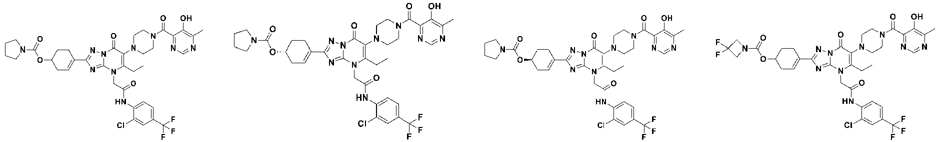

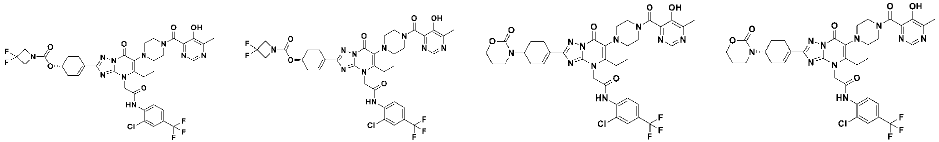

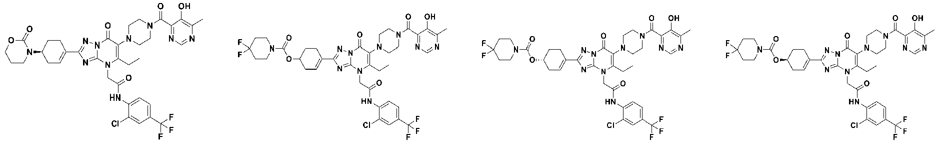

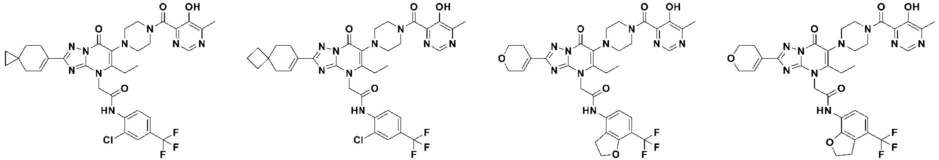

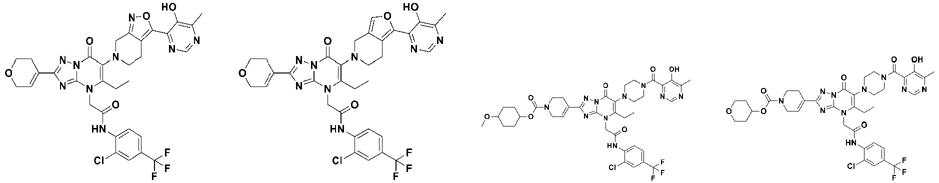

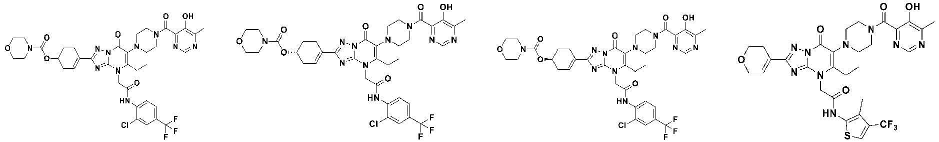

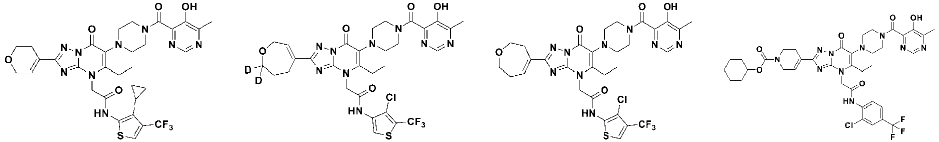

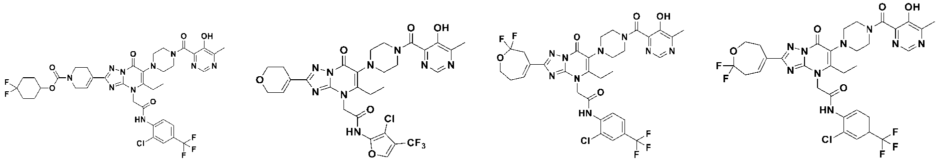

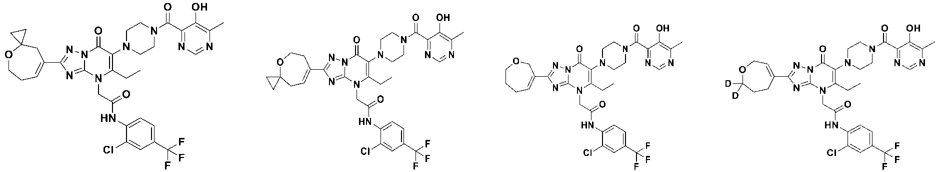

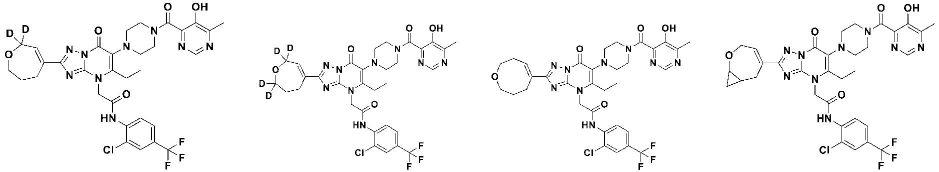

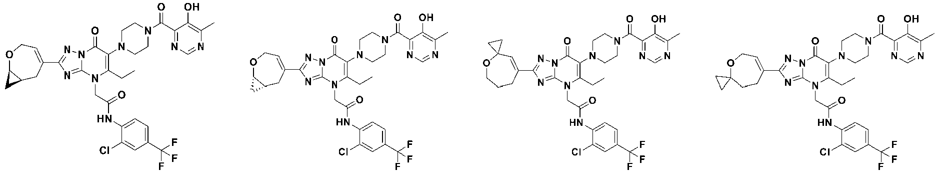

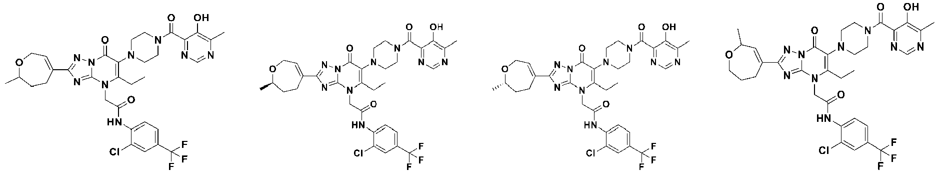

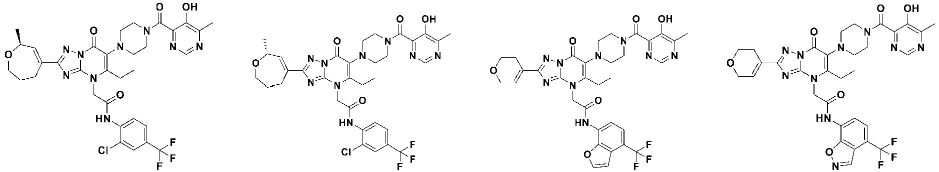

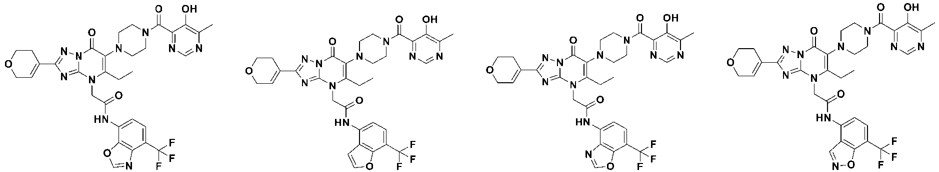

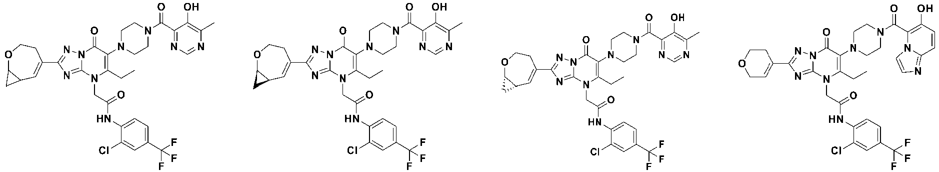

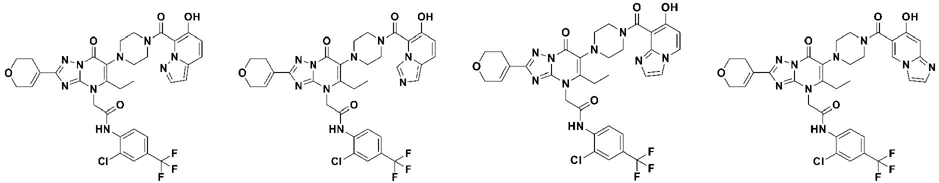

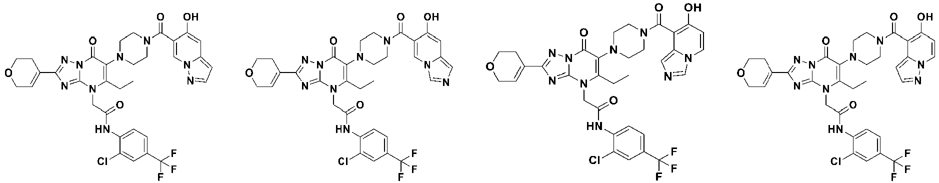

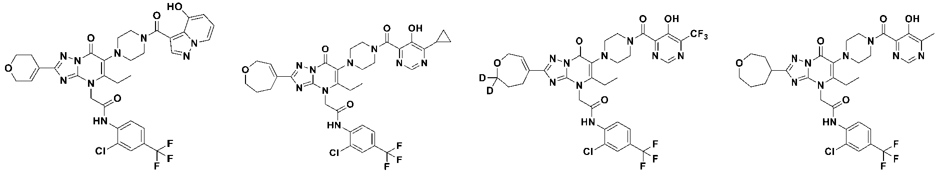

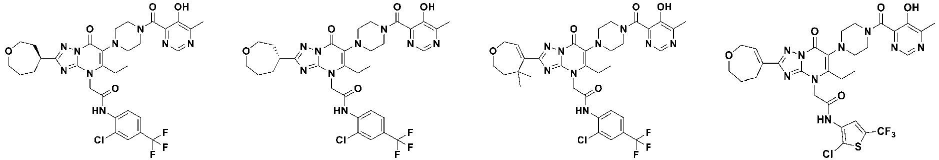

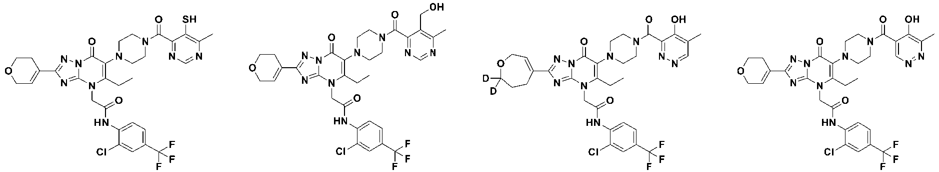

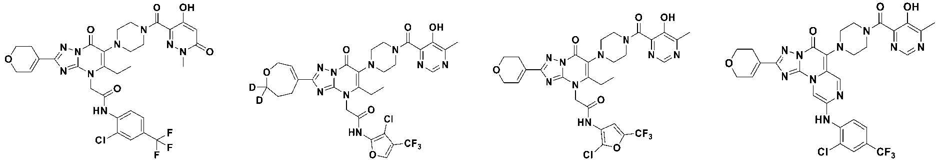

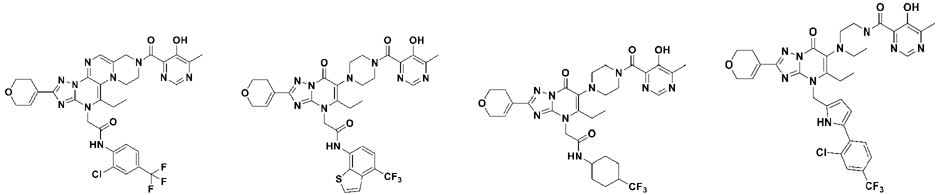

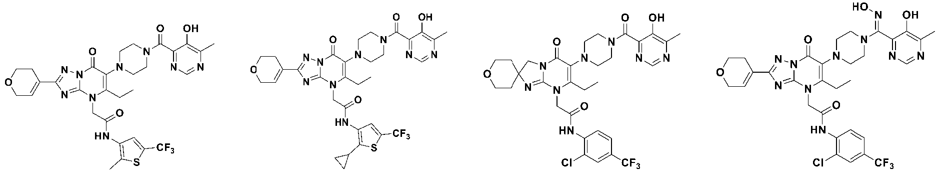

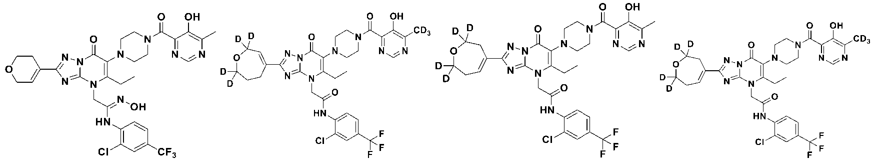

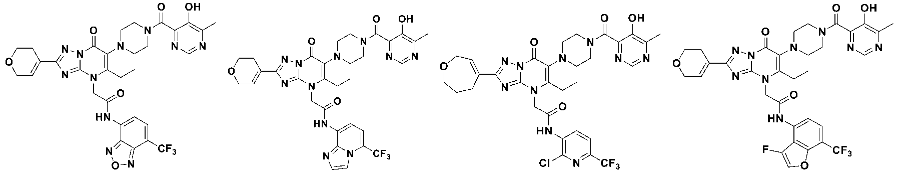

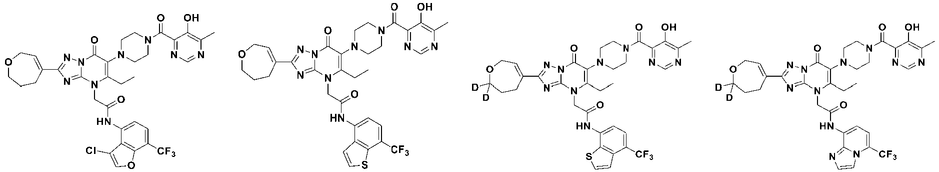

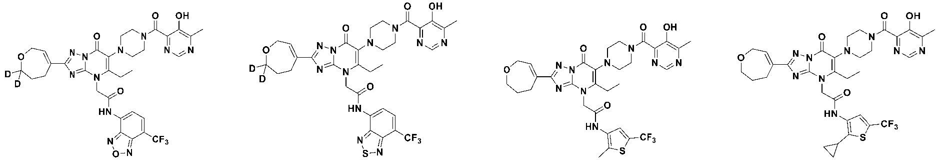

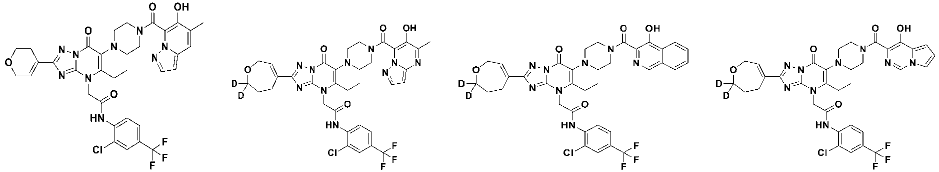

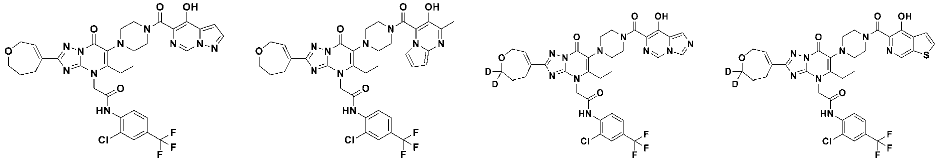

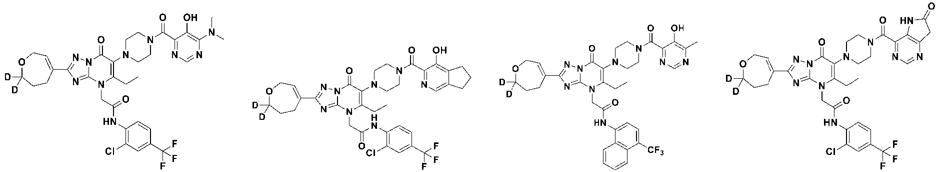

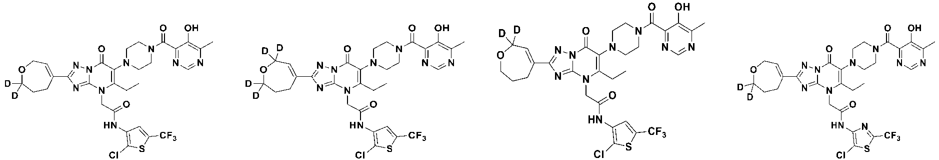

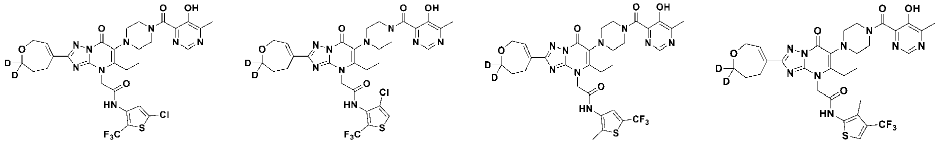

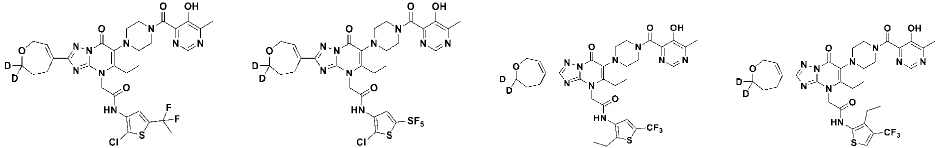

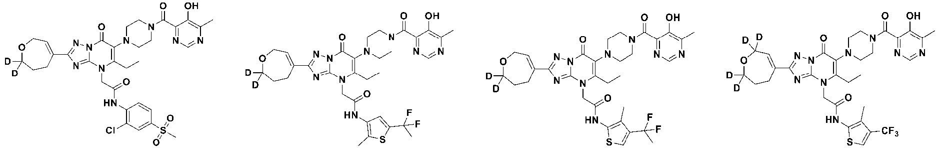

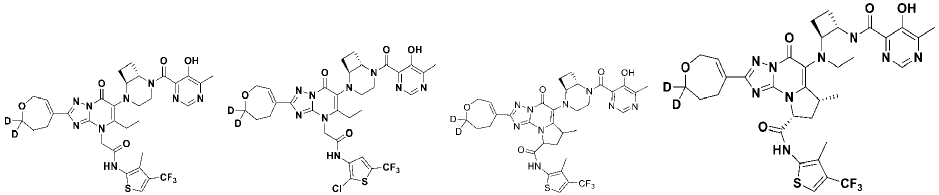

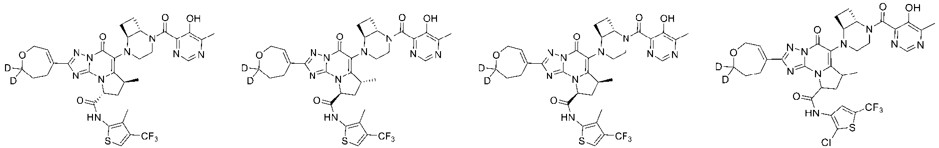

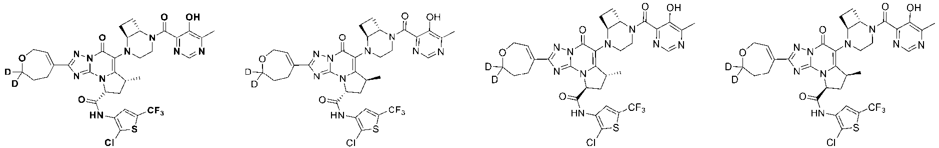

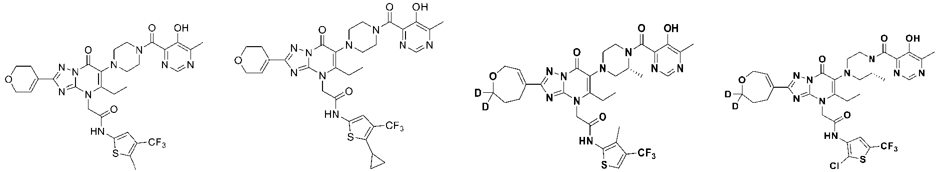

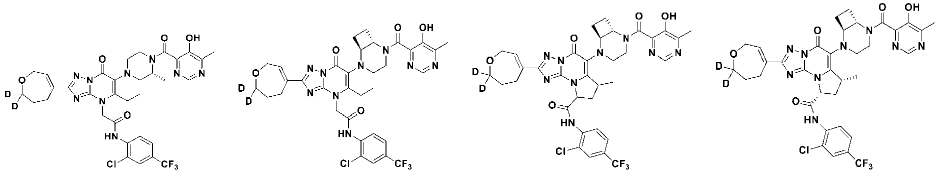

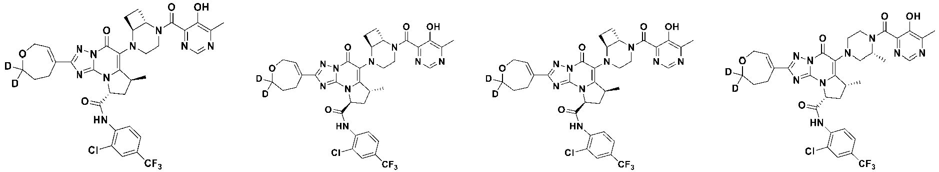

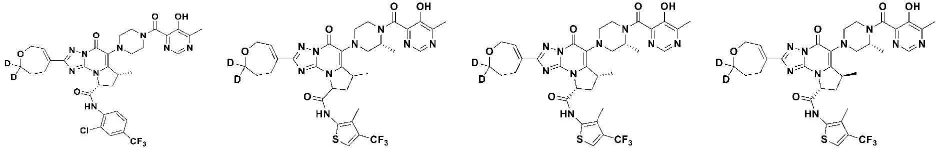

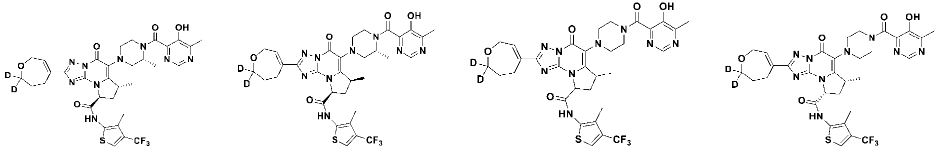

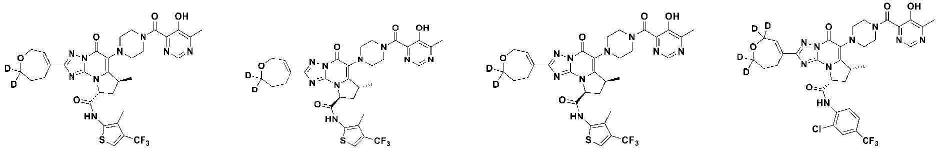

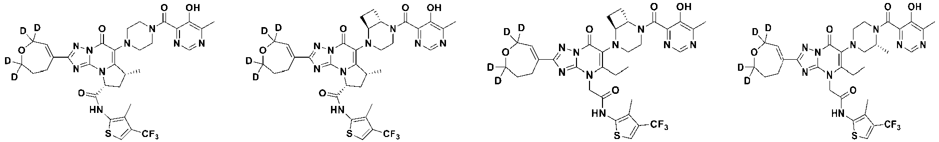

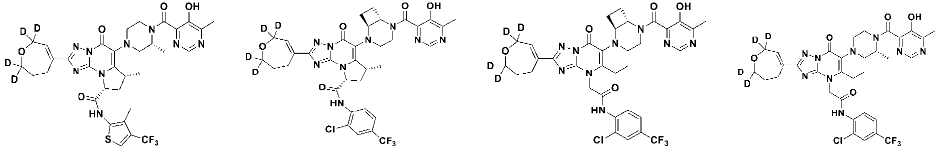

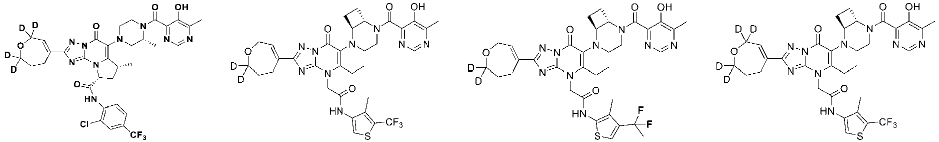

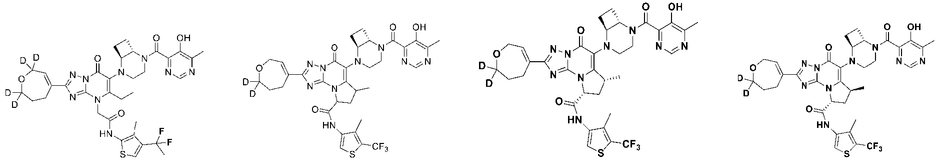

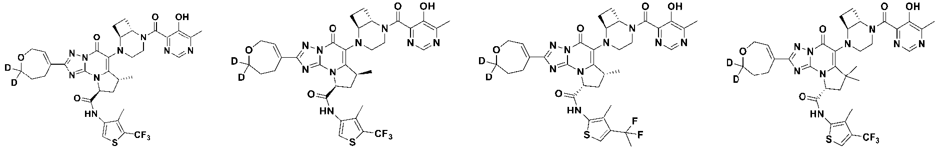

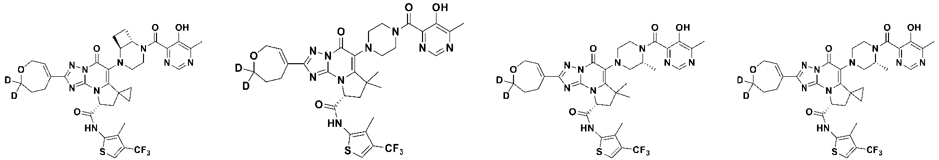

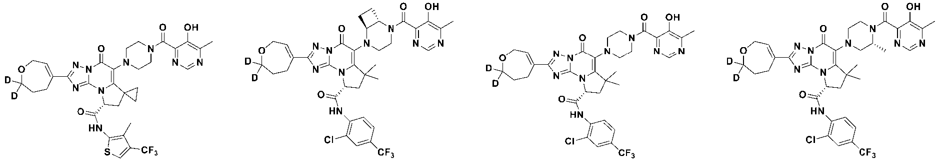

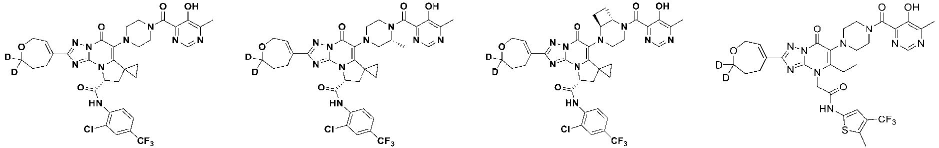

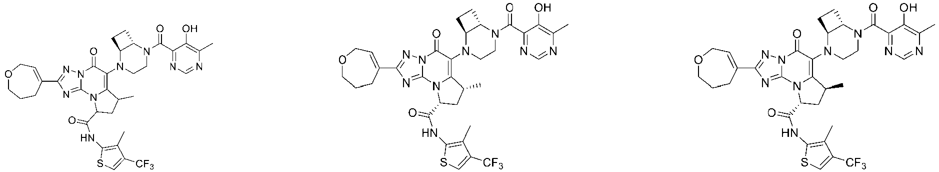

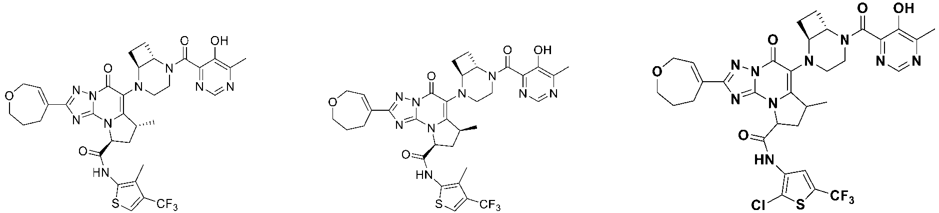

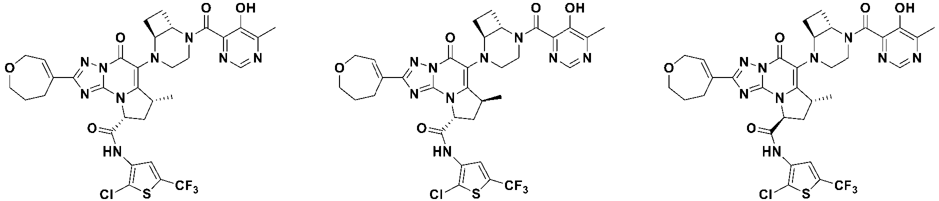

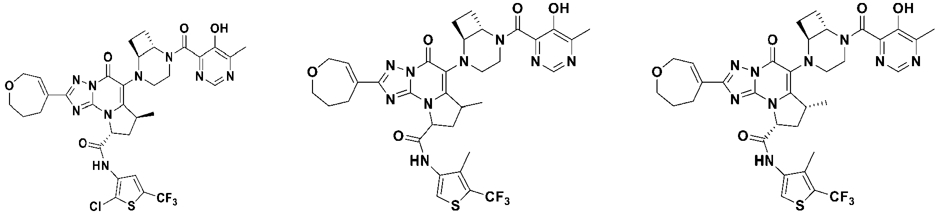

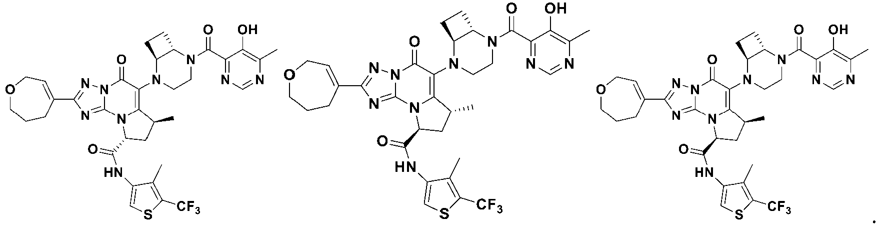

22. The compound, the stereoisomer thereof, or the pharmaceutically acceptable salt thereof according to any one of claims 6 to 9, wherein the compound, the stereoisomer thereof, or the pharmaceutically acceptable salt thereof satisfies one or more of the following conditions:
(1) R₁ is independently selected from the group consisting of hydroxyl and C₁₋₆ alkyl;
(2) R₄ is deuterium;
(3) R₅ is independently selected from the group consisting of halogen, C₁₋₆ alkyl, and C₁₋₆ haloalkyl;
(4) x is selected from the group consisting of 0, 1, and 2; preferably 2;
(5) R₂₋₁ is hydrogen; (6) R₇₋₁ and R₇₋₂ are each independently selected from the group consisting of hydrogen and C₁₋₆ alkyl;
(7) z is selected from the group consisting of 0, 1, and 2; preferably 2;
(8) u is selected from the group consisting of 0, 1, and 2; preferably 2.

23. A pharmaceutical composition comprising the compound, the stereoisomer thereof, or the pharmaceutically acceptable salt thereof according to any one of claims 1 to 22, and a pharmaceutically acceptable carrier.

24. Use of the compound, the stereoisomer thereof, or the pharmaceutically acceptable salt thereof according to any one of claims 1 to 22, or the pharmaceutical composition according to claim 23 in the manufacture of a medicament for treating cancer; the cancer is selected from the group consisting of colorectal cancer, endometrial cancer, ovarian cancer, and gastric cancer; more preferably, the cancer is microsatellite instability-high or mismatch repair-deficient cancer.
